# EUROPEAN PATENT SPECIFICATION

(11) **EP 4 055 019 B1**
(45) Date of publication and mention of the grant of the patent: **22.01.2025**
(21) Application number: 19816986.4
(22) Date of filing: 08.11.2019
(51) Int. Cl.: C07D 487/04, A61P 35/00, A61P 37/00

(54) **BIFUNCTIONAL COMPOUNDS FOR DEGRADING BTK VIA UBIQUITIN PROTEOSOME PATHWAY**
BIFUNKTIONELLE VERBINDUNGEN ZUM ABBAU VON BTK ÜBER DEN UBIQUITIN-PROTEOSOM-SIGNALWEG
COMPOSÉS BIFONCTIONNELS POUR LA DÉGRADATION DE BTK PAR L'INTERMÉDIAIRE DE LA VOIE DE L'UBIQUITINE-PROTÉOSOME

(43) Date of publication of application: 14.09.2022
(73) Proprietor: Nurix Therapeutics, Inc., San Francisco CA 94158 (US)
(72) Inventor: ROBBINS, Daniel, W., San Francisco, CA 94158 (US); PENG, Ge, San Francisco, CA 94158 (US); MIHALIC, Jeffrey, San Francisco, CA 94158 (US); SANDS, Arthur, T., San Francisco, CA 94158 (US)
(74) Representative: Petty, Catrin Helen
(86) International application number: PCT/US2019/060584
(87) International publication number: WO 2021/091575

(56) References cited:
- WO-A1-2009/098144
- WO-A1-2011/140488
- WO-A1-2012/020008
- WO-A1-2013/067264
- WO-A1-2013/067274
- WO-A1-2014/040965
- WO-A1-2018/098275
- WO-A1-2019/148150

## Description

### FIELD OF THE INVENTION

The present invention provides novel bifunctional compounds for proteolytically degrading targeted Bruton's tyrosine kinases (BTK), and compositions comprising the same. The disclosure also extends to medical uses of said compounds and compositions in the treatment of diseases modulated by BTK.

### BACKGROUND

B cell receptor (BCR) signaling controls B cell development, as well as mature B cell activation, signaling and survival. Mis-regulation of the BCR signaling pathway is associated with numerous disease indications involving B cell function, and targeting B cells and BCR signaling has clear therapeutic potential (Woyach, et al.; Blood. 120(6); 1175-1184. 2012.). For example, depletion of B cells with monoclonal antibodies targeting CD20 has significant effects in treatment of B cell malignancies and auto-immune and inflammatory diseases (Cang, et al.; J Hematolo Oncol. 5; 64, 2012.).

BTK is a member of the TEC family of kinases and is a crucial signaling hub in the BCR pathway. Mutations in BTK result in X-linked agammaglobulinaemia (XLA), in which B cell maturation is impaired, resulting in reduced immunoglobulin production (Hendriks, et al.; Expert Opin Ther Targets 15; 1002-1021, 2011.). The central role of BTK in B cell signaling and function makes BTK an attractive therapeutic target for B cell malignancies as well as autoimmune and inflammatory diseases. Ibrutinib, a covalent inhibitor of BTK, has been approved to treat chronic lymphocytic leukemia (CLL), mantle cell lymphoma (MCL) and other B cell malignancies, as well as graft-versus-host disease (GvHD) (Miklos, et al.; Blood. 120(21); 2243-2250. 2017). Currently, ibrutinib and second-generation BTK inhibitors are being investigated for oncology and immune-related indications such as rheumatoid arthritis (Akinleye, et al.; J of Hematolo Oncol. 6: 59, 2013; Liu, et al.; J Pharm and Exper Ther. 338(1): 154-163. 2011; Di Paolo, et al.; Nat Chem Biol. 7(1): 41-50. 2011).

As an alternative to stoichiometric inhibition, proteolytic degradation of BTK could have dramatic consequences for B cell function by effectively blocking BCR signaling. Removal of BTK protein would eliminate BTK kinase activity as well as any protein interaction or scaffolding function of BTK. Specific degradation of BTK could be accomplished using heterobifunctional small molecules to recruit BTK to a ubiquitin ligase and thus promoting ubiquitylation and proteasomal degradation of BTK. Thalidomide derivatives, such as lenalidomide or pomalidomide, can be used to recruit potential substrates to cereblon (CRBN), a component of a ubiquitin ligase complex. This unique therapeutic approach could present a mechanism of action for interfering with BTK activity and BCR signaling that is distinct from the mechanism of stoichiometric BTK inhibition. Furthermore, this degradative approach could effectively target the C481S mutated form of BTK, which mutation has been clinically observed and confers resistance to inhibition by ibrutinib (Woyach, et al.; Blood. 120(6): 1175-1184. 2012.).

WO 2019/148150 and WO 2018/098275 relate to bifunctional compounds which act as protein degradation inducing moieties for BTK, and methods of targeted degradation of BTK through use of said compounds.

Presently, there remains a need for bifunctional molecules that can induce the proteolytic degradation of BTK via a ubiquitin proteolytic pathway.

### SUMMARY OF THE INVENTION

The subject-matter for which protection is sought is as defined by the claims. Any reference to a "disclosure" or an "embodiment" not falling within the scope of the claims is present for explanatory purposes only and does not form part of the invention.

The present disclosure provides bifunctional compounds that induce the proteolytic degradation of BTK via a ubiquitin proteolysis pathway. The present disclosure provides a compound of Formula (I): 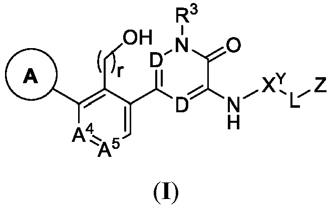 or a pharmaceutically acceptable salt thereof, wherein each of A⁴ and A⁵ is independently N or C-R¹⁵, each R¹⁵ is independently -H or an optionally substituted C₁₋₆ alkyl; each D is independently N or CH; ring A is 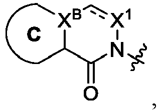 wherein --- is a bond or is absent, X¹ is -N= or -CH= when --- is a bond, X¹ is -NH- or -CH₂- when --- is absent, X^{B} is N or C, and ring C is a 6 membered fully unsaturated carbocycle when X^{B} is C, or a 5-6 membered partially or fully unsaturated heterocycle having 1-2 nitrogen atoms when X^{B} is N, wherein ring C is optionally substituted with R¹ and R² groups, wherein each of R¹ and R² is independently halo, -H, -OH, -CN, -CF₃, -C₁₋₆ alkyl, or a 3-6 membered cycloaliphatic, or R¹ and R² together with the atoms to which they are attached form a 5-6 membered cycloalkyl fused to ring C, wherein the cycloalkyl is optionally substituted with 1-2 of R⁶, wherein each R⁶ is independently -H, halo, or C₁₋₃ alkyl; R³ is C₁₋₆ alkyl; X^{Y} is 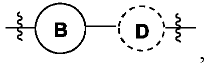 wherein ring B is phenyl, or a 5-6 membered monocyclic heteroaryl having 1-2 nitrogen atoms, or a 7-10 membered fused bicyclic heterocycle having 2-3 nitrogen atoms, wherein at least one of the rings of the bicyclic heterocycle is partially or fully unsaturated, and ring D is absent or an optionally substituted 4-7 membered heterocycle having at least 1 nitrogen atom and optionally 2 additional heteroatoms independently selected from N, O, or S; L is a bivalent C₁₋₂₀ alkyl group, wherein one or more methylene units of the C₁₋₂₀ alkyl group is optionally and independently replaced by -C(O)-, -C(S)-, -(O)CO-, -OC(O)-, -O-, -N(R')-, -S-, -N(R')C(O)-, bivalent mono- or bicyclic heterocycloalkyl, bivalent mono- or bicyclic cycloalkyl, bivalent mono- or bicyclic aryl, or bivalent mono- or bicyclic heteroaryl, wherein each of the bivalent heterocycloalkyl, cycloalkyl, aryl, and heteroaryl are optionally substituted; and Z is 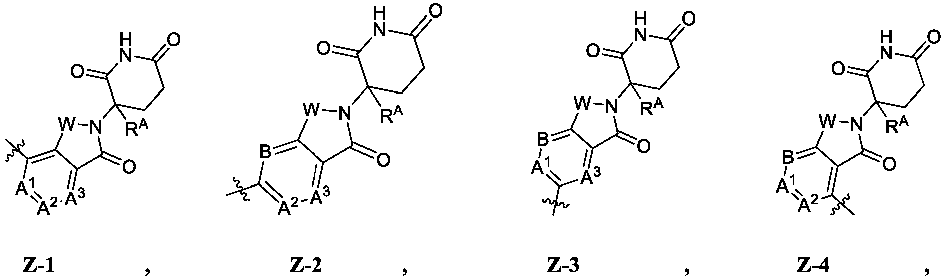 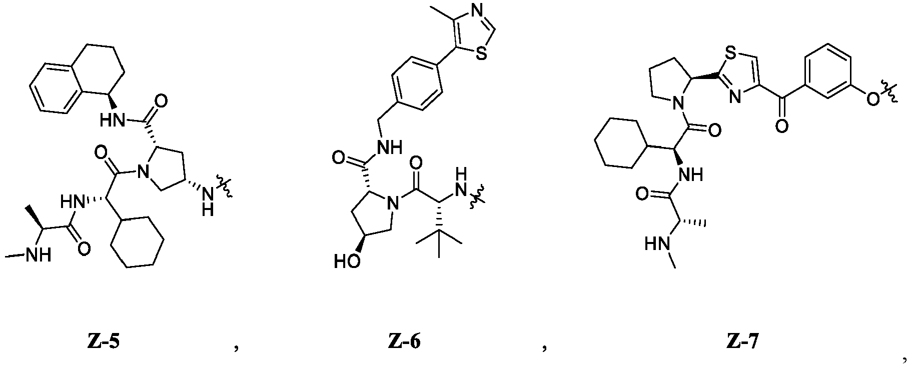 or 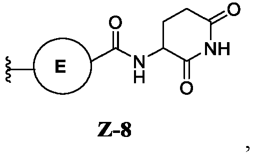 wherein ring E is phenyl or a 5-6 membered heteroaryl having at least 1 heteroatom independently selected from N or S; each of A¹, A², and A³ is independently -C(R^{B})= or -N=; B is -C(R^{C})= or -N=; W is -C(R^{D})₂- or -C(O)-; R^{A} is -H, -CH₃, or -F; each R^{B} is independently-H, halo, -OH, -C₁₋₄ alkyl, or -C₁₋₄ alkoxy; R^{C} is -H, halo, or C₁₋₄ alkyl; and each R^{D} is independently-H or -C₁₋₃ alkyl; and r is 1, 2, or 3.

In some examples, Z is

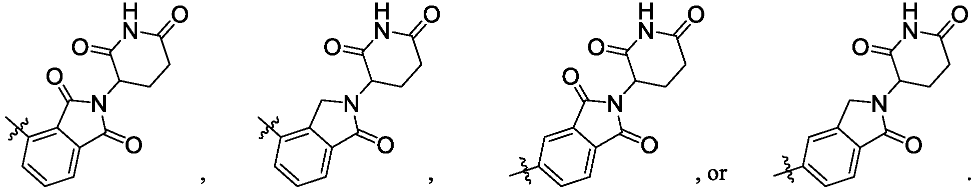

In some examples, Z is 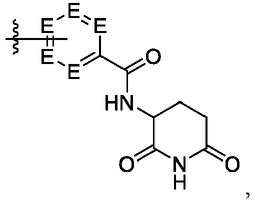 wherein one instance of E is 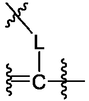 and the remaining instances of E are each independently =CH- or =N-, where L is as defined above for the compound of Formula (I). For example, Z is

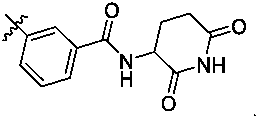

In some examples, one D is CH and the other D is N. In other examples, each D is CH.

In some examples, at least one of A⁴ and A⁵ is N. For example, A⁴ is N, A⁵ is CR¹⁵, and R¹⁵ is -H or C₁₋₃ alkyl.

In some examples, R¹⁵ is -H or methyl.

This disclosure also provides a compound of Formula (**I-A**) 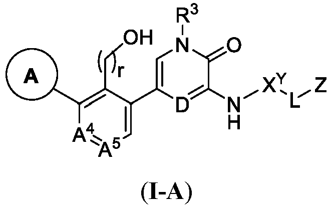 or a pharmaceutically acceptable salt thereof, wherein ring A, A⁴, A⁵, r, R³, X^{Y}, L and Z are as defined in any of the compounds of Formulae (**I**), (**I-A1**), (**I-A2**), (**I-A3**), (**I-B1**), (**I-B2**), (**I-B3**), (**II**), (**II-A**), (**II-B**), (**III**), (**III-A**), (**III-B**), and (**III-C**) (as applicable).

This disclosure also provides a compound of Formula (**I-A1**) 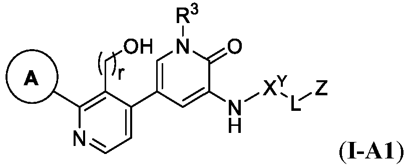 or a pharmaceutically acceptable salt thereof, wherein ring A, r, R³, X^{Y}, L and Z are as defined in any of the compounds of Formulae (**I**), (**I-A**), (**I-A2**), (**I-A3**), (**I-B1**), (**I-B2**), (**I-B3**), (**II**), (**II-A**), (**II-B**), (**III**), (**III-A**), (**III-B**), and (**III-C**) (as applicable).

In some examples, X^{Y} is 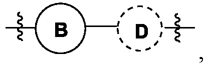 ring B is 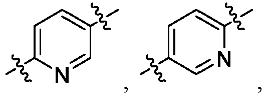 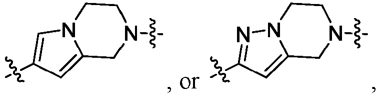 and ring D is absent.

In some examples, X^{Y} is 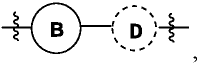 ring B is 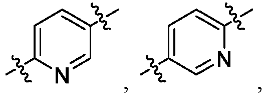 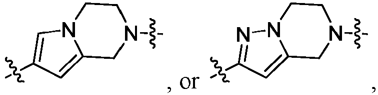 and ring D is 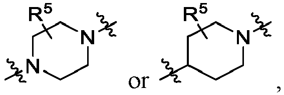 and R⁵ is -H or C₁₋₃ alkyl.

In some examples, X^{Y} is 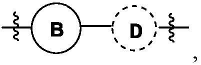 ring B is 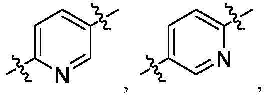 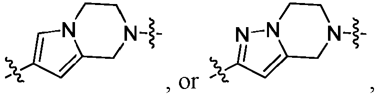 and ring D is

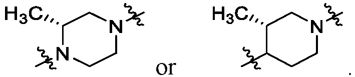

This disclosure also provides a compound of Formula (**I-A2**) 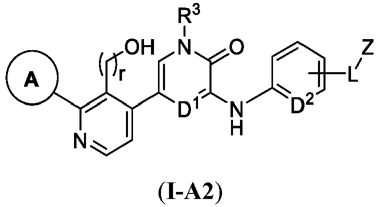
or a pharmaceutically acceptable salt thereof, wherein D¹ is CH or N; D² is CH or N;
at least one of D¹ and D² is N; and each of ring A, r, R³, L, and Z are as defined in any of the compounds of Formulae (**I**), (**I-A**), (**I-A1**), (**I-A3**), (**I-B1**), (**I-B2**), (**I-B3**), (**II**), (**II-A**), (**II-B**), (**III**), (**III-A**), **(III-B),** and (**III-C**) (as applicable).

In some examples, D¹ is =CH- and D² is =N-. In other examples, D¹ is =N- and D² is =CH-.

This disclosure also provides a compound of Formula (I-A3) 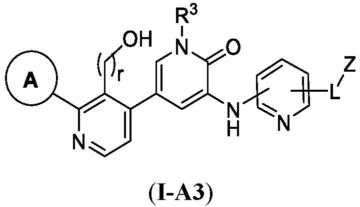 or a pharmaceutically acceptable salt thereof, wherein each of ring A, r, R³, L, and Z are as defined in any of the compounds of Formulae (**I**), (**I-A**), (**I-A1**), (**I-A2**), (**I-B1**), (**I-B2**), (I-**B3**), (**II**), (**II-A**), (**II-B**), (**III**), (**III-A**), (**III-B**), and (**III-C**) (as applicable).

This disclosure also provides a compound of Formula (**I-B1**), a compound of Formula (I-B2), or a compound of Formula (**I-B3**) 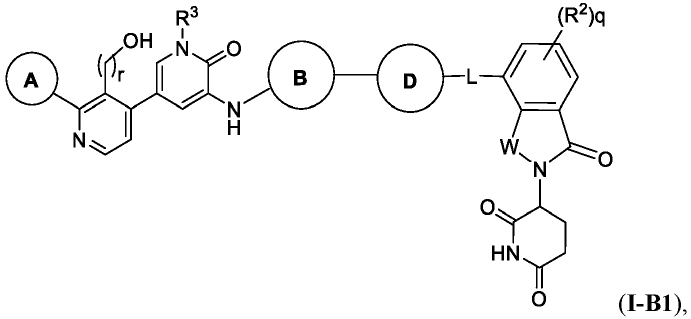 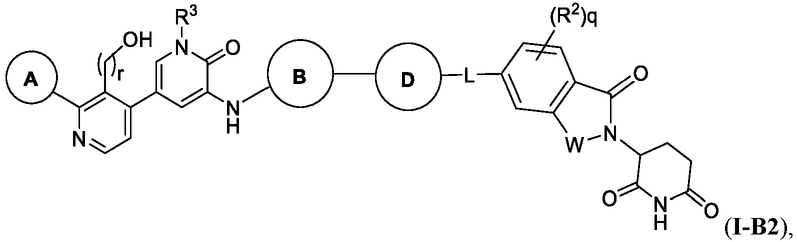 or 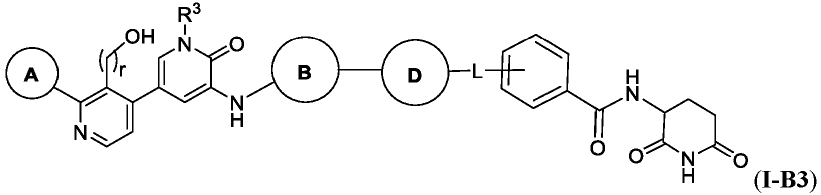 or a pharmaceutically acceptable salt thereof. For each of these compounds, ring A, r, R³, ring B and ring D are as defined in any of the compounds of Formulae (**I**), (**I-A**), (**I-A1**), (**I-A2),** (**I-B1**), **(I-B2), (I-B3), (II), (II-A), (II-B), (III), (III-A), (III-B),** and (**III-C**) (as applicable).

In some examples, ring B is phenyl, pyridine-yl, or pyrimidine-yl.

In some examples, ring D is an optionally substituted 4-7 membered heterocycle having 1-2 nitrogen atoms. For example, ring D is an optionally substituted 5-6 membered heterocycle having 1-2 nitrogen atoms.

In some examples, --- is a bond, X¹ is N, X^{B} is C, and ring C is a 6 membered fully unsaturated carbocycle, optionally substituted with R¹ and R² groups. For example, ring A is 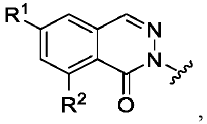 wherein each of R¹ and R² is independently halo, -H, -OH, -CN, -CF₃, -C₁₋₆ alkyl, or a 3-6 membered cycloaliphatic.

In some examples, --- is absent, X¹ is -CH₂- , X^{B} is N, and ring C is a 5-6 membered partially or fully unsaturated heterocycle having 1-2 nitrogen atoms, wherein ring C is substituted with R¹ and R² groups.

In some examples, ring A is 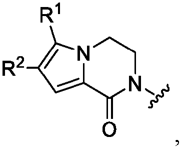 wherein each of R¹ and R² is independently halo, -H, -OH, -CN, -CF₃, -C₁₋₆ alkyl, or a 3-6 membered cycloaliphatic, or R¹ and R² together with the atoms to which they are attached form a 5-6 membered cycloalkyl, wherein the cycloalkyl is optionally substituted with 1-2 of R⁶. For example, ring A is

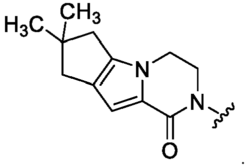

In some examples, R³ is a C₁₋₃ alkyl. For example, R³ is methyl or ethyl.

In some examples, r is 1 or 2. For example, r is 1.

In some examples, L is -Y¹-Y²-Y³-Y⁴-Y⁵-Y⁶-, wherein Y¹ is -C(O)-, -C(O)-N(R)-, -(CH₂-CH₂-O)ₘ-, -(O-CH₂-CH₂)ₘ-, or -C₁₋₆ alkyl-; Y² is a bond, -C(O)-, -O-, -(CH₂-CH₂-O)ₙ-, -(O-CH₂-CH₂)ₙ-, -C₁₋₄ alkyl-, an optionally substituted 3-6 membered monocyclic cycloalkyl, or an optionally substituted 7-11 membered fused or spiro bicycloalkyl; Y³ is a bond, -O-, -N(R)-, -C₁₋₄ alkyl-, -(CH₂-CH₂-O)ₚ-, -(O-CH₂-CH₂)ₚ-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S; Y⁴ is a bond, -C(O)-, -N(R)-, -C(O)N(R)-, -N(R)C(O)-, -C₁₋₄ alkyl-, -(CH₂-CH₂-O)_{q}-, -(O-CH₂-CH₂)_{q}-, -(CH₂-CH₂-N(R))-, an optionally substituted 3-6 membered cycloalkyl, or an optionally substituted 4-6 membered heterocycle having 1-3 heteroatoms independently selected from N, O, or S; Y⁵ is a bond, -N(R)-, -C(O)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S; Y⁶ is a bond, or -(CH₂-CH₂-N(R))-; each of m and n is independently 1, 2, 3, 4, or 5; each of p, q, and s is independently 1, 2, or 3; and R is -H or -C₁₋₃ alkyl.

In some examples, Y¹ is -C(O)- or -C(O)-N(R)-.

In some examples,Y¹ is -C(O)-, Y² is -(CH₂-CH₂-O)ₙ-, -(O-CH₂-CH₂)ₙ-, -C₁₋₄ alkyl-, a 3-6 membered monocyclic cycloalkyl, or a 7-11 membered fused or spiro bicycloalkyl, and n is 1 or 2. For example, Y² is -(CH₂-CH₂-O)ₙ-, -(O-CH₂-CH₂)ₙ-, -CH₂-, -CH₂-CH₂-, -CH₂-CH₂-CH₂-, cyclopentyl, cyclohexyl, or a 7-11 membered spiro bicycloalkyl, and n is 1 or 2.

In some examples, Y³ is a bond, -O-, -N(R)-, -C₁₋₄ alkyl-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S. For example, Y³ is a bond, -N(R)-, -O-, -CH₂-, -CH₂-CH₂-, -CH₂-CH₂-CH₂-, -(CH₂-CH₂-N(R))-, phenyl,

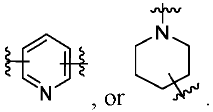

In some examples, Y⁴ is a bond, -C(O)-, -N(R)-, -C(O)N(R)-, -N(R)C(O)-, -C₁₋₄ alkyl-, -(CH₂-CH₂-N(R))-, an optionally substituted 3-6 membered cycloalkyl, or an optionally substituted 4-6 membered heterocycle having 1-3 heteroatoms independently selected from N, O, or S. For example, Y⁴ is a bond, -C(O)-, -N(R)-, -C(O)N(R)-, -N(R)C(O)-, -CH₂-, -CH₂-CH₂-,

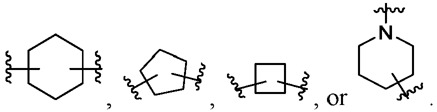

In some examples, Y⁵ is a bond, -N(R)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S. For example, Y⁵ is a bond, -N(R)-, -CH₂-, -CH₂-CH₂-, -CH₂-CH₂-CH₂-, -(CH₂-CH₂-O)ₛ-, -(O-CH₂-CH₂)ₛ-, or 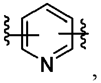 and s is 1 or 2.

In some examples, Y⁶ is a bond.

In some examples, Y¹ is -(CH₂-CH₂-O)ₘ-, -(O-CH₂-CH₂)ₘ-, or -C₁₋₆ alkyl-.

In some examples,Y² is -C(O)-, -O-, -(CH₂-CH₂-O)ₙ-, -(O-CH₂-CH₂)ₙ-, 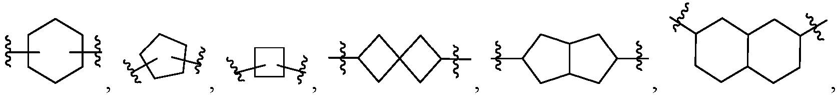 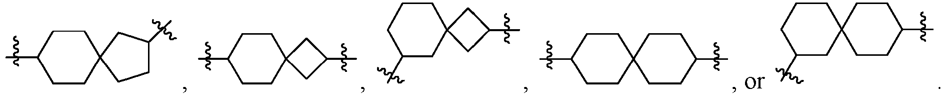 For example, Y² is -C(O)-, -O-, -(CH₂-CH₂-O)ₙ-, or -(O-CH₂-CH₂)ₙ-, and n is 2 or 3.

In some examples, Y³ is a bond, -O-, -N(R)-, -(CH₂-CH₂-O)ₚ-, -(O-CH₂-CH₂)ₚ-, -(CH₂-CH₂-N(R))-, phenyl, 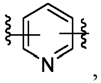 an optionally substituted 3-6 membered monocyclic cycloalkyl, or an optionally substituted 4-6 membered monocyclic heterocycle having 1-3 heteroatoms independently selected from N, O, or S. For example, Y³ is a bond, -O-, -N(R)-, -(CH₂-CH₂-O)ₚ-, or -(O-CH₂-CH₂)ₚ-, and p is 1 or 2. In other examples, Y³ is

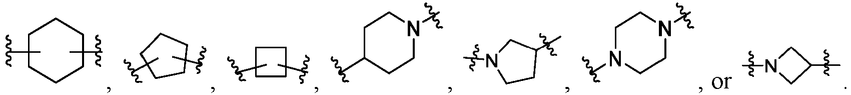

In some examples, Y⁴ is a bond, -N(R)-, -C(O)N(R)-, -N(R)C(O)-, -C₁₋₄ alkyl-, - (CH₂-CH₂-N(R))-, an optionally substituted 3-6 membered cycloalkyl, or an optionally substituted 4-6 membered heterocycle having 1-3 heteroatoms independently selected from N, O, or S. For example, Y⁴ is a bond, -N(H)-, -N(CH₃)-, -N(H)C(O)-, -CH₂-, -CH₂-CH₂-, -CH₂-CH₂-CH₂-, -(CH₂-CH₂-N(H))-,

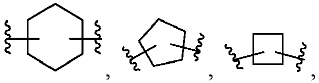

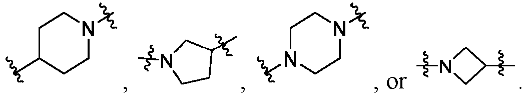

In some examples, Y⁵ is a bond, -C(O)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S. For example, Y⁵ is a bond, -C(O)-, -CH₂-, -CH₂-CH₂-, -CH₂-CH₂-CH₂-, -(CH₂-CH₂-O)ₛ-, -(O-CH₂-CH₂)ₛ-, or 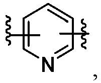 and s is 1 or 2.

In some examples, Y⁶ is a bond or -CH₂-CH₂-N(H)-.

This disclosure also provides a compound of Formula (II) 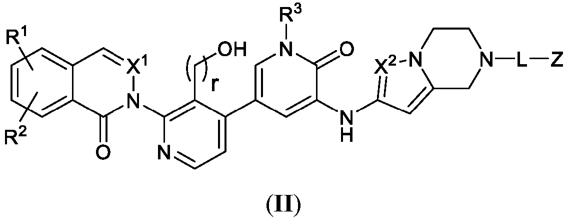 or a pharmaceutically acceptable salt thereof, wherein R¹ is C₁₋₆ alkyl, hydroxyl, halo, cyano, or a 3-6 membered cycloaliphatic; R² is C₁₋₆ alkyl, hydroxyl, halo, cyano, or a 3-6 membered cycloaliphatic; R³ is C₁₋₆ alkyl; X¹ and X² are each independently =CH- or =N-; r is 1, 2, or 3; L is a bivalent C₁₋₂₀ alkyl group, optionally substituted with C₁₋₆ alkyl, acyl, oxo, halo, or alkoxy, wherein one or more methylene units of said C₁₋₂₀ alkyl group is optionally and independently replaced by -CO-, -CS-, -CONH-, -CONHNH-, -CO₂-, -OCO-, -NHCO₂-, -O-, -NHCONH-, -OCONH-, -NHNH-, -NHCO-, -S-, -S(O)-, -S(O)₂-, -NH-, -S(O)₂NH-, -NHS(O)₂-, -NHS(O)₂NH-, bivalent mono- or bicyclic heterocycle, bivalent mono- or bicyclic cycloalkyl, bivalent mono- or bicyclic aryl, or bivalent mono- or bicyclic heteroaryl, wherein the heterocycle and heteroaryl of L has 1-3 heteroatoms independently selected from N, O, or S; and Z is 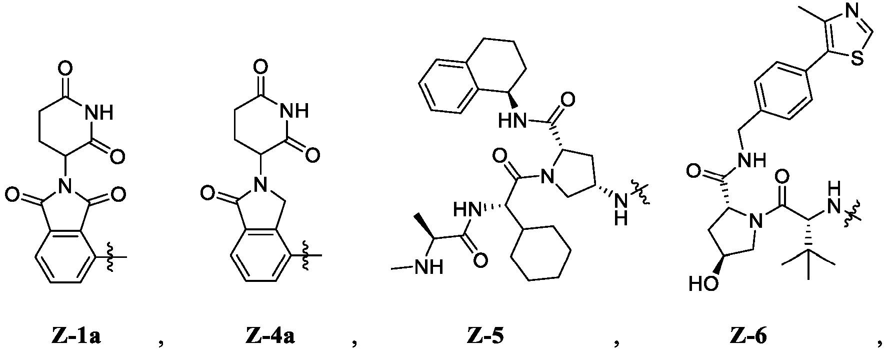 or

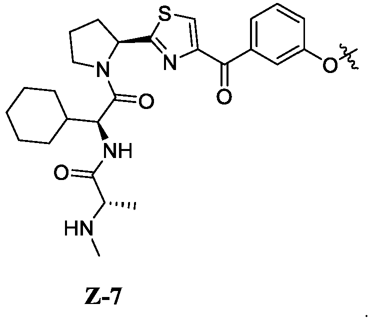

In some examples, R² is C₁₋₆ alkyl, hydroxyl, halo, or cyano.

This disclosure also provides a compound of Formula (**II-A**) 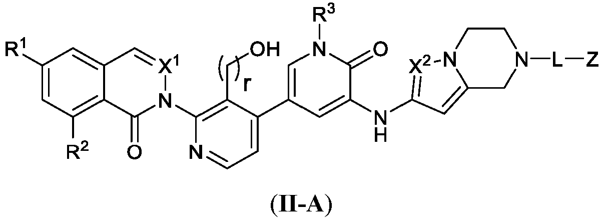 or a pharmaceutically acceptable salt thereof, wherein R¹, R², X¹, r, R³, X², L and Z are as defined in any of the compounds of Formulae (**I**), (**I-A**), (**I-A1**), (**I-A2**), (**I-B1**), (**I-B2**), (**I-B3**), (**II**), (**II-B**), (**III**), (**III-A**), (**III-B**), and (**III-C**) (as applicable).

In some examples, R¹ is C₁₋₆ alkyl. For example, R¹ is methyl, ethyl, propyl, *iso-*propyl, sec-butyl, or *tert-butyl.* In other examples, R¹ is *tert*-butyl.

In some examples, R¹ is 3-6 membered cycloaliphatic. For example, R¹ is cyclopropyl, cyclopentyl, or cyclohexyl.

In some examples, R² is halo. For example, R² is -F.

In some examples, R³ is methyl, ethyl, propyl, *iso*-propyl, *sec*-butyl, or *tert-butyl.* For example, R³ is methyl.

In some examples, n is 1.

In some examples, at least one of X¹ and X² is =N-. For example, both of X¹ and X² are =N-.

In some examples, L is a bivalent C₁₋₂₀ alkyl group, wherein one or more methylene units of the C₁₋₂₀ alkyl group is optionally and independently replaced by -CO-, -CS-, -OCO-, -O-, -NHCO-, -S-, -NH-, bivalent mono- or bicyclic heterocycle, bivalent mono- or bicyclic cycloalkyl, bivalent mono- or bicyclic aryl, or bivalent mono- or bicyclic heteroaryl, wherein the heterocycle and heteroaryl have 1-3 heteroatoms independently selected from N, O, or S. For example, L is a bivalent C₁₋₂₀ alkyl group, wherein one or more methylene units of the C₁₋₂₀ alkyl group is optionally and independently replaced by -CO-, -O-, -NH-, bivalent mono- or bicyclic heterocycloalkyl having 1-3 heteroatoms independently selected from N, O, or S, or bivalent mono- or bicyclic cycloalkyl. In other examples, L is a bivalent C₁₋₁₀ alkyl group, wherein one or more methylene units of the C₁₋₁₀ alkyl group is optionally and independently replaced by -CO-, -OCO-, -O-, -NHCO-, -NH-, bivalent mono- or bicyclic heterocycle, bivalent mono- or bicyclic cycloalkyl, bivalent mono- or bicyclic aryl, or bivalent mono- or bicyclic heteroaryl, wherein the heterocycle and heteroaryl have 1-3 heteroatoms independently selected from N, O, or S.

In some examples, the bivalent heterocycle of L is selected from piperazine, piperidine, dioxane, pyran, morpholine, pyrrolidine, and tetrahydrofuran. For example, the bivalent heterocycloalkyl is piperazine.

In some examples, the bivalent cycloalkyl is selected from cyclopropyl, cyclobutyl, cyclopentyl, cyclohexyl, and cycloheptyl. For example, the bivalent cycloalkyl is cyclohexyl.

In some examples, L is -Y¹-Y²-Y³-Y⁴-Y⁵-, wherein Y¹ is -C(O)- or -C₁₋₆ alkyl-; Y² is a bond, -O-, -(CH₂-CH₂-O)ₙ-, -(O-CH₂-CH₂)ₙ-, -C₁₋₄ alkyl-, 3-6 membered cycloalkyl; Y³ is a bond, -O-, -N(R)-, -C₁₋₄ alkyl-, -(CH₂-CH₂-O)ₚ-, -(O-CH₂-CH₂)ₚ-, -(CH₂-CH₂-N(R))-, phenyl, 3-6 membered cycloalkyl, or 4-6 membered heterocycle having 1-3 heteroatoms independently selected from N, O, or S; Y⁴ is a bond, -C(O)-, -C(O)N(R)-, -C(O)N(R)-, -C₁₋₄ alkyl-, -(CH₂-CH₂-O)_{q}-, -(O-CH₂-CH₂)_{q}-, -(CH₂-CH₂-N(R))-, 3-6 membered cycloalkyl, or 4-6 membered heterocycle having 1-3 heteroatoms independently selected from N, O, or S; Y⁵ is a bond, -N(R)-, -C(O)-, -C₁₋₄ alkyl-, or a 5-6 membered heteroaryl having 1-3 heteroatoms independently selected from N, O or S; n is 1, 2, 3, 4, or 5; p is 1, 2, or 3; q is 1, 2, or 3; and R is -H or -C₁₋₃ alkyl.

In some examples, Y¹ is -C(O)- or -C₄₋₆ alkyl-.

In some examples, Y² is -(CH₂-CH₂-O)ₙ-, -(O-CH₂-CH₂)ₙ-, or -C₁₋₄ alkyl-.

In some examples, Y³ is a bond, -O-, -C₁₋₄ alkyl-, -(CH₂-CH₂-N(R))-,

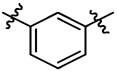

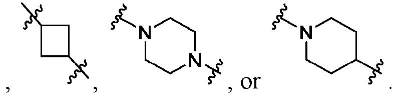

In some examples, Y⁴ is a bond, -C(O)-, -C(O)N(R)-, -C(O)N(R)-, or -C₁₋₄ alkyl-

In some examples, Y⁵ is a bond, -N(R)-, -C(O)-, -C₁₋₄ alkyl-, or

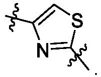

This disclosure also provides a compound of Formula (II-B) 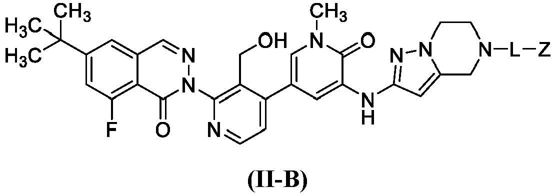 or a pharmaceutically acceptable salt thereof, wherein L and Z are as defined in any of the compounds of Formulae (**I**), (I-A), (**I-A1**), (**I-A2**), (**I-B1**), (**I-B2**), (**I-B3**), (**II**), (**II-A**), (**III**), (**III-A**), (**III-B**), and **(III-C**) (as applicable).

In some examples, L is selected from methylene, ethylene, n-propylene, n-butylene, n-pentylene, n-hexylene, 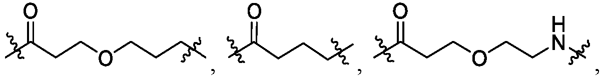 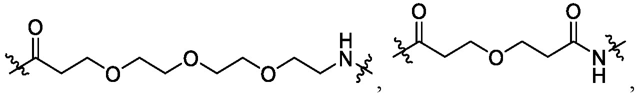 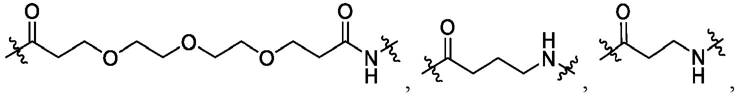 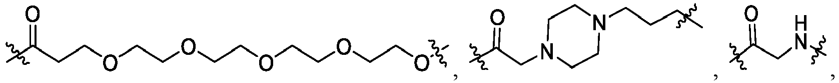 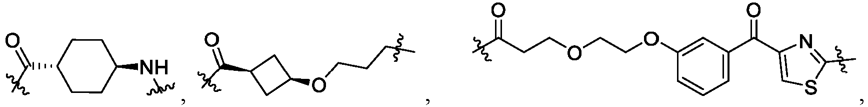 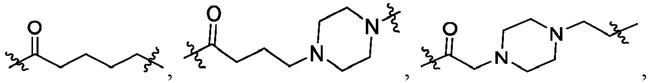 or

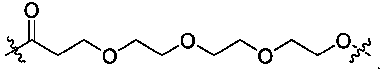

This disclosure also provides a compound of Formula (**III**) 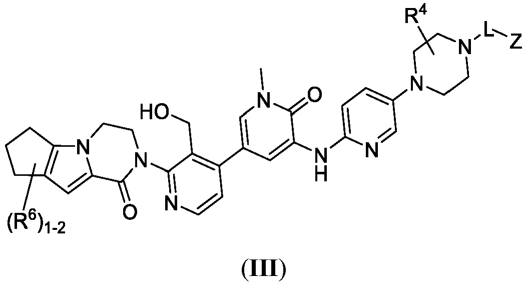 or pharmaceutically acceptable salt thereof, wherein each of R⁶, R⁴, L and Z are as defined in any of the compounds of Formulae (**I**), (**I-A**), (**I-A1**), (**I-A2**), (**I-B1**), (**I-B2**), (**I-B3**), (**II**), (**II-**A), (**II-B**), (**III-A**), (**III-B**), and (**III-C**) (as applicable).

In some examples, each R⁶ is independently -H or C₁₋₃ alkyl.

In some examples, R⁴ is independently -H or C₁₋₃ alkyl.

This disclosure also provides a compound of Formula (**III-A**) 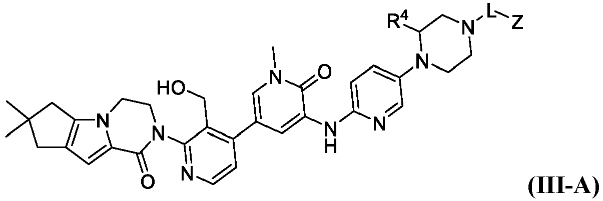 or a pharmaceutically acceptable salt thereof, wherein R⁴ is -H or -C₁₋₃ alkyl; L is -Y¹-Y²-Y³-Y⁴-Y⁵-Y⁶-; Y¹ is -C(O)-, -C(O)-N(R)-, -(CH₂-CH₂-O)ₘ-, -(O-CH₂-CH₂)ₘ-, or -C₁₋₄ alkyl-; Y² is a bond, -O-, -C₁₋₄ alkyl-, -(O-CH₂-CH₂)ₙ-, -(CH₂-CH₂-O)ₙ-, -(CH₂-CH₂-N(R))-, or 3-6 membered cycloalkyl; Y³ is a bond, -O-, -N(R)-, -C₁₋₄ alkyl-, -(CH₂-CH₂-O)ₚ-, -(O-CH₂-CH₂)ₚ-, -(CH₂-CH₂-N(R))-, 3-6 membered cycloalkyl, or 4-6 membered heterocycloalkyl having 1-3 heteroatoms independently selected from N, O, or S; Y⁴ is a bond, -N(R)-, -C₁₋₄ alkyl-, -(CH₂-CH₂-O)_{q}-, -(O-CH₂-CH₂)_{q}-, -(CH₂-CH₂-N(R))-, 3-6 membered cycloalkyl, or 4-6 membered heterocycloalkyl having 1-3 heteroatoms independently selected from N, O, or S; Y⁵ is a bond, -N(R)-, -(CH₂-CH₂-O)ₛ-, or -(O-CH₂-CH₂)ₛ-; Y⁶ is a bond or -(CH₂-CH₂-N(R))-; each R is independently-H or -C₁₋₃ alkyl; and each of m, p, q, and s is independently 1, 2, or 3; Z is 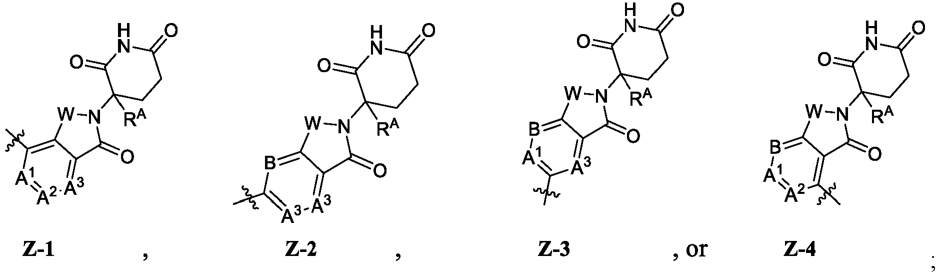 each of A¹, A², and A³ is independently -C(R^{B})= or -N=; B is -C(R^{C})= or -N=; W is -C(R^{D})₂- or -C(O)-; R^{A} is -H, -CH₃, or -F; each R^{B} is independently-H, halo, -OH, -C₁₋₄ alkyl, or -C₁₋₄ alkoxy; R^{C} is -H, halo, or C₁₋₄ alkyl; and each R^{D} is independently-H or -C₁₋₃ alkyl; provided that when each of Y³, Y⁴, Y⁵, and Y⁶ are a bond, then Y² is not a bond.

The compound or pharmaceutically acceptable salt of any one of claims 82-85, wherein Z is

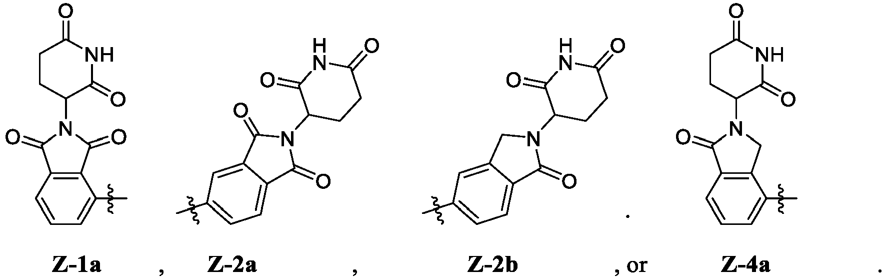

This disclosure also provides a compound of Formula (III-B) 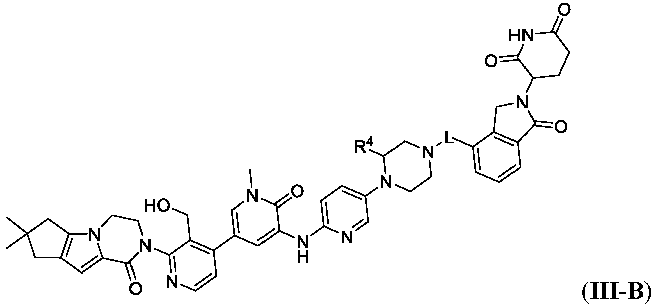 or a pharmaceutically acceptable salt thereof, wherein R⁴ and L are as defined in any of the compounds of Formulae **(I), (I-A), (I-A1), (I-A2), (I-B1), (I-B2), (I-B3), (II), (II-A), (II-B), (III), (III-A),** and **(III-C)** (as applicable).

In some examples, Y¹ is -C(O)- or -C(O)-N(R)-.

In some examples, Y¹ is -C(O)-, and Y² is -(O-CH₂-CH₂)ₙ-, -(CH₂-CH₂-O)ₙ-, or - C₁₋₄ alkyl-.

In some examples, Y² is -(O-CH₂-CH₂)ₙ- or -(CH₂-CH₂-O)ₙ- and n is 1.

In some examples, Y³ is -C₁₋₄ alkyl- or -(CH₂-CH₂-N(R))-, and at least one of Y⁴, Y⁵, and Y⁶ is a bond.

In some examples, Y³ is methylene, ethylene, or propylene, and each of Y⁴, Y⁵, and Y⁶ is a bond.

In some examples, Y³ is -(CH₂-CH₂-N(R))-, and each of Y⁴, Y⁵, and Y⁶ is a bond.

In some examples, L is -C(O)-(CH₂-CH₂-O)-(CH₂)₃- or -C(O)-(CH₂-CH₂-O)-(CH₂-CH₂-N(R))-.

In some examples, R⁴ is methyl.

In some examples, Y¹ is -C(O)-N(R)- and Y² is -(O-CH₂-CH₂)ₙ-, -(CH₂-CH₂-O)ₙ-, or -C₁₋₄ alkyl-.

In some examples, Y² is -(O-CH₂-CH₂)ₙ- or -(CH₂-CH₂-O)ₙ-, wherein n is 3.

In some examples, Y³ is -(O-CH₂-CH₂)ₚ-, -(CH₂-CH₂-O)ₚ-, or -C₁₋₄ alkyl-, wherein p is 1 or 2.

In some examples, Y³ is methylene, ethylene, or propylene, and Y⁴ is a bond.

In some examples, Y³ is -(O-CH₂-CH₂)ₚ- or -(CH₂-CH₂-O)ₚ-, and Y⁴ is a bond or - C₁₋₄ alkyl-.

In some examples, Y⁴ is methylene, ethylene, or propylene, and each of Y⁵ and Y⁶ is a bond.

In some examples, Y¹ is -C(O)-, Y² is 3-6 membered cycloalkyl or -C₁₋₄ alkyl-, and Y³ is -(O-CH₂-CH₂)ₚ-, -(CH₂-CH₂-O)ₚ-, 3-6 membered cycloalkyl, or 4-6 membered heterocycloalkyl having 1-3 heteroatoms independently selected from N, O, or S.

In some examples, Y² is methylene, 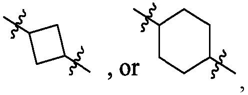 and Y³ is -(O-CH₂-CH₂)ₚ-, -(CH₂-CH₂-O)ₚ-, -N(R)-, 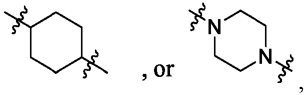 wherein p is 1.

In some examples, Y² is methylene and Y³ is

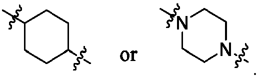

In some examples, L is selected from 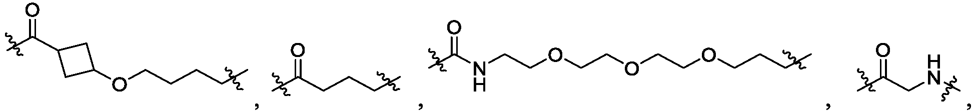 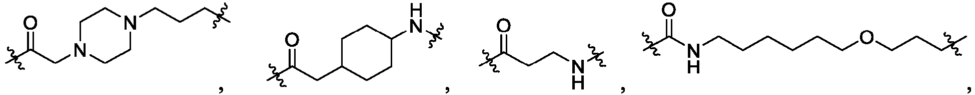 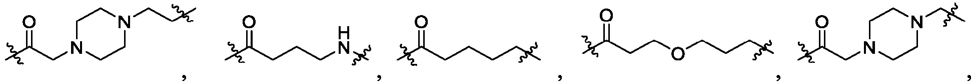 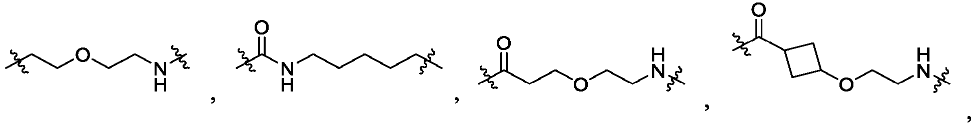 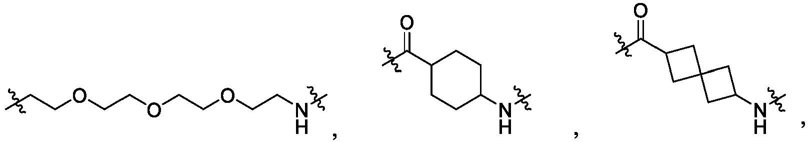 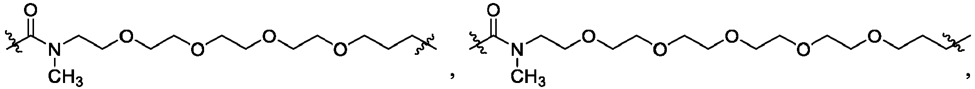 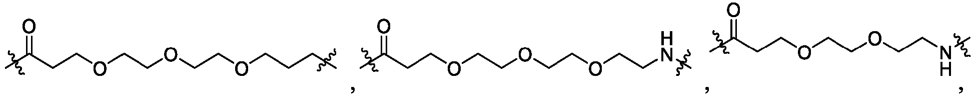 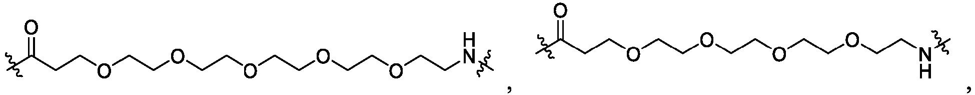 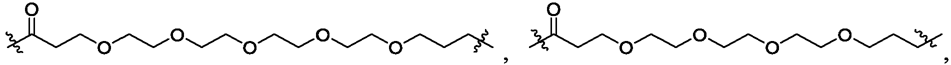 or

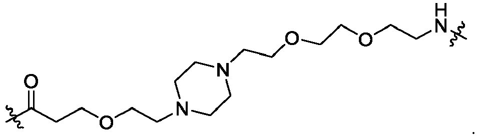

This disclosure also provides a compound of Formula (**III-C**) 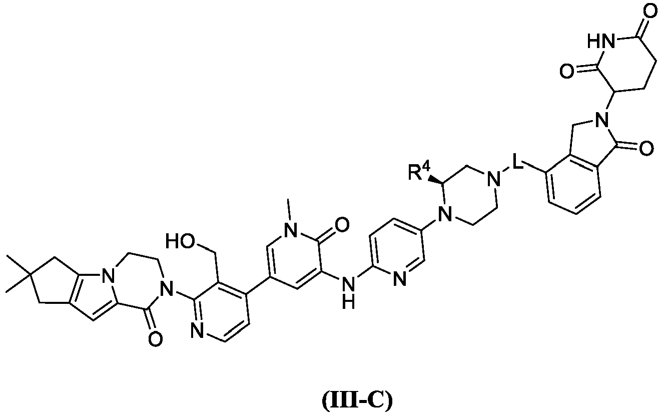 or a pharmaceutically acceptable salt thereof, wherein L and R4 are as defined in any of the compounds of Formulae (**I**), (**I-A**), (**I-A1**), (**I-A2**), (**I-B1**), (**I-B2**), (**I-B3**), (**II**), (**II-A**), (**II-B**), (**III**), (**III-A**), and (**III-B**) (as applicable).

In some examples, L is -Y¹-Y²-Y³-Y⁴-Y⁵-Y⁶-, wherein Y¹ is -C(O)-, -C(O)-N(R)-, -(CH₂-CH₂-O)ₘ-, -(O-CH₂-CH₂)ₘ-, or -C₁₋₆ alkyl-; Y² is a bond, -C(O)-, -O-, -(CH₂-CH₂-O)ₙ-, -(O-CH₂-CH₂)ₙ-, -C₁₋₄ alkyl-, an optionally substituted 3-6 membered monocyclic cycloalkyl, or an optionally substituted 7-11 membered fused or spiro bicycloalkyl; Y³ is a bond, -O-, -N(R)-, -C₁₋₄ alkyl-, -(CH₂-CH₂-O)ₚ-, -(O-CH₂-CH₂)ₚ-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S; Y⁴ is a bond, -C(O)-, -N(R)-, -C(O)N(R)-, -N(R)C(O)-, -C₁₋₄ alkyl-, -(CH₂-CH₂-O)_{q}-, -(O-CH₂-CH₂)_{q}-, -(CH₂-CH₂-N(R))-, an optionally substituted 3-6 membered cycloalkyl, or an optionally substituted 4-6 membered heterocycle having 1-3 heteroatoms independently selected from N, O, or S; Y⁵ is a bond, -N(R)-, -C(O)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S; Y⁶ is a bond, or -(CH₂-CH₂-N(R))-; each of m and n is independently 1, 2, 3, 4, or 5; each of p, q, and s is independently 1, 2, or 3; and R is -H or -C₁₋₃ alkyl.

In some examples, Y¹ is -C(O)- or -C(O)-N(R)-.

In some examples, Y¹ is -C(O)-, Y² is -(CH₂-CH₂-O)ₙ-, -(O-CH₂-CH₂)ₙ-, -C₁₋₄ alkyl-, a 3-6 membered monocyclic cycloalkyl, or a 7-11 membered fused or spiro bicycloalkyl, and n is 1 or 2. For example, Y² is -(CH₂-CH₂-O)ₙ-, -(O-CH₂-CH₂)ₙ-, -CH₂-, -CH₂-CH₂-, -CH₂-CH₂-CH₂-, cyclopentyl, cyclohexyl, or a 7-11 membered spiro bicycloalkyl, and n is 1 or 2.

In some examples, Y³ is a bond, -O-, -N(R)-, -C₁₋₄ alkyl-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S. For example, Y³ is a bond, -N(R)-, -O-, -CH₂-, -CH₂-CH₂-, -CH₂-CH₂-CH₂-, -(CH₂-CH₂-N(R))-, phenyl,

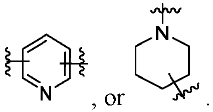

In some examples, Y⁴ is a bond, -C(O)-, -N(R)-, -C(O)N(R)-, -N(R)C(O)-, -C₁₋₄ alkyl-, -(CH₂-CH₂-N(R))-, an optionally substituted 3-6 membered cycloalkyl, or an optionally substituted 4-6 membered heterocycle having 1-3 heteroatoms independently selected from N, O, or S. For example, Y⁴ is a bond, -C(O)-, -N(R)-, -C(O)N(R)-, -N(R)C(O)-, -CH₂-, -CH₂-CH₂-,

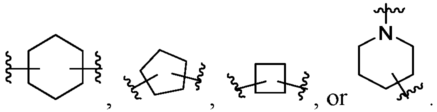

In some examples, Y⁵ is a bond, -N(R)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S. For example, Y⁵ is a bond, -N(R)-, -CH₂-, -CH₂-CH₂-, -CH₂-CH₂-CH₂-, -(CH₂-CH₂-O)ₛ-, -(O-CH₂-CH₂)ₛ-, or 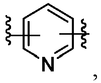 and s is 1 or 2. In some instances, s is 1.

In some examples, Y⁶ is a bond.

In some examples, Y¹ is -(CH₂-CH₂-O)ₘ-, -(O-CH₂-CH₂)ₘ-, or -C₁₋₆ alkyl-.

In some examples, Y² is -C(O)-, -O-, -(CH₂-CH₂-O)ₙ-, -(O-CH₂-CH₂)ₙ-, 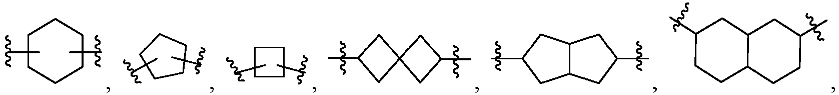 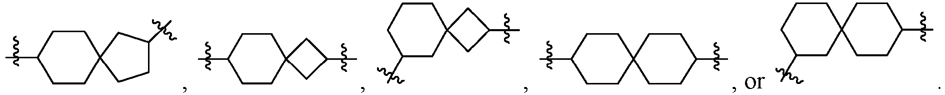 For example, Y² is -C(O)-, -O-, -(CH₂-CH₂-O)ₙ-, or -(O-CH₂-CH₂)ₙ-, and n is 2 or 3.

In some examples, Y³ is a bond, -O-, -N(R)-, -(CH₂-CH₂-O)ₚ-, -(O-CH₂-CH₂)ₚ-, -(CH₂-CH₂-N(R))-, phenyl, 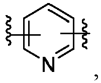 an optionally substituted 3-6 membered monocyclic cycloalkyl, or an optionally substituted 4-6 membered monocyclic heterocycle having 1-3 heteroatoms independently selected from N, O, or S. For example, Y³ is a bond, -O-, -N(R)-, -(CH₂-CH₂-O)ₚ-, or -(O-CH₂-CH₂)ₚ-, and p is 1 or 2. In other examples, Y³ is

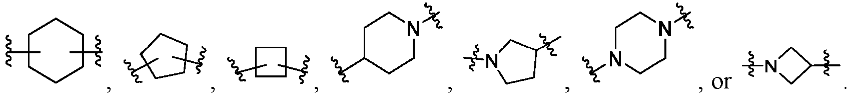

In some examples, Y⁴ is a bond, -N(R)-, -C(O)N(R)-, -N(R)C(O)-, -C₁₋₄ alkyl-, - (CH₂-CH₂-N(R))-, an optionally substituted 3-6 membered cycloalkyl, or an optionally substituted 4-6 membered heterocycle having 1-3 heteroatoms independently selected from N, O, or S. For example, Y⁴ is a bond, -N(H)-, -N(CH₃)-, -N(H)C(O)-, -CH₂-, -CH₂-CH₂-, -CH₂-CH₂-CH₂-, -(CH₂-CH₂-N(H))-,

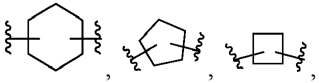

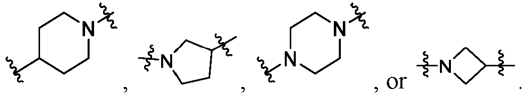

In some examples, Y⁵ is a bond, -C(O)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S. For example, Y⁵ is a bond, -C(O)-, -CH₂-, -CH₂-CH₂-, -CH₂-CH₂-CH₂-, -(CH₂-CH₂-O)ₛ-, -(O-CH₂-CH₂)ₛ-, or 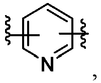 and s is 1 or 2.

In some examples, Y⁶ is a bond or -CH₂-CH₂-N(H)- or -N(H)-CH₂-CH₂-.

### DETAILED DESCRIPTION

The present disclosure provides bifunctional compounds that induce the proteolytic degradation of BTK via a ubiquitin proteolysis pathway. The present disclosure also provides a compound of Formula (**I**) or a pharmaceutically acceptable salt thereof.

As used herein, the following definitions shall apply unless otherwise indicated.

### I. DEFINITIONS

For purposes of this disclosure, the chemical elements are identified in accordance with the Periodic Table of the Elements, CAS version, Handbook of Chemistry and Physics, 75th Ed. Additionally, general principles of organic chemistry are described in "Organic Chemistry," Thomas Sorrell, University Science Books, Sausalito: 1999, and "March's Advanced Organic Chemistry," 5th Ed., Ed.: Smith, M.B. and March, J., John Wiley & Sons, New York: 2001.

As described herein, "protecting group" refers to a moiety or functionality that is introduced into a molecule by chemical modification of a functional group in order to obtain chemoselectivity in a subsequent chemical reaction. Standard protecting groups are provided in Wuts and Greene: "Greene's Protective Groups in Organic Synthesis," 4th Ed, Wuts, P.G.M. and Greene, T.W., Wiley-Interscience, New York:2006.

As described herein, compounds of this disclosure optionally may be substituted with one or more substituents, such as are illustrated generally above, or as exemplified by particular classes, subclasses, and species of the disclosure.

As used herein, the term "hydroxyl" or "hydroxy" refers to an -OH moiety.

As used herein the term "aliphatic" encompasses the terms alkyl, alkenyl, and alkynyl, each of which being optionally substituted as set forth below.

As used herein, an "alkyl" group refers to a saturated aliphatic hydrocarbon group containing 1-12 (e.g., 1-8, 1-6, or 1-4) carbon atoms. An alkyl group can be straight or branched. Examples of alkyl groups include, but are not limited to, methyl, ethyl, propyl, isopropyl, butyl, isobutyl, sec-butyl, tert-butyl, n-pentyl, n-heptyl, or 2-ethylhexyl. An alkyl group can be substituted (i.e., optionally substituted) with one or more substituents such as halo, phospho, cycloaliphatic [e.g., cycloalkyl or cycloalkenyl], heterocycloaliphatic [e.g., heterocycloalkyl or heterocycloalkenyl], aryl, heteroaryl, alkoxy, aroyl, heteroaroyl, acyl [e.g., (aliphatic)carbonyl, (cycloaliphatic)carbonyl, or (heterocycloaliphatic)carbonyl], nitro, cyano, amido [e.g., (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino alkylaminocarbonyl, cycloalkylaminocarbonyl, heterocycloalkylaminocarbonyl, arylaminocarbonyl, or heteroarylaminocarbonyl], amino [e.g., aliphaticamino, cycloaliphaticamino, or heterocycloaliphaticamino], sulfonyl [e.g., aliphatic-SO₂-], sulfinyl, sulfanyl, sulfoxy, urea, thiourea, sulfamoyl, sulfamide, oxo, carboxy, carbamoyl, cycloaliphaticoxy, heterocycloaliphaticoxy, aryloxy, heteroaryloxy, aralkyloxy, heteroarylalkoxy, alkoxycarbonyl, alkylcarbonyloxy, or hydroxy. Without limitation, some examples of substituted alkyls include carboxyalkyl (such as HOOC-alkyl, alkoxycarbonylalkyl, and alkylcarbonyloxyalkyl), cyanoalkyl, hydroxyalkyl, alkoxyalkyl, acylalkyl, aralkyl, (alkoxyaryl)alkyl, (sulfonylamino)alkyl (such as (alkyl-SO₂-amino)alkyl), aminoalkyl, amidoalkyl, (cycloaliphatic)alkyl, or haloalkyl.

As used herein, an "alkenyl" group refers to an aliphatic carbon group that contains 2-8 (e.g., 2-12, 2-6, or 2-4) carbon atoms and at least one double bond. Like an alkyl group, an alkenyl group can be straight or branched. Examples of an alkenyl group include, but are not limited to allyl, 1- or 2-isopropenyl, 2-butenyl, and 2-hexenyl. An alkenyl group can be optionally substituted with one or more substituents such as halo, phospho, cycloaliphatic [e.g., cycloalkyl or cycloalkenyl], heterocycloaliphatic [e.g., heterocycloalkyl or heterocycloalkenyl], aryl, heteroaryl, alkoxy, aroyl, heteroaroyl, acyl [e.g., (aliphatic)carbonyl, (cycloaliphatic)carbonyl, or (heterocycloaliphatic)carbonyl], nitro, cyano, amido [e.g., (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino alkylaminocarbonyl, cycloalkylaminocarbonyl, heterocycloalkylaminocarbonyl, arylaminocarbonyl, or heteroarylaminocarbonyl], amino [e.g., aliphaticamino, cycloaliphaticamino, heterocycloaliphaticamino, or aliphaticsulfonylamino], sulfonyl [e.g., alkyl-SO₂-, cycloaliphatic-SO₂-, or aryl-SO₂-], sulfinyl, sulfanyl, sulfoxy, urea, thiourea, sulfamoyl, sulfamide, oxo, carboxy, carbamoyl, cycloaliphaticoxy, heterocycloaliphaticoxy, aryloxy, heteroaryloxy, aralkyloxy, heteroaralkoxy, alkoxycarbonyl, alkylcarbonyloxy, or hydroxy. Without limitation, some examples of substituted alkenyls include cyanoalkenyl, alkoxyalkenyl, acylalkenyl, hydroxyalkenyl, aralkenyl, (alkoxyaryl)alkenyl, (sulfonylamino)alkenyl (such as (alkyl-SO₂-amino)alkenyl), aminoalkenyl, amidoalkenyl, (cycloaliphatic)alkenyl, or haloalkenyl.

As used herein, an "alkynyl" group refers to an aliphatic carbon group that contains 2-8 (e.g., 2-12, 2-6, or 2-4) carbon atoms and has at least one triple bond. An alkynyl group can be straight or branched. Examples of an alkynyl group include, but are not limited to, propargyl and butynyl. An alkynyl group can be optionally substituted with one or more substituents such as aroyl, heteroaroyl, alkoxy, cycloalkyloxy, heterocycloalkyloxy, aryloxy, heteroaryloxy, aralkyloxy, nitro, carboxy, cyano, halo, hydroxy, sulfo, mercapto, sulfanyl [e.g., aliphaticsulfanyl or cycloaliphaticsulfanyl], sulfinyl [e.g., aliphaticsulfinyl or cycloaliphaticsulfinyl], sulfonyl [e.g., aliphatic-SO₂-, aliphaticamino-SO₂-, or cycloaliphatic-SO₂-], amido [e.g., aminocarbonyl, alkylaminocarbonyl, alkylcarbonylamino, cycloalkylaminocarbonyl, heterocycloalkylaminocarbonyl, cycloalkylcarbonylamino, arylaminocarbonyl, arylcarbonylamino, aralkylcarbonylamino, (heterocycloalkyl)carbonylamino, (cycloalkylalkyl)carbonylamino, heteroaralkylcarbonylamino, heteroarylcarbonylamino or heteroarylaminocarbonyl], urea, thiourea, sulfamoyl, sulfamide, alkoxycarbonyl, alkylcarbonyloxy, cycloaliphatic, heterocycloaliphatic, aryl, heteroaryl, acyl [e.g., (cycloaliphatic)carbonyl or (heterocycloaliphatic)carbonyl], amino [e.g., aliphaticamino], sulfoxy, oxo, carboxy, carbamoyl, (cycloaliphatic)oxy, (heterocycloaliphatic)oxy, or (heteroaryl)alkoxy.

As used herein, an "amido" encompasses both "aminocarbonyl" and "carbonylamino." These terms when used alone or in connection with another group refer to an amido group such as -N(R^{X})-C(O)-R^{Y} or -C(O)-N(R^{X})₂, when used terminally, and -C(O)-N(R^{X})- or -N(R^{X})-C(O)- when used internally, wherein R^{X} and R^{Y} can be aliphatic, cycloaliphatic, aryl, araliphatic, heterocycloaliphatic, heteroaryl or heteroaraliphatic. Examples of amido groups include alkylamido (such as alkylcarbonylamino or alkylaminocarbonyl), (heterocycloaliphatic)amido, (heteroaralkyl)amido, (heteroaryl)amido, (heterocycloalkyl)alkylamido, arylamido, aralkylamido, (cycloalkyl)alkylamido, or cycloalkylamido.

As used herein, an "amino" group refers to -NR^{X}R^{Y} wherein each of R^{X} and R^{Y} is independently hydrogen, aliphatic, cycloaliphatic, (cycloaliphatic)aliphatic, aryl, araliphatic, heterocycloaliphatic, (heterocycloaliphatic)aliphatic, heteroaryl, carboxy, sulfanyl, sulfinyl, sulfonyl, (aliphatic)carbonyl, (cycloaliphatic)carbonyl, ((cycloaliphatic)aliphatic)carbonyl, arylcarbonyl, (araliphatic)carbonyl, (heterocycloaliphatic)carbonyl, ((heterocycloaliphatic)aliphatic)carbonyl, (heteroaryl)carbonyl, or (heteroaraliphatic)carbonyl, each of which being defined herein and being optionally substituted. Examples of amino groups include alkylamino, dialkylamino, or arylamino. When the term "amino" is not the terminal group (e.g., alkylcarbonylamino), it is represented by -NR^{X}-, where R^{X} has the same meaning as defined above.

As used herein, an "aryl" group used alone or as part of a larger moiety as in "aralkyl," "aralkoxy," or "aryloxyalkyl" refers to monocyclic (e.g., phenyl); bicyclic (e.g., indenyl, naphthalenyl, tetrahydronaphthyl, tetrahydroindenyl); and tricyclic (e.g., fluorenyl tetrahydrofluorenyl, or tetrahydroanthracenyl, anthracenyl) ring systems in which the monocyclic ring system is aromatic or at least one of the rings in a bicyclic or tricyclic ring system is aromatic. The bicyclic and tricyclic groups include benzofused 2-3 membered carbocyclic rings. For example, a benzofused group includes phenyl fused with two or more C₄₋₈ carbocyclic moieties. An aryl is optionally substituted with one or more substituents including aliphatic [e.g., alkyl, alkenyl, or alkynyl]; cycloaliphatic; (cycloaliphatic)aliphatic; heterocycloaliphatic; (heterocycloaliphatic)aliphatic; aryl; heteroaryl; alkoxy; (cycloaliphatic)oxy; (heterocycloaliphatic)oxy; aryloxy; heteroaryloxy; (araliphatic)oxy; (heteroaraliphatic)oxy; aroyl; heteroaroyl; amino; oxo (on a non-aromatic carbocyclic ring of a benzofused bicyclic or tricyclic aryl); nitro; carboxy; amido; acyl [e.g., (aliphatic)carbonyl; (cycloaliphatic)carbonyl; ((cycloaliphatic)aliphatic)carbonyl; (araliphatic)carbonyl; (heterocycloaliphatic)carbonyl; ((heterocycloaliphatic)aliphatic)carbonyl; or (heteroaraliphatic)carbonyl]; sulfonyl [e.g., aliphatic-SO₂- or amino-SO₂-]; sulfinyl [e.g., aliphatic-S(O)- or cycloaliphatic-S(O)-]; sulfanyl [e.g., aliphatic-S-]; cyano; halo; hydroxy; mercapto; sulfoxy; urea; thiourea; sulfamoyl; sulfamide; or carbamoyl. Alternatively, an aryl can be unsubstituted.

Non-limiting examples of substituted aryls include haloaryl [e.g., mono-, di (such as p,m-dihaloaryl), and (trihalo)aryl]; (carboxy)aryl [e.g., (alkoxycarbonyl)aryl, ((aralkyl)carbonyloxy)aryl, and (alkoxycarbonyl)aryl]; (amido)aryl [e.g., (aminocarbonyl)aryl, (((alkylamino)alkyl)aminocarbonyl)aryl, (alkylcarbonyl)aminoaryl, (arylaminocarbonyl)aryl, and (((heteroaryl)amino)carbonyl)aryl]; aminoaryl [e.g., ((alkylsulfonyl)amino)aryl or ((dialkyl)amino)aryl]; (cyanoalkyl)aryl; (alkoxy)aryl; (sulfamoyl)aryl [e.g., (aminosulfonyl)aryl]; (alkylsulfonyl)aryl; (cyano)aryl; (hydroxyalkyl)aryl; ((alkoxy)alkyl)aryl; (hydroxy)aryl, ((carboxy)alkyl)aryl; (((dialkyl)amino)alkyl)aryl; (nitroalkyl)aryl; (((alkylsulfonyl)amino)alkyl)aryl; ((heterocycloaliphatic)carbonyl)aryl; ((alkylsulfonyl)alkyl)aryl; (cyanoalkyl)aryl; (hydroxyalkyl)aryl; (alkylcarbonyl)aryl; alkylaryl; (trihaloalkyl)aryl; *p-amino-m-*alkoxycarbonylaryl; *p*-amino-*m*-cyanoaryl; *p*-halo-*m*-aminoaryl; or (m-(heterocycloaliphatic)-*o*-(alkyl))aryl.

As used herein, an "araliphatic" such as an "aralkyl" group refers to an aliphatic group (e.g., a C₁₋₄ alkyl group) that is substituted with an aryl group. "Aliphatic," "alkyl," and "aryl" are defined herein. An example of an araliphatic such as an aralkyl group is benzyl.

As used herein, an "aralkyl" group refers to an alkyl group (e.g., a C₁₋₄ alkyl group) that is substituted with an aryl group. Both "alkyl" and "aryl" have been defined above. An example of an aralkyl group is benzyl. An aralkyl is optionally substituted with one or more substituents such as aliphatic [e.g., alkyl, alkenyl, or alkynyl, including carboxyalkyl, hydroxyalkyl, or haloalkyl such as trifluoromethyl], cycloaliphatic [e.g., cycloalkyl or cycloalkenyl], (cycloalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, nitro, carboxy, alkoxycarbonyl, alkylcarbonyloxy, amido [e.g., aminocarbonyl, alkylcarbonylamino, cycloalkylcarbonylamino, (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, or heteroaralkylcarbonylamino], cyano, halo, hydroxy, acyl, mercapto, alkylsulfanyl, sulfoxy, urea, thiourea, sulfamoyl, sulfamide, oxo, or carbamoyl.

As used herein, a "bicyclic ring system" includes 6-12 (e.g., 8-12 or 9, 10, or 11) membered structures that form two rings, wherein the two rings have at least one atom in common (e.g., 2 atoms in common). Bicyclic ring systems include bicycloaliphatics (e.g., bicycloalkyl or bicycloalkenyl), bicycloheteroaliphatics, bicyclic aryls, and bicyclic heteroaryls.

As used herein, a "cycloaliphatic" group encompasses a "cycloalkyl" group and a "cycloalkenyl" group, each of which being optionally substituted as set forth below.

As used herein, a "cycloalkyl" group refers to a saturated carbocyclic mono- or bicyclic (fused or bridged) ring of 3-10 (e.g., 5-10) carbon atoms. Examples of cycloalkyl groups include cyclopropyl, cyclobutyl, cyclopentyl, cyclohexyl, cycloheptyl, adamantyl, norbornyl, cubyl, octahydro-indenyl, decahydro-naphthyl, bicyclo[3.2.1]octyl, bicyclo[2.2.2]octyl, bicyclo[3.3.1]nonyl, bicyclo[3.3.2.]decyl, bicyclo[2.2.2]octyl, adamantyl, or ((aminocarbonyl)cycloalkyl)cycloalkyl.

A "cycloalkenyl" group, as used herein, refers to a non-aromatic carbocyclic ring of 3-10 (e.g., 4-8) carbon atoms having one or more double bonds. Examples of cycloalkenyl groups include cyclopentenyl, 1,4-cyclohexa-di-enyl, cycloheptenyl, cyclooctenyl, hexahydro-indenyl, octahydro-naphthyl, cyclohexenyl, bicyclo[2.2.2]octenyl, or bicyclo[3.3.1]nonenyl.

A cycloalkyl or cycloalkenyl group can be optionally substituted with one or more substituents such as phospho, aliphatic [e.g., alkyl, alkenyl, or alkynyl], cycloaliphatic, (cycloaliphatic) aliphatic, heterocycloaliphatic, (heterocycloaliphatic) aliphatic, aryl, heteroaryl, alkoxy, (cycloaliphatic)oxy, (heterocycloaliphatic)oxy, aryloxy, heteroaryloxy, (araliphatic)oxy, (heteroaraliphatic)oxy, aroyl, heteroaroyl, amino, amido [e.g., (aliphatic)carbonylamino, (cycloaliphatic)carbonylamino, ((cycloaliphatic)aliphatic)carbonylamino, (aryl)carbonylamino, (araliphatic)carbonylamino, (heterocycloaliphatic)carbonylamino, ((heterocycloaliphatic)aliphatic)carbonylamino, (heteroaryl)carbonylamino, or (heteroaraliphatic)carbonylamino], nitro, carboxy [e.g., HOOC-, alkoxycarbonyl, or alkylcarbonyloxy], acyl [e.g., (cycloaliphatic)carbonyl, ((cycloaliphatic) aliphatic)carbonyl, (araliphatic)carbonyl, (heterocycloaliphatic)carbonyl, ((heterocycloaliphatic)aliphatic)carbonyl, or (heteroaraliphatic)carbonyl], cyano, halo, hydroxy, mercapto, sulfonyl [e.g., alkyl-SO₂- and aryl-SO₂-], sulfinyl [e.g., alkyl-S(O)-], sulfanyl [e.g., alkyl-S-], sulfoxy, urea, thiourea, sulfamoyl, sulfamide, oxo, or carbamoyl.

As used herein, the term "heterocycloaliphatic" encompasses heterocycloalkyl groups and heterocycloalkenyl groups, each of which being optionally substituted as set forth below.

As used herein, a "heterocycloalkyl" group refers to a 3-10 membered mono- or bicylic (fused or bridged) (e.g., 5- to 10-membered mono- or bicyclic) saturated ring structure, in which one or more of the ring atoms is a heteroatom (e.g., N, O, S, or combinations thereof). Examples of a heterocycloalkyl group include piperidyl, piperazyl, tetrahydropyranyl, tetrahydrofuryl, 1,4-dioxolanyl, 1,4-dithianyl, 1,3-dioxolanyl, oxazolidyl, isoxazolidyl, morpholinyl, thiomorpholyl, octahydrobenzofuryl, octahydrochromenyl, octahydrothiochromenyl, octahydroindolyl, octahydropyrindinyl, decahydroquinolinyl, octahydrobenzo[b]thiopheneyl, 2-oxa-bicyclo[2.2.2]octyl, 1-aza-bicyclo[2.2.2]octyl, 3-aza-bicyclo[3.2.1]octyl, and 2,6-dioxa-tricyclo[3.3.1.0^{3,7}]nonyl. A monocyclic heterocycloalkyl group can be fused with a phenyl moiety to form structures, such as tetrahydroisoquinoline, that would be categorized as heteroaryls.

A "heterocycloalkenyl" group, as used herein, refers to a mono- or bicylic (e.g., 5- to 10-membered mono- or bicyclic) non-aromatic ring structure having one or more double bonds, and wherein one or more of the ring atoms is a heteroatom (e.g., N, O, or S). Monocyclic and bicyclic heterocycloaliphatics are numbered according to standard chemical nomenclature.

A heterocycloalkyl or heterocycloalkenyl group can be optionally substituted with one or more substituents such as phospho, aliphatic [e.g., alkyl, alkenyl, or alkynyl], cycloaliphatic, (cycloaliphatic)aliphatic, heterocycloaliphatic, (heterocycloaliphatic)aliphatic, aryl, heteroaryl, alkoxy, (cycloaliphatic)oxy, (heterocycloaliphatic)oxy, aryloxy, heteroaryloxy, (araliphatic)oxy, (heteroaraliphatic)oxy, aroyl, heteroaroyl, amino, amido [e.g., (aliphatic)carbonylamino, (cycloaliphatic)carbonylamino, ((cycloaliphatic) aliphatic)carbonylamino, (aryl)carbonylamino, (araliphatic)carbonylamino, (heterocycloaliphatic)carbonylamino, ((heterocycloaliphatic) aliphatic)carbonylamino, (heteroaryl)carbonylamino, or (heteroaraliphatic)carbonylamino], nitro, carboxy [e.g., HOOC-, alkoxycarbonyl, or alkylcarbonyloxy], acyl [e.g., (cycloaliphatic)carbonyl, ((cycloaliphatic) aliphatic)carbonyl, (araliphatic)carbonyl, (heterocycloaliphatic)carbonyl, ((heterocycloaliphatic)aliphatic)carbonyl, or (heteroaraliphatic)carbonyl], nitro, cyano, halo, hydroxy, mercapto, sulfonyl [e.g., alkylsulfonyl or arylsulfonyl], sulfinyl [e.g., alkylsulfinyl], sulfanyl [e.g., alkylsulfanyl], sulfoxy, urea, thiourea, sulfamoyl, sulfamide, oxo, or carbamoyl.

A "heteroaryl" group, as used herein, refers to a monocyclic, bicyclic, or tricyclic ring system having 4 to 15 ring atoms wherein one or more of the ring atoms is a heteroatom (e.g., N, O, S, or combinations thereof) and in which the monocyclic ring system is aromatic or at least one of the rings in the bicyclic or tricyclic ring systems is aromatic. A heteroaryl group includes a benzofused ring system having 2 to 3 rings. For example, a benzofused group includes benzo fused with one or two 4 to 8 membered heterocycloaliphatic moieties (e.g., indolizyl, indolyl, isoindolyl, 3H-indolyl, indolinyl, benzo[b]furyl, benzo[b]thiophene-yl, quinolinyl, or isoquinolinyl). Some examples of heteroaryl are azetidinyl, pyridyl, 1H-indazolyl, furyl, pyrrolyl, thienyl, thiazolyl, oxazolyl, imidazolyl, tetrazolyl, benzofuryl, isoquinolinyl, benzthiazolyl, xanthene, thioxanthene, phenothiazine, dihydroindole, benzo[1,3]dioxole, benzo[b]furyl, benzo[b]thiophenyl, indazolyl, benzimidazolyl, benzthiazolyl, puryl, cinnolyl, quinolyl, quinazolyl,cinnolyl, phthalazyl, quinazolyl, quinoxalyl, isoquinolyl, 4H-quinolizyl, benzo-1,2,5-thiadiazolyl, or 1,8-naphthyridyl.

Without limitation, monocyclic heteroaryls include furyl, thiophene-yl, 2H-pyrrolyl, pyrrolyl, oxazolyl, thazolyl, imidazolyl, pyrazolyl, isoxazolyl, isothiazolyl, 1,3,4-thiadiazolyl, 2H-pyranyl, 4-H-pranyl, pyridyl, pyridazyl, pyrimidyl, pyrazolyl, pyrazyl, or 1,3,5-triazyl. Monocyclic heteroaryls are numbered according to standard chemical nomenclature.

Without limitation, bicyclic heteroaryls include indolizyl, indolyl, isoindolyl, 3H-indolyl, indolinyl, benzo[b]furyl, benzo[b]thiophenyl, quinolinyl, isoquinolinyl, indolizyl, isoindolyl, indolyl, benzo[b]furyl, bexo[b]thiophenyl, indazolyl, benzimidazyl, benzthiazolyl, purinyl, 4H-quinolizyl, quinolyl, isoquinolyl, cinnolyl, phthalazyl, quinazolyl, quinoxalyl, 1,8-naphthyridyl, or pteridyl. Bicyclic heteroaryls are numbered according to standard chemical nomenclature.

A heteroaryl is optionally substituted with one or more substituents such as aliphatic [e.g., alkyl, alkenyl, or alkynyl]; cycloaliphatic; (cycloaliphatic)aliphatic; heterocycloaliphatic; (heterocycloaliphatic)aliphatic; aryl; heteroaryl; alkoxy; (cycloaliphatic)oxy; (heterocycloaliphatic)oxy; aryloxy; heteroaryloxy; (araliphatic)oxy; (heteroaraliphatic)oxy; aroyl; heteroaroyl; amino; oxo (on a non-aromatic carbocyclic or heterocyclic ring of a bicyclic or tricyclic heteroaryl); carboxy; amido; acyl [ e.g., aliphaticcarbonyl; (cycloaliphatic)carbonyl; ((cycloaliphatic)aliphatic)carbonyl; (araliphatic)carbonyl; (heterocycloaliphatic)carbonyl; ((heterocycloaliphatic)aliphatic)carbonyl; or (heteroaraliphatic)carbonyl]; sulfonyl [e.g., aliphaticsulfonyl or aminosulfonyl]; sulfinyl [e.g., aliphaticsulfinyl]; sulfanyl [e.g., aliphaticsulfanyl]; nitro; cyano; halo; hydroxy; mercapto; sulfoxy; urea; thiourea; sulfamoyl; sulfamide; or carbamoyl. Alternatively, a heteroaryl can be unsubstituted.

Non-limiting examples of substituted heteroaryls include (halo)heteroaryl [e.g., mono- and di-(halo)heteroaryl]; (carboxy)heteroaryl [e.g., (alkoxycarbonyl)heteroaryl]; cyanoheteroaryl; aminoheteroaryl [e.g., ((alkylsulfonyl)amino)heteroaryl and ((dialkyl)amino)heteroaryl]; (amido)heteroaryl [e.g., aminocarbonylheteroaryl, ((alkylcarbonyl)amino)heteroaryl, ((((alkyl)amino)alkyl)aminocarbonyl)heteroaryl, (((heteroaryl)amino)carbonyl)heteroaryl, ((heterocycloaliphatic)carbonyl)heteroaryl, and ((alkylcarbonyl)amino)heteroaryl]; (cyanoalkyl)heteroaryl; (alkoxy)heteroaryl; (sulfamoyl)heteroaryl [e.g., (aminosulfonyl)heteroaryl]; (sulfonyl)heteroaryl [e.g., (alkylsulfonyl)heteroaryl]; (hydroxyalkyl)heteroaryl; (alkoxyalkyl)heteroaryl; (hydroxy)heteroaryl; ((carboxy)alkyl)heteroaryl; (((dialkyl)amino)alkyl]heteroaryl; (heterocycloaliphatic )heteroaryl; (cycloaliphatic)heteroaryl; (nitroalkyl)heteroaryl; (((alkylsulfonyl)amino)alkyl)heteroaryl; ((alkylsulfonyl)alkyl)heteroaryl; (cyanoalkyl)heteroaryl; (acyl)heteroaryl [e.g., (alkylcarbonyl)heteroaryl]; (alkyl)heteroaryl; or (haloalkyl)heteroaryl [e.g., trihaloalkylheteroaryl].

As used herein, a "heteroaraliphatic" (such as a heteroaralkyl group) refers to an aliphatic group (e.g., a C₁₋₄ alkyl group) that is substituted with a heteroaryl group. "Aliphatic," "alkyl," and "heteroaryl" have been defined above.

As used herein, a "heteroaralkyl" group refers to an alkyl group (e.g., a C₁₋₄ alkyl group) that is substituted with a heteroaryl group. Both "alkyl" and "heteroaryl" have been defined above. A heteroaralkyl is optionally substituted with one or more substituents such as alkyl (including carboxyalkyl, hydroxyalkyl, and haloalkyl such as trifluoromethyl), alkenyl, alkynyl, cycloalkyl, (cycloalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, nitro, carboxy, alkoxycarbonyl, alkylcarbonyloxy, aminocarbonyl, alkylcarbonylamino, cycloalkylcarbonylamino, (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino, cyano, halo, hydroxy, acyl, mercapto, alkylsulfanyl, sulfoxy, urea, thiourea, sulfamoyl, sulfamide, oxo, or carbamoyl.

As used herein, "cyclic moiety" and "cyclic group" refer to mono-, bi-, and tri-cyclic ring systems including cycloaliphatic, heterocycloaliphatic, aryl, or heteroaryl, each of which has been previously defined.

As used herein, a "bridged bicyclic ring system" refers to a bicyclic heterocyclicalipahtic ring system or bicyclic cycloaliphatic ring system in which the rings are bridged. Examples of bridged bicyclic ring systems include, but are not limited to, adamantanyl, norbornanyl, bicyclo[3.2.1]octyl, bicyclo[2.2.2]octyl, bicyclo[3.3.1]nonyl, bicyclo[3.3.2]decyl, 2-oxabicyclo[2.2.2]octyl, 1-azabicyclo[2.2.2]octyl, 3-azabicyclo[3.2.1]octyl, and 2,6-dioxa-tricyclo[3.3.1.0^{3,7}]nonyl. A bridged bicyclic ring system can be optionally substituted with one or more substituents such as alkyl (including carboxyalkyl, hydroxyalkyl, and haloalkyl such as trifluoromethyl), alkenyl, alkynyl, cycloalkyl, (cycloalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, nitro, carboxy, alkoxycarbonyl, alkylcarbonyloxy, aminocarbonyl, alkylcarbonylamino, cycloalkylcarbonylamino, (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino, cyano, halo, hydroxy, acyl, mercapto, alkylsulfanyl, sulfoxy, urea, thiourea, sulfamoyl, sulfamide, oxo, or carbamoyl.

As used herein, an "acyl" group refers to a formyl group or R^{X}-C(O)- (such as alkyl-C(O)-, also referred to as "alkylcarbonyl") where R^{X} and "alkyl" have been defined previously. Acetyl and pivaloyl are examples of acyl groups.

As used herein, an "aroyl" or "heteroaroyl" refers to an aryl-C(O)- or a heteroaryl-C(O)-. The aryl and heteroaryl portion of the aroyl or heteroaroyl is optionally substituted as previously defined.

As used herein, an "alkoxy" group refers to an alkyl-O- group where "alkyl" has been defined previously.

As used herein, a "carbamoyl" group refers to a group having the structure -O-CO-NR^{X}R^{Y} or -NR^{X}-CO-O-R^{Z}, wherein R^{X} and R^{Y} have been defined above and R^{Z} can be aliphatic, aryl, araliphatic, heterocycloaliphatic, heteroaryl, or heteroaraliphatic.

As used herein, a "carboxy" group refers to -COOH, -COOR^{X}, -OC(O)H, -OC(O)R^{X}, when used as a terminal group; or -OC(O)- or -C(O)O- when used as an internal group.

As used herein, a "haloaliphatic" group refers to an aliphatic group substituted with 1-3 halogen. For instance, the term haloalkyl includes the group -CF₃.

As used herein, a "mercapto" group refers to -SH.

As used herein, a "sulfo" group refers to -SO₃H or -SO₃R^{X} when used terminally or -S(O)₃- when used internally.

As used herein, a "sulfamide" group refers to the structure -NR^{X}-S(O)₂-NR^{Y}R^{Z} when used terminally and -NR^{X}-S(O)₂-NR^{Y}- when used internally, wherein R^{X}, R^{Y}, and R^{Z} have been defined above.

As used herein, a "sulfamoyl" group refers to the structure -O-S(O)₂-NR^{Y}R^{Z} wherein R^{Y} and R^{Z} have been defined above.

As used herein, a "sulfonamide" group refers to the structure -S(O)₂-NR^{X}R^{Y} or -NRX-S(O)₂-R^{Z} when used terminally; or -S(O)₂-NR^{X}- or -NR^{X} -S(O)₂- when used internally, wherein R^{X}, R^{Y}, and R^{Z} are defined above.

As used herein a "sulfanyl" group refers to -S-R^{X} when used terminally and -S-when used internally, wherein R^{X} has been defined above. Examples of sulfanyls include aliphatic-S-, cycloaliphatic-S-, aryl-S-, or the like.

As used herein a "sulfinyl" group refers to -S(O)-R^{X} when used terminally and - S(O)- when used internally, wherein R^{X} has been defined above. Examples of sulfinyl groups include aliphatic-S(O)-, aryl-S(O)-, (cycloaliphatic(aliphatic))-S(O)-, cycloalkylS(O)-, heterocycloaliphatic-S(O)-, heteroaryl-S(O)-, or the like.

As used herein, a "sulfonyl" group refers to-S(O)₂-R^{X} when used terminally and -S(O)₂- when used internally, wherein R^{X} has been defined above. Examples of sulfonyl groups include aliphatic-S(O)₂-, aryl-S(O)₂-, (cycloaliphatic(aliphatic))-S(O)₂-, cycloaliphatic-S(O)₂-, heterocycloaliphatic-S(O)₂-, heteroaryl-S(O)₂-, (cycloaliphatic(amido(aliphatic)))-S(O)₂-or the like.

As used herein, a "sulfoxy" group refers to -O-S(O)-R^{X} or -S(O)-O-R^{X}, when used terminally and -O-S(O)- or -S(O)-O- when used internally, where R^{X} has been defined above.

As used herein, a "halogen" or "halo" group refers to fluorine, chlorine, bromine or iodine.

As used herein, an "alkoxycarbonyl," which is encompassed by the term carboxy, used alone or in connection with another group refers to a group such as alkyl-O-C(O)-.

As used herein, an "alkoxyalkyl" refers to an alkyl group such as alkyl-O-alkyl-, wherein alkyl has been defined above.

As used herein, a "carbonyl" refers to -C(O)-.

As used herein, an "oxo" refers to =O.

As used herein, the term "phospho" refers to phosphinates and phosphonates. Examples of phosphinates and phosphonates include -P(O)(R^{P})₂, wherein R^{P} is aliphatic, alkoxy, aryloxy, heteroaryloxy, (cycloaliphatic)oxy, (heterocycloaliphatic)oxy aryl, heteroaryl, cycloaliphatic or amino.

As used herein, an "aminoalkyl" refers to the structure (R^{X})₂N-alkyl-.

As used herein, a "cyanoalkyl" refers to the structure (NC)-alkyl-.

As used herein, a "urea" group refers to the structure -NR^{X}-CO-NR^{Y}R^{Z} and a "thiourea" group refers to the structure -NR^{X}-CS-NR^{Y}R^{Z} when used terminally and -NR^{X}-CO-NR^{Y}- or -NR^{X}-CS-NR^{Y}- when used internally, wherein R^{X}, R^{Y}, and R^{Z} have been defined above.

As used herein, a "guanidine" group refers to the structure -N=C(N(R^{X}R^{Y}))N(R^{X}R^{Y}) or -NR^{X}-C(=NR^{X})NR^{X}R ^{Y} wherein R^{X} and R^{Y} have been defined above.

As used herein, the term "amidino" group refers to the structure -C=(NR^{X})N(R^{X}R^{Y}) wherein R^{X} and R^{Y} have been defined above.

As used herein, the term "vicinal" generally refers to the placement of substituents on a group that includes two or more carbon atoms, wherein the substituents are attached to adjacent carbon atoms.

As used herein, the term "geminal" generally refers to the placement of substituents on a group that includes two or more carbon atoms, wherein the substituents are attached to the same carbon atom.

The terms "terminally" and "internally" refer to the location of a group within a substituent. A group is terminal when the group is present at the end of the substituent not further bonded to the rest of the chemical structure. Carboxyalkyl, i.e., R^{X}O(O)C-alkyl, is an example of a carboxy group used terminally. A group is internal when the group is present in the middle of a substituent of the chemical structure. Alkylcarboxy (e.g., alkyl-C(O)O- or alkyl-OC(O)-) and alkylcarboxyaryl (e.g., alkyl-C(O)O-aryl- or alkyl-O(CO)-aryl-) are examples of carboxy groups used internally.

As used herein, an "aliphatic chain" refers to a branched or straight aliphatic group (e.g., alkyl groups, alkenyl groups, or alkynyl groups). A straight aliphatic chain has the structure -[CH₂]ᵥ-, where v is 1-12. A branched aliphatic chain is a straight aliphatic chain that is substituted with one or more aliphatic groups. A branched aliphatic chain has the structure -[CQQ]ᵥ- where Q is independently a hydrogen or an aliphatic group; however, Q shall be an aliphatic group in at least one instance. The term aliphatic chain includes alkyl chains, alkenyl chains, and alkynyl chains, where alkyl, alkenyl, and alkynyl are defined above.

The phrase "optionally substituted" is used herein interchangeably with the phrase "substituted or unsubstituted." As described herein, compounds of the disclosure can optionally be substituted with one or more substituents, such as are illustrated generally above, or as exemplified by particular classes, subclasses, and species of the disclosure. As described herein, the variables R, R¹, R², R³, R⁴, R⁶, R¹⁵, R^{A}, R^{B}, R^{C}, R^{D}, L, X¹, X², X^{B}, X^{Y}, Y¹, Y², Y³, Y⁴, Y⁵, Y⁶, Z, A¹, A², A³, A⁴, A⁵, D, D¹, D², E, and other variables contained in Formulae (**I**), (**I-A**), (**I-A1**), (**I-A2**), (**I-A3**), (**I-B1**), (**I-B2**), (**I-B3**), (**II**), (**II-A**), (**II-B**), (**III**), (**III-A**), (**III-B**), and (**III-C**) described herein encompass specific groups, such as alkyl and aryl. Unless otherwise noted, each of the specific groups for the variables R, R¹, R², R³, R⁴, R¹⁵, R^{A}, R^{B}, R^{C}, R^{D}, L, X¹, X², X^{B}, X^{Y}, Y¹, Y², Y³, Y⁴, Y⁵, Y⁶, Z, A¹, A², A³, A⁴, A⁵, D, and other variables contained therein can be optionally substituted with one or more substituents described herein. Each substituent of a specific group is further optionally substituted with one to three of halo, cyano, oxo, alkoxy, hydroxy, amino, nitro, aryl, cycloaliphatic, heterocycloaliphatic, heteroaryl, haloalkyl, and alkyl. For instance, an alkyl group can be substituted with alkylsulfanyl and the alkylsulfanyl can be optionally substituted with one to three of halo, cyano, oxo, alkoxy, hydroxy, amino, nitro, aryl, haloalkyl, and alkyl. As an additional example, the cycloalkyl portion of a (cycloalkyl)carbonylamino can be optionally substituted with one to three of halo, cyano, alkoxy, hydroxy, nitro, haloalkyl, and alkyl. When two alkoxy groups are bound to the same atom or adjacent atoms, the two alkoxy groups can form a ring together with the atom(s) to which they are bound.

As used herein, the term "substituted," whether preceded by the term "optionally" or not, refers generally to the replacement of hydrogen atoms in a given structure with the radical of a specified substituent. Specific substituents are described above in the definitions and below in the description of compounds and examples thereof. Unless otherwise indicated, an optionally substituted group can have a substituent at each substitutable position of the group, and when more than one position in any given structure can be substituted with more than one substituent selected from a specified group, the substituent can be either the same or different at every position. A ring substituent, such as a heterocycloalkyl, can be bound to another ring, such as a cycloalkyl, to form a spiro-bicyclic ring system, e.g., both rings share one common atom. As one of ordinary skill in the art will recognize, combinations of substituents envisioned by the disclosure are those combinations that result in the formation of stable or chemically feasible compounds.

As used herein, the phrase "stable or chemically feasible" refers to compounds that are not substantially altered when subjected to conditions to allow for their production, detection, and preferably their recovery, purification, and use for one or more of the purposes disclosed herein. In some examples, a stable compound or chemically feasible compound is one that is not substantially altered when kept at a temperature of 40 °C or less, in the absence of moisture or other chemically reactive conditions, for at least a week.

As used herein, an "effective amount" is defined as the amount required to confer a therapeutic effect on the treated patient, and is typically determined based on age, surface area, weight, and condition of the patient. The interrelationship of dosages for animals and humans (based on milligrams per meter squared of body surface) is described by Freireich et al., Cancer Chemother. Rep., 50: 219 (1966). Body surface area may be approximately determined from height and weight of the patient. See, e.g., Scientific Tables, Geigy Pharmaceuticals, Ardsley, New York, 537 (1970). As used herein, "patient" refers to a mammal, including a human.

Unless otherwise stated, structures depicted herein also are meant to include all isomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of this disclosure. Unless otherwise stated, all tautomeric forms of the compounds of this disclosure are within the scope of this disclosure. Additionally, unless otherwise stated, structures depicted herein also are meant to include compounds that differ only in the presence of one or more isotopically enriched atoms. For example, compounds having the present structures except for the replacement of hydrogen by deuterium or tritium, or the replacement of a carbon by a ¹³C- or ¹⁴C-enriched carbon are within the scope of this disclosure. Such compounds are useful, for example, as analytical tools or probes in biological assays, or as therapeutic agents.

Chemical structures and nomenclature are derived from ChemDraw, version 11.0.1, Cambridge, MA.

It is noted that the use of the descriptors "first," "second," "third," or the like is used to differentiate separate elements (e.g., solvents, reaction steps, processes, reagents, or the like) and may or may not refer to the relative order or relative chronology of the elements described.

### II. BIFUNCTIONAL COMPOUNDS OF THE PRESENT INVENTION

The present disclosure provides bifunctional compounds that induce the proteolytic degradation of targeted BTK via a ubiquitin proteosome pathway.

### A. Bifunctional Compounds

The present disclosure provides bifunctional compounds that induce the proteolytic degradation of BTK via a ubiquitin proteolysis pathway. The present disclosure provides a compound of Formula (I): 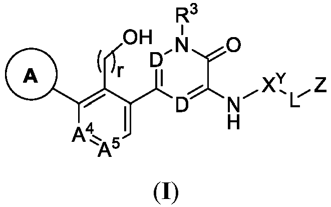 or a pharmaceutically acceptable salt thereof, wherein each of A⁴ and A⁵ is independently N or C-R¹⁵, each R¹⁵ is independently -H or an optionally substituted C₁₋₆ alkyl; each D is independently N or CH; ring A is 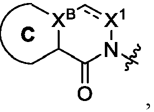 wherein --- is a bond or is absent, X¹ is -N= or -CH= when --- is a bond, X¹ is -NH- or -CH₂- when --- is absent, X^{B} is N or C, and ring C is a 6 membered fully unsaturated carbocycle when X^{B} is C, or a 5-6 membered partially or fully unsaturated heterocycle having 1-2 nitrogen atoms when X^{B} is N, wherein ring C is optionally substituted with R¹ and R² groups, wherein each of R¹ and R² is independently halo, -H, -OH, -CN, -CF₃, -C₁₋₆ alkyl, or a 3-6 membered cycloaliphatic, or R¹ and R² together with the atoms to which they are attached form a 5-6 membered cycloalkyl fused to ring C, wherein the cycloalkyl is optionally substituted with 1-2 of R⁶, wherein each R⁶ is independently -H, halo, or C₁₋₃ alkyl; R³ is C₁₋₆ alkyl; X^{Y} is 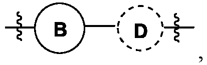 wherein ring B is phenyl, or a 5-6 membered monocyclic heteroaryl having 1-2 nitrogen atoms, or a 7-10 membered fused bicyclic heterocycle having 2-3 nitrogen atoms, wherein at least one of the rings of the bicyclic heterocycle is partially or fully unsaturated, and ring D is absent or an optionally substituted 4-7 membered heterocycle having at least 1 nitrogen atom and optionally 2 additional heteroatoms independently selected from N, O, or S; L is a bivalent C₁₋₂₀ alkyl group, wherein one or more methylene units of the C₁₋₂₀ alkyl group is optionally and independently replaced by -C(O)-, -C(S)-, -(O)CO-, -OC(O)-, -O-, -N(R')-, -S-, -N(R')C(O)-, bivalent mono- or bicyclic heterocycloalkyl, bivalent mono- or bicyclic cycloalkyl, bivalent mono- or bicyclic aryl, or bivalent mono- or bicyclic heteroaryl, wherein each of the bivalent heterocycloalkyl, cycloalkyl, aryl, and heteroaryl are optionally substituted; and Z is 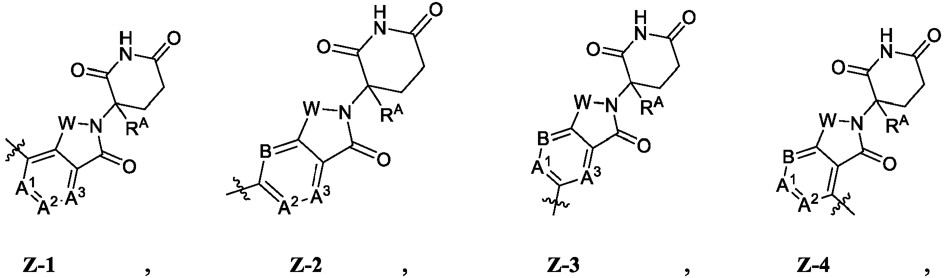 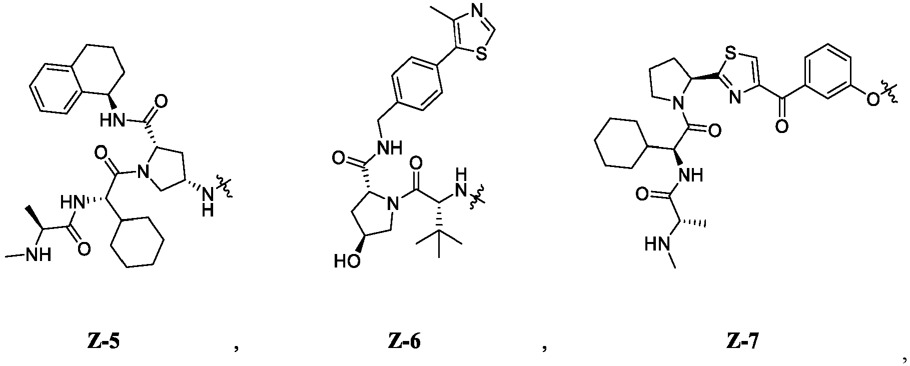 or 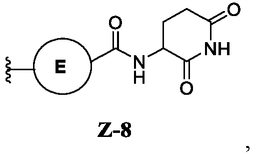 wherein ring E is phenyl or a 5-6 membered heteroaryl having at least 1 heteroatom independently selected from N or S; each of A¹, A², and A³ is independently -C(R^{B})= or -N=; B is -C(R^{C})= or -N=; W is -C(R^{D})₂- or -C(O)-; R^{A} is -H, -CH₃, or -F; each R^{B} is independently-H, halo, -OH, -C₁₋₄ alkyl, or -C₁₋₄ alkoxy; R^{C} is -H, halo, or C₁₋₄ alkyl; and each R^{D} is independently-H or -C₁₋₃ alkyl; and r is 1, 2, or 3.

In some examples, Z is

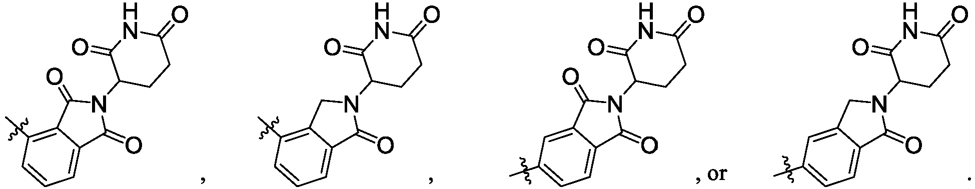

In some examples, Z is 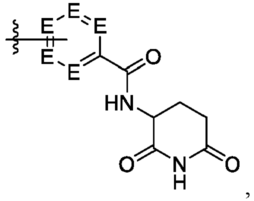 wherein one instance of E is 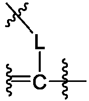 and the remaining instances of E are each independently =CH- or =N-, where L is as defined above for the compound of Formula (I). In some examples, Z is 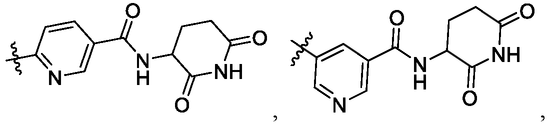 or

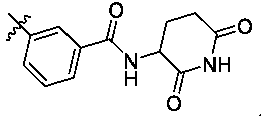

In some examples, one D is =CH- and the other D is =N-. In other examples, each D is =CH-. And, in some examples, both instances of D are =N-.

In some examples, at least one of A⁴ and A⁵ is N. For example, A⁴ is N, A⁵ is CR¹⁵, and R¹⁵ is -H or C₁₋₃ alkyl (e.g., methyl or ethyl).

In some examples, R¹⁵ is -H or methyl.

In some examples, L is a bivalent C₁₋₂₀ (e.g., C₁₋₁₅, C₁₋₁₂, C₁₋₁₀, C₁₋₇, C₁₋₆, or C₁₋₅) alkyl group, wherein one or more methylene units of the C₁₋₂₀ (e.g., C₁₋₁₅, C₁₋₁₂, C₁₋₁₀, C₁₋₇, C₁₋₆, or C₁₋₅) alkyl group is optionally and independently replaced by -CO-, -CS-, -OCO-, -O-, -NHCO-, -S-, -NH-, bivalent mono- or bicyclic heterocycle, bivalent mono- or bicyclic cycloalkyl, bivalent mono- or bicyclic aryl, or bivalent mono- or bicyclic heteroaryl, wherein each heterocycle and heteroaryl of L have 1-3 heteroatoms independently selected from N, O, or S.

In some examples, L is a bivalent C₁₋₂₀ (e.g., C₁₋₁₅, C₁₋₁₂, C₁₋₁₀, C₁₋₇, C₁₋₆, or C₁₋₅) alkyl group, wherein one or more methylene units of the C₁₋₂₀ (e.g., C₁₋₁₅, C₁₋₁₂, C₁₋₁₀, C₁₋₇, C₁₋₆, or C₁₋₅) alkyl group is optionally and independently replaced by -CO-, -O-, -NH-, bivalent mono- or bicyclic heterocycloalkyl having 1-3 heteroatoms independently selected from N, O, or S, or bivalent mono- or bicyclic cycloalkyl.

In some examples, L is a bivalent C₁₋₁₀ alkyl group, wherein one or more methylene units of the C₁₋₁₀ alkyl group is optionally and independently replaced by -CO-, -OCO-, -O-, -NHCO-, -NH-, bivalent mono- or bicyclic heterocycle, bivalent mono- or bicyclic cycloalkyl, bivalent mono- or bicyclic aryl, or bivalent mono- or bicyclic heteroaryl, wherein the heterocycle and heteroaryl of L have 1-3 heteroatoms independently selected from N, O, or S.

In some examples, L has at least one bivalent heterocycle selected from piperazine, piperidine, dioxane, pyran, morpholine, pyrrolidine, and tetrahydrofuran.

In some examples, L has at least one bivalent piperazine.

In some examples, L has at least one bivalent cycloalkyl selected from cyclopropyl, cyclobutyl, cyclopentyl, cyclohexyl, and cycloheptyl.

In some examples, L has at least one bivalent cyclohexyl.

In some examples, L is -Y¹-Y²-Y³-Y⁴-Y⁵-Y⁶-, wherein Y¹ is -C(O)- or -C₁₋₆ alkyl-; Y² is a bond, -O-, -(CH₂-CH₂-O)ₘ-, -(O-CH₂-CH₂)ₘ-, -C₁₋₄ alkyl-, 3-6 membered cycloalkyl; Y³ a bond, -O-, -N(R)-, -C₁₋₄ alkyl-, -(CH₂-CH₂-O)ₚ-, -(O-CH₂-CH₂)ₚ-, -(CH₂-CH₂-N(R))-, phenyl, 3-6 membered cycloalkyl, or 4-6 membered heterocycle having 1-3 heteroatoms independently selected from N, O, or S; Y⁴ is a bond, -C(O)-, -C(O)N(R)-, -C(O)N(R)-, -C₁₋₄ alkyl-, -(CH₂-CH₂-O)_{q}-, -(O-CH₂-CH₂)_{q}-, -(CH₂-CH₂-N(R))-, 3-6 membered cycloalkyl, or 4-6 membered heterocycle having 1-3 heteroatoms independently selected from N, O, or S; Y⁵ is a bond, -N(R)-, -C(O)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S; Y⁶ is a bond, or -(CH₂-CH₂-N(R))-; each of m and n is independently 1, 2, 3, 4, or 5; each of p, q, and s is independently 1, 2, or 3; and R is -H or -C₁₋₃ alkyl.

In some examples, Y¹ is -C(O)- or -C₄₋₆ alkyl-.

In some examples, Y² is -(CH₂-CH₂-O)ₘ-, -(O-CH₂-CH₂)ₘ-, or -C₁₋₄ alkyl-.

In some examples, Y³ a bond, -O-, -C₁₋₄ alkyl-, -(CH₂-CH₂-N(R))-,

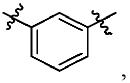

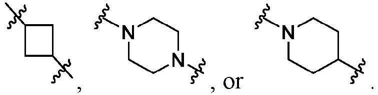

In some examples, Y⁴ is a bond, -C(O)-, -C(O)N(R)-, -C(O)N(R)-, or -C₁₋₄ alkyl-.

In some examples, Y⁵ is a bond, -N(R)-, -C(O)-, -C₁₋₄ alkyl-, or

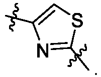

In some examples, Y⁶ is a bond or -(CH₂-CH₂-N(R))-.

In some examples, --- is a bond, X¹ is N, X^{B} is C, and ring C is a 6 membered fully unsaturated carbocycle, optionally substituted with R¹ and R² groups.

In some examples, ring A is 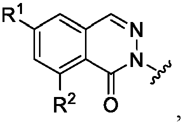 wherein each of R¹ and R² is independently halo, -H, -OH, -CN, -CF₃, -C₁₋₆ alkyl, or a 3-6 membered cycloaliphatic.

In some examples, --- is absent, X¹ is -CH₂- , X^{B} is N, and ring C is a 5-6 membered partially or fully unsaturated heterocycle having 1-2 nitrogen atoms, wherein ring C is substituted with R¹ and R² groups.

In some examples, ring A is 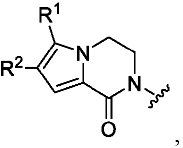 wherein each of R¹ and R² is independently halo, -H, -OH, -CN, -CF₃, -C₁₋₆ alkyl, or a 3-6 membered cycloaliphatic, or R¹ and R² together with the atoms to which they are attached form a 5-6 membered cycloalkyl, wherein the cycloalkyl is optionally substituted with 1-2 C₁₋₃ alkyl groups. In some of these examples, R¹ and R² together with the atoms to which they are attached form a 5-6 membered cycloalkyl (e.g., cyclopentyl or cyclohexyl), wherein the cycloalkyl is optionally substituted with 1-2 C₁₋₃ alkyl (e.g., methyl or ethyl) groups.

In some examples, ring A is 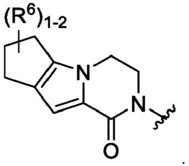 For instance, ring A is

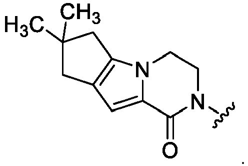

This disclosure also provides a compound of Formula (**I-A**) 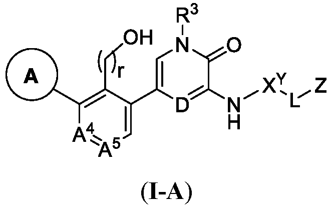 or a pharmaceutically acceptable salt thereof, wherein ring A, A⁴, A⁵, r, R³, X^{Y}, L and Z are as defined in any of the compounds of Formulae **(I), (I-A1), (I-A2), (I-A3),** (I-B1), **(I-B2), (I-B3), (II), (II-A), (II-B), (III), (III-A), (III-B),** and (**III-C**) (as applicable).

In some examples, each of A ⁴ and A⁵ is independently N or C-R¹⁵, each R¹⁵ is independently -H or an optionally substituted C₁₋₆ alkyl; D is =N- or =CH-; ring A is 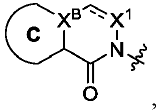 wherein --- is a bond or is absent, X¹ is -N= or -CH= when --- is a bond, X¹ is -NH- or -CH₂- when --- is absent, X^{B} is N or C, and ring C is a 6 membered fully unsaturated carbocycle when X^{B} is C, or a 5-6 membered partially or fully unsaturated heterocycle having 1-2 nitrogen atoms when X^{B} is N, wherein ring C is optionally substituted with R¹ and R² groups, wherein each of R¹ and R² is independently halo, -H, -OH, -CN, -CF₃, -C₁₋₆ alkyl, or a 3-6 membered cycloaliphatic, or R¹ and R² together with the atoms to which they are attached form a 5-6 membered cycloalkyl fused to ring C, wherein the cycloalkyl is optionally substituted with 1-2 of R⁶, wherein each R⁶ is independently -H, halo, or C₁₋₃ alkyl; R³ is C₁₋₆ alkyl (e.g., C₁₋₃ alkyl); X^{Y} is 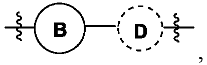 wherein ring B is phenyl, or a 5-6 membered monocyclic heteroaryl having 1-2 nitrogen atoms, or a 7-10 membered fused bicyclic heterocycle having 2-3 nitrogen atoms, wherein at least one of the rings of the bicyclic heterocycle is partially or fully unsaturated, and ring D is absent or an optionally substituted 4-7 membered heterocycle having at least 1 nitrogen atom and optionally 2 additional heteroatoms independently selected from N, O, or S; L is a bivalent C₁₋₂₀ alkyl group, wherein one or more methylene units of the C₁₋₂₀ alkyl group is optionally and independently replaced by -C(O)-, -C(S)-, -(O)CO-, -OC(O)-, -O-, -N(R')-, -S-, -N(R')C(O)-, bivalent mono- or bicyclic heterocycloalkyl, bivalent mono- or bicyclic cycloalkyl, bivalent mono- or bicyclic aryl, or bivalent mono- or bicyclic heteroaryl, wherein each of the bivalent heterocycloalkyl, cycloalkyl, aryl, and heteroaryl are optionally substituted; and Z is 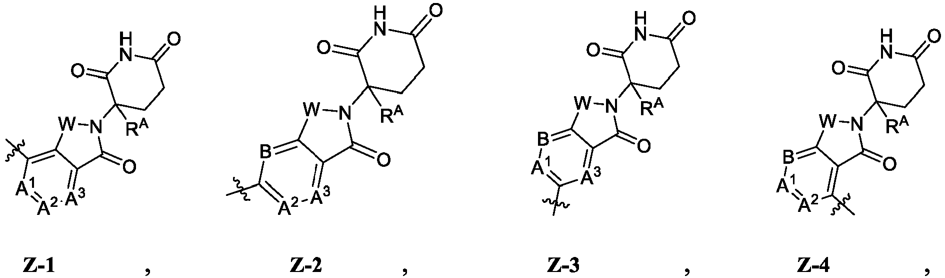 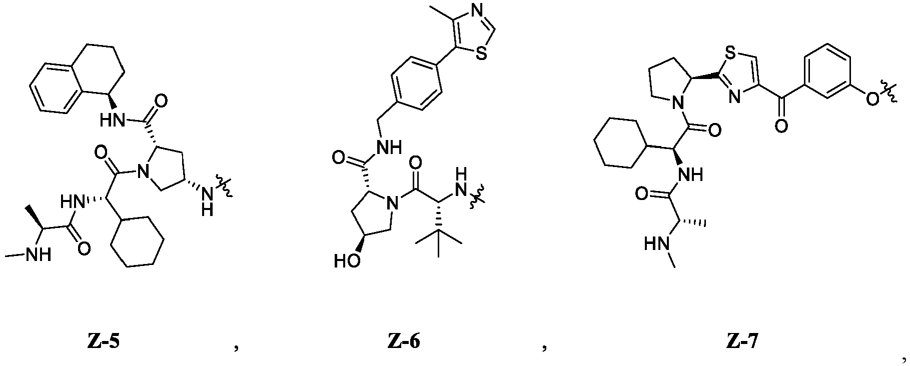 or 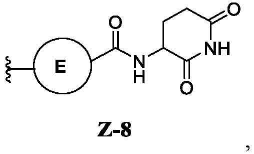 wherein ring E is phenyl or a 5-6 membered heteroaryl having at least 1 heteroatom independently selected from N or S; each of A¹, A², and A³ is independently -C(R^{B})= or -N=; B is -C(R^{C})= or -N=; W is -C(R^{D})₂- or -C(O)-; R^{A} is -H, -CH₃, or -F; each R^{B} is independently-H, halo, -OH, -C₁₋₄ alkyl, or -C₁₋₄ alkoxy; R^{C} is -H, halo, or C₁₋₄ alkyl; and each R^{D} is independently-H or -C₁₋₃ alkyl; and r is 1, 2, or 3.

In some examples, ring A is 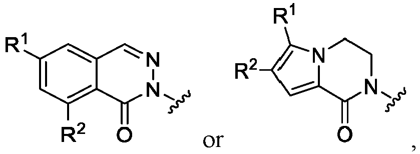 wherein each of R¹ and R² is independently halo, -H, -OH, -CN, -CF₃, -C₁₋₆ alkyl, or a 3-6 membered cycloaliphatic, or R¹ and R² together with the atoms to which they are attached form a 5-6 membered cycloalkyl, wherein the cycloalkyl is optionally substituted with 1-2 of R⁶; and D is =CH-.

In some examples, Z is 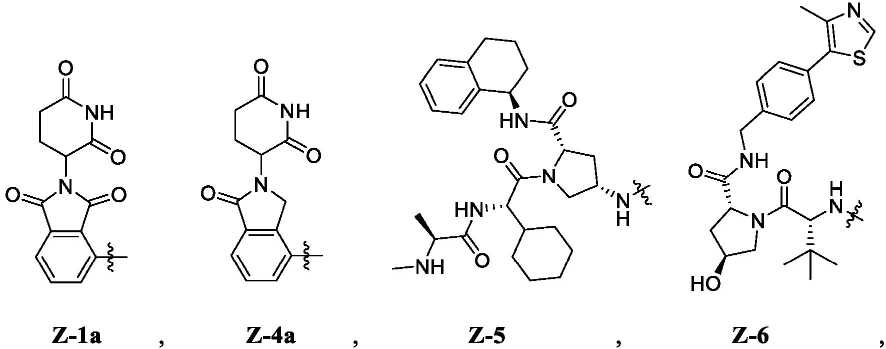 or

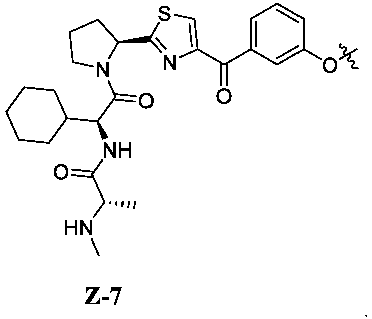

In some examples, --- is a bond, X¹ is N, X^{B} is C, and ring C is a 6 membered fully unsaturated carbocycle, optionally substituted with R¹ and R² groups.

In some examples, ring A is 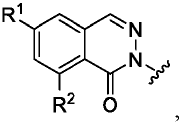 wherein each of R¹ and R² is independently halo, -H, -OH, -CN, -CF₃, -C₁₋₆ alkyl, or a 3-6 membered cycloaliphatic. For example, one of R¹ and R² is cyclopropyl, cyclobutyl, cyclopentyl, and the other is halo, -H, -OH, -CN, -CF₃, or -C₁₋₆ alkyl.

In some examples, --- is absent, X¹ is -CH₂- , X^{B} is N, and ring C is a 5-6 membered partially or fully unsaturated heterocycle having 1-2 nitrogen atoms, wherein ring C is substituted with R¹ and R² groups.

In some examples, ring A is 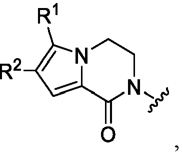 wherein each of R¹ and R² is independently halo, -H, -OH, -CN, -CF₃, -C₁₋₆ alkyl, or a 3-6 membered cycloaliphatic, or R¹ and R² together with the atoms to which they are attached form a 5-6 membered cycloalkyl, wherein the cycloalkyl is optionally substituted with 1-2 C₁₋₃ alkyl groups. In some of these examples, R¹ and R² together with the atoms to which they are attached form a 5-6 membered cycloalkyl (e.g., cyclopentyl or cyclohexyl), wherein the cycloalkyl is optionally substituted with 1-2 C₁₋₃ alkyl (e.g., methyl or ethyl) groups.

In some examples, ring A is 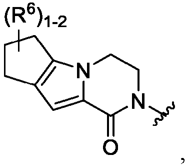 where R⁶ is as defined in the compound of Formula (I). For example, ring A is

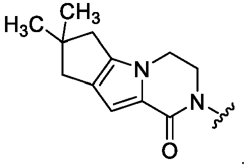

In some examples, R³ is C₁₋₆ alkyl. In other examples, R³ is a C₁₋₃ alkyl. For example, R³ is methyl, ethyl, or propyl. In other examples, R³ is methyl.

In some examples, r is 1 or 2. For example, r is 1.

In some examples, X^{Y} is 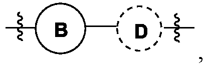 ring B is 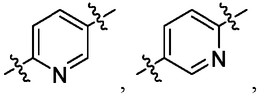 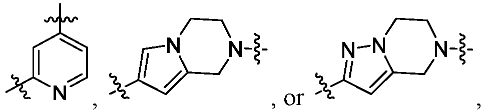 and ring D is absent. For example, ring B is 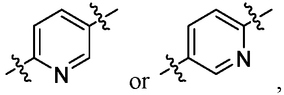 and ring C is absent. In other examples, ring B is 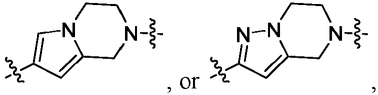 and ring C is absent.

In some examples, X^{Y} is 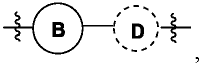 ring B is 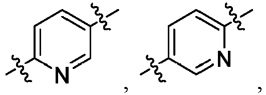 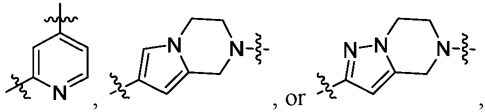 and ring D is 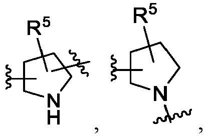 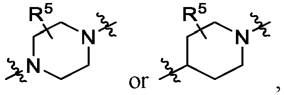 and *R⁵* is -H or C₁₋₃ alkyl.

In some examples, X^{Y} is 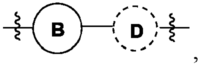 ring B is 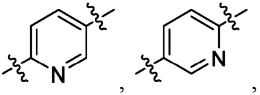 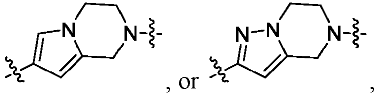 and ring D is 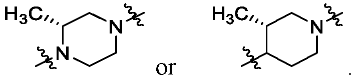 For example, ring B is 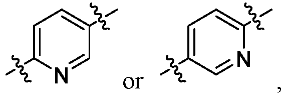 and ring D is 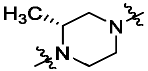 or 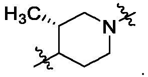 In other examples, ring B is 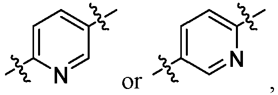 and ring D is 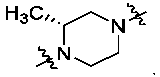 In some examples, ring B is 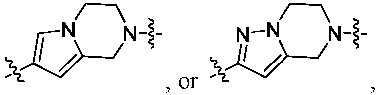 and ring D is 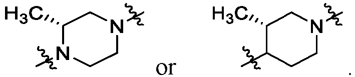 For instance, ring B is 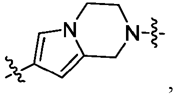 or 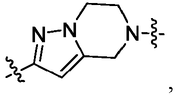 and ring D is

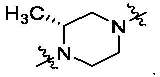

In some examples, L is -Y¹-Y²-Y³-Y⁴-Y⁵-Y⁶-, wherein Y¹ is -C(O)-, -C(O)-N(R)-, -(CH₂-CH₂-O)ₘ-, -(O-CH₂-CH₂)ₘ-, or -C₁₋₆ alkyl-; Y² is a bond, -C(O)-, -O-, -(CH₂-CH₂-O)ₙ-, -(O-CH₂-CH₂)ₙ-, -C₁₋₄ alkyl-, an optionally substituted 3-6 membered monocyclic cycloalkyl, or an optionally substituted 7-11 membered fused or spiro bicycloalkyl; Y³ is a bond, -O-, -N(R)-, -C₁₋₄ alkyl-, -(CH₂-CH₂-O)ₚ-, -(O-CH₂-CH₂)ₚ-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S; Y⁴ is a bond, -C(O)-, -N(R)-, -C(O)N(R)-, -N(R)C(O)-, -C₁₋₄ alkyl-, -(CH₂-CH₂-O)_{q}-, -(O-CH₂-CH₂)_{q}-, -(CH₂-CH₂-N(R))-, an optionally substituted 3-6 membered cycloalkyl, or an optionally substituted 4-6 membered heterocycle having 1-3 heteroatoms independently selected from N, O, or S; Y⁵ is a bond, -N(R)-, -C(O)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S; Y⁶ is a bond, or -(CH₂-CH₂-N(R))-; each of m and n is independently 1, 2, 3, 4, or 5; each of p, q, and s is independently 1, 2, or 3; and R is -H or -C₁₋₃ alkyl.

In some examples, Y¹ is -C(O)- or -C(O)-N(R)-. For instance, Y¹ is -C(O)-. In other instances, Y¹ is-C(O)-N(R)-.

In some examples, Y¹ is -C(O)-, Y² is -(CH₂-CH₂-O)ₙ-, -(O-CH₂-CH₂)ₙ-, -C₁₋₄ alkyl-, a 3-6 membered monocyclic cycloalkyl, or a 7-11 membered fused or spiro bicycloalkyl, and n is 1 or 2. For example, Y² is -(CH₂-CH₂-O)ₙ-, -(O-CH₂-CH₂)ₙ-, -CH₂-, -CH₂-CH₂-, -CH₂-CH₂-CH₂-, cyclopentyl, cyclohexyl, or a 7-11 membered spiro bicycloalkyl, and n is 1 or 2. In other examples, Y² is -(CH₂-CH₂-O)ₙ-, -(O-CH₂-CH₂)ₙ-, -CH₂-, -CH₂-CH₂-, or cyclohexyl, and n is 1.

In some of these examples, Y³ is a bond, -O-, -N(R)-, -C₁₋₄ alkyl-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S. For example, Y³ is a bond, -N(R)-, -O-, -CH₂-, -CH₂-CH₂-, -CH₂-CH₂-CH₂-, -(CH₂-CH₂-N(R))-, phenyl, 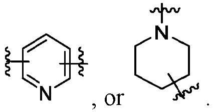 In some examples, Y³ is a bond, -N(H)-, -O-, -CH₂-, -CH₂-CH₂-, -CH₂-CH₂-CH₂-, or phenyl.

In some of these examples, Y⁴ is a bond, -C(O)-, -N(R)-, -C(O)N(R)-, -N(R)C(O)-, -C₁₋₄ alkyl-, -CH₂-CH₂-N(R)-, an optionally substituted 3-6 membered cycloalkyl, or an optionally substituted 4-6 membered heterocycle having 1-3 heteroatoms independently selected from N, O, or S. For example, Y⁴ is a bond, -C(O)-, -N(R)-, -C(O)N(R)-, -N(R)C(O)-, -CH₂-, -CH₂-CH₂-, 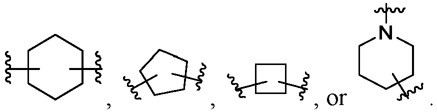 In other examples, Y⁴ is a bond, -N(H)-, -CH₂-, -CH₂-CH₂-, 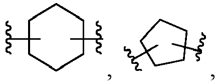 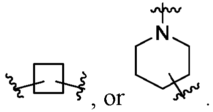 Or in other examples, Y⁴ is-N(H)-.

In some of these examples, Y⁵ is a bond, -N(R)-, -C₁₋₄ alkyl-, -(CH₂-CH₂-O)ₛ-, - (O-CH₂-CH₂)ₛ-, or a 5-6 membered heteroaryl having 1-3 heteroatoms independently selected from N or S. For example, Y⁵ is a bond, -N(R)-, -CH₂-, -CH₂-CH₂-, -CH₂-CH₂-CH₂-, -(CH₂-CH₂-O)ₛ-, -(O-CH₂-CH₂)ₛ-, or 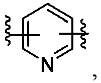 and s is 1 or 2. In some instances, s is 1. In other examples, Y⁵ is a bond, -N(H)-, or

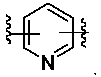

In some of these examples, Y⁶ is a bond.

In some examples, Y¹ is -C(O)-N(R)-, Y² is -(CH₂-CH₂-O)ₙ-, -(O-CH₂-CH₂)ₙ-, - C₁₋₄ alkyl-, an optionally substituted 3-6 membered monocyclic cycloalkyl, or an optionally substituted 7-11 membered fused or spiro bicycloalkyl. For example, Y² is -(CH₂-CH₂-O)-, -(O-CH₂-CH₂)-, -(CH₂-CH₂-O)₂-, -(O-CH₂-CH₂)₂-, -CH₂-, -CH₂-CH₂-, -CH₂-CH₂-CH₂-, cyclobutyl, cyclopentyl, cyclohexyl, or a 7-11 membered spiro bicycloalkyl.

In some of these examples, Y³ is a bond, -O-, -N(R)-, -C₁₋₄ alkyl-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S. For example, Y³ is a bond, -N(R)-, -O-, -CH₂-, -CH₂-CH₂-, -CH₂-CH₂-CH₂-, -(CH₂-CH₂-N(R))-, phenyl,

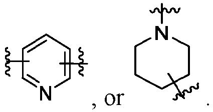

In some of these examples, Y⁴ is a bond, -C(O)-, -N(R)-, -C(O)N(R)-, -N(R)C(O)-, -C₁₋₄ alkyl-, -(CH₂-CH₂-N(R))-, an optionally substituted 3-6 membered cycloalkyl, or an optionally substituted 4-6 membered heterocycle having 1-3 heteroatoms independently selected from N, O, or S. For example, Y⁴ is a bond, -C(O)-, -N(R)-, -C(O)N(R)-, -N(R)C(O)-, -CH₂-, -CH₂-CH₂-,

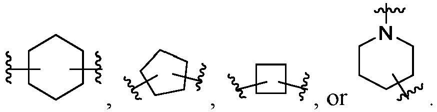

In some examples, Y⁵ is a bond, -N(R)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S. For example, Y⁵ is a bond, -N(R)-, -CH₂-, -CH₂-CH₂-, -CH₂-CH₂-CH₂-, -(CH₂-CH₂-O)ₛ-, -(O-CH₂-CH₂)ₛ-, or 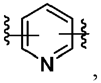 and s is 1 or 2. In some instances, s is 1.

In some of these examples, Y⁶ is a bond.

In some examples, Y¹ is -(CH₂-CH₂-O)ₘ-, -(O-CH₂-CH₂)ₘ-, or -C₁₋₆ alkyl-, and m is 1, 2, 3, 4, or 5. For example, Y¹ is -(CH₂-CH₂-O)-, -(O-CH₂-CH₂)-, -(CH₂-CH₂-O)₂-, - (O-CH₂-CH₂)₂-, -(CH₂-CH₂-O)₃-, -(O-CH₂-CH₂)₃-, -(CH₂-CH₂-O)₄-, -(O-CH₂-CH₂)₄-, -(CH₂-CH₂-O)₅-, -(O-CH₂-CH₂)₅-, or -C₁₋₆ alkyl-. In other examples, Y¹ is -(CH₂-CH₂-O)₁₋₃-, -(O-CH₂-CH₂)₁₋₃-, or -C₁₋₆ alkyl- (e.g., methylene, ethylene, propylene, butylene, and the like).

In some examples, Y² is -C(O)-, -O-, -(CH₂-CH₂-O)ₙ-, -(O-CH₂-CH₂)ₙ-, -C₁₋₄ alkyl-, an optionally substituted 3-6 membered monocyclic cycloalkyl, or an optionally substituted 7-11 membered fused or spiro bicycloalkyl. For example, Y² is an optionally substituted 3-6 membered monocyclic cycloalkyl. In some examples, Y² is 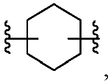 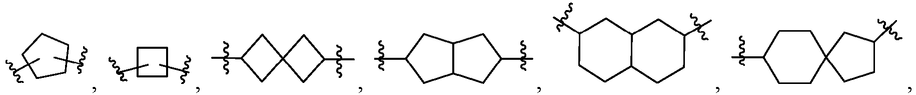 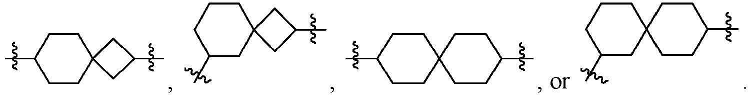 In other examples, For example, Y² is -C(O)-, -O-, -(CH₂-CH₂-O)ₙ-, or -(O-CH₂-CH₂)ₙ-, and n is 2 or 3. For instance, Y² is -C(O)- or -O-. In other instances, Y² is -C(O)-, -O-, -(CH₂-CH₂-O)₁₋₂-, -(O-CH₂-CH₂)₁₋₂-,

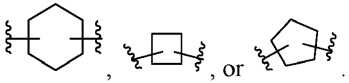

In some examples, Y³ is a bond, -O-, -N(R)-, -(CH₂-CH₂-O)ₚ-, -(O-CH₂-CH₂)ₚ-, -(CH₂-CH₂-N(R))-, phenyl, 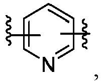 an optionally substituted 3-6 membered monocyclic cycloalkyl, or an optionally substituted 4-6 membered monocyclic heterocycle having 1-3 heteroatoms independently selected from N, O, or S. For example, Y³ is a bond, -O-, -N(R)-, -(CH₂-CH₂-O)ₚ-, or -(O-CH₂-CH₂)ₚ-, and p is 1 or 2. In other examples, Y³ is 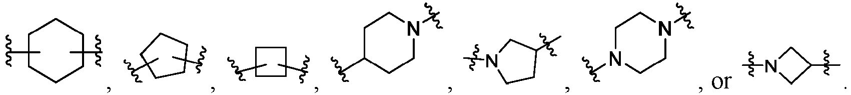 In some examples, Y³ is a bond, -O-, or -N(H)-. In other instances, Y³ is phenyl or

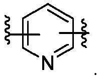

In some examples, Y⁴ is a bond, -N(R)-, -C(O)N(R)-, -N(R)C(O)-, -C₁₋₄ alkyl-, - (CH₂-CH₂-N(R))-, an optionally substituted 3-6 membered cycloalkyl, or an optionally substituted 4-6 membered heterocycle having 1-3 heteroatoms independently selected from N, O, or S. For example, Y⁴ is a bond, -N(H)-, -N(CH₃)-, -N(H)C(O)-, -CH₂-, -CH₂-CH₂-, -CH₂-CH₂-CH₂-, -(CH₂-CH₂-N(H))-,

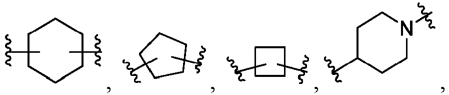

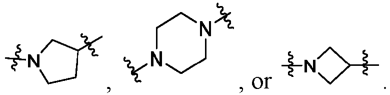

In some examples, Y⁵ is a bond, -C(O)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S. For example, Y⁵ is a bond, -C(O)-, -CH₂-, -CH₂-CH₂-, -CH₂-CH₂-CH₂-, -(CH₂-CH₂-O)ₛ-, -(O-CH₂-CH₂)ₛ-, or 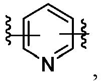 and s is 1 or 2.

In some examples, Y⁶ is a bond or -CH₂-CH₂-N(H)- or -N(H)-CH₂-CH₂-.

This disclosure also provides a compound of Formula **(I-A1)** 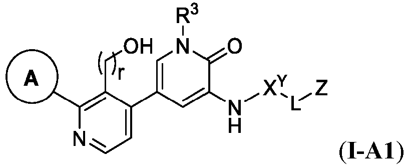 or a pharmaceutically acceptable salt thereof, wherein ring A, r, R³, X^{Y}, L and Z are as defined in any of the compounds of Formulae **(I), (I-A), (I-A2), (I-A3), (I-B1), (I-B2), (I-B3), (II), (II-A), (II-B), (III), (III-A), (III-B),** and **(III-C)** (as applicable).

In some examples, X^{Y} is 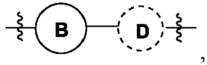 ring B is 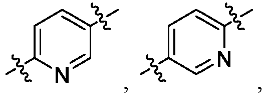 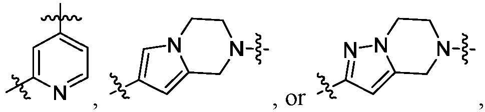 and ring D is absent.

In some examples, X^{Y} is 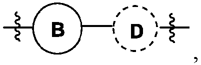 ring B is 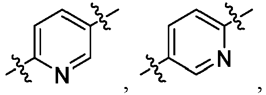 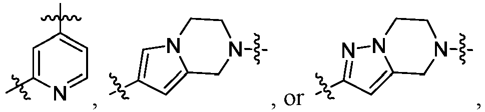 and ring D is 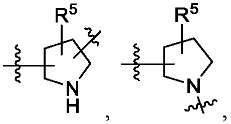 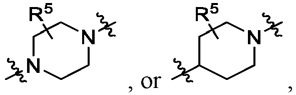 and R⁵ is -H or C₁₋₃ alkyl.

In some examples, X^{Y} is 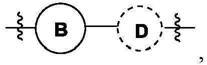 ring B is 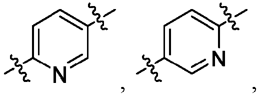 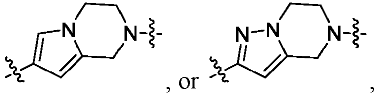 and ring D is

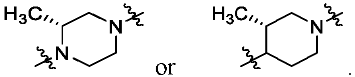

In some examples, L is -Y¹-Y²-Y³-Y⁴-Y⁵-Y⁶-, wherein Y¹ is -C(O)-, -C(O)-N(R)-, -(CH₂-CH₂-O)ₘ-, -(O-CH₂-CH₂)ₘ-, or -C₁₋₆ alkyl-; Y² is a bond, -C(O)-, -O-, -(CH₂-CH₂-O)ₙ-, -(O-CH₂-CH₂)ₙ-, -C₁₋₄ alkyl-, an optionally substituted 3-6 membered monocyclic cycloalkyl, or an optionally substituted 7-11 membered fused or spiro bicycloalkyl; Y³ is a bond, -O-, -N(R)-, -C₁₋₄ alkyl-, -(CH₂-CH₂-O)ₚ-, -(O-CH₂-CH₂)ₚ-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S; Y⁴ is a bond, -C(O)-, -N(R)-, -C(O)N(R)-, -N(R)C(O)-, -C₁₋₄ alkyl-, -(CH₂-CH₂-O)_{q}-, -(O-CH₂-CH₂)_{q}-, -(CH₂-CH₂-N(R))-, an optionally substituted 3-6 membered cycloalkyl, or an optionally substituted 4-6 membered heterocycle having 1-3 heteroatoms independently selected from N, O, or S; Y⁵ is a bond, -N(R)-, -C(O)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S; Y⁶ is a bond, or -(CH₂-CH₂-N(R))-; each of m and n is independently 1, 2, 3, 4, or 5; each of p, q, and s is independently 1, 2, or 3; and R is -H or -C₁₋₃ alkyl.

In some examples, Z is

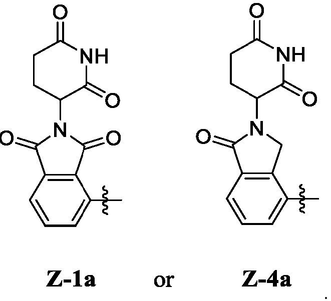

This disclosure also provides a compound of Formula **(I-A2)** 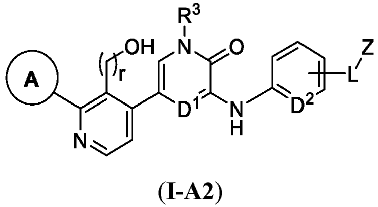 or a pharmaceutically acceptable salt thereof, wherein D¹ is =CH- or =N-; D² is =CH- or =N-; at least one of D¹ and D² is =N-; and each of ring A, r, R³, L, and Z are as defined in any of the compounds of Formulae **(I), (I-A), (I-A1), (I-A3), (I-B1), (I-B2), (I-B3), (II), (II-A), (II-B), (III), (III-A), (III-B),** and **(III-C)** (as applicable).

In some examples, D¹ is =CH- and D² is =N-. In other examples, D¹ is =N- and D² is =CH-.

In some examples, In some examples, L is -Y¹-Y²-Y³-Y⁴-Y⁵-Y⁶-, wherein Y¹ is - C(O)-, -C(O)-N(R)-, -(CH₂-CH₂-O)ₘ-, -(O-CH₂-CH₂)ₘ-, or -C₁₋₆ alkyl-; Y² is a bond, - C(O)-, -O-, -(CH₂-CH₂-O)ₙ-, -(O-CH₂-CH₂)ₙ-, -C₁₋₄ alkyl-, an optionally substituted 5-6 membered monocyclic cycloalkyl, or an optionally substituted 7-11 membered fused or spiro bicycloalkyl; Y³ is a bond, -O-, -N(R)-, -(CH₂-CH₂-O)ₚ-, -(O-CH₂-CH₂)ₚ-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S; Y⁴ is a bond, -C(O)-, -N(R)-, -C(O)N(R)-, -N(R)C(O)-, -C₁₋₄ alkyl-, -(CH₂-CH₂-O)_{q}-, -(O-CH₂-CH₂)_{q}-, -(CH₂-CH₂-N(R))-, an optionally substituted 3-6 membered cycloalkyl, or an optionally substituted 4-6 membered heterocycle having 1-3 heteroatoms independently selected from N, O, or S; Y⁵ is a bond, -N(R)-, or a 5-6 membered heteroaryl having 1-3 heteroatoms independently selected from N or S; Y⁶ is a bond, or -(CH₂-CH₂-N(R))-; each of m and n is independently 1, 2, 3, 4, or 5; each of p, q, and s is independently 1, 2, or 3; and R is -H or -C₁₋₃ alkyl.

This disclosure also provides a compound of Formula **(I-A3)** 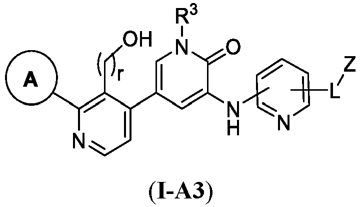 or a pharmaceutically acceptable salt thereof, wherein each of ring A, r, R³, L, and Z are as defined in any of the compounds of Formulae **(I), (I-A), (I-A1), (I-A2), (I-B1), (I-B2), (I-B3), (II), (II-A), (II-B), (III), (III-A), (III-B),** and **(III-C)** (as applicable).

This disclosure also provides a compound of Formula **(I-B1),** a compound of Formula **(I-B2),** or a compound of Formula **(I-B3):** 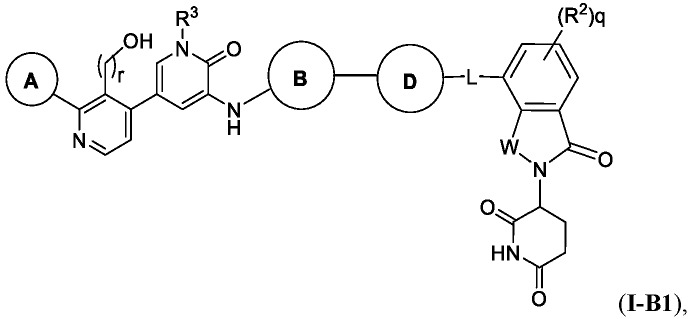 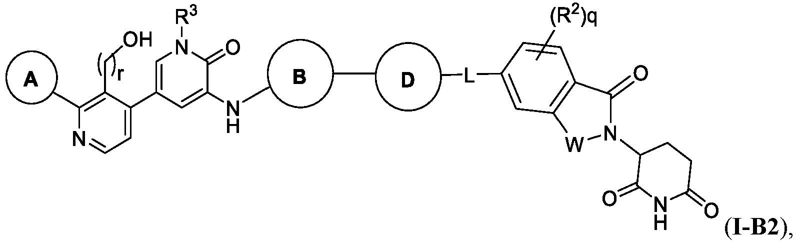 or 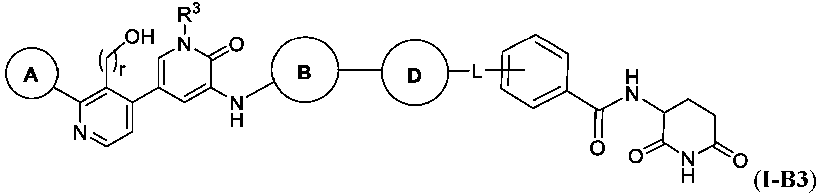 or a pharmaceutically acceptable salt thereof. For each of these compounds, ring A, r, R³, ring B and ring D are as defined in any of the compounds of Formulae **(I), (I-A), (I-A1), (I-A2), (I-B1), (I-B2), (I-B3), (II), (II-A), (II-B), (III), (III-A), (III-B),** and **(III-C)** (as applicable).

In some examples, ring B is phenyl, pyridine-yl, or pyrimidine-yl.

In some examples, ring D is an optionally substituted 4-7 membered heterocycle having 1-2 nitrogen atoms. For example, ring D is an optionally substituted 5-6 membered heterocycle having 1-2 nitrogen atoms.

In some examples, ring B is 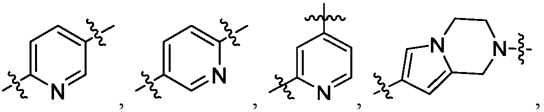 or 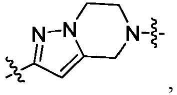 ring D is 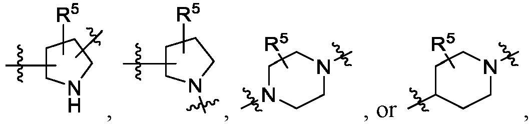 and R⁵ is -H or C₁₋₃ alkyl.

In some examples, ring B is 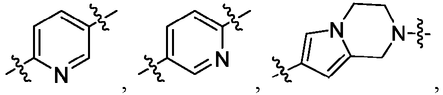 or 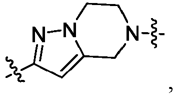 and ring D is

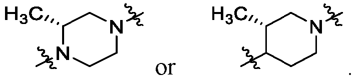

In some examples, --- is a bond, X¹ is N, X^{B} is C, and ring C is a 6 membered fully unsaturated carbocycle, optionally substituted with R¹ and R² groups. For example, ring A is 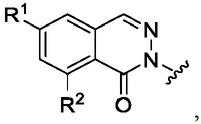 wherein each of R¹ and R² is independently halo, -H, -OH, -CN, -CF₃, -C₁₋₆ alkyl, or a 3-6 membered cycloaliphatic.

In some examples, --- is absent, X¹ is -CH₂- , X^{B} is N, and ring C is a 5-6 membered partially or fully unsaturated heterocycle having 1-2 nitrogen atoms, wherein ring C is substituted with R¹ and R² groups.

In some examples, ring A is 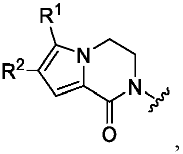 wherein each of R¹ and R² is independently halo, -H, -OH, -CN, -CF₃, -C₁₋₆ alkyl, or a 3-6 membered cycloaliphatic, or R¹ and R² together with the atoms to which they are attached form a 5-6 membered cycloalkyl, wherein the cycloalkyl is optionally substituted with 1-2 of R⁶. For example, ring A is

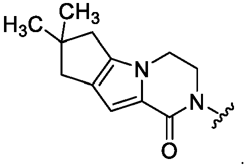

In some examples, R³ is a C₁₋₃ alkyl. For example, R³ is methyl or ethyl.

In some examples, r is 1 or 2. For example, r is 1.

In some examples, L is -Y¹-Y²-Y³-Y⁴-Y⁵-Y⁶-, wherein Y¹ is -C(O)-, -C(O)-N(R)-, -(CH₂-CH₂-O)ₘ-, -(O-CH₂-CH₂)ₘ-, or -C₁₋₆ alkyl-; Y² is a bond, -C(O)-, -O-, -(CH₂-CH₂-O)ₙ-, -(O-CH₂-CH₂)ₙ-, -C₁₋₄ alkyl-, an optionally substituted 3-6 membered monocyclic cycloalkyl, or an optionally substituted 7-11 membered fused or spiro bicycloalkyl; Y³ is a bond, -O-, -N(R)-, -C₁₋₄ alkyl-, -(CH₂-CH₂-O)ₚ-, -(O-CH₂-CH₂)ₚ-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S; Y⁴ is a bond, -C(O)-, -N(R)-, -C(O)N(R)-, -N(R)C(O)-, -C₁₋₄ alkyl-, -(CH₂-CH₂-O)_{q}-, -(O-CH₂-CH₂)_{q}-, -(CH₂-CH₂-N(R))-, an optionally substituted 3-6 membered cycloalkyl, or an optionally substituted 4-6 membered heterocycle having 1-3 heteroatoms independently selected from N, O, or S; Y⁵ is a bond, -N(R)-, -C(O)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S; Y⁶ is a bond, or -(CH₂-CH₂-N(R))-; each of m and n is independently 1, 2, 3, 4, or 5; each of p, q, and s is independently 1, 2, or 3; and R is -H or -C₁₋₃ alkyl.

In some examples, Y¹ is -C(O)- or -C(O)-N(R)-.

In some examples, Y¹ is -C(O)-, Y² is -(CH₂-CH₂-O)ₙ-, -(O-CH₂-CH₂)ₙ-, -C₁₋₄ alkyl-, a 3-6 membered monocyclic cycloalkyl, or a 7-11 membered fused or spiro bicycloalkyl, and n is 1 or 2. For example, Y² is -(CH₂-CH₂-O)ₙ-, -(O-CH₂-CH₂)ₙ-, -CH₂-, -CH₂-CH₂-, -CH₂-CH₂-CH₂-, cyclopentyl, cyclohexyl, or a 7-11 membered spiro bicycloalkyl, and n is 1 or 2.

In some examples, Y³ is a bond, -O-, -N(R)-, -C₁₋₄ alkyl-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S. For example, Y³ is a bond, -N(R)-, -O-, -CH₂-, -CH₂-CH₂-, -CH₂-CH₂-CH₂-, -(CH₂-CH₂-N(R))-, phenyl,

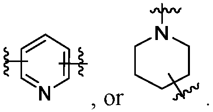

In some examples, Y⁴ is a bond, -C(O)-, -N(R)-, -C(O)N(R)-, -N(R)C(O)-, -C₁₋₄ alkyl-, -(CH₂-CH₂-N(R))-, an optionally substituted 3-6 membered cycloalkyl, or an optionally substituted 4-6 membered heterocycle having 1-3 heteroatoms independently selected from N, O, or S. For example, Y⁴ is a bond, -C(O)-, -N(R)-, -C(O)N(R)-, -N(R)C(O)-, -CH₂-, -CH₂-CH₂-,

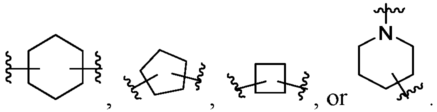

In some examples, Y⁵ is a bond, -N(R)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S. For example, Y⁵ is a bond, -N(R)-, -CH₂-, -CH₂-CH₂-, -CH₂-CH₂-CH₂-, -(CH₂-CH₂-O)ₛ-, -(O-CH₂-CH₂)ₛ-, or 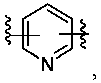 and s is 1 or 2.

In some examples, Y⁶ is a bond.

In some examples, Y¹ is -(CH₂-CH₂-O)ₘ-, -(O-CH₂-CH₂)ₘ-, or -C₁₋₆ alkyl-.

In some examples,Y² is -C(O)-, -O-, -(CH₂-CH₂-O)ₙ-, -(O-CH₂-CH₂)ₙ-, 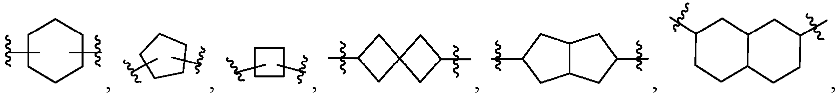 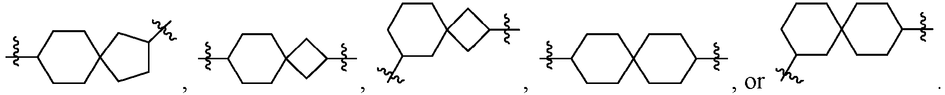 For example, Y² is -C(O)-, -O-, -(CH₂-CH₂-O)ₙ-, or -(O-CH₂-CH₂)ₙ-, and n is 2 or 3.

In some examples, Y³ is a bond, -O-, -N(R)-, -(CH₂-CH₂-O)ₚ-, -(O-CH₂-CH₂)ₚ-, -(CH₂-CH₂-N(R))-, phenyl, 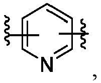 an optionally substituted 3-6 membered monocyclic cycloalkyl, or an optionally substituted 4-6 membered monocyclic heterocycle having 1-3 heteroatoms independently selected from N, O, or S. For example, Y³ is a bond, -O-, -N(R)-, -(CH₂-CH₂-O)ₚ-, or -(O-CH₂-CH₂)ₚ-, and p is 1 or 2. In other examples, Y³ is

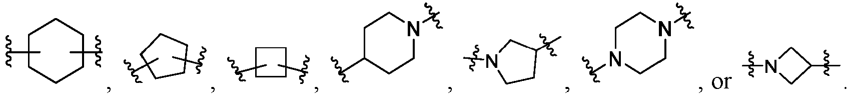

In some examples, Y⁴ is a bond, -N(R)-, -C(O)N(R)-, -N(R)C(O)-, -C₁₋₄ alkyl-, - (CH₂-CH₂-N(R))-, an optionally substituted 3-6 membered cycloalkyl, or an optionally substituted 4-6 membered heterocycle having 1-3 heteroatoms independently selected from N, O, or S. For example, Y⁴ is a bond, -N(H)-, -N(CH₃)-, -N(H)C(O)-, -CH₂-, -CH₂-CH₂-, -CH₂-CH₂-CH₂-, -(CH₂-CH₂-N(H))-,

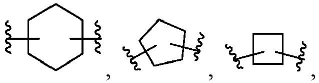

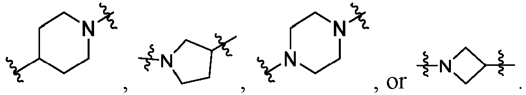

In some examples, Y⁵ is a bond, -C(O)-, -C₁₋₄ alkyl-, -(CH₂-CH₂-O)ₛ-, -(O-CH₂-CH₂)ₛ-, or a -6 membered heteroaryl having 1-3 heteroatoms independently selected from N or S. For example, Y⁵ is a bond, -C(O)-, -CH₂-, -CH₂-CH₂-, -CH₂-CH₂-CH₂-, -(CH₂-CH₂-O)ₛ-, -(O-CH₂-CH₂)ₛ-, or 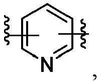 and s is 1 or 2.

In some embodiments, Y⁶ is a bond or -CH₂-CH₂-N(H)-.

This disclosure also provides a compound of Formula (II) 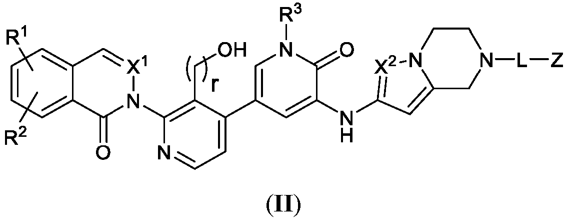 or a pharmaceutically acceptable salt thereof, wherein R¹ is C₁₋₆ alkyl, hydroxyl, halo, cyano, or a 3-6 membered cycloaliphatic; R² is C₁₋₆ alkyl, hydroxyl, halo, cyano, or a 3-6 membered cycloaliphatic; R³ is C₁₋₆ alkyl; X¹ and X² are each independently =CH- or =N-; r is 1, 2, or 3; L is a bivalent C₁₋₂₀ alkyl group, optionally substituted with C₁₋₆ alkyl, acyl, oxo, halo, or alkoxy, wherein one or more methylene units of said C₁₋₂₀ alkyl group is optionally and independently replaced by -CO-, -CS-, -CONH-, -CONHNH-, -CO₂-, -OCO-, -NHCO₂-, -O-, -NHCONH-, -OCONH-, -NHNH-, -NHCO-, -S-, -S(O)-, -S(O)₂-, -NH-, -S(O)₂NH-, -NHS(O)₂-, -NHS(O)₂NH-, bivalent mono- or bicyclic heterocycle, bivalent mono- or bicyclic cycloalkyl, bivalent mono- or bicyclic aryl, or bivalent mono- or bicyclic heteroaryl, wherein the heterocycle and heteroaryl of L has 1-3 heteroatoms independently selected from N, O, or S; and Z is

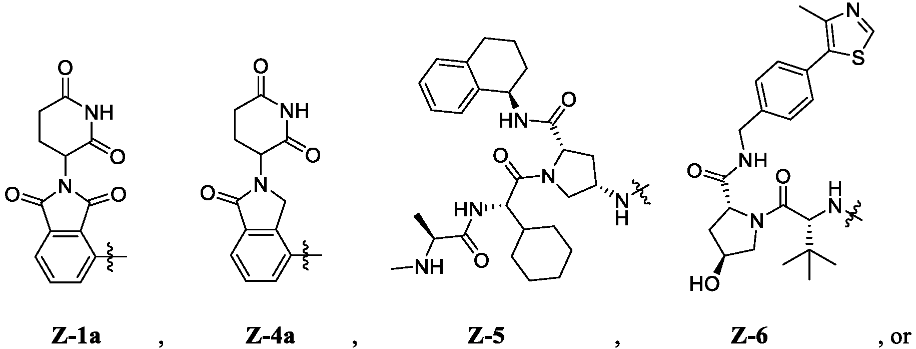

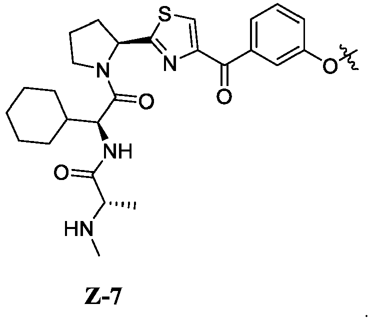

In some examples, R¹ is C₁₋₆ alkyl. For example, R¹ is methyl, ethyl, propyl, *iso-*propyl, *sec*-butyl, or *tert-*butyl*.* In other instances, R¹ is *tert*-butyl.

In some examples, R¹ is 3-6 membered cycloaliphatic. For example, R¹ is cyclopropyl, cyclopentyl, or cyclohexyl.

In some examples, R² is C₁₋₆ alkyl, hydroxyl, halo, or cyano. For example, R² is halo. In other examples, R² is -F or -Cl. For instance, R² is -F.

In some examples, R³ is methyl, ethyl, propyl, *iso*-propyl, *sec*-butyl, or *tert-*butyl*.* In some examples, R³ is methyl.

In some examples, for the compound of Formula (**II**), r is 1 or 2. For instance, r is 1.

In some examples, at least one of X¹ and X² is =N-. For example, both of X¹ and X² are =N-.

In some examples, L is a bivalent C₁₋₂₀ (e.g., C₁₋₁₅, C₁₋₁₂, C₁₋₁₀, C₁₋₇, C₁₋₆, or C₁₋₅) alkyl group, wherein one or more methylene units of the C₁₋₂₀ (e.g., C₁₋₁₅, C₁₋₁₂, C₁₋₁₀, C₁₋₇, C₁₋₆, or C₁₋₅) alkyl group is optionally and independently replaced by -CO-, -CS-, -OCO-, -O-, -NHCO-, -S-, -NH-, bivalent mono- or bicyclic heterocycle, bivalent mono- or bicyclic cycloalkyl, bivalent mono- or bicyclic aryl, or bivalent mono- or bicyclic heteroaryl, wherein each heterocycle and heteroaryl of L have 1-3 heteroatoms independently selected from N, O, or S.

In some examples, L is a bivalent C₁₋₂₀ (e.g., C₁₋₁₅, C₁₋₁₂, C₁₋₁₀, C₁₋₇, C₁₋₆, or C₁₋₅) alkyl group, wherein one or more methylene units of the C₁₋₂₀ (e.g., C₁₋₁₅, C₁₋₁₂, C₁₋₁₀, C₁₋₇, C₁₋₆, or C₁₋₅) alkyl group is optionally and independently replaced by -CO-, -O-, -NH-, bivalent mono- or bicyclic heterocycloalkyl having 1-3 heteroatoms independently selected from N, O, or S, or bivalent mono- or bicyclic cycloalkyl.

In some examples, L is a bivalent C₁₋₁₀ alkyl group, wherein one or more methylene units of the C₁₋₁₀ alkyl group is optionally and independently replaced by -CO-, -OCO-, -O-, -NHCO-, -NH-, bivalent mono- or bicyclic heterocycle, bivalent mono- or bicyclic cycloalkyl, bivalent mono- or bicyclic aryl, or bivalent mono- or bicyclic heteroaryl, wherein the heterocycle and heteroaryl of L have 1-3 heteroatoms independently selected from N, O, or S.

In some examples, L has at least one bivalent heterocycle selected from piperazine, piperidine, dioxane, pyran, morpholine, pyrrolidine, and tetrahydrofuran.

In some examples, L has at least one bivalent piperazine.

In some examples, L has at least one bivalent cycloalkyl selected from cyclopropyl, cyclobutyl, cyclopentyl, cyclohexyl, and cycloheptyl.

In some examples, L has at least one bivalent cyclohexyl.

In some examples, L is -Y¹-Y²-Y³-Y⁴-Y⁵-, wherein Y¹ is -C(O)- or -C₁₋₆ alkyl-; Y² is a bond, -O-, -(CH₂-CH₂-O)ₘ-, -(O-CH₂-CH₂)ₘ-, -C₁₋₄ alkyl-, 3-6 membered cycloalkyl; Y³ a bond, -O-, -N(R)-, -C₁₋₄ alkyl-, -(CH₂-CH₂-O)ₚ-, -(O-CH₂-CH₂)ₚ-, -(CH₂-CH₂-N(R))-, phenyl, 3-6 membered cycloalkyl, or 4-6 membered heterocycle having 1-3 heteroatoms independently selected from N, O, or S; Y⁴ is a bond, -C(O)-, -C(O)N(R)-, -C(O)N(R)-, -C₁₋₄ alkyl-, -(CH₂-CH₂-O)_{q}-, -(O-CH₂-CH₂)_{q}-, -(CH₂-CH₂-N(R))-, 3-6 membered cycloalkyl, or 4-6 membered heterocycle having 1-3 heteroatoms independently selected from N, O, or S; Y⁵ is a bond, -N(R)-, -C(O)-, -C₁₋₄ alkyl-, or a 5-6 membered heteroaryl having 1-3 heteroatoms independently selected from N, O or S; m is 1, 2, 3, 4, or 5; p is 1, 2, or 3; q is 1, 2, or 3; and R is -H or -C₁₋₃ alkyl (e.g., methyl, ethyl, or propyl).

In some examples, Y¹ is -C(O)- or -C₄₋₆ alkyl-.

In some examples, Y² is -(CH₂-CH₂-O)ₘ-, -(O-CH₂-CH₂)ₘ-, or -C₁₋₄ alkyl-.

In some examples, Y³ a bond, -O-, -C₁₋₄ alkyl-, -(CH₂-CH₂-N(R))-,

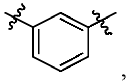

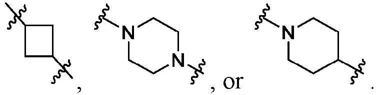

In some examples, Y⁴ is a bond, -C(O)-, -C(O)N(R)-, -C(O)N(R)-, or -C₁₋₄ alkyl-.

In some examples, Y⁵ is a bond, -N(R)-, -C(O)-, -C₁₋₄ alkyl-, or

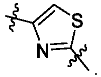

This disclosure also provides a compound of Formula **(II-A)** 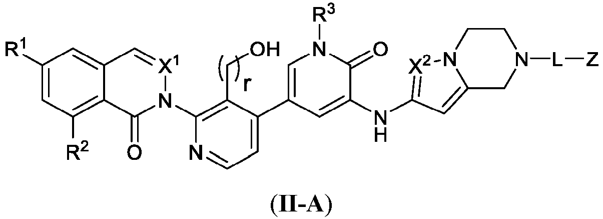 or a pharmaceutically acceptable salt thereof, wherein R¹, R², X¹, r, R³, X², L and Z are as defined in any of the compounds of Formulae **(I), (I-A), (I-A1), (I-A2), (I-B1), (I-B2), (I-B3), (II), (II-B), (III), (III-A), (III-B),** and **(III-C)** (as applicable).

In some examples, R¹ is C₁₋₆ alkyl. For example, R¹ is methyl, ethyl, propyl, *iso-*propyl, *sec*-butyl, or *tert-*butyl*.* In other examples, R¹ is *tert*-butyl.

In some examples, R¹ is 3-6 membered cycloaliphatic. For example, R¹ is cyclopropyl, cyclopentyl, or cyclohexyl.

In some examples, R² is halo. For example, R² is -F.

In some examples, R³ is methyl, ethyl, propyl, *iso*-propyl, *sec*-butyl, or *tert-butyl.* For example, R³ is methyl.

In some examples, n is 1.

In some examples, at least one of X¹ and X² is =N-. For example, both of X¹ and X² are =N-.

In some examples, L is a bivalent C₁₋₂₀ alkyl group, wherein one or more methylene units of the C₁₋₂₀ alkyl group is optionally and independently replaced by -CO-, -CS-, -OCO-, -O-, -NHCO-, -S-, -NH-, bivalent mono- or bicyclic heterocycle, bivalent mono- or bicyclic cycloalkyl, bivalent mono- or bicyclic aryl, or bivalent mono- or bicyclic heteroaryl, wherein the heterocycle and heteroaryl have 1-3 heteroatoms independently selected from N, O, or S. For example, L is a bivalent C₁₋₂₀ alkyl group, wherein one or more methylene units of the C₁₋₂₀ alkyl group is optionally and independently replaced by -CO-, -O-, -NH-, bivalent mono- or bicyclic heterocycloalkyl having 1-3 heteroatoms independently selected from N, O, or S, or bivalent mono- or bicyclic cycloalkyl. In other examples, L is a bivalent C₁₋₁₀ alkyl group, wherein one or more methylene units of the C₁₋₁₀ alkyl group is optionally and independently replaced by -CO-, -OCO-, -O-, -NHCO-, -NH-, bivalent mono- or bicyclic heterocycle, bivalent mono- or bicyclic cycloalkyl, bivalent mono- or bicyclic aryl, or bivalent mono- or bicyclic heteroaryl, wherein the heterocycle and heteroaryl have 1-3 heteroatoms independently selected from N, O, or S.

In some examples, the bivalent heterocycle of L is selected from piperazine, piperidine, dioxane, pyran, morpholine, pyrrolidine, and tetrahydrofuran. For example, the bivalent heterocycloalkyl is piperazine.

In some examples, the bivalent cycloalkyl is selected from cyclopropyl, cyclobutyl, cyclopentyl, cyclohexyl, and cycloheptyl. For example, the bivalent cycloalkyl is cyclohexyl.

In some examples, L is -Y¹-Y²-Y³-Y⁴-Y⁵-, wherein Y¹ is -C(O)- or -C₁₋₆ alkyl-; Y² is a bond, -O-, -(CH₂-CH₂-O)ₙ-, -(O-CH₂-CH₂)ₙ-, -C₁₋₄ alkyl-, 3-6 membered cycloalkyl; Y³ is a bond, -O-, -N(R)-, -C₁₋₄ alkyl-, -(CH₂-CH₂-O)ₚ-, -(O-CH₂-CH₂)ₚ-, -(CH₂-CH₂-N(R))-, phenyl, 3-6 membered cycloalkyl, or 4-6 membered heterocycle having 1-3 heteroatoms independently selected from N, O, or S; Y⁴ is a bond, -C(O)-, -C(O)N(R)-, -C(O)N(R)-, -C₁₋₄ alkyl-, -(CH₂-CH₂-O)_{q}-, -(O-CH₂-CH₂)_{q}-, -(CH₂-CH₂-N(R))-, 3-6 membered cycloalkyl, or 4-6 membered heterocycle having 1-3 heteroatoms independently selected from N, O, or S; Y⁵ is a bond, -N(R)-, -C(O)-, -C₁₋₄ alkyl-, or a 5-6 membered heteroaryl having 1-3 heteroatoms independently selected from N, O or S; n is 1, 2, 3, 4, or 5; p is 1, 2, or 3; q is 1, 2, or 3; and R is -H or -C₁₋₃ alkyl.

In some examples, Y¹ is -C(O)- or -C₄₋₆ alkyl-.

In some examples, Y² is -(CH₂-CH₂-O)ₙ-, -(O-CH₂-CH₂)ₙ-, or -C₁₋₄ alkyl-.

In some examples, Y³ is a bond, -O-, -C₁₋₄ alkyl-, -(CH₂-CH₂-N(R))-,

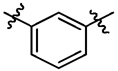

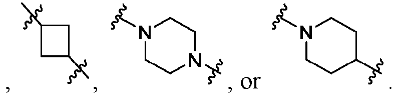

In some examples, Y⁴ is a bond, -C(O)-, -C(O)N(R)-, -C(O)N(R)-, or -C₁₋₄ alkyl-

In some examples, Y⁵ is a bond, -N(R)-, -C(O)-, -C₁₋₄ alkyl-, or

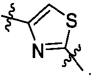

This disclosure also provides a compound of Formula **(II-B)** 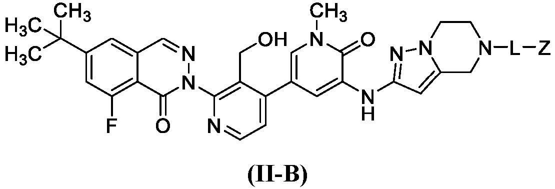 or a pharmaceutically acceptable salt thereof, wherein L and Z are as defined in any of the compounds of Formulae **(I), (I-A), (I-A1), (I-A2), (I-B1), (I-B2), (I-B3), (II), (II-A), (III), (III-A), (III-B),** and **(III-C)** (as applicable).

In some examples, L is selected from methylene, ethylene, n-propylene, n-butylene, n-pentylene, n-hexylene, 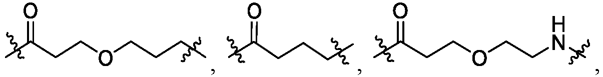 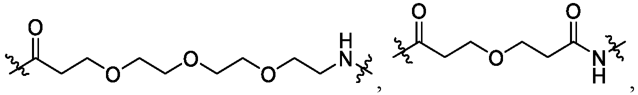 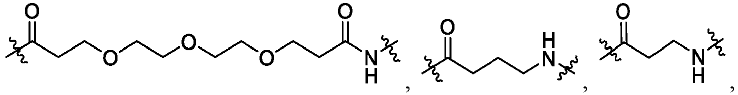 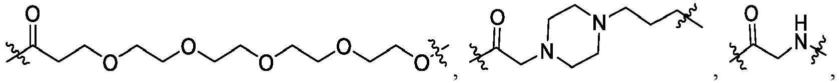 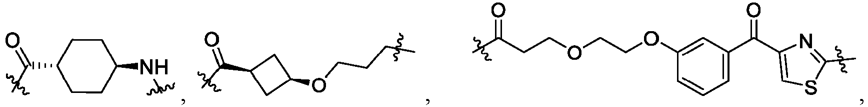 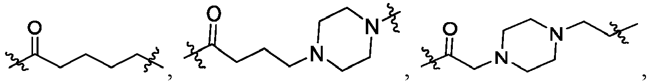 or

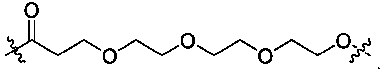

This disclosure also provides a compound of Formula **(III)** 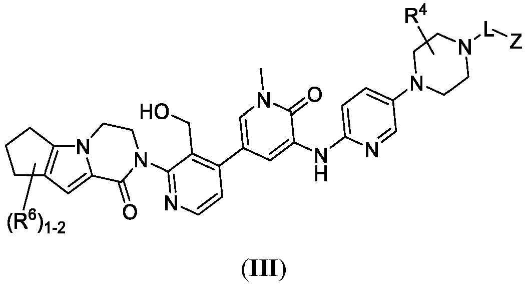 or pharmaceutically acceptable salt thereof, wherein each of R⁶, R⁴, L and Z are as defined in any of the compounds of Formulae **(I), (I-A), (I-A1), (I-A2), (I-B1), (I-B2), (I-B3), (II), (II-A), (II-B), (III-A), (III-B),** and **(III-C)** (as applicable).

In some examples, each R⁶ is independently -H or C₁₋₃ alkyl.

In some examples, R⁴ is independently -H or C₁₋₃ alkyl.

This disclosure also provides a compound of Formula **(III-A)** 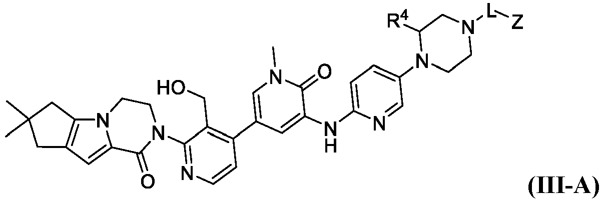 or a pharmaceutically acceptable salt thereof, wherein R⁴ is -H or -C₁₋₃ alkyl; L is -Y¹-Y²-Y³-Y⁴-Y⁵-Y⁶-; Y¹ is -C(O)-, -C(O)-N(R)-, -(CH₂-CH₂-O)ₘ-, -(O-CH₂-CH₂)ₘ-, or -C₁₋₄ alkyl-; Y² is a bond, -O-, -C₁₋₄ alkyl-, -(O-CH₂-CH₂)ₙ-, -(CH₂-CH₂-O)ₙ-, -(CH₂-CH₂-N(R))-, or 3-6 membered cycloalkyl; Y³ is a bond, -O-, -N(R)-, -C₁₋₄ alkyl-, -(CH₂-CH₂-O)ₚ-, -(O-CH₂-CH₂)ₚ-, -(CH₂-CH₂-N(R))-, 3-6 membered cycloalkyl, or 4-6 membered heterocycloalkyl having 1-3 heteroatoms independently selected from N, O, or S; Y⁴ is a bond, -N(R)-, -C₁₋₄ alkyl-, -(CH₂-CH₂-O)_{q}-, -(O-CH₂-CH₂)_{q}-, -(CH₂-CH₂-N(R))-, 3-6 membered cycloalkyl, or 4-6 membered heterocycloalkyl having 1-3 heteroatoms independently selected from N, O, or S; Y⁵ is a bond, -N(R)-, -(CH₂-CH₂-O)ₛ-, or -(O-CH₂-CH₂)ₛ-; Y⁶ is a bond or -(CH₂-CH₂-N(R))-; each R is independently-H or -C₁₋₃ alkyl; and each of m, p, q, and s is independently 1, 2, or 3; Z is 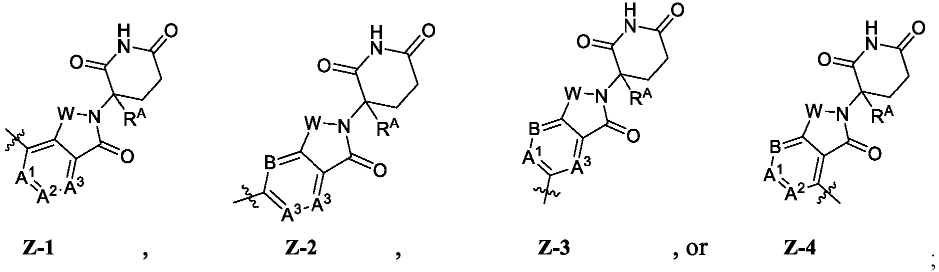 each of A¹, A², and A³ is independently -C(R^{B})= or -N=; B is -C(R^{C})= or -N=; W is -C(R^{D})₂- or -C(O)-; R^{A} is -H, -CH₃, or -F; each R^{B} is independently-H, halo, -OH, -C₁₋₄ alkyl, or -C₁₋₄ alkoxy; R^{C} is -H, halo, or C₁₋₄ alkyl; and each R^{D} is independently-H or -C₁₋₃ alkyl; provided that when each of Y³, Y⁴, Y⁵, and Y⁶ are a bond, then Y² is not a bond.

The compound or pharmaceutically acceptable salt of any one of claims 82-85, wherein Z is

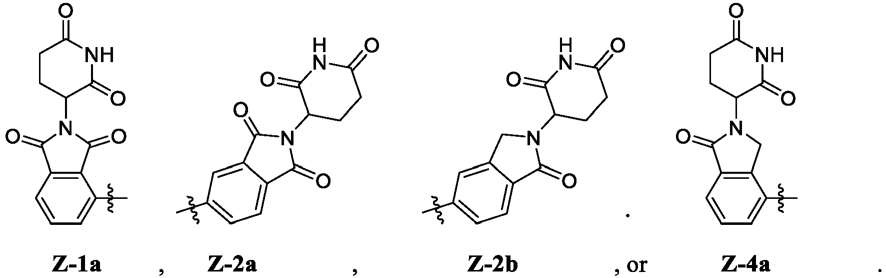

This disclosure also provides a compound of Formula **(III-B)** 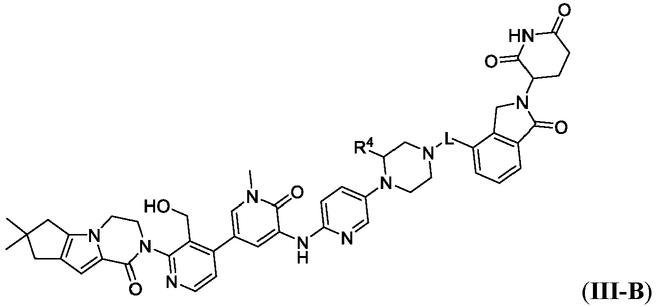 or a pharmaceutically acceptable salt thereof, wherein R⁴ and L are as defined in any of the compounds of Formulae **(I), (I-A), (I-A1), (I-A2), (I-B1), (I-B2), (I-B3), (II), (II-A), (II-B), (III),** (III-**A**), and **(III-C)** (as applicable).

In some examples, Y¹ is -C(O)- or -C(O)-N(R)-.

In some examples, Y¹ is -C(O)-, and Y² is -(O-CH₂-CH₂)ₙ-, -(CH₂-CH₂-O)ₙ-, or - C₁₋₄ alkyl-.

In some examples, Y² is -(O-CH₂-CH₂)ₙ- or -(CH₂-CH₂-O)ₙ- and n is 1.

In some examples, Y³ is -C₁₋₄ alkyl- or -(CH₂-CH₂-N(R))-, and at least one of Y⁴, Y⁵, and Y⁶ is a bond.

In some examples, Y³ is methylene, ethylene, or propylene, and each of Y⁴, Y⁵, and Y⁶ is a bond.

In some examples, Y³ is -(CH₂-CH₂-N(R))-, and each of Y⁴, Y⁵, and Y⁶ is a bond.

In some examples, L is -C(O)-(CH₂-CH₂-O)-(CH₂)₃- or -C(O)-(CH₂-CH₂-O)-(CH₂-CH₂-N(R))-.

In some examples, R⁴ is methyl.

In some examples, Y¹ is -C(O)-N(R)- and Y² is -(O-CH₂-CH₂)ₙ-, -(CH₂-CH₂-O)ₙ-, or -C₁₋₄ alkyl-.

In some examples, Y² is -(O-CH₂-CH₂)ₙ- or -(CH₂-CH₂-O)ₙ-, wherein n is 3.

In some examples, Y³ is -(O-CH₂-CH₂)ₚ-, -(CH₂-CH₂-O)ₚ-, or -C₁₋₄ alkyl-, wherein p is 1 or 2.

In some examples, Y³ is methylene, ethylene, or propylene, and Y⁴ is a bond.

In some examples, Y³ is -(O-CH₂-CH₂)ₚ- or -(CH₂-CH₂-O)ₚ-, and Y⁴ is a bond or - C₁₋₄ alkyl-.

In some examples, Y⁴ is methylene, ethylene, or propylene, and each of Y⁵ and Y⁶ is a bond.

In some examples, Y¹ is -C(O)-, Y² is 3-6 membered cycloalkyl or -C₁₋₄ alkyl-, and Y³ is -(O-CH₂-CH₂)ₚ-, -(CH₂-CH₂-O)ₚ-, 3-6 membered cycloalkyl, or 4-6 membered heterocycloalkyl having 1-3 heteroatoms independently selected from N, O, or S.

In some examples, Y² is methylene, 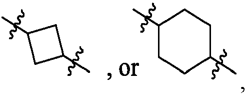 and Y³ is -(O-CH₂-CH₂)ₚ-, -(CH₂-CH₂-O)ₚ-, -N(R)-, 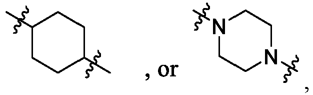 wherein p is 1.

In some examples, Y² is methylene and Y³ is

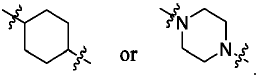

In some examples, L is selected from 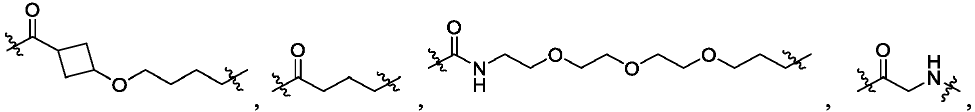 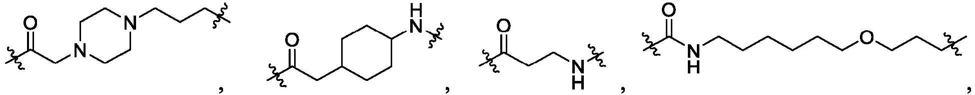 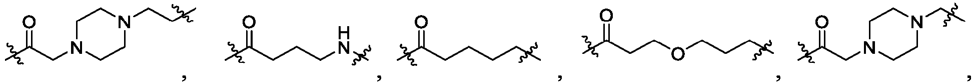 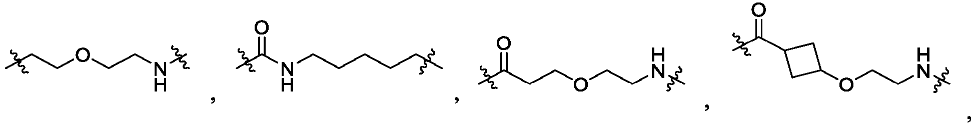 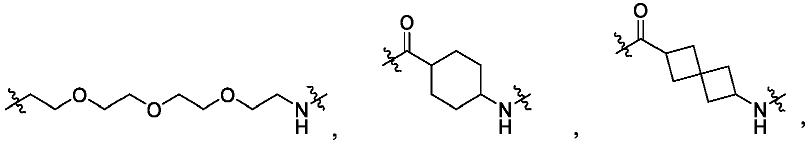 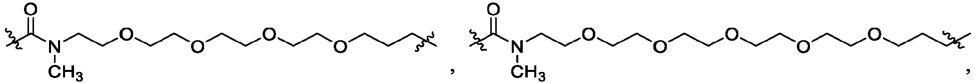 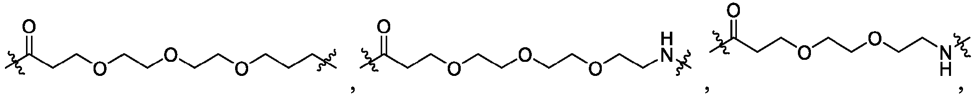 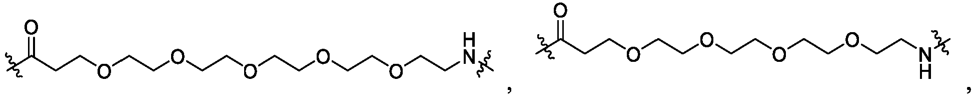 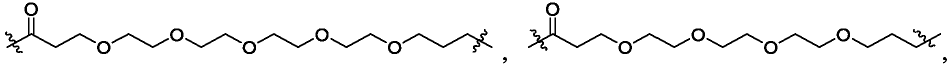 or

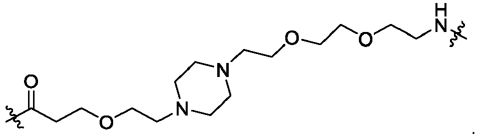

This disclosure also provides a compound of Formula **(III-C)** 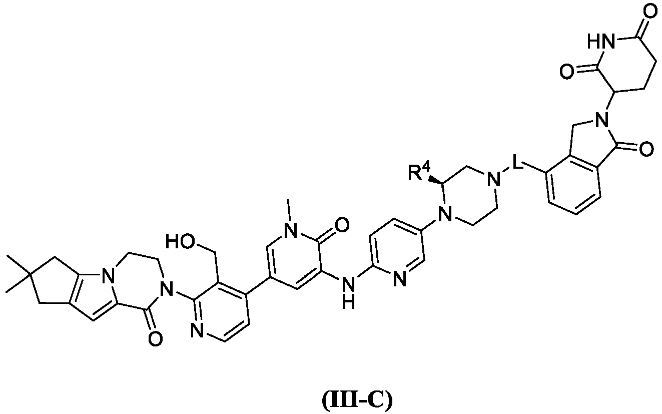 or a pharmaceutically acceptable salt thereof, wherein L and R4 are as defined in any of the compounds of Formulae **(I), (I-A), (I-A1), (I-A2), (I-B1), (I-B2), (I-B3), (II), (II-A), (II-B), (III), (III-A),** and **(III-B)** (as applicable).

In some examples, L is -Y¹-Y²-Y³-Y⁴-Y⁵-Y⁶-, wherein Y¹ is -C(O)-, -C(O)-N(R)-, -(CH₂-CH₂-O)ₘ-, -(O-CH₂-CH₂)ₘ-, or -C₁₋₆ alkyl-; Y² is a bond, -C(O)-, -O-, -(CH₂-CH₂-O)ₙ-, -(O-CH₂-CH₂)ₙ-, -C₁₋₄ alkyl-, an optionally substituted 3-6 membered monocyclic cycloalkyl, or an optionally substituted 7-11 membered fused or spiro bicycloalkyl; Y³ is a bond, -O-, -N(R)-, -C₁₋₄ alkyl-, -(CH₂-CH₂-O)ₚ-, -(O-CH₂-CH₂)ₚ-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S; Y⁴ is a bond, -C(O)-, -N(R)-, -C(O)N(R)-, -N(R)C(O)-, -C₁₋₄ alkyl-, -(CH₂-CH₂-O)_{q}-, -(O-CH₂-CH₂)_{q}-, -(CH₂-CH₂-N(R))-, an optionally substituted 3-6 membered cycloalkyl, or an optionally substituted 4-6 membered heterocycle having 1-3 heteroatoms independently selected from N, O, or S; Y⁵ is a bond, -N(R)-, -C(O)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S; Y⁶ is a bond, or -(CH₂-CH₂-N(R))-; each of m and n is independently 1, 2, 3, 4, or 5; each of p, q, and s is independently 1, 2, or 3; and R is -H or -C₁₋₃ alkyl.

In some examples, Y¹ is -C(O)- or -C(O)-N(R)-.

In some examples, Y¹ is -C(O)-, Y² is -(CH₂-CH₂-O)ₙ-, -(O-CH₂-CH₂)ₙ-, -C₁₋₄ alkyl-, a 3-6 membered monocyclic cycloalkyl, or a 7-11 membered fused or spiro bicycloalkyl, and n is 1 or 2. For example, Y² is -(CH₂-CH₂-O)ₙ-, -(O-CH₂-CH₂)ₙ-, -CH₂-, -CH₂-CH₂-, -CH₂-CH₂-CH₂-, cyclopentyl, cyclohexyl, or a 7-11 membered spiro bicycloalkyl, and n is 1 or 2.

In some examples, Y³ is a bond, -O-, -N(R)-, -C₁₋₄ alkyl-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S. For example, Y³ is a bond, -N(R)-, -O-, -CH₂-, -CH₂-CH₂-, -CH₂-CH₂-CH₂-, -(CH₂-CH₂-N(R))-, phenyl,

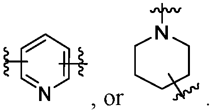

In some examples, Y⁴ is a bond, -C(O)-, -N(R)-, -C(O)N(R)-, -N(R)C(O)-, -C₁₋₄ alkyl-, -(CH₂-CH₂-N(R))-, an optionally substituted 3-6 membered cycloalkyl, or an optionally substituted 4-6 membered heterocycle having 1-3 heteroatoms independently selected from N, O, or S. For example, Y⁴ is a bond, -C(O)-, -N(R)-, -C(O)N(R)-, -N(R)C(O)-, -CH₂-, -CH₂-CH₂-,

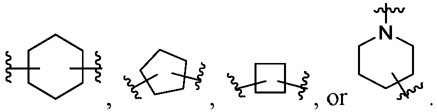

In some examples, Y⁵ is a bond, -N(R)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S. For example, Y⁵ is a bond, -N(R)-, -CH₂-, -CH₂-CH₂-, -CH₂-CH₂-CH₂-, -(CH₂-CH₂-O)ₛ-, -(O-CH₂-CH₂)ₛ-, or 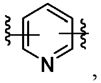 and s is 1 or 2. In some instances, s is 1.

In some examples, Y⁶ is a bond.

In some examples, Y¹ is -(CH₂-CH₂-O)ₘ-, -(O-CH₂-CH₂)ₘ-, or -C₁₋₆ alkyl-.

In some examples, Y² is -C(O)-, -O-, -(CH₂-CH₂-O)ₙ-, -(O-CH₂-CH₂)ₙ-, 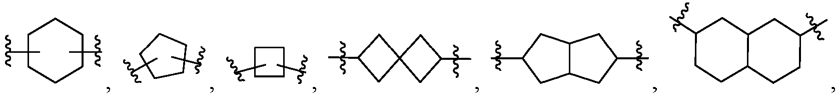 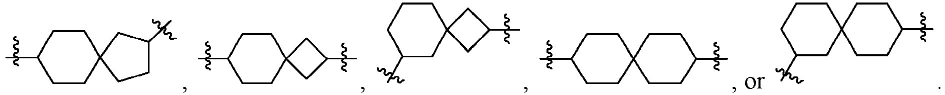 For example, Y² is -C(O)-, -O-, -(CH₂-CH₂-O)ₙ-, or -(O-CH₂-CH₂)ₙ-, and n is 2 or 3.

In some examples, Y³ is a bond, -O-, -N(R)-, -(CH₂-CH₂-O)ₚ-, -(O-CH₂-CH₂)ₚ-, -(CH₂-CH₂-N(R))-, phenyl, 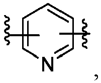 an optionally substituted 3-6 membered monocyclic cycloalkyl, or an optionally substituted 4-6 membered monocyclic heterocycle having 1-3 heteroatoms independently selected from N, O, or S. For example, Y³ is a bond, -O-, -N(R)-, -(CH₂-CH₂-O)ₚ-, or -(O-CH₂-CH₂)ₚ-, and p is 1 or 2. In other examples, Y³ is

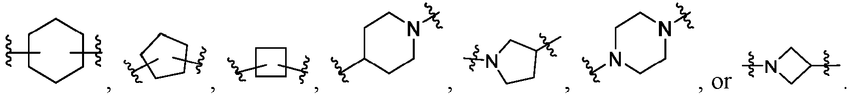

In some examples, Y⁴ is a bond, -N(R)-, -C(O)N(R)-, -N(R)C(O)-, -C₁₋₄ alkyl-, - (CH₂-CH₂-N(R))-, an optionally substituted 3-6 membered cycloalkyl, or an optionally substituted 4-6 membered heterocycle having 1-3 heteroatoms independently selected from N, O, or S. For example, Y⁴ is a bond, -N(H)-, -N(CH₃)-, -N(H)C(O)-, -CH₂-, -CH₂-CH₂-, -CH₂-CH₂-CH₂-, -(CH₂-CH₂-N(H))-,

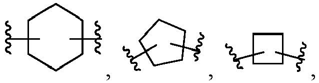

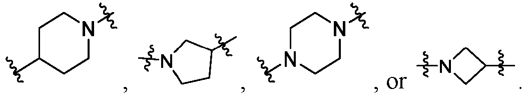

In some examples, Y⁵ is a bond, -C(O)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S. For example, Y⁵ is a bond, -C(O)-, -CH₂-, -CH₂-CH₂-, -CH₂-CH₂-CH₂-, -(CH₂-CH₂-O)ₛ-, -(O-CH₂-CH₂)ₛ-, or 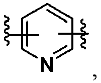 and s is 1 or 2.
In some examples, Y⁶ is a bond or -CH₂-CH₂-N(H)- or -N(H)-CH₂-CH₂-.

### B. General Synthetic Schemes

The present disclosure provides a method of synthesizing a compound of Formula **(I)** 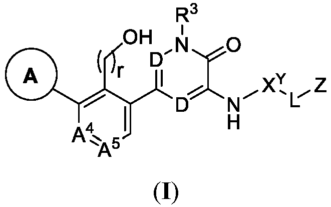 or a pharmaceutically acceptable salt thereof wherein ring A, X^{Y}, L, D, Z, A⁴, A⁵, R³ and r are defined herein, comprising the steps of:
(i) reacting a compound of Formula **(S1),** 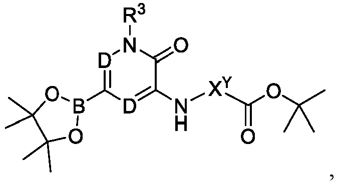 with a compound of Formula **(S2),** 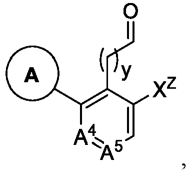 in the presence of a palladium catalyst and a base to generate the compound of Formula (S3), 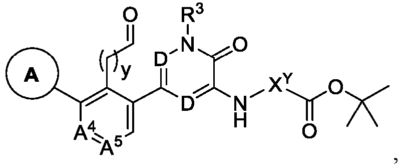 wherein X^{Z} is-Cl or -I, and y is 0, 1, or 2; and
(ii) reacting the compound of Formula (S3) with a reducing agent (e.g., NaBH₄) to generate a compound of Formula **(S4),** 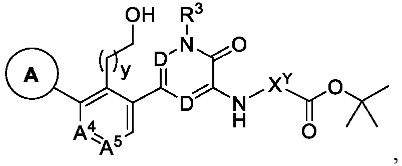 deprotecting the compound of Formula **(S4)** (e.g., treating with an acid) to generate a compound of Formula **(S5),** 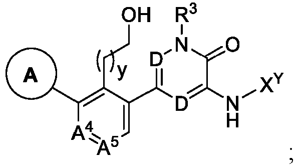 and
(iiia) reacting the compound of Formula **(S5)** with HOOC-Y²-Y³-Y⁴-Y⁵-Z in the presence of a coupling reagent and a base to generate a compound of Formula **(I);** or
(iiib) reacting the compound of Formula **(S5)** with HOC-Y²-Y³-Y⁴-Y⁵-Z in the presence of a reducing agent to generate a compound of Formula **(I);** or
(iiic) reacting the compound of Formula **(S5)** with LG-(C₁₋₄ alkyl)-Y²-Y³-Y⁴-Y⁵-Y⁶-Z, wherein LG is a leaving group (e.g., Cl, Br, I, and alkyl sulfonate groups such as methane sulfonate and toluene sulfonate) under nucleophilic substitution conditions to generate a compound of Formula **(I);** or
(iiid) reacting the compound of Formula **(S5)** with 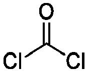 (phosgene or a phosgene equivalent) to generate an intermediate of Formula **(S6),** 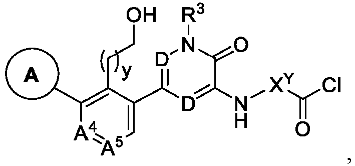 and reacting the Intermediate of Formula **(S6)** with H₂N-Y²-Y³-Y⁴-Y⁵-Y⁶-Z to generate a compound of Formula **(I).**

Bifunctional compounds of the present disclosure can be generated according to the following synthetic schemes. In these schemes, y is 0, 1, or 2, and R¹, R², R³, X^{Z}, X¹, and X² are as defined herein.

### Scheme 1: Synthesis of intermediate (AA-3).

The synthesis of intermediate **(AA-3)** can be accomplished via a nucleophilic substitution reaction with bicyclic heteroaryl intermediate **(AA-1)** and the 2-fluoro-4-iodo-pyridine-3-aldehyde **(AA-2)** in the presence of a strong base (e.g., NaH).

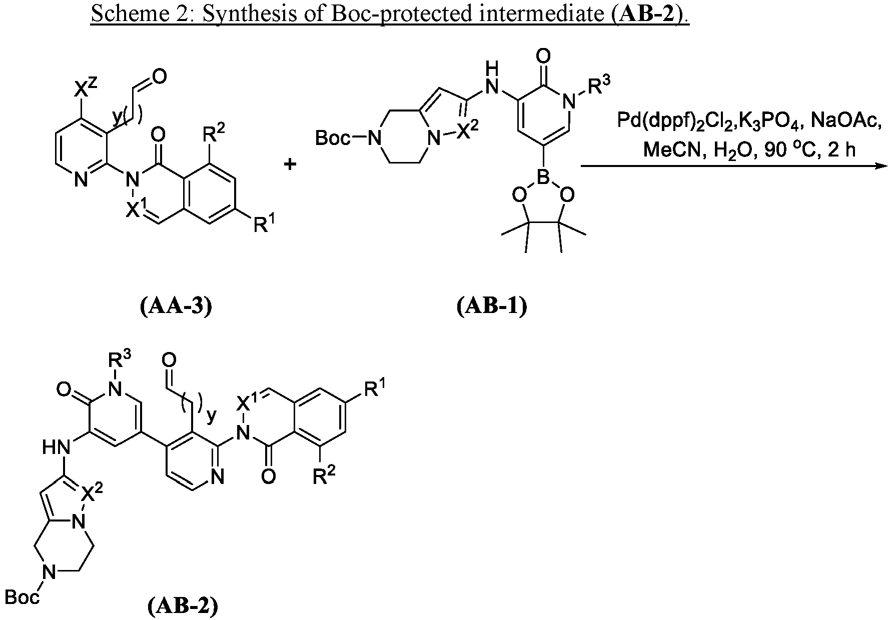

Intermediate **(AA-3)** undergoes a palladium catalyzed cross-coupling reaction with boronate ester **(AB-1)** to generate intermediate **(AB-2).**

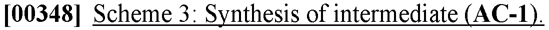

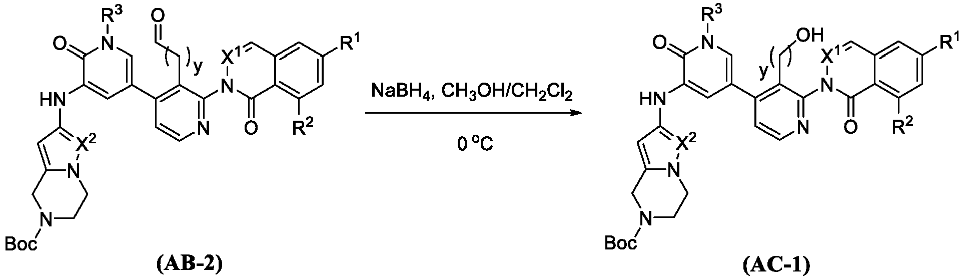

The treatment of intermediate **(AB-2)** with a reducing agent (e.g., NaBH₄) generates the Boc-protected hydroxyalkyl intermediate **(AC-1).**

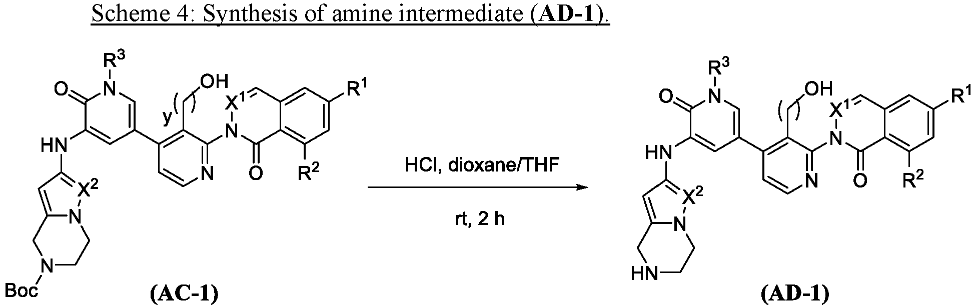

Deprotection of Boc-protected hydroxyalkyl intermediate **(AC-1)** generates the amine intermediate **(AD-1).**

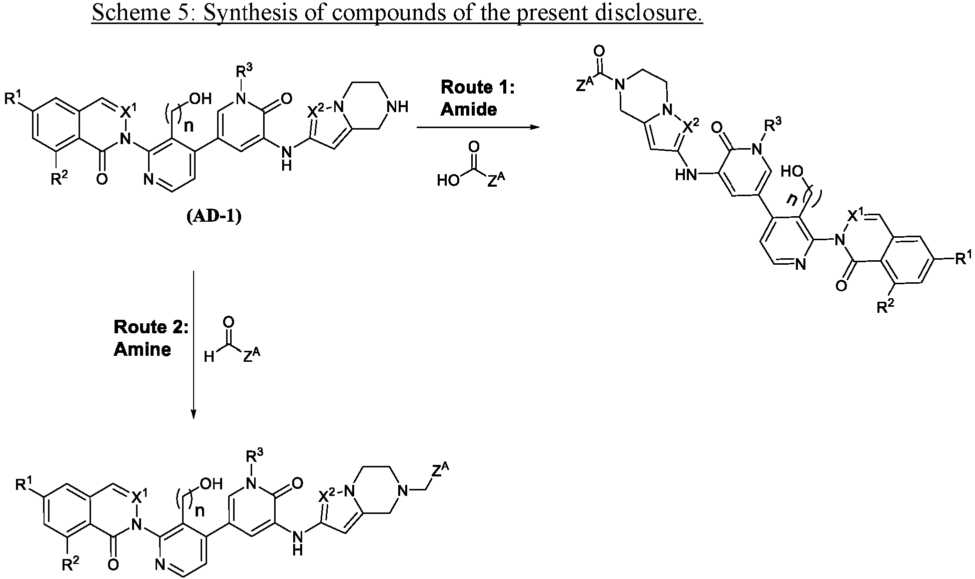

The synthesis of compounds of the present disclosure can be accomplished using either of two general synthetic strategies provided in Scheme 5. Intermediate **(AD-1)** can be coupled to an acid via any commonly known peptide coupling strategy (route 1), for example, the use of the coupling agent, HATU, with a base, such as DIEA, in a solvent, such as DMF. The product in route 1 is an amide, wherein Z^{A} is a chemical moiety that includes the Z group and optionally includes at least a portion of the L group, for instance, any of Y¹, Y², Y³, Y⁴, and Y⁵, wherein the definitions of Z, L, Y¹, Y², Y³, Y⁴, and Y⁵ are each independently described herein, for example in the compounds of Formula **(I).** Route 2 in Scheme 5 produces an amine by reductive amination and direct nucleophilic substitution, respectively. The reductive amination of route 2 can be accomplished by reacting intermediate (AD-1) with an aldehyde, in the presence of a reducing agent, such as NaB(OAc)₃, to convert the resulting imine into the amine moiety.

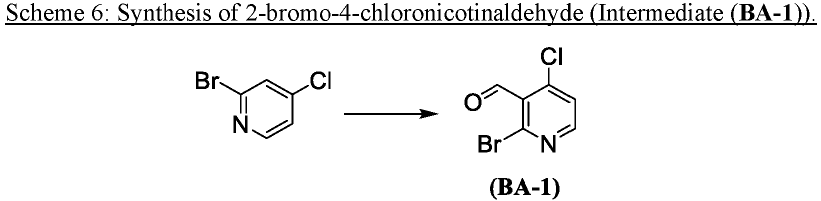

The synthesis of 2-bromo-4-chloronicotinaldehyde (BA-1) can be accomplished according to the general procedure provided by Scheme 6. Treatment of 2-bromo-4-chloropyridine with a strong base, such as lithium diisopropylamide (LDA), followed by treatment with dimethylformamide (DMF) provides 2-bromo-4-chloronicotinaldehyde.

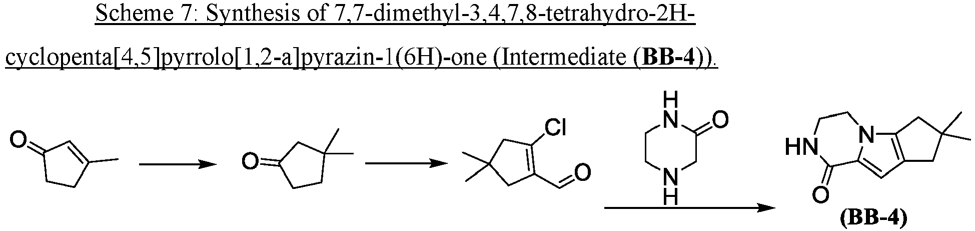

The synthesis of 7,7-dimethyl-3,4,7,8-tetrahydro-2H-cyclopenta[4,5]pyrrolo[1,2-a]pyrazin-1(6H)-one can be accomplished according to the general procedure provided by Scheme 7. Treatment of cyclopent-2-en-1-one with a methylating reagent, such as a methylmagnesium or methylcopper reagent, provides a 3,3-dimethylcyclopentanone. 2-Chloro-cyclopent-1-enecarbaldehyde can be provided by treatment of 3,3-dimethylcyclopentanone with an adduct resulting from the reaction of P(O)Cl₃ and DMF, followed by treatment with sodium acetate. 7,7-dimethyl-3,4,7,8-tetrahydro-2H-cyclopenta[4,5]pyrrolo[1,2-a]pyrazin-1(6H)-one (BB-4) can be readily provided by treatment of the 2-chloro-cyclopent-1-enecarbaldehyde with piperazine-2-one, which reacts at the ring nitrogen with the aldehyde to create an inner pyrrole structure as shown.

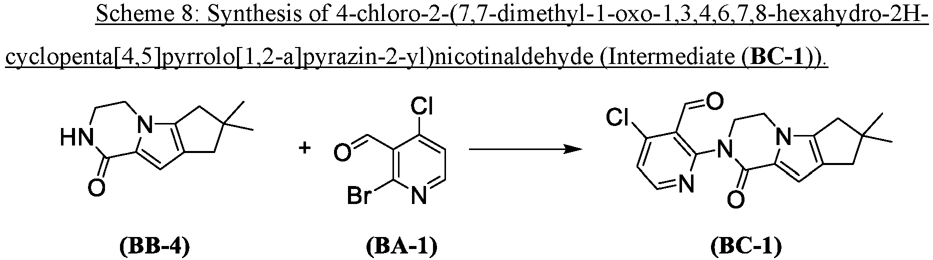

The synthesis of 4-chloro-2-(7,7-dimethyl-1-oxo-1,3,4,6,7,8-hexahydro-2H-cyclopenta[4,5]pyrrolo[1,2-a]pyrazin-2-yl)nicotinaldehyde (BC-1) can be accomplished according to the general procedure provided by Scheme 8. 7,7-dimethyl-3,4,7,8-tetrahydro-2H-cyclopenta[4,5]pyrrolo[1,2-a]pyrazin-1(6H)-one (BB-4) can be coupled with 2-bromo-4-chloronicotinaldehyde (BA-1) to form the title compound. The coupling reaction can be accomplished under coupling conditions, for example palladium catalyzed coupling conditions (e.g., Pd₂(dba)₃, XantPhos, and Cs₂CO₃ in an organic solvent).

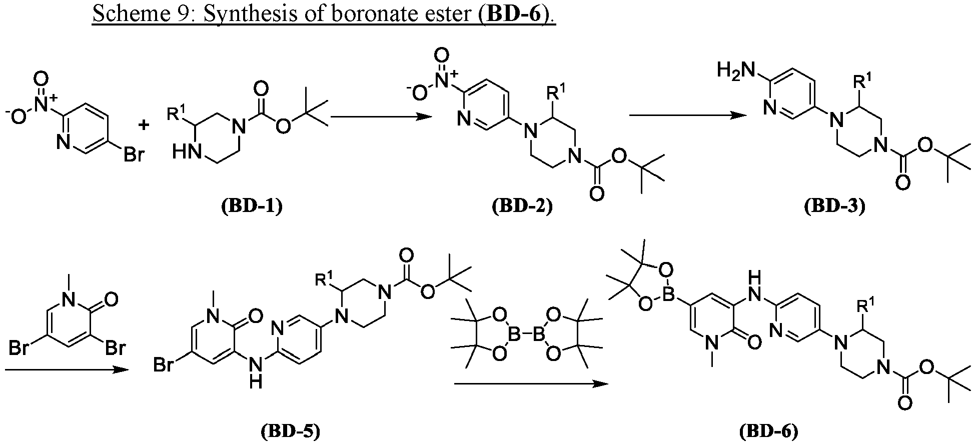

The synthesis of boronate ester **(BD-6)** can be accomplished according to the general procedure provided by Scheme 9. In this scheme, 5-bromo-2-nitropyridine is first coupled with *tert*-butyl piperazine-1-carboxylate **(BD-1)** under coupling conditions, for example palladium catalyzed coupling conditions (e.g., Pd₂(dba)₃, XantPhos, and Cs₂CO₃ in an organic solvent), to provide a nitropyridin-3-ylpiperazine-1-carboxylate **(BD-2).** The nitropyridin-3-ylpiperazine-1-carboxylate **(BD-2)** is then reduced to aminopyridin-3-ylpiperazine-1-carboxylate **(BD-3)** under reducing conditions, such as palladium on carbon and hydrogen gas. Coupling of aminopyridin-3-ylpiperazine-1-carboxylate **(BD-3)** with 3,5-dibromo-1-methylpyridin-2(1H)-one via coupling conditions, such a palladium catalyzed coupling conditions (e.g., Pd₂(dba)₃, XantPhos, and Cs₂CO₃ in an organic solvent), provides tert-butyl 4-(6-((5-bromo-1-methyl-2-oxo-1,2-dihydropyridin-3-yl)amino)pyridin-3-yl)piperazine-1-carboxylate **(BD-5).** Further coupling of tert-butyl 4-(6-((5-bromo-1-methyl-2-oxo-1,2-dihydropyridin-3-yl)amino)pyridin-3-yl)piperazine-1-carboxylate **(BD-5)** with a diborane compound, such as 4,4,4',4',5,5,5',5'-octamethyl-2,2'-bi(1,3,2-dioxaborolane), can be accomplished under coupling conditions, for example palladium catalyzed coupling conditions (e.g., Pd₂(dba)₃, XPhos, and KOAc in an organic solvent), to provide the boronate ester **(BD-6).**

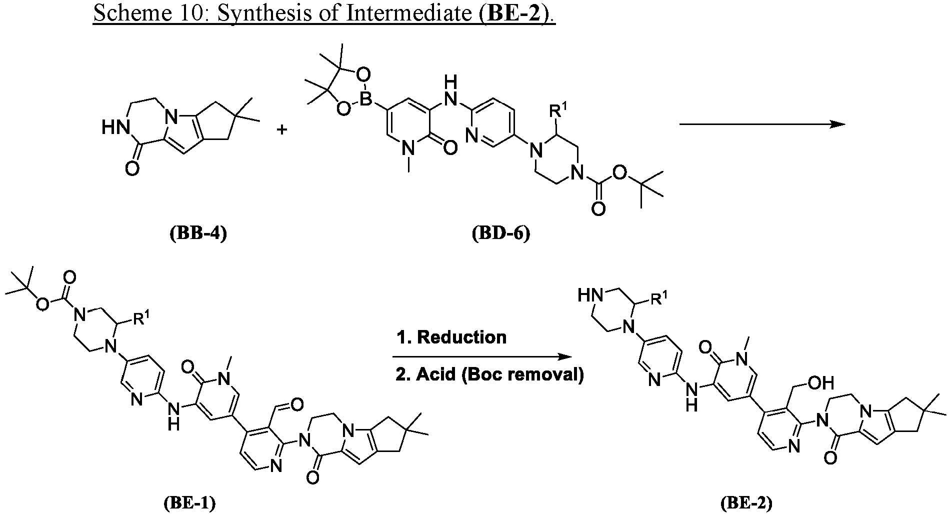

The synthesis of Intermediate **(BE-1)** can be accomplished according to the general procedure provided by Scheme 10. 7,7-dimethyl-3,4,7,8-tetrahydro-2H-cyclopenta[4,5]pyrrolo[1,2-a]pyrazin-1(6H)-one **(BB-4)** can be coupled to the boronate ester **(BD-6)** under coupling conditions, for example under palladium catalyzed coupling conditions (e.g., Pd(dppf)Cl₂, K₃PO₄, and NaOAc), to provide Intermediate **(BE-1).** Intermediate **(BE-1)** can then be converted to Intermediate **(BE-2)** by first reducing the aldehyde moiety under reducing conditions, such as sodium borohydride, followed by removal of the BOC group under deprotection conditions, such as anhydrous acidic conditions, for example HCl in a mixture of MeOH and THF.

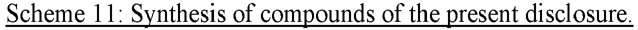

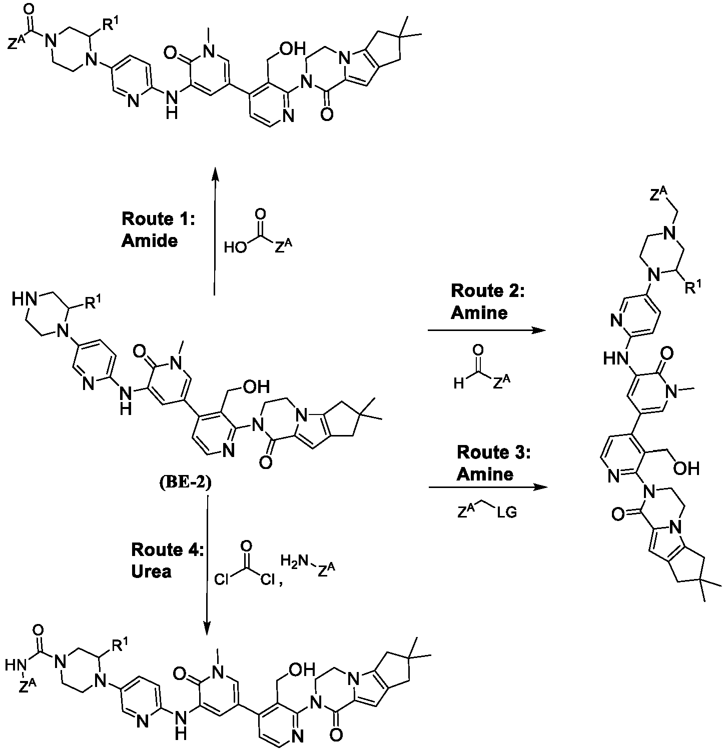

The synthesis of compounds of the present disclosure can be accomplished using any of four general synthetic strategies provided in Scheme 11. Intermediate (BE-2) can be coupled to an acid via any commonly known peptide coupling strategy (route 1), for example, the use of the coupling agent, HATU, with a base, such as DIEA, in a solvent, such as DMF. The product in route 1 is an amide, wherein Z^{A} is a chemical moiety that includes the Y group and optionally includes at least a portion of the L group, for instance, any of Y¹, Y², Y³, Y⁴, Y⁵, and Y⁶, wherein the definitions of Z, L, Y¹, Y², Y³, Y⁴, Y⁵, and Y⁶ (including provisos) are each independently described herein in the compounds of this disclosure, for example Formula **(I).** Routes 2 and 3 in Scheme 11 produce amine by reductive amination and direct nucleophilic substitution, respectively. The reductive amination of route 2 can be accomplished by reacting Intermediate (BE-2) with an aldehyde, in the presence of a reducing agent, such as NaB(OAc)₃, to convert the resulting imine into the amine moiety. In route 3, the amine is produced by nucleophilic substitution of the leaving group of an aliphatic moiety by the amine moiety of Intermediate (BE-2). Examples of suitable leaving groups are Cl, Br, I, and alkyl sulfonate groups such as methane sulfonate and toluene sulfonate. Compounds of the present disclosure possessing a urea linking group in L can be produced using the strategy provided by route 4 of Scheme 11. Phosgene, or a phosgene equivalent, can be reacted with the amine moiety of Intermediate (BE-2), followed by treatment with another amine to provide the urea derivative.

The above-mentioned synthetic schemes were used to synthesize the compounds in Table 1.

**Table 1: Example compounds of the present disclosure.**

| **Cmpd No.** | **Structure** |
|---|---|
| 1 | 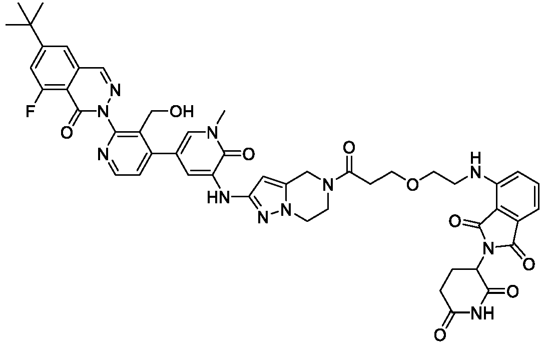 |
| 2 | 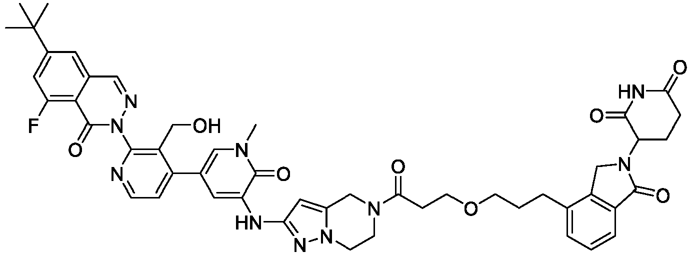 |
| 3 | 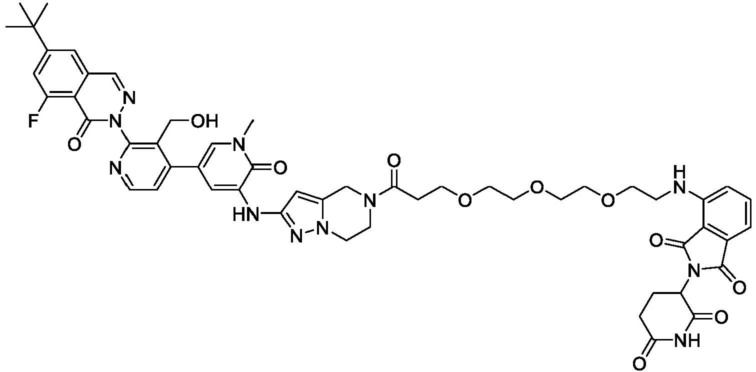 |
| 4 | 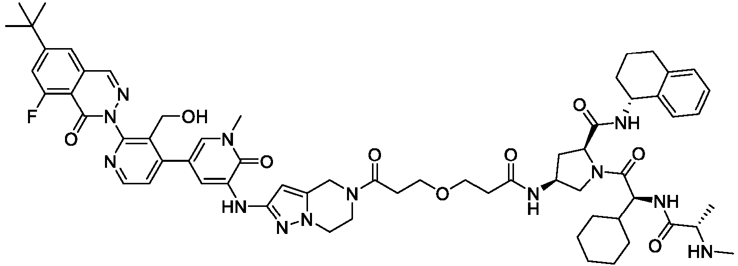 |
| 5 | 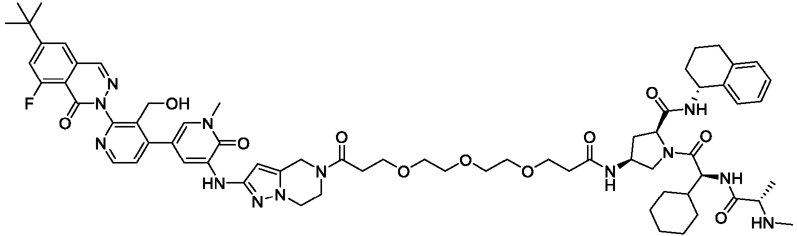 |
| 6 | 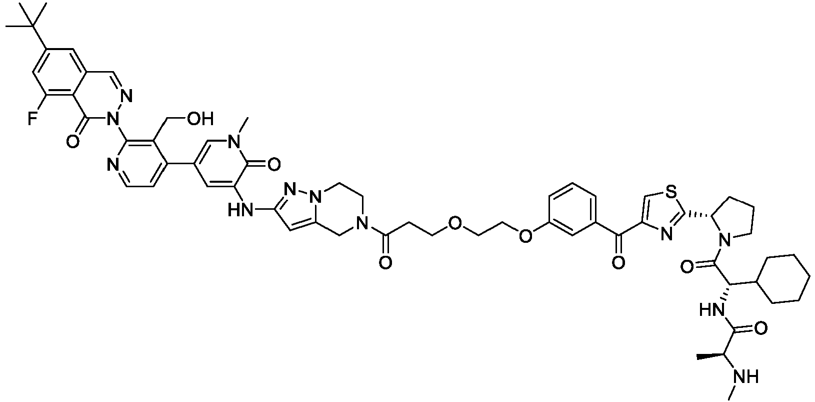 |
| 7 | 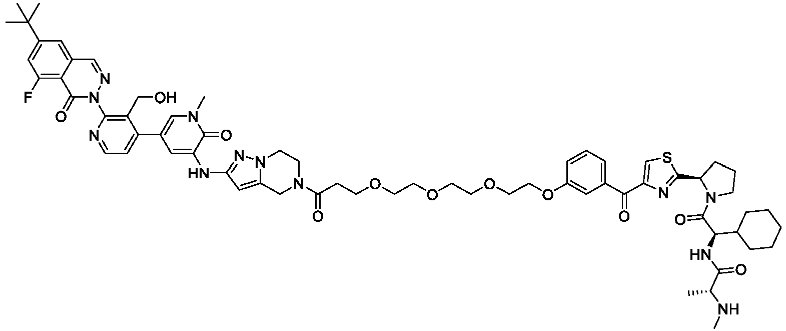 |
| 8 | 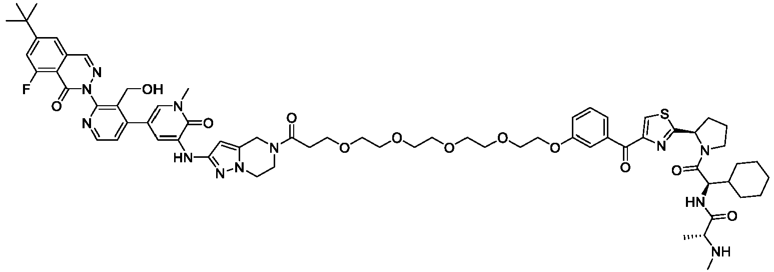 |
| 9 | 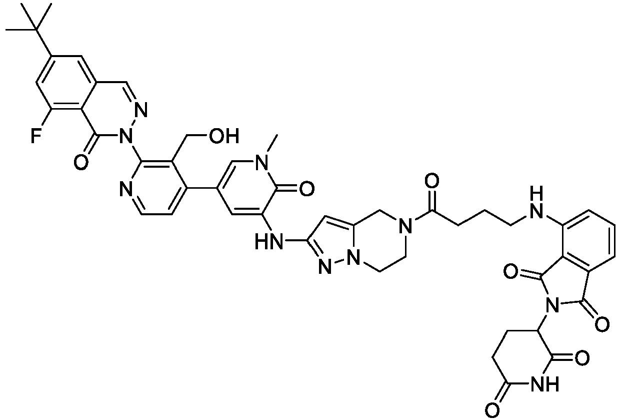 |
| 10 | 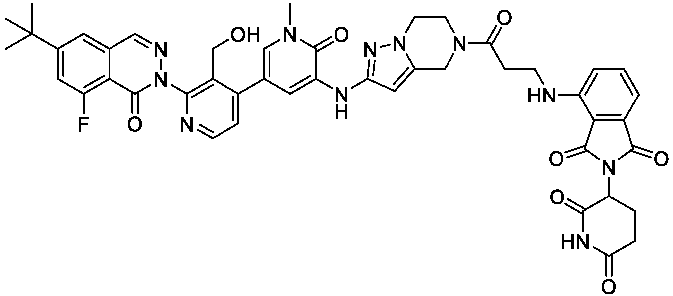 |
| 11 | 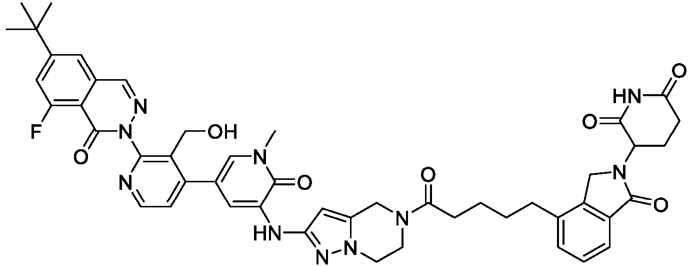 |
| 12 | 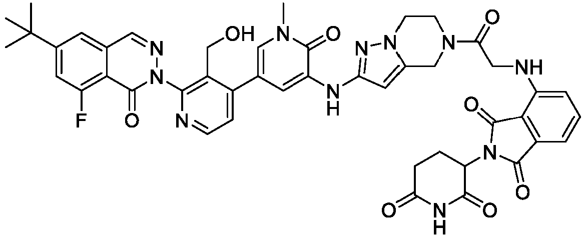 |
| 13 | 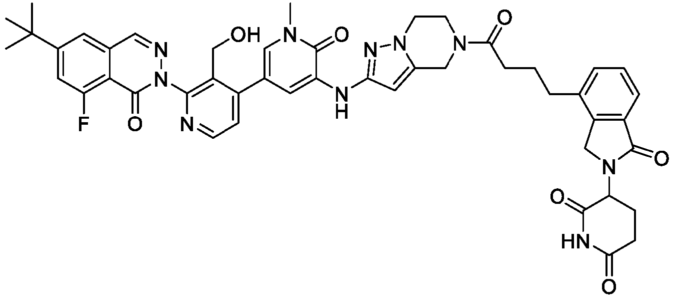 |
| 14 | 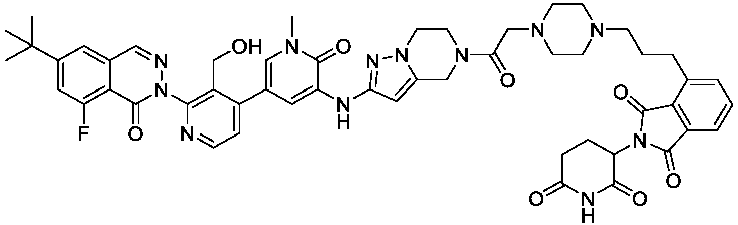 |
| 15 | 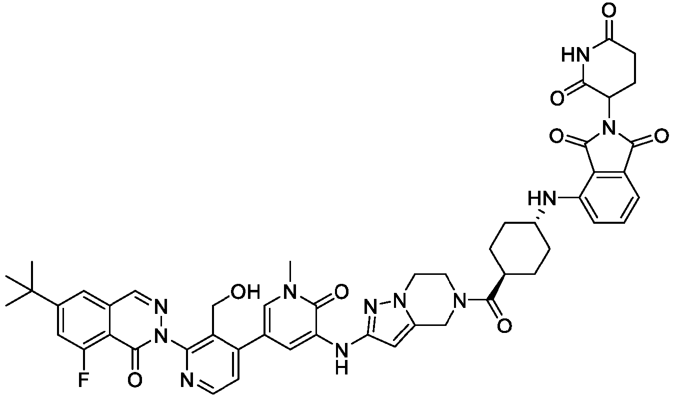 |
| 16 | 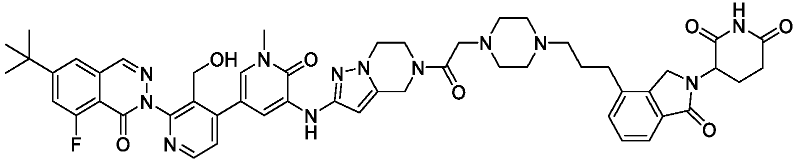 |
| 17 | 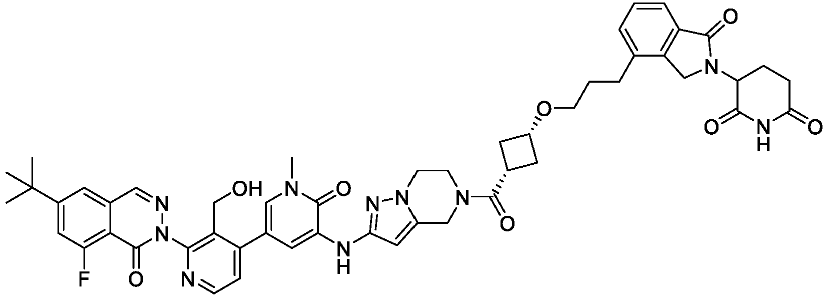 |
| 18 | 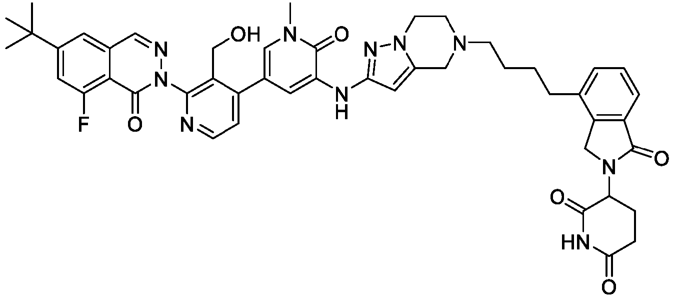 |
| 19 | 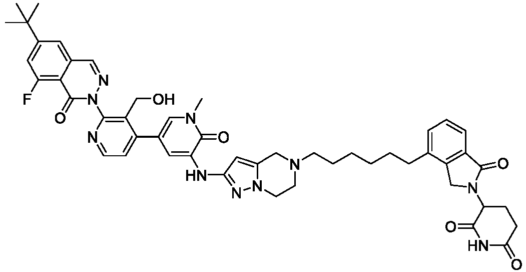 |
| 20 | 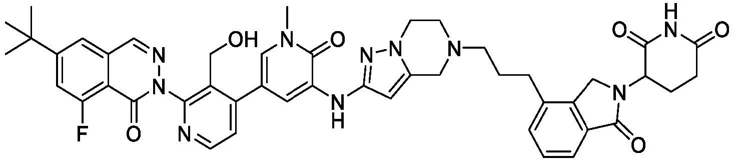 |
| 21 | 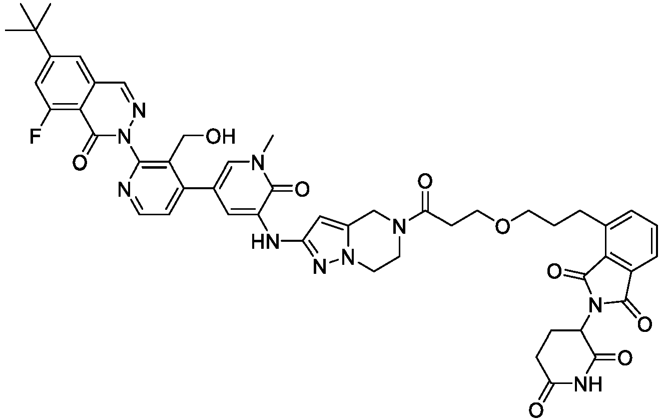 |
| 22 | 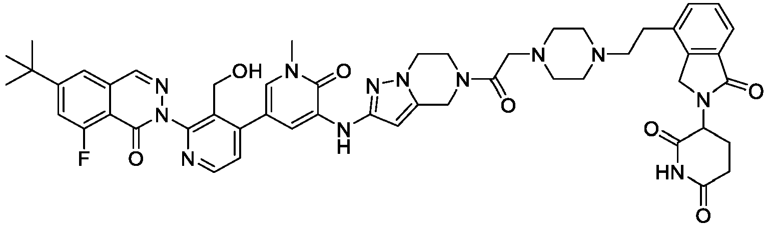 |
| 23 | 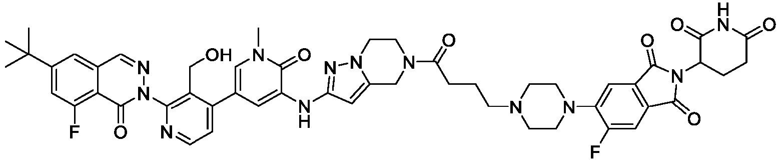 |
| 24 | 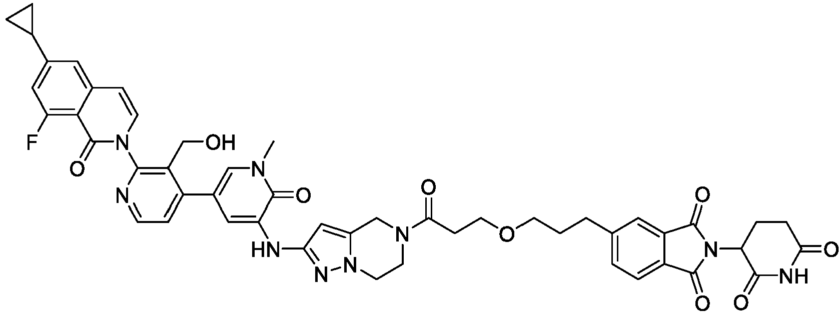 |
| 25 | 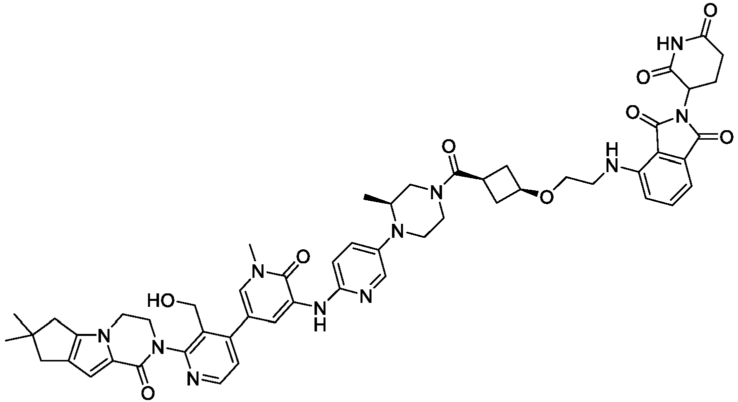 |
| 26 | 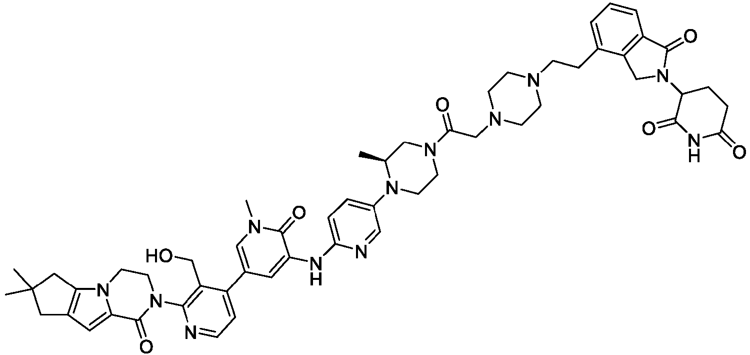 |
| 27 | 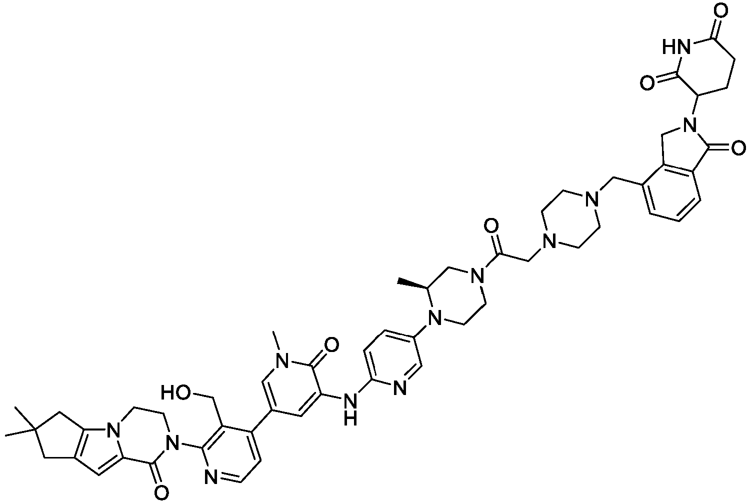 |
| 28 | 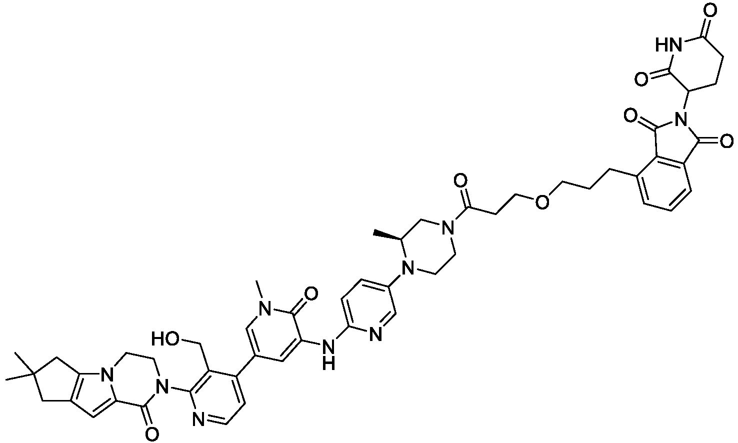 |
| 29 | 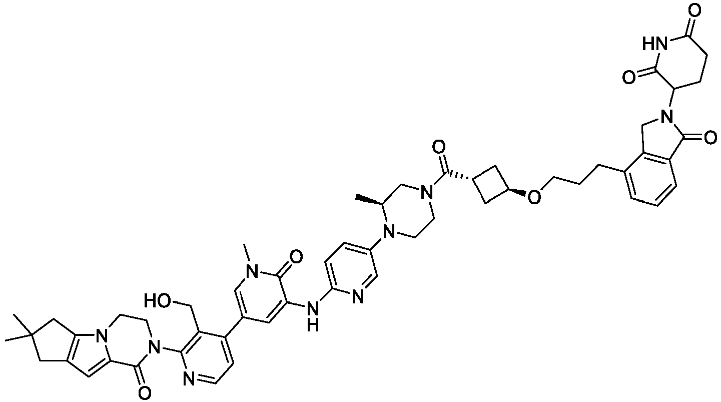 |
| 30 | 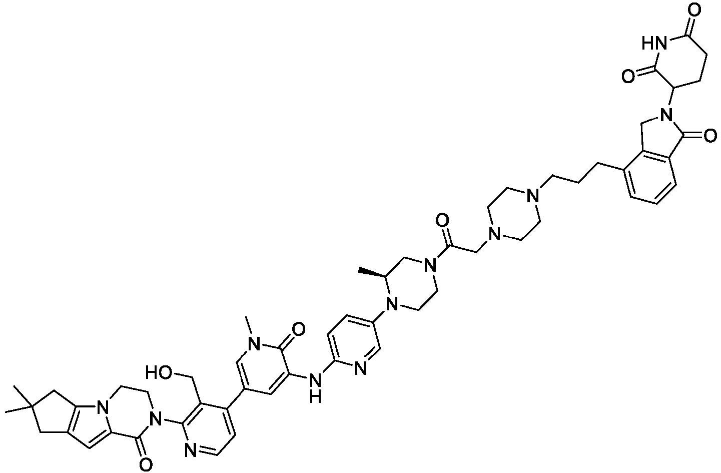 |
| 31 | 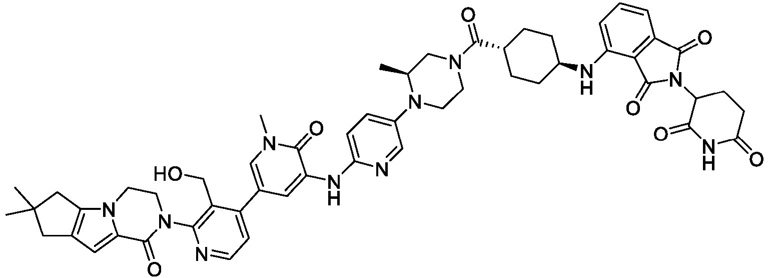 |
| 32 | 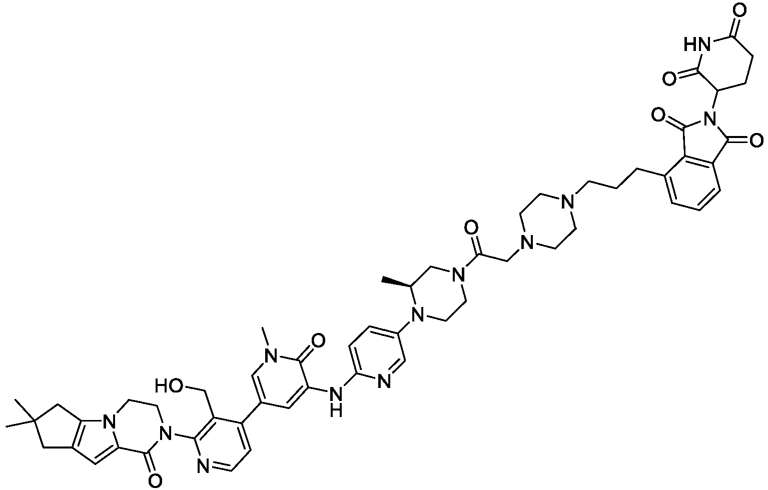 |
| 33 | 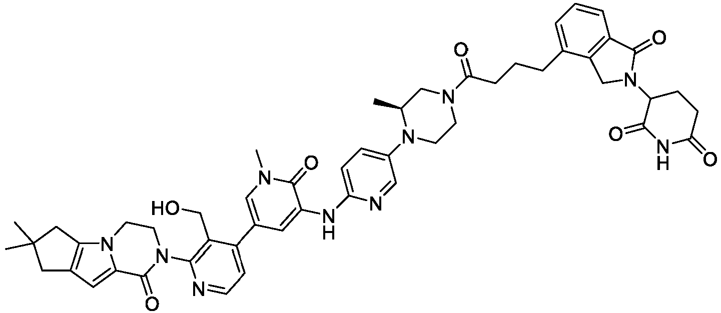 |
| 34 | 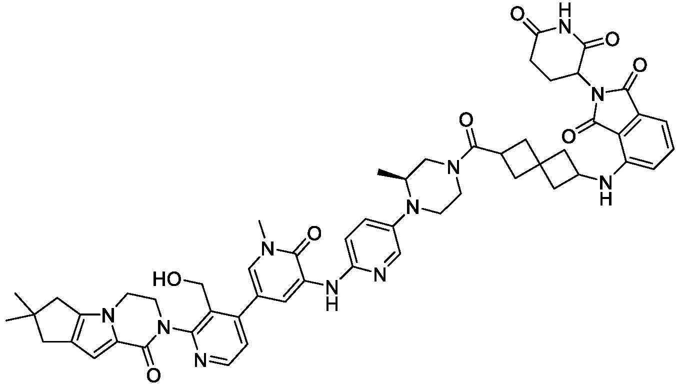 |
| 35 | 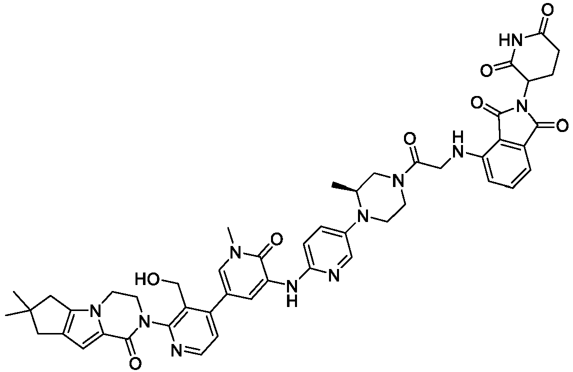 |
| 36 | 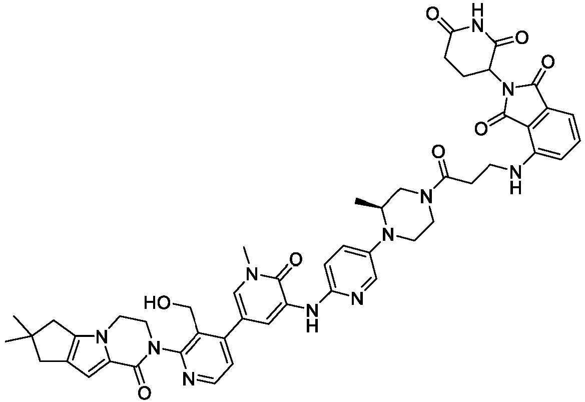 |
| 37 | 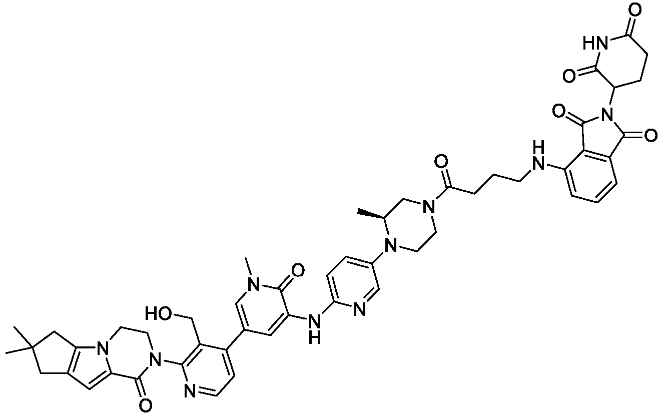 |
| 38 | 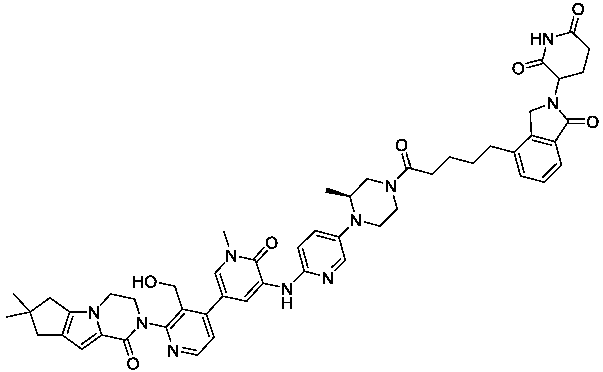 |
| 39 | 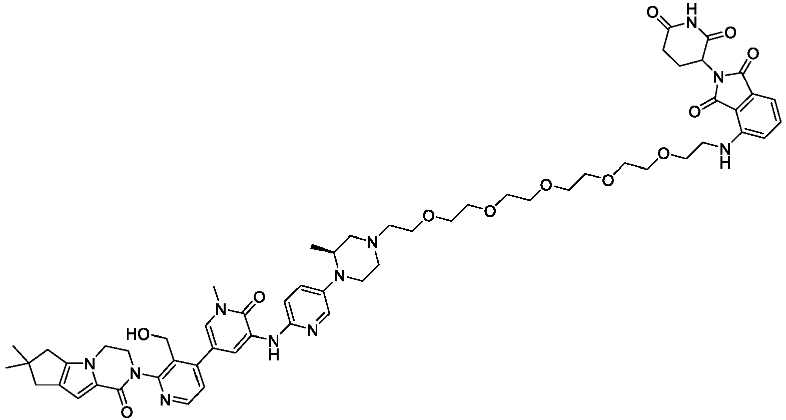 |
| 40 | 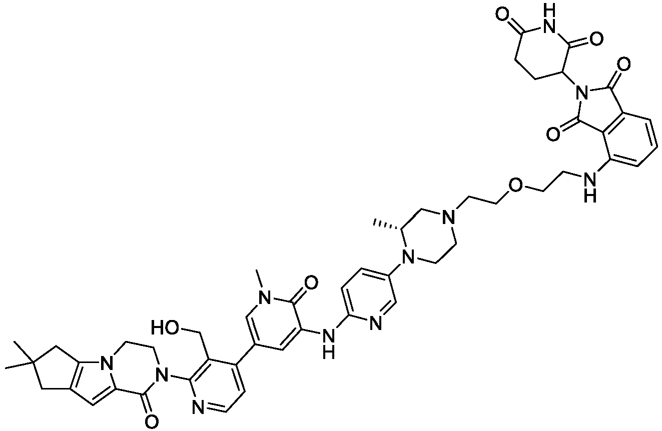 |
| 41 | 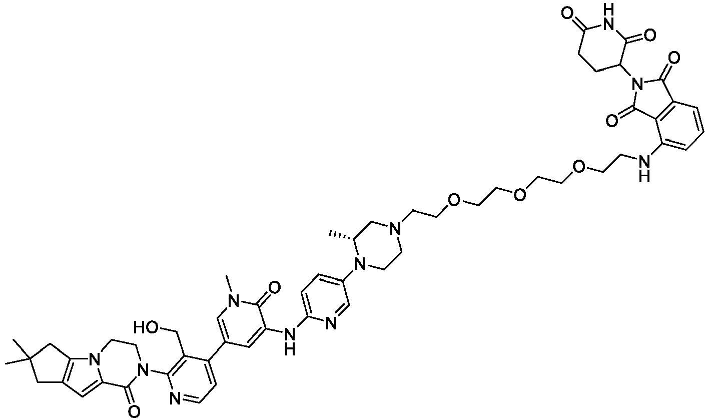 |
| 42 | 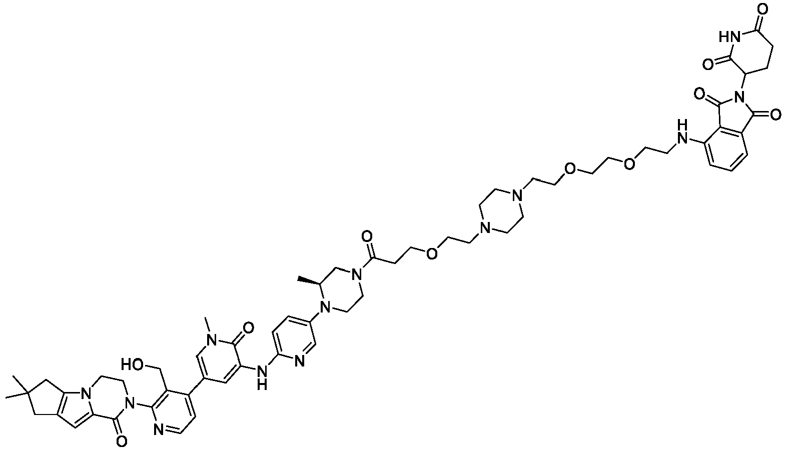 |
| 43 | 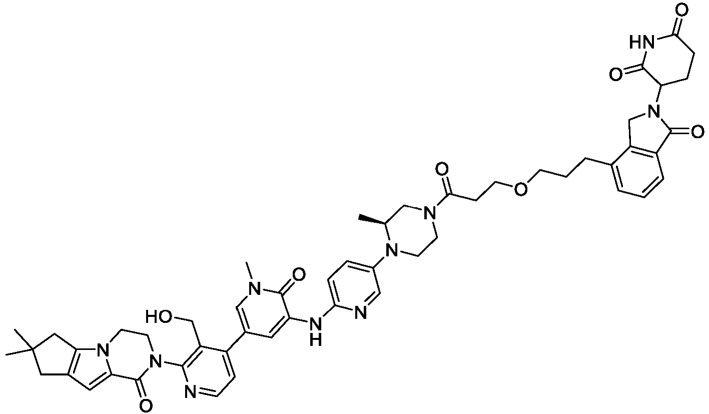 |
| 44 | 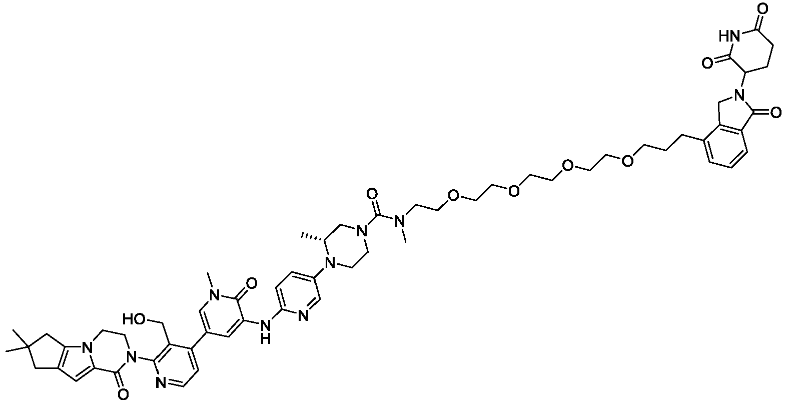 |
| 45 | 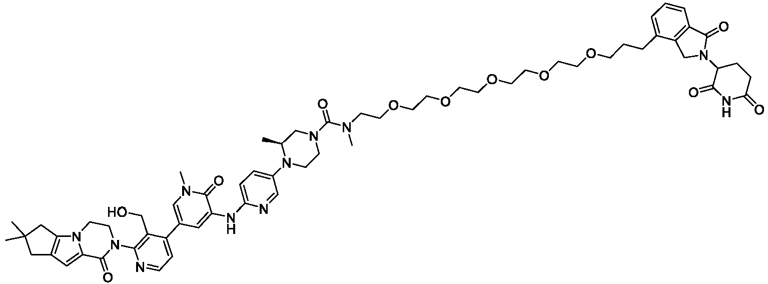 |
| 46 | 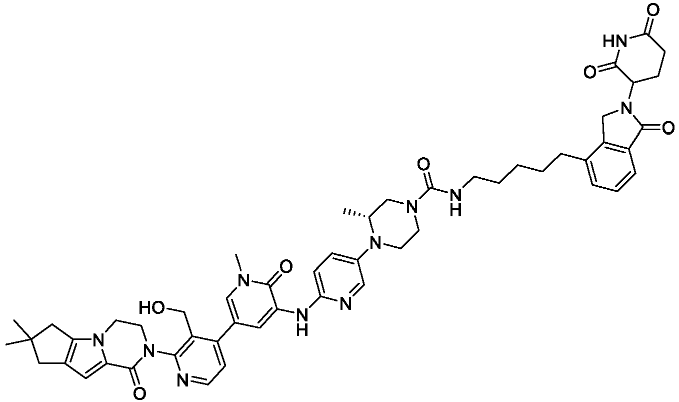 |
| 47 | 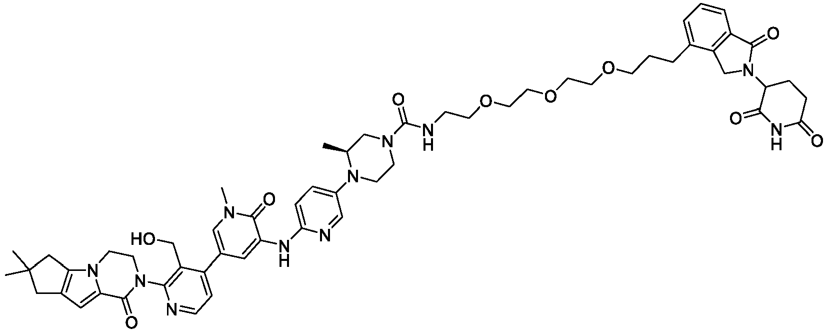 |
| 48 | 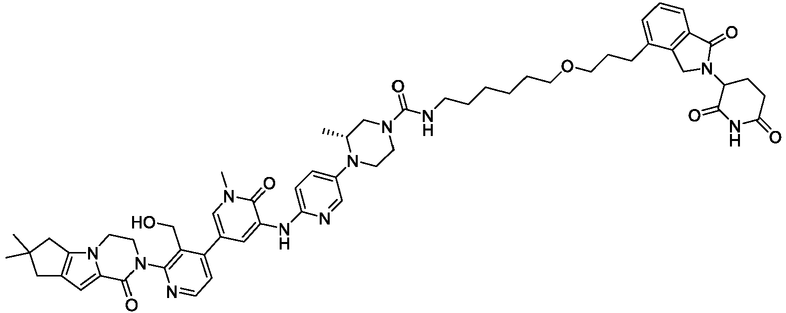 |
| 49 | 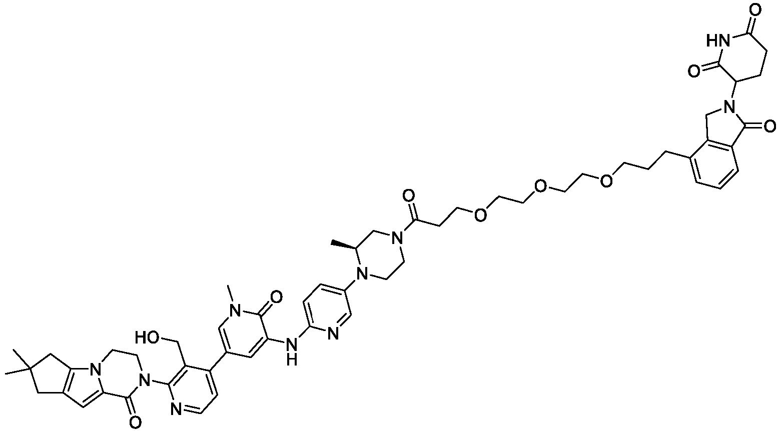 |
| 50 | 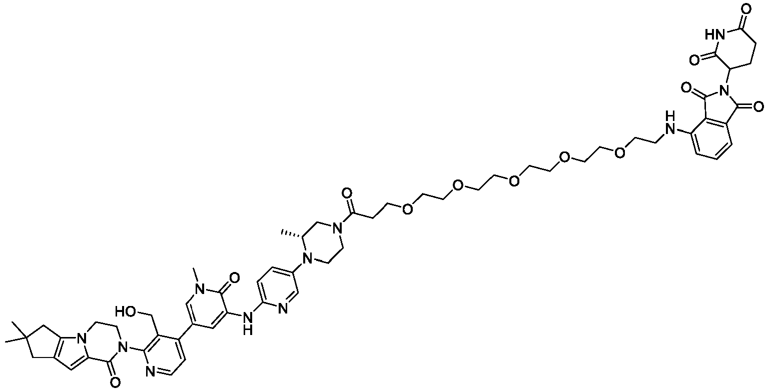 |
| 51 | 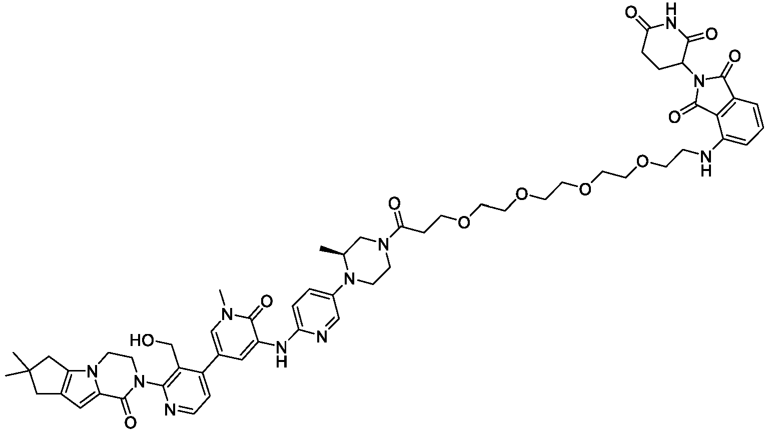 |
| 52 | 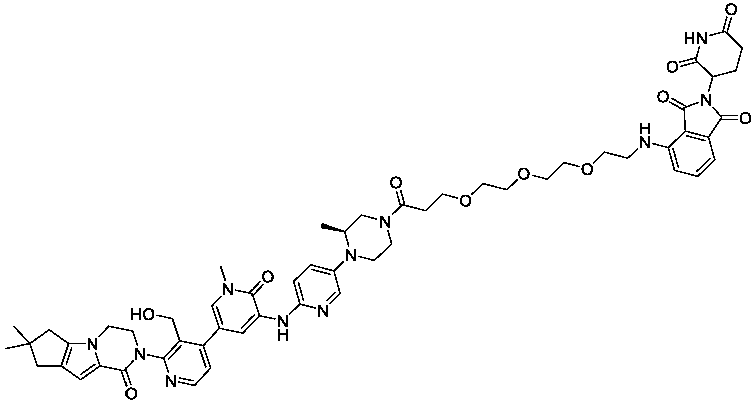 |
| 53 | 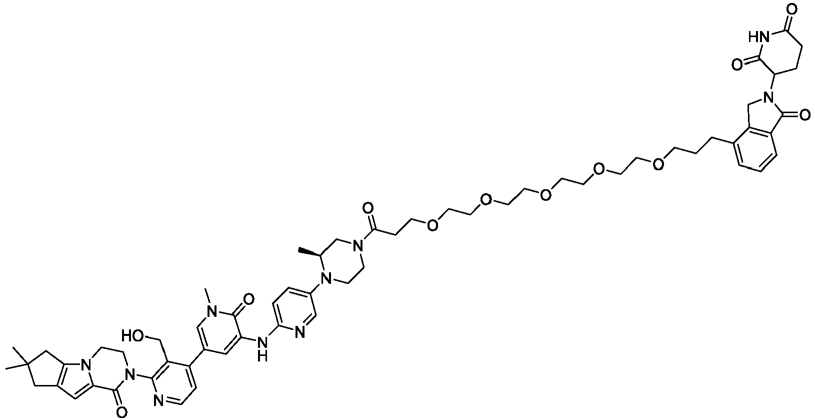 |
| 54 | 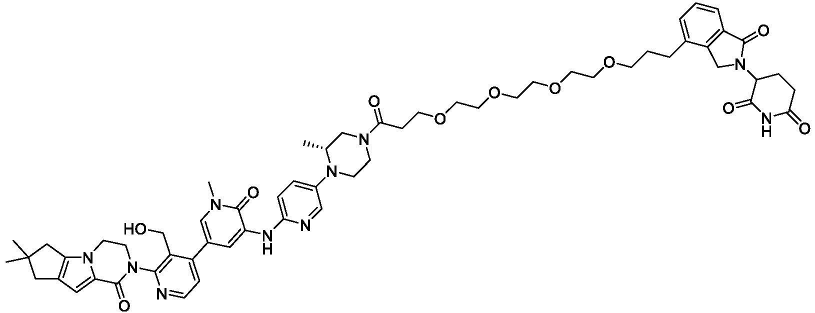 |
| 55 | 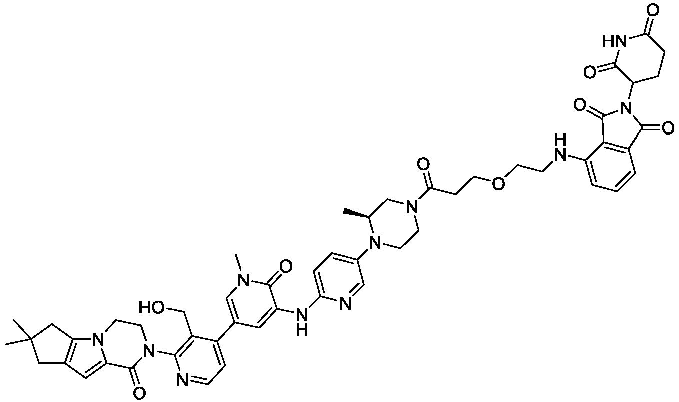 |
| 56 | 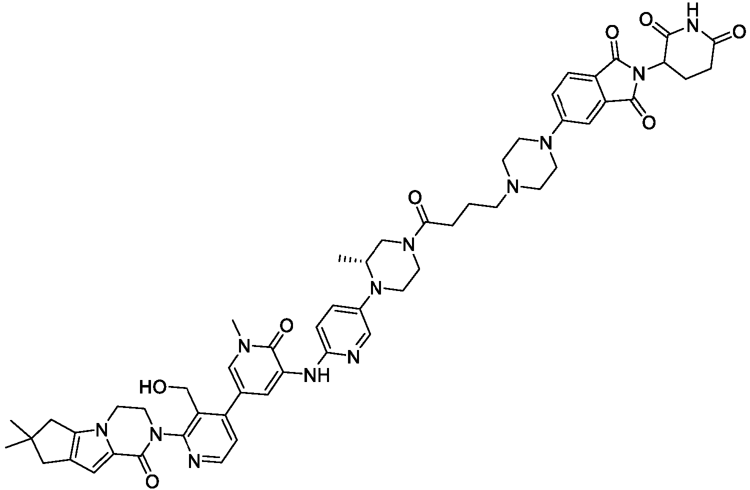 |
| 57 | 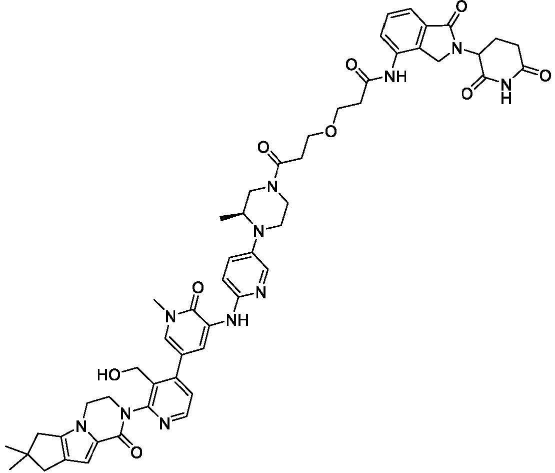 |
| 58 | 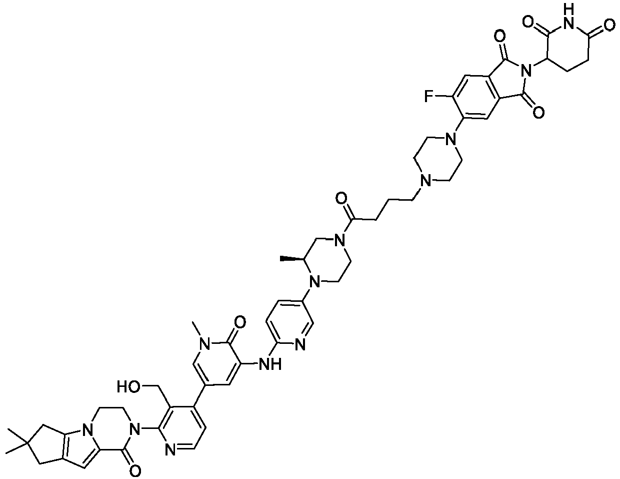 |

or a pharmaceutically acceptable salt thereof.

### III. USES, FORMULATIONS, AND ADMINISTRATION

### A. Pharmaceutical Compositions

The compounds described herein can be formulated into pharmaceutical compositions that further comprise a pharmaceutically acceptable carrier, diluent, adjuvant or vehicle. In one example, the present disclosure provides a pharmaceutical composition comprising a compound of this disclosure described above, and a pharmaceutically acceptable carrier, diluent, adjuvant or vehicle. In one example, the present disclosure provides a pharmaceutical composition comprising an effective amount of a compound of the present disclosure or a pharmaceutically acceptable salt thereof and a pharmaceutically acceptable carrier, diluent, adjuvant or vehicle. Pharmaceutically acceptable carriers include, for example, pharmaceutical diluents, excipients or carriers suitably selected with respect to the intended form of administration, and consistent with conventional pharmaceutical practices.

According to another example, this disclosure provides a composition comprising a compound of this invention or a pharmaceutically acceptable salt thereof and a pharmaceutically acceptable carrier, adjuvant, or vehicle. Pharmaceutical compositions of this disclosure comprise a therapeutically effective amount of a compound of Formula (I), (I-**A), (II), (II-A), (III), (III-A),** or **(III-B)**, wherein a "therapeutically effective amount" is an amount that is (a) effective to measurably degrade BTK (or reduce the amount of BTK) in a biological sample or in a patient, or (b) effective in treating and/or ameliorating a disease or disorder that is mediated by BTK.

The term "patient," as used herein, means an animal, preferably a mammal, and most preferably a human.

It also will be appreciated that certain of the compounds of the present disclosure can exist in free form for treatment, or where appropriate, as a pharmaceutically acceptable derivative (e.g., a salt) thereof. According to the present disclosure, a pharmaceutically acceptable derivative includes, but is not limited to, pharmaceutically acceptable prodrugs, salts, esters, salts of such esters, or any other adduct or derivative that upon administration to a patient in need is capable of providing, directly or indirectly, a compound as otherwise described herein, or a metabolite or residue thereof.

As used herein, the term "pharmaceutically acceptable salt" refers to those salts that are, within the scope of sound medical judgement, suitable for use in contact with the tissues of humans and lower animals without undue toxicity, irritation, allergic response and the like.

Pharmaceutically acceptable salts are well known in the art. For example, S. M. Berge et al., describe pharmaceutically acceptable salts in detail in J. Pharmaceutical Sciences, 1977, 66, 1-19. Pharmaceutically acceptable salts of the compounds of this disclosure include those derived from suitable inorganic and organic acids and bases. Examples of pharmaceutically acceptable, nontoxic acid addition salts include salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N⁺(C₁₋₄alkyl)₄ salts. This disclosure also envisions the quaternization of any basic nitrogen-containing groups of the compounds disclosed herein. Water or oil-soluble or dispersable products may be obtained by such quaternization. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate.

A pharmaceutically acceptable carrier may contain inert ingredients that do not unduly inhibit the biological activity of the compounds. The pharmaceutically acceptable carriers should be biocompatible, e.g., non-toxic, non-inflammatory, non-immunogenic or devoid of other undesired reactions or side-effects upon the administration to a subject. Standard pharmaceutical formulation techniques can be employed.

The pharmaceutically acceptable carrier, adjuvant, or vehicle, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutically acceptable compositions and known techniques for the preparation thereof. Except insofar as any conventional carrier medium is incompatible with the compounds described herein, such as by producing any undesirable biological effect or otherwise interacting in a deleterious manner with any other component(s) of the pharmaceutically acceptable composition, the use of such conventional carrier medium is contemplated to be within the scope of this disclosure. As used herein, the phrase "side effects" encompasses unwanted and adverse effects of a therapy (e.g., a prophylactic or therapeutic agent). Side effects are always unwanted, but unwanted effects are not necessarily adverse. An adverse effect from a therapy (e.g., prophylactic or therapeutic agent) might be harmful, uncomfortable, or risky. Side effects include, but are not limited to, fever, chills, lethargy, gastrointestinal toxicities (including gastric and intestinal ulcerations and erosions), nausea, vomiting, neurotoxicities, nephrotoxicities, renal toxicities (including such conditions as papillary necrosis and chronic interstitial nephritis), hepatic toxicities (including elevated serum liver enzyme levels), myelotoxicities (including leukopenia, myelosuppression, thrombocytopenia and anemia), dry mouth, metallic taste, prolongation of gestation, weakness, somnolence, pain (including muscle pain, bone pain and headache), hair loss, asthenia, dizziness, extra-pyramidal symptoms, akathisia, cardiovascular disturbances and sexual dysfunction.

Some examples of materials that can serve as pharmaceutically acceptable carriers include, but are not limited to, ion exchangers, alumina, aluminum stearate, lecithin, serum proteins (such as human serum albumin), buffer substances (such as twin 80, phosphates, glycine, sorbic acid, or potassium sorbate), partial glyceride mixtures of saturated vegetable fatty acids, water, salts or electrolytes (such as protamine sulfate, disodium hydrogen phosphate, potassium hydrogen phosphate, sodium chloride, or zinc salts), colloidal silica, magnesium trisilicate, polyvinyl pyrrolidone, polyacrylates, waxes, polyethylene-polyoxypropylene-block polymers, methylcellulose, hydroxypropyl methylcellulose, wool fat, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol or polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents. Preservatives and antioxidants can also be present in the composition, according to the judgment of the formulator.

As used herein, the term "measurably degrade," means a measurable reduction in (a) BTK activity, between a sample comprising a compound of this disclosure and a BTK and an equivalent sample comprising a BTK in the absence of said compound, or (b) the concentration of the BTK in a sample over time.

The compositions of the present disclosure may be administered orally, parenterally, by inhalation spray, topically, rectally, nasally, buccally, vaginally or via an implanted reservoir. As used herein, the term "parenteral" includes subcutaneous, intravenous, intramuscular, intra-articular, intra-synovial, intrasternal, intrathecal, intraocular, intrahepatic, intralesional and intracranial injection or infusion techniques. Preferably, the compositions are administered orally, intraperitoneally or intravenously. Sterile injectable forms of the compositions of this disclosure may be aqueous or oleaginous suspension. These suspensions may be formulated according to techniques known in the art using suitable dispersing or wetting agents and suspending agents. The sterile injectable preparation also may be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or di-glycerides. Fatty acids, such as oleic acid and its glyceride derivatives, are useful in the preparation of injectables, as are natural pharmaceutically acceptable oils, such as olive oil or castor oil, especially in their polyoxyethylated versions. These oil solutions or suspensions also may contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers that are commonly used in the manufacture of pharmaceutically acceptable solid, liquid, or other dosage forms may also be used for the purposes of formulation.

The pharmaceutically acceptable compositions of this disclosure may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents also may be added.

Alternatively, the pharmaceutically acceptable compositions of this disclosure may be administered in the form of suppositories for rectal or vaginal administration. These can be prepared by mixing the agent with a suitable non-irritating excipient that is solid at room temperature but liquid at rectal temperature and therefore will melt in the rectum or vaginal cavity to release the drug. Such materials include cocoa butter, polyethylene glycol or a suppository wax that is solid at ambient temperature but liquid at body temperature and therefore melt in the rectum or vaginal cavity and release the active compound.

The pharmaceutically acceptable compositions of this disclosure also may be administered topically, especially when the target of treatment includes areas or organs readily accessible by topical application, including diseases of the eye, skin, or lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches also may be used.

For topical applications, the pharmaceutically acceptable compositions may be formulated in a suitable ointment containing the active component suspended or dissolved in one or more carriers. Carriers for topical administration of the compounds of this disclosure include, but are not limited to, mineral oil, liquid petrolatum, white petrolatum, propylene glycol, polyoxyethylene, polyoxypropylene compound, emulsifying wax and water. Alternatively, the pharmaceutically acceptable compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutically acceptable compositions may be formulated, e.g., as micronized suspensions in isotonic, pH adjusted sterile saline or other aqueous solution, or, preferably, as solutions in isotonic, pH adjusted sterile saline or other aqueous solution, either with or without a preservative such as benzylalkonium chloride. Alternatively, for ophthalmic uses, the pharmaceutically acceptable compositions may be formulated in an ointment such as petrolatum. The pharmaceutically acceptable compositions of this disclosure also may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions also can include adjuvants such as wetting agents, emulsifying and suspending agents, sweetening, flavoring, and perfuming agents.

Injectable preparations, for example, sterile injectable aqueous or oleaginous suspensions, may be formulated according to the known art using suitable dispersing or wetting agents and suspending agents. The sterile injectable preparation also may be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid may be used in the preparation of injectables.

The injectable formulations can be sterilized, for example, by filtration through a bacterial-retaining filter, or by incorporating sterilizing agents in the form of sterile solid compositions that can be dissolved or dispersed in sterile water or other sterile injectable medium prior to use.

In order to prolong the effect of a compound of the present disclosure, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations also are prepared by entrapping the compound in liposomes or microemulsions that are compatible with body tissues.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form also may comprise buffering agents.

Solid compositions of a similar type also may be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. Solid dosage forms optionally may contain opacifying agents. These solid dosage forms also can be of a composition such that they release the active ingredient(s) only, or preferentially, in a certain part of the intestinal tract, optionally, in a delayed manner. Examples of embedding compositions that can be used include polymeric substances and waxes. Solid compositions of a similar type also may be employed as fillers in soft and hard-filled gelatin capsules using such excipients as lactose or milk sugar as well as high molecular weight polethylene glycols and the like.

The active compounds also can be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms also may comprise, as is normal practice, additional substances other than inert diluents, e.g., tableting lubricants and other tableting aids such a magnesium stearate and microcrystalline cellulose. In the case of capsules, tablets and pills, the dosage forms also may comprise buffering agents. They may optionally contain opacifying agents and also can be of a composition such that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of this disclosure include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops also are contemplated as being within the scope of this disclosure. Additionally, the present disclosure contemplates the use of transdermal patches, which have the added advantage of providing controlled delivery of a compound to the body. Such dosage forms can be made by dissolving or dispensing the compound in the proper medium. Absorption enhancers also can be used to increase the flux of the compound across the skin. The rate can be controlled by either providing a rate controlling membrane or by dispersing the compound in a polymer matrix or gel.

The compounds of this disclosure preferably are formulated in dosage unit form for ease of administration and uniformity of dosage. As used herein, the phrase "dosage unit form" refers to a physically discrete unit of agent appropriate for the patient to be treated. It will be understood, however, that the total daily usage of the compounds and compositions of the present disclosure will be decided by the attending physician within the scope of sound medical judgment. The specific effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts.

The amount of the compounds of the present disclosure that may be combined with the carrier materials to produce a composition in a single dosage form will vary depending upon the host treated, the particular mode of administration, and other factors. Preferably, the compositions should be formulated so that a dosage of between 0.01 - 100 mg/kg body weight/day of the inhibitor can be administered to a patient receiving these compositions.

Depending upon the particular condition, or disease, to be treated or prevented, additional therapeutic agents, which are normally administered to treat or prevent that condition, also may be present in the compositions of this disclosure. As used herein, additional therapeutic agents that are normally administered to treat or prevent a particular disease, or condition, are known as "appropriate for the disease, or condition, being treated."

For example, chemotherapeutic agents or other anti-proliferative agents may be combined with the compounds of this disclosure to treat proliferative diseases and cancer. Examples of known chemotherapeutic agents include, but are not limited to, PI3K inhibitors (e.g., idelalisib and copanlisib), BCL-2 inhibitors (e.g., venetoclax), BTK inhibitors (e.g., ibrutinib and acalabrutinib), etoposide, CD20 antibodies (e.g., rituximab, ocrelizumab, obinutuzumab, ofatumumab, ibritumomab tiuxetan, tositumomab, and ublituximab), aletuzumab, bendamustine, cladribine, doxorubicin, chlorambucil, prednisone, midostaurin, lenalidomide, pomalidomide, checkpoint inhibitors (e.g., ipilimumab, nivolumab, pembolizumab, atezolizumab, avelumab, durvalumab), engineered cell therapy (e.g., CAR-T therapy - Kymriah^{®}, Yescarta^{®}), Gleevec^{™}, adriamycin, dexamethasone, vincristine, cyclophosphamide, fluorouracil, topotecan, taxol, interferons, and platinum derivatives.

And, in some instances, radiation therapy is administered during the treatment course wherein a compound of the present disclosure (or a pharmaceutically acceptable salt thereof) is administered to a patient in need thereof.

Other examples of agents with which the inhibitors of this disclosure also may be combined include, without limitation: treatments for Alzheimer's Disease such as Aricept^{®} and Excelon^{®}; treatments for Parkinson's Disease such as L-DOPA/carbidopa, entacapone, ropinrole, pramipexole, bromocriptine, pergolide, trihexephendyl, and amantadine; agents for treating Multiple Sclerosis (MS) such as beta interferon (e.g., Avonex^{®} and Rebif^{®}), Copaxone^{®}, and mitoxantrone; treatments for asthma such as albuterol and Singulair^{®}; agents for treating schizophrenia such as zyprexa, risperdal, seroquel, and haloperidol; antiinflammatory agents such as corticosteroids, TNF blockers, IL-1 RA, azathioprine, cyclophosphamide, and sulfasalazine; immunomodulatory and immunosuppressive agents such as cyclosporin, tacrolimus, rapamycin, mycophenolate mofetil, interferons, corticosteroids, cyclophophamide, azathioprine, and sulfasalazine; neurotrophic factors such as acetylcholinesterase inhibitors, MAO inhibitors, interferons, anti-convulsants, ion channel blockers, riluzole, and anti-Parkinsonian agents; agents for treating cardiovascular disease such as beta-blockers, ACE inhibitors, diuretics, nitrates, calcium channel blockers, and statins; agents for treating liver disease such as corticosteroids, cholestyramine, interferons, and anti-viral agents; agents for treating blood disorders such as corticosteroids, anti-leukemic agents, and growth factors; and agents for treating immunodeficiency disorders such as gamma globulin.

The amount of additional therapeutic agent present in the compositions of this disclosure will be no more than the amount that would normally be administered in a composition comprising that therapeutic agent as the only active agent. Preferably the amount of additional therapeutic agent in the presently disclosed compositions will range from about 50% to 100% of the amount normally present in a composition comprising that agent as the only therapeutically active agent.

### B. Uses of the Compounds and Compositions.

The bifunctional compounds of the present disclosure are useful for degrading BTK in biological samples or in patients via a ubiquitin proteolytic pathway. Thus, an example of the present disclosure provides medical uses of the compounds and compositions of this disclosure in the treatment of a BTK-mediated disease or disorder. As used herein, the term "BTK-mediated disease or disorder" means any disease, disorder, or other deleterious condition in which a BTK is known to play a role. In some instances, a BTK-mediated disease or disorder is a proliferative disorder or an autoimmune disorder. Examples of proliferative disorders include cancer.

The term "cancer" includes, but is not limited to, the following cancers: epidermoid Oral: buccal cavity, lip, tongue, mouth, pharynx; Cardiac: sarcoma (angiosarcoma, fibrosarcoma, rhabdomyosarcoma, liposarcoma), myxoma, rhabdomyoma, fibroma, lipoma, and teratoma; Lung: bronchogenic carcinoma (squamous cell or epidermoid, undifferentiated small cell, undifferentiated large cell, adenocarcinoma), alveolar (bronchiolar) carcinoma, bronchial adenoma, sarcoma, lymphoma, chondromatous hamartoma, mesothelioma; gastrointestinal: esophagus (squamous cell carcinoma, larynx, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel or small intestines (adenocarcinoma, lymphoma, carcinoid tumors, Karposi's sarcoma, leiomyoma, hemangioma, lipoma, neurofibroma, fibroma), large bowel or large intestines (adenocarcinoma, tubular adenoma, villous adenoma, hamartoma, leiomyoma), colon, colon-rectum, colorectal, rectum; Genitourinary tract: kidney (adenocarcinoma, Wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma, biliary passages; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses ), benign chondroma, chondroblastoma, chondromyxofibroma, osteoid osteoma and giant cell tumors; Nervous system: skull ( osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, meningioma, glioma, sarcoma); Gynecological: uterus ( endometrial carcinoma), cervix ( cervical carcinoma, pre-tumor cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma), breast; Hematologic: blood (myeloid leukemia (acute and chronic), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma) hairy cell; lymphoid disorders (e.g., mantle cell lymphoma, Waldenström's macroglobulinemia, Marginal zone lymphoma, and Follicular lymphoma); Skin: malilymphgnant melanoma, basal cell carcinoma, squamous cell carcinoma, Karposi's sarcoma, keratoacanthoma, moles dysplastic nevi, lipoma, angioma, dermatofibroma, keloids, psoriasis, Thyroid gland: papillary thyroid carcinoma, follicular thyroid carcinoma; medullary thyroid carcinoma, undifferentiated thyroid cancer, multiple endocrine neoplasia type 2A, multiple endocrine neoplasia type 2B, familial medullary thyroid cancer, pheochromocytoma, paraganglioma; and Adrenal glands: neuroblastoma.

Examples of autoimmune disorders include uticaria, graft-versus-host disease, pemphigus vulgaris, achalasia, Addison's disease, Adult Still's disease, agammaglobulinemia, alopecia areata, amyloidosis, ankylosing spondylitis, anti-GBM/anti-TBM nephritis, antiphospholipid syndrome, autoimmune angioedema, autoimmune dysautonomia, autoimmune encephalomyelitis, autoimmune hepatitis, autoimmune inner ear disease (AIED), autoimmune myocarditis, autoimmune oophoritis, autoimmune orchitis, autoimmune pancreatitis, autoimmune retinopathy, axonal and neuronal neuropathy (AMAN), Bal6 disease, Behcet's disease, benign mucosal pemphigoid, bullous pemphigoid, Castleman disease (CD), Celiac disease, Chagas disease, chronic inflammatory demyelinating polyneuropathy (CIDP), chronic recurrent multifocal osteomyelitis (CRMO), Churg-Strauss Syndrome (CSS) or Eosinophilic Granulomatosis (EGPA), cicatricial pemphigoid, Cogan's syndrome, cold agglutinin disease, congenital heart block, coxsackie myocarditis, CREST syndrome, Crohn's disease, dermatitis herpetiformis, dermatomyositis, Devic's disease (neuromyelitis optica), discoid lupus, Dressler's syndrome, endometriosis, eosinophilic esophagitis (EoE), eosinophilic fasciitis, erythema nodosum, essential mixed cryoglobulinemia, evans syndrome, fibromyalgia, fibrosing alveolitis, giant cell arteritis (temporal arteritis), giant cell myocarditis, glomerulonephritis, goodpasture's syndrome, granulomatosis with polyangiitis, Graves' disease, Guillain-Barre syndrome, Hashimoto's thyroiditis, hemolytic anemia, Henoch-Schonlein purpura (HSP), herpes gestationis or pemphigoid gestationis (PG), hidradenitis suppurativa (HS) (Acne Inversa), hypogammaglobulinemia, IgA nephropathy, IgG4-related sclerosing disease, immune thrombocytopenic purpura (ITP), inclusion body myositis (IBM), interstitial cystitis (IC), juvenile arthritis, juvenile diabetes (Type 1 diabetes), juvenile myositis (JM), Kawasaki disease, Lambert-Eaton syndrome, leukocytoclastic vasculitis, lichen planus, lichen sclerosus, ligneous conjunctivitis, linear IgA disease (LAD), lupus, lyme disease chronic, Meniere's disease, microscopic polyangiitis (MPA), mixed connective tissue disease (MCTD), Mooren's ulcer, Mucha-Habermann disease, multifocal motor neuropathy (MMN) or MMNCB, multiple sclerosis, myasthenia gravis, myositis, narcolepsy, neonatal lupus, neuromyelitis optica, neutropenia, ocular cicatricial pemphigoid, optic neuritis, palindromic rheumatism (PR), PANDAS, paraneoplastic cerebellar degeneration (PCD), paroxysmal nocturnal hemoglobinuria (PNH), Parry Romberg syndrome, pars planitis (peripheral uveitis), Parsonnage-Turner syndrome, pemphigus, peripheral neuropathy, perivenous encephalomyelitis, pernicious anemia (PA), POEMS syndrome, polyarteritis nodosa, polyglandular syndromes type I, II, III, polymyalgia rheumatica, polymyositis, postmyocardial infarction syndrome, postpericardiotomy syndrome, primary biliary cirrhosis, primary sclerosing cholangitis, progesterone dermatitis, psoriasis, psoriatic arthritis, pure red cell aplasia (PRCA), pyoderma gangrenosum, Raynaud's phenomenon, reactive Arthritis, reflex sympathetic dystrophy, relapsing polychondritis, restless legs syndrome (RLS), retroperitoneal fibrosis, rheumatic fever, rheumatoid arthritis, sarcoidosis, Schmidt syndrome, scleritis, scleroderma, Sjögren's syndrome, sperm and testicular autoimmunity, stiff person syndrome (SPS), subacute bacterial endocarditis (SBE), Susac's syndrome, sympathetic ophthalmia (SO), Takayasu's arteritis, temporal arteritis (giant cell arteritis), thrombocytopenic purpura (TTP), Tolosa-Hunt syndrome (THS), transverse myelitis, Type 1 diabetes, ulcerative colitis (UC), undifferentiated connective tissue disease (UCTD), uveitis, vasculitis, vitiligo, Vogt-Koyanagi-Harada Disease, and Wegener's granulomatosis (or Granulomatosis with Polyangiitis (GPA)).

### IV. EXAMPLES

Additional examples are disclosed in further detail in the following examples, which are not in any way intended to limit the scope of the claims.

### Example 1: Intermediates useful for generating bifunctional compounds of the present disclosure.

Intermediates useful for generating bifunctional compounds of the present disclosure were synthesized as described below in Examples 1A-1E.

### Example 1A: Synthesis of 6-cyclopropyl-8-fluoro-2-(3'-(hydroxymethyl)-1-methyl-6-oxo-5-((4,5,6,7-tetrahydropyrazolo[1,5-a]pyrazin-2-yl)amino)-1,6-dihydro-[3,4'-bipyridin]-2'-yl)isoquinolin-1(2H)-one.

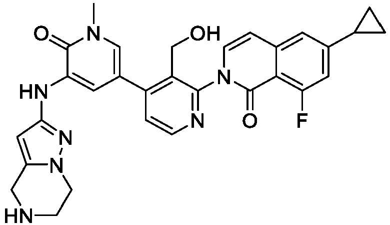

### Step 1: Synthesis of (3-nitro-1H-pyrazol-5-yl)methanol.

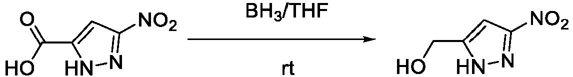

To a solution of 3-nitro-1H-pyrazole-5-carboxylic acid (28.0 g, 178.25 mmol) in THF (280 mL) was added BH₃ (1 M in in THF, 178 mL, 178.00 mmol) dropwise at 0 °C. The resulting solution was stirred at room temperature for 16 h. The reaction was quenched by the addition of 70 mL H₂O and 70 mL 4 *N* HCl cautiously and then stirred at reflux for another 1 h. After cooled to room temperature, the mixture was diluted with H₂O and the aqueous layer was extracted with ethyl acetate. The organic layers were combined, washed with of saturated NaHCO₃ aqueous solution, dried over anhydrous magnesium sulfate and filtered. The filtrate was concentrated under vacuum to afford (3-nitro-1H-pyrazol-5-yl)methanol (21.0 g, crude) as a white solid, which was used for the next step without further purification. MS (ESI) calculated for (C₄H₅N₃O₃) [M+Na]⁺, 166.0; found, 166.1.

### Step 2: Synthesis of (1-(2-bromoethyl)-3-nitro-1H-pyrazol-5-yl)methanol.

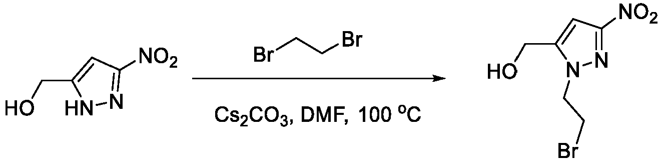

To a solution of (3-nitro-1H-pyrazol-5-yl)methanol (13.0 g, 90.84 mmol) in DMF (200 mL) was added Cs₂CO₃ (38.5 g, 118.10 mmol) in portions at 100 °C during 30 min and then cooled to room temperature. 1,2-dibromoethane (170.7 g, 908.44 mmol) was added to the above solution at room temperature. The resulting solution was stirred at room temperature for 3 h. The reaction was then quenched by the addition of H₂O and the aqueous layer was extracted with ethyl acetate. The organic layers were combined, washed with brine, dried over anhydrous sodium sulfate and filtered. The filtrate was concentrated under vacuum. The residue was purified by flash column chromatography on silica gel with 0~50% ethyl acetate in petroleum ether to afford (1-(2-bromoethyl)-3-nitro-1H-pyrazol-5-yl)methanol (12.0 g, 52%) as a yellow oil. MS (ESI) calculated for (C₆H₈BrN₃O₃) [M+H]⁺, 250.1, 252.1; found, 249.9, 252.1.

### Step 3: Synthesis of 1-(2-bromoethyl)-5-(bromomethyl)-3-nitro-1H-pyrazole.

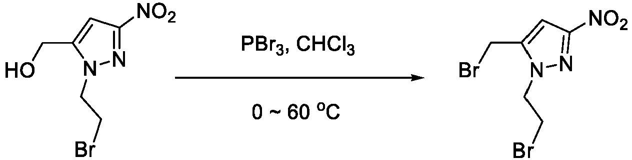

To a solution of (1-(2-bromoethyl)-3-nitro-1H-pyrazol-5-yl)methanol (12.0 g, 48.00 mmol) in CHCl₃ (200mL) was added PBr₃ (13.0 g, 48.00 mmol) dropwise at 0 °C. The resulting solution was stirred at 60 °C for 2 h. The reaction was quenched by the addition of saturated NaHCO₃ aqueous solution and the aqueous layer was extracted with ethyl acetate. The organic layers were combined, washed with brine, dried over anhydrous sodium sulfate and filtered. The filtrate was concentrated under vacuum. The residue was purified by flash column chromatography on silica gel with 0~60% ethyl acetate in petroleum ether to afford 1-(2-bromoethyl)-5-(bromomethyl)-3-nitro-1H-pyrazole (7.2 g, 48%) as a yellow oil. MS (ESI) calculated for (C₆H₇Br₂N₃O₂) [M+H]⁺, 311.9; found, 312.0.

### Step 4: Synthesis of 2-nitro-4,5,6,7-tetrahydropyrazolo[1,5-a]pyrazine.

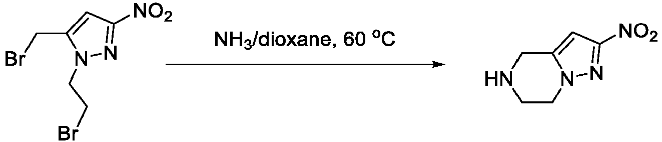

A solution of 1-(2-bromoethyl)-5-(bromomethyl)-3-nitro-1H-pyrazole (7.2 g, 23.01 mmol) in ammonia (0.4 M in dioxane, 403.8 mL, 161.52 mmol) was stirred at 60 °C for 5 h. The solvent was removed under vacuum to afford 2-nitro-4,5,6,7-tetrahydropyrazolo[1,5-a]pyrazine (6.0 g, crude) as a yellow solid, which was used for the next step without further purification. MS (ESI) calculated for (C₆H₈N₄O₂) [M+H]⁺, 169.1; found, 169.0.

### Step 5: Synthesis of tert-butyl 2-nitro-6,7-dihydropyrazolo[1,5-a]pyrazine-5(4H)-carboxylate.

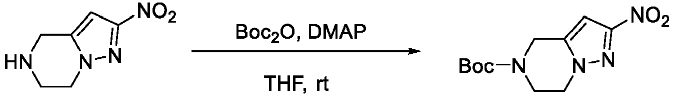

To a solution of 2-nitro-4,5,6,7-tetrahydropyrazolo[1,5-a]pyrazine (3.6 g, 21.41 mmol) in THF (50 mL) were added Boc₂O (5.6 g, 25.69 mmol) and DMAP (0.5 g, 4.28 mmol). The resulting solution was stirred at room temperature for 16 h. The reaction was then diluted with water and the aqueous layer was extracted with ethyl acetate. The organic layers were combined, washed with brine, dried over anhydrous sodium sulfate and filtered. The filtrate was concentrated under vacuum. The residue was purified by flash column chromatography on silica gel with 0~30% ethyl acetate in petroleum ether to afford tert-butyl 2-nitro-6,7-dihydropyrazolo[1,5-a]pyrazine-5(4H)-carboxylate (3.6 g, 62%) as a yellow solid. MS (ESI) calculated for (C₁₁H₁₆N₄O₄) [M+H]⁺, 269.1; found, 269.0.

### Step 6: Synthesis of tert-butyl 2-amino-6,7-dihydropyrazolo[1,5-a]pyrazine-5(4H)-carboxylate.

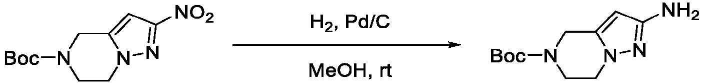

To a solution of tert-butyl 2-nitro-6,7-dihydropyrazolo[1,5-a]pyrazine-5(4H)-carboxylate (3.6 g, 13.42 mmol) in methanol (50 mL) was added Pd/C (dry, 0.72 g) under nitrogen. The resulting solution was stirred at room temperature under H₂ atmosphere (2 atm) for 16 h. The solids were filtered out. The filtrate was concentrated under vacuum to afford tert-butyl 2-amino-6,7-dihydropyrazolo[1,5-a]pyrazine-5(4H)-carboxylate (2.9 g, crude) as a yellow solid, which was used for the next step without further purification. MS (ESI) calculated for (C₁₁H₁₈N₄O₂) [M+H]⁺, 239.1; found,239.2.

### Step 7: Synthesis of tert-butyl 2-(5-bromo-1-methyl-2-oxo-1,2-dihydropyridin-3-ylamino)-6,7-dihydropyrazolo[1,5-a]pyrazine-5(4H)-carboxylate.

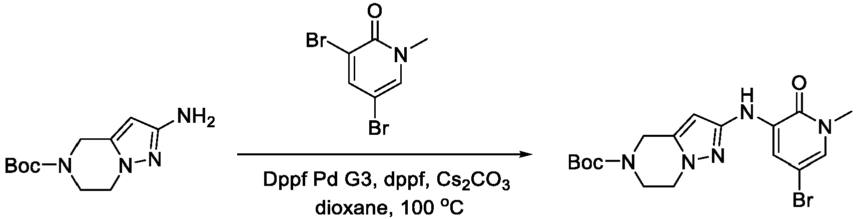

To a degassed solution of tert-butyl 2-amino-6,7-dihydropyrazolo[1,5-a]pyrazine-5(4H)-carboxylate (2.0 g, 8.39 mmol) in dioxane (20 mL) were added 3,5-dibromo-1-methyl-1,2-dihydropyridin-2-one (3.3 g, 12.51 mmol), Dppf (930 mg, 1.68 mmol), Dppf palladium(II) biphenyl-2-amine (775 mg, 0.84 mmol) and Cs₂CO₃ (5.5 g, 16.79 mmol). The mixture was stirred at 100 °C for 16 h under nitrogen. The solvent was removed under vacuum. The mixture was diluted with water and extracted with dichloromethane. The organic layers were combined, washed with brine, dried over anhydrous sodium sulfate and filtered. The filtrate was concentrated under vacuum. The residue was triturated with Et₂O to afford tert-butyl 2-(5-bromo-1-methyl-2-oxo-1,2-dihydropyridin-3-ylamino)-6,7-dihydropyrazolo[1,5-a]pyrazine-5(4H)-carboxylate (3.2 g, 89%) as a brown solid. MS (ESI) calculated for (C₁₇H₂₂BrN₅O₃) [M+H]⁺, 424.1,426.1, found, 424.1,426.1.

### Step 8: Synthesis of tert-butyl 2-(1-methyl-2-oxo-5-(4,4,5,5-tetramethyl-1,3,2-dioxaborolan-2-yl)-1,2-dihydropyridin-3-ylamino)-6,7-dihydropyrazolo[1,5-a]pyrazine-5(4H)-carboxylate.

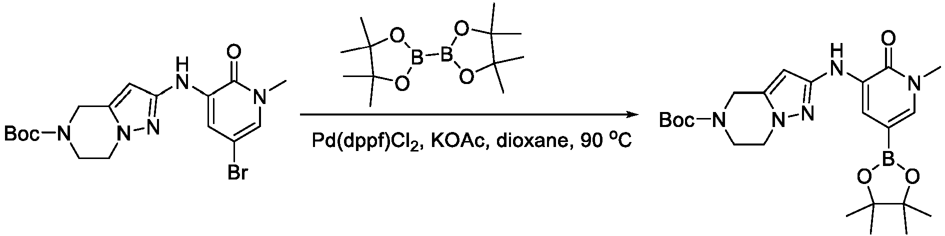

To a degassed solution of tert-butyl 2-[(5-bromo-1-methyl-2-oxo-1,2-dihydropyridin-3-yl)amino]-4H,5H,6H,7H-pyrazolo[1,5-a]pyrazine-5-carboxylate (3 g, 7.07 mmol) in dioxane (30 mL) were added 4,4,5,5-tetramethyl-2-(4,4,5,5-tetramethyl-1,3,2-dioxaborolan-2-yl)-1,3,2-dioxaborolane (2.2 g, 8.51 mmol), KOAc (1.4 g, 14.26 mmol) and Pd(dppf)Cl₂ (520 mg, 0.71 mmol). The mixture was stirred at 100 °C for 16 h under nitrogen. The solvent was removed under vacuum. The residue was purified by flash column chromatography with 0~50% ethyl acetate in dichloromethane to afford tert-butyl 2-(1-methyl-2-oxo-5-(4,4,5,5-tetramethyl-1,3,2-dioxaborolan-2-yl)-1,2-dihydropyridin-3-ylamino)-6,7-dihydropyrazolo[1,5-a]pyrazine-5(4H)-carboxylate (1.8 g, 54%) as a brown solid. MS (ESI) calculated for (C₂₃H₃₄BN₅O₅) [M+H]⁺, 472.3, found, 472.2. ¹H NMR (300 MHz, DMSO-*d*₆) δ 7.99 (s, 1H), 7.90 (d, *J =* 1.8 Hz, 1H), 7.42 (d, *J=* 1.8 Hz, 1H), 5.92 (s, 1H), 4.54 (s, 2H), 4.05 - 3.99 (m, 2H), 3.82 - 3.78 (m, 2H), 3.54 (s, 3H), 1.44 (s, 9H), 1.28 (s, 12H).

### Step 9: Synthesis of 4-bromo-2-fluoro-6-methylbenzamide.

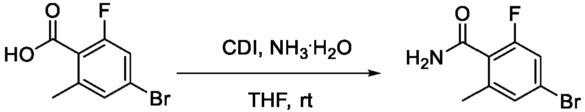

To a solution of 4-bromo-2-fluoro-6-methylbenzoic acid (20.0 g, 85.82 mmol) in THF (200 mL) was added CDI (18.1 g, 111.57 mmol). The resulting solution was stirred at room temperature for 3 h. Then ammonia (40 mL) was added to the above solution and stirred at room temperature for another 3 h. The resulting mixture was concentrated under vacuum. The residue was diluted with H₂O. The solids were collected by filtration and dried under vacuum to afford 4-bromo-2-fluoro-6-methylbenzamide (17.5 g, 87%) as a white solid. MS (ESI) calculated for (C₈H₇BrFNO) [M+H]⁺, 232.0, 234.0; found, 232.1, 234.0.

### Step 10: Synthesis of 4-cyclopropyl-2-fluoro-6-methylbenzamide.

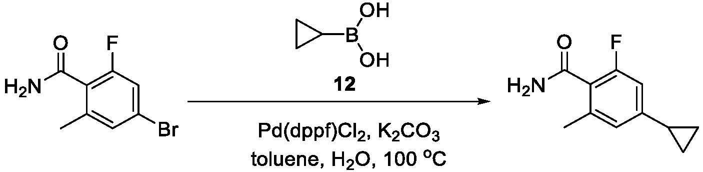

To a degassed solution of 4-bromo-2-fluoro-6-methylbenzamide (20.0 g, 11.85 mmol) in toluene (200 mL) and H₂O (20 mL) were added cyclopropylboronic acid (8.9 g, 103.43 mmol), Pd(dppf)Cl₂ (6.3 g, 8.62 mmol) and K₂CO₃ (35.7 g, 258.56 mmol). The resulting solution was stirred at 100 °C for 4 h under nitrogen. The reaction was quenched by the addition of water and the aqueous layer was extracted with ethyl acetate. The organic layers were combined, washed with brine, dried over anhydrous sodium sulfate and filtered. The filtrate was concentrated under vacuum. The residue was purified by flash column chromatography on silica gel with 0~60% ethyl acetate in petroleum ether to afford 4-cyclopropyl-2-fluoro-6-methylbenzamide (11.0 g, 66%) as a yellow solid. MS (ESI) calculated for (C₁₁H₁₂FNO) [M+H]⁺, 194.1; found,194.0.

### Step 11: Synthesis of (E)-4-cyclopropyl-N-((dimethylamino)methylene)-2-fluoro-6-methylbenzamide.

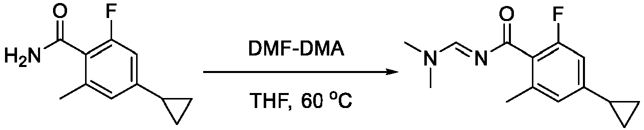

To a solution of 4-cyclopropyl-2-fluoro-6-methylbenzamide (11.0 g, 46.93 mmol) in THF (50 mL) was added DMF-DMA (8.8 g, 74.01 mmol). The resulting solution was stirred at 60 °C for 2 h. The resulting mixture was concentrated under vacuum to afford (E)-4-cyclopropyl-N-((dimethylamino)methylene)-2-fluoro-6-methylbenzamide (15.0 g, crude) as a yellow oil, which was used for the next step without further purification. MS (ESI) calculated for (C₁₄H₁₇FN₂O) [M+H]⁺, 249.1; found, 249.0.

### Step 12: Synthesis of 6-cyclopropyl-8-fluoroisoquinolin-1(2H)-one.

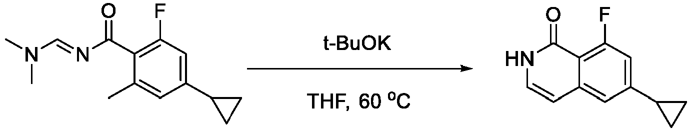

To a solution of (E)-4-cyclopropyl-N-((dimethylamino)methylene)-2-fluoro-6-methylbenzamide (15.0 g, 60.41 mmol) in THF (150 mL) was added t-BuOK (1M in THF, 90.73 mL, 90.73 mmol). The resulting solution was stirred at 60 °C for 2 h. The resulting mixture was concentrated under vacuum. The residue was diluted with H₂O. The precipitated solids were collected by filtration and dried under vacuum to afford 6-cyclopropyl-8-fluoroisoquinolin-1(2H)-one (10.0 g, 81%) as a yellow solid. MS (ESI) calculated for (C₁₂H₁₀FNO) [M+H]⁺, 204.1; found, 204.0.

### Step 13: Synthesis of 4-chloro-2-(6-cyclopropyl-8-fluoro-1-oxoisoquinolin-2(1H)-yl)nicotinaldehyde.

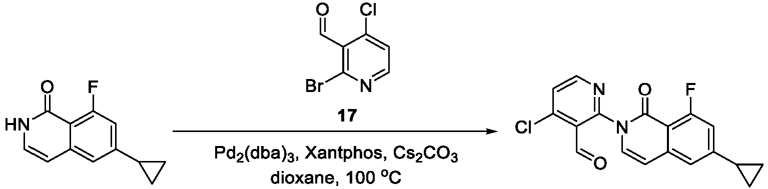

To a degassed solution of 6-cyclopropyl-8-fluoroisoquinolin-1(2H)-one (5.0 g, 24.60 mmol) in dioxane (100 mL) were added 2-bromo-4-chloronicotinaldehyde (10.9 g, 49.21 mmol), Pd₂(dba)₃ (2.3 g, 2.46 mmol), XantPhos (2.9 g, 4.92 mmol) and Cs₂CO₃ (16.0 g, 49.21 mmol). The resulting solution was stirred at 100 °C for 4 h under nitrogen. The reaction was quenched by the addition of water and the aqueous layer was extracted with ethyl acetate. The organic layers were combined, washed with brine, dried over anhydrous sodium sulfate and filtered. The filtrate was concentrated under vacuum. The residue was purified by flash column chromatography on silica gel with 0~10% methanol in dichloromethane to afford 4-chloro-2-(6-cyclopropyl-8-fluoro-1-oxoisoquinolin-2(1H)-yl)nicotinaldehyde (2.3 g, 27%) as a yellow solid. MS (ESI) calculated for (C₁₈H₁₂ClFN₂O₂) [M+H]⁺, 343.1; found, 343.2.

### Step 14: Synthesis of tert-butyl 2-(5-(2-(6-cyclopropyl-8-fluoro-1-oxoisoquinolin-2(1H)-yl)-3-formylpyridin-4-yl)-1-methyl-2-oxo-1,2-dihydropyridin-3-ylamino)-6,7-dihydropyrazolo[1,5-a]pyrazine-5(4H)-carboxylate.

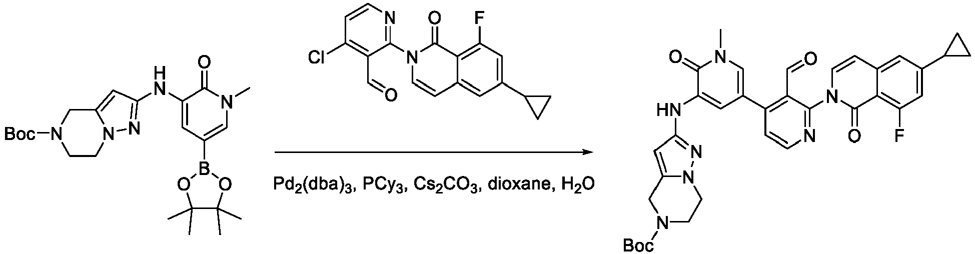

To a degassed solution of tert-butyl 2-(1-methyl-2-oxo-5-(4,4,5,5-tetramethyl-1,3,2-dioxaborolan-2-yl)-1,2-dihydropyridin-3-ylamino)-6,7-dihydropyrazolo[1,5-a]pyrazine-5(4H)-carboxylate (2 g, 4.24 mmol) in dioxane (20 mL) and water (2 mL) were added 4-chloro-2-(6-cyclopropyl-8-fluoro-1-oxo-1,2-dihydroisoquinolin-2-yl)pyridine-3-carbaldehyde (1.6 g, 4.6 mmol), Pd₂(dba)₃ (388 mg, 0.42 mmol), PCy₃ (363 mg, 1.29 mmol) and Cs₂CO₃ (2.77 g, 8.50 mmol). The mixture was stirred at reflux for 16 h under nitrogen. The solvent was removed under vacuum. The residue was purified by flash column chromatography with 0~60% dichloromethane in ethyl acetate to afford tert-butyl 2-(5-(2-(6-cyclopropyl-8-fluoro-1-oxoisoquinolin-2(1H)-yl)-3-formylpyridin-4-yl)-1-methyl-2-oxo-1,2-dihydropyridin-3-ylamino)-6,7-dihydropyrazolo[1,5-a]pyrazine-5(4H)-carboxylate (2 g, 72%) as a brown solid. MS (ESI) calculated for (C₃₅H₃₄FN₇O₅) [M+H]⁺, 652.3, found, 652.3.

### Step 15: Synthesis of tert-butyl 2-(5-(2-(6-cyclopropyl-8-fluoro-1-oxoisoquinolin-2(1H)-yl)-3-(hydroxymethyl)pyridin-4-yl)-1-methyl-2-oxo-1,2-dihydropyridin-3-ylamino)-6,7-dihydropyrazolo[1,5-a]pyrazine-5(4H)-carboxylate.

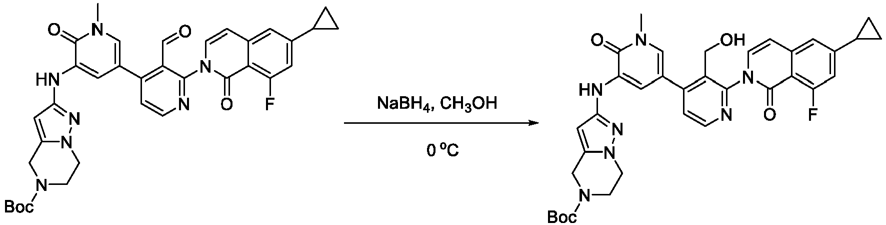

To a solution of tert-butyl 2-(5-(2-(6-cyclopropyl-8-fluoro-1-oxoisoquinolin-2(1H)-yl)-3-formylpyridin-4-yl)-1-methyl-2-oxo-1,2-dihydropyridin-3-ylamino)-6,7-dihydropyrazolo[1,5-a]pyrazine-5(4H)-carboxylate (1.0 g, 1.53 mmol) in methanol (20 mL) was added NaBH₄ (60 mg, 1.58 mmol). The mixture was stirred at 0 °C for 1 h. The reaction was quenched by the addition of water and the aqueous layer was extracted with ethyl acetate. The organic layers were combined, washed with brine, dried over anhydrous sodium sulfate and filtered. The filtrate was concentrated under vacuum. The residue was purified by reverse phase flash column chromatography with 5~55% acetonitrile in water to afford tert-butyl 2-(5-(2-(6-cyclopropyl-8-fluoro-1-oxoisoquinolin-2(1H)-yl)-3-(hydroxymethyl)pyridin-4-yl)-1-methyl-2-oxo-1,2-dihydropyridin-3-ylamino)-6,7-dihydropyrazolo[1,5-a]pyrazine-5(4H)-carboxylate (330 mg, 32%) as an off-white solid. MS (ESI) calculated for (C₃₅H₃₆FN₇O₅) [M+H]⁺, 654.3, found, 654.4. ¹H NMR (300 MHz, DMSO-d6) δ 8.57 (d, *J =* 5.1 Hz, 1H), 8.32 (s, 1H), 8.10 (d, *J* = 2.4 Hz, 1H), 7.51 (d, *J =* 5.1 Hz, 1H), 7.47 - 7.37 (m, 2H), 7.29 (d, *J=* 1.5 Hz, 1H), 7.01 (dd, *J =* 13.2, 1.5 Hz, 1H), 6.64 (dd, *J =* 7.5, 2.1 Hz, 1H), 6.00 (s, 1H), 4.98 (t, *J* = 4.5 Hz, 1H), 4.53 (s, 2H), 4.47 - 4.43 (m, 1H), 4.38 - 4.29 (m, 1H), 3.96 - 3.91 (m, 2H), 3.81 - 3.78 (m, 2H), 3.60 (s, 3H), 2.13 - 2.04 (m, 1H), 1.43 (s, 9H), 1.25 - 0.98 (m, 2H), 0.95 - 0.73 (m, 2H). F NMR (300 MHz, DMSO-d6) δ -111.05.

### Step 16: Synthesis of 6-cyclopropyl-8-fluoro-2-(3'-(hydroxymethyl)-1-methyl-6-oxo-5-((4,5,6,7-tetrahydropyrazolo[1,5-a]pyrazin-2-yl)amino)-1,6-dihydro-[3,4'-bipyridin]-2'-yl)isoquinolin-1(2H)-one.

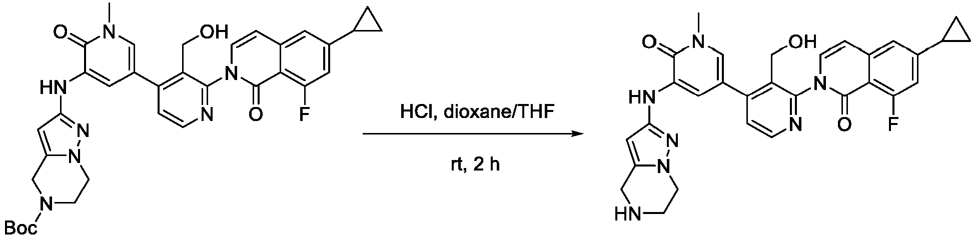

A mixture of tert-butyl 2-{[2'-(6-cyclopropyl-8-fluoro-1-oxoisoquinolin-2-yl)-3'-(hydroxymethyl)-1-methyl-6-oxo-[3,4'-bipyridin]-5-yl]amino}-4H,6H,7H-pyrazolo[1,5-a]pyrazine-5-carboxylate (23.00 mg, 0.04 mmol) , THF (0.2 mL) and hydrogen chloride (4M in dioxane, 0.22 mL, 0.88 mmol) was allowed to stir at r.t. for 1 h. The volatiles were removed to afford 2'-(6-cyclopropyl-8-fluoro-1-oxoisoquinolin-2-yl)-3'-(hydroxymethyl)-1-methyl-5-{4H,5H,6H,7H-pyrazolo[1,5-a]pyrazin-2-ylamino}-[3,4'-bipyridin]-6-one (19.00 mg, 97.5%). LCMS: C₃₀H₂₈FN₇O₃ requires: 553, found: m/z = 554 [M+H]⁺.

### Example 1B: Synthesis of 6-(tert-butyl)-8-fluoro-2-(3'-(hydroxymethyl)-1-methyl-6-oxo-5-((4,5,6,7-tetrahydropyrazolo[1,5-a]pyrazin-2-yl)amino)-1,6-dihydro-[3,4'-bipyridin]-2'-yl)phthalazin-1(2H)-one.

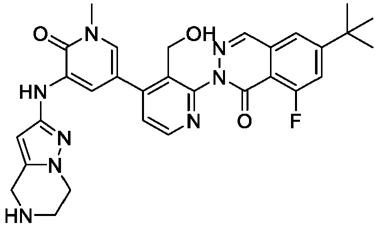

### Step 1: Synthesis of 2-(6-(tert-butyl)-8-fluoro-1-oxophthalazin-2(1H)-yl)-4-iodonicotinaldehyde.

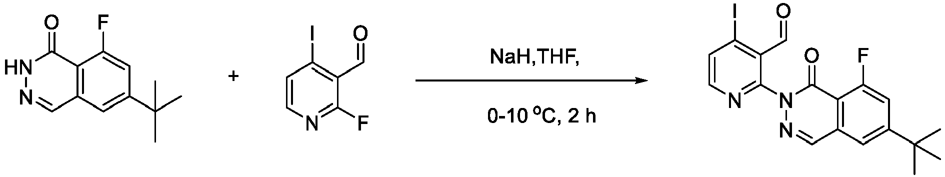

To a solution of 6-tert-butyl-8-fluoro-2H-phthalazin-1-one (1 g, 4.54 mmol, 1 eq) in THF (50 mL) was added NaH (199.78 mg, 4.99 mmol, 60% purity, 1.1 eq) at 0°C. The mixture was stirred at 10 °C for 0.5 hr. 2-fluoro-4-iodo-pyridine-3-carbaldehyde (1.25 g, 4.99 mmol, 1.1 eq) was then added. The mixture was stirred at 10 °C for 2 hr. The mixture was poured into water (30mL), and extracted with ethyl acetate (20 mL*2). The combined organic phase was washed with brine (20 mL), dried with anhydrous Na₂SO₄, and concentrated. The yellow solid was triturated with ethyl acetate (20 mL). 2-(6-tert-butyl-8-fluoro-1-oxo-phthalazin-2-yl)-4-iodo-pyridine-3-carbaldehyde (1.2 g, 2.61 mmol, 57.4% yield) was obtained as yellow solid. LCMS; C₁₈H₁₅FIN₃O₂ requires: 451, found: m/z = 452 [M+H]⁺.

### Step 2: Synthesis of tert-butyl 2-((2'-(6-(tert-butyl)-8-fluoro-1-oxophthalazin-2(1H)-yl)-3'-formyl-1-methyl-6-oxo-1,6-dihydro-[3,4'-bipyridin]-5-yl)amino)-6,7-dihydropyrazolo[1,5-a]pyrazine-5(4H)-carboxylate.

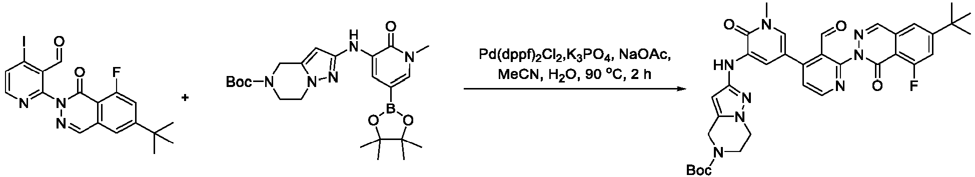

To a solution of tert-butyl 2-[[1-methyl-2-oxo-5-(4,4,5,5-tetramethyl-1,3,2-dioxaborolan-2-yl)-3-pyridyl]amino]-6,7-dihydro-4H-pyrazolo[1,5-a]pyrazine-5-carboxylate (6.5 g, 13.79 mmol, 1 eq), 2-(6tert-butyl-8-fluoro-1-oxo-phthalazin-2-yl)-4-iodopyridine-3-carbaldehyde (6.35 g, 13.79 mmol, 1 eq), K₃PO₄ (5.85 g, 27.58 mmol, 2 eq) and NaOAc (2.26 g, 27.58 mmol, 2 eq) in MeCN (65 mL) and H₂O (2 mL) was added Pd(dppf)Cl₂ (504.51 mg, 689.50 µmol, 0.05 eq) under N₂. The resulting mixture was stirred at 90 °C for 2 hr under N₂. The mixture was diluted with DCM (500 mL), filtered, and concentrated in vacuo. The residue was purified by silica gel chromatography (petroleum ether : ethyl acetate = 1:1 ~ 0:1 (10% DCM)). tert-butyl 2-[[5-[2-(6-tert-butyl-8-fluoro-1-oxo-phthalazin-2-yl)-3-formyl-4-pyridyl]-1-methyl-2-oxo-3-pyridyl]amino]-6,7-dihydro-4H-pyrazolo[1,5-a]pyrazine-5-carboxylate (5.3 g, 7.93 mmol, 57.47% yield) was obtained as a brown solid. LCMS; C₃₅H₃₇FN₈O₅ requires: 668, found: m/z = 669 [M+H]⁺.

### Step 3: Synthesis of tert-butyl 2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazine-5(4H)-carboxylate.

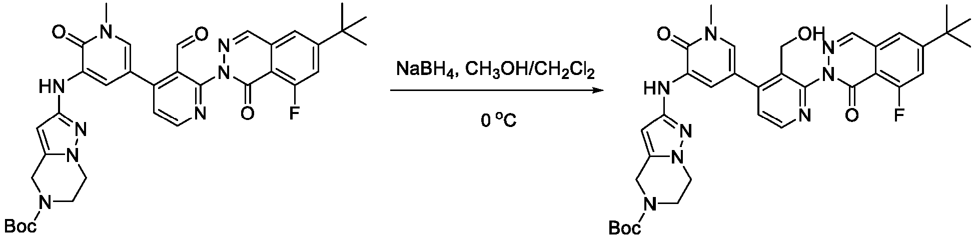

To a solution of tert-butyl 2-[[5-[2-(6-tert-butyl-8-fluoro-1-oxo-phthalazin-2-yl)-3-formyl-4-pyridyl]-1-methyl-2-oxo-3-pyridyl]amino]-6,7-dihydro-4H-pyrazolo[1,5-a]pyrazine-5-carboxylate (5.3 g, 7.93 mmol, 1 eq) in MeOH (25 mL) and DCM (25 mL) was added NaBH₄ (899.54 mg, 23.78 mmol, 3 eq) at 0 °C in portions. The mixture was stirred at 0 °C for 3 hr. The mixture was quenched with H₂O (100 mL), and extracted with DCM (100 mL*2). The combined organic layers were washed with brine (50 mL), dried over Na₂SO₄, and concentrated in vacuo. tert-butyl 2-[[5-[2-(6-tert-butyl-8-fluoro-1-oxo-phthalazin-2-yl)-3-(hydroxymethyl)-4-pyridyl]-1-methyl-2-oxo-3-pyridyl]amino]-6,7-dihydro-4H-pyrazolo[1,5-a]pyrazine-5-carboxylate (5.4 g, 7.08 mmol, 89.39% yield) was obtained as a brown solid. LCMS; C₃₅H₃₉FN₈O₅ requires: 670, found: m/z = 671 [M+H]⁺.

### Step 4: Synthesis of 6-(tert-butyl)-8-fluoro-2-(3'-(hydroxymethyl)-1-methyl-6-oxo-5-((4,5,6,7-tetrahydropyrazolo[1,5-a]pyrazin-2-yl)amino)-1,6-dihydro-[3,4'-bipyridin]-2'-yl)phthalazin-1(2H)-one.

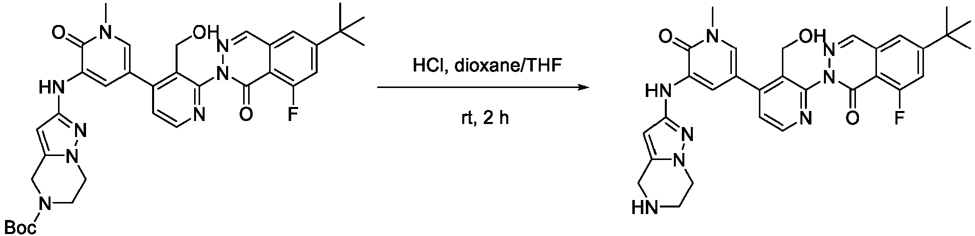

A mixture of tert-butyl 2-[[5-[2-(6-tert-butyl-8-fluoro-1-oxo-phthalazin-2-yl)-3-(hydroxymethyl)-4-pyridyl]-1-methyl-2-oxo-3-pyridyl]amino]-6,7-dihydro-4H-pyrazolo[1,5-a]pyrazine-5-carboxylate (6.39 g, 8.38 mmol, 1 eq) and HCl/dioxane (4 M, 26.40 mL, 12.60 eq) in DCM (30 mL) was stirred at 15 °C for 2 hr. The mixture was filtered to give a solid. The solid was purified by prep-HPLC (23-53% MeCN in H₂O with 0.05% HCl). 6-tert-butyl-8-fluoro-2-[3-(hydroxymethyl)-4-[1-methyl-6-oxo-5-(4,5,6,7-tetrahydropyrazolo[1,5-a]pyrazin-2-ylamino)-3-pyridyl]-2-pyridyl]phthalazin-1-one (2.88 g, 4.46 mmol, 53.23% yield, 2HCl) was obtained as an orange solid. LCMS; C₃₀H₃₁FN₈O₃ requires: 570, found: m/z = 571 [M+H]⁺.

### Example 1C: Synthesis of 3-(4-bromo-1-oxoisoindolin-2-yl)piperidine-2,6-dione.

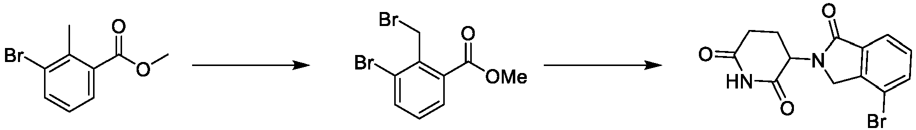

### Step 1: Synthesis of methyl 3-bromo-2-(bromomethyl)benzoate.

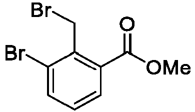

To a solution of methyl 3-bromo-2-methyl-benzoate (50 g, 218.27 mmol, 1 *eq),* NBS (46.62 g, 261.93 mmol, 1.2 *eq)* in CHCl₃ (400 mL) was added AIBN (3.58 g, 21.83 mmol, 0.1 *eq).* The mixture was stirred at 70 °C for 12 h. The reaction mixture was concentrated in vacuum, diluted with DCM (400 mL), washed with H₂O (100 mL) and brine (100 mL), extracted with DCM (100 mL), and washed with brine (50 mL) again. The organic phase was combined, dried over Na₂SO₄, and concentrated in vacuum. The residue was purified by flash silica gel chromatography (Petroleum ether/Ethyl acetate = 100/1) to yield 3-bromo-2-(bromomethyl)benzoate (63 g, 204.57 mmol, 93.72% yield) as a light yellow solid.

### Step 2: Synthesis of 3-(4-bromo-1-oxo-isoindolin-2-yl)piperidine-2,6-dione.

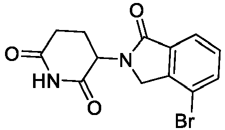

To a solution of methyl 3-bromo-2-(bromomethyl)benzoate (88.2 g, 286.39 mmol, 1 *eq)* in ACN (600 mL) was added DIEA (49.23 g, 380.91 mmol, 66.35 mL, 1.33 *eq)* and 3-aminopiperidine-2,6-dione hydrochloride (51.01 g, 309.94 mmol, 1.08 *eq).* The mixture was stirred at 80 °C for 16 hr. The reaction mixture was filtered. The filter cake was triturated by a mixture solution (EtOAc : H₂O = 100 mL : 200 mL) to yield 3-(4-bromo-1-oxo-isoindolin-2-yl)piperidine-2,6-dione (56.5 g, 174.85 mmol, 61.05% yield) as a purple powder.

### Example 1D: Synthesis of tert-butyl ((S)-1-(((S)-1-cyclohexyl-2-((S)-2-(4-(3-hydroxybenzoyl)thiazol-2-yl)pyrrolidin-1-yl)-2-oxoethyl)amino)-1-oxopropan-2-yl)(methyl)carbamate.

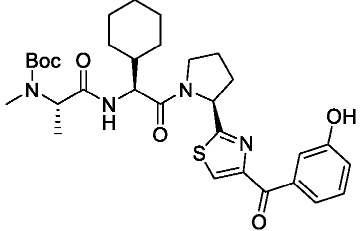

### Step 1: Synthesis of methyl (S)-2-((S)-2-((tert-butoxycarbonyl)(methyl)amino)propanamido)-2-cyclohexylacetate.

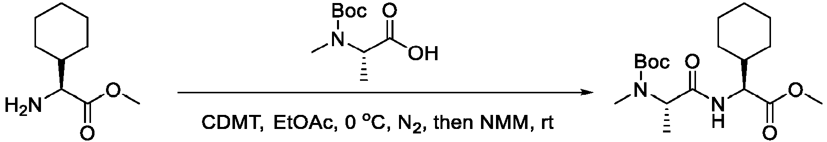

A solution of (S)-methyl-2-amino cyclohexyl acetate hydrochloride (70.0 g, 0.34 mol) and (S)-2-(tert-butoxycarbonyl(methyl)amino)propanoic acid (69.0 g, 0.34 mol) in ethyl acetate (300 mL) was treated with 2-chloro-4,6-dimethoxy-1,3,5-triazine (CDMT) (64.7 g, 0.37 mol) under nitrogen. The reaction mixture was cooled to 0 °C and treated with N-methylmorpholine (85.8 g, 0.85 mol). The reaction mixture was warmed to room temperature and stirred for 4 h. The solid precipitate was filtered out and rinsed with ethyl acetate. The filtrate was washed with saturated NaHCO₃ aqueous solution and then 10% citric acid and brine, dried over anhydrous sodium sulfate and concentrated under vacuum to afford methyl (S)-2-((S)-2-((tert-butoxycarbonyl)(methyl)amino)propanamido)-2-cyclohexylacetate (85.0 g, 71%) as an off-white solid. MS (ESI) calculated for (C₁₈H₃₂N₂O₅) [M+H]⁺, 357.2; found, 357.0.

### Step 2: Synthesis of (S)-2-((S)-2-((tert-butoxycarbonyl)(methyl)amino)propanamido)-2-cyclohexylacetic acid.

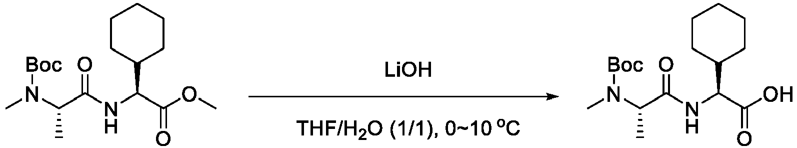

To a solution of methyl (S)-2-((S)-2-((tert-butoxycarbonyl)(methyl)amino)propanamido)-2-cyclohexylacetate (85.0 g, 0.24 mol) in THF (1.2 L) was added a solution of LiOH.H₂O (25.2 g, 0.60 mol) in water (1.2 L) maintained the temperature at 0~10 °C under nitrogen. The resulting mixture was stirred at 0~10 °C for 3 h. The organic solvent was removed under vacuum and the pH value of aqueous phase was adjusted to ~3 by citric acid. The mixture was extracted with ethyl acetate twice. The combined organic layer was washed with brine, dried over anhydrous sodium sulfate and concentrated under vacuum to afford (S)-2-((S)-2-((tert-butoxycarbonyl)(methyl)amino)propanamido)-2-cyclohexylacetic acid (100 g, crude) as colorless oil, which was used for the next step without further purification. MS (ESI) calculated for (C₁₇H₃₀N₂O₅) [M-H]⁻, 341.2; found, 341.0.

### Step 3: Synthesis of tert-butyl (S)-2-carbamothioylpyrrolidine-1-carboxylate.

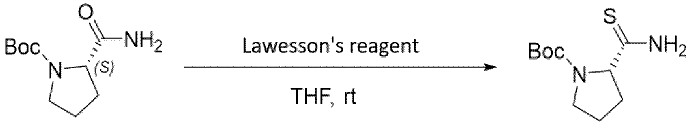

To a solution of tert-butyl (2S)-2-carbamoylpyrrolidine-1-carboxylate (100 g, 466.72 mmol) in tetrahydrofuran (1.2 L) was added Lawesson's reagent (113 g, 279.70 mmol). The resulting mixture was stirred at room temperature for 16 h. The mixture was then diluted with saturated NaHCO₃ aqueous solution and extracted with ethyl acetate. The combined organic layer was washed with brine, dried over anhydrous sodium sulfate and concentrated under vacuum to afford tert-butyl (S)-2-carbamothioylpyrrolidine-1-carboxylate (110 g, crude) as a white solid, which was used for the next step without further purification. MS (ESI) calculated for (C₁₀H₁₈N₂O₂S) [M+H]⁺, 231.1; found, 231.0.

### Step 4: Synthesis of ethyl (S)-2-(1-(tert-butoxycarbonyl)pyrrolidin-2-yl)thiazole-4-carboxylate.

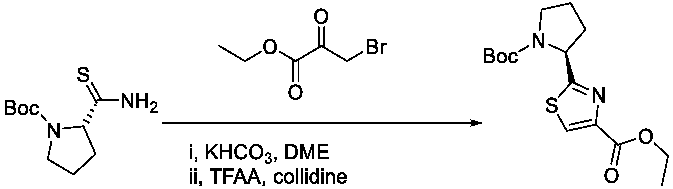

To a mixture of tert-butyl (S)-2-carbamothioylpyrrolidine-1-carboxylate (100.0 g, 0.44 mol) and potassium bicarbonate (348.0 g, 3.48 mol) in dimethoxyethane (1.5 L) was added ethyl 3-bromo-2-oxopropanoate (253.1 g, 1.30 mol) dropwise at room temperature. The resulting mixture was stirred at room temperature for 1 h and then cooled to 0 °C. And then trifluoroacetic acid (365.4 g, 1.74 mol) and collidine (298.2 g, 2.78 mol) were added dropwise to the above solution at 0 °C. The resulting mixture was stirred at room temperature for 8 h. The reaction was quenched by the addition of water and the aqueous phase was extracted with dichloromethane. The combined organic layer was washed with HCl (0.5 *N*) and brine, dried over anhydrous sodium sulfate and concentrated under vacuum. The crude residue was purified by flash column chromatography with 10~30% ethyl acetate in petroleum ether to afford ethyl (S)-2-(1-(tert-butoxycarbonyl)pyrrolidin-2-yl)thiazole-4-carboxylate (51.5 g, 34% over two steps) as a brown solid. MS (ESI) calculated for (C₁₅H₂₂N₂O₄S) [M+H]⁺, 327.1; found, 327.0.

### Step 5: Synthesis of (S)-2-(1-(tert-butoxycarbonyl)pyrrolidin-2-yl)thiazole-4-carboxylic acid.

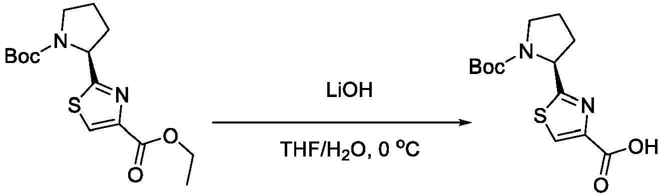

To a mixture of ethyl (S)-2-(1-(tert-butoxycarbonyl)pyrrolidin-2-yl)thiazole-4-carboxylate (51.5 g, 0.16 mol) in THF (300 mL) and water (200 mL) was added a solution of lithium hydroxide hydrate (26.5 g, 0.63 mol) in water (100 mL) dropwise at 0 °C. The resulting mixture was stirred at 0 °C for 5 h. The organic layer was removed under vacuum. The residue was diluted with 200 mL of water and the pH value was adjusted to 3 by HCl (6 *N*). The solution was extracted with ethyl acetate. The combined organic layer was washed with brine, dried over anhydrous sodium sulfate and concentrated under vacuum to afford (S)-2-(1-(tert-butoxycarbonyl)pyrrolidin-2-yl)thiazole-4-carboxylic acid (45.0 g, 95%) as a light brown solid. MS (ESI) calculated for (C₁₃H₁₈N₂O₄S) [M-H]⁻, 297.1; found, 297.0.

### Step 6: Synthesis of tert-butyl (S)-2-(4-(methoxy(methyl)carbamoyl)thiazol-2-yl)pyrrolidine-1-carboxylate.

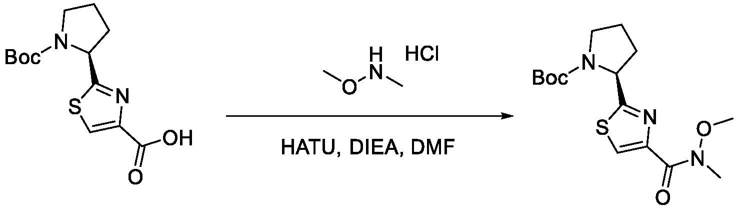

A mixture of (S)-2-(1-(tert-butoxycarbonyl)pyrrolidin-2-yl)thiazole-4-carboxylic acid (90.0 g, 0.30 mol), methoxy(methyl)amine hydrogen chloride (43.6 g, 0.45 mol), HATU (114.0 g, 0.30 mol) and DIEA (96.7 g, 0.75 mol) in DMF (500 mL) was stirred at room temperature for 16 h. The mixture was diluted with water and the aqueous phase was extracted with ethyl acetate. The combined organic layer was washed with brine, dried over anhydrous sodium sulfate and concentrated under vacuum. The crude residue was purified by flash column chromatography with 40~80% ethyl acetate in petroleum ether to afford tert-butyl (S)-2-(4-(methoxy(methyl)carbamoyl)thiazol-2-yl)pyrrolidine-1-carboxylate (60.0 g, 59%) as a light yellow oil. MS (ESI) calculated for (C₁₅H₂₃N₃O₄S) [M+H]⁺, 342.1; found, 342.0.

### Step 7: Synthesis of tert-butyl (S)-2-(4-(3-methoxybenzoyl)thiazol-2-yl)pyrrolidine-1-carboxylate.

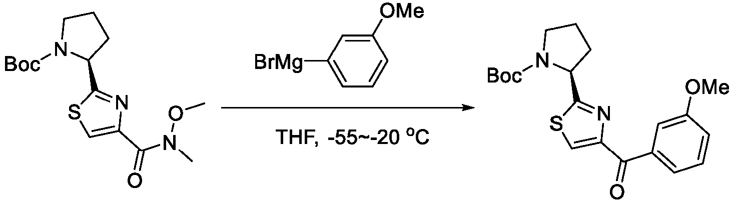

To a solution of tert-butyl (S)-2-(4-(methoxy(methyl)carbamoyl)thiazol-2-yl)pyrrolidine-1-carboxylate (30.0 g, 88.0 mmol) in anhydrous THF (300 mL) was added (3-methoxyphenyl)magnesium bromide (1M in THF, 530 mL, 0.53 mol) dropwise at -55 °C under nitrogen. The resulting mixture was stirred for 4 h below -20 °C. The reaction was then quenched by the addition of saturated NH₄Cl aqueous solution at 0 °C cautiously. The mixture was extracted with ethyl acetate. The combined organic layer was washed with brine, dried over anhydrous sodium sulfate and concentrated under vacuum. The crude residue was purified by flash column chromatography with 10~50% ethyl acetate in petroleum ether to afford tert-butyl (S)-2-(4-(3-methoxybenzoyl)thiazol-2-yl)pyrrolidine-1-carboxylate (24 g, 70%) as light yellow oil. MS (ESI) calculated for (C₂₀H₂₄N₂O₄S) [M+H]⁺, 389.1; found, 389.0.

### Step 8: Synthesis of (S)-(3-methoxyphenyl)(2-(pyrrolidin-2-yl)thiazol-4-yl)methanone HCl salt.

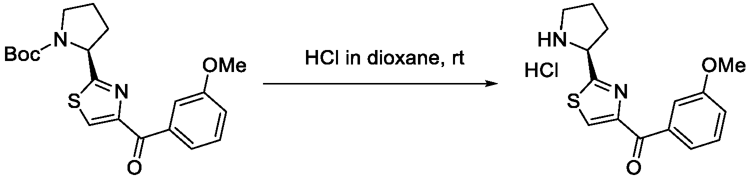

A mixture of tert-butyl (S)-2-(4-(3-methoxybenzoyl)thiazol-2-yl)pyrrolidine-1-carboxylate (24 g, 61.8 mmol) in HCl (4 M in dioxane, 200 mL) was stirred at room temperature for 2 h. The solvent was removed under vacuum to afford (S)-(3-methoxyphenyl)(2-(pyrrolidin-2-yl)thiazol-4-yl)methanone HCl salt (26 g, crude) as yellow oil, which was used for the next step without further purification. MS (ESI) calculated for (C₂₀H₁₆N₂O₂S) [M+H]⁺, 289.1; found, 289.0.

### Step 9: Synthesis of tert-butyl ((S)-1-(((S)-1-cyclohexyl-2-((S)-2-(4-(3-methoxybenzoyl)thiazol-2-yl)pyrrolidin-1-yl)-2-oxoethyl)amino)-1-oxopropan-2-yl)(methyl)carbamate.

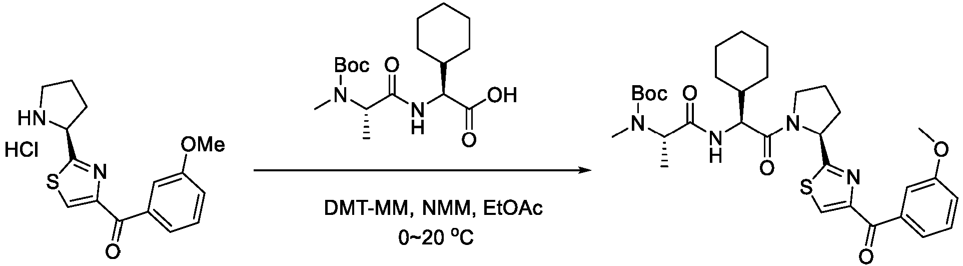

To a solution of 4-[(3-methoxyphenyl)carbonyl]-2-[(2S)-pyrrolidin-2-yl]-1,3-thiazole (25 g, 86.70 mmol) and (2S)-2-[(2S)-2-[[(tert-butoxy)carbonyl](methyl)amino]propanamido]-2-cyclohexylacetic acid (29.7 g, 86.73 mmol) in ethyl acetate (400 mL) were added 4-(4,6-dmethoxy-1,3,5-triazine-2-yl)-4-methyl morpholinium chloride (DMT-MM) (26.35 g, 95.47 mmol) and 4-methylmorpholine (21.9 g, 216.83 mmol) at 0 °C. The resulting mixture was stirred at room temperature for 3 h. The reaction was then quenched by the addition of water and the aqueous phase was extracted with ethyl acetate. The combined organic layer was washed with brine, dried over anhydrous sodium sulfate and concentrated under vacuum. The crude residue was purified by flash column chromatography with 0~30% ethyl acetate in petroleum ether to afford tert-butyl ((S)-1-(((S)-1-cyclohexyl-2-((S)-2-(4-(3-methoxybenzoyl)thiazol-2-yl)pyrrolidin-1-yl)-2-oxoethyl)amino)-1-oxopropan-2-yl)(methyl)carbamate (24 g, 46%) as light yellow oil. MS (ESI) calculated for (C₃₂H₄₄N₄O₆S) [M+H]⁺, 613.3; found, 613.0.

### Step 10: Synthesis of (S)-N-((S)-1-cyclohexyl-2-((S)-2-(4-(3-hydroxybenzoyl)thiazol-2-yl)pyrrolidin-1-yl)-2-oxoethyl)-2-(methylamino)propanamide.

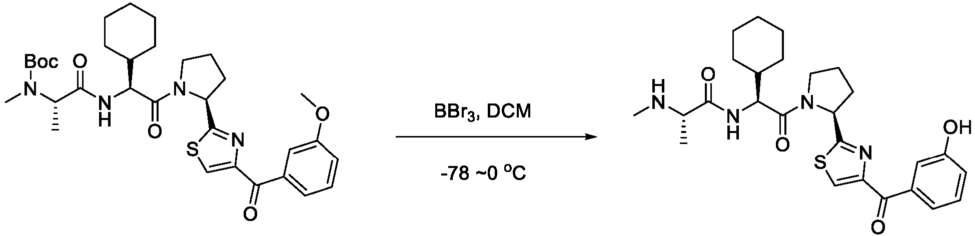

To a solution of tert-butyl ((S)-1-(((S)-1-cyclohexyl-2-((S)-2-(4-(3-methoxybenzoyl)thiazol-2-yl)pyrrolidin-1-yl)-2-oxoethyl)amino)-1-oxopropan-2-yl)(methyl)carbamate (9.0 g, 14.69 mmol) in dichloromethane (120 mL) was added BBr₃ (10.9 g, 44.1 mmol) dropwise at -78 °C. The resulting mixture was stirred below 0 °C for 4 h under nitrogen. The reaction was then quenched by the addition of water cautiously and the aqueous phase was extracted with dichloromethane. The combined organic layer was washed with brine, dried over anhydrous sodium sulfate and concentrated under vacuum to afford (S)-N-((S)-1-cyclohexyl-2-((S)-2-(4-(3-hydroxybenzoyl)thiazol-2-yl)pyrrolidin-1-yl)-2-oxoethyl)-2-(methylamino)propanamide (9 g, crude) as light brown oil, which was used for the next step without further purification. MS (ESI) calculated for (C₂₆H₃₄N₄O₄S) [M+H]⁺, 499.2; found, 499.0.

### Step 11: Synthesis of tert-butyl ((S)-1-(((S)-1-cyclohexyl-2-((S)-2-(4-(3-hydroxybenzoyl)thiazol-2-yl)pyrrolidin-1-yl)-2-oxoethyl)amino)-1-oxopropan-2-yl)(methyl)carbamate.

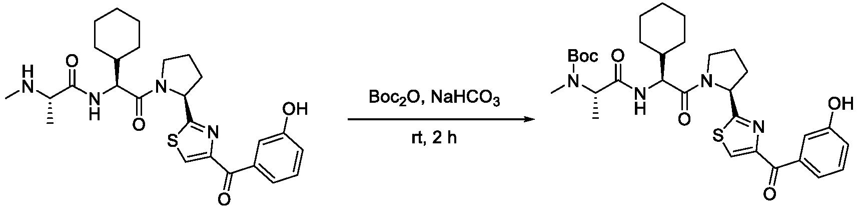

To a solution of (S)-N-((S)-1-cyclohexyl-2-((S)-2-(4-(3-hydroxybenzoyl)thiazol-2-yl)pyrrolidin-1-yl)-2-oxoethyl)-2-(methylamino)propanamide (10 g, 20.05 mmol) and sodium bicarbonate (3.6 g, 43.21 mmol) in dioxane (120 mL) was added a solution of Boc₂O (5.6 g, 25.48 mmol) in dioxane (30 mL) dropwise at 0 °C. The mixture was stirred at room temperature for 2 h. The reaction was diluted with water and the aqueous phase was extracted with ethyl acetate. The combined organic layer was washed with brine, dried over anhydrous sodium sulfate and concentrated under vacuum. The crude residue was purified by flash column chromatography with 10~50% ethyl acetate in petroleum ether to afford tert-butyl ((S)-1-(((S)-1-cyclohexyl-2-((S)-2-(4-(3-hydroxybenzoyl)thiazol-2-yl)pyrrolidin-1-yl)-2-oxoethyl)amino)-1-oxopropan-2-yl)(methyl)carbamate (5.2 g, 59% over 2 steps) as light yellow oil. MS (ESI) calculated for (C₃₁H₄₂N₄O₆S) [M+H]⁺, 599.3; found, 599.3. ¹H NMR (300 MHz, Chloroform-d) δ 8.60 (br, 1H), 8.09 (d, *J =* 2.0 Hz, 1H), 7.78 - 7.54 (m, 2H), 7.38 - 7.34 (m, 1H), 7.11 - 7.08 (m, 1H), 6.79 (br, 1H), 5.68 - 5.47 (m, 1H), 4.85 - 4.64 (m, 2H), 4.00 - 3.59 (m, 2H), 2.80 (s, 3H), 2.58 - 2.09 (m, 4H), 1.87 - 1.58 (m, 6H), 1.50 (s, 9H), 1.36 (d, *J=* 7.1 Hz, 3H), 1.18 - 0.81 (m, 5H).

### Example 1E: Synthesis of tert-butyl (S)-1-((S)-2-((2S,4S)-4-amino-2-((R)-1,2,3,4-tetrahydronaphthalen-1-ylcarbamoyl)pyrrolidin-1-yl)-1-cyclohexyl-2-oxoethylamino)-1-oxopropan-2-yl(methyl)carbamate.

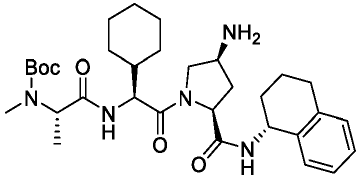

### Step 1: Synthesis of (2S,4S)-tert-butyl 4-(((9H-fluoren-9-yl)methoxy)carbonylamino)-2-((R)-1,2,3,4-tetrahydronaphthalen-1-ylcarbamoyl)pyrrolidine-1-carboxylate.

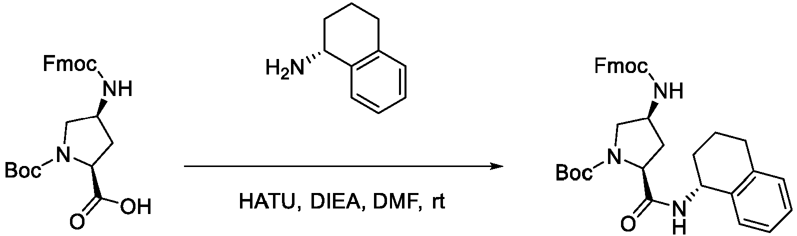

To a solution of (2S,4S)-4-(((9H-fluoren-9-yl)methoxy)carbonylamino)-1-(tert-butoxycarbonyl)pyrrolidine-2-carboxylic acid (10 g, 22.2 mmol), (R)-1,2,3,4-tetrahydronaphthalen-1-amine (3.26 g, 22.2 mmol) and DIEA (14.28 g, 111 mmol) in DMF (100 mL) was added HATU (9.26 g, 24.4 mmol). The solution was stirred at room temperature for 3 h. The reaction was quenched by the addition of 200 mL H₂O and then extracted with ethyl acetate (200 mL x 3). The combined organic layer was washed with brine, dried over anhydrous Na₂SO₄ and concentrated under vacuum. The crude residue was purified by flash column chromatography with 10~50% ethyl acetate in petroleum ether to afford (2S,4S)-tert-butyl 4-(((9H-fluoren-9-yl)methoxy)carbonylamino)-2-((R)-1,2,3,4-tetrahydronaphthalen-1-ylcarbamoyl)pyrrolidine-1-carboxylate (12.0 g, 93%) as a white solid. MS (ESI) calculated for (C₃₅H₃₉N₃O₅) [M+H]⁺, 582.3; found, 582.0.

Step 2: Synthesis of (9H-fluoren-9-yl)methyl ((3S,5S)-5-(((R)-1,2,3,4-tetrahydronaphthalen-1-yl)carbamoyl)pyrrolidin-3-yl)carbamate TFA salt.

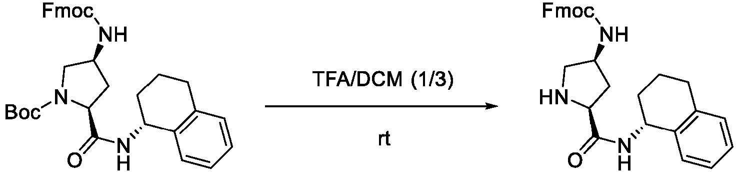

To a stirred solution of (2S,4S)-tert-butyl 4-(((9H-fluoren-9-yl)methoxy)carbonylamino)-2-((R)-1,2,3,4-tetrahydronaphthalen-1-ylcarbamoyl)pyrrolidine-1-carboxylate (12 g, 26.54 mmol) in DCM (120 mL) was added TFA (40 mL) at room temperature. The resulting mixture was stirred at room temperature overnight. The solvent was removed under vacuum to afford (9H-fluoren-9-yl)methyl ((3S,5S)-5-(((R)-1,2,3,4-tetrahydronaphthalen-1-yl)carbamoyl)pyrrolidin-3-yl)carbamate TFA salt (13 g, crude) as yellow oil, which was used for the next step without further purification. MS (ESI) calculated for (C₃₀H₃₁N₃O₃) [M+H]⁺, 482.2; found, 482.0.

### Step 3: Synthesis of 9H-fluoren-9-ylmethyl N-[(3S,5S)-1-[(2S)-2-[[(tert-butoxy)carbonyl]amino]-2-cyclohexylacetyl]-5-[[(1R)-1,2,3,4-tetrahydronaphthalen-1-yl]carbamoyl]pyrrolidin-3-yl]carbamate.

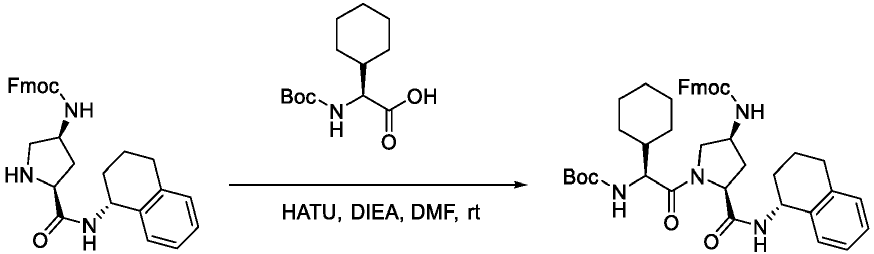

To a stirred solution of (9H-fluoren-9-yl)methyl (3S,5S)-5-((R)-1,2,3,4-tetrahydronaphthalen-1-ylcarbamoyl)pyrrolidin-3-ylcarbamate TFA salt (13 g, 27.0 mmol), DIEA (17.44 g, 135 mmol) and (S)-2-(tert-butoxycarbonylamino)-2-cyclohexylacetic acid (6.95 g, 27.0 mmol) in DMF (150 mL) was added HATU (12.33 g, 32.4 mmol). The resulting mixture was stirred at room temperature for 4 h. The reaction was quenched by the addition of 200 mL H₂O and extracted with ethyl acetate (200 mL x 3). The combined organic layer was washed with brine, dried over anhydrous Na₂SO₄ and concentrated under vacuum. The crude residue was purified by flash column chromatography with 10~40% ethyl acetate in petroleum ether to afford 9H-fluoren-9-ylmethyl N-[(3S,5S)-1-[(2S)-2-[[(tert-butoxy)carbonyl]amino]-2-cyclohexylacetyl]-5-[[(1R)-1,2,3,4-tetrahydronaphthalen-1-yl]carbamoyl]pyrrolidin-3-yl]carbamate (5.2 g, 27%) as colorless oil. MS (ESI) calculated for (C₄₃H₅₂N₄O₆) [M+H]⁺, 721.4; found, 721.0.

### Step 4: Synthesis of (9H-fluoren-9-yl)methyl (3S,5S)-1-((S)-2-amino-2-cyclohexylacetyl)-5-((R)-1,2,3,4-tetrahydronaphthalen-1-ylcarbamoyl)pyrrolidin-3-ylcarbamate TFA salt.

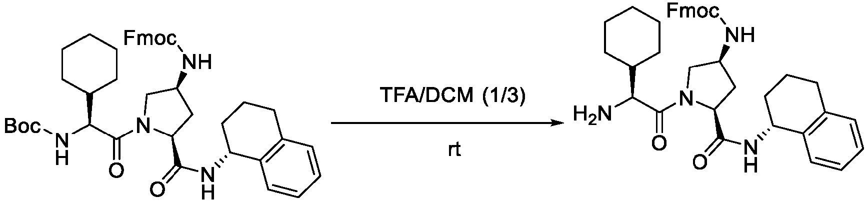

To a stirred solution of 9H-fluoren-9-ylmethyl N-[(3S,5S)-1-[(2S)-2-[[(tert-butoxy)carbonyl]amino]-2-cyclohexylacetyl]-5-[[(1R)-1,2,3,4-tetrahydronaphthalen-1-yl]carbamoyl]pyrrolidin-3-yl]carbamate (5.2 g, 7.22 mmol) in DCM (90 mL) was added TFA (30 mL). The solution was stirred at room temperature overnight. The solvents were removed under vacuum to afford (9H-fluoren-9-yl)methyl (3S,5S)-1-((S)-2-amino-2-cyclohexylacetyl)-5-((R)-1,2,3,4-tetrahydronaphthalen-1-ylcarbamoyl)pyrrolidin-3-ylcarbamate TFA salt (4.48 g, crude) as yellow oil. MS (ESI) calculated for (C₃₈H₄₄N₄O₄) [M+H]⁺, 621.3; found, 621.0.

### Step 5: Synthesis of 9H-fluoren-9-ylmethyl N-[(3S,5S)-1-[(2S)-2-[(2S)-2-[[(tert-butoxy)carbonyl](methyl)amino]propanamido]-2-cyclohexylacetyl]-5-[[(1R)-1,2,3,4-tetrahydronaphthalen-1-yl]carbamoyl]pyrrolidin-3-yl]carbamate.

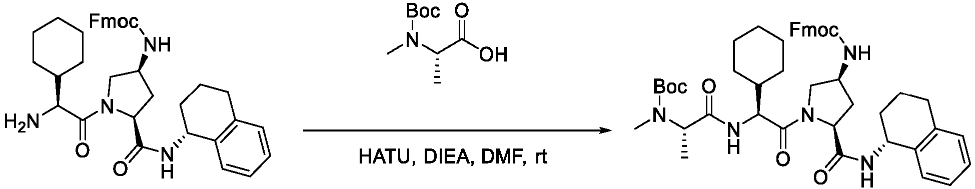

To a stirred solution of (9H-fluoren-9-yl)methyl (3S,5S)-1-((S)-2-amino-2-cyclohexylacetyl)-5-((R)-1,2,3,4-tetrahydronaphthalen-1-ylcarbamoyl)pyrrolidin-3-ylcarbamate (4.48 g, 7.22 mmol), DIEA (4.66 g, 36.1 mmol) and (S)-2-(tert-butoxycarbonyl(methyl)amino)propanoic acid (1.46 g, 7.22 mmol) in DMF (50 mL) was added HATU (3.3 g, 8.68 mmol). The resulting mixture was stirred at room temperature for 3 h. The reaction was quenched by the addition of 100 mL H₂O and extracted with ethyl acetate (100 mL x 3). The combined organic layer was washed with brine, dried over anhydrous Na₂SO₄ and concentrated under vacuum. The crude residue was purified by flash column chromatography with 20~60% ethyl acetate in petroleum ether to afford 9H-fluoren-9-ylmethyl N-[(3S,5S)-1-[(2S)-2-[(2S)-2-[[(tert-butoxy)carbonyl](methyl)amino]propanamido]-2-cyclohexylacetyl]-5-[[(1R)-1,2,3,4-tetrahydronaphthalen-1-yl]carbamoyl]pyrrolidin-3-yl]carbamate (5.2 g, 89%) as colorless oil. MS (ESI) calculated for (C₄₇H₅₉N₅O₇) [M+H]⁺, 806.4; found, 806.0.

### Step 6: Synthesis of tert-butyl (S)-1-((S)-2-((2S,4S)-4-amino-2-((R)-1,2,3,4-tetrahydronaphthalen-1-ylcarbamoyl)pyrrolidin-1-yl)-1-cyclohexyl-2-oxoethylamino)-1-oxopropan-2-yl(methyl)carbamate.

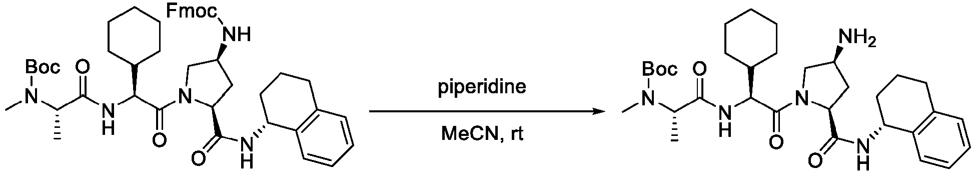

To a stirred solution of 9H-fluoren-9-ylmethyl N-[(3S,5S)-1-[(2S)-2-[(2S)-2-[[(tert-butoxy)carbonyl](methyl)amino]propanamido]-2-cyclohexylacetyl]-5-[[(1R)-1,2,3,4-tetrahydronaphthalen-1-yl]carbamoyl]pyrrolidin-3-yl]carbamate (5.2 g, 6.46 mmol) in acetonitrile (80 mL) was added piperidine (5.2 mL). The mixture was stirred at room temperature for 1 h. The solids were filtered out by filtration and the filtrate was concentrated under vacuum. The crude residue was purified by reverse phase flash column chromatography with 5~95% acetonitrile in water to afford tert-butyl (S)-1-((S)-2-((2S,4S)-4-amino-2-((R)-1,2,3,4-tetrahydronaphthalen-1-ylcarbamoyl)pyrrolidin-1-yl)-1-cyclohexyl-2-oxoethylamino)-1-oxopropan-2-yl(methyl)carbamate (3.1656 g, 84%) as a white solid. ¹H NMR (300 MHz, DMSO-d6) δ 8.45 - 8.12 (m, 1H), 7.71 (m, 1H), 7.39 - 6.99 (m, 4H), 4.94 - 4.91 (m, 1H), 4.61 - 4.45 (m, 1H), 4.34 - 4.19 (m, 2H), 3.90 - 3.88 (m, 1H), 3.29 - 3.16 (m, 1H), 2.75 - 2.72 (m, 5H), 2.50 - 2.27 (m, 1H), 2.01 - 1.82 (m, 4H), 1.81 - 1.50 (m, 9H), 1.41 (s, 9H), 1.29 - 0.85 (m, 9H). MS (ESI) calculated for (C₃₂H₄₉N₅O₅) [M+H]⁺, 584.4; found, 584.4.

### Example 2: General procedure for the synthesis of amide compounds according to Route 1 of Scheme 5.

A mixture of 10-[3'-(hydroxymethyl)-1-methyl-5-({5-[(2S)-2-methylpiperazin-1-yl]pyridin-2-yl}amino)-6-oxo-[3,4'-bipyridin]-2'-yl]-4,4-dimethyl-1,10-diazatricyclo[6.4.0.0-{2,6}]dodeca-2(6),7-dien-9-one as the free amine (0.55 mmol), a carboxylic acid (0.58 mmol), HATU (273 mg, 0.72 mmol), and DIEA (360 mg, 2.76 mmol) in *N,N-*dimethylformamide (7 mL) was stirred at 25 °C for 0.5 hours. The crude residue was purified by prep-HPLC to afford the desired product.

### Example 2A: Synthesis of 3-(3-(2-(2,6-dioxopiperidin-3-yl)-1,3-dioxoisoindolin-4-yl)propoxy)propanoic acid.

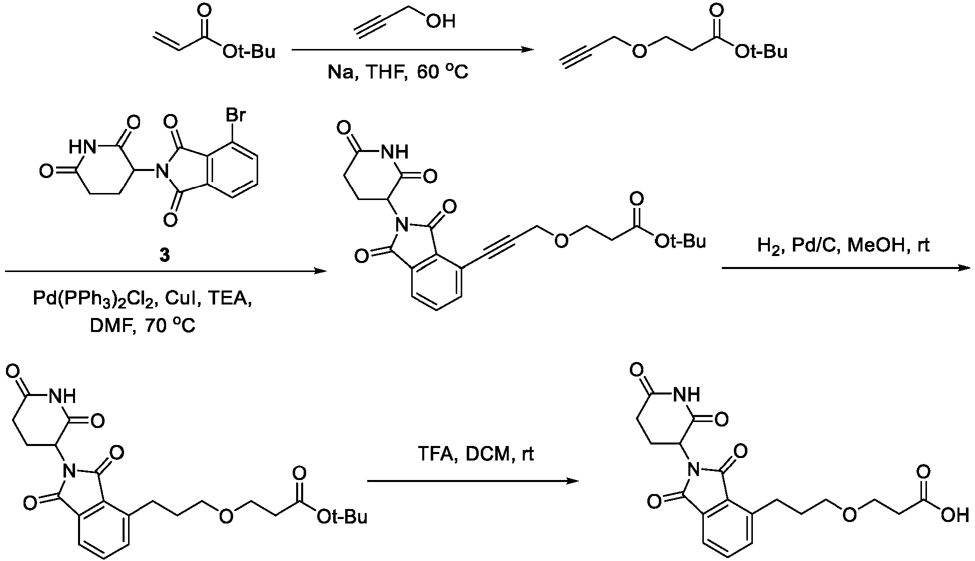

### Step 1: Synthesis of tert-butyl 3-(prop-2-yn-1-yloxy)propanoate.

A mixture of Na (108 mg, 4.68 mmol) and prop-2-yn-1-ol (6.6 g, 117.03 mmol) in anhydrous THF (60 mL) was stirred at 60 °C for 15 min under nitrogen atmosphere. The mixture was then cooled to room temperature and was added tert-butyl acrylate (10.0 g, 78.02 mmol). The resulting solution was stirred at room temperature for 16 h. The reaction was quenched by the addition of water, and then extracted with ethyl acetate. The combined organic layer was washed with brine, dried over anhydrous Na₂SO₄, filtered and concentrated under vacuum. The residue was purified by flash column chromatography with 0~10% ethyl acetate in petroleum ether to afford tert-butyl 3-(prop-2-yn-1-yloxy)propanoate (7.8 g, 54%) as a colorless oil. MS (ESI) calculated for (C₁₀H₁₆O₃) [M+H]⁺, 185.1; found, 185.2. ¹H NMR (400 MHz, DMSO-d6) δ 4.12 (d, *J =* 2.4 Hz, 2H), 3.63 (t, *J =* 6.2 Hz, 2H), 3.43 (t, *J* = 2.4 Hz, 1H), 2.45 (t, *J =* 6.2 Hz, 2H), 1.41 (s, 9H).

### Step 2: Synthesis of tert-butyl 3-((3-(2-(2,6-dioxopiperidin-3-yl)-1,3-dioxoisoindolin-4-yl)prop-2-yn-1-yl)oxy)propanoate.

A degassed mixture of 4-bromo-2-(2,6-dioxopiperidin-3-yl)isoindoline-1,3-dione (5.0 g, 14.83 mmol), Pd(PPh₃)₂Cl₂ (1.6 g, 2.23 mmol), CuI (706.15 mg, 3.708 mmol) and tert-butyl 3-(prop-2-yn-1-yloxy)propanoate (4.1 g, 22.25 mmol) in DIEA (30 mL) and DMF (30 mL) was stirred at 80 °C for 16 h under nitrogen atmosphere. The resulting mixture was diluted with saturated NH₄Cl aqueous solution and extracted with ethyl acetate. The combined organic layer was washed with brine, dried over anhydrous Na₂SO₄, filtered and concentrated under vacuum. The residue was purified by flash column chromatography with 0~60% ethyl acetate in petroleum ether to afford tert-butyl 3-((3-(2-(2,6-dioxopiperidin-3-yl)-1,3-dioxoisoindolin-4-yl)prop-2-yn-1-yl)oxy)propanoate (7 g, ~80% purity) as a yellow oil, which was used in the next step without further purification. MS (ESI) calculated for (C₂₃H₂₄N₂O₇) [M+H]⁺, 441.2; found, 441.0. ¹H NMR (300 MHz, Methanol-d4) δ 7.97 - 7.74 (m, 3H), 5.20 - 5.14 (m, 1H), 4.49 (s, 2H), 3.93 (t, *J =* 6.3 Hz, 2H), 2.95 - 2.65 (m, 3H), 2.56 (t, *J* = 6.3 Hz, 2H), 2.20 - 2.12 (m, 1H), 1.47 (s, 9H).

### Step 3: Synthesis of tert-butyl 3-(3-(2-(2,6-dioxopiperidin-3-yl)-1,3-dioxoisoindolin-4-yl)propoxy)propanoate.

To a solution of tert-butyl 3-((3-(2-(2,6-dioxopiperidin-3-yl)-1,3-dioxoisoindolin-4-yl)prop-2-yn-1-yl)oxy)propanoate (7.0 g, 15.89 mmol) in methanol (100 mL) was added Pd/C (10%, 1.4 g) under nitrogen atmosphere. The mixture was stirred at room temperature for 16 h under hydrogen atmosphere (2 atm). The solids were filtered out and the filtrate was concentrated under vacuum. The residue was purified by reverse phase flash column chromatography with 10~50% acetonitrile in water to afford tert-butyl 3-(3-(2-(2,6-dioxopiperidin-3-yl)-1,3-dioxoisoindolin-4-yl)propoxy)propanoate (4.2 g, 59%) as a white solid. MS (ESI) calculated for (C₂₃H₂₈N₂O₇) [M+H]⁺, 445.2; found, 445.3.

### Step 4: Synthesis of 3-(3-(2-(2,6-dioxopiperidin-3-yl)-1,3-dioxoisoindolin-4-yl)propoxy)propanoic acid.

A mixture of tert-butyl 3-(3-(2-(2,6-dioxopiperidin-3-yl)-1,3-dioxoisoindolin-4-yl)propoxy)propanoate (4.2 g, 9.45 mmol) in dichloromethane (20 mL) and trifluoroacetic acid (20 mL) was stirred at room temperature for 3 h before concentrated under vacuum. The residue was purified by reverse phase flash column chromatography with 5~30% acetonitrile in water to afford 3-(3-(2-(2,6-dioxopiperidin-3-yl)-1,3-dioxoisoindolin-4-yl)propoxy)propanoic acid (1.8681 g, 51%) as a light yellow semi-solid. MS (ESI) calculated for (C₁₉H₂₀N₂O₇) [M+H]⁺, 389.1; found, 388.8. ¹H NMR (400 MHz, DMSO-*d*₆) δ 12.14 (s, 1H), 11.12 (s, 1H), 7.87 - 7.59 (m, 3H), 5.20 - 5.11 (m, 1H), 3.57 (t, *J =* 6.0 Hz, 2H), 3.40 (t, *J=* 6.4 Hz, 2H), 3.12 - 3.06 (m, 2H), 2.94 - 2.85 (m, 1H), 2.68 - 2.52 (m, 2H), 2.44 (t, *J =* 6.4 Hz, 2H), 2.13 - 2.01 (m, 1H), 1.89 - 1.79 (m, 2H).

### Example 2B: Synthesis of 2-(4-(3-(2-(2,6-dioxopiperidin-3-yl)-1,3-dioxoisoindolin-4-yl)propyl)piperazin-1-yl)acetic acid.

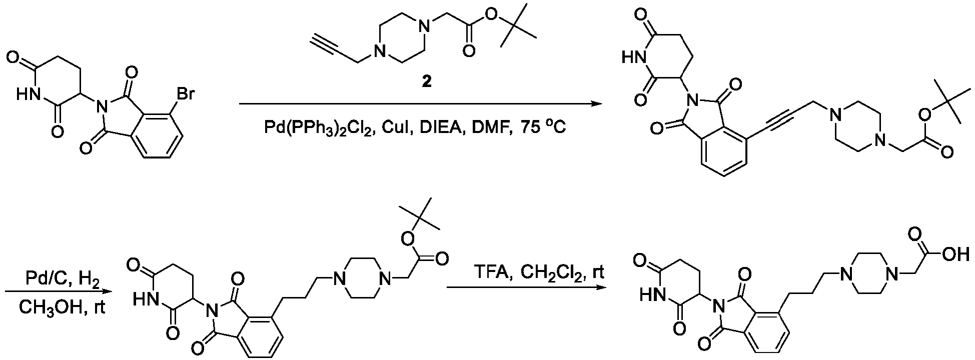

### Step 1: Synthesis of tert-butyl 2-(4-(3-(2-(2,6-dioxopiperidin-3-yl)-1,3-dioxoisoindolin-4-yl)prop-2-yn-1-yl)piperazin-1-yl)acetate.

To a degassed solution of 4-bromo-2-(2,6-dioxopiperidin-3-yl)isoindoline-1,3-dione (1.3 g, 3.86 mmol) in N,N-dimethylformamide (18 mL) were added tert-butyl 2-(4-(prop-2-ynyl)piperazin-1-yl)acetate (1.4 g, 5.57 mmol), Pd(PPh₃)₂Cl₂ (423.3 mg, 0.60 mmol), DIEA (12 mL) and CuI (251.1 mg, 1.32 mmol). The resulting solution was stirred at 75 °C for 4 h under nitrogen. The reaction was quenched by the addition of water, and then extracted with ethyl acetate. The combined organic layer was washed with brine, dried over anhydrous sodium sulfate and filtered. The filtrate was evaporated under vacuum. The residue was purified by flash column chromatography with 0~10% methanol in dichloromethane to afford tert-butyl 2-(4-(3-(2-(2,6-dioxopiperidin-3-yl)-1,3-dioxoisoindolin-4-yl)prop-2-yn-1-yl)piperazin-1-yl)acetate (2.5 g, 70%) as a yellow solid. MS (ESI) calculated for (C₂₆H₃₀N₄O₆) [M+H]⁺, 495.2; found, 495.1.

### Step 2: Synthesis of tert-butyl 2-(4-(3-(2-(2,6-dioxopiperidin-3-yl)-1,3-dioxoisoindolin-4-yl)propyl)piperazin-1-yl)acetate.

To a solution of tert-butyl 2-(4-(3-(2-(2,6-dioxopiperidin-3-yl)-1,3-dioxoisoindolin-4-yl)prop-2-ynyl)piperazin-1-yl)acetate (2.1 g, 4.25 mmol) in methanol (50 mL) was added Pd/C (dry, 0.42 g). The resulting solution was stirred at room temperature for 16 h under hydrogen (2 atm). The solids were filtered out. The filtrate was evaporated under vacuum to afford tert-butyl 2-(4-(3-(2-(2,6-dioxopiperidin-3-yl)-1,3-dioxoisoindolin-4-yl)propyl)piperazin-1-yl)acetate (1.6 g, crude) as a yellow solid, which was used in the next step without further purification. MS (ESI) calculated for (C₂₆H₃₄N₄O₆) [M+H]⁺, 499.2; found,499.0.

### Step 3: Synthesis of 2-(4-(3-(2-(2,6-dioxopiperidin-3-yl)-1,3-dioxoisoindolin-4-yl)propyl)piperazin-1-yl)acetic acid TFA salt.

To a solution of tert-butyl 2-(4-(3-(2-(2,6-dioxopiperidin-3-yl)-1,3-dioxoisoindolin-4-yl)propyl)piperazin-1-yl)acetate (2.1 g, 4.21 mmol) in dichloromethane (20 mL) was added trifluoroacetic acid (20 mL). The resulting solution was stirred at room temperature for 4 h before concentrated under vacuum. The residue was purified by Pre-HPLC with the following conditions: [Column: XSelect CSH Prep C18 OBD Column, 5um,19*150 mm; Mobile Phase A: Water (0.05%TFA ), Mobile Phase B: ACN; Flow rate: 25 mL/min; Gradient: 5% B to 20% B in 7 min; 254/220 nm] to afford 2-(4-(3-(2-(2,6-dioxopiperidin-3-yl)-1,3-dioxoisoindolin-4-yl)propyl)piperazin-1-yl)acetic acid TFA salt (398.0 mg, 21%) as a yellow solid. MS (ESI) calculated for (C₂₂H₂₆N₄O₆) [M+H]⁺, 443.2; found, 442.9. ¹H NMR (300 MHz, DMSO-*d*₆) δ 11.15 (s, 1H), 7.95 - 7.73 (m, 3H), 5.17 - 5.11 (m, 1H), 3.74 - 3.29 (m, 3H), 3.25 - 2.73 (m, 11H), 2.64 (s, 1H), 2.60 - 2.52 (m, 1H), 2.46 - 2.45 (m, 1H), 2.11 - 1.92 (m, 3H).

### Example 2C: General procedure (A) for the synthesis of carboxylic acid intermediates for use in Example 2.

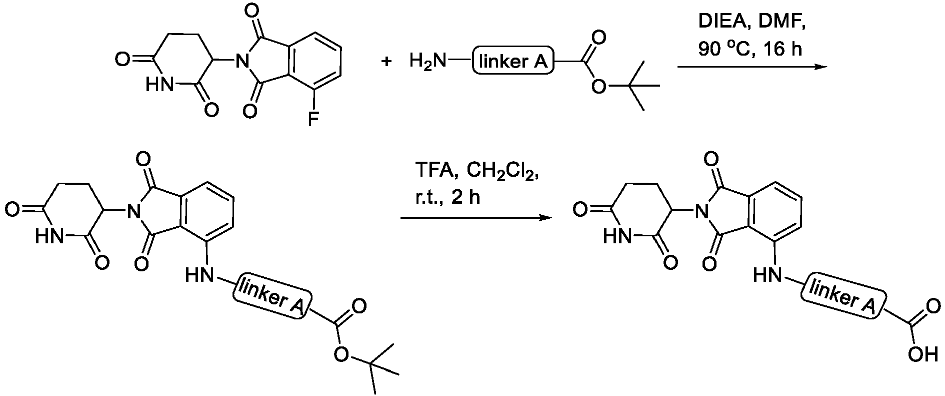

As used in this Example 2C, "linker A" is -Y²-Y³-Y⁴-, wherein each of Y², Y³, and Y⁴, are defined herein.

Step 1: A mixture of 2-(2,6-dioxopiperidin-3-yl)-4-fluoro-2,3-dihydro-1H-isoindole-1,3-dione (0.26 mmol), aminoester (0.26 mmol), ethylbis(propan-2-yl)amine (0.52 mmol) and DMF (1 mL) was allowed to stir at 90°C overnight. The mixture was cooled and purified by HPLC (5-95% MeCN in H₂O with 0.1% TFA) to afford the tert-butylester intermediate.

Step 2: A mixture of tert-butyl 4-{[2-(2,6-dioxopiperidin-3-yl)-1,3-dioxo-2,3-dihydro-1H-isoindol-4-yl]amino}butanoate (0.10 mmol) , CH₂Cl₂ (1 mL), and TFA (1 mL) was allowed to stir at r.t. for 2 h. The mixture was concentrated to afford the carboxylic acid product.

The general method of Example 2C, was used to synthesize the intermediates described in Examples 2C-1 through 2C-6.

The following molecules were obtained from commercial sources: 2-(2,6-Dioxo-piperidin-3-yl)-4-fluoroisoindoline-1,3-dione (Advanced ChemBlocks Inc), methyl 3-bromo-2-methyl-benzoate (Oakwood Chemical), 3-aminopiperidine-2,6-dione hydrochloride (Oakwood Chemical), tert-butyl (2-(2-(2-(prop-2-yn-1-yloxy)ethoxy)ethoxy)ethyl)carbamate (BroadPharm), benzyl piperazine-1-carboxylate (Sigma Aldrich), tert-butyl 2-(piperazin-1-yl)acetate (CombiBlocks), N-Boc-4-pentyne-1-amine (Sigma Aldrich), tert-butyl 3-(2-hydroxyethoxy)propanoate (BroadPharm), 2-(2-(2-aminoethoxy)ethoxy)ethan-1-ol (BroadPharm).

### Example 2C-1: Synthesis of 4-((2-(2,6-dioxopiperidin-3-yl)-1,3-dioxoisoindolin-4-yl)amino)butanoic acid.

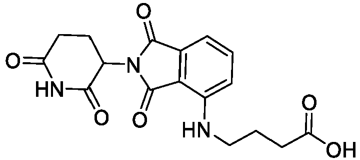

Step 1 product: tert-butyl 4-{[2-(2,6-dioxopiperidin-3-yl)-1,3-dioxoisoindol-4-yl]amino}butanoate (40.0 mg, 36.9%). LCMS: C₂₁H₂₅N₃O₆ requires: 415, found: m/z = 416 [M+H]⁺.

Step 2 product: 4-{[2-(2,6-dioxopiperidin-3-yl)-1,3-dioxoisoindol-4-yl]amino}butanoic acid (34.00 mg, 98.3%). LCMS: C₂₁H₂₅N₃O₆ requires: 359, found: m/z = 360 [M+H]⁺.

### Example 2C-2: Synthesis of 3-((2-(2,6-dioxopiperidin-3-yl)-1,3-dioxoisoindolin-4-yl)amino)propanoic acid.

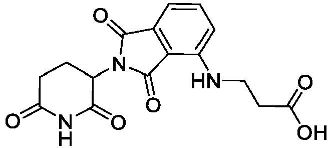

Step 1 product: tert-butyl 3-{[2-(2,6-dioxopiperidin-3-yl)-1,3-dioxoisoindol-4-yl]amino}propanoate (62.0 mg, 38.4%). LCMS: C₂₀H₂₃N₃O₆ requires: 401, found: m/z = 402 [M+H]⁺.

Step 2 product: 3-((2-(2,6-dioxopiperidin-3-yl)-1,3-dioxoisoindolin-4-yl)amino)propanoic acid (53 mg, 99%). LCMS: C₁₆H₁₅N₃O₆ requires: 345, found: m/z = 346 [M+H]⁺.

### Example 2C-3: Synthesis of (2-(2,6-dioxopiperidin-3-yl)-1,3-dioxoisoindolin-4-yl)glycine.

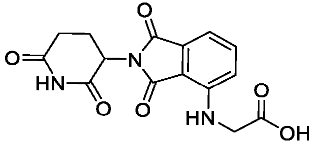

Step 1 product: tert-butyl 2-{[2-(2,6-dioxopiperidin-3-yl)-1,3-dioxoisoindol-4-yl]amino}acetate (28.0 mg, 20.6%). LCMS: C₁₉H₂₁N₃O₆ requires: 387, found: m/z = 388 [M+H]⁺.

Step 2 product: (2-(2,6-dioxopiperidin-3-yl)-1,3-dioxoisoindolin-4-yl)glycine (23 mg, 96%). LCMS: C₁₅H₁₃N₃O₆ requires: 331, found: m/z = 332 [M+H]⁺.

### Example 2C-4: Synthesis of trans-4-((2-(2,6-dioxopiperidin-3-yl)-1,3-dioxoisoindolin-4-yl)amino)cyclohexane-1-carboxylic acid.

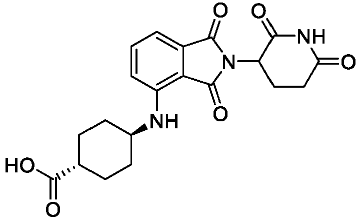

Step 1 product: trans-tert-butyl 4-((2-(2,6-dioxopiperidin-3-yl)-1,3-dioxoisoindolin-4-yl)amino)cyclohexane-1-carboxylate (43.40 mg, 47.0%).

Step 2 product: trans-4-((2-(2,6-dioxopiperidin-3-yl)-1,3-dioxoisoindolin-4-yl)amino)cyclohexane-1-carboxylic acid (38 mg, 99%).

### Example 2C-5: Synthesis of 3-(2-((2-(2,6-dioxopiperidin-3-yl)-1,3-dioxoisoindolin-4-yl)amino)ethoxy)propanoic acid.

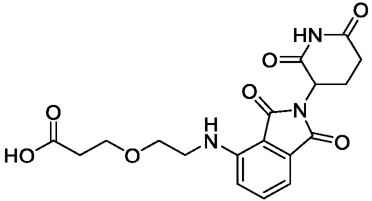

Step 1 product: tert-butyl 3-[2-[[2-(2,6-dioxo-3-piperidyl)-1,3-dioxo-isoindolin-4-yl]amino]ethoxy]propanoate (1.8 g, 51.9%). LCMS; C₂₂H₂₇N₃O₇ requires: 445, found: m/z = 468 [M+Na]⁺.

Step 2 product: 3-[2-[[2-(2,6-dioxo-3-piperidyl)-1,3-dioxo-isoindolin-4-yl]amino]ethoxy]propanoic acid (526.8 mg, 32%). LCMS; C₁₈H₁₉N₃O₇ requires: 389, found: m/z = 390 [M+H]⁺.

### Example 2C-6: Synthesis of 3-(2-(2-(2-((2-(2,6-dioxopiperidin-3-yl)-1,3-dioxoisoindolin-4-yl)amino)ethoxy)ethoxy)ethoxy)propanoic acid.

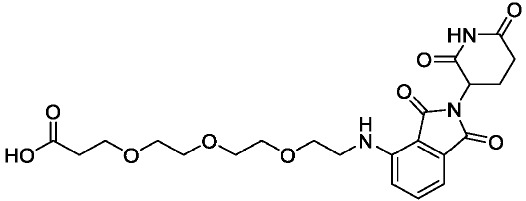

Step 1 product: tert-butyl 3-[2-[2-[2-[[2-(2,6-dioxo-3-piperidyl)-1,3-dioxoisoindolin-4-yl]amino]ethoxy]ethoxy]ethoxy]propanoate (1.6 g, 41%). LCMS; C₂₆H₃₅N₃O₉ requires: 533, found: m/z = 534 [M+H]⁺.

Step 2 product: 3-[2-[2-[2-[[2-(2,6-dioxo-3-piperidyl)-1,3-dioxo-isoindolin-4-yl]amino]ethoxy]ethoxy]ethoxy]propanoic acid (1.2 g, 73.62%). LCMS; C₂₂H₂₇N₃O₉ requires: 477, found: m/z = 478 [M+H]⁺.

### Example 2D: General procedure (B) for the synthesis of a carboxylic acid intermediates for use in Example 2.

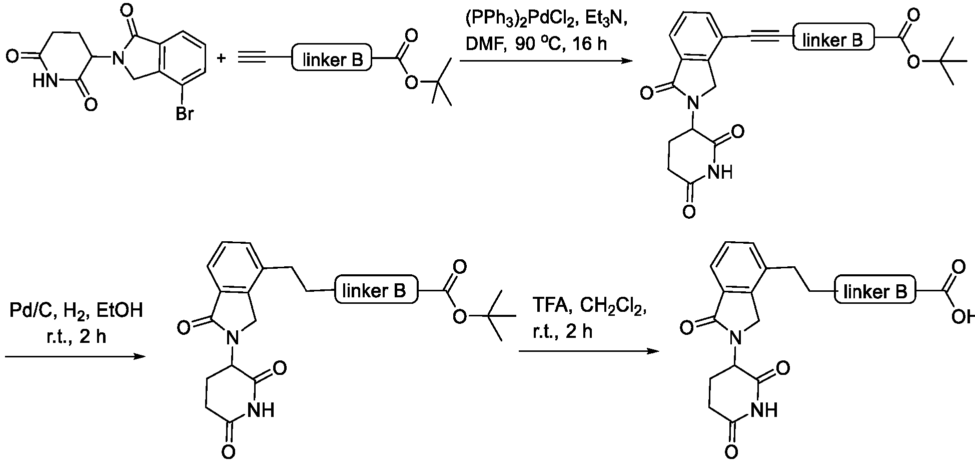

As used in this Example, "linker B" is -(CH₂-CH₂-O)ₘ-, wherein m is an integer from 1 to 5.

Step 1: A mixture of 3-(4-bromo-1-oxo-2,3-dihydro-1H-isoindol-2-yl)piperidine-2,6-dione (2.52 mmol), (PPh₃)₂PdCl₂ (0.15 mmol),CuI (0.25 mmol) , alkyne ester (5.04 mmol) were added to a vial. The vial was evacuated and backfilled with N₂ 5 times. DMF and triethylamine (30.3 mmol) were added and the mixture was allowed to stir at 90 °C overnight. The mixture was filtered through celite, washing with MeOH and EtOAc. EtOAc and saturated aqueous NaCl were added. The organic layer was dried with MgSO₄, filtered, concentrated and purified by reverse phase MPLC (5-100% MeCN in H₂O on C18 column) to afford the product.

Step 2: A mixture of disubstituted alkyne (0.81 mmol), Pd/C 10wt% (0.08 mmol) and EtOH were mixed in a flask. The flask was evacuated and backfilled with H₂ 5 times and allowed to stir at r.t. for 2h. The mixture was filtered through celite washing with MeOH and EtOAc, concentrated and carried to the next step.

Step 3: A mixture of tert-butylester (0.81 mmol), CH₂Cl₂ (2 mL), and TFA (2 mL) was allowed to stir at r.t. for 2 h. The mixture was concentrated to afford the carboxylic acid product.

The general method of Example 2B, was used to synthesize the intermediates described in Example 2D-1.

### Example 2D-1: Synthesis of 3-(3-(2-(2.6-dioxopiperidin-3-yl)-1-oxoisoindolin-4-yl)propoxy)propanoic acid.

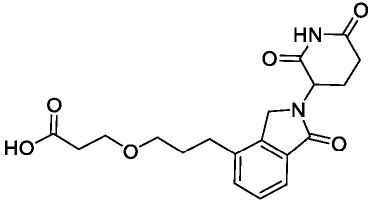

Step 1 product: tert-butyl 3-((3-(2-(2,6-dioxopiperidin-3-yl)-1-oxoisoindolin-4-yl)prop-2-yn-1-yl)oxy)propanoate (347 mg, 32.3%). LCMS: C₂₃H₂₆N₂O₆ requires: 426, found: m/z = 427 [M+H]⁺.

Step 2 product: tert-butyl 3-(3-(2-(2,6-dioxopiperidin-3-yl)-1-oxoisoindolin-4-yl)propoxy)propanoate (350 mg, 99%). LCMS: C₂₃H₃₀N₂O₆ requires: 430, found: m/z = 431 [M+H]⁺.

Step 3 product: 3-(3-(2-(2,6-dioxopiperidin-3-yl)-1-oxoisoindolin-4-yl)propoxy)propanoic acid (304 mg, 99%). LCMS: C₁₉H₂₂N₂O₆ requires: 374, found: m/z = 375 [M+H]⁺.

### Example 2E: General procedure (C) for the synthesis of a carboxylic acid intermediates for use in Example 2.

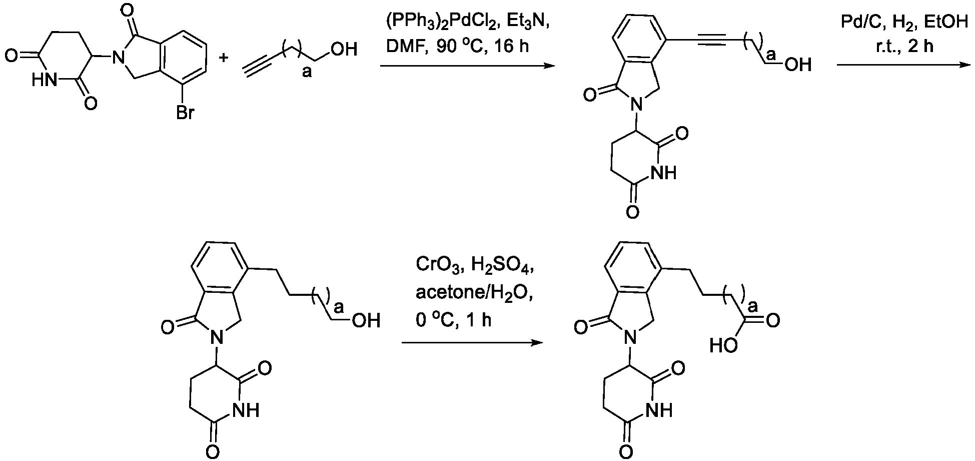

As used in this Example, "a" is an integer from 1 to 4.

Step 1: 3-(4-Bromo-1-oxoisoindolin-2-yl)piperidine-2,6-dione (4.6 mmol), copper iodide (177 mg) and bis-triphenylphosphine-palladium dichloride (326 mg) were evacuated and flushed with nitrogen 3 times. DMF (5 mL), triethylamine (6.5 mL) and the alkyne (27.9 mmol) were added and the vial was flushed with nitrogen, sealed and heated to 80 °C for 20 h. The mixture was cooled to room temperature and was diluted with DCM/ethyl acetate (1:1, 20 mL) and the solid was filtered over a pad of Celite. The solid was stirred with acetonitrile for 16 h. The solids were filtered and concentrated to give the disubstituted alkyne product.

Step 2: The disubstituted alkyne (2.2 mmol) was dissolved in methanol (40 mL). Palladium over charcoal (10%, 235 mg) was added and the flask was filled with hydrogen at 65 psi for 3 h. The mixture was filtered over Celite and washed with methanol to give the alcohol product.

Step 3: Chromic acid (360 mg, 3.6 mmol) was added to 3 M sulfuric acid (3 mL) to make a solution of chromium oxidant (Jones' reagent). The alcohol (1.2 mmol) was suspended in acetone (2.5 mL) and 3 M sulfuric acid (0.5 mL) and the suspension was cooled to 0 °C. The Jones' reagent was slowly added to the alcohol suspension and allowed to stir for 1 h. The mixture was poured into of iced water (20 mL) and the solid was filtered and washed with water The aqueous solution was extracted with (2 x 20 mL) EtOAc, washed with brine and concentrated. The organic fractions were combined with the solid and the mixture was purified by flash column chromatography (0-25% methanol in DCM) to afford the acid product.

The general method of this Example, was used to synthesize the intermediates described in Examples 2E-1 and 2E-2.

### Example 2E-1: Synthesis of 4-(2-(2,6-dioxopiperidin-3-yl)-1-oxoisoindolin-4-yl)butanoic acid.

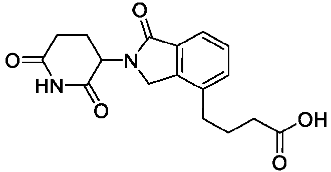

Step 1 product: 3-(4-(4-hydroxybut-1-yn-1-yl)-1-oxoisoindolin-2-yl)piperidine-2,6-dione (690 mg, 47%). LCMS; C₁₇H₁₆N₂O₄ requires: 312, found: m/z = 335 [M+Na]⁺.

Step 2 product: 3-(4-(4-hydroxybutyl)-1-oxoisoindolin-2-yl)piperidine-2,6-dione (361 mg, 52%). LCMS; C₁₇H₂₀N₂O₄ requires: 316, found: m/z = 339 [M+Na]⁺.

Step 3 product: 4-(2-(2,6-dioxopiperidin-3-yl)-1-oxoisoindolin-4-yl)butanoic acid (214 mg, 57%). LCMS; C₁₇H₁₈N₂O₅ requires: 330, found: m/z = 353 [M+Na]⁺.

### Example 2E-2: Synthesis of 5-(2-(2,6-dioxopiperidin-3-yl)-1-oxoisoindolin-4-yl)pentanoic acid.

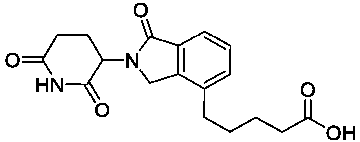

Step 1 product: 3-(4-(5-hydroxypent-1-yn-1-yl)-1-oxoisoindolin-2-yl)piperidine-2,6-dione (325 mg, 58%). LCMS; C₁₈H₁₈N₂O₄ requires: 326, found: m/z = 349 [M+Na]⁺.

Step 2 product: 3-(4-(5-hydroxypentyl)-1-oxoisoindolin-2-yl)piperidine-2,6-dione (160 mg, 99%). LCMS; C₁₈H₂₂N₂O₄ requires: 330, found: m/z = 353 [M+Na]⁺.

Step 3 product: 5-(2-(2,6-dioxopiperidin-3-yl)-1-oxoisoindolin-4-yl)pentanoic acid (74 mg, 60%). LCMS; C₁₈H₂₀N₂O₅ requires: 344, found: m/z = 367 [M+Na]⁺.

### Example 2F: Synthesis of 2-(4-(3-(2-(2,6-dioxopiperidin-3-yl)-1-oxoisoindolin-4-yl)propyl)piperazin-1-yl)acetic acid.

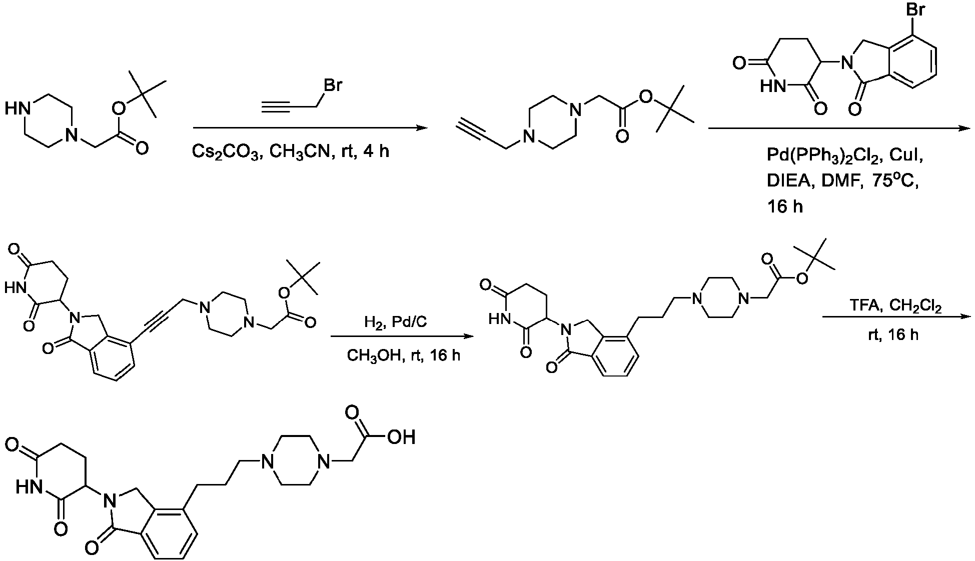

### Step 1: Synthesis of tert-butyl 2-(4-(prop-2-ynyl)piperazin-1-yl)acetate.

To a solution of tert-butyl 2-(piperazin-1-yl)acetate (1.5 g, 7.49 mmol) in acetonitrile (50 mL) were added 3-bromoprop-1-yne (892.5 mg, 7.50 mmol) and Cs₂CO₃ (2.4 g, 7.50 mmol). The resulting solution was stirred at room temperature for 4 h. The solids were filtered out and the filtrate was evaporated under vacuum. The residue was purified by phase flash column chromatography with 0~30% ethyl acetate in petroleum ether to afford tert-butyl 2-(4-(prop-2-ynyl)piperazin-1-yl)acetate (1.1 g, 62%) as a yellow oil. MS (ESI) calculated for (C₁₃H₂₂N₂O₂) [M+H]⁺, 239.2; found, 239.1.

### Step 2: Synthesis of tert-butyl 2-(4-(3-(2-(2,6-dioxopiperidin-3-yl)-1-oxoisoindolin-4-yl)prop-2-ynyl)piperazin-1-yl)acetate.

To a degassed solution of 3-(4-bromo-1-oxoisoindolin-2-yl)piperidine-2,6-dione (1.5 g, 4.64 mmol) in N,N-dimethylformamide (30 mL) were added tert-butyl 2-(4-(prop-2-ynyl)piperazin-1-yl)acetate (1.5 g, 6.29mmol), Pd(PPh₃)₂Cl₂ (489.0 mg, 0.70 mmol,), DIEA (20 mL) and CuI (221.7 mg, 1.16 mmol). The resulting solution was stirred at 75 °C for 16 h under nitrogen. The reaction was quenched by the addition of water, and then extracted with ethyl acetate. The combined organic layer was washed with brine, dried over anhydrous sodium sulfate and filtered. The filtrate was evaporated under vacuum. The residue was purified by flash column chromatography with 0~10% methanol in dichloromethane to afford tert-butyl 2-(4-(3-(2-(2,6-dioxopiperidin-3-yl)-1-oxoisoindolin-4-yl)prop-2-ynyl)piperazin-1-yl)acetate (1.5 g, 68%) as a yellow solid. MS (ESI) calculated for (C₂₆H₃₂N₄O₅) [M+H]⁺, 481.2; found, 481.1.

### Step 3: Synthesis of tert-butyl 2-(4-(3-(2-(2,6-dioxopiperidin-3-yl)-1-oxoisoindolin-4-yl)propyl)piperazin-1-yl)acetate.

To a solution of tert-butyl 2-(4-(3-(2-(2,6-dioxopiperidin-3-yl)-1-oxoisoindolin-4-yl)prop-2-ynyl)piperazin-1-yl)acetate (2.2 g, 4.58 mmol) in methanol (50 mL) was added Pd/C (dry, 0.44 g). The resulting solution was stirred at room temperature for 16 h under hydrogen (2 atm). The solids were filtered out. The filtrate was evaporated under vacuum to afford tert-butyl 2-(4-(3-(2-(2,6-dioxopiperidin-3-yl)-1-oxoisoindolin-4-yl)propyl)piperazin-1-yl)acetate (1.4 g, crude) as a yellow oil, which was used in the next step without further purification. MS (ESI) calculated for (C₂₆H₃₆N₄O₅) [M+H]⁺, 485.3; found,485.2.

### Step 4: Synthesis of 2-(4-(3-(2-(2,6-dioxopiperidin-3-yl)-1-oxoisoindolin-4-yl)propyl)piperazin-1-yl)acetic acid TFA salt.

To a solution of tert-butyl 2-(4-(3-(2-(2,6-dioxopiperidin-3-yl)-1-oxoisoindolin-4-yl)propyl)piperazin-1-yl)acetate (1.4 g, 2.89 mmol) in dichloromethane (20 mL) was added trifluoroacetic acid (20 mL). The resulting solution was stirred at room temperature for 16 h before concentrated under vacuum. The residue was purified by Pre-HPLC with the following conditions: [Column: XSelect CSH Prep C18 OBD Column, 5um,19*150mm; Mobile Phase A: Water (0.05% TFA), Mobile Phase B: ACN; Flow rate: 25 mL/min; Gradient: 5% B to 20% B in 7 min; 254/220 nm] to afford 2-(4-(3-(2-(2,6-dioxopiperidin-3-yl)-1-oxoisoindolin-4-yl)propyl)piperazin-1-yl)acetic acid TFA salt (434.3 mg, 35%) as a yellow solid. MS (ESI) calculated for (C₂₂H₂₈N₄O₅) [M+H]⁺, 429.2; found, 429.0. ¹H NMR (300 MHz, DMSO-*d*₆) δ 11.08 (s, 1H), 7.60 - 7.65 (m, 1H), 7.52 - 7.47 (m, 2H), 5.20 - 5.13 (m, 1H), 4.52 - 4.46 (m, 1H), 4.3

### Example 2G: Synthesis of 2-(4-(2-(2-(2,6-dioxopiperidin-3-yl)-1-oxoisoindolin-4-yl)ethyl)piperazin-1-yl)acetic acid.

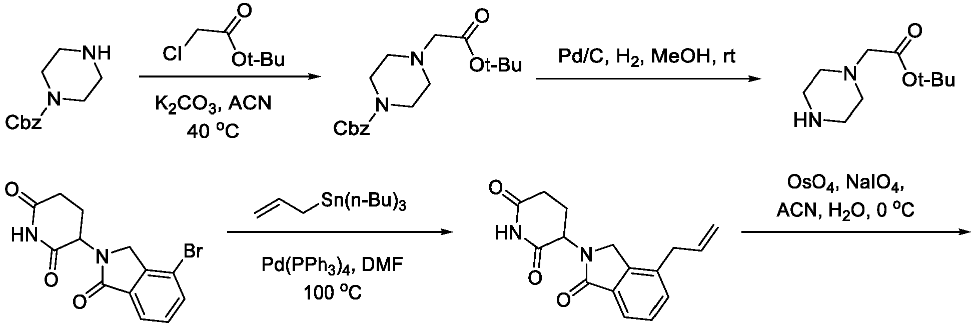

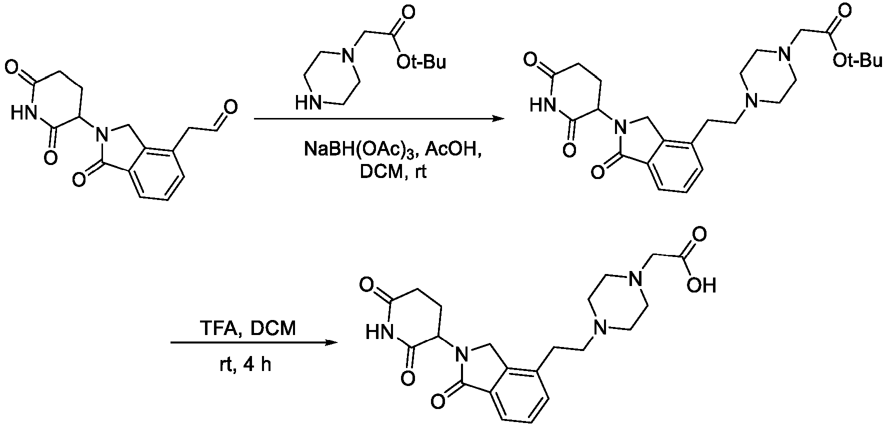

### Step 1: Synthesis of benzyl 4-(2-(tert-butoxy)-2-oxoethyl)piperazine-1-carboxylate.

To a solution of benzyl piperazine-1-carboxylate (10.0 g, 45.4 mmol) and K₂CO₃ (12.6 g, 90.8 mmol) in acetonitrile (150 mL) was added tert-butyl 2-chloroacetate (7.5 g, 49.9 mmol). The resulting solution was stirred at 40 °C for 16 h under nitrogen atmosphere. The solids were filtered out and the filtrate was concentrated under vacuum. The residue was purified by flash column chromatography with 0~50% ethyl acetate in petroleum ether to afford benzyl 4-(2-(tert-butoxy)-2-oxoethyl)piperazine-1-carboxylate (9.6 g, 63%) as a light yellow oil. MS (ESI) calculated for (C₁₈H₂₆N₂O₄) [M+H]⁺, 335.2; found, 335.3.

### Step 2: Synthesis of tert-butyl 2-(piperazin-1-yl)acetate.

To a solution of benzyl 4-(2-(tert-butoxy)-2-oxoethyl)piperazine-1-carboxylate (9.6 g, 28.7 mmol) in methanol (100 mL) was added Pd/C (10%, 2.0 g) under nitrogen atmosphere. The mixture was stirred at room temperature for 16 h under hydrogen atmosphere (2 atm). The solids were filtered out and the filtrate was concentrated under vacuum to afford tert-butyl 2-(piperazin-1-yl)acetate (6.2 g, crude) as a light yellow oil, which was used in the next step without further purification. MS (ESI) calculated for (C₁₀H₂₀N₂O₂) [M+H]⁺, 201.2; found, 201.0.

### Step 3: Synthesis of 3-(4-allyl-1-oxoisoindolin-2-yl)piperidine-2,6-dione.

A degassed mixture of 3-(4-bromo-1-oxo-2,3-dihydro-1H-isoindol-2-yl)piperidine-2,6-dione (10.0 g, 30.9 mmol), allyltributylstannane (15.4 g, 46.4 mmol) and Pd(PPh₃)₄ (3.6 g, 3.1 mmol) in DMF (80 mL) was stirred at 100 °C for 16 h under nitrogen atmosphere. When the reaction was completed by LCMS, the resulting mixture was diluted with water and extracted with ethyl acetate. The combined organic layer was washed with brine, dried over anhydrous Na₂SO₄, filtered and concentrated under vacuum. The residue was purified by flash column chromatography with 0~10% methanol in dichloromethane to afford 3-(4-allyl-1-oxoisoindolin-2-yl)piperidine-2,6-dione (7.0 g, 79%) as a white solid. MS (ESI) calculated for (C₁₆H₁₆N₂O₃) [M+H]⁺, 285.1; found, 285.2. ¹H NMR (400 MHz, DMSO-d6) δ 10.99 (s, 1H), 7.62 - 7.60 (m, 1H), 7.52 - 7.27 (m, 2H), 6.02 - 5.92 (m, 1H), 5.16 - 5.09 (m, 3H), 4.45 (d, *J=* 17.2 Hz, 1H), 4.30 (d, *J=* 17.2 Hz, 1H), 3.46 - 3.44 (m, 2H), 2.97 - 2.86 (m, 1H), 2.70 - 2.57 (m, 1H), 2.04 - 1.99 (m, 1H), 1.68 - 1.55 (m, 1H).

### Step 4: Synthesis of 2-(2-(2,6-dioxopiperidin-3-yl)-1-oxoisoindolin-4-yl)acetaldehyde.

A mixture of 3-(4-allyl-1-oxoisoindolin-2-yl)piperidine-2,6-dione (7.0 g, 24.6 mmol), OsO₄ (625 mg, 2.5 mmol) and NaIO₄ (10.5 g, 49.2 mmol) in MeCN (60 mL) and H₂O (20 mL) was stirred at 0 °C for 6 h. when the reaction was completed, the resulting mixture was diluted with water and extracted with ethyl acetate. The combined organic layer was washed with brine, dried over anhydrous Na₂SO₄, filtered and concentrated under vacuum to afford 2-(2-(2,6-dioxopiperidin-3-yl)-1-oxoisoindolin-4-yl)acetaldehyde (4.0 g, crude) as a brown solid, which was used in the next step without further purification. MS (ESI) calculated for (C₁₅H₁₄N₂O₄) [M+H]⁺, 287.1; found, 287.2.

### Step 5: Synthesis of tert-butyl 2-(4-(2-(2-(2,6-dioxopiperidin-3-yl)-1-oxoisoindolin-4-yl)ethyl)piperazin-1-yl)acetate.

A mixture of 2-[2-(2,6-dioxopiperidin-3-yl)-1-oxo-2,3-dihydro-1H-isoindol-4-yl]acetaldehyde (4.0 g, 13.9 mmol), tert-butyl 2-(piperazin-1-yl)acetate(3.4 g, 16.8 mmol), AcOH (1 mL) and NaBH(OAc)₃ (5.9 g, 27.9 mmol) in dichloromethane (50 mL) was stirred at room temperature for 16 h. The resulting mixture was diluted with water and extracted with ethyl acetate. The combined organic layer was washed with brine, dried over anhydrous Na₂SO₄, filtered and concentrated under vacuum. The crude residue was purified by reverse phase flash column chromatography with 10~50% acetonitrile in water to afford tert-butyl 2-(4-(2-(2-(2,6-dioxopiperidin-3-yl)-1-oxoisoindolin-4-yl)ethyl)piperazin-1-yl)acetate (2.5 g, 22% over two steps) as a light brown syrup. MS (ESI) calculated for (C₂₅H₃₄N₄O₅) [M+H]⁺, 471.2; found, 471.0.

### Step 6: Synthesis of 2-(4-(2-(2-(2,6-dioxopiperidin-3-yl)-1-oxoisoindolin-4-yl)ethyl)piperazin-1-yl)acetic acid TFA salt.

To a solution of tert-butyl 2-(4-(2-(2-(2,6-dioxopiperidin-3-yl)-1-oxoisoindolin-4-yl)ethyl)piperazin-1-yl)acetate (2.5 g, 5.3 mmol) in dichloromethane (20 mL) was added trifluoroacetic acid (20 mL). The resulting mixture was stirred at room temperature for 16 h before concentrated under vacuum. The residue was purified by reverse phase flash column chromatography with 5~30% acetonitrile in water to afford 2-(4-(2-(2-(2,6-dioxopiperidin-3-yl)-1-oxoisoindolin-4-yl)ethyl)piperazin-1-yl)acetic acid (1.7214 g, 78%) as a light brown solid. MS (ESI) calculated for (C₂₁H₂₆N₄O₅) [M+H]⁺, 415.2; found, 415.4. ¹H NMR (300 MHz, DMSO-*d*₆) δ 11.08 (s, 1H), 7.66 - 7.62 (m, 1H), 7.54 - 7.47 (m, 2H), 5.14 - 5.08 (m, 1H), 4.54 - 4.48 (m, 1H), 4.40 - 4.31 (m, 1H), 3.76 (s, 2H), 3.60 - 3.10 (m, 10H), 3.10 - 2.78 (m, 3H), 2.68 - 2.54 (m, 1H), 2.40 - 2.31 (m, 1H), 2.10 - 1.94 (m, 1H).

### Example 2H: Synthesis of 4-(4-(2-(2,6-dioxopiperidin-3-yl)-6-fluoro-1,3-dioxoisoindolin-5-yl)piperazin-1-yl)butanoic acid.

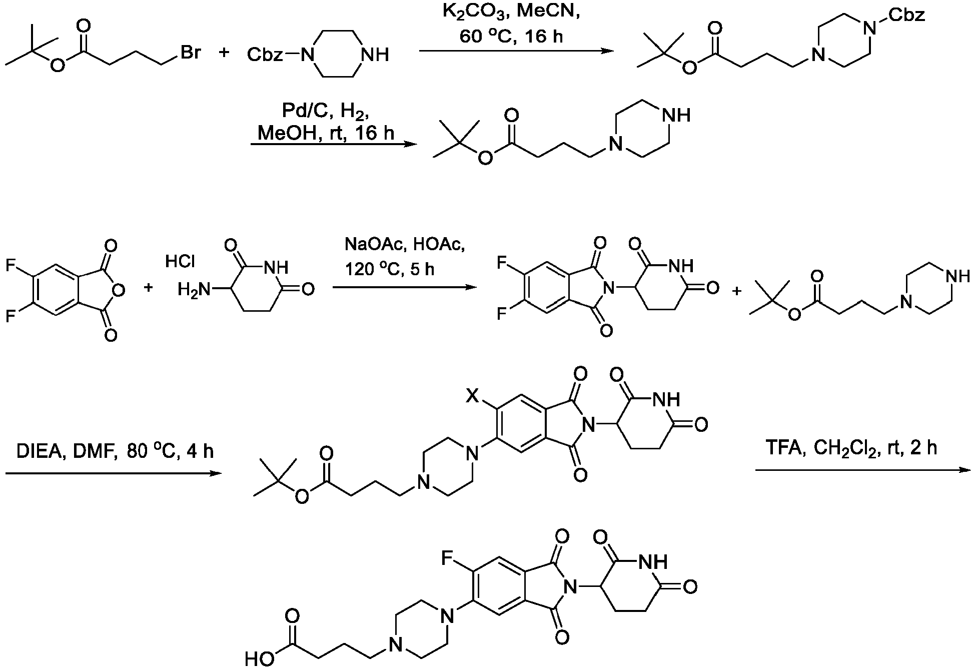

### Synthesis of tert-butyl 4-(piperazin-1-yl)butanoate.

Step 1: To a solution of tert-butyl 4-bromobutanoate (8.5 g, 38.10 mmol) and benzyl piperazine-1-carboxylate (10.1 g, 45.72 mmol) in acetonitrile (100 mL) was added K₂CO₃ (10.5 g, 76.20 mmol). The resulting mixture was stirred at 60 °C for 16 h under nitrogen atmosphere. The solids were filtered out and the filtrate was concentrated under vacuum. The residue was purified by flash column chromatography with 10~70% ethyl acetate in petroleum ether to afford benzyl 4-(4-(tert-butoxy)-4-oxobutyl)piperazine-1-carboxylate (11.0 g, 79%) as colorless oil. LCMS: C₂₀H₃₀N₂O₄ requires: 362, found: m/z = 363 [M+H]⁺.

Step 2: To a solution of benzyl 4-(4-(tert-butoxy)-4-oxobutyl)piperazine-1-carboxylate (11.0 g, 30.39 mmol) in methanol (150 mL) was added Pd/C (10%, 2 g) under nitrogen atmosphere. The mixture was stirred at room temperature for 16 h under hydrogen atmosphere (2 atm). The solids were filtered out and the filtrate was concentrated under vacuum to afford tert-butyl 4-(piperazin-1-yl)butanoate (6.6 g, crude) which was used in the next step without further purification. LCMS: C₁₂H₂₄N₂O₂ requires: 228, found: m/z = 229 [M+H]⁺.

### Synthesis of 4-(4-(2-(2,6-dioxopiperidin-3-yl)-6-fluoro-1,3-dioxoisoindolin-5-yl)piperazin-1-yl)butanoic acid.

Step 1: To a solution of 5,6-difluoroisobenzofuran-1,3-dione (27.16 mmol) in HOAc (50 mL) were added sodium acetate (46.17 mmol) and 3-aminopiperidine-2,6-dione hydrochloride (38.02 mmol). The reaction mixture was stirred at 120 °C for 5 h. The mixture was cooled to room temperature and diluted with water. The solids were collected by filtration and dried to afford 2-(2,6-dioxopiperidin-3-yl)-5,6-difluoroisoindoline-1,3-dione (4.0 g, 50%). LCMS: C₁₃H₈F₂N₂O₄ requires: 294, found: m/z = 295 [M+H]⁺.

Step 2: To a solution of 2-(2,6-dioxopiperidin-3-yl)-5,6-difluoroisoindoline-1,3-dione (0.68 mmol) in DMF (30 mL) was added tert-butyl 4-(piperazin-1-yl)butanoate (0.68 mmol) and N-ethyl-N-isopropylpropan-2-amine (1.4 mmol). The reaction mixture was stirred at 80 °C for 4 h. The resulting mixture was cooled to room temperature and diluted with water. The aqueous phase was extracted with ethyl acetate. The combined organic layer was washed with brine and water, dried over anhydrous sodium sulfate, filtered and concentrated under vacuum to afford the tert-butyl ester intermediate (3.3 g, crude) which was used in the next step without further purification. LCMS: C₂₅H₃₁FN₄O₆ requires: 502, found: m/z = 503 [M+H]⁺.

Step 3: To a solution of the tert-butyl ester intermediate (6.57 mmol) in dichloromethane (20 mL) was added trifluoroacetic acid (10 mL). The reaction mixture was stirred at room temperature for 2 h. The solvent was removed under vacuum. The residue was purified by reverse phase flash column chromatography (20-80% acetonitrile in water) to afford the acid product (1.4516 g, 38% over 2 steps). LCMS: C₂₁H₂₃FN₄O₆ requires: 446, found: m/z = 447 [M+H]⁺.

### Example 2I: Synthesis of 3-(3-(2-(2,6-dioxopiperidin-3-yl)-1,3-dioxoisoindolin-5-yl)propoxy)propanoic acid.

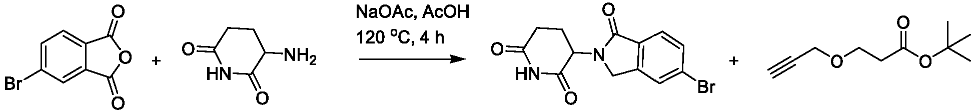

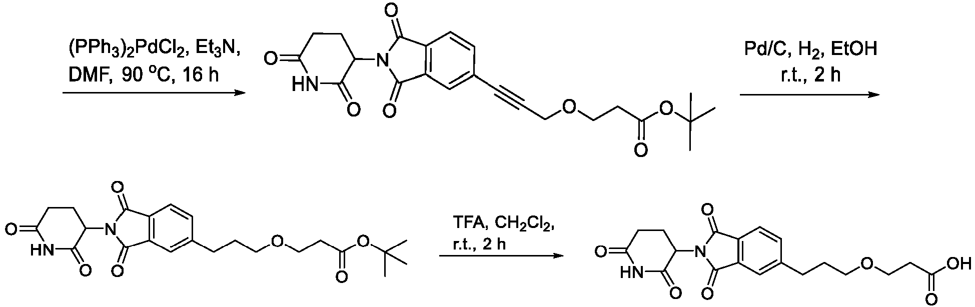

Step 1: To a mixture of 5-bromoisobenzofuran-1,3-dione (10 g, 44.3 mmol) in acetic acid (100 mL) was added 3-aminopiperidine-2,6-dione hydrochloride (7.9 g, 61.7 mmol) and sodium acetate (6.1 g, 74.9 mmol). The reaction mixture was stirred at 120 °C for 4 h, and then concentrated under vacuum. The residue was diluted with water. The precipitated solid was collected by filtration and washed with H₂O (100 mL x 3) and ethyl acetate (50 mL x 2) and dried over vacuum to afford 5-bromo-2-(2,6-dioxopiperidin-3-yl)isoindoline-1,3-dione (14.2 g, 96%) as a light pink solid. MS (ESI) calc'd for (C₁₃H₉BrN₂O₄) [M+1]⁺, 337.0, 339.0; found 337.4, 339.4. LCMS: C₁₃H₉BrN₂O₄ requires: 336, 338, found: m/z = 337, 339 [M+H]⁺.

Step 2: 5-bromo-2-(2,6-dioxopiperidin-3-yl)isoindole-1,3-dione (347.00 mg, 1.03 mmol), (PPh₃)₂PdCl₂ (43.4 mg, 0.06 mmol), and CuI (19.6 mg, 0.10 mmol) were added to a vial. The vial was evacuated and backfilled with N₂ 5 times. DMF (5 mL), tert-butyl 3-(prop-2-yn-1-yloxy)propanoate (189.6 mg, 1.03 mmol) and triethylamine (1.72 mL, 12.4 mmol) were added and the mixture was allowed to stir at 90 °C overnight. The mixture was purified by HPLC (5-95% MeCN in H₂O with 0.1% TFA) to afford tert-butyl 3-({3-[2-(2,6-dioxopiperidin-3-yl)-1,3-dioxoisoindol-5-yl]prop-2-yn-1-yl}oxy)propanoate (173 mg, 38.2%). LCMS: C₂₃H₂₄N₂O₇ requires: 440, found: m/z = 441 [M+H]⁺.

Step 3: A mixture of tert-butyl 3-({3-[2-(2,6-dioxopiperidin-3-yl)-1,3-dioxoisoindol-5-yl]prop-2-yn-1-yl}oxy)propanoate (173 mg, 0.39 mmol), Pd/C 10wt% (3.97 mg, 0.04 mmol) and EtOH (8 mL) were mixed in a flask. The flask was evacuated and backfilled with H₂ 5 times and allowed to stir at r.t. for 2h. The mixture was filtered through celite washing with MeOH and EtOAc. The mixture was concentrated to afford tert-butyl 3-(3-(2-(2,6-dioxopiperidin-3-yl)-1,3-dioxoisoindolin-5-yl)propoxy)propanoate (174 mg, 99%). LCMS: C₂₃H₂₈N₂O₇ requires: 444, found: m/z = 445 [M+H]⁺.

Step 4: A mixture of tert-butyl 3-{3-[2-(2,6-dioxopiperidin-3-yl)-1,3-dioxoisoindol-5-yl]propoxy}propanoate (174 mg, 0.39 mmol), CH₂Cl₂ (3 mL) and trifluoroacetic acid (1 mL) was allowed to stir at rt for 2 h. The volatiles were removed to afford 3-{3-[2-(2,6-dioxopiperidin-3-yl)-1,3-dioxoisoindol-5-yl]propoxy}propanoic acid (151 mg, 99%). LCMS: C₁₉H₂₀N₂O₇ requires: 388, found: m/z = 389 [M+H]⁺.

### Example 2J: Synthesis of (1s,3s)-3-(3-(2-(2,6-dioxopiperidin-3-yl)-1-oxoisoindolin-4-yl)propoxy)cyclobutane-1-carboxylic acid.

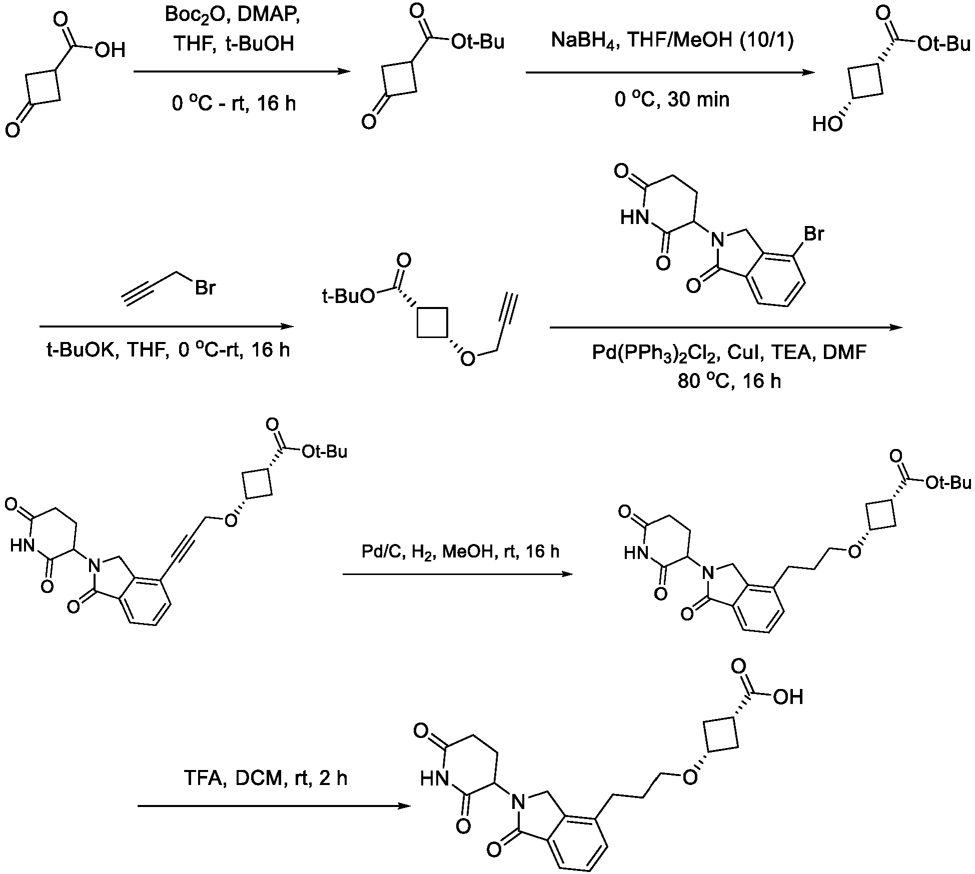

Step 1: To a solution of 3-oxocyclobutane-1-carboxylic acid (5.0 g, 43.82 mmol) and DMAP (2.7 g, 21.91 mmol) in t-BuOH (20 mL) and THF (20 mL) was added a solution of Boc₂O (14.3 g, 65.73 mmol) in THF (10 mL) dropwise at 0 °C under nitrogen atmosphere. The mixture was stirred at room temperature for 16 h. The reaction was quenched by the addition of water and extracted with ethyl acetate. The combined organic layer was washed with brine, dried over anhydrous Na₂SO₄ and concentrated under vacuum. The residue was purified by flash column chromatography with 0~15% ethyl acetate in petroleum ether to afford tert-butyl 3-oxocyclobutane-1-carboxylate (6.2 g, 83%) as colorless oil. LCMS: C₉H₁₄O₃ requires: 170, found: m/z = 171 [M+H]⁺.

Step 2: To a solution of tert-butyl 3-oxocyclobutane-1-carboxylate (5.1 g, 29.96 mmol) in THF (50 mL) and MeOH (5 mL) was added NaBH₄ (566.8 mg, 14.98 mmol) in portions at 0 °C under nitrogen atmosphere. The resulting mixture was stirred at 0 °C for 30 min. The reaction was then quenched by the addition of ice water and extracted with ethyl acetate. The combined organic layer was washed with brine, dried over anhydrous Na₂SO₄ and concentrated under vacuum to afford tert-butyl (1s,3s)-3-hydroxycyclobutane-1-carboxylate (4.8 g, 93%) as light yellow oil, which was used for the next step without further purification. LCMS: C₉H₁₆O₃ requires: 172, found: m/z = 173 [M+H]⁺.

Step 3: To a solution of tert-butyl (1s,3s)-3-hydroxycyclobutane-1-carboxylate (5.0 g, 29.03 mmol) and 3-bromoprop-1-yne (3.8 g, 31.94 mmol) in THF was added t-BuOK (32 mL, 1 M in THF, 32.0 mmol) dropwise at 0 °C under nitrogen atmosphere. The mixture was stirred at room temperature for 16 h. The reaction was then quenched by the addition of ice water and extracted with ethyl acetate. The combined organic layer was washed with brine, dried over anhydrous Na₂SO₄ and concentrated under vacuum. The residue was purified by flash column chromatography with 0~20% ethyl acetate in petroleum ether to afford tert-butyl (1s,3s)-3-(prop-2-yn-1-yloxy)cyclobutane-1-carboxylate (3.2 g, 52%) as light yellow oil. LCMS: C₁₂H₁₈O₃ requires: 210, found: m/z = 211 [M+H]⁺.

Step 4: A mixture of 3-(4-bromo-1-oxoisoindolin-2-yl)piperidine-2,6-dione (3.3 g, 10.21 mmol), tert-butyl (1s,3s)-3-(prop-2-yn-1-yloxy)cyclobutane-1-carboxylate (3.2 g, 15.32 mmol), Pd(PPh₃)₂Cl₂ (1.1 g, 1.53 mmol) and CuI (486.2 mg, 2.55 mmol) in triethylamine (30 mL) and DMF (30 mL) was stirred at 80 °C for 16 h under nitrogen atmosphere. After cooled down to room temperature, the reaction was diluted with saturated NH₄Cl aqueous solution and then extracted with ethyl acetate. The combined organic layer was washed with brine, dried over anhydrous Na₂SO₄ and concentrated under vacuum. The residue was purified by flash column chromatography with 0~10% ethyl acetate in methanol to afford tert-butyl (1s,3s)-3-((3-(2-(2,6-dioxopiperidin-3-yl)-1-oxoisoindolin-4-yl)prop-2-yn-1-yl)oxy)cyclobutane-1-carboxylate (1.5 g, 27%) as a light yellow solid. LCMS: C₂₅H₂₈N₂O₆ requires: 452, found: m/z = 453 [M+H]⁺.

Step 5: To a solution of tert-butyl (1s,3s)-3-((3-(2-(2,6-dioxopiperidin-3-yl)-1-oxoisoindolin-4-yl)prop-2-yn-1-yl)oxy)cyclobutane-1-carboxylate (1.5 g, 2.75 mmol) in MeOH (20 mL) was added Pd/C (10%, 200 mg) under nitrogen atmosphere. The mixture was stirred at room temperature for 16 h under hydrogen atmosphere (2 atm). The solid was filtered out through a Celite pad and the filtrate was concentrated under vacuum. The residue was purified by reverse phase flash column chromatography with 10-70% acetonitrile in water to afford tert-butyl (1s,3s)-3-(3-(2-(2,6-dioxopiperidin-3-yl)-1-oxoisoindolin-4-yl)propoxy)cyclobutane-1-carboxylate (650 mg, 43%) as a light yellow solid. LCMS: C₂₅H₃₂N₂O₆ requires: 456, found: m/z = 457 [M+H]⁺.

Step 6: A mixture of tert-butyl (1s,3s)-3-(3-(2-(2,6-dioxopiperidin-3-yl)-1-oxoisoindolin-4-yl)propoxy)cyclobutane-1-carboxylate (1.2 g, 2.63 mmol) in TFA (4 mL) and DCM (12 mL) was stirred at room temperature for 2 h before concentrated under vacuum. The residue was purified by reverse phase flash column chromatography with the following conditions: [column, C18 silica gel; mobile phase, ACN in water, 10% to 60% gradient in 60 min; detector, UV 254 nm] to afford (1s,3s)-3-(3-(2-(2,6-dioxopiperidin-3-yl)-1-oxoisoindolin-4-yl)propoxy)cyclobutane-1-carboxylic acid (968.7 mg, 92%) as an off-white solid. LCMS: C₂₁H₂₄N₂O₆ requires: 400, found: m/z = 401 [M+H]⁺.

### Example 2K: General procedure (D) for the synthesis of a carboxylic acid intermediates for use in Example 2.

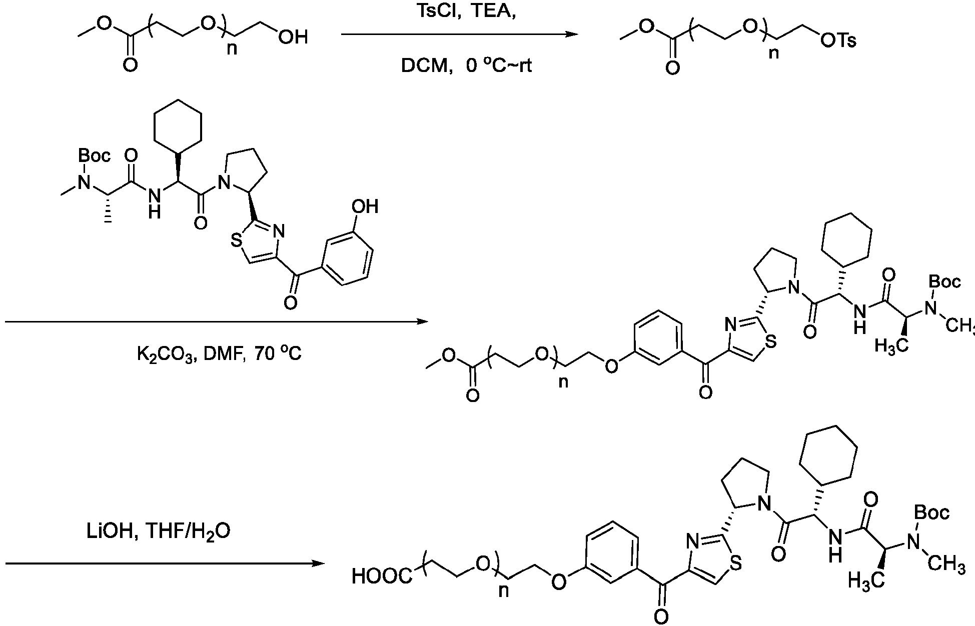

Step 1: To a solution of alcohol (3.46 mmol) in dichloromethane (20 mL) was added triethylamine (6.93 mmol) and p-TsCl (5.19 mmol). The mixture was stirred at room temperature for 16 h. The reaction mixture was diluted with water and the aqueous phase was extracted with ethyl acetate. The combined organic layer was washed with brine, dried over anhydrous sodium sulfate, filtered and concentrated under vacuum. The residue was purified by flash column chromatography with 0~50% ethyl acetate in petroleum ether to afford the alkyl tosylate (85%).

Step 2: To a solution of alkyl tosylate (2.95 mmol) in DMF (10 mL) was added tert-butyl ((S)-1-(((S)-1-cyclohexyl-2-((S)-2-(4-(3-hydroxybenzoyl)thiazol-2-yl)pyrrolidin-1-yl)-2-oxoethyl)amino)-1-oxopropan-2-yl)(methyl)carbamate (1.96 mmol) and potassium carbonate (2.68 mmol). The mixture was stirred at 50 °C for 16 h. The reaction mixture was then diluted with water and the aqueous phase was extracted with ethyl acetate. The combined organic layer was washed with brine, dried over anhydrous sodium sulfate, filtered and concentrated under vacuum. The residue was purified by flash column chromatography with 0~50% ethyl acetate in petroleum ether to afford the methyl ester intermediate (60%).

Step 3: To a solution of the methyl ester intermediate (1.39 mmol) in tetrahydrofuran (10 mL) and H₂O (10 mL) was added lithium hydroxide hydrate (3.33 mmol). The mixture was stirred at room temperature for 16 h. The reaction mixture was diluted with water and the pH was adjusted to ~3 by HCl (2 N). The aqueous phase was extracted with ethyl acetate. The combined organic layer was washed with brine, dried over anhydrous sodium sulfate, filtered and concentrated under vacuum. The residue was purified by flash column chromatography with 10~80% acetonitrile in water to afford the carboxylic acid product (68%).

### Example 2K-1: Synthesis of 3-(2-(3-(2-((S)-1-((S)-2-((S)-2-((tert-butoxycarbonyl)(methyl)amino)propanamido)-2-cyclohexylacetyl)pyrrolidin-2-yl)thiazole-4-carbonyl)phenoxy)ethoxy)propanoic acid.

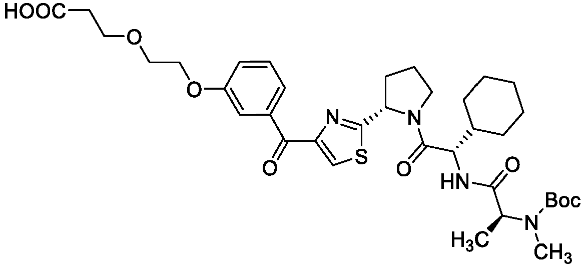

Step 1 product: methyl 3-[2-[(4-methylbenzenesulfonyl)oxy]ethoxy]propanoate (1.05 g, 51%). LCMS; C₁₃H₁₈O₆S requires: 302, found: m/z = 303 [M+H]⁺.

Step 2 product: methyl 3-(2-(3-(2-((S)-1-((S)-2-((S)-2-((tert-butoxycarbonyl)(methyl)amino)propanamido)-2-cyclohexylacetyl)pyrrolidin-2-yl)thiazole-4-carbonyl)phenoxy)ethoxy)propanoate (1.0 g, 62%). LCMS; C₃₇H₅₂N₄O₉S requires: 728, found: m/z = 729 [M+H]⁺.

Step 3 product: 3-(2-(3-(2-((S)-1-((S)-2-((S)-2-((tert-butoxycarbonyl)(methyl)amino)propanamido)-2-cyclohexylacetyl)pyrrolidin-2-yl)thiazole-4-carbonyl)phenoxy)ethoxy)propanoic acid (733.9 mg, 75%). LCMS; C₃₆H₅₀N₄O₉S requires: 714, found: m/z = 715 [M+H]⁺.

### Example 2K-2: Synthesis of 3-(2-(2-(2-(3-(2-((S)-1-((S)-2-((S)-2-((tert-butoxycarbonyl)(methyl)amino)propanamido)-2-cyclohexylacetyl)pyrrolidin-2-yl)thiazole-4-carbonyl)phenoxy)ethoxy)ethoxy)ethoxy)propanoic acid.

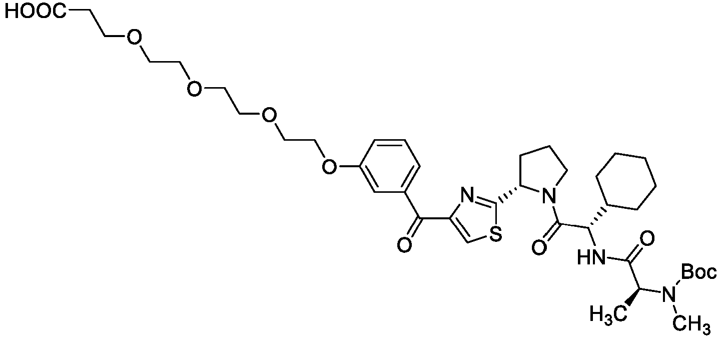

Step 1 product: methyl 3-(2-(2-(2-(tosyloxy)ethoxy)ethoxy)ethoxy)propanoate (1.0 g, 60%). LCMS; C₁₇H₂₆O₈S requires: 390, found: m/z = 391 [M+H]⁺.

Step 2 product: methyl 3-(2-(2-(2-(3-(2-((S)-1-((S)-2-((S)-2-((tert-butoxycarbonyl)(methyl)amino)propanamido)-2-cyclohexylacetyl)pyrrolidin-2-yl)thiazole-4-carbonyl)phenoxy)ethoxy)ethoxy)ethoxy)propanoate (1.0 g, 61%). LCMS; C₄₁H₆₀N₄O₁₁S requires: 816, found: m/z = 817 [M+H]⁺.

Step 3 product: 3-(2-(2-(2-(3-(2-((S)-1-((S)-2-((S)-2-((tert-butoxycarbonyl)(methyl)amino)propanamido)-2-cyclohexylacetyl)pyrrolidin-2-yl)thiazole-4-carbonyl)phenoxy)ethoxy)ethoxy)ethoxy)propanoic acid (573.3 mg, 58%). LCMS; C₄₀H₅₈N₄O₁₁S requires: 802, found: m/z = 803 [M+H]⁺.

### Example 2K-3: Synthesis of 1-(3-(2-((S)-1-((S)-2-((S)-2-((tert-butoxycarbonyl)(methyl)amino)propanamido)-2-cyclohexylacetyl)pyrrolidin-2-yl)thiazole-4-carbonyl)phenoxy)-3,6,9,12-tetraoxapentadecan-15-oic acid.

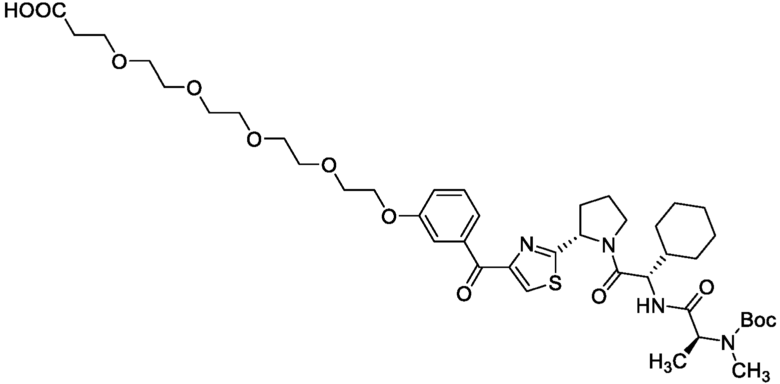

Step 1 product: methyl 1-[(4-methylbenzenesulfonyl)oxy]-3,6,9,12-tetraoxapentadecan-15-oate (1.28 g, 85%). LCMS; C₁₉H₃₀O₉S requires: 434, found: m/z = 435 [M+H]⁺.

Step 2 product: methyl 1-(3-(2-((S)-1-((S)-2-((S)-2-((tert-butoxycarbonyl)(methyl)amino)propanamido)-2-cyclohexylacetyl)pyrrolidin-2-yl)thiazole-4-carbonyl)phenoxy)-3,6,9,12-tetraoxapentadecan-15-oate (1.2 g, 60%). LCMS; C₄₃H₆₄N₄O₁₂S requires: 860, found: m/z = 861 [M+H]⁺.

Step 3 product: 1-(3-(2-((S)-1-((S)-2-((S)-2-((tert-butoxycarbonyl)(methyl)amino)propanamido)-2-cyclohexylacetyl)pyrrolidin-2-yl)thiazole-4-carbonyl)phenoxy)-3,6,9,12-tetraoxapentadecan-15-oic acid (805.2 mg, 68%). LCMS; C₄₂H₆₂N₄O₁₂S requires: 846, found: m/z = 847 [M+H]⁺.

### Example 2L: Synthesis of 3-(3-(((3S,5S)-1-((S)-2-((S)-2-((tert-butoxycarbonyl)(methyl)amino)propanamido)-2-cyclohexylacetyl)-5-(((R)-1,2,3,4-tetrahydronaphthalen-1-yl)carbamoyl)pyrrolidin-3-yl)amino)-3-oxopropoxy)propanoic acid.

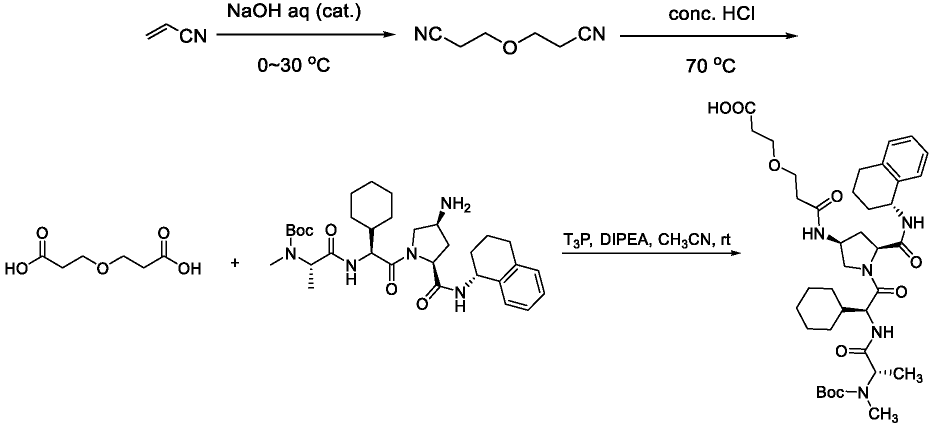

### Step 1: Synthesis of 3,3'-oxydipropanenitrile.

To a stirred solution of NaOH aqueous (3 mL, 40% wt) was added acrylonitrile (17.5 g, 330 mmol) dropwise at 0 °C. The solution was stirred at 30 °C for 16 h. When the reaction was completed, the reaction was diluted with 100 mL H₂O and neutralized to pH 7 by HCl (2 N). The aqueous solution was extracted with ethyl acetate (100 mL × 3). The combined organic solution was dried over anhydrous Na₂SO₄ and concentrated to give 3,3'-oxydipropanenitrile (4.1 g, crude) as yellow oil, which was used for the next step without further purification. ¹H NMR (300 MHz, Chloroform-d) δ 3.74 (t, *J* = 6.3 Hz, 4H), 2.65 (t, *J* = 6.3 Hz, 4H).

### Step 2: Synthesis of 3,3'-oxydipropionic acid.

A mixture of 3,3'-oxydipropanenitrile (4.1 g, 33 mmol) and concentrated HCl (38 mL) was stirred at 70 °C for 16 h. After cooled to room temperature, the solids were filtered out by filtration and the filtrate was concentrated under vacuum. The crude residue was purified by flash column chromatography with 30~ 100% ethyl acetate in petroleum ether to afford 3,3'-oxydipropionic acid (3.2 g, 12% over 2 steps) as yellow oil. ¹H NMR (400 MHz, DMSO-*d₆*) δ 12.20 (s, 2H), 3.62 - 3.55 (m, 4H), 2.42 - 2.40 (m, 4H).

### Step 3: Synthesis of 3-(3-(((3S,5S)-1-((S)-2-((S)-2-((tert-butoxycarbonyl)(methyl)amino)propanamido)-2-cyclohexylacetyl)-5-(((R)-1,2,3,4-tetrahydronaphthalen-1-yl)carbamoyl)pyrrolidin-3-yl)amino)-3-oxopropoxy)propanoic acid.

To a stirred solution of tert-butyl ((S)-1-(((S)-2-((2S,4S)-4-amino-2-(((R)-1,2,3,4-tetrahydronaphthalen-1-yl)carbamoyl)pyrrolidin-1-yl)-1-cyclohexyl-2-oxoethyl)amino)-1-oxopropan-2-yl)(methyl)carbamate (1.5 g, 2.57 mmol), 3,3'-oxydipropionic acid (2.78 g, 12.86 mmol) and DIEA (1.65 g, 12.86 mmol) in acetonitrile (30 mL) was added T₃P (12.3 g, 10.28 mmol, 50% in ethyl acetate) under nitrogen. The solution was stirred at 20 °C for 16 h. When the reaction was completed, the reaction was quenched by the addition of 50 mL H₂O and the aqueous solution was extracted with ethyl acetate (50 mL × 3). The combined organic layer was washed with brine, dried over anhydrous Na₂SO₄ and concentrated under vacuum_{.} The crude residue was purified by reverse phase flash column chromatography with 5~50% acetonitrile in water to afford 3-(3-(((3S,5S)-1-((S)-2-((S)-2-((tert-butoxycarbonyl)(methyl)amino)propanamido)-2-cyclohexylacetyl)-5-(((R)-1,2,3,4-tetrahydronaphthalen-1-yl)carbamoyl)pyrrolidin-3-yl)amino)-3-oxopropoxy)propanoic acid (1.0929 g, 58%) as a white solid. ¹H NMR (400 MHz, Methanol-*d₄*) δ 7.48 - 7.36 (m, 1H), 7.23 - 7.03 (m, 3H), 5.07 - 5.06 (m, 1H), 4.63 - 4.30 (m, 4H), 4.21 - 4.18 (m, 1H), 3.72 - 3.67 (m, 4H), 3.55 - 3.51 (m, 1H), 2.91 (s, 3H), 2.91 - 2.73 (m, 2H), 2.67 - 2.41 (m, 5H), 2.04 - 1.61 (m, 11H), 1.49 (s, 9H), 1.38 - 1.00 (m, 8H). MS (ESI) calculated for (C₃₈H₅₇N₅O₉) [M+H]⁺, 728.4; found, 728.7.

### Example 2M: Synthesis of 3-(2-(2-(3-(((3S,5S)-1-((S)-2-((S)-2-((tert-butoxycarbonyl)(methyl)amino)propanamido)-2-cyclohexylacetyl)-5-(((R)-1,2,3,4-tetrahydronaphthalen-1-yl)carbamoyl)pyrrolidin-3-yl)amino)-3-oxopropoxy)ethoxy)ethoxy)propanoic acid.

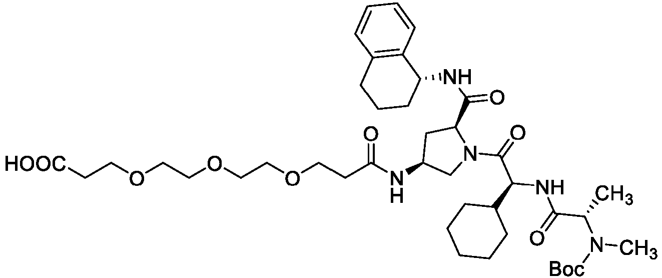

### Step 1: Synthesis of 3,3'-((oxybis(ethane-2,1-diyl))bis(oxy))dipropanenitrile.

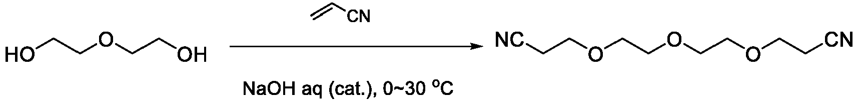

To a stirred solution of 2,2'-oxybis(ethan-1-ol) (15 g, 141 mmol) and NaOH aqueous (1.7 mL, 40% wt) was added acrylonitrile (17.25 g, 325 mmol) dropwise at 0 °C. The solution was stirred at 30 °C for 16 h. When the reaction was completed, the reaction was diluted with 100 mL H₂O and neutralized to pH 7 by HCl (2 N). The aqueous solution was extracted with ethyl acetate. The combined organic layer was dried over anhydrous Na₂SO₄ and concentrated under vacuum to afford 3,3'-((oxybis(ethane-2,1-diyl))bis(oxy))dipropanenitrile (26 g, crude) as yellow oil, which was used for the next step without further purification. ¹H NMR (300 MHz, Chloroform-d) δ 3.72 (t, *J=* 6.3 Hz, 4H), 3.67 (s, 8H), 2.62 (t, *J* = 6.3 Hz, 4H).

### Step 2: Synthesis of 3,3'-((oxybis(ethane-2,1-diyl))bis(oxy))dipropionic acid.

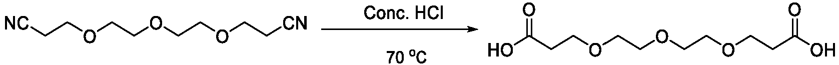

A mixture of 3,3'-((oxybis(ethane-2,1-diyl))bis(oxy))dipropanenitrile (26 g, 123 mmol) and concentrated HCl (140 mL) was stirred at 70 °C overnight. After cooled to room temperature, the solids were filtered out by filtration and the filtrate was concentrated under vacuum. The crude residue was purified by flash column chromatography with 30~100% ethyl acetate in petroleum ether to afford 3,3'-((oxybis(ethane-2,1-diyl))bis(oxy))dipropionic acid (20.9 g, 70% over 2 steps) as yellow oil. ¹H NMR (400 MHz, DMSO-*d6*) δ 12.16 (s, 2H), 3.61 - 3.57 (m, 4H), 3.51 - 3.47 (m, 8H), 2.44 (t, *J* = 6.3 Hz, 4H).

### Step 3: Synthesis of 3-(2-(2-(3-(((3S,5S)-1-((S)-2-((S)-2-((tert-butoxycarbonyl)(methyl)amino)propanamido)-2-cyclohexylacetyl)-5-(((R)-1,2,3,4-tetrahydronaphthalen-1-yl)carbamoyl)pyrrolidin-3-yl)amino)-3-oxopropoxy)ethoxy)ethoxy)propanoic acid.

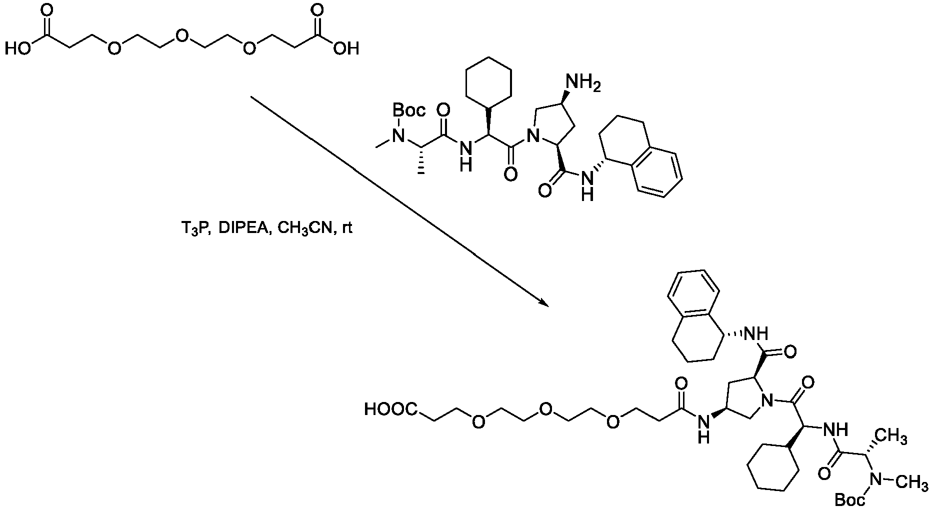

To a stirred solution of tert-butyl ((S)-1-(((S)-2-((2S,4S)-4-amino-2-(((R)-1,2,3,4-tetrahydronaphthalen-1-yl)carbamoyl)pyrrolidin-1-yl)-1-cyclohexyl-2-oxoethyl)amino)-1-oxopropan-2-yl)(methyl)carbamate (1.0 g, 1.71 mmol), 3,3'-((oxybis(ethane-2,1-diyl))bis(oxy))dipropionic acid (1.15 g, 3.43 mmol) and DIEA (1.1 g, 8.57 mmol) in acetonitrile (20 mL) was added T₃P (8.66 g, 6.86 mmol, 50% in EtOAc) under nitrogen. The resulting mixture was stirred at room temperature for 16 h. When the reaction was completed, the reaction was quenched by the addition of 50 mL H₂O. The aqueous solution was extracted with ethyl acetate. The combined organic layer was washed with brine, dried over anhydrous Na₂SO₄ and concentrated under vacuum. The residue was purified by pre-HPLC with the following conditions: [(Column: X Bridge Prep OBD C18 Column 30×150mm 5um; Mobile Phase A: Water(10mmol/L NH₄HCO₃), Mobile Phase B: ACN; Flow rate: 60 mL/min; Gradient: 28% B to 44% B in 7 min; 254/220 nm] to afford 3-(2-(2-(3-(((3S,5S)-1-((S)-2-((S)-2-((tert-butoxycarbonyl)(methyl)amino)propanamido)-2-cyclohexylacetyl)-5-(((R)-1,2,3,4-tetrahydronaphthalen-1-yl)carbamoyl)pyrrolidin-3-yl)amino)-3-oxopropoxy)ethoxy)ethoxy)propanoic acid (215.4 mg, 15%) as a white solid. ¹H NMR (300 MHz, DMSO-*d₆*) δ 8.39 (d, *J=* 8.7 Hz, 1H), 8.22 (d, *J=* 7.5 Hz, 1H), 7.80 - 7.70 (m, 1H), 7.32 (d, *J=* 7.2 Hz, 1H), 7.22 - 6.98 (m, 3H), 4.94 - 4.92 (m, 1H), 4.51 - 4.49 (m, 1H), 4.28 - 4.26 (m, 3H), 4.09 (t, *J* = 8.7 Hz, 1H), 3.60 - 3.58 (m, 4H), 3.49 (s, 8H), 2.75 - 2.73 (m, 5H), 2.35 - 2.31 (m, 5H), 1.99 - 1.50 (m, 11H), 1.40 (s, 9H), 1.30 - 0.82 (m, 9H). MS (ESI) calculated for (C₄₂H₆₅N₅O₁₁) [M+H]⁺, 816.5; found, 816.5.

### Example 2N: General procedure (E) for the synthesis of a compound of the present disclosure according to route 1 of synthetic scheme 5.

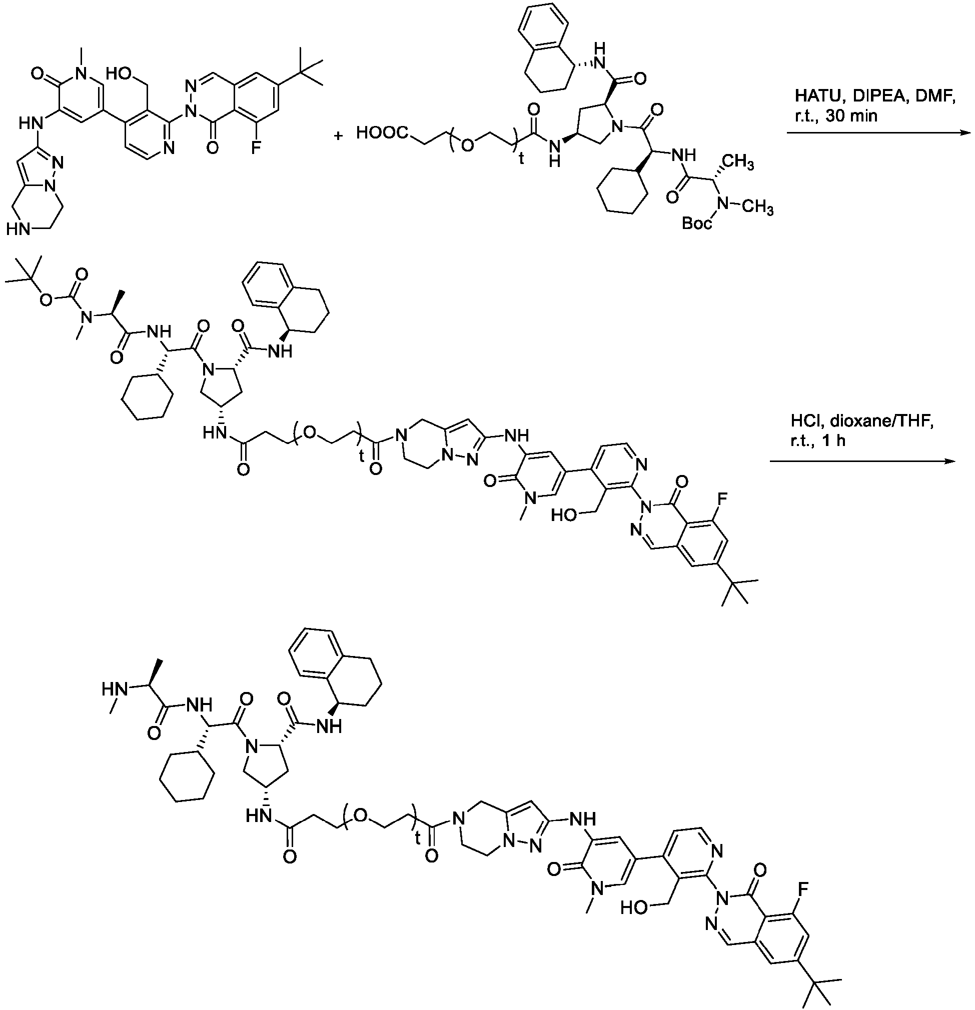

Step 1: A mixture of 2'-(6-tert-butyl-8-fluoro-1-oxo-1,2-dihydrophthalazin-2-yl)-3'-(hydroxymethyl)-1-methyl-5-({4H,5H,6H,7H-pyrazolo[1,5-a]pyrazin-2-yl}amino)-1,6-dihydro-[3,4'-bipyridin]-6-one (0.04 mmol), carboxylic acid (where t is an integer from 1 to 11) (0.05 mmol), HATU (0.06 mmol), ethylbis(propan-2-yl)amine (0.22 mmol) and DMF (0.60 mL) was allowed to stir at r.t. for 30 min. The mixture was purified by HPLC (10-95% MeCN in H₂O with 0.1% TFA) to afford the amide product (72%).

Step 2: A mixture of the amide (0.03 mmol), THF (0.20 mL), hydrogen chloride (4M in dioxane, 0.20 mL) was allowed to stir at r.t. for 1 hour. The volatiles were removed and the mixture was purified by HPLC (10-95% MeCN in H₂O with 0.1% TFA) to afford the product (63%).

### Example 2N-1: Synthesis of (2S,4S)-4-(3-(3-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridinl-5-yl)amino)-6,7-dihydropyrazolo[1,5-a]pyrazin-5(4H)-yl)-3-oxopropoxy)propanamido)-1-((S)-2-cyclohexyl-2-((S)-2-(methylamino)propanamido)acetyl)-N-((R)-1,2,3,4-tetrahydronaphthalen-1-yl)pyrrolidine-2-carboxamide. (Compound 4)

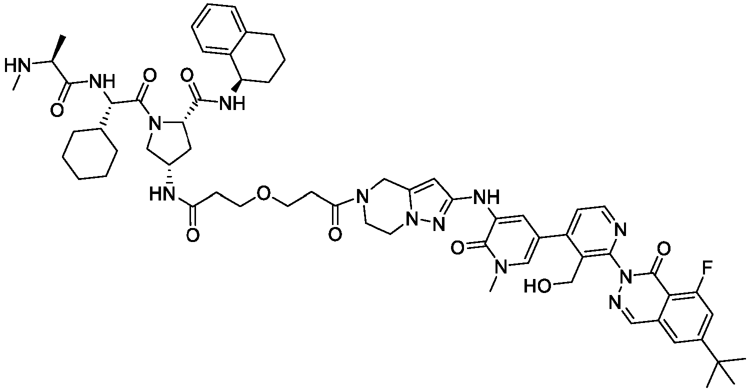

Step 1 product: tert-butyl ((S)-1-(((S)-2-((2S,4S)-4-(3-(3-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-3-oxopropoxy)propanamido)-2-(((R)-1,2,3,4-tetrahydronaphthalen-1-yl)carbamoyl)pyrrolidin-1-yl)-1-cyclohexyl-2-oxoethyl)amino)-1-oxopropan-2-yl)(methyl)carbamate (40.1 mg, 71.5%). LCMS; C₆₈H₈₆FN₁₃O₁₁ requires: 1279, found: m/z = 1280 [M+H]⁺.

Step 2 product: (2S,4S)-4-(3-(3-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-3-oxopropoxy)propanamido)-1-((S)-2-cyclohexyl-2-((S)-2-(methylamino)propanamido)acetyl)-N-((R)-1,2,3,4-tetrahydronaphthalen-1-yl)pyrrolidine-2-carboxamide (23.3 mg, 63%). LCMS; C₆₃H₇₈FN₁₃O₉ requires: 1279, found: m/z = 1280 [M+H]⁺.

### Example 2N-2: Synthesis of (2S,4S)-4-(3-(2-(2-(3-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-3-oxopropoxy)ethoxy)ethoxy)propanamido)-1-((S)-2-cyclohexyl-2-((S)-2-(methylamino)propanamido)acetyl)-N-((R)-1,2,3,4-tetrahydronaphthalen-1-yl)pyrrolidine-2-carboxamide. (Compound 5)

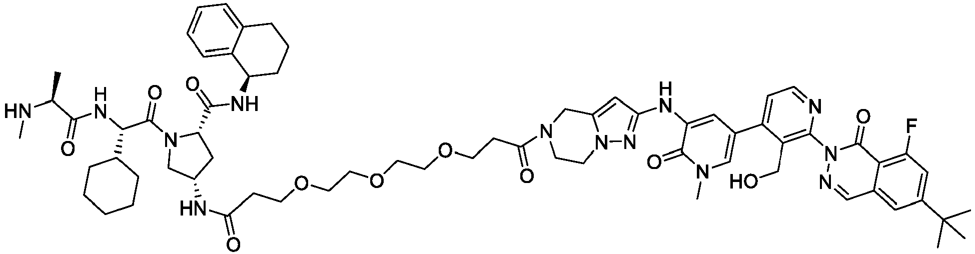

Step 1 product: tert-butyl ((S)-1-(((S)-2-((2S,4S)-4-(3-(2-(2-(3-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-3-oxopropoxy)ethoxy)ethoxy)propanamido)-2-(((R)-1,2,3,4-tetrahydronaphthalen-1-yl)carbamoyl)pyrrolidin-1-yl)-1-cyclohexyl-2-oxoethyl)amino)-1-oxopropan-2-yl)(methyl)carbamate (40.9 mg, 68.2%). LCMS; C₇₂H₉₄FN₁₃O₁₃ requires: 1367, found: m/z = 1368 [M+H]⁺.

Step 2 product: (2S,4S)-4-(3-(2-(2-(3-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-3-oxopropoxy)ethoxy)ethoxy)propanamido)-1-((S)-2-cyclohexyl-2-((S)-2-(methylamino)propanamido)acetyl)-N-((R)-1,2,3,4-tetrahydronaphthalen-1-yl)pyrrolidine-2-carboxamide (31.8 mg, 83.8%). LCMS; C₆₇H₈₆FN₁₃O₁₁ requires: 1267, found: m/z = 1268 [M+H]⁺.

### Example 2O: General procedure (F) for the synthesis of a compound of the present disclosure according to route 1 of synthetic scheme 5.

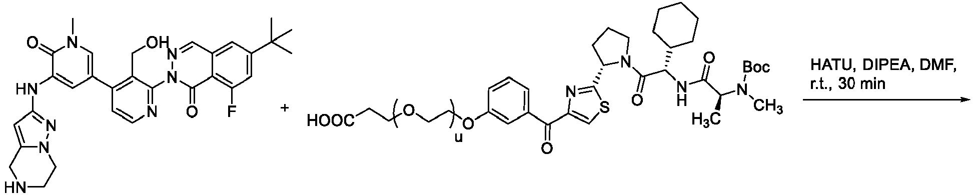

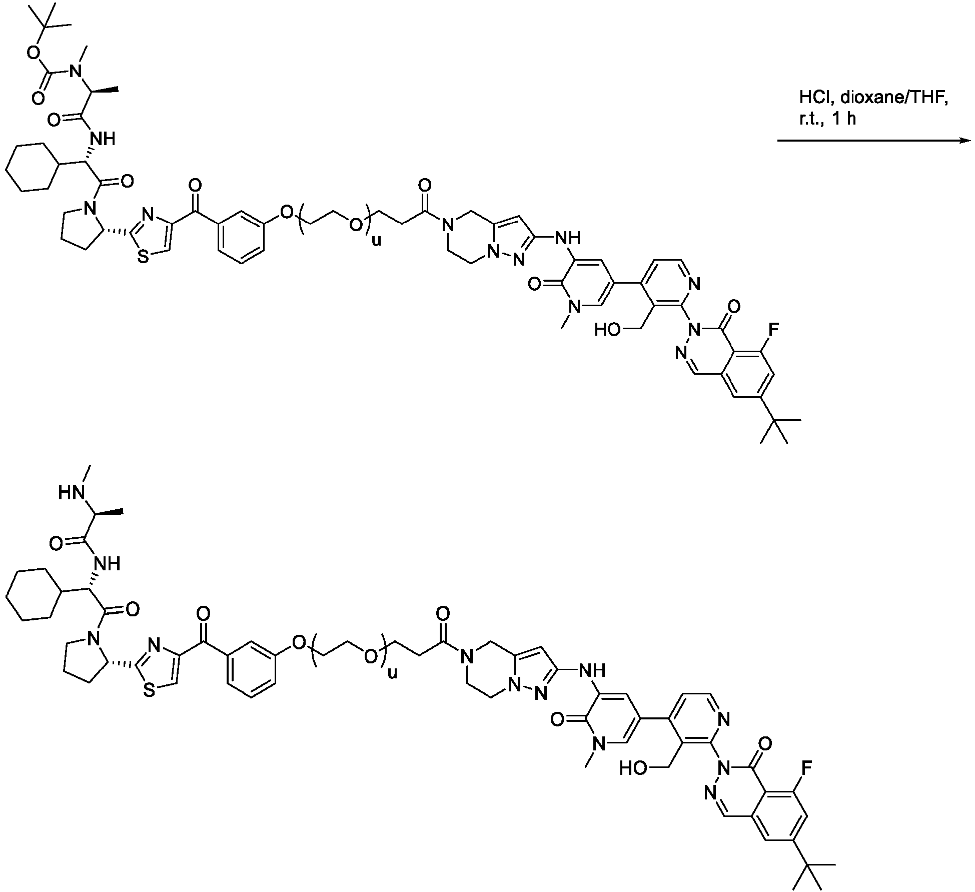

Step 1: A mixture of 2'-(6-tert-butyl-8-fluoro-1-oxo-1,2-dihydrophthalazin-2-yl)-3'-(hydroxymethyl)-1-methyl-5-({4H,5H,6H,7H-pyrazolo[1,5-a]pyrazin-2-yl}amino)-1,6-dihydro-[3,4'-bipyridin]-6-one (0.04 mmol), carboxylic acid (where u is an integer from 1 to 11) (0.05 mmol), HATU (0.06 mmol), ethylbis(propan-2-yl)amine (0.22 mmol) and DMF (0.60 mL) was allowed to stir at r.t. for 30 min. The mixture was purified by HPLC (10-95% MeCN in H₂O with 0.1% TFA) to afford the amide product (84%).

Step 2: A mixture of the amide (0.03 mmol), THF (0.20 mL), hydrogen chloride (4M in dioxane, 0.20 mL) was allowed to stir at r.t. for 1 hour. The volatiles were removed and the mixture was purified by HPLC (10-95% MeCN in H₂O with 0.1% TFA) to afford the product (38%).

### Example 2O-1: Synthesis of (S)-N-((S)-2-((S)-2-(4-(3-(2-(3-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-3-oxopropoxy)ethoxy)benzoyl)thiazol-2-yl)pyrrolidin-1-yl)-1-cyclohexyl-2-oxoethyl)-2-(methylamino)propanamide. (Compound 6)

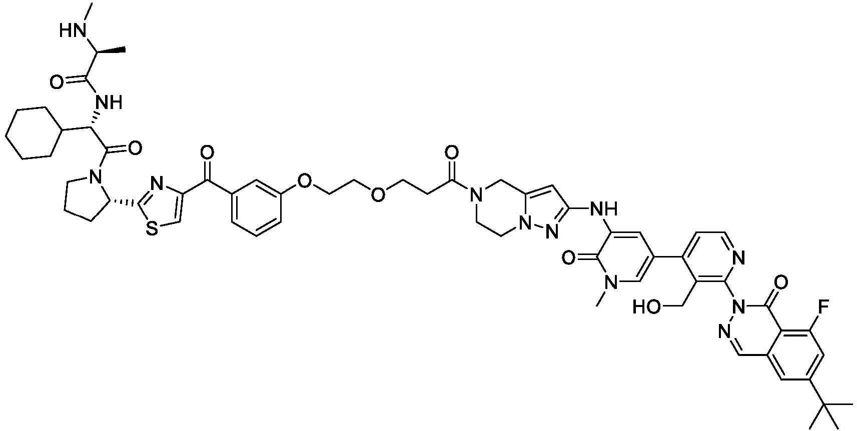

Step 1 product: tert-butyl ((S)-1-(((S)-2-((S)-2-(4-(3-(2-(3-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-3-oxopropoxy)ethoxy)benzoyl)thiazol-2-yl)pyrrolidin-1-yl)-1-cyclohexyl-2-oxoethyl)amino)-1-oxopropan-2-yl)(methyl)carbamate (40 mg, 74%). LCMS; C₆₆H₇₉FN₁₂O₁₁S requires: 1266, found: m/z = 1267 [M+H]⁺.

Step 2 product: (S)-N-((S)-2-((S)-2-(4-(3-(2-(3-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-3-oxopropoxy)ethoxy)benzoyl)thiazol-2-yl)pyrrolidin-1-yl)-1-cyclohexyl-2-oxoethyl)-2-(methylamino)propanamide (17.9 mg, 42%). LCMS; C₆₁H₇₁FN₁₂O₉S requires: 1166, found: m/z = 1167 [M+H]⁺.

### Example 2O-2: Synthesis of (S)-N-((S)-2-((S)-2-(4-(3-(2-(2-(2-(3-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-3-oxopropoxy)ethoxy)ethoxy)ethoxy)benzoyl)thiazol-2-yl)pyrrolidin-1-yl)-1-cyclohexyl-2-oxoethyl)-2-(methylamino)propanamide. (Compound 7)

Step 1 product: tert-butyl ((S)-1-(((S)-2-((S)-2-(4-(3-(2-(2-(2-(3-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-3-oxopropoxy)ethoxy)ethoxy)ethoxy)benzoyl)thiazol-2-yl)pyrrolidin-1-yl)-1-cyclohexyl-2-oxoethyl)amino)-1-oxopropan-2-yl)(methyl)carbamate (50.0 mg, 84%). LCMS; C₇₀H₈₇FN₁₂O₁₃S requires: 1354, found: m/z = 1355 [M+H]⁺.

Step 2 product: (S)-N-((S)-2-((S)-2-(4-(3-(2-(2-(2-(3-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-3-oxopropoxy)ethoxy)ethoxy)ethoxy)benzoyl)thiazol-2-yl)pyrrolidin-1-yl)-1-cyclohexyl-2-oxoethyl)-2-(methylamino)propanamide (15.9 mg, 38%). LCMS; C₆₅H₇₉FN₁₂O₁₁S requires: 1254, found: m/z = 1255 [M+H]⁺.

### Example 2O-3: Synthesis of (S)-N-((S)-2-((S)-2-(4-(3-((15-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-15-oxo-3,6,9,12-tetraoxapentadecyl)oxy)benzoyl)thiazol-2-yl)pyrrolidin-1-yl)-1-cyclohexyl-2-oxoethyl)-2-(methylamino)propanamide. (Compound 8)

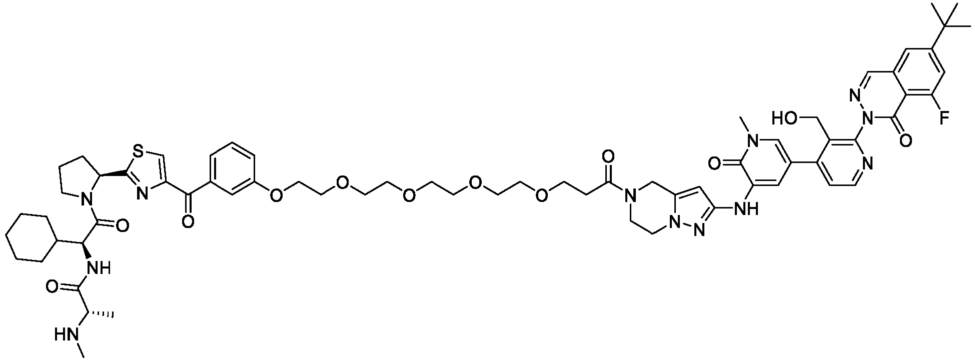

Step 1 product: tert-butyl ((S)-1-(((S)-2-((S)-2-(4-(3-((15-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-15-oxo-3,6,9,12-tetraoxapentadecyl)oxy)benzoyl)thiazol-2-yl)pyrrolidin-1-yl)-1-cyclohexyl-2-oxoethyl)amino)-1-oxopropan-2-yl)(methyl)carbamate (51.0 mg, 83.2%). LCMS; C₇₂H₉₁FN₁₂O₁₄S requires: 1398, found: m/z = 1399 [M+H]⁺.

Step 2 product: (S)-N-((S)-2-((S)-2-(4-(3-((15-(2-((2'-(6-(tert-butyl)-8-fluoro-1-oxophthalazin-2(1H)-yl)-3'-(hydroxymethyl)-1-methyl-6-oxo-1,6-dihydro-[3,4'-bipyridin]-5-yl)amino)-6,7-dihydropyrazolo[1,5-a]pyrazin-5(4H)-yl)-15-oxo-3,6,9,12-tetraoxapentadecyl)oxy)benzoyl)thiazol-2-yl)pyrrolidin-1-yl)-1-cyclohexyl-2-oxoethyl)-2-(methylamino)propanamide (19.2 mg, 71.3%). LCMS; C₆₇H₈₃FN₁₂O₁₂S requires: 1298, found: m/z = 1299 [M+H]⁺.

**Table 2: Bifunctional compounds generated according to Route 1 of Scheme 5 and using the procedures in Examples 1 and 2.**

| **Cmpd No.** | **Characterization data** |
|---|---|
| 1 | ¹H NMR (500 MHz, DMSO-*d*₆) δ 11.01 (s, 1H), 8.49 (d, *J* = 5.0 Hz, 1H), 8.46 (d, *J =* 2.5 Hz, 1H), 8.20 (d, *J =* 10.7 Hz, 1H), 7.96 (dd, *J =* 10.6, 2.3 Hz, 1H), 7.83 (d, *J* = 1.8 Hz, 1H), 7.70 (dd, *J* = 13.1, 1.7 Hz, 1H), 7.48 (ddd, *J* = 10.3, 7.4, 3.1 Hz, 1H), 7.42 (dd, *J = 5.1,* 1.7 Hz, 1H), 7.32 (t, *J =* 2.5 Hz, 1H), 7.04 (dd, *J =* 12.7, 8.5 Hz, 1H), 6.95 (dd, *J* = 7.4, 4.4 Hz, 1H), 6.49 (d, *J* = 22.5 Hz, 1H), 5.92 (d, *J =* 3.1 Hz, 1H), 4.96 (dd, *J* = 12.8, 5.4 Hz, 1H), 4.61 (d, *J =* 51.0 Hz, 2H), 4.35 (d, *J* = 2.9 Hz, 2H), 4.00 - 3.74 (m, 4H), 3.63 (t, *J* = 6.5 Hz, 2H), 3.56 - 3.47 (m, 5H), 3.37 (d, *J =* 20.4 Hz, 3H), 2.89 - 2.47 (m, 4H), 1.99 - 1.85 (m, 1H), 1.32 (s, 9H). |
| | LCMS: C₄₈H₄₈FN₁₁O₉ requires: 941, found: m/z = 942 [M+H]⁺. |
| 2 | ¹H NMR (500 MHz, DMSO-*d*₆) δ 10.91 (s, 1H), 8.51 - 8.42 (m, 2H), 8.22 (d, *J* = 26.8 Hz, 1H), 7.99 - 7.93 (m, 1H), 7.83 (d, *J = 1.7* Hz, 1H), 7.70 (dd, *J* = 13.2, 1.8 Hz, 1H), 7.59 - 7.52 (m, 1H), 7.51 - 7.45 (m, 1H), 7.45 - 7.29 (m, 4H), 5.94 (d, *J =* 9.1 Hz, 1H), 5.06 (dd, *J =* 13.3, 5.2 Hz, 1H), 4.62 (d, *J* = 52.6 Hz, 2H), 4.41 - 4.07 (m, 4H), 3.99 - 3.74 (m, 4H), 3.53 (d, *J* = 10.7 Hz, 5H), 3.29 (q, *J* = 7.2, 6.8 Hz, 2H), 2.85 (ddd, *J* = 17.9, 13.5, 5.3 Hz, 1H), 2.67 - 2.47 (m, 4H), 2.00 - 1.85 (m, 1H), 1.72 (dt, *J* = 19.8, 8.4 Hz, 2H), 1.32 (s, 9H). |
| | LCMS: C₄₉H₅₁FN₁₀O₈ requires: 926, found: m/z = 927 [M+H]⁺. |
| 3 | ¹H NMR (500 MHz, DMSO-*d*₆) δ 11.02 (s, 1H), 8.50 - 8.44 (m, 2H), 8.23 (d, *J* = 31.5 Hz, 1H), 7.98 (dd, *J =* 13.2, 2.3 Hz, 1H), 7.83 (d, *J =* 1.7 Hz, 1H), 7.70 (dd, *J =* 13.1, 1.8 Hz, 1H), 7.52 - 7.46 (m, 1H), 7.42 (d, *J* = 5.0 Hz, 1H), 7.32 (d, *J =* 2.4 Hz, 1H), 7.05 (dd, *J =* 8.6, 6.1 Hz, 1H), 6.96 (dd, *J =* 7.2, 3.3 Hz, 1H), 6.51 (s, 1H), 5.93 (d, *J* = 4.2 Hz, 1H), 4.98 (dd, *J* = 12.8, 5.5 Hz, 1H), 4.60 (d, *J* = 48.0 Hz, 2H), 4.35 (d, *J =* 2.6 Hz, 2H), 4.00 - 3.73 (m, 5H), 3.56 (t, *J* = 6.5 Hz, 2H), 3.48 - 3.27 (m, 16H), 2.81 (ddd, *J* = 16.9, 13.7, 5.4 Hz, 1H), 2.65 - 2.48 (m, 3H), 2.00 - 1.88 (m, 1H), 1.32 (s, 9H). |
| | LCMS: C₅₂H₅₆FN₁₁O₁₁ requires: 1029, found: m/z = 1030 [M+H]⁺. |
| 4 | ¹H NMR (500 MHz, DMSO-*d*₆) δ 8.79 - 8.66 (m, 3H), 8.51 - 8.45 (m, 2H), 8.33 - 8.25 (m, 1H), 8.21 (s, 1H), 8.10 (d, *J* = 7.5 Hz, 1H), 8.01 - 7.95 (m, 1H), 7.83 (d, *J* = 1.7 Hz, 1H), 7.70 (dd, *J* = 13.1, 1.8 Hz, 1H), 7.42 (dd, *J* = 5.2, 2.3 Hz, 1H), 7.33 (d, *J =* 2.3 Hz, 1H), 7.22 (d, *J* = 7.5 Hz, 1H), 7.11 - 6.98 (m, 3H), 5.94 (d, *J=* 2.6 Hz, 1H), 4.86 (q, *J* = 9.1, 8.5 Hz, 1H), 4.61 (d, *J* = 42.7 Hz, 2H), 4.39 - 4.27 (m, 3H), 4.19 (dt, *J* = 14.5, 7.9 Hz, 2H), 4.07 (dd, *J* = 9.8, 7.1 Hz, 1H), 3.95 (s, 1H), 3.90 - 3.72 (m, 4H), 3.55 (t, *J* = 6.8 Hz, 3H), 3.22 (t, *J =* 9.1 Hz, 1H), 2.72 - 2.56 (m, 4H), 2.33 - 2.18 (m, 3H), 1.88 - 1.47 (m, 12H), 1.32 (s, 9H), 1.25 (d, *J* = 6.9 Hz, 3H), 1.17 - 0.88 (m, 4H). |
| | LCMS: C₆₃H₇₈FN₁₃O₉ requires: 1179, found: m/z = 1180 [M+H]⁺. |
| 5 | ¹H NMR (500 MHz, DMSO-*d*₆) δ 8.78 - 8.66 (m, 5H), 8.51 - 8.43 (m, 2H), 8.31 (d, *J =* 8.7 Hz, 1H), 8.24 (d, *J =* 30.6 Hz, 1H), 8.12 (d, *J =* 7.6 Hz, 1H), 7.98 (dd, *J =* 14.5, 2.3 Hz, 1H), 7.83 (d, *J* = 1.8 Hz, 1H), 7.70 (dd, *J* = 13.1, 1.8 Hz, 1H), 7.42 (d, *J* = 5.1 Hz, 1H), 7.33 (d, *J* = 2.3 Hz, 1H), 7.22 (d, *J* = 7.5 Hz, 1H), 7.12 - 6.97 (m, 4H), 5.93 (s, 1H), 4.86 (td, *J* = 8.3, 4.4 Hz, 1H), 4.61 (d, *J* = 51.2 Hz, 2H), 4.40 - 4.28 (m, 3H), 4.19 (dt, *J* = 15.0, 8.0 Hz, 2H), 4.07 (dd, *J* = 9.6, 7.0 Hz, 1H), 3.96 (s, 1H), 3.90 - 3.74 (m, 5H), 3.57 (t, *J* = 6.5 Hz, 2H), 3.40 (dd, *J =* 10.9, 5.7 Hz, 9H), 3.21 (t, *J =* 9.1 Hz, 1H), 2.71 - 2.55 (m, 5H), 2.34 - 2.18 (m, 3H), 1.87 - 1.48 (m, 16H), 1.32 (s, 10H), 1.25 (d, *J =* 6.9 Hz, 3H), 1.17 - 0.87 (m, 6H). |
| | LCMS: C₆₇H₈₆FN₁₃O₁₁ requires: 1267, found: m/z = 1268 [M+H]⁺. |
| 6 | ¹H NMR (500 MHz, DMSO-*d*₆) δ 8.72 (s, 2H), 8.65 (d, *J =* 8.0 Hz, 1H), 8.51 - 8.40 (m, 3H), 8.22 (d, *J* = 22.5 Hz, 1H), 7.97 (dd, *J=* 11.2, 2.3 Hz, 1H), 7.83 (d, *J* = 1.7 Hz, 1H), 7.69 (dd, *J* = 13.1, 1.7 Hz, 1H), 7.58 (d, *J* = 7.7 Hz, 1H), 7.55 (dd, *J =* 2.7, 1.5 Hz, 1H), 7.42 (d, *J* = 5.0 Hz, 1H), 7.39 - 7.30 (m, 2H), 7.16 (dd, *J =* 8.1, 2.8 Hz, 1H), 5.93 (s, 1H), 5.31 (dd, *J* = 7.8, 3.3 Hz, 1H), 4.66 (s, 1H), 4.56 (s, 1H), 4.40 (t, *J* = 7.6 Hz, 1H), 4.35 (d, *J =* 2.5 Hz, 2H), 4.08 (dd, *J* = 11.1, 6.6 Hz, 2H), 3.95 (s, 1H), 3.88 - 3.62 (m, 11H), 2.65 (dt, *J* = 23.4, 6.5 Hz, 2H), 2.33 - 2.26 (m, 1H), 2.16 (d, *J* = 17.4 Hz, 2H), 1.98 (d, *J* = 20.3 Hz, 2H), 1.71 - 1.41 (m, 7H), 1.26 (d, *J =* 6.9 Hz, 3H), 0.99 (ddd, *J* = 41.7, 23.1, 9.0 Hz, 10H). |
| | LCMS: C₆₁H₇₁FN₁₂O₉S requires: 1166, found: m/z = 1167 [M+H]⁺. |
| 7 | ¹H NMR (500 MHz, DMSO-*d*₆) δ 8.72 (d, *J* = 12.6 Hz, 2H), 8.65 (d, *J* = 8.1 Hz, 1H), 8.48 (d, *J* = 5.0 Hz, 1H), 8.46 (d, *J* = 2.4 Hz, 1H), 8.43 (d, *J* = 8.4 Hz, 1H), 8.23 (d, *J =* 30.9 Hz, 1H), 7.98 (dd, *J =* 12.5, 2.3 Hz, 1H), 7.82 (d, *J* = 1.7 Hz, 1H), 7.69 (dd, *J* = 13.1, 1.7 Hz, 1H), 7.63 - 7.52 (m, 2H), 7.42 (d, *J =* 5.0 Hz, 1H), 7.38 (t, *J* = 7.9 Hz, 1H), 7.33 (d, *J* = 2.3 Hz, 1H), 7.17 (dd, *J* = 8.2, 2.9 Hz, 1H), 5.93 (d, *J* = 4.4 Hz, 1H), 5.32 (dd, *J* = 7.8, 3.3 Hz, 1H), 4.60 (d, *J* = 49.4 Hz, 2H), 4.41 (t, *J=* 7.6 Hz, 1H), 4.35 (d, *J=* 2.5 Hz, 2H), 4.07 (q, *J=* 4.8 Hz, 2H), 3.94 (d, *J* = 5.8 Hz, 1H), 3.90 - 3.65 (m, 7H), 3.56 (t, *J* = 6.5 Hz, 2H), 3.54 - 3.44 (m, 6H), 2.60 (dt, *J* = 22.1, 6.2 Hz, 2H), 2.32 - 2.26 (m, 1H), 2.24 - 2.08 (m, 2H), 1.97 (dt, *J* = 12.0, 8.4 Hz, 2H), 1.73 - 1.41 (m, 6H), 1.26 (d, *J* = 6.9 Hz, 2H), 1.14 - 0.78 (m, 8H). |
| | LCMS: C₆₅H₇₉FN₁₂O₁₁S requires: 1254, found: m/z = 1255 [M+H]⁺. |
| 8 | ¹H NMR (500 MHz, DMSO-*d*₆) δ 8.78 - 8.68 (m, 2H), 8.65 (d, *J* = 8.1 Hz, 1H), 8.48 (d, *J =* 5.0 Hz, 1H), 8.46 (d, *J = 2.5* Hz, 1H), 8.42 (s, 1H), 8.23 (d, *J = 30.2* Hz, 1H), 8.02 - 7.95 (m, 1H), 7.82 (d, *J* = 1.7 Hz, 1H), 7.69 (dd, *J =* 13.0, 1.7 Hz, 1H), 7.62 - 7.51 (m, 2H), 7.44 - 7.36 (m, 2H), 7.32 (d, *J* = 2.3 Hz, 1H), 7.18 (dd, *J =* 8.1, 2.5 Hz, 1H), 5.93 (d, *J = 4.0* Hz, 1H), 5.32 (dd, *J* = 7.7, 3.4 Hz, 1H), 4.60 (d, *J =* 50.0 Hz, 2H), 4.41 (t, *J =* 7.6 Hz, 1H), 4.35 (d, *J =* 2.6 Hz, 2H), 4.08 (p, *J =* 2.9 Hz, 2H), 3.94 (d, *J=* 5.8 Hz, 1H), 3.90 - 3.62 (m, 8H), 3.59 - 3.42 (m, 9H), 2.60 (dt, *J* = 21.5, 6.2 Hz, 2H), 2.33 - 2.24 (m, 1H), 2.25 - 2.08 (m, 1H), 2.06 - 1.89 (m, 2H), 1.70 - 1.41 (m, 5H), 1.26 (d, *J* = 6.9 Hz, 5H), 1.15 - 0.80 (m, 8H). |
| | LCMS: C₆₇H₈₃FN₁₂O₁₂S requires: 1298, found: m/z = 1299 [M+H]⁺. |
| 9 | ¹H NMR (500 MHz, DMSO-*d*₆) δ 11.01 (s, 1H), 8.49 (d, *J =* 5.0 Hz, 1H), 8.46 (d, *J=* 2.5 Hz, 1H), 8.23 (d, *J* = 26.6 Hz, 1H), 7.98 (dd, *J =* 11.9, 2.3 Hz, 1H), 7.83 (d, *J* = 1.7 Hz, 1H), 7.70 (dd, *J* = 13.1, 1.8 Hz, 1H), 7.49 (q, *J* = 8.0 Hz, 1H), 7.43 (dd, *J =* 5.1, 2.9 Hz, 1H), 7.33 (d, *J* = 2.3 Hz, 1H), 7.09 (dd, *J* = 8.7, 2.6 Hz, 1H), 6.93 (d, *J* = 7.0 Hz, 1H), 6.60 (s, 1H), 5.94 (s, 1H), 4.97 (dd, *J* = 12.7, 5.4 Hz, 1H), 4.61 (d, *J* = 33.3 Hz, 2H), 4.35 (d, *J* = 2.9 Hz, 2H), 4.00 - 3.76 (m, 4H), 3.52 (s, 2H), 3.26 (s, 2H), 2.81 (ddd, *J =* 17.0, 13.7, 5.4 Hz, 1H), 2.60 - 2.45 (m, 3H), 1.95 (tt, *J* = 7.7, 5.8, 4.7 Hz, 1H), 1.80 - 1.68 (m, 2H), 1.32 (s, 9H). |
| | LCMS: C₄₇H₄₆FN₁₁O₈ requires: 911, found: m/z = 912 [M+H]⁺. |
| 10 | ¹H NMR (500 MHz, DMSO-*d*₆) δ 11.01 (d, *J* = 3.2 Hz, 1H), 8.49 (dd, *J* = 5.0, 2.6 Hz, 1H), 8.46 (t, *J* = 2.4 Hz, 1H), 8.23 (d, *J* = 15.2 Hz, 1H), 7.97 (d, *J = 2.3* Hz, 1H), 7.83 (d, *J =* 1.6 Hz, 1H), 7.70 (dt, *J =* 13.1, 2.1 Hz, 1H), 7.51 (ddd, *J* = 8.6, 7.0, 5.6 Hz, 1H), 7.43 (d, *J =* 5.0 Hz, 1H), 7.32 (t, *J =* 2.1 Hz, 1H), 7.08 (dd, *J* = 18.4, 8.7 Hz, 1H), 6.94 (dd, *J =* 11.4, 7.0 Hz, 1H), 6.70 (s, 1H), 5.92 (d, *J* = 20.0 Hz, 1H), 4.96 (dt, *J* = 12.7, 4.8 Hz, 1H), 4.68 - 4.55 (m, 2H), 4.35 (d, *J* = 3.9 Hz, 2H), 4.02 - 3.75 (m, 5H), 3.51 (d, *J* = 7.3 Hz, 6H), 2.87 - 2.64 (m, 3H), 2.57 - 2.45 (m, 2H), 2.00 - 1.88 (m, 1H), 1.32 (s, 9H). |
| | LCMS: C₄₆H₄₄FN₁₁O₈ requires: 897, found: m/z = 898 [M+H]⁺. |
| 11 | ¹H NMR (500 MHz, DMSO-*d*₆) δ 10.92 (s, 1H), 8.49 (d, *J* = 5.0 Hz, 1H), 8.46 (d, *J* = 2.5 Hz, 1H), 8.24 (d, *J* = 31.3 Hz, 1H), 8.01 - 7.95 (m, 1H), 7.83 (d, *J* = 1.8 Hz, 1H), 7.70 (dd, *J* = 13.1, 1.7 Hz, 1H), 7.49 (dd, *J* = 5.6, 3.1 Hz, 1H), 7.43 (dd, *J* = 5.2, 2.3 Hz, 1H), 7.41 - 7.36 (m, 2H), 7.32 (d, *J* = 2.3 Hz, 1H), 5.93 (d, *J =* 7.6 Hz, 1H), 5.06 (dd, *J =* 13.3, 5.1 Hz, 1H), 4.59 (d, *J* = 46.0 Hz, 2H), 4.43 - 4.18 (m, 4H), 3.94 (s, 1H), 3.87 - 3.80 (m, 4H), 3.52 (s, 3H), 2.85 (ddd, *J* = 17.3, 13.6, 5.4 Hz, 1H), 2.64 - 2.49 (m, 3H), 2.39 - 2.27 (m, 1H), 1.94 (dtd, *J* = 11.1, 6.1, 5.4, 2.9 Hz, 1H), 1.61 - 1.45 (m, 5H), 1.32 (s, 9H). |
| | LCMS: C₄₈H₄₉FN₁₀O₇ requires: 896, found: m/z = 897 [M+H]⁺. |
| 12 | ¹H NMR (500 MHz, DMSO-*d*₆) δ 11.03 (s, 1H), 8.50 (d, *J=* 5.0 Hz, 1H), 8.46 (d, *J=* 2.5 Hz, 1H), 8.28 (d, *J* = 39.6 Hz, 1H), 8.00 (dd, *J* = 17.2, 2.3 Hz, 1H), 7.83 (d, *J=* 1.8 Hz, 1H), 7.70 (dd, *J* = 13.1, 1.7 Hz, 1H), 7.54 (dd, *J =* 8.5, 7.1 Hz, 1H), 7.43 (dd, *J* = 5.1, 1.7 Hz, 1H), 7.33 (d, *J* = 2.3 Hz, 1H), 7.07 (d, *J* = 8.6 Hz, 1H), 7.02 - 6.95 (m, 2H), 5.99 (d, *J* = 14.4 Hz, 1H), 5.00 (dd, *J* = 12.9, 5.4 Hz, 1H), 4.69 (d, *J* = 43.7 Hz, 2H), 4.40 - 4.19 (m, 4H), 4.05 (d, *J* = 5.9 Hz, 1H), 3.91 (d, *J* = 9.1 Hz, 3H), 3.52 (s, 3H), 2.82 (ddd, *J* = 17.2, 13.9, 5.4 Hz, 1H), 2.61 - 2.46 (m, 1H), 2.00 - 1.91 (m, 1H), 1.32 (s, 9H). |
| | LCMS: C₄₅H₄₂FN₁₁O₈ requires: 883, found: m/z = 884 [M+H]⁺. |
| 13 | ¹H NMR (500 MHz, DMSO-*d*₆) δ 10.92 (s, 1H), 8.49 (d, *J=* 5.1 Hz, 1H), 8.46 (d, *J=* 2.5 Hz, 1H), 8.24 (d, *J* = 28.4 Hz, 1H), 8.02 - 7.93 (m, 1H), 7.83 (s, 1H), 7.70 (dd, *J=* 13.1, 1.8 Hz, 1H), 7.50 (dd, *J* = 5.8, 2.8 Hz, 1H), 7.45 - 7.36 (m, 3H), 7.32 (d, *J* = 2.4 Hz, 1H), 5.94 (d, *J* = 5.4 Hz, 1H), 5.06 (dd, *J =* 13.3, 5.1 Hz, 1H), 4.59 (d, *J* = 24.9 Hz, 2H), 4.41 - 4.29 (m, 3H), 4.23 (d, *J* = 17.2 Hz, 1H), 3.98 - 3.76 (m, 6H), 3.52 (s, 3H), 2.84 (t, *J* = 14.2 Hz, 1H), 2.68 - 2.52 (m, 5H), 1.99 - 1.70 (m, 4H), 1.32 (s, 9H). |
| | LCMS: C₄₇H₄₇FN₁₀O₇ requires: 882, found: m/z = 883 [M+H]⁺. |
| 14 | ¹H NMR (500 MHz, DMSO-*d*₆) δ 11.06 (s, 1H), 8.50 (d, *J* = 5.0 Hz, 1H), 8.46 (d, *J =* 2.5 Hz, 1H), 8.28 (d, *J =* 23.1 Hz, 1H), 7.99 (dd, *J =* 9.0, 2.4 Hz, 1H), 7.83 (d, *J =* 1.8 Hz, 1H), 7.77 - 7.63 (m, 4H), 7.42 (dd, *J =* 5.1, 2.6 Hz, 1H), 7.33 (q, *J =* 2.4 Hz, 1H), 5.96 (d, *J =* 5.5 Hz, 1H), 5.06 (dd, *J =* 12.8, 5.5 Hz, 1H), 4.61 (d, *J* = 33.1 Hz, 2H), 4.39 - 4.30 (m, 2H), 4.06 - 3.76 (m, 4H), 3.52 (d, *J* = 2.2 Hz, 3H), 3.16 - 2.72 (m, 7H), 2.61 - 2.46 (m, 1H), 2.04 - 1.82 (m, 4H), 1.32 (s, 9H). |
| | LCMS: C₅₂H₅₅FN₁₂O₈ requires: 994, found: m/z = 995 [M+H]⁺. |
| 15 | ¹H NMR (500 MHz, DMSO-*d*₆) δ 11.03 (s, 1H), 8.49 (dd, *J* = 5.1, 1.9 Hz, 1H), 8.46 (d, *J* = 2.5 Hz, 1H), 8.26 (d, *J =* 41.3 Hz, 1H), 8.00 (d, *J =* 5.7 Hz, 1H), 7.83 (d, *J =* 1.7 Hz, 1H), 7.70 (dd, *J =* 13.1, 1.8 Hz, 1H), 7.51 (dd, *J* = 8.7, 7.1 Hz, 1H), 7.43 (d, *J* = 5.0 Hz, 1H), 7.33 (d, *J* = 2.3 Hz, 1H), 7.14 (d, *J* = 8.6 Hz, 1H), 6.97 (dd, *J* = 7.0, 1.9 Hz, 1H), 6.09 (d, *J* = 8.1 Hz, 1H), 5.96 (d, *J* = 13.4 Hz, 1H), 4.99 (dd, *J* = 12.8, 5.5 Hz, 1H), 4.74 (s, 1H), 4.57 (s, 1H), 4.35 (d, *J=* 2.9 Hz, 2H), 4.04 - 3.80 (m, 4H), 3.52 (d, *J* = 2.0 Hz, 3H), 2.90 - 2.47 (m, 3H), 2.03 - 1.90 (m, 3H), 1.76 - 1.42 (m, 4H), 1.32 (d, *J =* 1.9 Hz, 9H). |
| | LCMS: C₅₀H₅₀FN₁₁O₈ requires: 951, found: m/z = 952 [M+H]⁺. |
| 16 | ¹H NMR (500 MHz, DMSO-*d*₆) δ 10.95 (s, 1H), 8.50 (d, *J* = 5.0 Hz, 1H), 8.46 (d, *J =* 2.5 Hz, 1H), 8.29 (d, *J =* 22.6 Hz, 1H), 7.99 (dd, *J =* 9.8, 2.3 Hz, 1H), 7.84 (d, *J =* 1.7 Hz, 1H), 7.70 (dd, *J =* 13.2, 1.7 Hz, 1H), 7.54 (t, *J* = 4.3 Hz, 1H), 7.42 (t, *J* = 4.7 Hz, 3H), 7.33 (t, *J* = 2.5 Hz, 1H), 5.96 (d, *J* = 6.0 Hz, 1H), 5.09 (dd, *J =* 13.3, 5.2 Hz, 1H), 4.61 (d, *J* = 31.3 Hz, 2H), 4.46 - 4.30 (m, 3H), 4.25 (d, *J =* 17.0 Hz, 1H), 4.00 (s, 1H), 3.86 (dd, *J* = 18.5, 8.2 Hz, 3H), 3.52 (s, 3H), 3.22 - 2.77 (m, 6H), 2.67 - 2.50 (m, 4H), 2.34 - 2.18 (m, 1H), 2.02 - 1.81 (m, 4H), 1.32 (s, 9H). |
| | LCMS: C₅₂H₅₇FN₁₂O₇ requires: 980, found: m/z = 981 [M+H]⁺. |
| 17 | ¹H NMR (500 MHz, DMSO-*d*₆) δ 10.91 (s, 1H), 8.51 - 8.48 (m, 1H), 8.46 (d, *J* = 2.5 Hz, 1H), 8.25 (d, *J* = 28.6 Hz, 1H), 7.98 (dd, *J =* 7.7, 2.4 Hz, 1H), 7.83 (d, *J = 1.8 Hz,* 1H), 7.70 (dd, *J* = 13.1, 1.8 Hz, 1H), 7.49 (dd, *J* = 5.2, 3.4 Hz, 1H), 7.43 (t, *J=* 4.5 Hz, 1H), 7.38 (q, *J=* 2.0 Hz, 2H), 7.35 - 7.30 (m, 1H), 5.94 (s, 1H), 5.06 (dd, *J* = 13.3, 5.2 Hz, 1H), 4.55 (s, 2H), 4.45 - 4.30 (m, 3H), 4.23 (d, *J* = 17.1 Hz, 1H), 3.98 - 3.69 (m, 6H), 3.52 (s, 3H), 3.22 (t, *J* = 6.2 Hz, 3H), 2.99 - 2.79 (m, 2H), 2.65 - 2.50 (m, 4H), 2.35 (tt, *J* = 8.7, 4.9 Hz, 2H), 1.99 - 1.85 (m, 4H), 1.80 - 1.67 (m, 3H), 1.32 (s, 14H). |
| | LCMS: C₅₁H₅₃FN₁₀O₈ requires: 952, found: m/z = 953 [M+H]⁺. |
| 21 | ¹H NMR (500 MHz, DMSO-*d*₆) δ 11.04 (s, 1H), 8.47 (dd, *J* = 14.1, 3.8 Hz, 2H), 8.20 (d, *J =* 12.9 Hz, 1H), 7.95 (d, *J* = 2.2 Hz, 1H), 7.83 (d, *J =* 1.8 Hz, 1H), 7.73 - 7.62 (m, 3H), 7.61 - 7.54 (m, 1H), 7.41 (dd, *J=* 8.5, 5.0 Hz, 1H), 7.32 (t, *J* = 2.4 Hz, 1H), 5.93 (d, *J* = 2.3 Hz, 1H), 5.04 (dd, *J* = 12.6, 5.3 Hz, 1H), 4.62 (d, *J* = 55.0 Hz, 2H), 4.35 (s, 2H), 4.03 - 3.77 (m, 4H), 3.58 - 3.48 (m, 4H), 3.37 - 3.24 (m, 2H), 3.01 - 2.74 (m, 3H), 2.67 - 2.48 (m, 3H), 1.97 (t, *J =* 8.1 Hz, 1H), 1.72 (dt, *J* = 22.6, 7.3 Hz, 2H), 1.32 (d, *J* = 1.5 Hz, 9H). |
| | LCMS: C₄₉H₄₉FN₁₀O₉ requires: 940, found: m/z = 941 [M+H]⁺. |
| 22 | ¹H NMR (500 MHz, DMSO-*d*₆) δ 10.97 (s, 1H), 8.50 (d, *J* = 5.0 Hz, 1H), 8.46 (d, *J=* 2.5 Hz, 1H), 8.29 (d, *J=* 23.5 Hz, 1H), 7.99 (dd, *J=* 11.5, 2.3 Hz, 1H), 7.83 (d, *J* = 1.7 Hz, 1H), 7.70 (dd, *J* = 13.1, 1.7 Hz, 1H), 7.61 - 7.56 (m, 1H), 7.49 - 7.39 (m, 3H), 7.33 (d, *J* = 2.3 Hz, 1H), 5.97 (d, *J=* 5.8 Hz, 1H), 5.10 (dd, *J=* 13.3, 5.2 Hz, 1H), 4.63 (d, *J* = 31.6 Hz, 2H), 4.44 (d, *J=* 17.0 Hz, 1H), 4.39 - 4.24 (m, 3H), 4.02 (s, 1H), 3.94 - 3.79 (m, 3H), 3.51 (d, *J=* 2.4 Hz, 3H), 3.27 - 2.79 (m, 9H), 2.62 - 2.52 (m, 1H), 2.35 - 2.18 (m, 1H), 2.02 - 1.92 (m, 1H), 1.32 (s, 9H). |
| | LCMS: C₅₁H₅₅FN₁₂O₇ requires: 966, found: m/z = 967 [M+H]⁺. |
| 23 | ¹H NMR (500 MHz, DMSO-*d*₆) δ 11.05 (s, 1H), 9.53 (s, 1H), 8.50 (d, *J* = 5.0 Hz, 1H), 8.46 (d, *J* = 2.5 Hz, 1H), 8.27 (d, *J=* 30.3 Hz, 1H), 7.99 (dd, *J =* 11.2, 2.3 Hz, 1H), 7.83 (d, *J =* 1.8 Hz, 1H), 7.75 (d, *J=* 11.2 Hz, 1H), 7.70 (dd, *J =* 13.1, 1.6 Hz, 1H), 7.54 (d, *J =* 7.3 Hz, 1H), 7.42 (dd, *J* = 5.0, 2.8 Hz, 1H), 7.33 (d, *J =* 2.4 Hz, 1H), 5.96 (d, *J* = 11.6 Hz, 1H), 5.06 (dd, *J* = 12.9, 5.4 Hz, 1H), 4.63 (d, *J =* 28.6 Hz, 2H), 4.35 (d, *J =* 2.3 Hz, 2H), 4.00 (s, 1H), 3.93 - 3.63 (m, 6H), 3.52 (s, 5H), 3.16 (t, *J* = 20.3 Hz, 8H), 2.82 (ddd, *J* = 16.9, 13.9, 5.5 Hz, 1H), 2.63 - 2.47 (m, 3H), 2.04 - 1.92 (m, 1H), 1.86 (q, *J* = 7.7, 7.2 Hz, 3H), 1.32 (s, 9H). |
| | LCMS: C₅₁H₅₂F₂N₁₂O₈ requires: 998, found: m/z = 999 [M+H]⁺. |
| 24 | ¹H NMR (500 MHz, DMSO-*d*₆) δ 11.04 (s, 1H), 8.48 (dd, *J* = 5.0, 2.5 Hz, 1H), 8.22 (d, *J* = 20.5 Hz, 1H), 8.00 (d, *J=* 2.2 Hz, 1H), 7.74 - 7.65 (m, 2H), 7.58 (dd, *J=* 12.9, 7.7 Hz, 1H), 7.40 (dd, *J* = 14.9, 5.1 Hz, 1H), 7.33 (td, *J* = 5.1*,* 4.7, 2.8 Hz, 2H), 7.20 (s, 1H), 6.93 (d, *J=* 12.9 Hz, 1H), 6.56 (dd, *J* = 7.6, 2.0 Hz, 1H), 5.94 (d, *J=* 11.9 Hz, 1H), 5.06 (dd, *J* = 12.9, 5.4 Hz, 1H), 4.63 (d, *J* = 54.6 Hz, 2H), 4.43 - 4.23 (m, 2H), 4.01 - 3.81 (m, 4H), 3.55 (t, *J* = 6.4 Hz, 2H), 3.35 - 3.24 (m, 2H), 2.81 (ddd, *J* = 16.7, 13.9, 5.4 Hz, 1H), 2.74 - 2.47 (m, 4H), 1.99 (qd, *J=* 12.3, 10.2, 4.0 Hz, 2H), 1.73 (dt, *J* = 22.8, 7.5 Hz, 2H), 1.08 - 0.98 (m, 2H), 0.84 - 0.73 (m, 2H). |
| | LCMS: C₄₉H₄₆FN₉O₉ requires: 923, found: m/z = 924 [M+H]⁺. |

### Example 3: General procedure for the synthesis of aldehyde intermediates for use in synthetic Route 2 of Scheme 5.

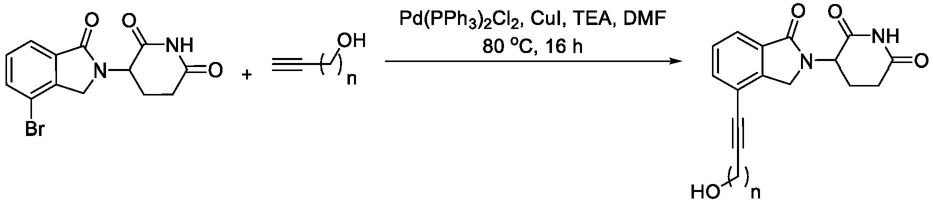

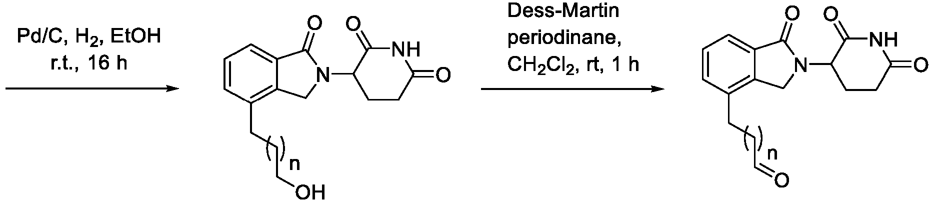

Step 1: 3-(4-bromo-1-oxo-2,3-dihydro-1H-isoindol-2-yl)piperidine-2,6-dione (1.79 mmol), (PPh₃)₂PdCl₂ (0.11 mmol), CuI (0.15 mmol) were added to a vial. The vial was evacuated and backfilled with N₂ 5 times. DMF (5 mL), alkyne (4.37 mmol) and triethylamine (18.05 mmol) were added and the mixture was allowed to stir at 90 °C overnight. The mixture was filtered through celite and purified by HPLC (5-95% MeCN in H₂O with 0.1% TFA) to afford the aryl alkyne (58%).

Step 2: A mixture of aryl alkyne (1.04 mmol), Pd/C 10wt% (0.12 mmol) and EtOH (015 mL) were mixed in a flask. The flask was evacuated and backfilled with H₂ 5 times and allowed to stir at rt for 16 h. The mixture was filtered through celite washing with MeOH and EtOAc and concentrated to afford the alkyl alcohol (74%).

Step 3: Dess-Martin periodinane (1.54 mmol) was added to a mixture of the alkyl alcohol (0.77 mmol) and CH₂Cl₂ (10 mL). The mixture was allowed to stir at r.t, for 1 h. CH₂Cl₂ and aqueous Na₂SO₃ were added. The organic layer was dried with MgSO4, filtered, concentrated and purified by MPLC (20-100% EtOAc in hexanes) to afford the aldehyde.

### Example 3A: Synthesis of 3-(2-(2,6-dioxopiperidin-3-yl)-1-oxoisoindolin-4-yl)propanal.

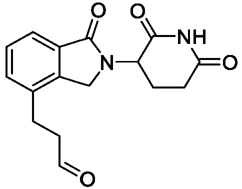

Step 1 product: 3-(4-(3-hydroxyprop-1-yn-1-yl)-1-oxoisoindolin-2-yl)piperidine-2,6-dione (127.6 mg, 23.1%). LCMS; C₁₆H₁₄N₂O₄ requires: 298, found: m/z = 299 [M+H]⁺.

Step 2 product: 3-(4-(3-hydroxypropyl)-1-oxoisoindolin-2-yl)piperidine-2,6-dione (129 mg, 99%). LCMS; C₁₆H₁₈N₂O₄ requires: 302, found: m/z = 303 [M+H]⁺.

Step 3 product: 3-(2-(2,6-dioxopiperidin-3-yl)-1-oxoisoindolin-4-yl)propanal (29 mg, 99%). LCMS; C₁₆H₁₆N₂O₄ requires: 300, found: m/z = 301 [M+H]⁺.

### Example 3B: Synthesis of 4-(2-(2,6-dioxopiperidin-3-yl)-1-oxoisoindolin-4-yl)butanal.

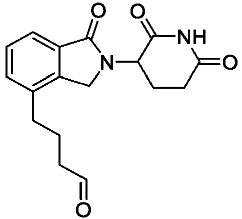

Step 1 product: 3-(4-(4-hydroxybut-1-yn-1-yl)-1-oxoisoindolin-2-yl)piperidine-2,6-dione (325 mg, 58.1%). LCMS; C₁₇H₁₆N₂O₄ requires: 312, found: m/z = 313 [M+H]⁺.

Step 2 product: 3-(4-(4-hydroxybutyl)-1-oxoisoindolin-2-yl)piperidine-2,6-dione (244 mg, 74.1%). LCMS; C₁₇H₂₀N₂O₄ requires: 316, found: m/z = 317 [M+H]⁺.

Step 3 product: 4-(2-(2,6-dioxopiperidin-3-yl)-1-oxoisoindolin-4-yl)butanal (178 mg, 73.4%). LCMS; C₁₇H₁₈N₂O₄ requires: 314, found: m/z = 315 [M+H]⁺.

### Example 3C: Synthesis of 6-(2-(2,6-dioxopiperidin-3-yl)-1-oxoisoindolin-4-yl)hexanal.

Step 1 product: 3-(4-(6-hydroxyhex-1-yn-1-yl)-1-oxoisoindolin-2-yl)piperidine-2,6-dione (104 mg, 99%). LCMS; C₁₉H₂₀N₂O₄ requires: 340, found: m/z = 341 [M+H]⁺.

Step 2 product: 3-(4-(6-hydroxyhexyl)-1-oxoisoindolin-2-yl)piperidine-2,6-dione (100 mg, 95%). LCMS; C₁₉H₂₄N₂O₄ requires: 344, found: m/z = 345 [M+H]⁺.

Step 3 product: 6-(2-(2,6-dioxopiperidin-3-yl)-1-oxoisoindolin-4-yl)hexanal (38 mg, 95%). LCMS; C₁₉H₂₄N₂O₄ requires: 342, found: m/z = 343 [M+H]⁺.

**Table 3: Bifunctional compounds generated according to Route 2 of Scheme 5 and using the procedures in Examples 1 and 3.**

| **Cmpd No.** | **Characterization data** |
|---|---|
| 18 | ¹H NMR (500 MHz, DMSO-*d*₆) δ 10.94 (s, 1H), 8.50 (dd, *J =* 5.2, 1.9 Hz, 1H), 8.47 (t, *J =* 2.4 Hz, 2H), 8.01 (d, *J* = 2.5 Hz, 1H), 7.83 (d, *J =* 1.7 Hz, 1H), 7.70 (dd, *J =* 13.1, 1.9 Hz, 1H), 7.53 (dd, *J* = 5.7, 2.9 Hz, 1H), 7.46 - 7.39 (m, 3H), 7.36 (d, *J=* 2.3 Hz, 1H), 6.08 (s, 1H), 5.09 (dd, *J* = 13.3, 5.1 Hz, 1H), 4.47 - 4.01 (m, 7H), 3.52 (d, *J* = 2.2 Hz, 3H), 3.32 (d, *J* = 125.7 Hz, 11H), 2.87 (ddd, *J* = 17.1, 13.7, 5.4 Hz, 1H), 2.69 - 2.50 (m, 3H), 2.37 - 2.24 (m, 1H), 2.02 - 1.90 (m, 1H), 1.76 - 1.53 (m, 2H), 1.32 (d, *J* = 2.1 Hz, 11H). |
| | LCMS: C₄₇H₄₉FN₁₀O₆ requires: 868, found: m/z = 869 [M+H]⁺. |
| 19 | ¹H NMR (500 MHz, Methanol-*d*₄) δ 8.57 (d, *J* = 5.1 Hz, 1H), 8.48 (d, *J* = 2.4 Hz, 1H), 8.12 (d, *J* = 2.3 Hz, 1H), 7.70 (dd, *J* = 13.0, 1.8 Hz, 1H), 7.64 (dd, *J* = 5.9, 2.7 Hz, 1H), 7.59 (d, *J* = 5.1 Hz, 1H), 7.52 - 7.36 (m, 3H), 6.06 (s, 1H), 5.17 (dd, *J=* 13.3, 5.2 Hz, 1H), 4.60 - 4.42 (m, 6H), 4.32 (s, 2H), 3.84 (s, 2H), 3.69 (s, 3H), 2.90 (ddd, *J=* 17.7, 13.6, 5.5 Hz, 1H), 2.82 - 2.68 (m, 3H), 2.50 (qd, *J* = 13.2, 4.6 Hz, 1H), 2.17 (dtd, *J=* 12.9, 5.4, 2.4 Hz, 1H), 1.76 (d, *J* = 33.4 Hz, 4H), 1.44 (s, 14H). |
| | LCMS: C₄₉H₅₃FN₁₀O₆ requires: 896, found: m/z = 897 [M+H]⁺. |
| 20 | N/A |

### Example 4: Synthesis of intermediate compound 10-[3'-(hydroxymethyl)-1-methyl-5-({5-[(2S)-2-methylpiperazin-1-yl]pyridin-2-yl}amino)-6-oxo-[3,4'-bipyridin]-2'-yl]-4,4-dimethyl-1,10-diazatricyclo[6.4.0.0^{2,6}]dodeca-2(6),7-dien-9-one.

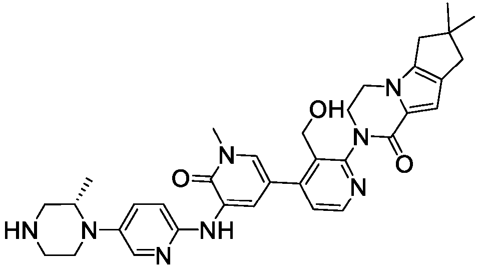

### Step 1: Synthesis of 2-bromo-4-chloronicotinaldehyde (BA-1).

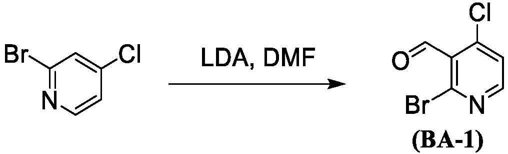

To a solution of 2-bromo-4-chloropyridine (30.0 g, 155.89 mmol) in THF (300.0 mL) was added LDA (62.5 mL, 2 M) dropwise at -70 °C under N₂. The reaction mixture was stirred at -70 °C for 1.5 hours, and then N,N-dimethylformamide (15.2 g, 208.07 mmol) was added dropwise to the above mixture at -70°C. The resulting mixture was stirred at -70 °C for another 2 hours. The reaction mixture was then quenched with aqueous saturated ammonium chloride and the aqueous phase was extracted with ethyl acetate. The combined organic layer was washed with brine, dried over anhydrous sodium sulfate and filtered. The filtrate was evaporated under vacuum. The residue was purified by flash column chromatography with 0~30% ethyl acetate in petroleum ether to afford 2-bromo-4-chloronicotinaldehyde (24.0 g, 70%) as a yellow solid. MS (ESI) calculated for (C₆H₃BrClNO) [M+H]⁺, 219.9; found, 220.1.

### Step 2: Synthesis of 3,3-dimethylcyclopentanone.

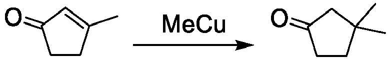

To a solution of CuI (47.6 g, 249.93 mmol) in ethyl ether (400.0 mL) was added MeLi (286.0 mL, 1.6 M) dropwise at 0 °C over a time period of 1 hour. The reaction mixture was stirred at 0 °C for 1.5 hours under N₂, then 3-methylcyclopent-2-en-1-one (20.0 g, 208.05 mmol) was added dropwise to the above mixture at 0 °C. The resulting mixture was stirred at 0 °C for another 2 hours, and the reaction was then quenched by the addition of aqueous saturated ammonium chloride, and then the aqueous phase was extracted with ethyl ether. The combined organic layer was washed with brine, dried over anhydrous sodium sulfate and filtered. The filtrate was evaporated under vacuum to afford 3,3-dimethylcyclopentanone (17.0 g, crude) as a yellow oil, which was used in the next step without further purification. GCMS (ESI) calculated for (C₇H₁₂O), 112.1; found, 112.1.

### Step 3: Synthesis of 2-chloro-4,4-dimethylcyclopent-1-enecarbaldehyde.

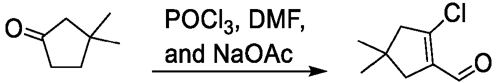

To a solution of DMF (33.2 g, 454.78 mmol) in DCM (200.0 mL) was added POCl₃ (46.5 g, 302.92 mmol) dropwise at 0 °C. The reaction was stirred at 0 °C for 1.5 hours under N₂, and then 3,3-dimethylcyclopentanone (17.0 g, 151.55 mmol) was added dropwise to the above mixture at room temperature. The resulting mixture was stirred at 55 °C for 16 hours. The reaction mixture was then quenched by aqueous saturated sodium acetate, and the aqueous phase was extracted with DCM. The combined organic layers were washed with brine, dried over anhydrous sodium sulfate and filtered. The filtrate was evaporated under vacuum to afford 2-chloro-4,4-dimethylcyclopent-1-enecarbaldehyde (19.0 g, crude) as a yellow oil, which was used in the next step without further purification. MS (ESI) calculated for (C₈H₁₁ClO) [M+H]⁺, 159.0; found, 159.1.

### Step 4: Synthesis of 7,7-dimethyl-3,4,7,8-tetrahydro-2H-cyclopenta[4,5]pyrrolo[1,2-a]pyrazin-1(6H)-one (BB-4).

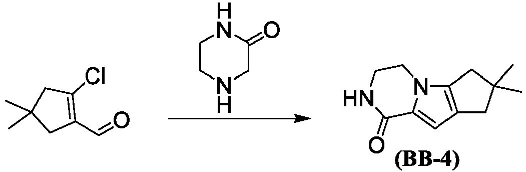

To a solution of piperazin-2-one (18.4 g, 183.28 mmol) in NMP (90.0 mL) was added 2-chloro-4,4-dimethylcyclopent-1-enecarbaldehyde (29.0 g, crude). The resulting mixture was stirred at 100 °C for 1 hour. The resulting mixture was cooled to room temperature and then diluted with H₂O. The formed solids were collected and dried to afford 7,7-dimethyl-3,4,7,8-tetrahydro-2H-cyclopenta[4,5]pyrrolo[1,2-a]pyrazin-1(6H)-one (BB-4) (13.0 g, 35%) as a yellow solid, which was used in the next step without further purification. MS (ESI) calculated for (C₁₂H₁₆N₂O) [M+H]⁺, 205.1; found, 205.2.

### Step 5: Synthesis of 4-chloro-2-(7,7-dimethyl-1-oxo-1,3,4,6,7,8-hexahydro-2H-cyclopenta[4,5]pyrrolo[1,2-a]pyrazin-2-yl)nicotinaldehyde (BC-1):

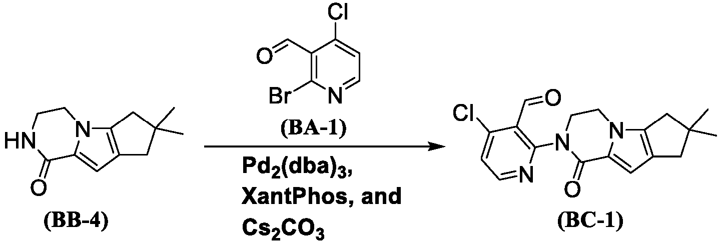

To a degassed solution of 7,7-dimethyl-3,4,7,8-tetrahydro-2H-cyclopenta[4,5]pyrrolo[1,2-a]pyrazin-1(6H)-one (BB-4) (1.0 g, 4.90 mmol) in dioxane (20.0 mL) were added 2-bromo-4-chloropyridine-3-carbaldehyde (BA-1) (3.2 g, 14.68 mmol), Pd₂(dba)₃ (449.0 mg, 0.49 mmol), XantPhos (567.7 mg, 0.98 mmol) and Cs₂CO₃ (3.2 g, 9.75 mmol). The resulting mixture was stirred at 100 °C for 4 hours under nitrogen. The reaction was diluted with water and extracted with ethyl acetate. The combined organic layer was washed with brine, dried over anhydrous sodium sulfate and filtered. The filtrate was evaporated under vacuum, and the residue was purified by flash column chromatography with 0~10% methanol in dichloromethane to afford 4-chloro-2-(7,7-dimethyl-1-oxo-1,3,4,6,7,8-hexahydro-2H-cyclopenta[4,5]pyrrolo[1,2-a]pyrazin-2-yl)nicotinaldehyde (BC-1) (1.1 g, 65%) as a yellow solid. MS (ESI) calculated for (C₁₈H₁₈ClN₃O₂) [M+H]⁺, 344.1; found, 344.2.

### Step 6: Synthesis of tert-butyl (3S)-3-methyl-4-(6-nitropyridin-3-yl)piperazine-1-carboxylate.

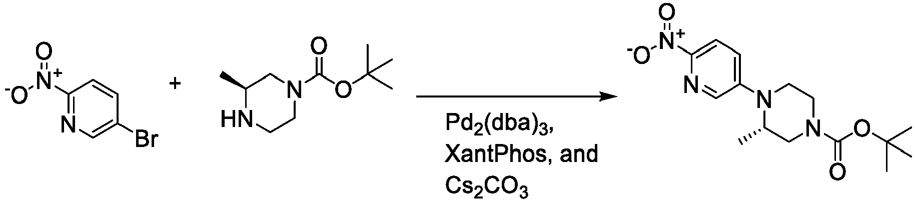

To a solution of 5-bromo-2-nitropyridine (10.4 g, 51.23 mmol) in 1,4-dioxane (400.0 mL) were added tert-butyl (3S)-3-methylpiperazine-1-carboxylate (10.7 g, 53.42 mmol), Pd₂(dba)₃ (6.5 g, 7.10 mmol), XantPhos (8.1 g, 14.01 mmol) and Cs₂CO₃ (37.3 g, 114.48 mmol). The resulting mixture was stirred at 100 °C for 16 hours under nitrogen. After the reaction was completed, the resulting mixture was diluted with 200 mL of ethyl acetate. The solids were filtered out, the filtrate was concentrated under vacuum, and the residue was purified by flash column chromatography with 0~55% ethyl acetate in petroleum ether to afford tert-butyl (3S)-3-methyl-4-(6-nitropyridin-3-yl)piperazine-1-carboxylate (4.4 g, 27%) as a yellow solid. MS (ESI) calculated for (C₁₅H₂₂N₄O₄) [M+H]⁺, 323.2; found, 323.1.

### Step 7: Synthesis of tert-butyl (3S)-4-(6-aminopyridin-3-yl)-3-methylpiperazine-1-carboxylate.

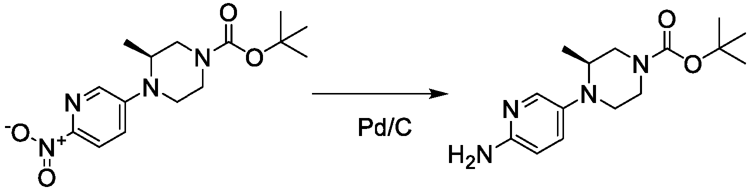

To a solution of tert-butyl (3S)-3-methyl-4-(6-nitropyridin-3-yl)piperazine-1-carboxylate (3.9 g, 12.10 mmol) in methanol (40.0 mL) was added palladium on carbon (400.0 mg, dry) under nitrogen. The reaction mixture was stirred at room temperature for 16 hours under H₂ (2 atm). After the reaction was completed, the solids were filtered out, and the filtrate was concentrated under vacuum to afford tert-butyl (3S)-4-(6-aminopyridin-3-yl)-3-methylpiperazine-1-carboxylate (3.2 g, crude) as a yellow solid, which was used in the next step without further purification. MS (ESI) calculated for (C₁₅H₂₄N₄O₂) [M+H]⁺, 293.2; found, 293.1.

### Step 8: Synthesis of tert-butyl (3S)-4-[6-[(5-bromo-1-methyl-2-oxo-1,2-dihydropyridin-3-yl)amino]pyridin-3-yl]-3-methylpiperazine-1-carboxylate.

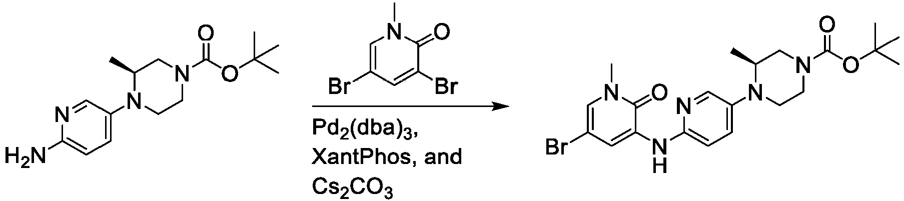

To a degassed solution of tert-butyl (3S)-4-(6-aminopyridin-3-yl)-3-methylpiperazine-1-carboxylate (10.0 g, 34.20 mmol) in 1,4-dioxane (150.0 mL) was added 3,5-dibromo-1-methyl-1,2-dihydropyridin-2-one (9.6 g, 35.93 mmol), Pd₂(dba)₃ (3.3 g, 3.60 mmol), XantPhos (4.1 g, 7.19 mmol) and Cs₂CO₃ (23.4 g, 71.85 mmol). The resulting mixture was stirred at 100 °C for 16 hours under nitrogen. After the reaction was completed, the resulting mixture was diluted with ethyl acetate. The solids were filtered out, and the filtrate was concentrated under vacuum. The residue was purified by flash column chromatography with 0~100% ethyl acetate in petroleum ether to afford tert-butyl (3S)-4-[6-[(5-bromo-1-methyl-2-oxo-1,2-dihydropyridin-3-yl)amino]pyridin-3-yl]-3-methylpiperazine-1-carboxylate (10.0 g, 61%) as a yellow solid. MS (ESI) Calculated for (C₂₁H₂₈BrN₅O₃) [M+H]⁺, 478.1; found, 478.1.

### Step 9: Synthesis of (S)-tert-butyl 3-methyl-4-(6-(1-methyl-2-oxo-5-(4,4,5,5-tetramethyl-1,32-dioxaborolan-2-yl)-12-dihydropyridin-3-ylamino)pyridin-3-yl)piperazine-1-carboxylate.

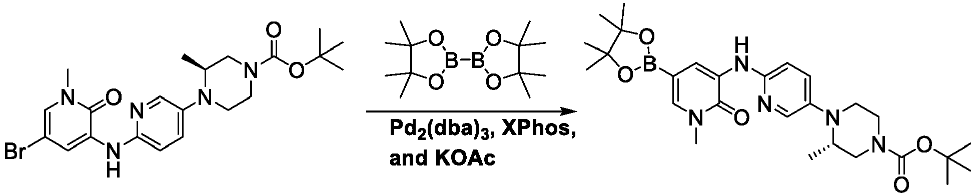

To a degassed solution of tert-butyl (3S)-4-[6-[(5-bromo-1-methyl-2-oxo-1,2-dihydropyridin-3-yl)amino]pyridin-3-yl]-3-methylpiperazine-1-carboxylate (10.0 g, 20.90 mmol) in 1,4-dioxane (100 mL) were added 4,4,5,5-tetramethyl-2-(4,4,5,5-tetramethyl-1,3,2-dioxaborolan-2-yl)-1,3,2-dioxaborolane (13.7 g, 54.03 mmol), Pd₂(dba)₃ (1.9 g, 2.11 mmol), KOAc (6.1 g, 62.67 mmol) and XPhos (2.0 g, 4.20 mmol) under N₂. The resulting mixture was stirred at 100 °C for 16 hours under nitrogen. After the reaction was completed, the solids were filtered out, and the filtrate was concentrated under vacuum. The residue was purified by flash column chromatography with 0~100% ethyl acetate in petroleum ether to afford (S)-tert-butyl 3-methyl-4-(6-(1-methyl-2-oxo-5-(4,4,5,5-tetramethyl-1,3,2-dioxaborolan-2-yl)-1,2-dihydropyridin-3-ylamino)pyridin-3-yl)piperazine-1-carboxylate (10.2 g, 93%) as a red solid. MS (ESI) calculated for (C₂₇H₄₀BN₅O₅) [M+H]⁺, 526.3; found, 526.3. ¹H NMR (400 MHz, DMSO-*d*₆) δ 8.47 (d, *J* = 1.6 Hz, 1H), 8.27 (s, 1H), 7.88 (d, *J* = 2.4 Hz, 1H), 7.49 (d, *J* = 1.6 Hz, 1H), 7.38 - 7.35 (m, 1H), 7.18 (d, *J* = 9.2 Hz, 1H), 3.90 - 3.60 (m, 2H), 3.47 (s, 3H), 3.50 - 3.40 (m, 1H), 3.40 - 3.30 (m, 1H), 3.23 - 2.80 (m, 3H), 1.42 (s, 9H), 1.28 (s, 12 H), 0.86 (d, *J* = 7.2 Hz, 3H).

### Step 10: Synthesis of tert-butyl (S)-4-(6-((2'-(7,7-dimethyl-1-oxo-1,3,4,6,7,8-hexahydro-2H-cyclopenta[4,5]pyrrolo[1,2-a]pyrazin-2-yl)-3'-formyl-1-methyl-6-oxo-1,6-dihydro-[3,4'-bipyridin]-5-yl)amino)pyridin-3-yl)-3-methylpiperazine-1-carboxylate.

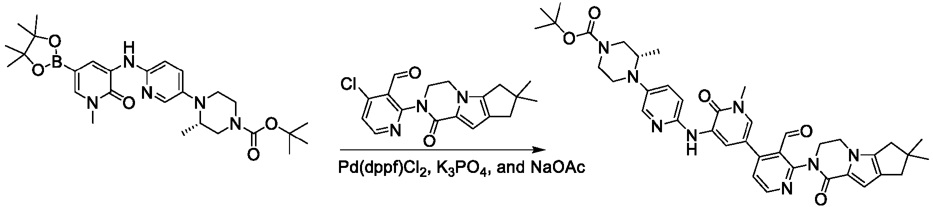

To a degassed solution of 4-chloro-2-[4,4-dimethyl-9-oxo-1,10-diazatricyclo[6.4.0.0-[2,6]]dodeca-2(6),7-dien-10-yl]pyridine-3-carbaldehyde (8.0 g, 23.27 mmol) in CH_{5C}N/H₂O (200.0/50.0 mL) were added tert-butyl (3S)-3-methyl-4-(6-[[1-methyl-2-oxo-5-(4,4,5,5-tetramethyl-1,3,2-dioxaborolan-2-yl)-1,2-dihydropyridin-3-yl]amino]pyridin-3-yl)piperazine-1-carboxylate (12.2 g, 23.23 mmol), Pd(dppf)Cl₂ (1.7 g, 2.33 mmol), K₃PO₄ (9.9 g, 46.73 mmol) and NaOAc (3.8 g, 46.65 mmol). The resulting mixture was stirred at 100 °C for 3 hours under nitrogen. The reaction mixture was diluted with water and the aqueous phase was extracted with ethyl acetate. The combined organic layer was washed with brine, dried over anhydrous sodium sulfate and filtered. The filtrate was evaporated under vacuum. The residue was purified by flash column chromatography with 0~10% methanol in dichloromethane to afford tert-butyl (S)-4-(6-((2'-(7,7-dimethyl-1-oxo-1,3,4,6,7,8-hexahydro-2H-cyclopenta[4,5]pyrrolo[1,2-a]pyrazin-2-yl)-3'-formyl-1-methyl-6-oxo-1,6-dihydro-[3,4'-bipyridin]-5-yl)amino)pyridin-3-yl)-3-methylpiperazine-1-carboxylate (10.0 g, 61%) as a brown solid. MS (ESI) calculated for (C₃₉H₄₆N₈O₅) [M+H]⁺, 707.4; found, 707.2.

### Step 11: Synthesis of tert-butyl (S)-4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)-3-methylpiperazine-1-carboxylate.

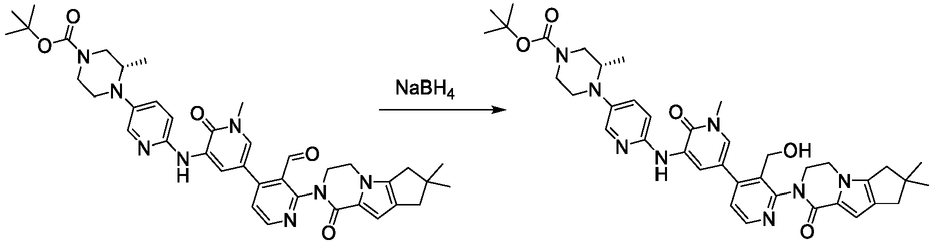

To a solution of tert-butyl (S)-4-(6-((2'-(7,7-dimethyl-1-oxo-1,3,4,6,7,8-hexahydro-2H-cyclopenta[4,5]pyrrolo[1,2-a]pyrazin-2-yl)-3'-formyl-1-methyl-6-oxo-1,6-dihydro-[3,4'-bipyridin]-5-yl)amino)pyridin-3-yl)-3-methylpiperazine-1-carboxylate (10.0 g, 14.15 mmol) in MeOH (200.0 mL) was added NaBH₄ (806.2 mg, 21.31 mmol). The resulting mixture was stirred at room temperature for 2 hours. The reaction mixture was then diluted with water and the aqueous phase was extracted with ethyl acetate. The combined organic layer was washed with brine, dried over anhydrous sodium sulfate and filtered. The filtrate was evaporated under vacuum. The residue was purified by reverse phase flash column chromatography with 5~100% acetonitrile in water to afford tert-butyl (S)-4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)-3-methylpiperazine-1-carboxylate (4.8 g, 48%) as a purple solid. MS (ESI) calculated for (C₃₉H₄₈N₈O₅) [M+H]⁺, 709.4; found, 709.4. ¹H NMR (300 MHz, DMSO-*d*₆) δ 8.63 (d, *J*= 2.1 Hz, 1H), 8.49 - 8.47 (m, 2H), 7.84 (d, *J* = 2.7 Hz, 1H), 7.47 (d, *J* = 2.1 Hz, 1H), 7.39 - 7.34 (m, 2H), 7.27 - 7.23 (m, 1H), 6.56 (s, 1H), 4.98 - 4.95 (m, 1H), 4.43 - 4.38 (m, 2H), 4.30 - 4.15 (m, 3H), 3.87 - 3.68 (m, 3H), 3.60 (s, 3H), 3.51 - 3.46 (m, 1H), 3.40 - 3.33 (m, 1H), 3.30 - 3.20 (m, 1H), 3.08 - 2.98 (m, 1H), 2.97 - 2.83 (m, 1H), 2.58 - 2.50 (m, 2H), 2.47 - 2.41 (m, 2H), 1.41 (s, 9H), 1.22 (s, 6H), 0.82 (*J* = 6.3 Hz, 3H).

### Step 12: Synthesis of (S)-2-(3'-(hydroxymethyl)-1-methyl-5-((5-(2-methylpiperazin-1-yl)pyridin-2-yl)amino)-6-oxo-1,6-dihydro-[3,4'-bipyridin]-2'-yl)-7,7-dimethyl-3,4,7,8-tetrahydro-2H-cyclopenta[4,5]pyrrolo[1,2-a]pyrazin-1(6H)-one.

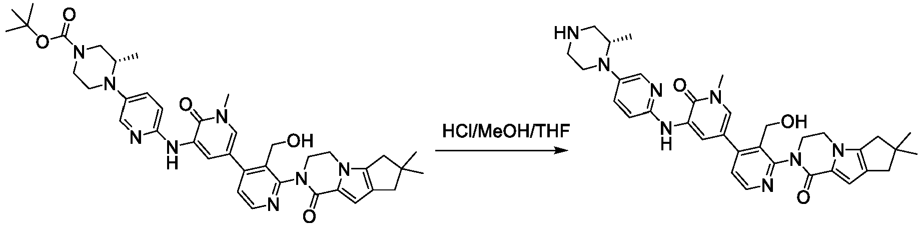

A mixture of tert-butyl (3S)-4-{6-[(2'-{4,4-dimethyl-9-oxo-1,10-diazatricyclo[6.4.0.0-{2,6}]dodeca-2(6),7-dien-10-yl}-3'-(hydroxymethyl)-1-methyl-6-oxo-1,6-dihydro-[3,4'-bipyridin]-5-yl)amino]pyridin-3-yl}-3-methylpiperazine-1-carboxylate (547 mg, 0.770 mmol), hydrogen chloride (4M in dioxane, 4.82 mL, 19.3 mmol) and THF (2 mL) was allowed to stir at room temperature for 2 hours. The volatiles were removed to afford 10-[3'-(hydroxymethyl)-1-methyl-5-({5-[(2S)-2-methylpiperazin-1-yl]pyridin-2-yl}amino)-6-oxo-[3,4'-bipyridin]-2'-yl]-4,4-dimethyl-1,10-diazatricyclo[6.4.0.0-{2,6}]dodeca-2(6),7-dien-9-one (460 mg, 97.9%). LCMS: C₃₄H₄₀N₈O₃ requires: 608, found: m/z = 609 [M+H]⁺.

### Example 5: General procedure for the synthesis of amide compounds according to Route 1 of Scheme 11.

A mixture of 10-[3'-(hydroxymethyl)-1-methyl-5-({5-[(2S)-2-methylpiperazin-1-yl]pyridin-2-yl}amino)-6-oxo-[3,4'-bipyridin]-2'-yl]-4,4-dimethyl-1,10-diazatricyclo[6.4.0.0-{2,6}]dodeca-2(6),7-dien-9-one as the free amine (0.55 mmol), a carboxylic acid (0.58 mmol), HATU (273 mg, 0.72 mmol), and DIEA (360 mg, 2.76 mmol) in *N,N-*dimethylformamide (7 mL) was stirred at 25 °C for 0.5 hours. The crude residue was purified by prep-HPLC to afford the desired product.

### Example 5A: General procedure for the synthesis of carboxylic acid intermediates for use in Example 9.

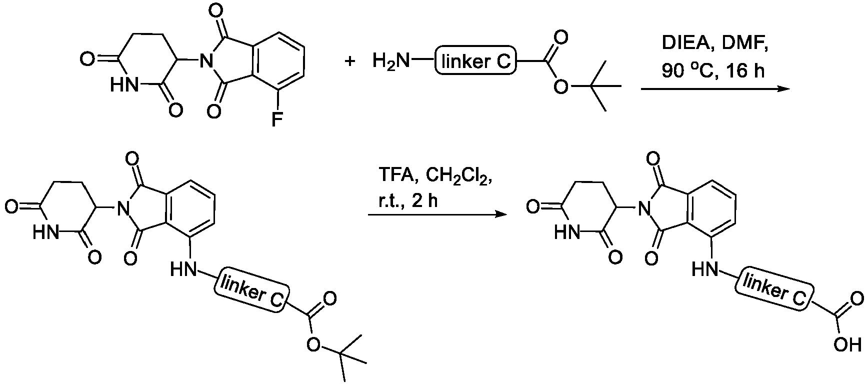

As used in this Example, "linker C" is -Y²-Y³-Y⁴-Y⁵-, wherein each of Y², Y³, Y⁴, and Y⁵ are defined herein.

Step 1: A mixture of 2-(2,6-dioxopiperidin-3-yl)-4-fluoro-2,3-dihydro-1H-isoindole-1,3-dione (0.26 mmol), aminoester (0.26 mmol), ethylbis(propan-2-yl)amine (0.52 mmol) and DMF (1 mL) was allowed to stir at 90°C overnight. The mixture was cooled and purified by HPLC (5-95% MeCN in H₂O with 0.1% TFA) to afford the tert-butylester intermediate.

Step 2: A mixture of tert-butyl 4-{[2-(2,6-dioxopiperidin-3-yl)-1,3-dioxo-2,3-dihydro-1H-isoindol-4-yl]amino }butanoate (0.10 mmol) , CH₂Cl₂ (1 mL), and TFA (1 mL) was allowed to stir at r.t. for 2 h. The mixture was concentrated to afford the carboxylic acid product.

The general method of this Example, was used to synthesize the intermediates described in Examples 5A-1 through 5A-16.

The following molecules were obtained from commercial sources: 2-(2,6-Dioxo-piperidin-3-yl)-4-fluoroisoindoline-1,3-dione (Advanced ChemBlocks Inc), methyl 3-bromo-2-methyl-benzoate (Oakwood Chemical), 3-aminopiperidine-2,6-dione hydrochloride (Oakwood Chemical), tert-butyl (2-(2-(2-(prop-2-yn-1-yloxy)ethoxy)ethoxy)ethyl)carbamate (BroadPharm), benzyl piperazine-1-carboxylate (Sigma Aldrich), tert-butyl 2-(piperazin-1-yl)acetate (CombiBlocks), N-Boc-4-pentyne-1-amine (Sigma Aldrich), tert-butyl 3-(2-hydroxyethoxy)propanoate (BroadPharm), 2-(2-(2-aminoethoxy)ethoxy)ethan-1-ol (BroadPharm).

### Example 5A-1: Synthesis of 3-(4-bromo-1-oxoisoindolin-2-yl)piperidine-2,6-dione.

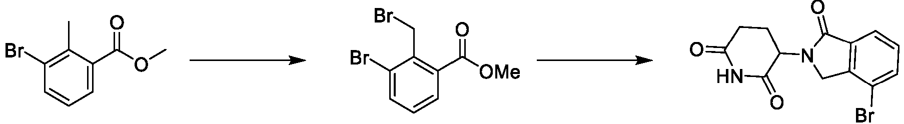

### Step 1: Synthesis of methyl 3-bromo-2-(bromomethyl)benzoate.

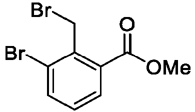

To a solution of methyl 3-bromo-2-methyl-benzoate (50 g, 218.27 mmol, 1 *eq),* NBS (46.62 g, 261.93 mmol, 1.2 *eq)* in CHCl₃ (400 mL) was added AIBN (3.58 g, 21.83 mmol, 0.1 *eq).* The mixture was stirred at 70 °C for 12 h. The reaction mixture was concentrated in vacuum, diluted with DCM (400 mL), washed with H₂O (100 mL) and brine (100 mL), extracted with DCM (100 mL), and washed with brine (50 mL) again. The organic phase was combined, dried over Na₂SO₄, and concentrated in vacuum. The residue was purified by flash silica gel chromatography (Petroleum ether/Ethyl acetate = 100/1) to yield 3-bromo-2-(bromomethyl)benzoate (63 g, 204.57 mmol, 93.72% yield) as a light yellow solid.

### Step 2: Synthesis of 3-(4-bromo-1-oxo-isoindolin-2-yl)piperidine-2,6-dione.

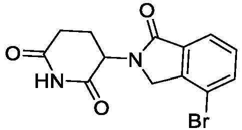

To a solution of methyl 3-bromo-2-(bromomethyl)benzoate (88.2 g, 286.39 mmol, 1 *eq)* in ACN (600 mL) was added DIEA (49.23 g, 380.91 mmol, 66.35 mL, 1.33 *eq)* and 3-aminopiperidine-2,6-dione hydrochloride (51.01 g, 309.94 mmol, 1.08 *eq).* The mixture was stirred at 80 °C for 16 hr. The reaction mixture was filtered. The filter cake was triturated by a mixture solution (EtOAc : H₂O = 100 mL : 200 mL) to yield 3-(4-bromo-1-oxo-isoindolin-2-yl)piperidine-2,6-dione (56.5 g, 174.85 mmol, 61.05% yield) as a purple powder.

### Example 5A-2: Synthesis of 2-(4-(3-(2-(2,6-dioxopiperidin-3-yl)-1-oxoisoindolin-4-yl)propyl)piperazin-1-yl)acetic acid.

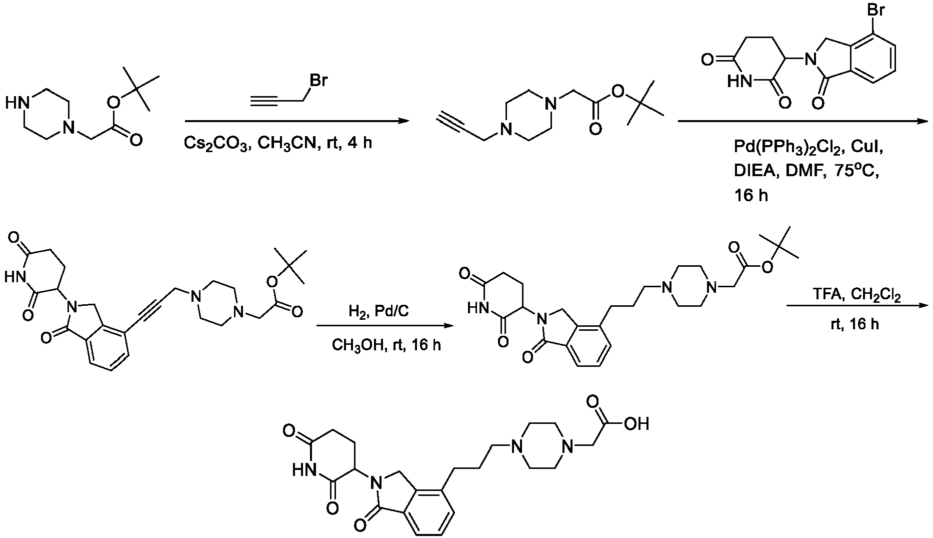

### Step 1: Synthesis of tert-butyl 2-(4-(prop-2-ynyl)piperazin-1-yl)acetate.

To a solution of tert-butyl 2-(piperazin-1-yl)acetate (1.5 g, 7.49 mmol) in acetonitrile (50 mL) were added 3-bromoprop-1-yne (892.5 mg, 7.50 mmol) and Cs₂CO₃ (2.4 g, 7.50 mmol). The resulting solution was stirred at room temperature for 4 h. The solids were filtered out and the filtrate was evaporated under vacuum. The residue was purified by phase flash column chromatography with 0~30% ethyl acetate in petroleum ether to afford tert-butyl 2-(4-(prop-2-ynyl)piperazin-1-yl)acetate (1.1 g, 62%) as a yellow oil. MS (ESI) calculated for (C₁₃H₂₂N₂O₂) [M+H]⁺, 239.2; found, 239.1.

### Step 2: Synthesis of tert-butyl 2-(4-(3-(2-(2,6-dioxopiperidin-3-yl)-1-oxoisoindolin-4-yl)prop-2-ynyl)piperazin-1-yl)acetate.

To a degassed solution of 3-(4-bromo-1-oxoisoindolin-2-yl)piperidine-2,6-dione (1.5 g, 4.64 mmol) in N,N-dimethylformamide (30 mL) were added tert-butyl 2-(4-(prop-2-ynyl)piperazin-1-yl)acetate (1.5 g, 6.29mmol), Pd(PPh₃)₂Cl₂ (489.0 mg, 0.70 mmol,), DIEA (20 mL) and CuI (221.7 mg, 1.16 mmol). The resulting solution was stirred at 75 °C for 16 h under nitrogen. The reaction was quenched by the addition of water, and then extracted with ethyl acetate. The combined organic layer was washed with brine, dried over anhydrous sodium sulfate and filtered. The filtrate was evaporated under vacuum. The residue was purified by flash column chromatography with 0~10% methanol in dichloromethane to afford tert-butyl 2-(4-(3-(2-(2,6-dioxopiperidin-3-yl)-1-oxoisoindolin-4-yl)prop-2-ynyl)piperazin-1-yl)acetate (1.5 g, 68%) as a yellow solid. MS (ESI) calculated for (C₂₆H₃₂N₄O₅) [M+H]⁺, 481.2; found, 481.1.

### Step 3: Synthesis of tert-butyl 2-(4-(3-(2-(2,6-dioxopiperidin-3-yl)-1-oxoisoindolin-4-yl)propyl)piperazin-1-yl)acetate.

To a solution of tert-butyl 2-(4-(3-(2-(2,6-dioxopiperidin-3-yl)-1-oxoisoindolin-4-yl)prop-2-ynyl)piperazin-1-yl)acetate (2.2 g, 4.58 mmol) in methanol (50 mL) was added Pd/C (dry, 0.44 g). The resulting solution was stirred at room temperature for 16 h under hydrogen (2 atm). The solids were filtered out. The filtrate was evaporated under vacuum to afford tert-butyl 2-(4-(3-(2-(2,6-dioxopiperidin-3-yl)-1-oxoisoindolin-4-yl)propyl)piperazin-1-yl)acetate (1.4 g, crude) as a yellow oil, which was used in the next step without further purification. MS (ESI) calculated for (C₂₆H₃₆N₄O₅) [M+H]⁺, 485.3; found,485.2.

### Step 4: Synthesis of 2-(4-(3-(2-(2,6-dioxopiperidin-3-yl)-1-oxoisoindolin-4-yl)propyl)piperazin-1-yl)acetic acid TFA salt.

To a solution of tert-butyl 2-(4-(3-(2-(2,6-dioxopiperidin-3-yl)-1-oxoisoindolin-4-yl)propyl)piperazin-1-yl)acetate (1.4 g, 2.89 mmol) in dichloromethane (20 mL) was added trifluoroacetic acid (20 mL). The resulting solution was stirred at room temperature for 16 h before concentrated under vacuum. The residue was purified by Pre-HPLC with the following conditions: [Column: XSelect CSH Prep C18 OBD Column, 5um,19*150mm; Mobile Phase A: Water (0.05% TFA ), Mobile Phase B: ACN; Flow rate: 25 mL/min; Gradient: 5% B to 20% B in 7 min; 254/220 nm] to afford 2-(4-(3-(2-(2,6-dioxopiperidin-3-yl)-1-oxoisoindolin-4-yl)propyl)piperazin-1-yl)acetic acid TFA salt (434.3 mg, 35%) as a yellow solid. MS (ESI) calculated for (C₂₂H₂₈N₄O₅) [M+H]⁺, 429.2; found, 429.0. ¹H NMR (300 MHz, DMSO-*d*₆) δ 11.08 (s, 1H), 7.60 - 7.65 (m, 1H), 7.52 - 7.47 (m, 2H), 5.20 - 5.13 (m, 1H), 4.52 - 4.46 (m, 1H), 4.35 - 4.29 (m, 1 H), 3.51 (s, 3 H), 3.47 - 2.84 (m, 9H), 2.72 - 2.50 (m, 4H), 2.49 - 2.31 (m, 1 H), 2.05 - 1.97 (m, 3H).

### Example 5A-3: Synthesis of (1s,3s)-3-(2-((2-(2,6-dioxopiperidin-3-yl)-1,3-dioxoisoindolin-4-yl)amino)ethoxy)cyclobutane-1-carboxylic acid.

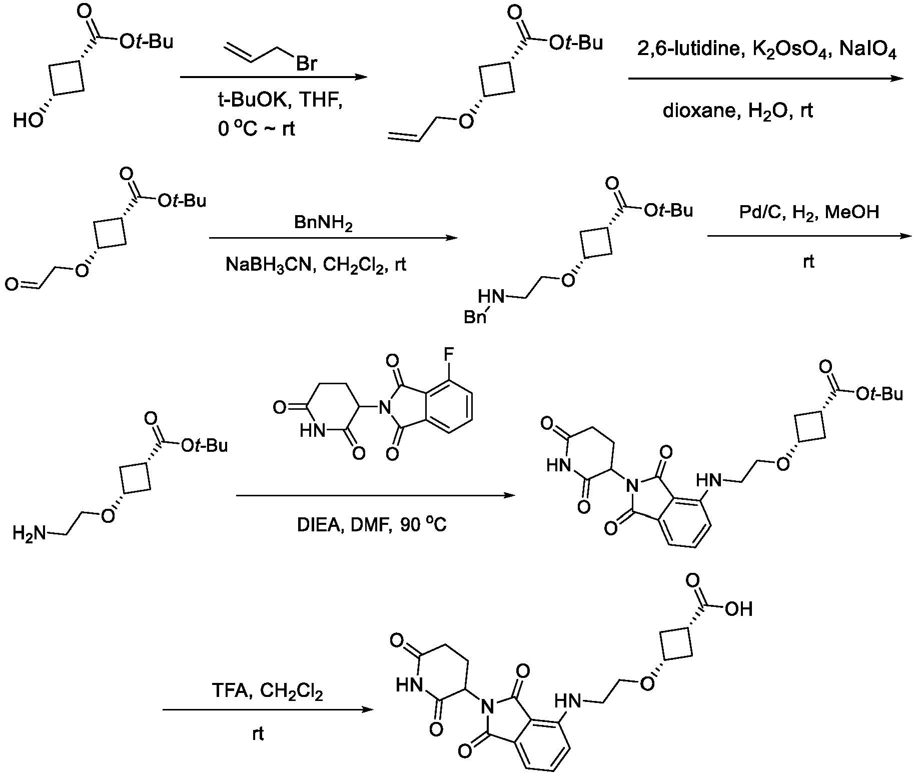

### Step 1: Synthesis of cis-tert-butyl-3-(prop-2-en-1-yloxy)cyclobutane-1-carboxylate.

To a solution of cis-tert-butyl-3-hydroxycyclobutane-1-carboxylate (10.0 g, 58.06 mmol) in tetrahydrofuran (100 mL) was added t-BuOK (64 mL, 1 M in THF) dropwise at 0 °C under nitrogen and stirred for 10 min. To the above solution was added 3-bromoprop-1-ene (7.02 g, 58.03 mmol) dropwise at 0 °C. The resulting mixture was stirred at room temperature for 16 h. After the reaction was completed, the resulting solution was quenched by the addition of saturated NH₄Cl aqueous solution. The aqueous phase was extracted with ethyl acetate. The combined organic layer was washed with brine, dried over anhydrous sodium sulfate, filtered and concentrated under vacuum. The residue was purified by flash column chromatography with 0~20% ethyl acetate in petroleum ether to afford cis-tert-butyl-3-(prop-2-en-1-yloxy)cyclobutane-1-carboxylate (11.3 g, 92%) as a colorless oil. ¹H NMR (300 MHz, Chloroform-*d*) δ 6.10 - 5.85 (m, 1H), 5.33 - 5.10 (m, 2H), 3.95 - 3.75 (m, 3H), 2.60 - 2.36 (m, 3H), 2.29 -2.07 (m, 2H), 1.44 (s, 9H).

### Step 2: Synthesis of cis-tert-butyl-3-(2-oxoethoxy)cyclobutane-1-carboxylate.

To a solution of cis-tert-butyl-3-(prop-2-en-1-yloxy)cyclobutane-1-carboxylate (1.0 g, 4.71 mmol) in dioxane (30 mL) and H₂O (15 mL) were added K₂OsO_{4.}2H₂O (86.28 mg, 0.24 mmol), 2,6-dimethylpyridine (1.01 g, 9.43 mmol) and NaIO₄ (2.02 g, 9.42 mmol). The resulting mixture was stirred at room temperature for 2 h. After the reaction was completed, the mixture was diluted with water and extracted with ethyl acetate. The combined organic layer was washed with brine, dried over anhydrous sodium sulfate, filtered and concentrated under vacuum. The residue was purified by flash column chromatography with 0~50% ethyl acetate in petroleum ether to afford cis-tert-butyl-3-(2-oxoethoxy)cyclobutane-1-carboxylate (505 mg, 50%) as a colorless oil. ¹H NMR (300 MHz, Chloroform-d) δ 9.72 (s, 1H), 6.97 (d, *J =* 7.8 Hz, 1H), 5.31 (s, 1H), 4.05 - 3.94 (m, 2H), 2.64 - 2.41 (m, 2H), 2.36 - 2.12 (m, 2H), 1.47 (s, 9H).

### Step 3: Synthesis of cis-tert-butyl-3-[2-(benzylamino)ethoxy]cyclobutane-1-carboxylate.

To a solution of cis-tert-butyl-3-(2-oxoethoxy)cyclobutane-1-carboxylate (2.0 g, 9.33 mmol) in methanol (20 mL) were added 1-phenylmethanamine (3.0 g, 28.00 mmol) and NaBH_{5C}N (1.76 g, 28.00 mmol). The resulting solution was stirred at room temperature for 16 h before concentrated under vacuum. The residue was purified by flash column chromatography with 0~100% ethyl acetate in petroleum ether to afford *cis*-tert-butyl-3-[2-(benzylamino)ethoxy]cyclobutane-1-carboxylate (1.1 g, 39%) as a colorless oil. MS (ESI) calculated for (C₁₈H₂₇NO₃) [M+H]⁺, 306.2; found, 306.1.

### Step 4: Synthesis of cis-tert-butyl-3-(2-aminoethoxy)cyclobutane-1-carboxylate.

To a solution of *cis*-tert-butyl-3-[2-(benzylamino)ethoxy]cyclobutane-1-carboxylate (2.0 g, 6.55 mmol) in methanol (20 mL) was added Pd/C (10%, 0.5 g). The resulting solution was stirred at room temperature for 72 h under hydrogen (40 atm). After the reaction was completed, the solids were filtered out and the filtrate was concentrated under vacuum to afford *cis*-tert-butyl-3-(2-aminoethoxy)cyclobutane-1-carboxylate (1.0 g, crude) as a colorless oil, which was used in the next step without further purification. MS (ESI) calculated for (C₁₁H₂₁NO₃) [M+H]⁺, 216.2; found, 216.1.

### Step 5: Synthesis of cis-tert-butyl-3-(2-[[2-(2,6-dioxopiperidin-3-yl)-1,3-dioxo-2,3-dihydro-1H-isoindol-4-yl]amino]ethoxy)cyclobutane-1-carboxylate.

To a solution of *cis*-tert-butyl-3-(2-aminoethoxy)cyclobutane-1-carboxylate (1.0 g, 4.64 mmol) in N,N-dimethylformamide (10 mL) were added DIEA (6.0 g, 46.43 mmol) and 2-(2,6-dioxopiperidin-3-yl)-4-fluoro-2,3-dihydro-1H-isoindole-1,3-dione (6.72 g, 24.33 mmol). The resulting solution was stirred at 90 °C for 4 h. After the reaction was completed, the resulting solution was diluted with water and extracted with ethyl acetate. The combined organic layer was washed with brine, dried over anhydrous sodium sulfate and concentrated under vacuum. The residue was purified by reverse phase flash column chromatography with 0~100% acetonitrile in water to afford *cis*-tert-butyl-3-(2-[[2-(2,6-dioxopiperidin-3-yl)-1,3-dioxo-2,3-dihydro-1H-isoindol-4-yl]amino]ethoxy)cyclobutane-1-carboxylate (250 mg, 11%) as a red solid. MS (ESI) calculated for (C₂₄H₂₉N₃O₇) [M+H]⁺, 472.2; found, 472.1.

### Step 6: Synthesis of cis-3-(2-[[2-(2,6-dioxopiperidin-3-yl)-1,3-dioxo-2,3-dihydro-1H-isoindol-4-yl]amino]ethoxy)cyclobutane-1-carboxylic acid.

To a solution of *cis*-tert-butyl-3-(2-[[2-(2,6-dioxopiperidin-3-yl)-1,3-dioxo-2,3-dihydro-1H-isoindol-4-yl]amino]ethoxy)cyclobutane-1-carboxylate (850 mg, 1.8 mmol) in dichloromethane (10 mL) was added trifluoroacetic acid (10 mL). The resulting solution was stirred at room temperature for 3 h before concentrated under vacuum. The residue was purified by reverse phase flash column chromatography with 0~100% acetonitrile in water to afford crude product, which was further purified by non-chiral prep-SFC with the following conditions [Column: Ultimate XB-NH₂, 21.2*250mm; 5um; Mobile Phase A: CO2: 50, Mobile Phase B: MeOH--Preparative: 50; Flow rate: 40 mL/min; 220 nm] to afford *cis-3-(2-*[[2-(2,6-dioxopiperidin-3-yl)-1,3-dioxo-2,3-dihydro-1H-isoindol-4-yl]amino]ethoxy)cyclobutane-1-carboxylic acid (359.1 mg, 37) as a yellow solid. MS (ESI) calculated for (C₂₀H₂₁N₃O₇) [M+H]⁺, 416.4; found, 416.2. ¹H NMR (400 MHz, DMSO-*d*₆) δ 12.16 (s, 1H), 11.11 (s, 1H), 7.63 - 7.55 (m, 1H), 7.15 (d, *J* = 8.0 Hz, 1H), 7.05 (d, *J* = 8.0 Hz, 1H), 6.59 (t, *J=* 5.6 Hz, 1H), 5.12 - 5.03 (m, 1H), 3.96 - 3.84 (m, 1H), 3.59 - 3.40 (m, 4H), 2.98 - 2.82 (m, 1H), 2.64 - 2.52 (m, 3H), 2.48 - 2.37 (m, 2H), 2.08 - 1.91 (m, 3H).

### Example 5A-4: Synthesis of 2-(4-((2-(2,6-dioxopiperidin-3-yl)-1-oxoisoindolin-4-yl)methyl)piperazin-1-yl)acetic acid.

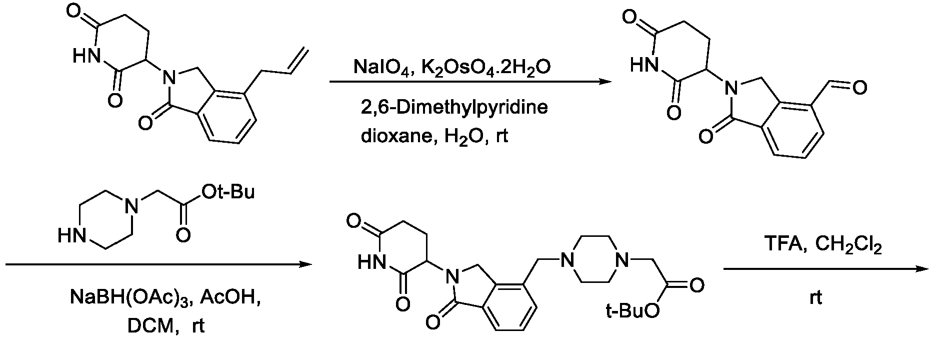

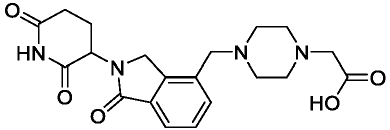

### Step 1: Synthesis of 2-(2,6-dioxopiperidin-3-yl)-1-oxoisoindoline-4-carbaldehyde.

A mixture of 3-(4-allyl-1-oxoisoindolin-2-yl)piperidine-2,6-dione (600 mg, 2.11 mmol), K₂OsO₄.2H₂O (77.7 mg, 0.21 mmol), 2,6-dimethylpyridine(452 mg, 4.22 mmol) and NaIO₄ (903 mg, 4.22 mmol) in dioxane (10 mL) and water (2 mL) was stirred at room temperature for 16 h. The resulting mixture was diluted with water and extracted with ethyl acetate. The combined organic layer was washed with brine, dried over anhydrous Na₂SO₄, filtered and concentrated under vacuum to afford 2-(2,6-dioxopiperidin-3-yl)-1-oxoisoindoline-4-carbaldehyde (400 mg, crude) as a light yellow oil, which was used in the next step without further purification. MS (ESI) calculated for (C₁₄H₁₂N₂O₄) [M+H]⁺, 273.1; found, 273.2.

### Step 2: Synthesis of tert-butyl 2-(4-((2-(2,6-dioxopiperidin-3-yl)-1-oxoisoindolin-4-yl)methyl)piperazin-1-yl)acetate.

A mixture of 2-(2,6-dioxopiperidin-3-yl)-1-oxoisoindoline-4-carbaldehyde (400 mg, 1.47 mmol), tert-butyl 2-(piperazin-1-yl)acetate (324 mg, 1.62 mmol), AcOH (0.5 mL) and NaBH(OAc)₃ (624 mg, 2.94 mmol) in DCM (10 mL) was stirred at room temperature for 16 h. The resulting mixture was diluted with water and extracted with dichloromethane. The combined organic layer was washed with brine, dried over anhydrous Na₂SO₄, filtered and concentrated under vacuum. The residue was purified by reverse phase flash column chromatography with 10~50% acetonitrile in water to afford tert-butyl 2-(4-((2-(2,6-dioxopiperidin-3-yl)-1-oxoisoindolin-4-yl)methyl)piperazin-1-yl)acetate (100 mg, 15%) as a light brown solid. MS (ESI) calculated for (C₂₄H₃₂N₄O₅) [M+H]⁺, 457.2; found, 457.0.

### Step 3: Synthesis of 2-(4-((2-(2,6-dioxopiperidin-3-yl)-1-oxoisoindolin-4-yl)methyl)piperazin-1-yl)acetic acid TFA salt.

A mixture of tert-butyl 2-(4-((2-(2,6-dioxopiperidin-3-yl)-1-oxoisoindolin-4-yl)methyl)piperazin-1-yl)acetate (100 mg, 0.219 mmol) in dichloromethane (3 mL) and trifluoroacetic acid (3 mL) was stirred at room temperature for 16 h before concentrated under vacuum. The residue was purified by reverse phase flash column chromatography with 5~40% acetonitrile in water (containing 0.05% TFA) to afford 2-(4-((2-(2,6-dioxopiperidin-3-yl)-1-oxoisoindolin-4-yl)methyl)piperazin-1-yl)acetic acid TFA salt (59.6 mg, 45%) as an off-white solid. MS (ESI) calculated for (C₂₀H₂₄N₄O₅) [M+H]⁺, 401.2; found, 401.1. ¹H NMR (300 MHz, DMSO-*d*_{6 +} D₂O) δ 7.77 - 7.75 (m, 1H), 7.67 - 7.64 (m, 1H), 7.59 - 7.54 (m, 1H), 5.06 - 5.00 (m, 1H), 4.67 - 4.36 (m, 2H), 4.05 (s, 2H), 3.75 (s, 2H), 3.23 - 2.96 (m, 8H), 2.93 - 2.76 (m, 1H), 2.70 - 2.61 (m, 1H), 2.43 - 2.28 (m, 1H), 2.13 - 1.92 (m, 1H).

### Example 5A-5: Synthesis of 3-(3-(2-(2,6-dioxopiperidin-3-yl)-1,3-dioxoisoindolin-4-yl)propoxy)propanoic acid.

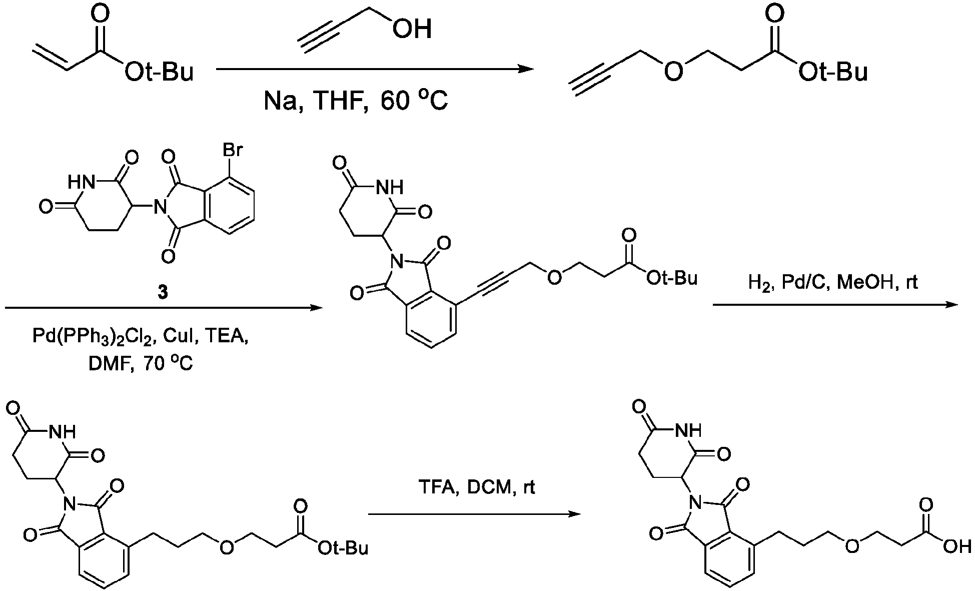

### Step 1: Synthesis of tert-butyl 3-(prop-2-yn-1-yloxy)propanoate.

A mixture of Na (108 mg, 4.68 mmol) and prop-2-yn-1-ol (6.6 g, 117.03 mmol) in anhydrous THF (60 mL) was stirred at 60 °C for 15 min under nitrogen atmosphere. The mixture was then cooled to room temperature and was added tert-butyl acrylate (10.0 g, 78.02 mmol). The resulting solution was stirred at room temperature for 16 h. The reaction was quenched by the addition of water, and then extracted with ethyl acetate. The combined organic layer was washed with brine, dried over anhydrous Na₂SO₄, filtered and concentrated under vacuum. The residue was purified by flash column chromatography with 0~10% ethyl acetate in petroleum ether to afford tert-butyl 3-(prop-2-yn-1-yloxy)propanoate (7.8 g, 54%) as a colorless oil. MS (ESI) calculated for (C₁₀H₁₆O₃) [M+H]⁺, 185.1; found, 185.2. ¹H NMR (400 MHz, DMSO-d6) δ 4.12 (d, *J* = 2.4 Hz, 2H), 3.63 (t, *J* = 6.2 Hz, 2H), 3.43 (t, *J=* 2.4 Hz, 1H), 2.45 (t, *J* = 6.2 Hz, 2H), 1.41 (s, 9H).

### Step 2: Synthesis of tert-butyl 3-((3-(2-(2,6-dioxopiperidin-3-yl)-1,3-dioxoisoindolin-4-yl)prop-2-yn-1-yl)oxy)propanoate.

A degassed mixture of 4-bromo-2-(2,6-dioxopiperidin-3-yl)isoindoline-1,3-dione (5.0 g, 14.83 mmol), Pd(PPh₃)₂Cl₂ (1.6 g, 2.23 mmol), CuI (706.15 mg, 3.708 mmol) and tert-butyl 3-(prop-2-yn-1-yloxy)propanoate (4.1 g, 22.25 mmol) in DIEA (30 mL) and DMF (30 mL) was stirred at 80 °C for 16 h under nitrogen atmosphere. The resulting mixture was diluted with saturated NH₄Cl aqueous solution and extracted with ethyl acetate. The combined organic layer was washed with brine, dried over anhydrous Na₂SO₄, filtered and concentrated under vacuum. The residue was purified by flash column chromatography with 0~60% ethyl acetate in petroleum ether to afford tert-butyl 3-((3-(2-(2,6-dioxopiperidin-3-yl)-1,3-dioxoisoindolin-4-yl)prop-2-yn-1-yl)oxy)propanoate (7 g, ~80% purity) as a yellow oil, which was used in the next step without further purification. MS (ESI) calculated for (C₂₃H₂₄N₂O₇) [M+H]⁺, 441.2; found, 441.0. ¹H NMR (300 MHz, Methanol-d4) δ 7.97 - 7.74 (m, 3H), 5.20 - 5.14 (m, 1H), 4.49 (s, 2H), 3.93 (t, *J* = 6.3 Hz, 2H), 2.95 - 2.65 (m, 3H), 2.56 (t, *J* = 6.3 Hz, 2H), 2.20 - 2.12 (m, 1H), 1.47 (s, 9H).

### Step 3: Synthesis of tert-butyl 3-(3-(2-(2,6-dioxopiperidin-3-yl)-1,3-dioxoisoindolin-4-yl)propoxy)propanoate.

To a solution of tert-butyl 3-((3-(2-(2,6-dioxopiperidin-3-yl)-1,3-dioxoisoindolin-4-yl)prop-2-yn-1-yl)oxy)propanoate (7.0 g, 15.89 mmol) in methanol (100 mL) was added Pd/C (10%, 1.4 g) under nitrogen atmosphere. The mixture was stirred at room temperature for 16 h under hydrogen atmosphere (2 atm). The solids were filtered out and the filtrate was concentrated under vacuum. The residue was purified by reverse phase flash column chromatography with 10~50% acetonitrile in water to afford tert-butyl 3-(3-(2-(2,6-dioxopiperidin-3-yl)-1,3-dioxoisoindolin-4-yl)propoxy)propanoate (4.2 g, 59%) as a white solid. MS (ESI) calculated for (C₂₃H₂₈N₂O₇) [M+H]⁺, 445.2; found, 445.3.

### Step 4: Synthesis of 3-(3-(2-(2,6-dioxopiperidin-3-yl)-1,3-dioxoisoindolin-4-yl)propoxy)propanoic acid.

A mixture of tert-butyl 3-(3-(2-(2,6-dioxopiperidin-3-yl)-1,3-dioxoisoindolin-4-yl)propoxy)propanoate (4.2 g, 9.45 mmol) in dichloromethane (20 mL) and trifluoroacetic acid (20 mL) was stirred at room temperature for 3 h before concentrated under vacuum. The residue was purified by reverse phase flash column chromatography with 5~30% acetonitrile in water to afford 3-(3-(2-(2,6-dioxopiperidin-3-yl)-1,3-dioxoisoindolin-4-yl)propoxy)propanoic acid (1.8681 g, 51%) as a light yellow semi-solid. MS (ESI) calculated for (C₁₉H₂₀N₂O₇) [M+H]⁺, 389.1; found, 388.8. ¹H NMR (400 MHz, DMSO-*d*₆) δ 12.14 (s, 1H), 11.12 (s, 1H), 7.87 - 7.59 (m, 3H), 5.20 - 5.11 (m, 1H), 3.57 (t, *J* = 6.0 Hz, 2H), 3.40 (t, *J =* 6.4 Hz, 2H), 3.12 - 3.06 (m, 2H), 2.94 - 2.85 (m, 1H), 2.68 - 2.52 (m, 2H), 2.44 (t, *J* = 6.4 Hz, 2H), 2.13 - 2.01 (m, 1H), 1.89 - 1.79 (m, 2H).

### Example 5A-6: Synthesis of (1r,3r)-3-(3-(2-(2,6-dioxopiperidin-3-yl)-1-oxoisoindolin-4-yl)propoxy)cyclobutane-1-carboxylic acid.

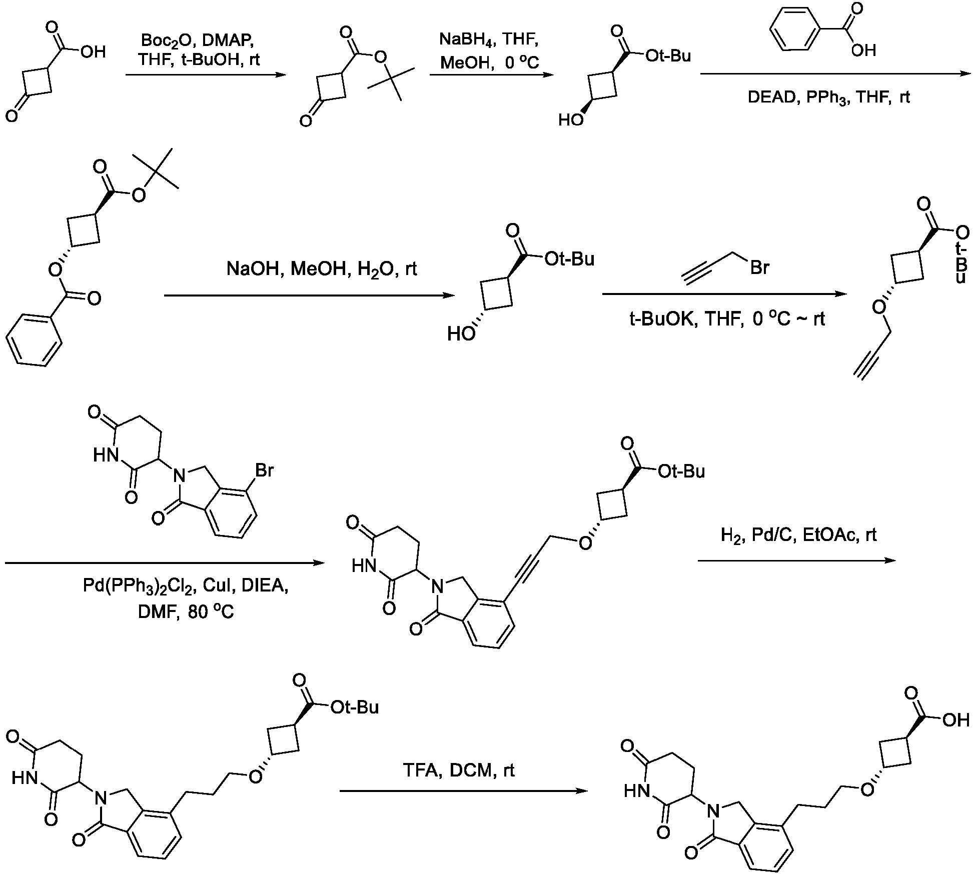

### Step 1: Synthesis of tert-butyl 3-oxocyclobutane-1-carboxylate.

To a solution of 3-oxocyclobutane-1-carboxylic acid (35.0 g, 306.7 mmol) in tetrahydrofuran (350 mL) and 2-methylpropan-2-ol (350 mL) were added N,N-dimethylpyridin-4-amine (100.4 g, 460.1 mmol) and Boc₂O (49.8 g, 228.6 mmol) at room temperature. After stirring for 5 h at room temperature under nitrogen, the reaction mixture was poured into water and extracted with ethyl acetate. The organic layers were dried over anhydrous sodium sulfate, filtered and concentrated under vacuum. The residue was purified by flash column chromatography with 0~20% ethyl acetate in petroleum ether to afford tert-butyl 3-oxocyclobutane-1-carboxylate (17.0 g, 23%) as a yellow oil. ¹H NMR (300 MHz, DMSO-*d*₆) δ 3.37 - 3.11 (m, 5H), 1.44 (s, 9H).

### Step 2: Synthesis of cis-tert-butyl-3-hydroxycyclobutane-1-carboxylate.

To a solution of tert-butyl 3-oxocyclobutane-1-carboxylate (17.0 g, 100.0 mmol) in tetrahydrofuran (170 mL) and methanol (20 mL) was added sodium borohydride (1.9 g, 50.00 mmol) in portions at 0 °C. After stirring for 15 min at 0 °C, the reaction mixture was concentrated under vacuum. The residue was diluted with water and extracted with ethyl acetate three times. The organic layers were dried over anhydrous sodium sulfate, filtered and concentrated under vacuum to afford cis-tert-butyl-3-hydroxycyclobutane-1-carboxylate (18.5 g, crude) as a yellow oil, which was used in the next step without further purification. ¹H NMR (300 MHz, Chloroform-d) δ 4.20 - 4.03 (m, 1H), 2.63 - 2.39 (m, 4H), 2.18 - 1.97 (m, 2H), 1.43 (s, 9H).

### Step 3: Synthesis of trans-3-(tert-butoxycarbonyl)cyclobutyl benzoate.

To a solution of cis-tert-butyl-3-hydroxycyclobutane-1-carboxylate (12.0 g, 69.77 mmol), benzoic acid (17.5 g, 143.7 mmol) and triphenylphosphine (35.6 g, 136.0 mmol) in tetrahydrofuran (100 mL) was added DEAD (25.0 g, 143.7 mmol) dropwise at room temperature. After stirring for 16 h at room temperature, the reaction mixture was quenched by the addition of water, and then extracted with ethyl acetate three times. The organic layers were dried over anhydrous sodium sulfate, filtered and concentrated under vacuum. The residue was purified by flash column chromatography with 0~20% ethyl acetate in petroleum ether to afford trans-3-(tert-butoxycarbonyl)cyclobutyl benzoate (14.3 g, 74%) as a yellow oil. ¹H NMR (300 MHz, DMSO-*d*₆) δ 8.03 - 7.92 (m, 2H), 7.74 - 7.61 (m, 1H), 7.61 - 7.48 (m, 2H), 5.34 - 5.19 (m, 1H), 3.18 - 2.99 (m, 1H), 2.66 - 2.34 (m, 4H), 1.44 (s, 9H).

### Step 4: Synthesis of trans-tert-butyl (1r,3r)-3-hydroxycyclobutane-1-carboxylate.

To a solution of *trans*-3-(tert-butoxycarbonyl)cyclobutyl benzoate (14.3 g, 51.81 mmol) in methanol (150 mL) and water (75 mL) was added sodium hydroxide (2.3 g, 57.5 mmol) at room temperature. After stirring for 16 h at room temperature, the reaction mixture was quenched by the addition of water and extracted with ethyl acetate three times. The organic layers were dried over anhydrous sodium sulfate, filtered and concentrated under vacuum. The residue was purified by flash column chromatography with 0~20% ethyl acetate in petroleum ether to afford *trans*-tert-butyl-3-hydroxycyclobutane-1-carboxylate (6.3 g, 71%) as a light yellow solid. ¹H NMR (300 MHz, Chloroform-d) δ 4.61 - 4.41 (m, 1H), 2.97 - 2.79 (m, 1H), 2.54 - 2.44 (m, 2H), 2.24 - 2.07 (m, 2H), 1.43 (s, 9H).

### Step 5: Synthesis of trans-tert-butyl-3-(prop-2-yn-1-yloxy)cyclobutane-1-carboxylate.

To a solution of *trans*-tert-butyl-3-hydroxycyclobutane-1-carboxylate (6.0 g, 34.88 mmol) and 3-bromoprop-1-yne (6.2 g, 52.32 mmol) in tetrahydrofuran (30 mL) was added t-BuOK (53 mL, 1 M in THF) dropwise at 0 °C. The mixture was stirred at room temperature at for 48 h before concentrated under vacuum. The residue was diluted with water and extracted with ethyl acetate three times. The organic layers were dried over anhydrous sodium sulfate, filtered and concentrated under vacuum. The residue was purified by flash column chromatography with 0~50% ethyl acetate in petroleum ether to afford *trans-tert-*butyl-3-(prop-2-yn-1-yloxy)cyclobutane-1-carboxylate (6.0 g, 82%) as a yellow oil. ¹H NMR (300 MHz, Chloroform-d) δ 4.42 - 4.29 (m, 1H), 4.14 - 4.01 (m, 2H), 3.03 - 2.87 (m, 1H), 2.56 - 2.45 (m, 2H), 2.42 (t, *J=* 2.4 Hz, 1H), 2.37 - 2.12 (m, 2H), 1.45 (s, 9H).

### Step 6: Synthesis of trans-tert-butyl-3-((3-(2-(2,6-dioxopiperidin-3-yl)-1-oxoisoindolin-4-yl)prop-2-yn-1-yl)oxy)cyclobutane-1-carboxylate.

To a degassed solution of 3-(4-bromo-1-oxoisoindolin-2-yl)piperidine-2,6-dione (6.0 g, 18.58 mmol) in anhydrous DMF (100 mL) were added [1,1'-Bis(diphenylphosphino)ferrocene]dichloropalladium(II) dichloromethane adduct (2.0 g, 2.79 mmol), copper(I) iodide (0.9 g, 4.64 mmol), N,N-diisopropylethylamine (70 mL) and *trans-*tert-butyl-3-(prop-2-yn-1-yloxy)cyclobutane-1-carboxylate (5.9 g, 27.86 mmol) at room temperature. After stirring for 16 h at 80 °C, the reaction mixture was poured into water and extracted with ethyl acetate. The organic layers were dried over anhydrous sodium sulfate, filtered and concentrated under vacuum. The residue was purified by reversed phase flash column chromatography with 15~100% acetonitrile in water to afford *trans*-tert-butyl-3-((3-(2-(2,6-dioxopiperidin-3-yl)-1-oxoisoindolin-4-yl)prop-2-yn-1-yl)oxy)cyclobutane-1-carboxylate (1.6 g, 19%) as a pink solid. MS (ESI) calculated for (C₂₅H₂₈N₂O₆) [M+H]⁺, 453.2; found, 453.2.

### Step 7: Synthesis of trans-tert-butyl-3-(3-(2-(2,6-dioxopiperidin-3-yl)-1-oxoisoindolin-4-yl)propoxy)cyclobu-tane-1-carboxylate.

A mixture of *trans*-tert-butyl-3-((3-(2-(2,6-dioxopiperidin-3-yl)-1-oxoisoindolin-4-yl)prop-2-yn-1-yl)oxy)cyclobutane-1-carboxylate (1.4 g, 3.10 mmol) and Pd/C (0.3 g, 10%) in ethyl acetate (50 mL) was stirred at room temperature for 16 h under H₂ atmosphere (2 atm). The solids were filtered off. The filtrate was concentrated under vacuum to afford *trans*-tert-butyl-3-(3-(2-(2,6-dioxopiperidin-3-yl)-1-oxoisoindolin-4-yl)propoxy)cyclobutane-1-carboxylate (1.1 g, crude) as a brown solid, which was used in the next step without further purification. MS (ESI) calculated for (C₂₅H₃₂N₂O₆) [M+H]⁺, 457.2; found, 457.2.

### Step 8: Synthesis of trans-3-(3-(2-(2,6-dioxopiperidin-3-yl)-1-oxoisoindolin-4-yl)propoxy)cyclobutane-1-carboxylic acid.

To a solution of *trans*-3-(3-(2-(2,6-dioxopiperidin-3-yl)-1-oxoisoindolin-4-yl)propoxy)cyclobutane-1-carboxylate (1.1 g, 2.41 mmol) in dichloromethane (10 mL) was added trifluoroacetic acid (10 mL) at room temperature. After stirring at room temperature for 16 h, the reaction mixture was concentrated under vacuum. The residue was purified by reversed phase flash column chromatography with 15~50% acetonitrile in water to afford *trans*-3-(3-(2-(2,6-dioxopiperidin-3-yl)-1-oxoisoindolin-4-yl)propoxy)cyclobutane-1-carboxylic acid (460.3 mg, 48%) as a white solid, which contained ~60% cis-isomer by HNMR. MS (ESI) calculated for (C₂₁H₂₄N₂O₆) [M+H]⁺, 401.2; found, 401.1.¹H NMR (400 MHz, DMSO-*d*₆) δ 12.17 (br, 1H), 11.00 (s, 1H), 7.63 - 7.54 (m, 1H), 7.51 - 7.42 (m, 2H), 5.22 - 5.06 (m, 1H), 4.47 (d, *J* = 17.2 Hz, 1H), 4.37 - 4.25 (m, 1H), 4.10 - 3.75 (m, 1H), 3.33 - 3.23 (m, 2H), 3.03 - 2.53 (m, 5H), 2.48 - 2.29 (m, 3H), 2.18 - 1.75 (m, 5H).

### Example 5A-7: Synthesis of 2-(4-(3-(2-(2,6-dioxopiperidin-3-yl)-1,3-dioxoisoindolin-4-yl)propyl)piperazin-1-yl)acetic acid.

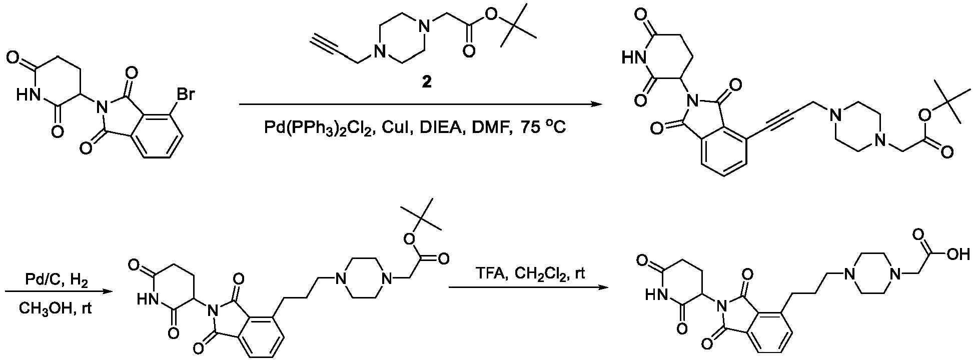

### Step 1: Synthesis of tert-butyl 2-(4-(3-(2-(2,6-dioxopiperidin-3-yl)-1,3-dioxoisoindolin-4-yl)prop-2-yn-1-yl)piperazin-1-yl)acetate.

To a degassed solution of 4-bromo-2-(2,6-dioxopiperidin-3-yl)isoindoline-1,3-dione (1.3 g, 3.86 mmol) in N,N-dimethylformamide (18 mL) were added tert-butyl 2-(4-(prop-2-ynyl)piperazin-1-yl)acetate (1.4 g, 5.57 mmol), Pd(PPh₃)₂Cl₂ (423.3 mg, 0.60 mmol), DIEA (12 mL) and CuI (251.1 mg, 1.32 mmol). The resulting solution was stirred at 75 °C for 4 h under nitrogen. The reaction was quenched by the addition of water, and then extracted with ethyl acetate. The combined organic layer was washed with brine, dried over anhydrous sodium sulfate and filtered. The filtrate was evaporated under vacuum. The residue was purified by flash column chromatography with 0~10% methanol in dichloromethane to afford tert-butyl 2-(4-(3-(2-(2,6-dioxopiperidin-3-yl)-1,3-dioxoisoindolin-4-yl)prop-2-yn-1-yl)piperazin-1-yl)acetate (2.5 g, 70%) as a yellow solid. MS (ESI) calculated for (C₂₆H₃₀N₄O₆) [M+H]⁺, 495.2; found, 495.1.

### Step 2: Synthesis of tert-butyl 2-(4-(3-(2-(2,6-dioxopiperidin-3-yl)-1,3-dioxoisoindolin-4-yl)propyl)piperazin-1-yl)acetate.

To a solution of tert-butyl 2-(4-(3-(2-(2,6-dioxopiperidin-3-yl)-1,3-dioxoisoindolin-4-yl)prop-2-ynyl)piperazin-1-yl)acetate (2.1 g, 4.25 mmol) in methanol (50 mL) was added Pd/C (dry, 0.42 g). The resulting solution was stirred at room temperature for 16 h under hydrogen (2 atm). The solids were filtered out. The filtrate was evaporated under vacuum to afford tert-butyl 2-(4-(3-(2-(2,6-dioxopiperidin-3-yl)-1,3-dioxoisoindolin-4-yl)propyl)piperazin-1-yl)acetate (1.6 g, crude) as a yellow solid, which was used in the next step without further purification. MS (ESI) calculated for (C₂₆H₃₄N₄O₆) [M+H]⁺, 499.2; found,499.0.

### Step 3: Synthesis of 2-(4-(3-(2-(2,6-dioxopiperidin-3-yl)-1,3-dioxoisoindolin-4-yl)propyl)piperazin-1-yl)acetic acid TFA salt.

To a solution of tert-butyl 2-(4-(3-(2-(2,6-dioxopiperidin-3-yl)-1,3-dioxoisoindolin-4-yl)propyl)piperazin-1-yl)acetate (2.1 g, 4.21 mmol) in dichloromethane (20 mL) was added trifluoroacetic acid (20 mL). The resulting solution was stirred at room temperature for 4 h before concentrated under vacuum. The residue was purified by Pre-HPLC with the following conditions: [Column: XSelect CSH Prep C18 OBD Column, 5um,19*150 mm; Mobile Phase A: Water (0.05%TFA ), Mobile Phase B: ACN; Flow rate: 25 mL/min; Gradient: 5% B to 20% B in 7 min; 254/220 nm] to afford 2-(4-(3-(2-(2,6-dioxopiperidin-3-yl)-1,3-dioxoisoindolin-4-yl)propyl)piperazin-1-yl)acetic acid TFA salt (398.0 mg, 21%) as a yellow solid. MS (ESI) calculated for (C₂₂H₂₆N₄O₆) [M+H]⁺, 443.2; found, 442.9. ¹H NMR (300 MHz, DMSO-*d*₆) δ 11.15 (s, 1H), 7.95 - 7.73 (m, 3H), 5.17 - 5.11 (m, 1H), 3.74 - 3.29 (m, 3H), 3.25 - 2.73 (m, 11H), 2.64 (s, 1H), 2.60 - 2.52 (m, 1H), 2.46 - 2.45 (m, 1H), 2.11 - 1.92 (m, 3H).

### Example 5A-8: Synthesis of 3-(2-(4-(2-(2-(2-((2-(2,6-dioxopiperidin-3-yl)-1,3-dioxoisoindolin-4-yl)amino)ethoxy)ethoxy)ethyl)piperazin-1-yl)ethoxy)propanoic acid.

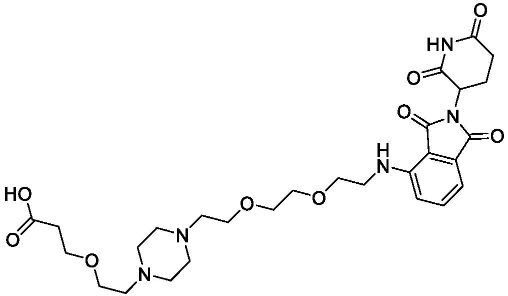

### Step 1: Synthesis of benzyl N-[2-[2-(2-hydroxyethoxy)ethoxy]ethyl]carbamate.

To a solution of 2-[2-(2-aminoethoxy)ethoxy]ethanol (10 g, 67.03 mmol, 1 eq) in THF (50 mL) and H₂O (50 mL) was added Na₂CO₃ (14.21 g, 134.06 mmol, 2 eq) and CbzCl (13.72 g, 80.44 mmol, 11.43 mL, 1.2 eq) dropwise. The mixture was stirred at 15 °C for 3 hr. The mixture was poured into EtOAc (200 mL), and petitioned. The organic layer was washed with H₂O (300 mL*2), dried over Na₂SO₄, and concentrated. The residue was purified by column chromatography (Petroleum ether/Ethyl acetate = 1/1). benzyl N-[2-[2-(2-hydroxyethoxy)ethoxy]ethyl]carbamate (9.1 g, 32.12 mmol, 47.92% yield) was obtained as colorless oil.

### Step 2: Synthesis of 2-[2-[2-(benzyloxycarbonylamino)ethoxy]ethoxy]ethyl 4-methylbenzenesulfonate.

To a solution of benzyl N-[2-[2-(2-hydroxyethoxy)ethoxy]ethyl]carbamate (9.1 g, 32.12 mmol, 1 eq) in DCM (100 mL) was added TsCl (7.35 g, 38.54 mmol, 1.2 eq) and Py (5.08 g, 64.24 mmol, 5.18 mL, 2 eq). The mixture was stirred at 15 °C for 16 hr. The mixture was sequentially washed with H₂O (200 mL) and aq. citric acid (200 mL), dried over Na₂SO₄, and concentrated. The residue was purified by column chromatography (Petroleum ether/Ethyl acetate = 1/1). 2-[2-[2-(benzyloxycarbonylamino)ethoxy]ethoxy]ethyl 4-methylbenzenesulfonate (11.5 g, 26.29 mmol, 81.84% yield) was obtained as colorless oil.

### Step 3: Synthesis of tert-butyl 3-[2-(p-tolylsulfonyloxy)ethoxy]propanoate.

To a solution of tert-butyl 3-(2-hydroxyethoxy)propanoate (10 g, 52.57 mmol, 1 eq) in DCM (100 mL) was added TsCl (13.03 g, 68.34 mmol, 1.3 eq) and Py (8.32 g, 105.13 mmol, 8.49 mL, 2 eq). The mixture was stirred at 18 °C for 16 hr. The mixture was sequentially washed with H₂O (200 mL), aq. citric acid (200 mL) and H₂O (300 mL), dried over Na₂SO₄, and concentrated. The residue was purified by column chromatography (Petroleum ether/Ethyl acetate = 1/1). tert-butyl 3-[2-(p-tolylsulfonyloxy)ethoxy]propanoate (7.69 g, 22.33 mmol, 42.47% yield) was obtained as colorless oil.

### Step 4: Synthesis of benzyl 4-[2-(3-tert-butoxy-3-oxo-propoxy)ethyl]piperazine-1-carboxylate.

To a solution of tert-butyl 3-[2-(p-tolylsulfonyloxy)ethoxy]propanoate (7.69 g, 22.33 mmol, 1 eq) and benzyl piperazine-1-carboxylate (7.38 g, 33.49 mmol, 6.47 mL, 1.5 eq) in MeCN (100 mL) was added K₂CO₃ (9.26 g, 66.98 mmol, 3 eq) and NaI (669.34 mg, 4.47 mmol, 0.2 eq) at 15 °C. The mixture was stirred at 70 °C for 16 hr. The water (100 ml) was added to the mixture. The resulted mixture was concentrated under vacuum to remove MeCN, and extracted with ethyl acetate (100 ml*3). The combined organic phase was dried over Na₂SO₄, and concentrated under vacuum. The residue was purified by column chromatography (Petroleum ether/Ethyl acetate = 1/1). benzyl 4-[2-(3-tert-butoxy-3-oxo-propoxy)ethyl]piperazine-1-carboxylate (7.58 g, 19.31 mmol, 86.50% yield) was obtained as yellow oil. LCMS; C₂₁H₃₃N₂O₅ requires: 392, found: m/z = 393 [M+H]⁺.

### Step 5: Synthesis of tert-butyl 3-(2-piperazin-1-ylethoxy)propanoate.

To a solution of benzyl 4-[2-(3-tert-butoxy-3-oxo-propoxy)ethyl]piperazine-1-carboxylate (7.58 g, 19.31 mmol, 1 eq) in THF (60 mL) and i-PrOH (20 mL) was added Pd/C (500 mg, 19.31 mmol, 10% purity, 1 eq) and Pd(OH)₂ (500 mg, 3.56 mmol, 0.18 eq) under N₂. The mixture was stirred at 15 °C for 16 hr under H₂ (15 psi). The mixture was filtered, and concentrated. tert-butyl 3-(2-piperazin-1-ylethoxy)propanoate (4.74 g, 18.35 mmol, 95% yield) was obtained as colorless oil. LCMS; C₁₃H₂₆N₂O₃ requires: 258, found: m/z = 259 [M+H]⁺.

### Step 6: Synthesis of tert-butyl 3-[2-[4-[2-[2-[2-(benzyloxycarbonylamino)ethoxy]ethoxy]ethyl]piperazin-1-yl]ethoxy]propanoate.

To a solution of tert-butyl 3-(2-piperazin-1-ylethoxy)propanoate (4.74 g, 18.34 mmol, 1.5 eq) and 2-[2-[2-(benzyloxycarbonylamino)ethoxy]ethoxy]ethyl 4-methylbenzenesulfonate (5.35 g, 12.23 mmol, 1 eq) in MeCN (80 mL) was added K₂CO₃ (5.07 g, 36.69 mmol, 3 eq) and NaI (366.59 mg, 2.45 mmol, 0.2 eq) at 15 °C. The mixture was stirred at 70 °C for 16 hr. The water (100 ml) was added to the mixture. The resulted mixture was concentrated in vacuum to remove MeCN, and extracted with ethyl acetate (100 ml*3). The combined organic layer was dried over Na₂SO₄, and concentrated in vacuum. The residue was purified by column chromatography (Petroleum ether/Ethyl acetate = 1/2). tert-butyl 3-[2-[4-[2-[2-[2-(benzyloxycarbonylamino)ethoxy]ethoxy]ethyl]piperazin-1-yl]ethoxy]propanoate (4.9 g, 9.36 mmol, 76.52% yield) was obtained as yellow oil. LCMS; C₂₇H₄₅N₃O₇ requires: 523, found: m/z = 524 [M+H]⁺.

### Step 7: Synthesis of tert-butyl 3-[2-[4-[2-[2-(2-aminoethoxy)ethoxy]ethyl]piperazin-1-yl]ethoxy]propanoate.

To a solution of tert-butyl 3-[2-[4-[2-[2-[2-(benzyloxycarbonylamino)ethoxy]ethoxy]ethyl]piperazin-1-yl]ethoxy]propanoate (4.9 g, 9.36 mmol, 1 eq) in THF (100 mL) was added Pd/C (500 mg, 10% purity) under N₂. The mixture was stirred at 15 °C for 16 hr under H₂ (15 psi). The mixture was then stirred at 30 °C for another 16 hr. The mixture was filtered, and concentrated. tert-butyl 3-[2-[4-[2-[2-(2-aminoethoxy)ethoxy]ethyl]piperazin-1-yl]ethoxy]propanoate (3.2 g, 8.22 mmol, 87.79% yield) was obtained as colorless oil. LCMS; C₁₉H₃₉N₃O₅ requires: 389, found: m/z = 390 [M+H]⁺.

### Step 8: Synthesis of tert-butyl 3-[2-[4-[2-[2-[2-[[2-(2,6-dioxo-3-piperidyl)-1,3-dioxo-isoindolin-4-yl]amino]ethoxy]ethoxy]ethyl]piperazin-1-yl]ethoxy]propanoate.

To a solution of tert-butyl 3-[2-[4-[2-[2-(2-aminoethoxy)ethoxy]ethyl]piperazin-1-yl]ethoxy]propanoate (2.9 g, 7.44 mmol, 1 eq) and 2-(2,6-dioxo-3-piperidyl)-4-fluoro-isoindoline-1,3-dione (2.1 g, 7.44 mmol, 1 eq) in DMSO (20 mL) was added DIPEA (1.92 g, 14.89 mmol, 2.59 mL, 2 eq) at 15 °C. The mixture was stirred at 80 °C for 3 hr. The mixture was purified by prep-HPLC ([water (0.225% FA) - MeCN]; B%: 15% - 45%). The fractions containing the desired product was concentrated to remove MeCN under vacuum, and extracted with Dichloromethane (100 mL × 10). The combined organic layer was dried over Na₂SO₄, and concentrated under vacuum. tert-butyl 3-[2-[4-[2-[2-[2-[[2-(2,6-dioxo-3-piperidyl)-1,3-dioxo-isoindolin-4-yl]amino]ethoxy]ethoxy]ethyl]piperazin-1-yl]ethoxy]propanoate (2.3 g, 3.53 mmol, 47.46% yield, 99.2% purity) was obtained as yellow solid. LCMS; C₃₂H₄₇N₅O₉ requires: 645, found: m/z = 646 [M+H]⁺.

### Step 9: Synthesis of 3-[2-[4-[2-[2-[2-[[2-(2,6-dioxo-3-piperidyl)-1,3-dioxo-isoindolin-4-yl]amino]ethoxy]ethoxy]ethyl]piperazin-1-yl]ethoxy]propanoic acid.

A solution of tert-butyl 3-[2-[4-[2-[2-[2-[[2-(2,6-dioxo-3-piperidyl)-1,3-dioxo-isoindolin-4-yl]amino]ethoxy]ethoxy]ethyl]piperazin-1-yl]ethoxy]propanoate (2.3 g, 3.53 mmol, 1 eq) in 4 M aq. HCl (20 mL) was stirred at 15 °C for 18 hr. The mixture was concentrated by lyophilization. 3-[2-[4-[2-[2-[2-[[2-(2,6-dioxo-3-piperidyl)-1,3-dioxo-isoindolin-4-yl]amino]ethoxy]ethoxy]ethyl]piperazin-1-yl]ethoxy]propanoic acid (2.03 g, 2.77 mmol, 78.33% yield, 95.3% purity, 3HCl) was obtained as yellow solid. LCMS; C₂₈H₃₉N₅O₉ requires: 589, found: m/z = 590 [M+H]⁺.

### Example 5A-9: Synthesis of 4-((2-(2,6-dioxopiperidin-3-yl)-1,3-dioxoisoindolin-4-yl)amino)butanoic acid.

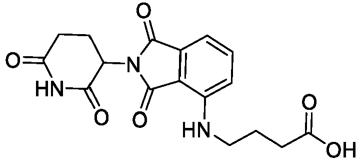

Step 1 product: tert-butyl 4-{[2-(2,6-dioxopiperidin-3-yl)-1,3-dioxoisoindol-4-yl]amino}butanoate (40.0 mg, 36.9%). LCMS: C₂₁H₂₅N₃O₆ requires: 415, found: m/z = 416 [M+H]⁺.

Step 2 product: 4-{[2-(2,6-dioxopiperidin-3-yl)-1,3-dioxoisoindol-4-yl]amino }butanoic acid (34.00 mg, 98.3%). LCMS: C₂₁H₂₅N₃O₆ requires: 359, found: m/z = 360 [M+H]⁺.

### Example 5A-10: Synthesis of 3-((2-(2,6-dioxopiperidin-3-yl)-1,3-dioxoisoindolin-4-yl)amino)propanoic acid.

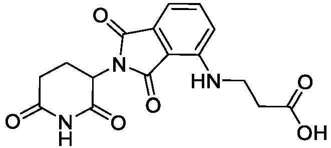

Step 1 product: tert-butyl 3-{[2-(2,6-dioxopiperidin-3-yl)-1,3-dioxoisoindol-4-yl]amino}propanoate (62.0 mg, 38.4%). LCMS: C₂₀H₂₃N₃O₆ requires: 401, found: m/z = 402 [M+H]⁺.

Step 2 product: 3-((2-(2,6-dioxopiperidin-3-yl)-1,3-dioxoisoindolin-4-yl)amino)propanoic acid (53 mg, 99%). LCMS: C₁₆H₁₅N₃O₆ requires: 345, found: m/z = 346 [M+H]⁺.

### Example 5A-11: Synthesis of (2-(2,6-dioxopiperidin-3-yl)-1,3-dioxoisoindolin-4-yl)glycine.

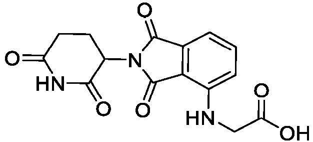

Step 1 product: tert-butyl 2-{[2-(2,6-dioxopiperidin-3-yl)-1,3-dioxoisoindol-4-yl]amino}acetate (28.0 mg, 20.6%). LCMS: C₁₉H₂₁N₃O₆ requires: 387, found: m/z = 388 [M+H]⁺.

Step 2 product: (2-(2,6-dioxopiperidin-3-yl)-1,3-dioxoisoindolin-4-yl)glycine (23 mg, 96%). LCMS: C₁₅H₁₃N₃O₆ requires: 331, found: m/z = 332 [M+H]⁺.

### Example 5A-12: Synthesis of trans-4-((2-(2,6-dioxopiperidin-3-yl)-1,3-dioxoisoindolin-4-yl)amino)cyclohexane-1-carboxylic acid.

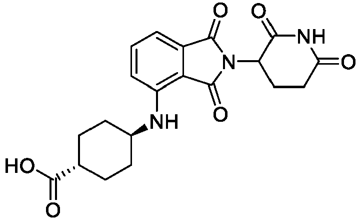

Step 1 product: trans-tert-butyl 4-((2-(2,6-dioxopiperidin-3-yl)-1,3-dioxoisoindolin-4-yl)amino)cyclohexane-1-carboxylate (43.40 mg, 47.0%).

Step 2 product: trans-4-((2-(2,6-dioxopiperidin-3-yl)-1,3-dioxoisoindolin-4-yl)amino)cyclohexane-1-carboxylic acid (38 mg, 99%).

### Example 5A-13: Synthesis of 3-(2-((2-(2,6-dioxopiperidin-3-yl)-1,3-dioxoisoindolin-4-yl)amino)ethoxy)propanoic acid.

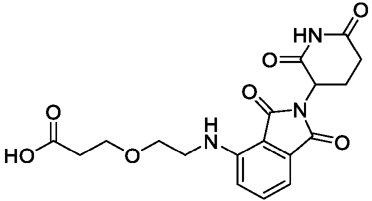

Step 1 product: tert-butyl 3-[2-[[2-(2,6-dioxo-3-piperidyl)-1,3-dioxo-isoindolin-4-yl]amino]ethoxy]propanoate (1.8 g, 51.9%). LCMS; C₂₂H₂₇N₃O₇ requires: 445, found: m/z = 468 [M+Na]⁺.

Step 2 product: 3-[2-[[2-(2,6-dioxo-3-piperidyl)-1,3-dioxo-isoindolin-4-yl]amino]ethoxy]propanoic acid (526.8 mg, 32%). LCMS; C₁₈H₁₉N₃O₇ requires: 389, found: m/z = 390 [M+H]⁺.

### Example 5A-14: Synthesis of 3-(2-(2-(2-((2-(2,6-dioxopiperidin-3-yl)-1,3-dioxoisoindolin-4-yl)amino)ethoxy)ethoxy)ethoxy)propanoic acid.

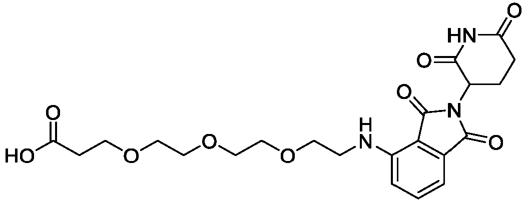

Step 1 product: tert-butyl 3-[2-[2-[2-[[2-(2,6-dioxo-3-piperidyl)-1,3-dioxoisoindolin-4-yl]amino]ethoxy]ethoxy]ethoxy]propanoate (1.6 g, 41%). LCMS; C₂₆H₃₅N₃O₉ requires: 533, found: m/z = 534 [M+H]⁺.

Step 2 product: 3-[2-[2-[2-[[2-(2,6-dioxo-3-piperidyl)-1,3-dioxo-isoindolin-4-yl]amino]ethoxy]ethoxy]ethoxy]propanoic acid (1.2 g, 73.62%). LCMS; C₂₂H₂₇N₃O₉ requires: **477,** found: m/z = 478 [M+H]⁺.

### Example 5A-15: Synthesis of 1-((2-(2,6-dioxopiperidin-3-yl)-1,3-dioxoisoindolin-4-yl)amino)-3,6,9,12-tetraoxapentadecan-15-oic acid.

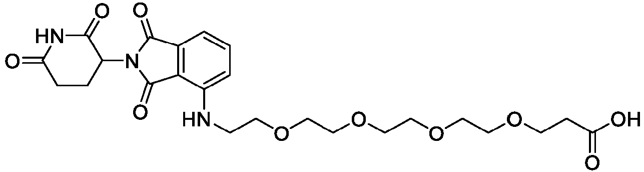

Step 1 product: tert-butyl 1-((2-(2,6-dioxopiperidin-3-yl)-1,3-dioxoisoindolin-4-yl)amino)-3,6,9,12-tetraoxapentadecan-15-oate (3.5 g, 21%). LCMS; C₂₈H₃₉N₃O₁₀ requires: 577, found: m/z = 578 [M+H]⁺.

Step 2 product: 1-((2-(2,6-dioxopiperidin-3-yl)-1,3-dioxoisoindolin-4-yl)amino)-3,6,9,12-tetraoxapentadecan-15-oic acid (2.1 g, 70%). LCMS; C₂₄H₃₁N₃O₁₀ requires: 521, found: m/z = 522 [M+H]⁺.

### Example 5A-16: Synthesis of 1-((2-(2,6-dioxopiperidin-3-yl)-1,3-dioxoisoindolin-4-yl)amino)-3,6,9,12,15-pentaoxaoctadecan-18-oic acid.

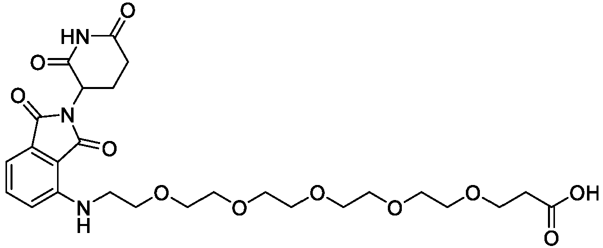

Step 1 product: tert-butyl 3-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]propanoate (1.8 g, 50.39%). LCMS: C₃₀H₄₃N₃O₁₁ requires: 621, found: m/z = 622 [M+H]⁺.

Step 2 product: 1-((2-(2,6-dioxopiperidin-3-yl)-1,3-dioxoisoindolin-4-yl)amino)-3,6,9,12,15-pentaoxaoctadecan-18-oic acid (1.2 g, 71.51%). LCMS: C₂₆H₃₅N₃O₁₁ requires: 565, found: m/z = 566 [M+H]⁺.

### Example 5B: Synthesis of 6-((2-(2,6-dioxopiperidin-3-yl)-1,3-dioxoisoindolin-4-yl)amino)spiro[3.3]heptane-2-carboxylic acid.

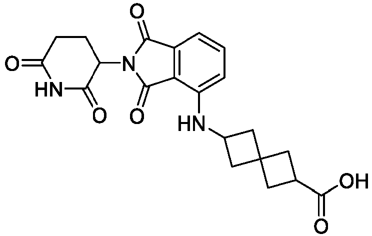

A mixture of (2,6-dioxopiperidin-3-yl)-4-fluoro-2,3-dihydro-1H-isoindole-1,3-dione (125 mg, 0.45 mmol), 6-aminospiro[3.3]heptane-2-carboxylic acid (78 mg, 0.50 mmol), ethylbis(propan-2-yl)amine (0.24 mL, 1.36 mmol) and DMF (3 mL) was allowed to stir at 90°C overnight. The mixture was purified by HPLC (5-95% MeCN in H₂O with 0.1% TFA) to afford 6-{[2-(2,6-dioxopiperidin-3-yl)-1,3-dioxoisoindol-4-yl]amino}spiro[3.3]heptane-2-carboxylic acid (10 mg, 5.4%). LCMS: C₂₁H₂₁N₃O₆ requires: 411, found: m/z = 412 [M+H]⁺.

### Example SC: Synthesis of 3-(3-((2-(2,6-dioxopiperidin-3-yl)-1-oxoisoindolin-4-yl)amino)-3-oxopropoxy)propanoic acid.

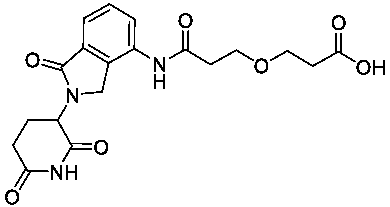

Step 1: A mixture of lenalidomide (270 mg, 1.04 mmol), 3-[3-(tert-butoxy)-3-oxopropoxy]propanoic acid (250 mg, 1.15 mmol), HATU (515 mg, 1.35 mmol), ethylbis(propan-2-yl)amine (0.73 mL, 4.17 mmol) and DMF (5 mL) was allowed to stir at rt for 6 h. EtOAc and H₂O were added. The organic layer was dried with MgSO₄, filtered, concentrated and purified by MPLC (20-100% EtOAc in hexanes) to afford tert-butyl 3-(2-{[2-(2,6-dioxopiperidin-3-yl)-1-oxo-3H-isoindol-4-yl]carbamoyl}ethoxy)propanoate (307 mg, 64%). LCMS: C₂₃H₂₉N₃O₇ requires: 459, found: m/z = 460 [M+H]⁺.

Step 2: A mixture of tert-butyl 3-(2-{[2-(2,6-dioxopiperidin-3-yl)-1-oxo-3H-isoindol-4-yl]carbamoyl}ethoxy)propanoate (307 mg, 0.67 mmol), CH₂Cl₂ (5 mL), and TFA (1 mL) was allowed to stir at r.t. for 2 h. The mixture was concentrated to afford 3-(3-((2-(2,6-dioxopiperidin-3-yl)-1-oxoisoindolin-4-yl)amino)-3-oxopropoxy)propanoic acid (269 mg, 99%). LCMS: C₁₉H₂₁N₃O₇ requires: 403, found: m/z = 404 [M+H]⁺.

### Example 5D: Synthesis of 3-(3-((2-(2,6-dioxopiperidin-3-yl)-1-oxoisoindolin-4-yl)amino)-3-oxopropoxy)propanoic acid.

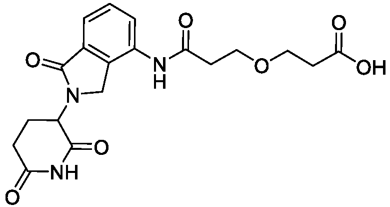

Step 1: A mixture of lenalidomide (270 mg, 1.04 mmol), 3-[3-(tert-butoxy)-3-oxopropoxy]propanoic acid (250 mg, 1.15 mmol), HATU (515 mg, 1.35 mmol), ethylbis(propan-2-yl)amine (0.73 mL, 4.17 mmol) and DMF (5 mL) was allowed to stir at rt for 6 h. EtOAc and H₂O were added. The organic layer was dried with MgSO₄, filtered, concentrated and purified by MPLC (20-100% EtOAc in hexanes) to afford tert-butyl 3-(2-{[2-(2,6-dioxopiperidin-3-yl)-1-oxo-3H-isoindol-4-yl]carbamoyl}ethoxy)propanoate (307 mg, 64%). LCMS: C₂₃H₂₉N₃O₇ requires: 459, found: m/z = 460 [M+H]⁺.

Step 2: A mixture of tert-butyl 3-(2-{[2-(2,6-dioxopiperidin-3-yl)-1-oxo-3H-isoindol-4-yl]carbamoyl}ethoxy)propanoate (307 mg, 0.67 mmol), CH₂Cl₂ (5 mL), and TFA (1 mL) was allowed to stir at r.t. for 2 h. The mixture was concentrated to afford 3-(3-((2-(2,6-dioxopiperidin-3-yl)-1-oxoisoindolin-4-yl)amino)-3-oxopropoxy)propanoic acid (269 mg, 99%). LCMS: C₁₉H₂₁N₃O₇ requires: 403, found: m/z = 404 [M+H]⁺.

### Example 5E: General procedure (B) for the synthesis of carboxylic acid intermediates for use in Example 4.

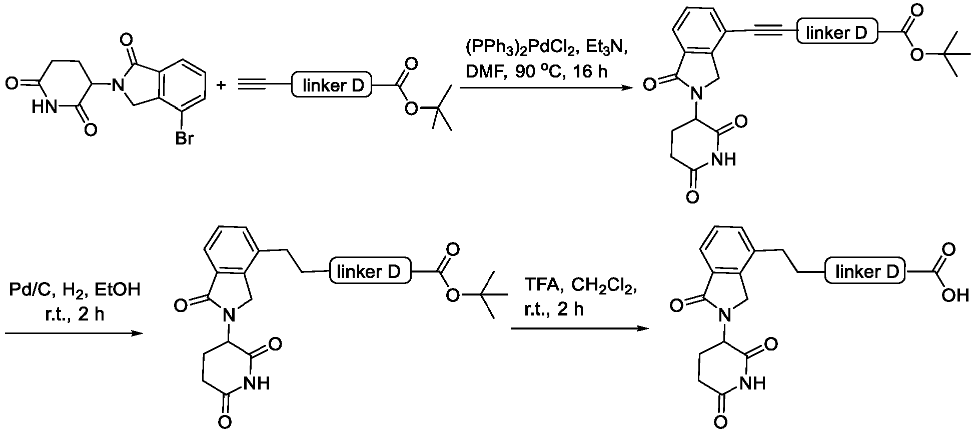

As used in this Example, "linker D" is -(CH₂-CH₂-O)ₓ-, wherein x is an integer from 1 to 5.

Step 1: A mixture of 3-(4-bromo-1-oxo-2,3-dihydro-1H-isoindol-2-yl)piperidine-2,6-dione (2.52 mmol), (PPh₃)₂PdCl₂ (0.15 mmol), CuI (0.25 mmol), and alkyne ester (5.04 mmol) were added to a vial. The vial was evacuated and backfilled with N₂ 5 times. DMF and triethylamine (30.3 mmol) were added and the mixture was allowed to stir at 90 °C overnight. The mixture was filtered through celite, washing with MeOH and EtOAc. EtOAc and saturated aqueous NaCl were added. The organic layer was dried with MgSO₄, filtered, concentrated and purified by reverse phase MPLC (5-100% MeCN in H2O on C18 column) to afford the product.

Step 2: A mixture of disubstituted alkyne (0.81 mmol), Pd/C 10wt% (0.08 mmol) and EtOH were mixed in a flask. The flask was evacuated and backfilled with H₂ 5 times and allowed to stir at r.t. for 2h. The mixture was filtered through celite washing with MeOH and EtOAc, concentrated and carried to the next step.

Step 3: A mixture of tert-butylester (0.81 mmol), CH₂Cl₂ (2 mL), and TFA (2 mL) was allowed to stir at r.t. for 2 h. The mixture was concentrated to afford the carboxylic acid product.

The general method of Example 4E, was used to synthesize the intermediates described in Examples 5E-1 through 5E-4.

### Example 5E-1: Synthesis of 3-(3-(2-(2,6-dioxopiperidin-3-yl)-1-oxoisoindolin-4-yl)propoxy)propanoic acid.

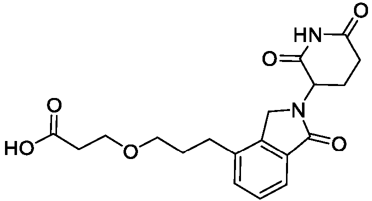

Step 1 product: tert-butyl 3-((3-(2-(2,6-dioxopiperidin-3-yl)-1-oxoisoindolin-4-yl)prop-2-yn-1-yl)oxy)propanoate (347 mg, 32.3%). LCMS: C₂₃H₂₆N₂O₆ requires: 426, found: m/z = 427 [M+H]⁺.

Step 2 product: tert-butyl 3-(3-(2-(2,6-dioxopiperidin-3-yl)-1-oxoisoindolin-4-yl)propoxy)propanoate (350 mg, 99%). LCMS: C₂₃H₃₀N₂O₆ requires: 430, found: m/z = 431 [M+H]⁺.

Step 3 product: 3-(3-(2-(2,6-dioxopiperidin-3-yl)-1-oxoisoindolin-4-yl)propoxy)propanoic acid (304 mg, 99%). LCMS: C₁₉H₂₂N₂O₆ requires: 374, found: m/z = 375 [M+H]⁺.

### Example 5E-2: Synthesis of 3-[2-[2-[3-[2-(2,6-dioxo-3-piperidyl)-1-oxo-isoindolin-4-yl]propoxy]ethoxy]ethoxy]propanoic acid.

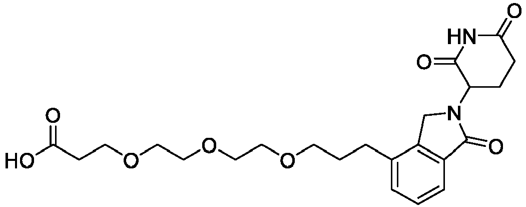

Step 1 product: tert-butyl 3-[2-[2-[3-[2-(2,6-dioxo-3-piperidyl)-1-oxo-isoindolin-4-yl]prop-2-ynoxy]ethoxy]ethoxy]propanoate (1.36 g, 55.29%). LCMS: C₂₇H₃₄N₂O₈ requires: 514, found: m/z = 537 [M+Na]⁺.

Step 2 product: tert-butyl 3-[2-[2-[3-[2-(2,6-dioxo-3-piperidyl)-1-oxo-isoindolin-4-yl]propoxy]ethoxy]ethoxy]propanoate (800 mg, 88.34%). LCMS: C₂₇H₃₈N₂O₈ requires: 518, found: m/z = 541 [M+Na]⁺.

Step 3 product: 3-[2-[2-[3-[2-(2,6-dioxo-3-piperidyl)-1-oxo-isoindolin-4-yl]propoxy]ethoxy]ethoxy]propanoic acid (270 mg, 62.28%). LCMS: C₂₃H₃₀N₂O₈ requires: 462, found: m/z = 463 [M+H]⁺.

### Example 5E-3: Synthesis of 3-[2-[2-[2-[3-[2-(2,6-dioxo-3-piperidyl)-1-oxo-isoindolin-4-yl]propoxy]ethoxy]ethoxy]ethoxy]propanoic acid.

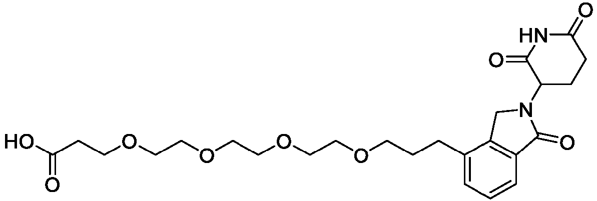

Step 1 product: tert-butyl 3-[2-[2-[2-[3-[2-(2,6-dioxo-3-piperidyl)-1-oxo-isoindolin-4-yl]prop-2-ynoxy]ethoxy]ethoxy]ethoxy]propanoate (320 mg, 13.6% yield). LCMS; C₂₉H₃₈N₂O₉ requires: 558, found: m/z = 581 [M+Na]⁺.

Step 2 product: tert-butyl 3-[2-[2-[2-[3-[2-(2,6-dioxo-3-piperidyl)-1-oxo-isoindolin-4-yl]propoxy]ethoxy]ethoxy]ethoxy]propanoate (278 mg, 86.3% yield). LCMS; C₂₉H₄₂N₂O₉ requires: 562, found: m/z = 563 [M+H]⁺.

Step 3 product: 3-[2-[2-[2-[3-[2-(2,6-dioxo-3-piperidyl)-1-oxo-isoindolin-4-yl]propoxy]ethoxy]ethoxy]ethoxy]propanoic acid (66.9 mg, 25.2%). LCMS; C₂₅H₃₄N₂O₉ requires: 506, found: m/z = 507 [M+H]⁺.

### Example 5E-4: Synthesis of 3-[2-[2-[2-[2-[3-[2-(2,6-dioxo-3-piperidyl)-1-oxo-isoindolin-4-yl]propoxy]ethoxy]ethoxy]ethoxy]ethoxy]propanoic acid.

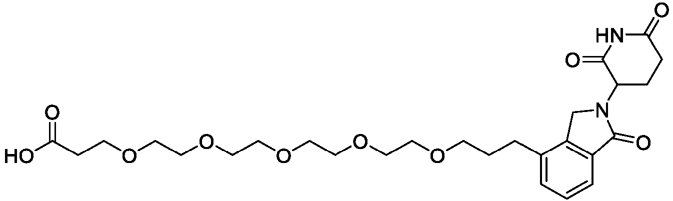

Step 1 product: tert-butyl 3-[2-[2-[2-[2-[3-[2-(2,6-dioxo-3-piperidyl)-1-oxo-isoindolin-4-yl]prop-2-ynoxy]ethoxy]ethoxy]ethoxy]ethoxy]propanoate (562 mg, 31.17%). LCMS; C₃₁H₄₂N₂O₁₀ requires: 602, found: m/z = 603 [M+H]⁺.

Step 2 product: tert-butyl 3-[2-[2-[2-[2-[3-[2-(2,6-dioxo-3-piperidyl)-1-oxo-isoindolin-4-yl]propoxy]ethoxy]ethoxy]ethoxy]ethoxy]propanoate (565 mg, 99%). LCMS: C₃₁H₄₆N₂O₁₀ requires: 606, found: m/z = 629 [M+Na]⁺.

Step 3 product: 3-[2-[2-[2-[2-[3-[2-(2,6-dioxo-3-piperidyl)-1-oxo-isoindolin-4-yl]propoxy]ethoxy]ethoxy]ethoxy]ethoxy]propanoic acid (223 mg, 41.06%). LCMS: C₂₇H₃₈N₂O₁₀ requires: 550, found: m/z = 551 [M+H]⁺.

### Example 5F: General procedure for the synthesis of a carboxylic acid intermediate.

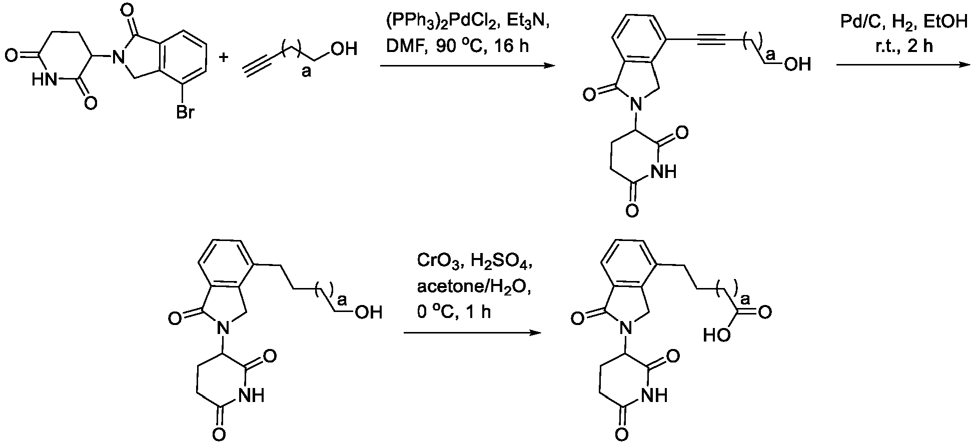

As used in this Example, "a" is an integer from 1 to 4.

Step 1: 3-(4-Bromo-1-oxoisoindolin-2-yl)piperidine-2,6-dione (4.6 mmol), copper iodide (177 mg) and bis-triphenylphosphine-palladium dichloride (326 mg) were evacuated and flushed with nitrogen 3 times. DMF (5 mL), triethylamine (6.5 mL) and the alkyne (27.9 mmol) were added and the vial was flushed with nitrogen, sealed and heated to 80 °C for 20 h. The mixture was cooled to room temperature and was diluted with DCM/ethyl acetate (1:1, 20 mL) and the solid was filtered over a pad of Celite. The solid was stirred with acetonitrile for 16 h. The solids were filtered and concentrated to give the disubstituted alkyne product.

Step 2: The disubstituted alkyne (2.2 mmol) was dissolved in methanol (40 mL). Palladium over charcoal (10%, 235 mg) was added and the flask was filled with hydrogen at 65 psi for 3 h. The mixture was filtered over Celite and washed with methanol to give the alcohol product.

Step 3: Chromic acid (360 mg, 3.6 mmol) was added to 3 M sulfuric acid (3 mL) to make a solution of chromium oxidant (Jones' reagent). The alcohol (1.2 mmol) was suspended in acetone (2.5 mL) and 3 M sulfuric acid (0.5 mL) and the suspension was cooled to 0 °C. The Jones' reagent was slowly added to the alcohol suspension and allowed to stir for 1 h. The mixture was poured into of iced water (20 mL) and the solid was filtered and washed with water The aqueous solution was extracted with (2 × 20 mL) EtOAc, washed with brine and concentrated. The organic fractions were combined with the solid and the mixture was purified by flash column chromatography (0-25% methanol in DCM) to afford the acid product.

The general method was used to synthesize the intermediates described in Examples 5F-1 and 5F-2.

### Example SF-1: Synthesis of 4-(2-(2,6-dioxopiperidin-3-yl)-1-oxoisoindolin-4-yl)butanoic acid.

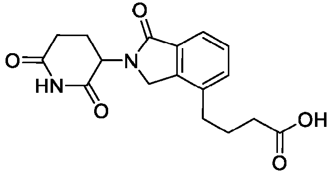

Step 1 product: 3-(4-(4-hydroxybut-1-yn-1-yl)-1-oxoisoindolin-2-yl)piperidine-2,6-dione (690 mg, 47%). LCMS; C₁₇H₁₆N₂O₄ requires: 312, found: m/z = 335 [M+Na]⁺.

Step 2 product: 3-(4-(4-hydroxybutyl)-1-oxoisoindolin-2-yl)piperidine-2,6-dione (361 mg, 52%). LCMS; C₁₇H₂₀N₂O₄ requires: 316, found: m/z = 339 [M+Na]⁺.

Step 3 product: 4-(2-(2,6-dioxopiperidin-3-yl)-1-oxoisoindolin-4-yl)butanoic acid (214 mg, 57%). LCMS; C₁₇H₁₈N₂O₅ requires: 330, found: m/z = 353 [M+Na]⁺.

### Example SF-2: Synthesis of 5-(2-(2,6-dioxopiperidin-3-yl)-1-oxoisoindolin-4-yl)pentanoic acid.

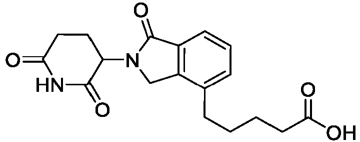

Step 1 product: 3-(4-(5-hydroxypent-1-yn-1-yl)-1-oxoisoindolin-2-yl)piperidine-2,6-dione (325 mg, 58%). LCMS; C₁₈H₁₈N₂O₄ requires: 326, found: m/z = 349 [M+Na]⁺.

Step 2 product: 3-(4-(5-hydroxypentyl)-1-oxoisoindolin-2-yl)piperidine-2,6-dione (160 mg, 99%). LCMS; C₁₈H₂₂N₂O₄ requires: 330, found: m/z = 353 [M+Na]⁺.

Step 3 product: 5-(2-(2,6-dioxopiperidin-3-yl)-1-oxoisoindolin-4-yl)pentanoic acid (74 mg, 60%). LCMS; C₁₈H₂₀N₂O₅ requires: 344, found: m/z = 367 [M+Na]⁺.

### Example 5G: General procedure for the synthesis of a carboxylic acid intermediate.

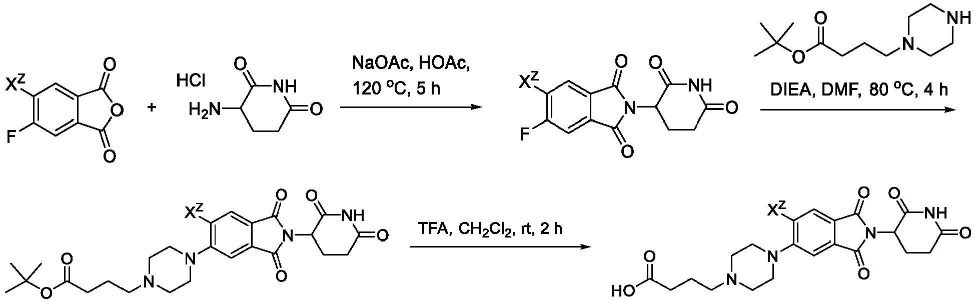

In this Example, X^{Z} is -H or -F.

Step 1: To a solution of fluoro-benzofuran-1,3-dione (27.16 mmol) in HOAc (50 mL) were added sodium acetate (46.17 mmol) and 3-aminopiperidine-2,6-dione hydrochloride (38.02 mmol). The reaction mixture was stirred at 120 °C for 5 h. The mixture was cooled to room temperature and diluted with water. The solids were collected by filtration and dried to afford the fluoroimide intermediate.

Step 2: To a solution of fluoroimide (0.68 mmol) in DMF (30 mL) was added tert-butyl 4-(piperazin-1-yl)butanoate (0.68 mmol) and N-ethyl-N-isopropylpropan-2-amine (1.4 mmol). The reaction mixture was stirred at 80 °C for 4 h. The resulting mixture was cooled to room temperature and diluted with water. The aqueous phase was extracted with ethyl acetate. The combined organic layer was washed with brine and water, dried over anhydrous sodium sulfate, filtered and concentrated under vacuum to afford the tert-butyl ester intermediate (3.3 g, crude) which was used in the next step without further purification.

Step 3: To a solution of the tert-butyl ester intermediate (6.57 mmol) in dichloromethane (20 mL) was added trifluoroacetic acid (10 mL). The reaction mixture was stirred at room temperature for 2 h. The solvent was removed under vacuum. The residue was purified by reverse phase flash column chromatography (20-80% acetonitrile in water) to afford the acid product (38% over 2 steps).

The general method was used to synthesize the intermediates described in Examples 5G-1 and 5G-3.

### Example 5G-1: Synthesis of tert-butyl 4-(piperazin-1-yl)butanoate.

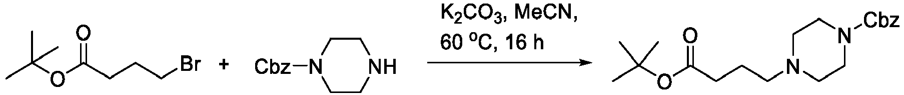

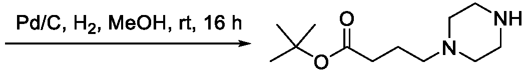

Step 1: To a solution of tert-butyl 4-bromobutanoate (8.5 g, 38.10 mmol) and benzyl piperazine-1-carboxylate (10.1 g, 45.72 mmol) in acetonitrile (100 mL) was added K₂CO₃ (10.5 g, 76.20 mmol). The resulting mixture was stirred at 60 °C for 16 h under nitrogen atmosphere. The solids were filtered out and the filtrate was concentrated under vacuum. The residue was purified by flash column chromatography with 10~70% ethyl acetate in petroleum ether to afford benzyl 4-(4-(tert-butoxy)-4-oxobutyl)piperazine-1-carboxylate (11.0 g, 79%) as colorless oil. LCMS: C₂₀H₃₀N₂O₄ requires: 362, found: m/z = 363 [M+H]⁺.

Step 2: To a solution of benzyl 4-(4-(tert-butoxy)-4-oxobutyl)piperazine-1-carboxylate (11.0 g, 30.39 mmol) in methanol (150 mL) was added Pd/C (10%, 2 g) under nitrogen atmosphere. The mixture was stirred at room temperature for 16 h under hydrogen atmosphere (2 atm). The solids were filtered out and the filtrate was concentrated under vacuum to afford tert-butyl 4-(piperazin-1-yl)butanoate (6.6 g, crude) which was used in the next step without further purification. LCMS: C₁₂H₂₄N₂O₂ requires: 228, found: m/z = 229 [M+H]⁺.

### Example 5G-2: Synthesis of 4-(4-(2-(2,6-dioxopiperidin-3-yl)-1,3-dioxoisoindolin-5-yl)piperazin-1-yl)butanoic acid.

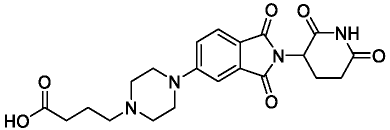

Step 1 product: 2-(2,6-dioxopiperidin-3-yl)-5-fluoroisoindoline-1,3-dione (3.0 g, 50%). LCMS: C₁₃H₉FN₂O₄ requires: 276, found: m/z = 277 [M+H]⁺.

Step 2 product: tert-butyl 4-(4-(2-(2,6-dioxopiperidin-3-yl)-1,3-dioxoisoindolin-5-yl)piperazin-1-yl)butanoate (4.4 g, 84%). LCMS: C₂₅H₃₂N₄O₆ requires: 484, found: m/z = 485 [M+H]⁺.

Step 3 product: 4-(4-(2-(2,6-dioxopiperidin-3-yl)-1,3-dioxoisoindolin-5-yl)piperazin-1-yl)butanoic acid TFA salt (3.35 g, 56%). LCMS: C₂₁H₂₄N₄O₆ requires: 428, found: m/z = 429 [M+H]⁺.

### Example 5G-3: Synthesis of 4-(4-(2-(2,6-dioxopiperidin-3-yl)-6-fluoro-1,3-dioxoisoindolin-5-yl)piperazin-1-yl)butanoic acid.

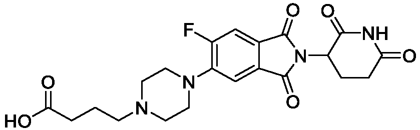

Step 1 product: 2-(2,6-dioxopiperidin-3-yl)-5,6-difluoroisoindoline-1,3-dione (4.0 g, 50%). LCMS: C₁₃H₈F₂N₂O₄ requires: 294, found: m/z = 295 [M+H]⁺.

Step 2 product: tert-butyl 4-[4-[2-(2,6-dioxopiperidin-3-yl)-6-fluoro-1,3-dioxo-2,3-dihydro-1H-isoindol-5-yl]piperazin-1-yl]butanoate (3.3 g, 99%). LCMS: C₂₅H₃₁FN₄O₆ requires: 502, found: m/z = 503 [M+H]⁺.

Step 3 product: 4-(4-(2-(2,6-dioxopiperidin-3-yl)-6-fluoro-1,3-dioxoisoindolin-5-yl)piperazin-1-yl)butanoic acid TFA salt (1.4516 g, 38% over 2 steps). LCMS: C₂₁H₂₃FN₄O₆ requires: 446, found: m/z = 447 [M+H]⁺.

### Example SH: Synthesis of 2-(4-(2-(2-(2,6-dioxopiperidin-3-yl)-1-oxoisoindolin-4-yl)ethyl)piperazin-1-yl)acetic acid.

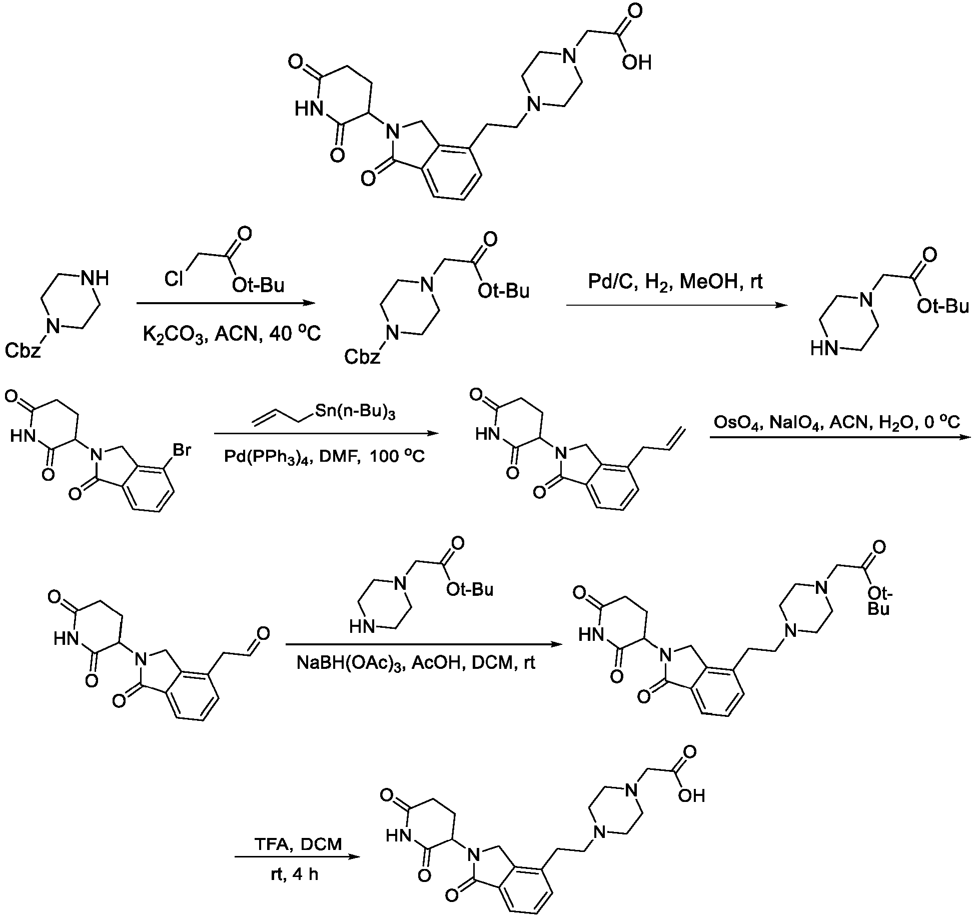

### Step-1: Synthesis of benzyl 4-(2-(tert-butoxy)-2-oxoethyl)piperazine-1-carboxylate.

To a solution of benzyl piperazine-1-carboxylate (10.0 g, 45.4 mmol) and K₂CO₃ (12.6 g, 90.8 mmol) in acetonitrile (150 mL) was added tert-butyl 2-chloroacetate (7.5 g, 49.9 mmol). The resulting solution was stirred at 40 °C for 16 h under nitrogen atmosphere. The solids were filtered out and the filtrate was concentrated under vacuum. The residue was purified by flash column chromatography with 0~50% ethyl acetate in petroleum ether to afford benzyl 4-(2-(tert-butoxy)-2-oxoethyl)piperazine-1-carboxylate (9.6 g, 63%) as a light yellow oil. MS (ESI) calculated for (C₁₈H₂₆N₂O₄) [M+H]⁺, 335.2; found, 335.3.

### Step-2: Synthesis of tert-butyl 2-(piperazin-1-yl)acetate.

To a solution of benzyl 4-(2-(tert-butoxy)-2-oxoethyl)piperazine-1-carboxylate (9.6 g, 28.7 mmol) in methanol (100 mL) was added Pd/C (10%, 2.0 g) under nitrogen atmosphere. The mixture was stirred at room temperature for 16 h under hydrogen atmosphere (2 atm). The solids were filtered out and the filtrate was concentrated under vacuum to afford tert-butyl 2-(piperazin-1-yl)acetate (6.2 g, crude) as a light yellow oil, which was used in the next step without further purification. MS (ESI) calculated for (C₁₀H₂₀N₂O₂) [M+H]⁺, 201.2; found, 201.0.

### Step-3: Synthesis of 3-(4-allyl-1-oxoisoindolin-2-yl)piperidine-2,6-dione.

A degassed mixture of 3-(4-bromo-1-oxo-2,3-dihydro-1H-isoindol-2-yl)piperidine-2,6-dione (10.0 g, 30.9 mmol), allyltributylstannane (15.4 g, 46.4 mmol) and Pd(PPh₃)₄ (3.6 g, 3.1 mmol) in DMF (80 mL) was stirred at 100 °C for 16 h under nitrogen atmosphere. When the reaction was completed by LCMS, the resulting mixture was diluted with water and extracted with ethyl acetate. The combined organic layer was washed with brine, dried over anhydrous Na₂SO₄, filtered and concentrated under vacuum. The residue was purified by flash column chromatography with 0~10% methanol in dichloromethane to afford 3-(4-allyl-1-oxoisoindolin-2-yl)piperidine-2,6-dione (7.0 g, 79%) as a white solid. MS (ESI) calculated for (C₁₆H₁₆N₂O₃) [M+H]⁺, 285.1; found, 285.2. ¹H NMR (400 MHz, DMSO-*d*₆) δ 10.99 (s, 1H), 7.62 - 7.60 (m, 1H), 7.52 - 7.27 (m, 2H), 6.02 - 5.92 (m, 1H), 5.16 - 5.09 (m, 3H), 4.45 (d, *J =* 17.2 Hz, 1H), 4.30 *(d, J=* 17.2 Hz, 1H), 3.46 - 3.44 (m, 2H), 2.97 - 2.86 (m, 1H), 2.70 - 2.57 (m, 1H), 2.04 - 1.99 (m, 1H), 1.68 - 1.55 (m, 1H).

### Step-4: Synthesis of 2-(2-(2,6-dioxopiperidin-3-yl)-1-oxoisoindolin-4-yl)acetaldehyde.

A mixture of 3-(4-allyl-1-oxoisoindolin-2-yl)piperidine-2,6-dione (7.0 g, 24.6 mmol), OsO₄ (625 mg, 2.5 mmol) and NaIO₄ (10.5 g, 49.2 mmol) in MeCN (60 mL) and H₂O (20 mL) was stirred at 0 °C for 6 h. when the reaction was completed, the resulting mixture was diluted with water and extracted with ethyl acetate. The combined organic layer was washed with brine, dried over anhydrous Na₂SO₄, filtered and concentrated under vacuum to afford 2-(2-(2,6-dioxopiperidin-3-yl)-1-oxoisoindolin-4-yl)acetaldehyde (4.0 g, crude) as a brown solid, which was used in the next step without further purification. MS (ESI) calculated for (C₁₅H₁₄N₂O₄) [M+H]⁺, 287.1; found, 287.2.

### Step-5: Synthesis of tert-butyl 2-(4-(2-(2-(2,6-dioxopiperidin-3-yl)-1-oxoisoindolin-4-yl)ethyl)piperazin-1-yl)acetate.

A mixture of 2-[2-(2,6-dioxopiperidin-3-yl)-1-oxo-2,3-dihydro-1H-isoindol-4-yl]acetaldehyde (4.0 g, 13.9 mmol), tert-butyl 2-(piperazin-1-yl)acetate(3.4 g, 16.8 mmol), AcOH (1 mL) and NaBH(OAc)₃ (5.9 g, 27.9 mmol) in dichloromethane (50 mL) was stirred at room temperature for 16 h. The resulting mixture was diluted with water and extracted with ethyl acetate. The combined organic layer was washed with brine, dried over anhydrous Na₂SO₄, filtered and concentrated under vacuum. The crude residue was purified by reverse phase flash column chromatography with 10~50% acetonitrile in water to afford tert-butyl 2-(4-(2-(2-(2,6-dioxopiperidin-3-yl)-1-oxoisoindolin-4-yl)ethyl)piperazin-1-yl)acetate (2.5 g, 22% over two steps) as a light brown syrup. MS (ESI) calculated for (C₂₅H₃₄N₄O₅) [M+H]⁺, 471.2; found, 471.0.

### Step-6: Synthesis of 2-(4-(2-(2-(2,6-dioxopiperidin-3-yl)-1-oxoisoindolin-4-yl)ethyl)piperazin-1-yl)acetic acid TFA salt.

To a solution of tert-butyl 2-(4-(2-(2-(2,6-dioxopiperidin-3-yl)-1-oxoisoindolin-4-yl)ethyl)piperazin-1-yl)acetate (2.5 g, 5.3 mmol) in dichloromethane (20 mL) was added trifluoroacetic acid (20 mL). The resulting mixture was stirred at room temperature for 16 h before concentrated under vacuum. The residue was purified by reverse phase flash column chromatography with 5~30% acetonitrile in water to afford 2-(4-(2-(2-(2,6-dioxopiperidin-3-yl)-1-oxoisoindolin-4-yl)ethyl)piperazin-1-yl)acetic acid (1.7214 g, 78%) as a light brown solid. MS (ESI) calculated for (C₂₁H₂₆N₄O₅) [M+H]⁺, 415.2; found, 415.4. ¹H NMR (300 MHz, DMSO-*d₆*) δ 11.08 (s, 1H), 7.66 - 7.62 (m, 1H), 7.54 - 7.47 (m, 2H), 5.14 - 5.08 (m, 1H), 4.54 - 4.48 (m, 1H), 4.40 - 4.31 (m, 1H), 3.76 (s, 2H), 3.60 - 3.10 (m, 10H), 3.10 - 2.78 (m, 3H), 2.68 - 2.54 (m, 1H), 2.40 - 2.31 (m, 1H), 2.10 - 1.94 (m, 1H).

Table 4: Bifunctional compounds generated according to Route 1 of Scheme 6 and using the procedures in Examples 1 and 2.

| **Cmpd No.** | **Characterization data** |
|---|---|
| 25 | ¹H NMR (500 MHz, DMSO-*d₆*) δ 11.02 (s, 1H), 8.70 (s, 1H), 8.42 (d, *J =* 5.0 Hz, 1H), 7.74 (s, 1H), 7.57 - 7.40 (m, 3H), 7.29 (d, *J =* 5.0 Hz, 1H), 7.23 (d, *J =* 9.1 Hz, 1H), 7.08 (d, *J =* 8.6 Hz, 1H), 6.97 (dd, *J =*7.0*,* 1.6 Hz, 1H), 6.50 (d, *J =* 14.4 Hz, 2H), 4.99 (ddd, *J =* 12.8, 5.5, 2.9 Hz, 1H), 4.36 (d, *J =* 12.7 Hz, 2H), 4.13 *(d, J=* 11.9 Hz, 4H), 3.96 - 3.71 (m, 2H), 3.61 (d, *J =* 32.5 Hz, 1H), 3.54 (s, 3H), 3.47 - 3.18 (m, 7H), 3.04 (s, 1H), 2.96 - 2.71 (m, 1H), 2.63 - 2.48 (m, 2H), 2.39 - 2.18 (m, 11H), 1.95 (q, *J =* 8.7, 7.9 Hz, 4H), 1.15 (s, 6H), 0.75 (dd, *J* = 19.4, 5.9 Hz, 3H). |
| | LCMS: C₅₄H₅₉N₁₁O₉ requires: 1005, found: m/z = 1006 [M+H]⁺. |
| 26 | ¹H NMR (500 MHz, DMSO-*d₆*) δ 10.97 (s, 1H), 8.51 (s, 2H), 8.42 (d, *J =* 5.0 Hz, 1H), 7.78 (d, *J* = 8.4 Hz, 1H), 7.58 (dd, *J* = 6.3, 2.2 Hz, 1H), 7.50 - 7.41 (m, 3H), 7.36 (d, *J =* 7.9 Hz, 1H), 7.27 (d, *J =* 5.1 Hz, 1H), 7.22 (d, *J =* 8.9 Hz, 1H), 6.48 (d, *J =* 1.9 Hz, 1H), 5.10 (dd, *J =* 13.2, 5.2 Hz, 1H), 4.53 - 4.28 (m, 4H), 4.13 (q, *J =* 7.3, 4.7 Hz, 3H), 3.70 (d, *J =* 60.8 Hz, 2H), 3.53 (s, 3H), 3.45 - 2.74 (m, 3H), 2.53 (dd, *J =* 25.3, 10.9 Hz, 2H), 2.37 - 2.21 (m, 3H), 2.04 - 1.93 (m, 1H), 1.15 (s, 7H), 0.80 (dd, *J =* 31.4, 6.2 Hz, 3H). |
| | LCMS: C₅₅H₆₄N₁₂O₇ requires: 1005, found: m/z = 1006 [M+H]⁺. |
| 27 | ¹H NMR (500 MHz, DMSO-*d₆*) δ 10.95 (s, 1H), 8.49 (s, 1H), 8.42 (d, *J =* 5.0 Hz, 1H), 7.81 - 7.74 (m, 1H), 7.63 (d, *J =* 7.3 Hz, 1H), 7.58 - 7.35 (m, 5H), 7.28 *(d, J= 5.0* Hz, 1H), 7.22 (dd, *J* = 9.1, 1.6 Hz, 1H), 6.48 (s, 1H), 5.09 (dd, *J =* 13.2, 5.2 Hz, 1H), 4.61 - 3.93 (m, 8H), 3.93 - 3.61 (m, 4H), 3.54 (s, 3H), 3.31 (d, *J =* 13.4 Hz, 3H), 3.20 - 2.78 (m, 3H), 2.62 - 2.48 (m, 3H), 2.38 - 2.22 (m, 15H), 2.02 - 1.90 (m, 1H), 1.15 (s, 6H), 0.80 (dd, *J=* 25.1, 6.2 Hz, 4H). |
| | LCMS: C₅₄H₆₂N₁₂O₇ requires: 990, found: m/z = 991 [M+H]⁺. |
| 28 | ¹H NMR (500 MHz, DMSO-*d₆*) δ 11.12 (s, 1H), 8.53 - 8.42 (m, 2H), 7.85 - 7.51 (m, 5H), 7.35 (dd, *J =* 27.4, 6.9 Hz, 2H), 6.57 (s, 1H), 5.13 (ddd, *J =* 12.9, 5.5, 2.0 Hz, 1H), 4.44 (d, *J =* 11.8 Hz, 2H), 4.21 (d, *J =* 11.1 Hz, 3H), 3.90 - 3.69 (m, 2H), 3.62 (s, 7H), 3.42 (td, *J =* 6.5, 2.6 Hz, 2H), 3.08 (t, *J =* 7.7 Hz, 2H), 2.66 - 2.55 (m, 3H), 2.11 - 2.00 (m, 1H), 1.84 (q, *J =* 7.0 Hz, 2H), 1.23 (s, 9H), 0.86 (dd, *J =* 29.8, 6.1 Hz, 3H). |
| | LCMS: C₄₉H₄₉FN₁₀O₉ requires: 978, found: m/z = 979 [M+H]⁺. |
| 29 | ¹H NMR (500 MHz, DMSO-*d₆*) δ 10.92 (s, 1H), 8.42 (t, *J =* 9.1 Hz, 2H), 7.74 (s, 1H), 7.50 (dd, *J =* 5.2, 3.4 Hz, 1H), 7.43 - 7.36 (m, 2H), 7.27 (dd, *J =* 26.3, 6.6 Hz, 3H), 6.49 (d, *J =* 2.2 Hz, 1H), 5.07 (dd, *J =* 13.3, 5.1 Hz, 1H), 4.38 (dd, *J =* 18.8, 14.1 Hz, 3H), 4.18 (dd, *J =* 52.8, 13.7 Hz, 6H), 3.96 - 3.57 (m, 4H), 3.54 (d, *J=* 2.4 Hz, 3H), 3.46 - 3.26 (m, 1H), 3.22 (dt, *J =* 6.3, 3.3 Hz, 2H), 3.06 (s, 1H), 2.95 - 2.76 (m, 3H), 2.68 - 2.47 (m, 7H), 2.38 - 2.19 (m, 3H), 1.94 (ddt, *J* = 14.1, 10.7, 7.6 Hz, 3H), 1.83 - 1.64 (m, 3H), 1.15 (s, 6H), 0.83 - 0.61 (m, 4H). |
| | LCMS: C₅₅H₆₂N₁₀O₈ requires: 990, found: m/z = 991 [M+H]⁺. |
| 30 | ¹H NMR (500 MHz, DMSO-*d₆*) δ 10.95 (s, 1H), 8.51 (s, 1H), 8.42 (d, *J =* 5.0 Hz, 1H), 7.81 - 7.75 (m, 1H), 7.55 (dd, *J =* 5.1, 3.5 Hz, 1H), 7.47 - 7.41 (m, 3H), 7.38 (s, 0H), 7.28 (d, *J =* 5.0 Hz, 1H), 7.22 (d, *J =* 9.0 Hz, 1H), 6.48 (d, *J =* 2.8 Hz, 1H), 5.10 (dd, *J =* 13.2, 5.1 Hz, 1H), 4.46 - 4.30 (m, 2H), 4.30 - 4.03 (m, 4H), 3.89 - 3.58 (m, 2H), 3.53 (s, 3H), 3.42 - 2.79 (m, 4H), 2.68 - 2.48 (m, 4H), 2.37 - 2.25 (m, 4H), 1.93 (d, *J=* 29.3 Hz, 3H), 1.15 (s, 6H), 0.79 (dd, *J =* 32.3, 6.3 Hz, 3H). |
| | LCMS: C₅₆H₆₆N₁₂O₇ requires: 1019, found: m/z = 1020 [M+H]⁺. |
| 31 | ¹H NMR (500 MHz, DMSO-*d₆*) δ 11.02 (s, 1H), 8.69 (s, 1H), 8.46 - 8.40 (m, 2H), 7.76 (s, 1H), 7.51 (dd, *J =* 8.5, 7.0 Hz, 2H), 7.29 (d, *J =* 5.0 Hz, 1H), 7.24 (d, *J=* 9.0 Hz, 1H), 7.15 (d, *J =* 8.7 Hz, 1H), 6.97 (d, *J =* 7.0 Hz, 1H), 6.49 (s, 1H), 6.09 (s, 1H), 4.98 (dd, *J =* 12.8, 5.5 Hz, 1H), 4.36 (d, *J =* 13.1 Hz, 2H), 4.13 (d, *J =* 12.1 Hz, 3H), 3.88 - 3.57 (m, 3H), 3.54 (s, 3H), 2.81 (ddd, *J =* 16.8, 13.8, 5.5 Hz, 1H), 2.61 - 2.45 (m, 3H), 1.97 (tt, *J =* 12.5, 8.3 Hz, 4H), 1.78 - 1.19 (m, 5H), 1.15 (s, 9H), 0.79 (dd, *J =* 44.8, 6.1 Hz, 3H). |
| | LCMS: C₅₄H₅₉N₁₁O₈ requires: 989, found: m/z = 990 [M+H]⁺. |
| 32 | ¹H NMR (500 MHz, DMSO-*d₆*) δ 11.06 (s, 1H), 8.52 (d, *J =* 19.6 Hz, 2H), 8.42 (d, *J=* 5.0 Hz, 1H), 7.80 - 7.72 (m, 3H), 7.67 (dd, *J =* 6.4, 2.3 Hz, 1H), 7.45 (t, *J* = 2.8 Hz, 1H), 7.38 (s, 1H), 7.28 (d, *J =* 5.0 Hz, 1H), 7.22 (d, *J =* 8.9 Hz, 1H), 6.48 *(d, J=* 1.8 Hz, 1H), 5.07 (dd, *J =* 12.8, 5.5 Hz, 1H), 4.43 - 4.01 (m, 6H), 3.88 - 3.60 (m, 3H), 3.54 (d, *J =* 2.0 Hz, 3H), 3.42 - 3.15 (m, 2H), 3.15 - 2.70 (m, 5H), 2.60 - 2.45 (m, 3H), 2.35 (s, 7H), 2.04 - 1.83 (m, 4H), 1.15 (s, 8H), 0.79 (dd, *J=* 32.2, 6.2 Hz, 4H). |
| | LCMS: C₅₆H₆₄N₁₂O₈ requires: 1033, found: m/z = 1034 [M+H]⁺. |
| 33 | ¹H NMR (500 MHz, DMSO-*d₆*) δ 10.93 (s, 1H), 8.68 (s, 1H), 8.43 (d, *J =* 5.0 Hz, 1H), 7.75 (s, 1H), 7.55 - 7.34 (m, 5H), 7.29 (d, *J =* 5.0 Hz, 1H), 7.23 (d, *J =* 9.1 Hz, 1H), 6.49 (s, 1H), 5.07 (dd, *J =* 13.3, 5.1 Hz, 1H), 4.43 - 4.03 (m, 10H), 3.77 (d, *J =* 10.2 Hz, 1H), 3.65 (s, 1H), 3.54 (s, 4H), 3.04 (d, *J =* 14.9 Hz, 1H), 2.85 (ddd, *J =* 18.3, 13.5, 5.4 Hz, 1H), 2.69 - 2.49 (m, 6H), 2.38 - 2.23 (m, 4H), 2.02 - 1.88 (m, 1H), 1.86 - 1.71 (m, 3H), 1.15 (s, 6H), 0.77 (dt, *J =* 12.7, 4.8 Hz, 4H). |
| | LCMS: C₅₁H₅₆N₁₀O₇ requires: 920, found: m/z = 921 [M+H]⁺. |
| 34 | ¹H NMR (500 MHz, DMSO-*d₆*) δ 11.02 (s, 1H), 8.60 (s, 1H), 8.47 (s, 1H), 8.42 (d, *J =* 5.0 Hz, 1H), 7.75 (s, 1H), 7.55 - 7.36 (m, 4H), 7.29 (d, *J =* 5.1 Hz, 1H), 7.22 (d, *J=* 9.2 Hz, 1H), 6.99 (d, *J=* 7.0 Hz, 1H), 6.93 (d, *J=* 8.5 Hz, 1H), 6.49 (s, 1H), 6.34 (d, *J =* 6.5 Hz, 1H), 4.98 (dd, *J =* 12.8, 5.5 Hz, 1H), 4.36 (d, *J =* 13.7 Hz, 2H), 4.12 (t, *J =* 7.9 Hz, 4H), 3.98 - 3.88 (m, 1H), 3.77 (d, *J =* 10.3 Hz, 1H), 3.54 (s, 3H), 3.23 (dt, *J =* 28.9, 8.5 Hz, 1H), 3.03 (s, 1H), 2.81 (ddd, *J =* 17.0, 13.8, 5.4 Hz, 1H), 2.62 - 2.47 (m, 2H), 2.37 - 2.32 (m, 40H), 2.27 - 1.77 (m, 7H), 1.15 (s, 6H), 0.82 - 0.69 (m, 4H). |
| | LCMS: C₅₅H₅₉N₁₁O₈ requires: 1001, found: m/z = 1002 [M+H]⁺. |
| 35 | ¹H NMR (500 MHz, DMSO-*d₆*) δ 11.03 (s, 1H), 8.72 (s, 1H), 8.43 (d, *J =* 5.0 Hz, 1H), 7.77 (d, *J =* 10.3 Hz, 1H), 7.59 - 7.46 (m, 3H), 7.30 (d, *J =* 5.0 Hz, 1H), 7.25 (d, *J = 9.1* Hz, 1H), 7.11 - 6.96 (m, 3H), 6.49 (s, 1H), 5.01 (dd, *J =* 12.8, 5.4 Hz, 1H), 4.45 - 4.04 (m, 8H), 3.75 (d, *J =* 24.5 Hz, 2H), 3.54 (s, 3H), 3.24 - 2.75 (m, 3H), 2.50 (dd, *J =* 10.9, 3.3 Hz, 2H), 2.36 (s, 2H), 1.97 (dp, *J =* 11.9, 4.3, 3.7 Hz, 1H), 1.15 (s, 6H), 0.83 (dd, *J =* 35.0, 6.3 Hz, 3H). |
| | LCMS: C₄₉H₅₁N₁₁O₈ requires: 921, found: m/z = 922 [M+H]⁺. |
| 36 | ¹H NMR (500 MHz, DMSO-*d₆*) δ 11.01 (s, 1H), 8.42 (d, *J =* 5.0 Hz, 1H), 7.74 (s, 1H), 7.53 (dd, *J =* 8.6, 7.0 Hz, 1H), 7.29 (d, *J =* 5.1 Hz, 1H), 7.23 (d, *J =* 8.7 Hz, 1H), 7.09 (t, *J =* 8.6 Hz, 1H), 6.96 (d, *J=* 7.0 Hz, 1H), 6.71 (s, 1H), 6.49 (s, 1H), 4.97 (dd, *J =* 12.8, 5.5 Hz, 1H), 4.36 (d, *J =* 13.1 Hz, 2H), 4.13 (d, *J =* 9.2 Hz, 3H), 3.70 (d, *J =* 65.5 Hz, 6H), 3.52 (d, *J =* 14.3 Hz, 7H), 2.88 - 2.46 (m, 3H), 2.36 - 2.27 (m, 21H), 1.92 (s, 0H), 1.15 (s, 9H), 0.82 - 0.66 (m, 4H). |
| | LCMS: C₅₀H₅₃N₁₁O₈ requires: 935, found: m/z = 936 [M+H]⁺. |
| 37 | ¹H NMR (500 MHz, DMSO-*d₆*) δ 11.02 (s, 1H), 8.67 (s, 1H), 8.43 (d, *J =* 5.0 Hz, 1H), 7.75 (s, 1H), 7.56 - 7.40 (m, 3H), 7.29 (d, *J =* 5.1 Hz, 1H), 7.23 (d, *J =* 9.1 Hz, 1H), 7.12 (d, *J =* 8.6 Hz, 1H), 6.95 (d, *J =* 7.0 Hz, 1H), 6.61 (s, 1H), 6.49 (s, 1H), 4.98 (dd, *J =* 12.7, 5.5 Hz, 1H), 4.36 (d, *J =* 12.9 Hz, 2H), 4.14 (d, *J =* 8.5 Hz, 3H), 3.82 - 3.59 (m, 3H), 3.54 (s, 3H), 3.27 (s, 2H), 2.81 (ddd, *J =* 16.8, 13.7, 5.4 Hz, 1H), 2.58 - 2.47 (m, 3H), 2.36 (s, 2H), 2.02 - 1.90 (m, 1H), 1.75 (d, *J =* 7.5 Hz, 3H), 1.15 (s, 9H), 0.77 (dd, *J =* 16.2, 6.3 Hz, 3H). |
| | LCMS: C₅₁H₅₅N₁₁O₈ requires: 949, found: m/z = 950 [M+H]⁺. |
| 38 | ¹H NMR (500 MHz, DMSO-*d₆*) δ 10.92 (s, 1H), 8.71 (s, 1H), 8.43 (d, *J =* 5.0 Hz, 1H), 7.74 (s, 1H), 7.56 - 7.42 (m, 2H), 7.39 (d, *J =* 5.3 Hz, 2H), 7.26 (dd, *J* = 28.5, 7.1 Hz, 2H), 6.49 (s, 1H), 5.07 (dd, *J =* 13.3, 5.1 Hz, 1H), 4.46 - 3.95 (m, 9H), 3.81 - 3.58 (m, 3H), 3.54 (s, 4H), 3.04 (s, 1H), 2.86 (ddd, *J =* 17.5, 13.6, 5.4 Hz, 1H), 2.55 (dd, *J =* 47.4, 10.0 Hz, 4H), 2.38 - 2.20 (m, 4H), 1.99 - 1.90 (m, 1H), 1.63 - 1.38 (m, 6H), 1.15 (s, 7H), 0.83 - 0.64 (m, 4H). |
| | LCMS: C₅₂H₅₈N₁₀O₇ requires: 934, found: m/z = 935 [M+H]⁺. |
| 42 | ¹H NMR (500 MHz, Methanol-*d₄*) δ 8.51 (d, *J =* 5.1 Hz, 1H), 8.26 - 8.05 (m, 1H), 7.93 - 7.72 (m, 2H), 7.68 - 7.50 (m, 2H), 7.44 (d, *J =* 5.0 Hz, 1H), 7.25 (dd, *J =* 9.3*,* 4.8 Hz, 1H), 7.07 (ddd, *J =* 16.7, 7.9, 3.2 Hz, 2H), 6.71 (s, 1H), 5.05 (dd, *J =* 12.8, 5.5 Hz, 1H), 4.58 (s, 2H), 4.24 (t, *J =* 5.6 Hz, 2H), 3.86 - 3.61 (m, 18H), 3.50 (t, *J =* 5.0 Hz, 3H), 3.18 (s, 5H), 2.91 - 2.62 (m, 6H), 2.60 (s, 2H), 2.48 (s, 2H), 1.25 (s, 6H), 1.04 - 0.91 (m, 3H). |
| | LCMS: C₆₂H₇₇N₁₃O₁₁ requires: 1180, found: m/z = 1181 [M+H]⁺. |
| 43 | ¹H NMR (500 MHz, DMSO-*d₆*) δ 10.92 (s, 1H), 8.56 (d, *J =* 2.3 Hz, 1H), 8.44 - 8.37 (m, 2H), 7.77 (t, *J =* 3.4 Hz, 1H), 7.49 (dt, *J =* 7.8, 4.1 Hz, 1H), 7.39 (dt, *J* = 11.6, 4.3 Hz, 3H), 7.33 - 7.25 (m, 2H), 7.19 (d, *J =* 8.9 Hz, 1H), 6.49 (s, 1H), 5.07 (dd, *J =* 13.3, 5.1 Hz, 1H), 4.89 (t, *J =* 5.2 Hz, 1H), 4.45 - 4.06 (m, 7H), 3.74 (dd, *J =* 31.3, 11.3 Hz, 2H), 3.54 (d, *J =* 5.5 Hz, 7H), 3.37 - 3.28 (m, 2H), 3.07 - 2.76 (m, 2H), 2.56 (dt, *J=* 52.7, 10.0 Hz, 7H), 2.36 (s, 2H), 1.93 (d, *J=* 12.1 Hz, 1H), 1.82 - 1.70 (m, 2H), 1.19 - 1.12 (m, 7H), 0.75 (dd, *J =* 29.8, 6.3 Hz, 3H). |
| | LCMS: C₅₃H₆₀N₁₀O₈ requires: 964, found: m/z = 965 [M+H]⁺. |
| 49 | 1H NMR (500 MHz, DMSO-d6) δ 10.92 (s, 1H), 8.83 (s, 2H), 8.47 - 8.33 (m, 3H), 7.73 (s, 2H), 7.61 - 7.47 (m, 4H), 7.38 (dd, J = 5.4, 3.4 Hz, 2H), 7.33 - 7.20 (m, 3H), 6.49 (s, 1H), 5.06 (dd, J = 13.3, 5.1 Hz, 1H), 4.37 (ddd, J = 17.3, 13.2, 6.0 Hz, 4H), 4.18 (ddd, J = 47.3, 13.1, 3.7 Hz, 6H), 3.89 - 3.63 (m, 4H), 3.56 (d, J = 20.3 Hz, 7H), 3.31 (q, J = 7.0 Hz, 3H), 3.16 - 2.77 (m, 3H), 2.66 - 2.46 (m, 8H), 2.35 (s, 4H), 2.00 - 1.88 (m, 2H), 1.76 (td, J = 7.6, 7.0, 3.3 Hz, 3H), 1.15 (s, 9H), 0.78 (dd, J = 24.3, 6.3 Hz, 4H). |
| | LCMS: C₅₇H₆₈N₁₀O₁₀ requires: 1053, found: m/z = 1054 [M+H]⁺. |
| 50 | ¹H NMR (500 MHz, DMSO-*d₆*) δ 11.02 (s, 1H), 8.42 (d, *J =* 5.0 Hz, 1H), 7.74 (s, 1H), 7.50 (p, *J =* 8.0, 7.0 Hz, 3H), 7.29 (d, *J =* 5.1 Hz, 1H), 7.24 (d, *J=* 9.1 Hz, 1H), 7.07 (dd, *J =* 8.6*,* 1.7 Hz, 1H), 6.96 (d, *J =* 7.1 Hz, 1H), 6.49 (s, 2H), 4.98 (dd, *J =* 12.7, 5.4 Hz, 1H), 4.57 - 3.98 (m, 6H), 3.99 - 3.63 (m, 4H), 3.61 - 3.43 (m, 10H), 3.14 - 2.74 (m, 2H), 2.68 - 2.46 (m, 6H), 2.35 (s, 2H), 2.00 - 1.90 (m, 1H), 1.15 (s, 6H), 0.78 (dd, *J =* 27.1, 6.3 Hz, 3H). |
| | LCMS: C₆₀H₇₃N₁₁O₁₃ requires: 1156, found: m/z = 1157 [M+H]⁺. |
| 51 | ¹H NMR (500 MHz, DMSO-*d₆*) δ 11.02 (s, 1H), 8.78 (s, 1H), 8.42 (d, *J=* 5.1 Hz, 1H), 7.73 (s, 1H), 7.62 - 7.42 (m, 2H), 7.29 (dd, *J =* 5.0, 1.7 Hz, 1H), 7.24 (d, *J =* 9.2 Hz, 1H), 7.06 (dd, *J =* 8.6, 3.8 Hz, 1H), 6.96 (d, *J =* 7.0 Hz, 1H), 6.49 (s, 2H), 4.98 (dd, *J =* 12.7, 5.4 Hz, 1H), 4.36 (d, *J =* 11.8 Hz, 2H), 4.13 (d, *J =* 11.4 Hz, 3H), 3.67 (d, *J =* 5.9 Hz, 1H), 3.61 - 3.44 (m, 8H), 3.15 - 2.67 (m, 1H), 2.68 - 2.47 (m, 4H), 2.35 (s, 13H), 1.95 (ddd, *J =* 15.1, 6.8, 4.3 Hz, 1H), 1.15 (s, 6H), 0.78 (dd, *J =* 26.8, 6.3 Hz, 3H). |
| | LCMS: C₅₈H₆₉N₁₁O₁₂ requires: 1112, found: m/z = 1113 [M+H]⁺. |
| 52 | ¹H NMR (500 MHz, DMSO-d6) δ 11.02 (s, 1H), 8.72 (s, 1H), 8.42 (d, *J =* 5.0 Hz, 1H), 7.73 (s, 1H), 7.57-7.40 (m, 2H), 7.29 (d, *J =* 5.0 Hz, 1H), 7.23 (d, *J =* 9.2 Hz, 1H), 7.06 (t, *J =* 8.3 Hz, 1H), 6.96 (dd, *J =* 7.0 Hz, 2.5 Hz, 1H), 6.49 (s, 2H), 4.98 (dd, *J =* 12.7 Hz, 5.5 Hz, 1H), 4.36 (d, *J =* 12.3 Hz, 2H), 4.21-4.06 (m, 3H), 3.82-3.60 (m, 2H), 3.51-3.39 (m, 11H), 3.16-2.73 (m, 2H), 2.65-2.48 (m, 4H), 2.35 (s, 2H), 1.95 (dd, *J =* 12.4, 5.9 Hz, 1H), 1.15 (s, 6H), 0.78 (dd, *J =* 25.9, 6.3 Hz, 3H). |
| | LCMS: C₅₆H₆₅N₁₁O₁₁ requires: 1067, found: m/z = 1068 [M+H]⁺. |
| 53 | ¹H NMR (500 MHz, Methanol-*d₄*) δ 8.64 (dd, J = 7.2, 2.3 Hz, 1H), 8.53 - 8.44 (m, 1H), 7.92 (dd, J = 9.1, 2.8 Hz, 1H), 7.62 (dt, J = 6.1, 2.2 Hz, 1H), 7.51 - 7.35 (m, 5H), 7.02 (d, J = 9.0 Hz, 1H), 6.71 (s, 1H), 5.17 - 5.11 (m, 1H), 4.66 (d, J = 11.9 Hz, 1H), 4.59 - 4.38 (m, 4H), 4.32 (d, J = 12.9 Hz, 1H), 4.23 (s, 2H), 3.93 (d, J = 12.5 Hz, 1H), 3.89 - 3.66 (m, 8H), 3.66 - 3.46 (m, 21H), 3.40 (dp, J = 9.1, 3.1 Hz, 3H), 3.10 (t, J = 5.1 Hz, 1H), 2.79 - 2.68 (m, 4H), 2.61 (dd, J = 14.9, 7.7 Hz, 3H), 2.47 (s, 3H), 1.89 (dt, J = 14.2, 6.9 Hz, 2H), 1.32 - 1.21 (m, 8H), 0.91 (dd, J = 17.3, 6.3 Hz, 4H). |
| | LCMS: C₆₁H₇₆N₁₀O₁₂ requires: 1141, found: m/z = 1142 [M+H]⁺. |
| 54 | ¹H NMR (500 MHz, Methanol-*d₄*) δ 8.62 (dd, J = 9.0, 2.3 Hz, 1H), 8.48 (dd, J = 5.1, 1.9 Hz, 1H), 7.91 (dd, J = 8.3, 2.9 Hz, 1H), 7.61 (ddd, J = 6.0, 4.1, 2.5 Hz, 1H), 7.53 - 7.36 (m, 5H), 7.02 (d, J = 8.9 Hz, 1H), 6.71 (s, 1H), 5.14 (dt, J = 13.4, 4.9 Hz, 1H), 4.66 (d, J = 12.1 Hz, 1H), 4.60 - 4.38 (m, 3H), 4.32 (d, J = 12.5 Hz, 1H), 4.27 - 4.15 (m, 2H), 3.93 (d, J = 12.6 Hz, 1H), 3.88 - 3.67 (m, 8H), 3.67 - 3.46 (m, 16H), 3.46 - 3.36 (m, 3H), 3.22 (q, J = 7.5 Hz, 1H), 2.95 - 2.82 (m, 1H), 2.74 (td, J = 14.5, 13.4, 6.2 Hz, 4H), 2.59 (d, J = 8.6 Hz, 3H), 2.47 (s, 3H), 1.87 (dt, J = 13.9, 7.0 Hz, 2H), 1.40 - 1.16 (m, 14H), 0.91 (dd, J = 14.9, 6.4 Hz, 4H). |
| | LCMS: C₅₉H₇₂N₁₀O₁₁ requires: 1097, found: m/z = 1098 [M+H]⁺. |
| 55 | ¹H NMR (500 MHz, DMSO-*d₆*) δ 11.02 (s, 1H), 8.58 - 8.53 (m, 1H), 8.42 (dd, *J* = 5.0, 1.7 Hz, 1H), 8.39 (d, *J =* 4.5 Hz, 1H), 7.74 (d, *J =* 19.6 Hz, 1H), 7.50 (q, *J* = 7.6 Hz, 1H), 7.40 (d, *J =* 2.3 Hz, 1H), 7.27 (p, *J =* 9.3 Hz, 2H), 7.17 (t, *J =* 9.2 Hz, 1H), 7.07 (dd, *J =* 8.8, 2.7 Hz, 1H), 6.96 (dd, *J =* 9.4, 7.0 Hz, 1H), 6.50 (d, *J* = 16.3 Hz, 2H), 4.97 (dd, *J =* 12.9, 5.4 Hz, 1H), 4.88 (d, *J =* 5.0 Hz, 1H), 4.37 (dd, *J=* 23.5, 9.5 Hz, 2H), 4.23 - 4.06 (m, 3H), 3.84 - 3.50 (m, 8H), 3.47 (s, 1H), 3.39 (d, *J =* 5.6 Hz, 2H), 2.97 (d, *J =* 11.5 Hz, 1H), 2.90 - 2.73 (m, 2H), 2.51 (d, *J=* 11.6 Hz, 1H), 2.36 (s, 2H), 1.98 - 1.89 (m, 0H), 1.15 (d, *J =* 3.3 Hz, 8H), 0.74 (dd, *J =* 27.8, 6.4 Hz, 3H). |
| | LCMS: C₅₂H₅₇N₁₁O₉ requires: 979, found: m/z = 980 [M+H]⁺. |
| 56 | ¹H NMR (500 MHz, DMSO-*d₆*) δ 11.02 (s, 1H), 9.60 (s, 1H), 8.59 (s, 1H), 8.48 (s, 1H), 8.42 (d, *J =* 5.0 Hz, 1H), 7.77 (s, 1H), 7.70 *(d, J=* 8.5 Hz, 1H), 7.51 - 7.36 (m, 3H), 7.33 - 7.26 (m, 2H), 7.23 (d, *J=* 9.1 Hz, 1H), 6.49 (s, 1H), 5.03 (dd, *J =* 12.8, 5.4 Hz, 1H), 4.36 (d, *J =* 11.4 Hz, 2H), 4.22 - 4.08 (m, 6H), 4.00 - 3.60 (m, 3H), 3.54 (s, 4H), 3.34 (s, 1H), 3.24 - 3.00 (m, 7H), 2.99 - 2.75 (m, 1H), 2.59 - 2.45 (m, 3H), 2.02 - 1.79 (m, 4H), 1.15 (s, 7H), 0.79 (dd, *J =* 27.1, 6.3 Hz, 3H). |
| | LCMS: C₅₅H₆₂N₁₂O₈ requires: 1018, found: m/z = 1019 [M+H]⁺. |
| 57 | ¹H NMR (500 MHz, DMSO-*d₆*) δ 10.95 (s, 1H), 9.74 (s, 1H), 8.71 (s, 1H), 8.42 (d, *J =* 5.0 Hz, 1H), 7.73 (d, *J =* 7.9 Hz, 2H), 7.52 (s, 1H), 7.43 (dt, *J =* 13.5, 7.1 Hz, 3H), 7.29 (d, *J =* 5.0 Hz, 1H), 7.23 (d, *J =* 9.1 Hz, 1H), 6.49 (s, 1H), 5.08 (dd, *J =* 13.4, 5.1 Hz, 1H), 4.44 - 4.01 (m, 11H), 3.83 - 3.73 (m, 1H), 3.70 - 3.57 (m, 5H), 3.54 (s, 3H), 3.03 (s, 1H), 2.85 (ddd, *J =* 18.3, 13.6, 5.5 Hz, 1H), 2.64 - 2.49 (m, 4H), 2.39 - 2.20 (m, 3H), 2.01 - 1.90 (m, 1H), 1.15 (s, 8H), 0.74 (dd, *J =* 35.9, 6.6 Hz, 3H). |
| | LCMS: C₅₃H₅₉N₁₁O₉ requires: 993, found: m/z = 994 [M+H]⁺. |
| 58 | ¹H NMR (500 MHz, DMSO-*d₆*) δ 11.05 (s, 1H), 9.60 (s, 1H), 8.49 (s, 1H), 8.42 (d, *J =* 5.1 Hz, 1H), 7.76 (d, *J =* 11.6 Hz, 2H), 7.54 (d, *J =* 7.4 Hz, 1H), 7.43 (dd, *J* = 32.4, 5.1 Hz, 2H), 7.28 (d, *J =* 5.0 Hz, 1H), 7.22 (dd, *J=* 9.1, 2.6 Hz, 1H), 6.49 (s, 1H), 5.06 (dd, *J =* 12.9, 5.4 Hz, 1H), 4.36 (d, *J=* 11.8 Hz, 2H), 4.26 - 3.86 (m, 6H), 3.70 (dd, *J =* 31.2, 12.7 Hz, 4H), 3.53 (s, 6H), 3.42 - 2.73 (m, 10H), 2.59 - 2.46 (m, 3H), 2.38 - 2.25 (m, 5H), 2.04 - 1.93 (m, 1H), 1.88 (d, *J =* 8.6 Hz, 3H), 1.15 (s, 6H), 0.79 (dd, *J =* 27.7, 6.2 Hz, 3H). |
| | LCMS: C₅₅H₆₁FN₁₂O₈ requires: 1037, found: m/z = 1038 [M+H]⁺. |

### Example 6: General procedure for the synthesis of urea compounds according to Route 4 of Scheme 11.

Phosgene in toluene (15%, 0.5 mL, 1.1 mmol) was added to a mixture of an amine (0.0518mmol), dichloromethane (1 mL), and DIEA (0.03 mL, 0.155 mmol). The mixture was allowed to stir at room temperature for 5 minutes. The volatiles were then removed and dichloromethane (1 mL) was added. The resulting solution was added to a mixture of 10-[3'-(hydroxymethyl)-1-methyl-5-({5-[(2S)-2-methylpiperazin-1-yl]pyridin-2-yl}amino)-6-oxo-[3,4'-bipyridin]-2'-yl]-4,4-dimethyl-1,10-diazatricyclo[6.4.0.0-{2,6}]dodeca-2(6),7-dien-9-one (E-2b) (20.7 mg, 0.0518 mmol) and DIEA (0.03 mL, 0.155 mmol) in dichloromethane (1 mL). The mixture was allowed to stir for 10 minutes. The volatiles were removed and the mixture was purified by HPLC (5-95% MeCN in H₂O with 0.1% TFA) to afford the desired product.

### Example 6A: General procedure for the synthesis of carbamate.

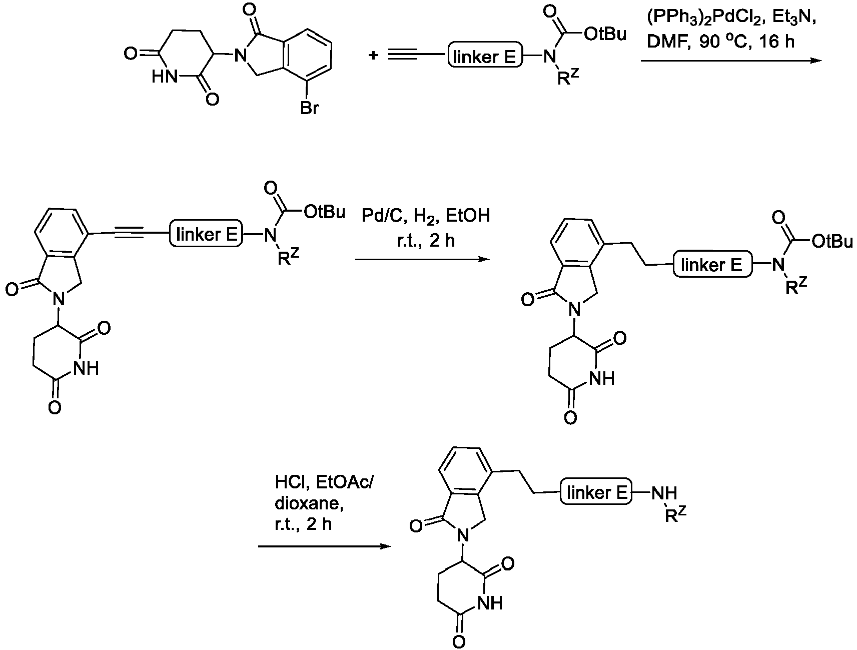

As used in this Example, "linker E" is -Y²-Y³-Y⁴-Y⁵-, wherein each of Y², Y³, Y⁴, and X⁵ are defined herein, and R^{Z} is -H or -C₁₋₄ alkyl.

Step 1: A mixture of 3-(4-bromo-1-oxo-2,3-dihydro-1H-isoindol-2-yl)piperidine-2,6-dione (2.52 mmol), (PPh₃)₂PdCl₂ (0.15 mmol),CuI (0.25 mmol) , alkyne (5.04 mmol) were added to a vial. The vial was evacuated and backfilled with N₂ 5 times. DMF and triethylamine (30.3 mmol) were added and the mixture was allowed to stir at 90°C overnight. The mixture was filtered through celite, washing with MeOH and EtOAc. EtOAc and saturated aqueous NaCl were added. The organic layer was dried with MgSO₄, filtered, concentrated and purified by reverse phase MPLC (5-100% MeCN in H₂O on C18 column) to afford the product.

Step 2: A mixture of disubstituted alkyne (0.81 mmol), Pd/C 10wt% (0.08 mmol) and EtOH were mixed in a flask. The flask was evacuated and backfilled with H₂ 5 times and allowed to stir at r.t. for 2 h. The mixture was filtered through celite washing with MeOH and EtOAc, concentrated and carried to the next step.

Step 3: A mixture of tert-butylcarbamate (0.81 mmol), CH₂Cl₂ (2 mL), and TFA (2 mL) was allowed to stir at r.t. for 2 h. The mixture was concentrated to afford the amine product.

### Example 6B: General procedure for the synthesis of carbamate intermediates.

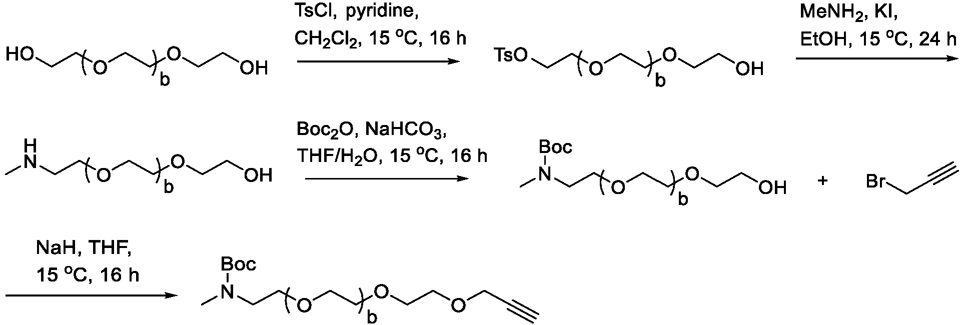

As used in this Example, "b" is an integer from 1 to 4.

Step 1: Pyridine (65.16 g, 823.78 mmol) was added into a mixture of diol (411.89 mmol) and TsCl (86.38 g, 453.08 mmol) in CH₂Cl₂ (1600 mL) at 15°C. The reaction mixture was stirred at this temperature for 24 h. The reaction was quenched by the addition of water (400 mL). The aqueous layer was extracted by DCM (100 mL*2). The combined organic layer was washed with 1N HCl (200 mL*2), brine(200ml*2), dried over Na₂SO₄, and concentrated under reduced pressure. The crude product was purified by column chromatography to afford the monotosylate product.

Step 2: The monotosylate product (14.35 mmol) and KI (2.87 mmol) were dissolved in MeNH₂ (50 mL, 30% purity) in EtOH at 15 °C. The reaction mixture was stirred at this temperature for 24 h. The reaction mixture was concentrated under reduced pressure. The crude methyl amine product was directly used in the next step without further purification.

Step 3: The methyl amine 100.45 mmol), NaHCO₃ (200.9 mmol) and Boc₂O (110.5 mmol) was dissolved in THF (200 mL) and H₂O (100 mL). The reaction mixture was stirred at 15 °C for 16 h. 100 mL of water was added into the reaction mixture. The reaction was extracted with EtOAc (100 mL*6), washed with brine (200 mL*2), dried over with Na₂SO₄, and concentrated under reduced pressure. The crude product was purified by column chromatography to afford the tert-butylcarbamate alcohol product (78.08 mmol, 77.73%).

Step 4: The tert-butylcarbamate alcohol (32.53 mmol) was dissolved in THF (65 mL). NaH (48.8 mmol,) was added slowly at 0° C. The reaction mixture was stirred at this temperature for 0.5 h. 3-bromoprop-1-yne (39.04 mmol) was added into the reaction mixture dropwise. The reaction mixture was warmed to 15°C, and stirred at 15°C for 16 h. The reaction was quenched by the addition of water (50 ml), and extracted with EtOAc (100 ml*3). The organic layer was washed with brine (100 ml), dried over with MgSO₄, and concentrated under reduced pressure. The crude product was purified by column chromatography to afford the alkyne (31.84 mmol, 97.88%).

The general methods of Examples 6A and 6B were used to synthesize the intermediates described in Examples 6B-1 and 6B-2.

### Example 6B-1: Synthesis of tert-butyl methyl(2-(2-(2-(prop-2-yn-1-yloxy)ethoxy)ethoxy)ethyl)carbamate.

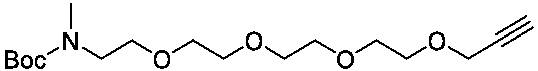

Step 1 product: 2-(2-(2-(2-hydroxyethoxy)ethoxy)ethoxy)ethyl 4-methylbenzenesulfonate (67 g, 53%). LCMS: C₁₅H₂₄O₇S requires: 348, found: m/z = 349 [M+H]⁺.

Step 2 product: 5,8,11-trioxa-2-azatridecan-13-ol (2.97 g, crude).

Step 3 product: tert-butyl N-[2-[2-[2-(2-hydroxyethoxy)ethoxy]ethoxy]ethyl]-N-methyl-carbamate (24 g, 78%).

### Example 6B-2: Synthesis of tert-butyl methyl(3,6,9,12,15-pentaoxaoctadec-17-yn-1-yl)carbamate.

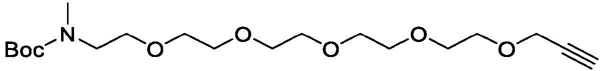

Step 1 product: 2-[2-[2-[2-(2-hydroxyethoxy)ethoxy]ethoxy]ethoxy]ethyl 4-methylbenzenesulfonate (21 g, 26%).

Step 2 product: 5,8,11,14-tetraoxa-2-azahexadecan-16-ol (15 g, crude).\

Step 3 product: tert-butyl N-[2-[2-[2-[2-(2-hydroxyethoxy)ethoxy]ethoxy]ethoxy]ethyl]-N-methyl-carbamate (16.4 g, 87.2%).

Step 4 product: tert-butyl N-methyl-N-[2-[2-[2-[2-(2-prop-2-ynoxyethoxy)ethoxy]ethoxy]ethoxy]ethyl]carbamate (17.2 g, 94.6%).

The general methods of Examples 6A and 6B were used to synthesize the intermediates described in Examples 6C-1 through 6C-5.

### Example 6C-1: Synthesis of 3-[4-[3-[2-[2-[2-[2-(methylamino)ethoxy]ethoxy]ethoxy]ethoxy]propyl]-1-oxo-isoindolin-2-yl]piperidine-2,6-dione.

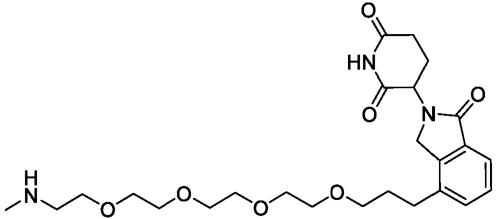

Step 1 product: tert-butyl N-[2-[2-[2-[2-[3-[2-(2,6-dioxo-3-piperidyl)-1-oxo-isoindolin-4-yl]prop-2-ynoxy]ethoxy]ethoxy]ethoxy]ethyl]-N-methyl-carbamate (2.5 g, 27%). LCMS: C₃₀H₄₁N₃O₉ requires: 587, found: m/z = 610 [M+Na]⁺.

Step 2 product: tert-butyl N-[2-[2-[2-[2-[3-[2-(2,6-dioxo-3-piperidyl)-1-oxo-isoindolin-4-yl]propoxy]ethoxy]ethoxy]ethoxy]ethyl]-Nmethyl-carbamate (2.2 g, 99%). LCMS: C₃₀H₄₅N₃O₉ requires: 591, found: m/z = 614 [M+Na]⁺.

Step 3 product: 3-[4-[3-[2-[2-[2-[2-(methylamino)ethoxy]ethoxy]ethoxy]ethoxy]propyl]-1-oxo-isoindolin-2-yl]piperidine-2,6-dione (1.73 g, 88%). LCMS: C₂₅H₃₇N₃O₇ requires: 491, found: m/z = 492 [M+H]⁺.

### Example 6C-2: Synthesis of 3-[4-[3-[2-[2-[2-[2-[2-(methylamino)ethoxy]ethoxy]ethoxy]ethoxy]ethoxy]propy]-1-oxo-isoindolin-2-yl]piperidine-2,6-dione.

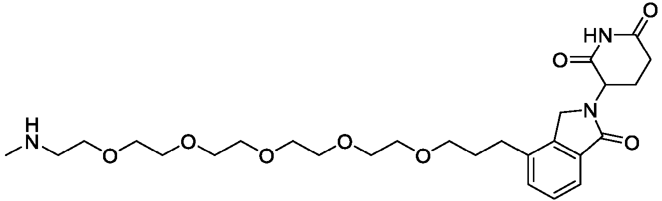

Step 1 product: tert-butyl N-[2-[2-[2-[2-[2-[3-[2-(2,6-dioxo-3-piperidyl)-1-oxo-isoindolin-4-yl]prop-2-ynoxy]ethoxy]ethoxy]ethoxy]ethoxy]ethyl]-N-methyl-carbamate (3.7 g, 31.54%). LCMS: C₃₂H₄₅N₃O₁₀ requires: 631, found: m/z = 654 [M+Na]⁺.

Step 2 product: tert-butyl N-[2-[2-[2-[2-[2-[3-[2-(2,6-dioxo-3-piperidyl)-1-oxo-isoindolin-4-yl]propoxy]ethoxy]ethoxy]ethoxy]ethoxy]ethyl]-N-methyl-carbamate (2.9 g, 99%). LCMS: C₃₂H₄₉N₃O₁₀ requires: 635, found: m/z = 636 [M+H]⁺.

Step 3 product: 3-[4-[3-[2-[2-[2-[2-[2-(methylamino)ethoxy]ethoxy]ethoxy]ethoxy]ethoxy]propyl]-1-oxo-isoindolin-2-yl]piperidine-2,6-dione (2.0 g, 73.65%). LCMS: C₂₇H₄₁N₃O₈ requires: 535, found: m/z = 536 [M+H]⁺.

### Example 6C-3: Synthesis of 3-[4-[3-[2-[2-(2-aminoethoxy)ethoxy]ethoxy]propy]-1-oxo-isoindolin-2-yl]piperidine-2,6-dione.

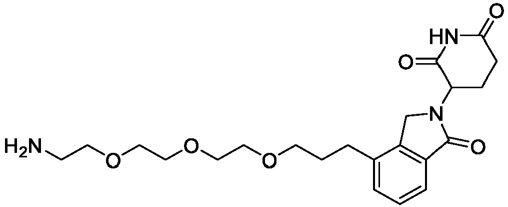

Step 1 product: tert-butyl N-[2-[2-[2-[3-[2-(2,6-dioxo-3-piperidyl)-1-oxo-isoindolin-4-yl]prop-2-ynoxy]ethoxy]ethoxy]ethyl]carbamate (1.45 g, 58.1%). LCMS: C₂₇H₃₅N₃O₈ requires: 529, found: m/z = 552 [M+Na]⁺.

Step 2 product: tert-butyl N-[2-[2-[2-[3-[2-(2,6-dioxo-3-piperidyl)-1-oxo-isoindolin-4-yl]propoxy]ethoxy]ethoxy]ethyl]carbamate (960 mg, 92.75%). LCMS: C₂₇H₃₉N₃O₈ requires: 533, found: m/z = 556 [M+Na]⁺.

Step 3 product: 3-[4-[3-[2-[2-(2-aminoethoxy)ethoxy]ethoxy]propyl]-1-oxo-isoindolin-2-yl]piperidine-2,6-dione (576.82 mg, 74.15%). LCMS: C₂₂H₃₁N₃O₆ requires: 433, found: m/z = 434 [M+H]⁺.

### Example 6C-4: Synthesis of 3-(4-(5-aminopentyl)-1-oxoisoindolin-2-yl)piperidine-2,6-dione.

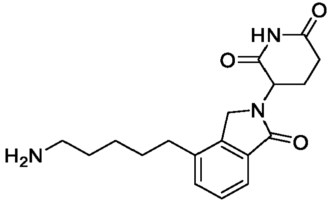

Step 1 product: tert-butyl N-[5-[2-(2,6-dioxo-3-piperidyl)-1-oxo-isoindolin-4-yl]pent-4-ynyl]carbamate (1.72 g, 85.3% yield). LCMS; C₂₃H₂₇N₃O₅ requires: 425, found: m/z = 448 [M+Na]⁺.

Step 2 product: tert-butyl N-[5-[2-(2,6-dioxo-3-piperidyl)-1-oxo-isoindolin-4-yl]pentyl]carbamate (1.09 g, 98.4%). LCMS; C₂₃H₃₁N₃O₅ requires: 429, found: m/z = 452 [M+Na]⁺.

Step 3 product: 3-[4-(5-aminopentyl)-1-oxo-isoindolin-2-yl]piperidine-2,6-dione (776 mg, 81.1%). LCMS; C₁₈H₂₃N₃O₃ requires: 329, found: m/z = 330 [M+H]⁺.

### Example 6C-5: Synthesis of 3-(4-(3-((6-aminohexyl)oxy)propyl)-1-oxoisoindolin-2-yl)piperidine-2,6-dione.

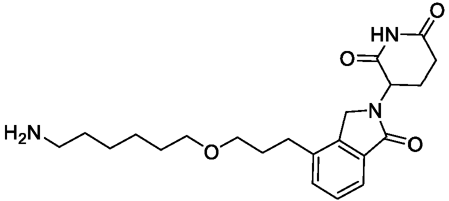

Step 1 product: tert-butyl (6-((3-(2-(2,6-dioxopiperidin-3-yl)-1-oxoisoindolin-4-yl)prop-2-yn-1-yl)oxy)hexyl)carbamate (211 mg, 61%). LCMS: C₂₇H₃₅N₃O₆ requires: 497, found: m/z = 498 [M+H]⁺.

Step 2 product: tert-butyl (6-(3-(2-(2,6-dioxopiperidin-3-yl)-1-oxoisoindolin-4-yl)propoxy)hexyl)carbamate (112 mg, 50%). LCMS: C₂₇H₃₉N₃O₆ requires: 501, found: m/z = 502 [M+H]⁺.

Step 3 product: 3-(4-(3-((6-aminohexyl)oxy)propyl)-1-oxoisoindolin-2-yl)piperidine-2,6-dione (89 mg, 99%). LCMS: C₂₂H₃₁N₃O₄ requires: 401, found: m/z = 402 [M+H]⁺.

The compounds provided in Table 3 were generated following the procedures set forth in Examples 1-3.

**Table 5: Bifunctional compounds generated according to Route 4 of Scheme 11 and using the procedures in Examples 4, 5A-1, and 6.**

| **Cmpd No.** | **Characterization data** |
|---|---|
| 44 | ¹H NMR (500 MHz, Methanol-*d₄*) δ 8.63 (d, *J =* 2.3 Hz, 1H), 8.48 (d, *J =* 5.1 Hz, 1H), 7.93 (d, *J = 2.7* Hz, 1H), 7.61 (dd, *J=* 6.3, 2.4 Hz, 1H), 7.50 - 7.36 (m, 4H), 7.01 (d, *J =* 8.9 Hz, 1H), 6.71 (s, 1H), 5.48 (s, 1H), 5.14 (dd, *J =* 13.3, 5.2 Hz, 1H), 4.66 (d, *J =* 11.9 Hz, 1H), 4.56 - 4.18 (m, 5H), 3.76 - 3.40 (m, 23H), 3.05 (s, 2H), 2.92 (s, 4H), 2.80 - 2.70 (m, 3H), 2.59 (d, *J =* 8.5 Hz, 2H), 2.47 (s, 3H), 2.19 - 2.06 (m, 1H), 1.96 - 1.78 (m, 2H), 1.40 - 1.19 (m, 11H), 0.94 (d, *J =* 6.3 Hz, 4H), 0.17 - -0.03 (m, 3H). |
| | LCMS: C₆₀H₇₅N₁₁O₁₁ requires: 1126, found: m/z = 1127 [M+H]⁺. |
| 45 | ¹H NMR (500 MHz, Methanol-*d4*) δ 8.64 (d, *J = 2.3* Hz, 1H), 8.48 (d, *J =* 5.1 Hz, 1H), 7.93 (d, *J = 2.9* Hz, 1H), 7.62 (dd, *J =* 6.4, 2.3 Hz, 1H), 7.52 -7.35 (m, 5H), 7.02 (d, *J =* 8.9 Hz, 1H), 6.71 (s, 1H), 5.14 (dd, *J =* 13.3, 5.2 Hz, 1H), 4.66 (d, *J =* 11.9 Hz, 1H), 4.58 -4.39 (m, 3H), 4.24 (s, 3H), 3.69 (s, 3H), 3.66 |
| | -3.38 (m, 25H), 3.18 (dd, *J =* 12.9, 5.7 Hz, 1H), 3.06 (td, *J* = 6.7, 6.3, 3.7 Hz, 2H), 2.99 -2.82 (m, 4H), 2.81 -2.71 (m, 3H), 2.59 (d, *J =* 8.6 Hz, 2H), 2.47 (s, 3H), 2.14 (dtd, *J =* 12.8, 5.3, 2.4 Hz, 1H), 1.94 -1.82 (m, 2H), 1.35 -1.12 (m, 9H), 0.94 (d, *J =* 6.4 Hz, 4H). |
| | LCMS: C₆₂H₇₉N₁₁O₁₂ requires: 1170, found: m/z = 1171 [M+H]⁺. |
| 46 | ¹H NMR (500 MHz, Methanol-*d₄*) δ 8.64 (d, *J =* 2.3 Hz, 1H), 8.49 (d, *J =* 5.1 Hz, 1H), 7.92 (d, *J* = 2.8 Hz, 1H), 7.62 (dd, *J* = 6.7, 1.9 Hz, 1H), 7.51 - 7.38 (m, 5H), 7.03 (d, *J =* 8.9 Hz, 1H), 6.71 (s, 1H), 5.15 (ddd, *J =* 13.4, 5.2, 1.5 Hz, 1H), 4.68 (d, *J =* 11.9 Hz, 1H), 4.59 - 4.42 (m, 3H), 4.34 (s, 1H), 4.24 (s, 2H), 3.94 (d, *J =* 12.2 Hz, 1H), 3.70 (s, 3H), 3.64 - 3.57 (m, 1H), 3.55 - 3.35 (m, 4H), 3.16 (h, *J =* 6.5 Hz, 2H), 3.00 (t, *J =* 5.2 Hz, 2H), 2.96 - 2.84 (m, 1H), 2.81 - 2.68 (m, 3H), 2.64 - 2.43 (m, 5H), 2.17 (dd, *J =* 12.3, 6.3 Hz, 1H), 1.70 (p, *J =* 7.7 Hz, 2H), 1.56 (p, *J =* 7.1 Hz, 2H), 1.39 (p, *J =* 7.6 Hz, 2H), 1.34 - 1.20 (m, 10H), 0.89 (dd, *J =* 6.3, 3.4 Hz, 4H). |
| | LCMS: C₅₃H₆₁N₁₁O₇ requires: 964, found: m/z = 965 [M+H]⁺. |
| 47 | ¹H NMR (500 MHz, Methanol-d4) δ 8.63 (d, J = 2.3 Hz, 1H), 8.48 (d, J = 5.1 Hz, 1H), 7.90 (t, J = 2.0 Hz, 1H), 7.61 (dd, J = 6.9, 1.7 Hz, 1H), 7.49 - 7.41 (m, 4H), 7.36 (ddd, J = 8.9, 2.9, 1.4 Hz, 1H), 7.00 (d, J = 8.9 Hz, 1H), 6.71 (s, 1H), 6.52 (t, J = 5.6 Hz, 1H), 5.14 (ddd, J = 13.4, 5.2, 1.3 Hz, 1H), 4.66 (d, J = 11.9 Hz, 1H), 4.59 - 4.53 (m, 1H), 4.51 - 4.40 (m, 2H), 4.23 (dt, J = 9.2, 4.3 Hz, 2H), 3.93 (d, J = 11.7 Hz, 1H), 3.70 (s, 3H), 3.66 - 3.51 (m, 13H), 3.48 - 3.39 (m, 4H), 3.36 (ddd, *J =* 11.5, 5.8, 4.0 Hz, 4H), 2.99 (t, J = 5.3 Hz, 2H), 2.88 (ddd, J = 17.6, 13.6, 5.4 Hz, 1H), 2.79 - 2.71 (m, 3H), 2.59 (d, J = 8.6 Hz, 2H), 2.47 (s, 3H), 2.14 (dtd, J = 12.8, 5.3, 2.4 Hz, 1H), 1.96 - 1.86 (m, 2H), 1.32 - 1.17 (m, 8H), 0.90 (d, J = 6.4 Hz, 3H). |
| | LCMS: C₅₇H₆₉N₁₁O₁₀ requires: 1068, found: m/z = 1069 [M+H]⁺. |
| 48 | ¹H NMR (500 MHz, Methanol-d4) δ 8.63 (d, J = 2.3 Hz, 1H), 8.48 (d, J = 5.1 Hz, 1H), 7.92 (d, J = 2.8 Hz, 1H), 7.63 (dd, J = 6.0, 2.6 Hz, 1H), 7.51 - 7.34 (m, 5H), 7.02 (d, J = 8.9 Hz, 1H), 6.71 (s, 1H), 5.16 (dd, J = 13.4, 5.2 Hz, 1H), 4.67 (d, J = 11.9 Hz, 1H), 4.59 - 4.41 (m, 3H), 4.33 (d, J = 12.7 Hz, 1H), 4.29 - 4.19 (m, 2H), 3.94 (d, J = 12.3 Hz, 1H), 3.70 (s, 3H), 3.67 - 3.61 (m, 1H), 3.57 - 3.46 (m, 2H), 3.46 - 3.35 (m, 7H), 3.17 (tt, J = 6.9, 4.1 Hz, 2H), 3.02 (t, J = 5.3 Hz, 2H), 2.90 (ddd, J = 18.4, 13.5, 5.3 Hz, 1H), 2.82 - 2.70 (m, 3H), 2.59 (d, J = 8.7 Hz, 2H), 2.47 (s, 3H), 2.16 (dtd, J = 13.0, 5.4, 2.5 Hz, 1H), 1.98 - 1.83 (m, 2H), 1.60 - 1.46 (m, 4H), 1.42 - 1.14 (m, 15H), 0.92 (d, J = 6.3 Hz, 4H). |
| | LCMS: C₅₇H₆₉N₁₁O₈ requires: 1036, found: m/z = 1037 [M+H]⁺. |

### Example 7: General procedure for the synthesis of amine compounds according to Route 2 of Scheme 11.

Sodium triacetoxyborohydride (0.11 mmol) was added to a mixture of an HCl salt of 10-[3'-(hydroxymethyl)-1-methyl-5-({ 5-[(2 S)-2-methylpiperazin-1-yl]pyridin-2-yl } amino)-6-oxo-[3,4'-bipyridin]-2'-yl]-4,4-dimethyl-1,10-diazatricyclo[6.4.0.0-{2,6}]dodeca-2(6),7-dien-9-one **(E-2b)** (0.05 mmol), aldehyde (0.10 mmol), triethylamine (0.10 mmol) and DCE (0.50 mL). The mixture was allowed to stir at room temperature for 1 hour. Dichloromethane and saturated aqueous sodium bicarbonate were then added, and the organic layer was dried with MgSO₄, filtered, concentrated and purified by HPLC (5-95% MeCN in H₂O with 0.1% TFA) to afford the desired product.

### Example 7A: General procedure for the synthesis of amine intermediates.

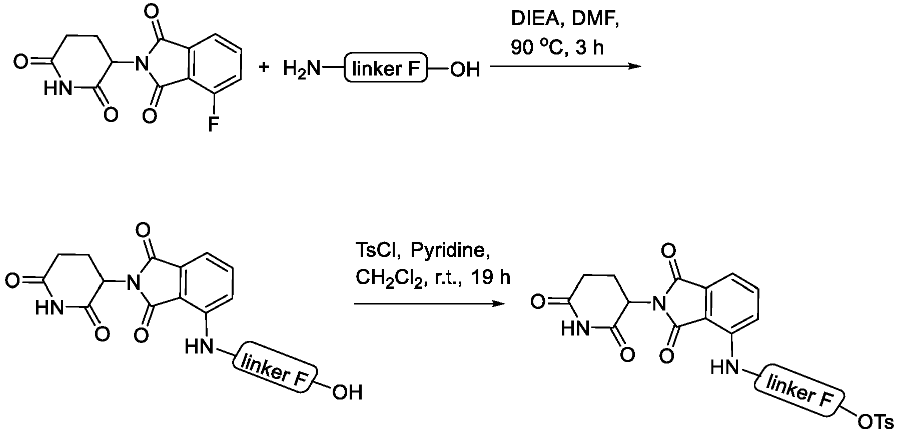

As used in this Example, "linker F" is -Y²-Y³-Y⁴-Y⁵-, wherein each of Y², Y³, Y⁴, and Y⁵ are defined herein.

Step 1: A solution of 2-(2,6-dioxo-3-piperidyl)-4-fluoro-isoindoline-1,3-dione (14.21 mmol), aminoalcohol (14.21 mmol) and DIPEA (28.41 mmol) in DMSO (50 mL) was stirred at 90°C for 3 hr under N₂. The reaction mixture was poured into water (100mL), and stirred for 3 min. The aqueous phase was extracted with ethyl acetate (40 mL × 6). The combined organic phase was washed with brine (20mL × 2), and concentrated in vacuum. The residue was purified by column chromatography to afford the alcohol intermediate.

Step 2: TsCl (14.63 mmol) was added into a solution of the alcohol intermediate (7.31 mmol) and pyridine (111.5 mmol) in DCM (20 mL) at 25 °C under N₂. The solution was stirred at 25 °C for 19 hrs. The reaction mixture was washed with brine (15mL × 2), dried over anhydrous Na₂SO₄, and concentrated in vacuo. The crude product was purified by HPLC to afford the tosylate.

The general methods of Example 5A were used to synthesize the intermediates described in Examples 7A-1 through 7A-3.

### Example 7A-1: Synthesis of 2-(2-((2-(2,6-dioxopiperidin-3-yl)-1,3-dioxoisoindolin-4-yl)amino)ethoxy)ethyl 4-methylbenzenesulfonate.

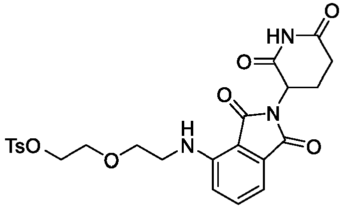

Step 1 product: 2-(2,6-dioxopiperidin-3-yl)-4-((2-(2-hydroxyethoxy)ethyl)amino)isoindoline-1,3-dione (2.8 g, 50%). LCMS; C₁₇H₁₉N₃O₆ requires: 361, found: m/z = 362 [M+H]⁺.

Step 2 product: 2-(2-((2-(2,6-dioxopiperidin-3-yl)-1,3-dioxoisoindolin-4-yl)amino)ethoxy)ethyl 4-methylbenzenesulfonate (1.2 g, 33% yield). LCMS; C₂₄H₂₅N₃O₈S requires: 515, found: m/z = 516 [M+H]⁺.

### Example 7A-2: Synthesis of 2-(2-(2-(2-((2-(2,6-dioxopiperidin-3-yl)-1,3-dioxoisoindolin-4-yl)amino)ethoxy)ethoxy)ethoxy)ethyl 4-methylbenzenesulfonate.

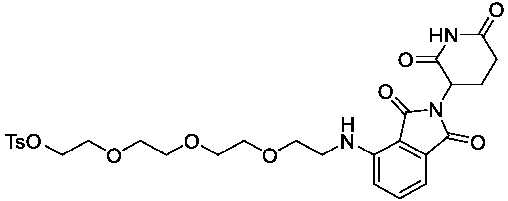

Step 1 product: 2-(2,6-dioxopiperidin-3-yl)-4-((2-(2-(2-(2-hydroxyethoxy)ethoxy)ethoxy)ethyl)amino)isoindoline-1,3-dione (2.04 g, 29.0%). LCMS; C₂₁H₂₇N₃O₈ requires: 449, found: m/z = 450 [M+H]⁺.

Step 2 product: 2-[2-[2-[2-[[2-(2,6-dioxo-3-piperidyl)-1,3-dioxo-isoindolin-4-yl]amino]ethoxy]ethoxy]ethoxy]ethyl 4-methylbenzenesulfonate was obtained (181.3 mg, 14.35%). LCMS; C₂₈H₃₃N₃O₁₀S requires: 603, found: m/z = 604 [M+H]⁺.

### Example 7A-3: Synthesis of 2-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]ethyl 4-methylbenzenesulfonate.

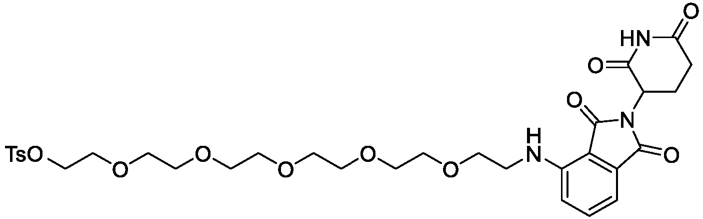

Step 1 product: 2-(2,6-dioxo-3-piperidyl)-4-[2-[2-[2-[2-[2-(2-hydroxyethoxy)ethoxy]ethoxy]ethoxy]ethoxy]ethylamino]isoindoline-1,3-dione (2.2 g, 56%). LCMS; C₂₅H₃₅N₃O₁₀ requires: 537, found: m/z = 538 [M+H]⁺.

Step 2 product: 2-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]ethyl 4-methylbenzenesulfonate (0.507 g, 17.9%). LCMS; C₃₂H₄₁N₃O₁₂S requires: 691, found: m/z = 692 [M+H]⁺.

**Table 6: Bifunctional compounds generated according to Route 2 of Scheme 6 and using the procedures in Examples 1, 2A-1, and 4.**

| **Cmpd No.** | **Characterization data** |
|---|---|
| 39 | ¹H NMR (500 MHz, Methanol-*d₄*) δ 8.65 (d, *J =* 2.3 Hz, 1H), 8.48 (d, *J=* 5.1 Hz, 1H), 7.94 (d, *J =* 2.8 Hz, 1H), 7.57 - 7.38 (m, 4H), 7.10 - 6.93 (m, 3H), 6.71 (s, 1H), 5.02 (dd, *J =* 12.6, 5.4 Hz, 1H), 4.70 - 4.64 (m, 1H), 4.55 (d, *J =* 11.9 Hz, 1H), 4.32 (d, *J =* 12.6 Hz, 1H), 4.29 - 4.19 (m, 2H), 3.75 - 3.53 (m, 24H), 3.49 - 3.39 (m, 3H), 3.02 (s, 2H), 2.87 - 2.53 (m, 10H), 2.53 - 2.37 (m, 3H), 1.26 (d, *J =* 15.2 Hz, 10H), 0.92 (d, *J =* 6.3 Hz, 4H). |
| | LCMS: C₅₉H₇₃N₁₁O₁₂ requires: 1128, found: m/z = 1129 [M+H]⁺. |
| 40 | ¹H NMR (500 MHz, Methanol-*d₄*) δ 8.66 (d, *J =* 2.2 Hz, 1H), 8.50 (d, *J=* 5.1 Hz, 1H), 7.93 (s, 1H), 7.61 - 7.45 (m, 3H), 7.41 (dd, *J =* 9.0, 2.9 Hz, 1H), 7.14 - 6.95 (m, 3H), 6.71 (s, 1H), 4.97 (d, *J =* 5.6 Hz, 1H), 4.68 (d, *J =* 11.9 Hz, 1H), 4.56 (d, *J =* 10.7 Hz, 1H), 4.38 - 4.20 (m, 3H), 3.70 (d, *J =* 8.1 Hz, 7H), 3.52 (t, *J =* 5.0 Hz, 2H), 3.38 (s, 1H), 3.05 - 2.95 (m, 2H), 2.82 - 2.53 (m, 10H), 2.48 (s, 2H), 2.43 - 2.35 (m, 1H), 1.99 (dd, *J =* 10.4, 5.9 Hz, 1H), 1.38 - 1.16 (m, 12H), 0.89 (d, *J =* 6.4 Hz, 5H). |
| | LCMS: C₅₁H₅₇N₁₁O₈ requires: 952, found: m/z = 953 [M+H]⁺. |
| 41 | ¹H NMR (500 MHz, Methanol-*d₄*) δ 8.65 (d, *J =* 2.3 Hz, 1H), 8.49 (d, *J=* 5.1 Hz, 1H), 7.92 (d, *J =* 2.8 Hz, 1H), 7.60 - 7.36 (m, 4H), 7.09 - 6.95 (m, 3H), 6.71 (s, 1H), 4.68 (d, *J =* 11.9 Hz, 1H), 4.55 (d, *J =* 12.0 Hz, 1H), 4.39 - 4.19 (m, 3H), 3.94 (d, *J =* 12.4 Hz, 1H), 3.75 - 3.56 (m, 15H), 3.47 (t, *J =* 5.3 Hz, 2H), 3.03 (dddd, *J =* 21.2, 17.2, 10.5, 5.6 Hz, 2H), 2.87 - 2.75 (m, 3H), 2.75 - 2.53 (m, 7H), 2.47 (s, 2H), 2.40 (dd, *J =* 11.4, 7.2 Hz, 1H), 1.38 - 1.21 (m, 11H), 0.90 (dd, *J =* 6.6, 4.1 Hz, 5H). |
| | LCMS: C₅₅H₆₅N₁₁O₁₀ requires: 1040, found: m/z = 1041 [M+H]⁺. |

### Example 7: Assays.

### Cell Culture

Ramos (CRL-1596) cells were obtained from American Type Culture Collection and were grown in RPMI-1640 media (ATCC, 30-2001) supplemented with 10% heat-inactivated FBS (Corning Premium Fetal Bovine Serum from Fisher, MT35015CV).

### Cellular BTK HTRF Assay

Compounds of the present disclosure were added to 50,000 Ramos cells in roundbottom 96 well plates with a final DMSO concentration of > 0.2% and were incubated at 37°C 5% CO₂ for four hours. BTK levels were determined using Cisbio Total-BTK HTRF (Homologous Time-Resolved Fluorescence) kit (63ADK064PEG) according to manufacturer's protocol. Briefly, cells were incubated in 1X supplied lysis buffer for 30 minutes. In an opaque white low volume 96 well plate (Cisbio, 66PL96005), cell lysate was combined with two different specific BTK antibodies, one conjugated with Eu³⁺-Cryptate FRET donor and one conjugated with d2 FRET acceptor. Assay controls include wells containing cell lysate with only the Eu³⁺-Cryptate FRET donor antibody and wells containing both HTRF antibodies and lysis buffer without cells or control lysate provided by Cisbio. HTRF ratio was calculated as (acceptor signal at 665 nm / donor signal at 620 nm) x 10⁴. Background HTRF levels were determined from the control well containing the donor, but no acceptor, antibody. Background HTRF levels were subtracted from all samples. Readouts were reported as HTRF levels relative to HTRF levels of DMSO-treated cells. Four-parameter non-linear regressions were performed in GraphPad Prism 7.02 to obtain DC₅₀ values. DC₅₀ values are provided in Table 7, wherein A < 1 nM, 1 nM ≤ B ≤ 10 nM, and 10 nM < C < 1 µM.

**Table 7: Activity of bifunctional compound of the present disclosure.**

| **Compound No.** | **Cellular BTK HTRF Ramos: DC₅₀ (uM)** |
|---|---|
| 1 | B |
| 2 | C |
| 3 | B |
| 4 | C |
| 5 | C |
| 6 | C |
| 7 | C |
| 8 | C |

| **Compound No.** | **Cellular BTK HTRF Ramos: DC₅₀ (uM)** |
|---|---|
| 9 | B |
| 10 | C |
| 11 | B |
| 12 | B |
| 13 | C |
| 14 | B |
| 15 | B |
| 16 | C |

| **Compound No.** | **Cellular BTK HTRF Ramos: DC₅₀ (uM)** |
|---|---|
| 17 | C |
| 18 | B |
| 19 | B |
| 20 | B |
| 21 | C |
| 22 | C |
| 23 | B |
| 24 | B |
| 25 | A |
| 26 | B |
| 27 | C |
| 28 | B |
| 29 | B |
| 30 | B |
| 31 | B |
| 32 | B |
| 33 | B |
| 34 | B |
| 35 | B |
| 36 | C |
| 37 | B |

| **Compound No.** | **Cellular BTK HTRF Ramos: DC₅₀ (uM)** |
|---|---|
| 38 | B |
| 39 | B |
| 40 | A |
| 41 | B |
| 42 | C |
| 43 | B |
| 44 | B |
| 45 | B |
| 46 | B |
| 47 | B |
| 48 | B |
| 49 | B |
| 50 | B |
| 51 | B |
| 52 | B |
| 53 | A |
| 54 | B |
| 55 | B |
| 56 | B |
| 57 | B |
| 58 | B |

## Claims

1. A compound of Formula (I): 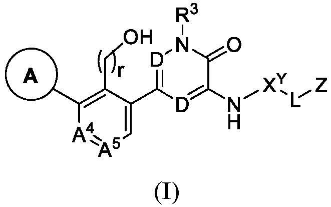 or a pharmaceutically acceptable salt thereof, wherein
each of A⁴ and *A⁵* is independently N or C-R¹⁵,
each R¹⁵ is independently -H or an optionally substituted C₁₋₆ alkyl;
each D is independently N or CH;
wherein ring A is: 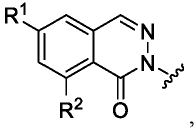 wherein each of R¹ and R² is independently halo, -H, -OH, -CN, -CF₃, -C₁₋₆ alkyl, or a 3-6 membered cycloaliphatic; 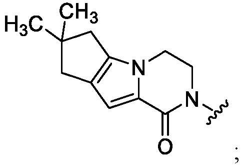 or 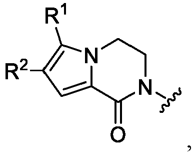 wherein each of R¹ and R² is independently halo, -H, -OH, -CN, -CF₃, -C₁₋₆ alkyl, or a 3-6 membered cycloaliphatic, or R¹ and R² together with the atoms to which they are attached form a 5-6 membered cycloalkyl, wherein the cycloalkyl is optionally substituted with 1-2 of R⁶;
each of R¹ and R² is independently halo, -H, -OH, -CN, -CF₃, -C₁₋₆ alkyl, or a 3-6 membered cycloaliphatic, or
R¹ and R² together with the atoms to which they are attached form a 5-6 membered cycloalkyl fused to ring C, wherein the cycloalkyl is optionally substituted with 1-2 of R⁶, wherein each R⁶ is independently -H, halo, or C₁₋₃ alkyl; R³ is C₁₋₆ alkyl;
X^{Y} is 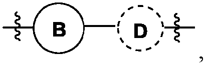
wherein
Ring B is phenyl, or a 5-6 membered monocyclic heteroaryl having 1-2 nitrogen atoms, or a 7-10 membered fused bicyclic heterocycle having 2-3 nitrogen atoms, wherein at least one of the rings of the bicyclic heterocycle is partially or fully unsaturated, and ring D is absent or an optionally substituted 4-7 membered heterocycle having at least 1 nitrogen atom and optionally 2 additional heteroatoms independently selected from N, O, or S;
L is a bivalent C₁₋₂₀ alkyl group, wherein one or more methylene units of the C₁₋₂₀ alkyl group is optionally and independently replaced by -C(O)-, -C(S)-, -(O)CO-, -OC(O)-, -O-, -N(R')-, -S-, -N(R')C(O)-, bivalent mono- or bicyclic heterocycloalkyl, bivalent mono- or bicyclic cycloalkyl, bivalent mono- or bicyclic aryl, or bivalent mono- or bicyclic heteroaryl, wherein each of the bivalent heterocycloalkyl, cycloalkyl, aryl, and heteroaryl are optionally substituted; and
Z is 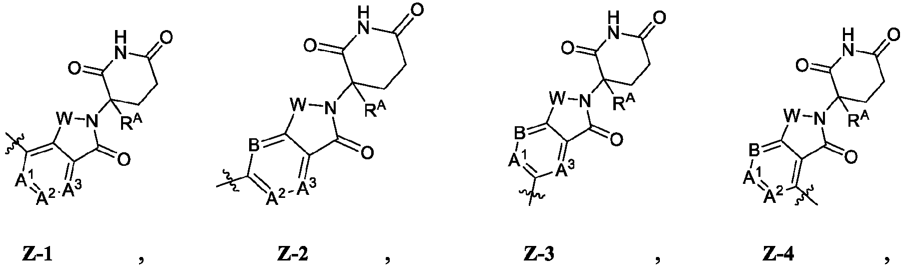 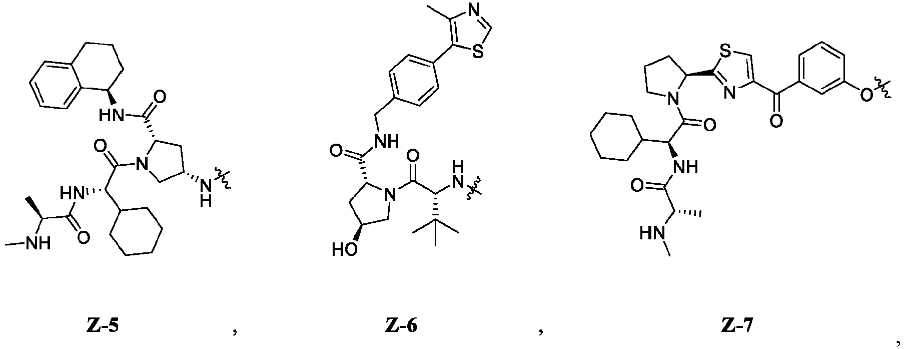
or 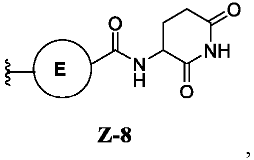
wherein
ring E is phenyl or a 5-6 membered heteroaryl having at least 1 heteroatom independently selected from N or S;
each of A¹, A², and A³ is independently -C(R^{B})= or -N=;
B is -C(R^{C})= or -N=;
W is -C(R^{D})₂- or -C(O)-;
R^{A} is -H, -CH₃, or -F;
each R^{B} is independently-H, halo, -OH, -C₁₋₄ alkyl, or -C₁₋₄ alkoxy;
R^{C} is -H, halo, or C₁₋₄ alkyl; and
each R^{D} is independently-H or -C₁₋₃ alkyl; and
r is 1, 2, or 3.

2. The compound or pharmaceutically acceptable salt of claim 1, wherein:
- Z is 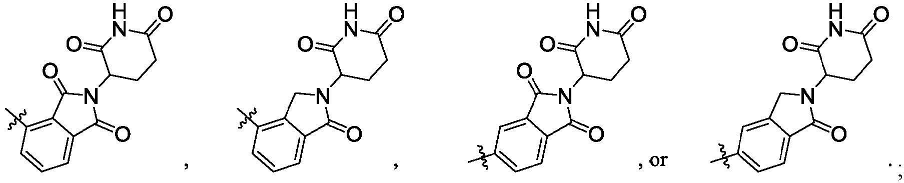 or Z is 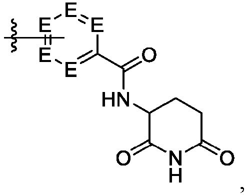 where one instance of E is 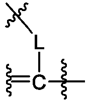 and the remaining instances of E are each independently =CH- or =N-; or Z is 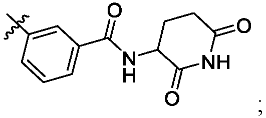
- one D is CH and the other D is N; or each D is CH;
- at least one of A⁴ and A⁵ is N; or A⁴ is N, A⁵ is CR¹⁵, and R¹⁵ is -H or C₁₋₃ alkyl;
- R¹⁵ is -H or methyl;
- X^{Y} is: 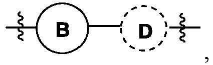
ring B is 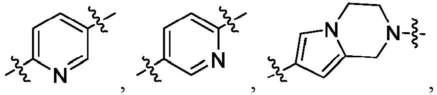
or 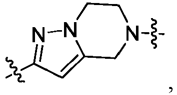
and ring D is absent; 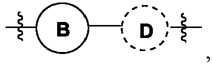
ring B is 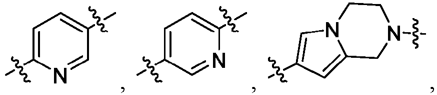
or 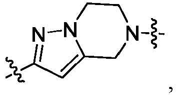
and ring D is 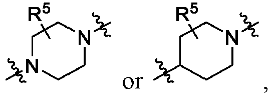
and R⁵ is -H or C₁₋₃ alkyl; or 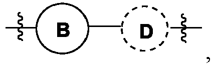
ring B is 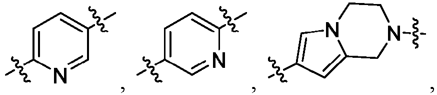
or 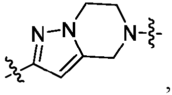
and ring D is 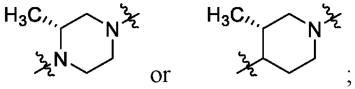
- R³ is a C₁₋₃ alkyl; and/or
- r is 1 or 2.

3. The compound or pharmaceutically acceptable salt of claim 1, wherein the compound of Formula (I) is:
- a compound of Formula (I-A) 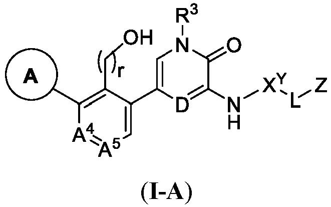 or a pharmaceutically acceptable salt thereof;
- a compound of Formula (I-A1) 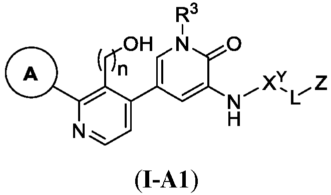 or a pharmaceutically acceptable salt thereof;
- a compound of Formula (I-A2) 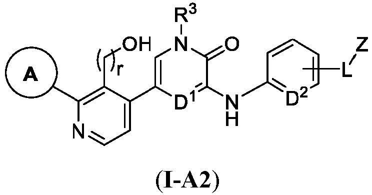 or a pharmaceutically acceptable salt thereof, wherein
D¹ is CH or N; D² is CH or N, and at least one of D¹ and D² is N;
optionally wherein D¹ is =CH- and D² is =N-; or D¹ is =N- and D² is =CH-; or
- a compound of Formula (I-A3) 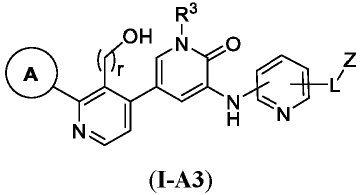 or a pharmaceutically acceptable salt thereof;
- a compound of Formula (I-B1), a compound of Formula (I-B2), or a compound of Formula (I-B3) 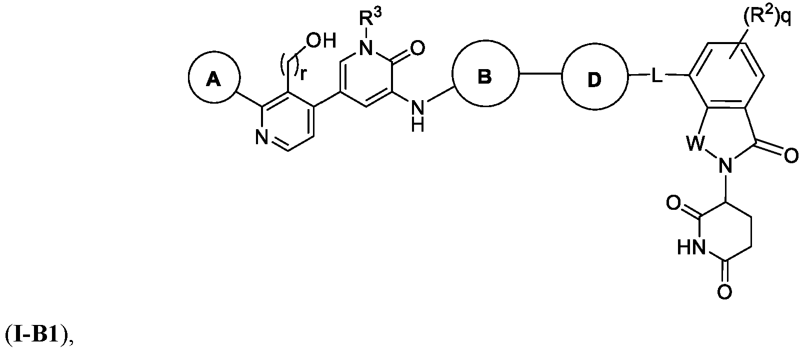 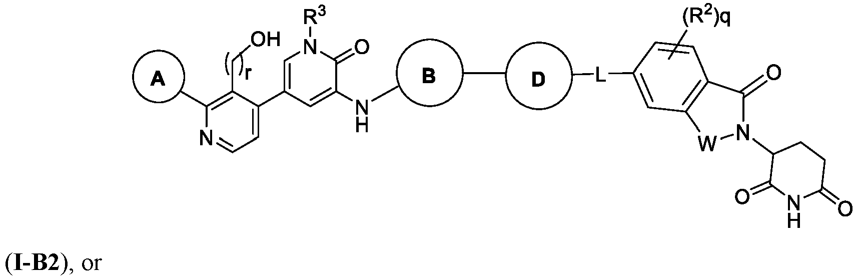 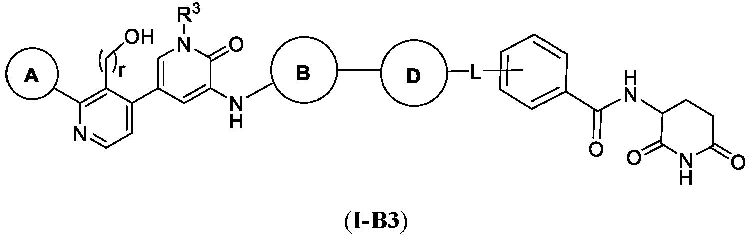
or a pharmaceutically acceptable salt thereof;
optionally wherein
ring B is phenyl, pyridine-yl, or pyrimidine-yl; and/or ring D is an optionally substituted 4-7 membered heterocycle having 1-2 nitrogen atoms or ring D is an optionally substituted 5-6 membered heterocycle having 1-2 nitrogen atoms.

4. The compound or pharmaceutically acceptable salt of any one of claims 1-3, wherein L is -Y¹-Y²-Y³-Y⁴-Y⁵-Y⁶-, wherein
Y¹ is:
- C(O)-, -C(O)-N(R)-, -(CH₂-CH₂-O)ₘ-, -(O-CH₂-CH₂)ₘ-, or -C₁₋₆ alkyl-;
- C(O)- or -C(O)-N(R)-;
- C(O)-, Y² is -(CH₂-CH₂-O)ₙ-, -(O-CH₂-CH₂)ₙ-, -C₁₋₄ alkyl-, a 3-6 membered monocyclic cycloalkyl, or a 7-11 membered fused or spiro bicycloalkyl, and n is 1 or 2; or
- (CH₂-CH₂-O)ₘ-, -(O-CH₂-CH₂)ₘ-, or -C₁₋₆ alkyl-;
Y² is:
a bond, -C(O)-, -O-, -(CH₂-CH₂-O)ₙ-, -(O-CH₂-CH₂)ₙ-, -C₁₋₄ alkyl-, an optionally substituted 3-6 membered monocyclic cycloalkyl, or an optionally substituted 7-11 membered fused or spiro bicycloalkyl;
-(CH₂-CH₂-O)ₙ-, -(O-CH₂-CH₂)ₙ-, -CH₂-, -CH₂-CH₂-, -CH₂-CH₂-CH₂-, cyclopentyl, cyclohexyl, or a 7-11 membered spiro bicycloalkyl, and n is 1 or 2;
-C(O)-, -O-, -(CH₂-CH₂-O)ₙ-, -(O-CH₂-CH₂)ₙ-, 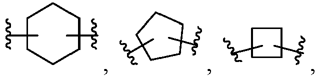 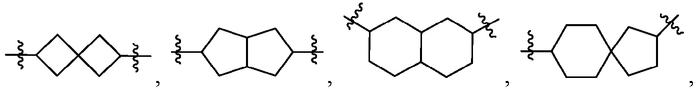 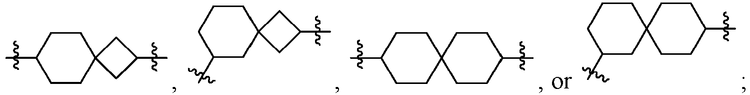 or
-C(O)-, -O-, -(CH₂-CH₂-O)ₙ-, or -(O-CH₂-CH₂)ₙ-, and n is 2 or 3;
Y³ is:
a bond, -O-, -N(R)-, -C₁₋₄ alkyl-, -(CH₂-CH₂-O)ₚ-, -(O-CH₂-CH₂)ₚ-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S;
a bond, -O-, -N(R)-, -C₁₋₄ alkyl-, -(CH₂-CH₂-N(R))-, phenyl, a 5-6 membered monocyclic heteroaryl having 1-2 heteroatoms independently selected from N and S, an optionally substituted 3-6 membered cycloalkyl, or an optionally substituted 4-6 membered heterocycle having 1-3 heteroatoms independently selected from N, O, or S;
a bond, -N(R)-, -O-, -CH₂-, -CH₂-CH₂-, -CH₂-CH₂-CH₂-, -(CH₂-CH₂-N(R))-, phenyl, 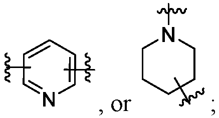
a bond, -O-, -N(R)-, -(CH₂-CH₂-O)ₚ-, -(O-CH₂-CH₂)ₚ-, -(CH₂-CH₂-N(R))-, phenyl, 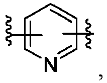 an optionally substituted 3-6 membered monocylic cycloalkyl, or an optionally substituted 4-6 membered monocyclic heterocycle having 1-3 heteroatoms independently selected from N, O, or S; or
a bond, -O-, -N(R)-, -(CH₂-CH₂-O)ₚ-, or -(O-CH₂-CH₂)ₚ-, and p is 1 or 2; or 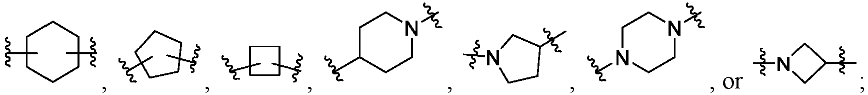
Y⁴ is:
a bond, -C(O)-, -N(R)-, -C(O)N(R)-, -N(R)C(O)-, -C₁₋₄ alkyl-, -(CH₂-CH₂-O)_{q}-, -(O-CH₂-CH₂)_{q}-, -(CH₂-CH₂-N(R))-, an optionally substituted 3-6 membered cycloalkyl, or an optionally substituted 4-6 membered heterocycle having 1-3 heteroatoms independently selected from N, O, or S;
a bond, -C(O)-, -N(R)-, -C(O)N(R)-, -N(R)C(O)-, -C₁₋₄ alkyl-, -(CH₂-CH₂-N(R))-, an optionally substituted 3-6 membered cycloalkyl, or an optionally substituted 4-6 membered heterocycle having 1-3 heteroatoms independently selected from N, O, or S;
a bond, -C(O)-, -N(R)-, -C(O)N(R)-, -N(R)C(O)-, -CH₂-, -CH₂-CH₂-, 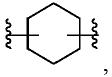 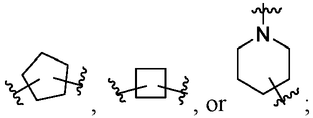
a bond, -N(R)-, -C(O)N(R)-, -N(R)C(O)-, -C₁₋₄ alkyl-, -(CH₂-CH₂-N(R))-, an optionally substituted 3-6 membered cycloalkyl, or an optionally substituted 4-6 membered heterocycle having 1-3 heteroatoms independently selected from N, O, or S; or
a bond, -N(H)-, -N(CH₃)-, -N(H)C(O)-, -CH₂-, -CH₂-CH₂-, -CH₂-CH₂-CH₂-, -(CH₂-CH₂-N(H))-, 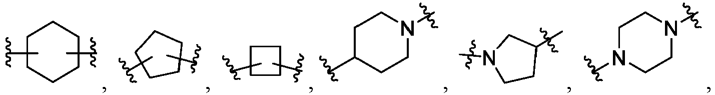
or 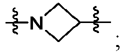
Y⁵ is:
a bond, -N(R)-, -C(O)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S;
a bond, -N(R)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S;
a bond, -N(R)-, -CH₂-, -CH₂-CH₂-, -CH₂-CH₂-CH₂-, -(CH₂-CH₂-O)ₛ-, -(O-CH₂-CH₂)ₛ-, or 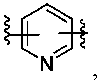 and s is 1 or 2;
a bond, -C(O)-, -C₁₋₄ alkyl-, -(CH₂-CH₂-O)ₛ-, -(O-CH₂-CH₂)ₛ-, or a 5-6 membered heteroaryl having 1-3 heteroatoms independently selected from N or S; or
a bond, -C(O)-, -CH₂-, -CH₂-CH₂-, -CH₂-CH₂-CH₂-, -(CH₂-CH₂-O)ₛ-, -(O-CH₂-CH₂)ₛ-, or 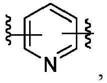 and s is 1 or 2; and Y⁶ is:
a bond, or -(CH₂-CH₂-N(R))-; or a bond or -CH₂-CH₂-N(H)-
Each of m and n is independently 1, 2, 3, 4, or 5;
Each of p, q, and s is independently 1, 2, or 3; and
R is -H or -C₁₋₃ alkyl.

5. The compound or pharmaceutically acceptable salt of claim 1, wherein the compound of Formula **(I)** is a compound of Formula (**II**) 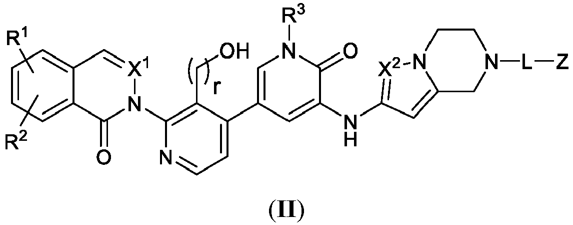 or a pharmaceutically acceptable salt thereof;
a compound of Formula **(II-A)** 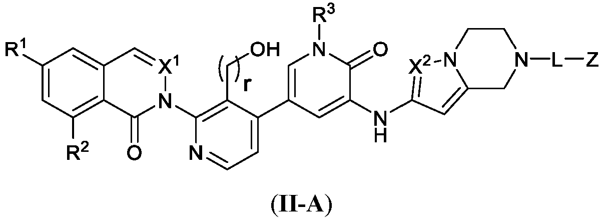 or a pharmaceutically acceptable salt thereof; or
a compound of Formula **(II-B)** 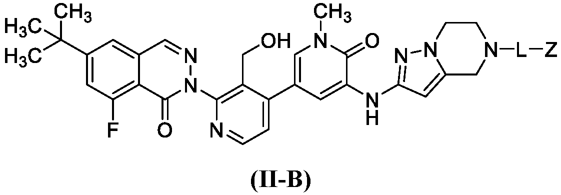 or a pharmaceutically acceptable salt thereof;
wherein
R¹ is C₁₋₆ alkyl, hydroxyl, halo, cyano, or a 3-6 membered cycloaliphatic;
R² is C₁₋₆ alkyl, hydroxyl, halo, cyano, or a 3-6 membered cycloaliphatic; or R² is C₁₋₆ alkyl, hydroxyl, halo, or cyano;
R³ is C₁₋₆ alkyl;
X¹ is =N-;
X² is =CH- or =N-;
r is 1, 2, or 3;
L is a bivalent C₁₋₂₀ alkyl group, optionally substituted with C₁₋₆ alkyl, acyl, oxo, halo, or alkoxy, wherein one or more methylene units of said C₁₋₂₀ alkyl group is optionally and
independently replaced by -CO-, -CS-, -CONH-, -CONHNH-, -CO₂-, -OCO-, - NHCO₂-, -O-, -NHCONH-, -OCONH-, -NHNH-, -NHCO-, -S-, -S(O)-, -S(O)₂-, -NH-, -S(O)₂NH-, -NHS(O)₂-, -NHS(O)₂NH-, bivalent mono- or bicyclic heterocycle, bivalent mono- or bicyclic cycloalkyl, bivalent mono- or bicyclic aryl, or bivalent mono- or bicyclic heteroaryl, wherein the heterocycle and heteroaryl of L has 1-3 heteroatoms independently selected from N, O, or S; and
Z is 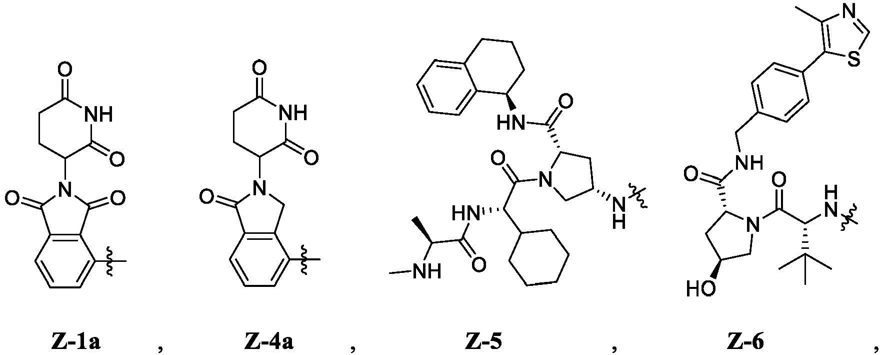
or

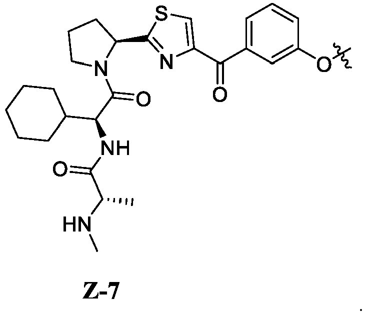

6. The compound or pharmaceutically acceptable salt of claim 5, wherein:
- R¹ is C₁₋₆ alkyl, optionally wherein R¹ is methyl, ethyl, propyl, iso-propyl, sec-butyl, or *tert*-butyl, preferably wherein R¹ is *tert*-butyl; or R¹ is 3-6 membered cycloaliphatic, optionally wherein R¹ is cyclopropyl, cyclopentyl, or cyclohexyl;
- R² is halo, optionally wherein R² is -F;
- R³ is methyl, ethyl, propyl, *iso*-propyl, *sec*-butyl, or *tert*-butyl, optionally wherein R³ is methyl;
- n is 1;
- at least one of X¹ and X² is =N-; optionally wherein both of X¹ and X² are =N-; and/or
- L is a bivalent C₁₋₂₀ alkyl group, wherein one or more methylene units of the C₁₋₂₀ alkyl group is optionally and independently replaced by -CO-, -CS-, -OCO-, -O-, -NHCO-, -S-, -NH-, bivalent mono- or bicyclic heterocycle, bivalent mono- or bicyclic cycloalkyl, bivalent mono- or bicyclic aryl, or bivalent mono- or bicyclic heteroaryl, wherein the heterocycle and heteroaryl have 1-3 heteroatoms independently selected from N, O, or S; or wherein L is a bivalent C₁₋₂₀ alkyl group, wherein one or more methylene units of the C₁₋₂₀ alkyl group is optionally and independently replaced by -CO-, -O-, -NH-, bivalent mono- or bicyclic heterocycloalkyl having 1-3 heteroatoms independently selected from N, O, or S, or bivalent mono- or bicyclic cycloalkyl; or wherein L is a bivalent C₁₋₁₀ alkyl group, wherein one or more methylene units of the C₁₋₁₀ alkyl group is optionally and independently replaced by -CO-, -OCO-, -O-, -NHCO-, -NH-, bivalent mono- or bicyclic heterocycle, bivalent mono- or bicyclic cycloalkyl, bivalent mono- or bicyclic aryl, or bivalent mono- or bicyclic heteroaryl, wherein the heterocycle and heteroaryl have 1-3 heteroatoms independently selected from N, O, or S.

7. The compound or pharmaceutically acceptable salt of claim 5 or claim 6, wherein L is -Y¹-Y²-Y³-Y⁴-Y⁵-, wherein
Y¹ is:
-C(O)- or -C₁₋₆ alkyl-; or
-C(O)- or -C₄₋₆ alkyl-;
Y² is:
a bond, -O-, -(CH₂-CH₂-O)ₙ-, -(O-CH₂-CH₂)ₙ-, -C₁₋₄ alkyl-, 3-6 membered cycloalkyl; or
-(CH₂-CH₂-O)ₙ-, -(O-CH₂-CH₂)ₙ-, or -C₁₋₄ alkyl;
Y³ is:
a bond, -O-, -N(R)-, -C₁₋₄ alkyl-, -(CH₂-CH₂-O)ₚ-, -(O-CH₂-CH₂)ₚ-, -(CH₂-CH₂-N(R))-, phenyl, 3-6 membered cycloalkyl, or 4-6 membered heterocycle having 1-3 heteroatoms independently selected from N, O, or S; or
a bond, -O-, -C₁₋₄ alkyl-, -(CH₂-CH₂-N(R))-, 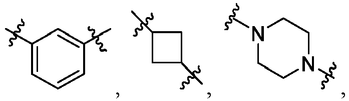 or 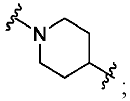
Y⁴ is
a bond, -C(O)-, -C(O)N(R)-, -C(O)N(R)-, -C₁₋₄ alkyl-, -(CH₂-CH₂-O)_{q}-, -(O-CH₂-CH₂)_{q}-, -(CH₂-CH₂-N(R))-, 3-6 membered cycloalkyl, or 4-6 membered heterocycle having 1-3 heteroatoms independently selected from N, O, or S; or
a bond, -C(O)-, -C(O)N(R)-, -C(O)N(R)-, or -C₁₋₄ alkyl-;
Y⁵ is
a bond, -N(R)-, -C(O)-, -C₁₋₄ alkyl-, or a 5-6 membered heteroaryl having 1-3 heteroatoms independently selected from N, O or S;
n is 1, 2, 3, 4, or 5;
p is 1, 2, or 3;
q is 1, 2, or 3; and
R is -H or -C₁₋₃ alkyl or
a bond, -N(R)-, -C(O)-, -C₁₋₄ alkyl-, or

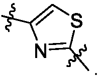

8. The compound or pharmaceutically acceptable salt of any one of claims 4-7, wherein L is selected from methylene, ethylene, n-propylene, n-butylene, n-pentylene, n-hexylene, 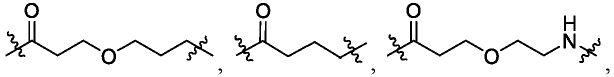 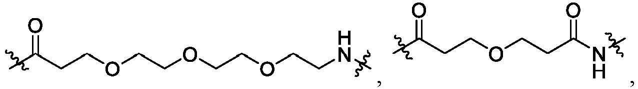 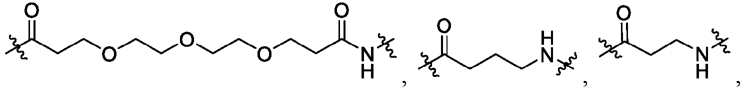 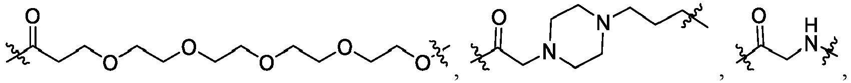 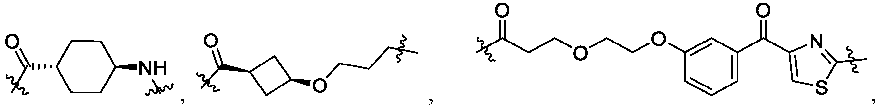 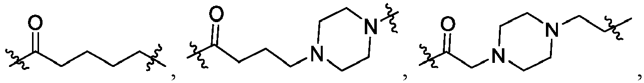 or

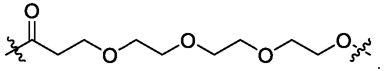

9. The compound or pharmaceutically acceptable salt of claim 1, wherein the compound of Formula **(I)** is a compound of Formula (**III**) 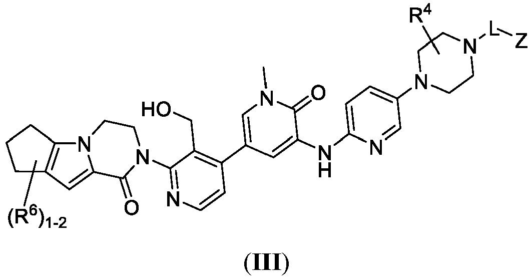 or pharmaceutically acceptable salt thereof;
optionally wherein each R⁶ is independently -H or C₁₋₃ alkyl, and/or R⁴ is independently -H or C₁₋₃ alkyl.

10. The compound or pharmaceutically acceptable salt of claim 9, wherein the compound of Formula (III) is:
a compound of Formula (III-A) 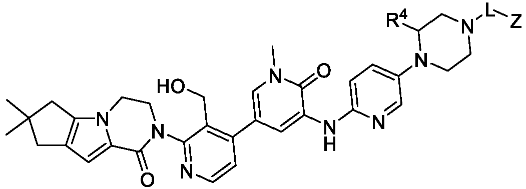 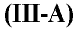 or a pharmaceutically acceptable salt thereof;
a compound of Formula **(III-B)** 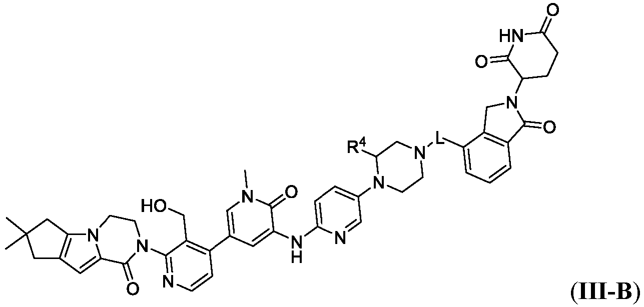 or a pharmaceutically acceptable salt thereof; or
a compound of Formula **(III-C)** 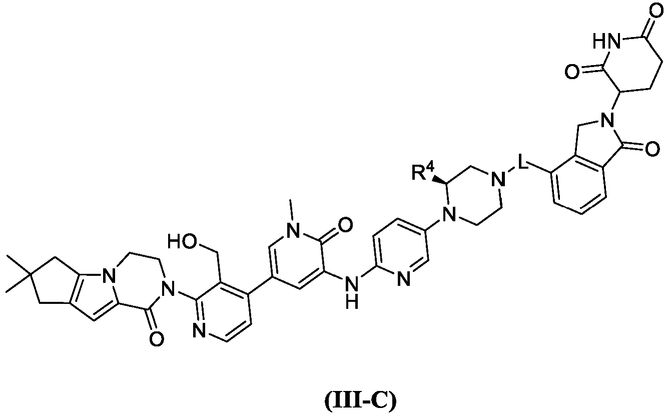 or a pharmaceutically acceptable salt thereof;
wherein:
R⁴ is -H or -C₁₋₃ alkyl;
L is -Y¹-Y²-Y³-Y⁴-Y⁵-Y⁶-;
Y¹ is -C(O)-, -C(O)-N(R)-, -(CH₂-CH₂-O)ₘ-, -(O-CH₂-CH₂)ₘ-, or -C₁₋₄ alkyl;
Y² is a bond, -O-, -C₁₋₄ alkyl-, -(O-CH₂-CH₂)ₙ-, -(CH₂-CH₂-O)ₙ-, -(CH₂-CH₂-N(R))-, or 3-6 membered cycloalkyl,
Y³ is a bond, -O-, -N(R)-, -C₁₋₄ alkyl-, -(CH₂-CH₂-O)ₚ-, -(O-CH₂-CH₂)ₚ-, -(CH₂-CH₂-N(R))-, 3-6 membered cycloalkyl, or 4-6 membered heterocycloalkyl having 1-3 heteroatoms independently selected from N, O, or S;
Y⁴ is a bond, -N(R)-, -C₁₋₄ alkyl-, -(CH₂-CH₂-O)_{q}-, -(O-CH₂-CH₂)_{q}-, -(CH₂-CH₂-N(R))-, 3-6 membered cycloalkyl, or 4-6 membered heterocycloalkyl having 1-3 heteroatoms independently selected from N, O, or S;
Y⁵ is a bond, -N(R)-, -(CH₂-CH₂-O)ₛ-, or -(O-CH₂-CH₂)ₛ-;
Y⁶ is a bond or -(CH₂-CH₂-N(R))-;
each R is independently-H or -C₁₋₃ alkyl; and
each of m, p, q, and s is independently 1, 2, or 3;
Z is 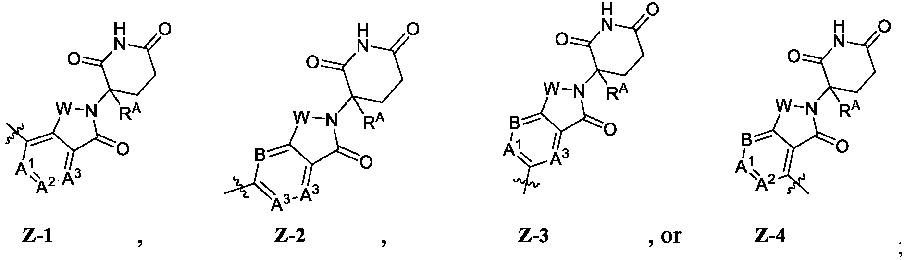
or 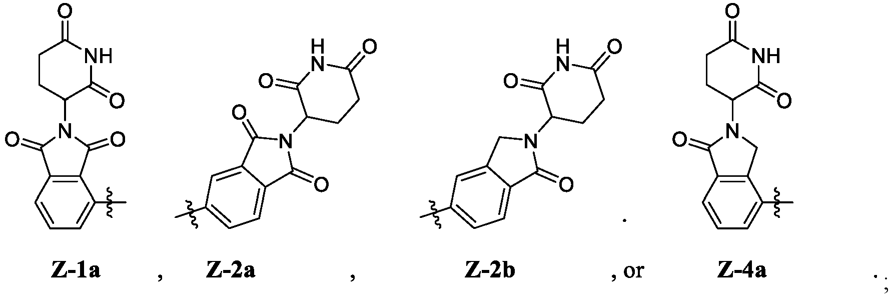
Each of A¹, A², and A³ is independently -C(R^{B})= or -N=;
B is -C(R^{C})= or -N=;
W is -C(R^{D})₂- or -C(O)-;
R^{A} is -H, -CH₃, or -F;
Each R^{B} is independently-H, halo, -OH, -C₁₋₄ alkyl, or -C₁₋₄ alkoxy;
R^{C} is -H, halo, or C₁₋₄ alkyl; and
Each R^{D} is independently-H or -C₁₋₃ alkyl;
provided that when each of Y³, Y⁴, Y⁵, and Y⁶ are a bond, then Y² is not a bond.

11. The compound or pharmaceutically acceptable salt of claim 9 or claim 10, wherein L is selected from 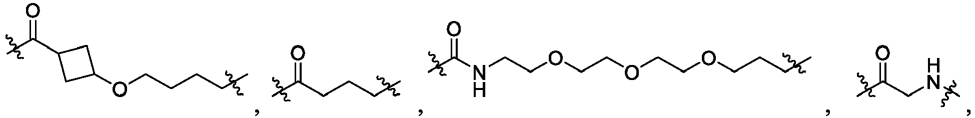 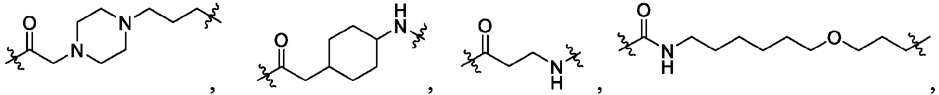 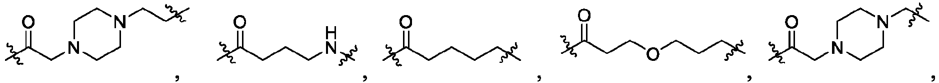 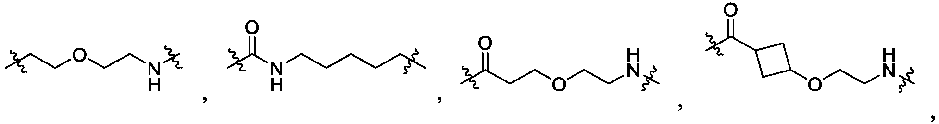 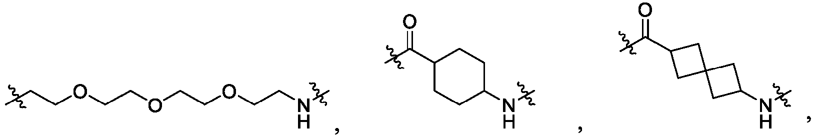 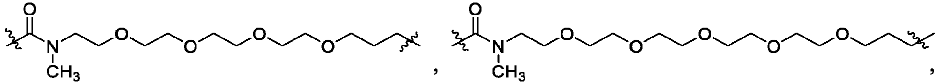 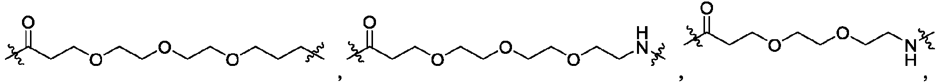 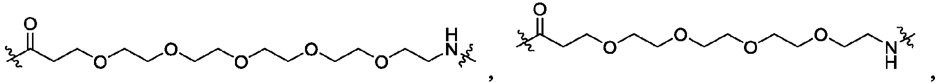 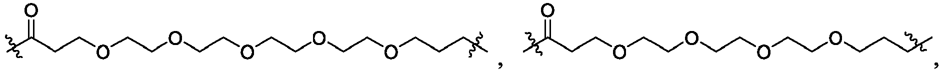 or

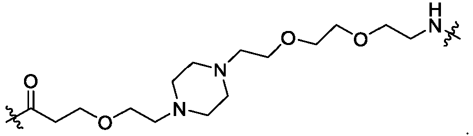

12. The compound of claim 1, selected from Table 1 or a pharmaceutically acceptable salt thereof:
**Table 1**
| **Cmpd No.** | **Structure** |
|---|---|
| 1 | 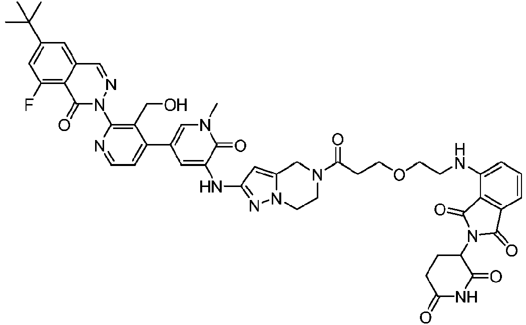 |
| 2 | 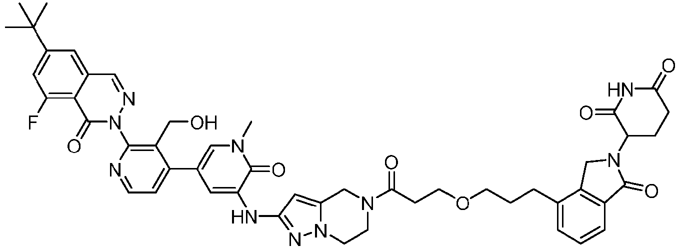 |
| 3 | 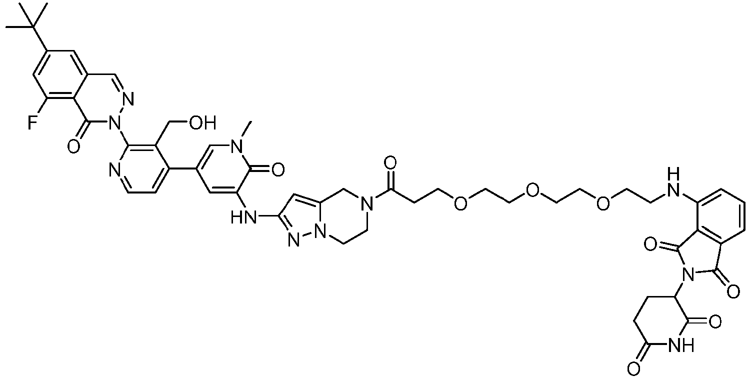 |
| 4 | 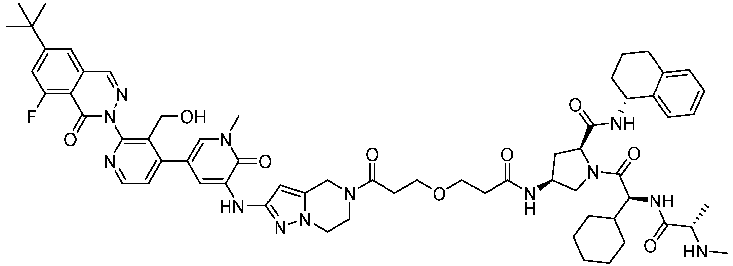 |
| 5 | 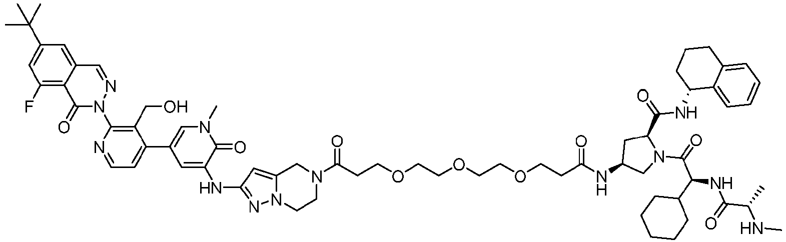 |
| 6 | 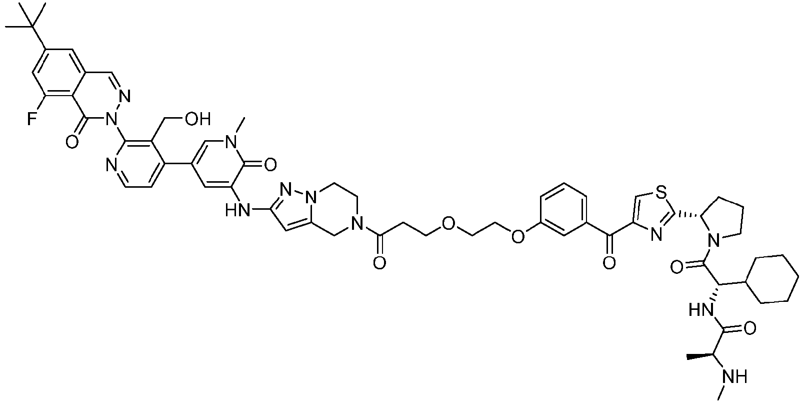 |
| 7 | 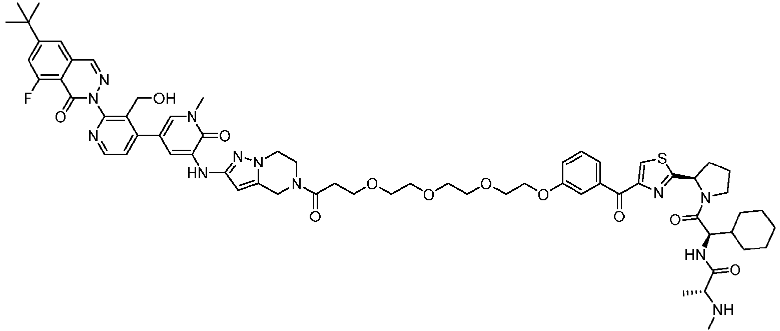 |
| 8 | 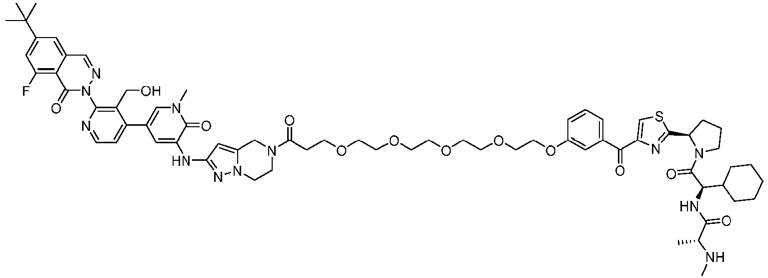 |
| 9 | 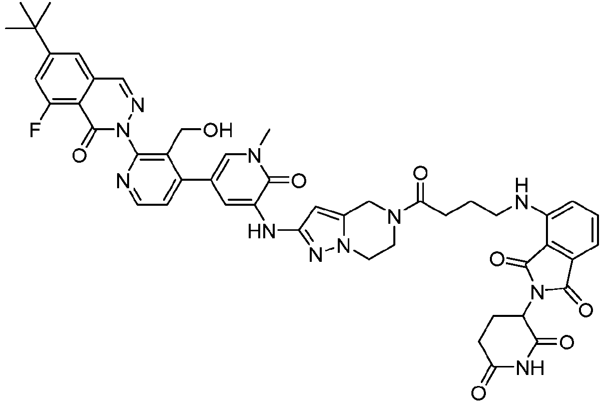 |
| 10 | 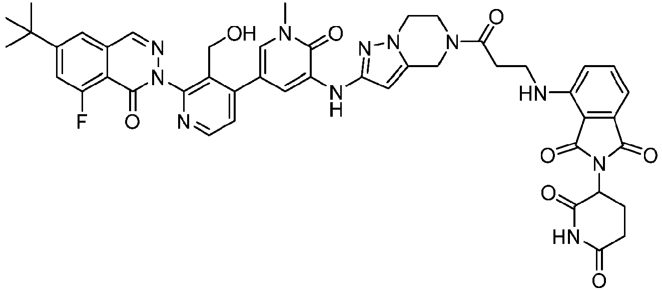 |
| 11 | 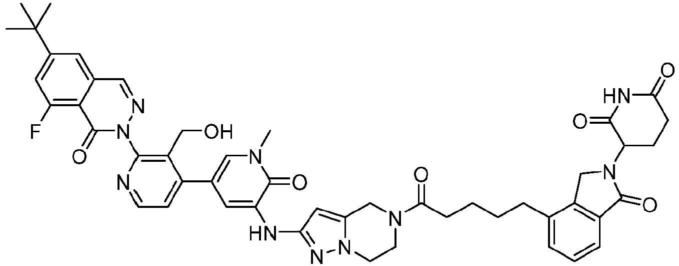 |
| 12 | 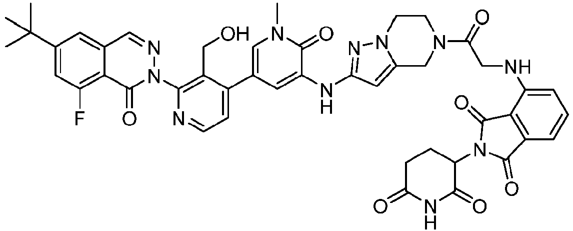 |
| 13 | 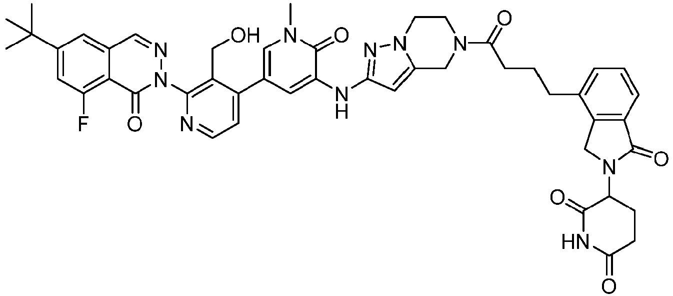 |
| 14 | 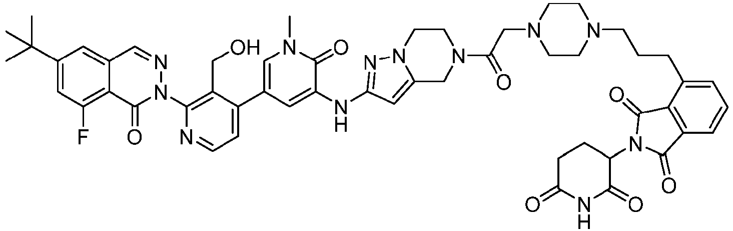 |
| 15 | 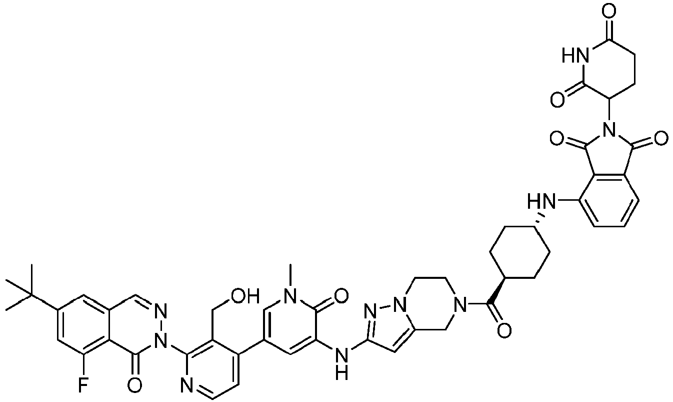 |
| 16 | 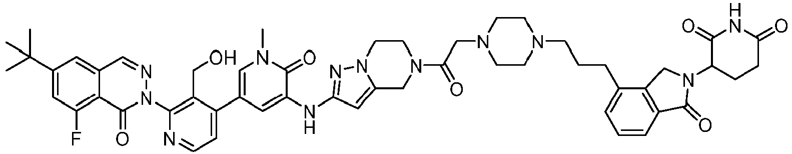 |
| 17 | 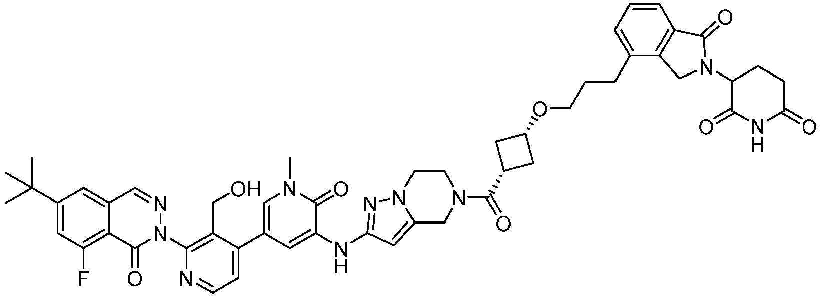 |
| 18 | 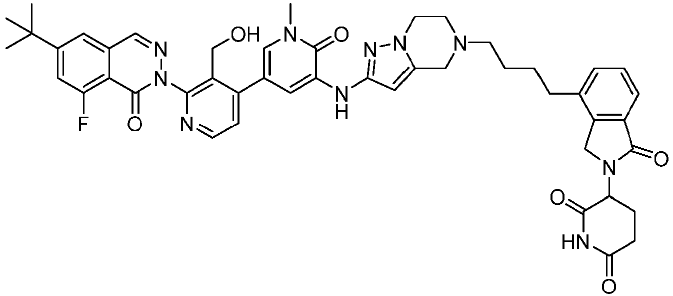 |
| 19 | 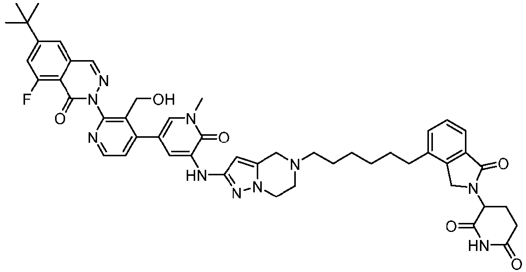 |
| 20 | 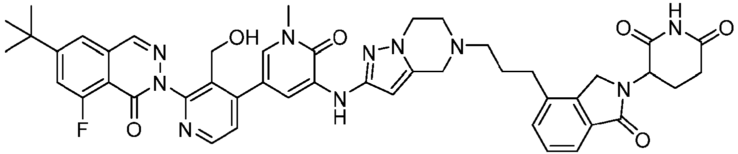 |
| 21 | 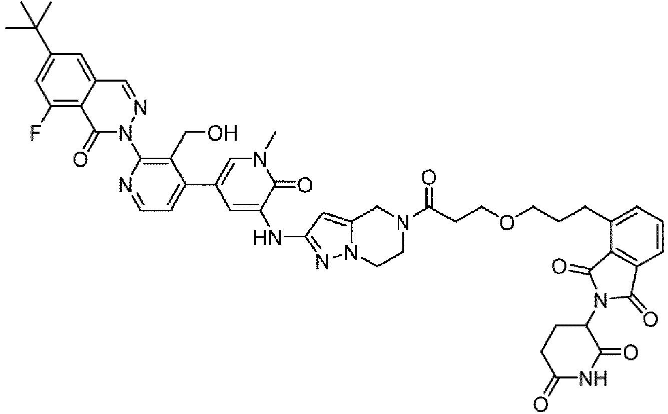 |
| 22 | 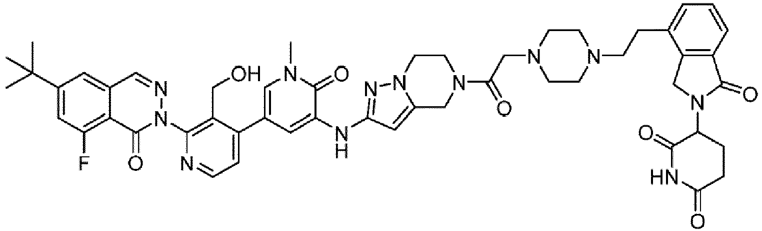 |
| 23 | 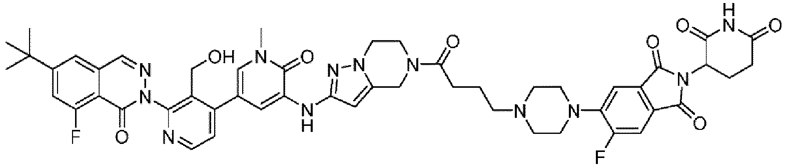 |
| 25 | 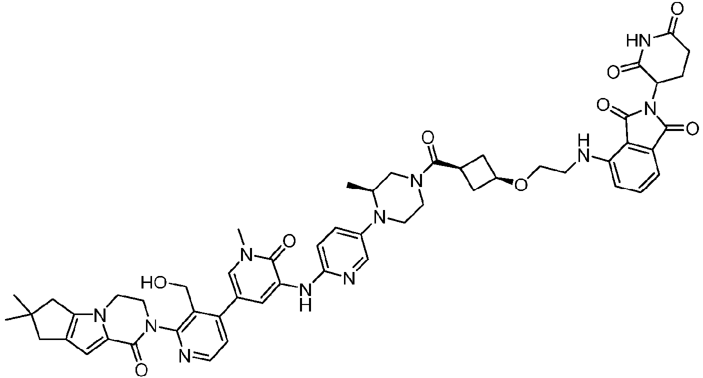 |
| 26 | 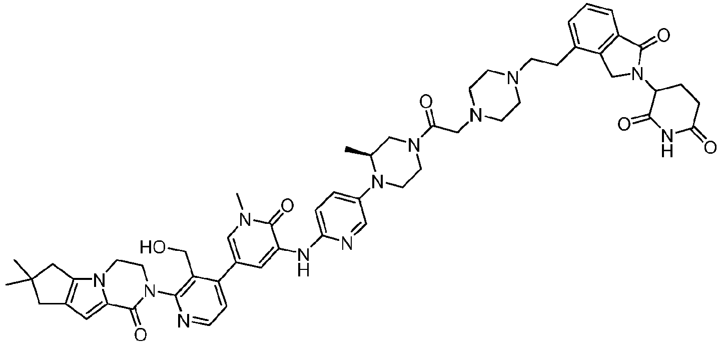 |
| 27 | 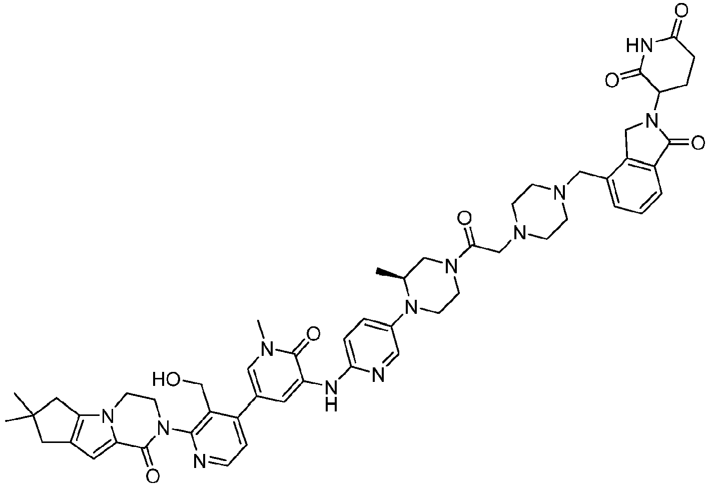 |
| 28 | 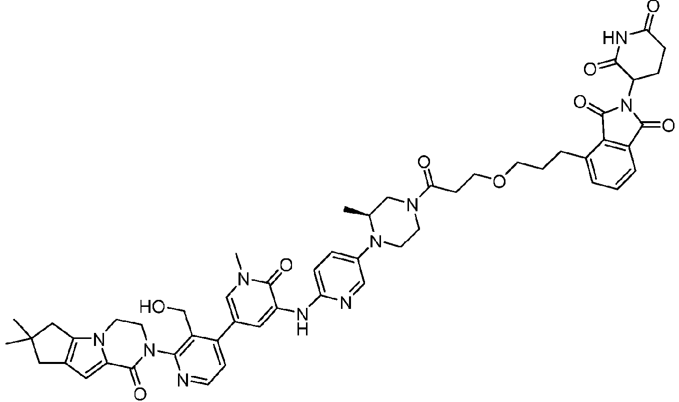 |
| 29 | 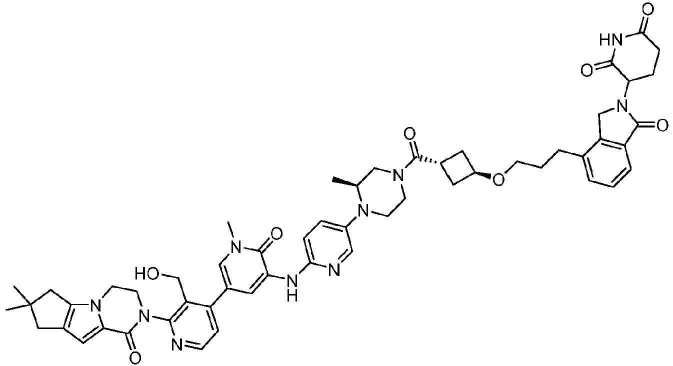 |
| 30 | 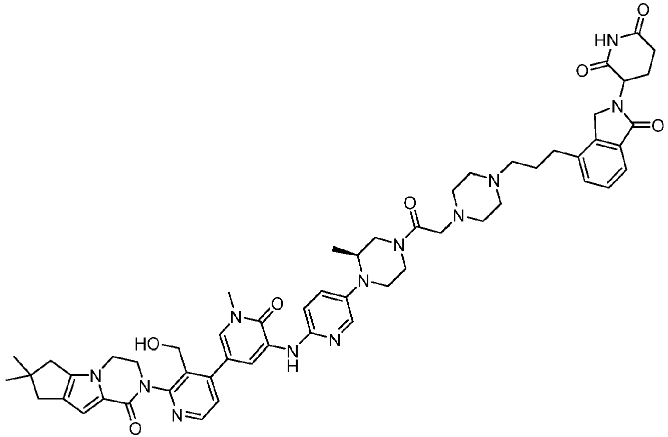 |
| 31 | 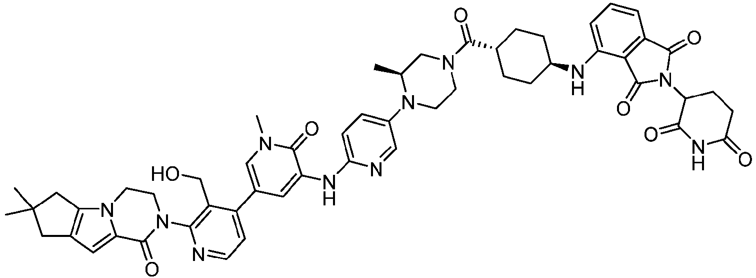 |
| 32 | 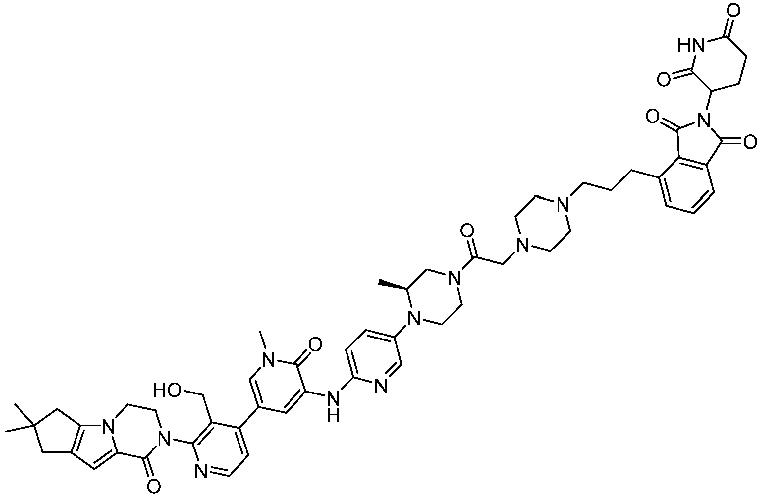 |
| 33 | 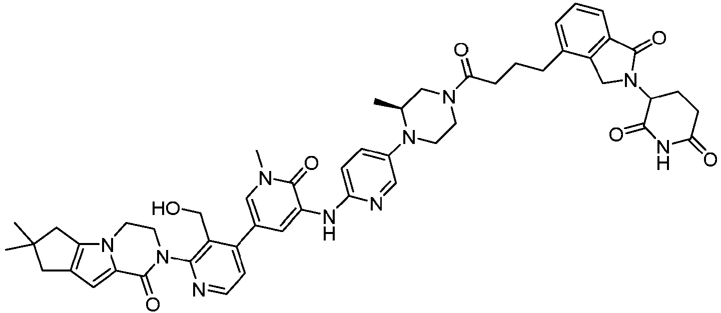 |
| 34 | 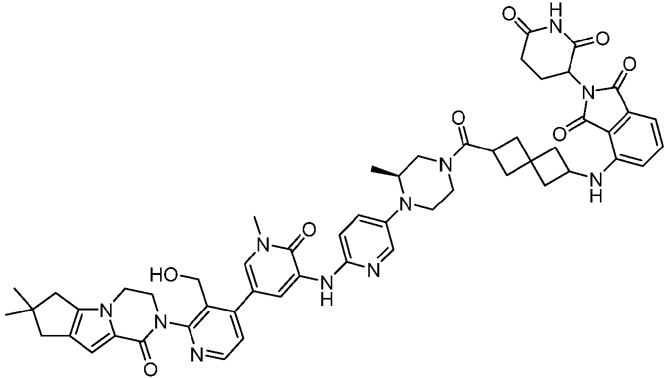 |
| 35 | 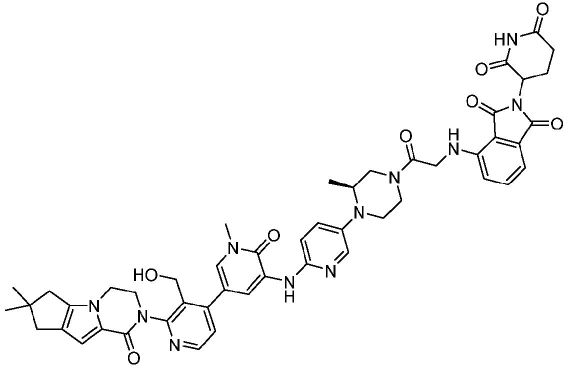 |
| 36 | 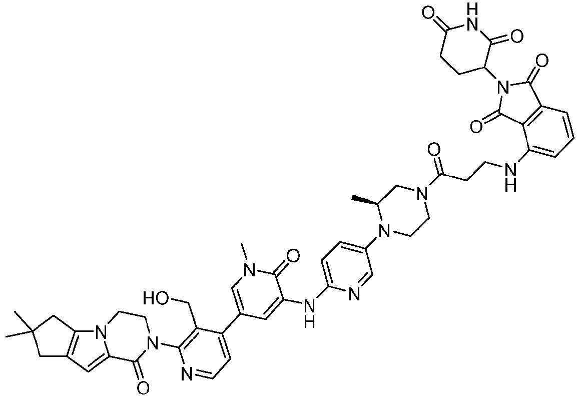 |
| 37 | 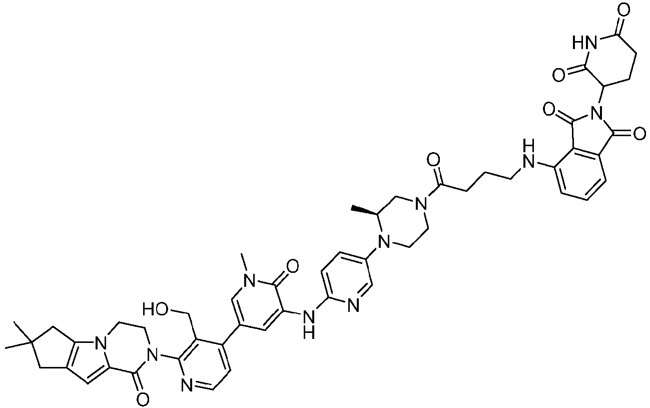 |
| 38 | 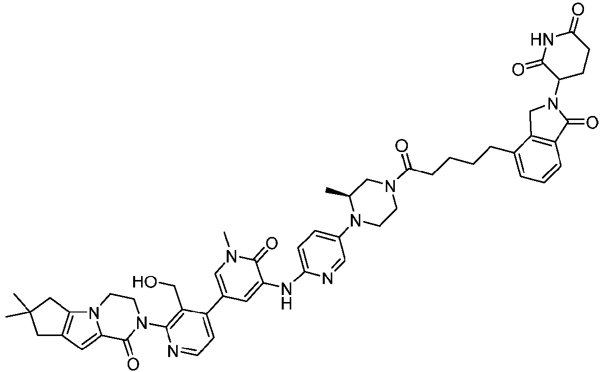 |
| 39 | 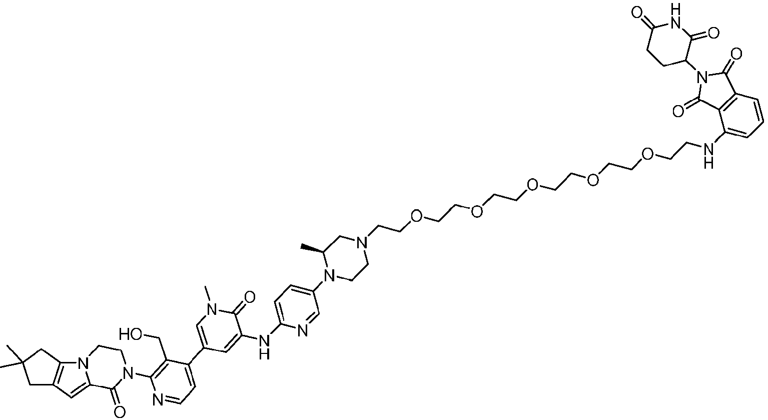 |
| 40 | 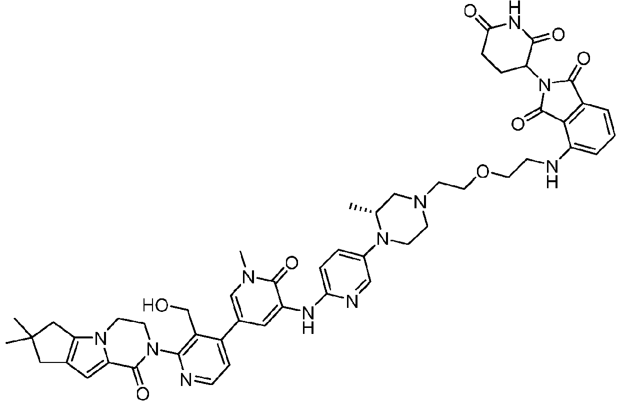 |
| 41 | 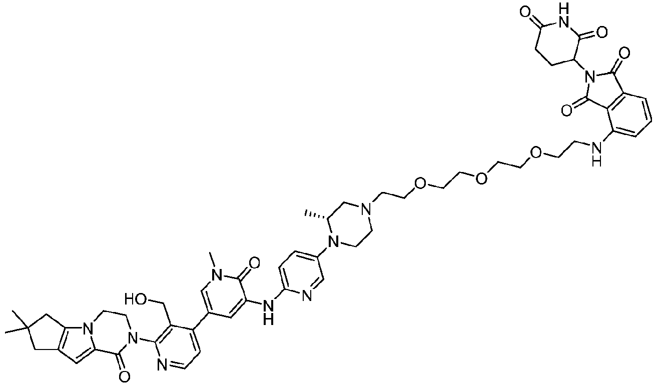 |
| 42 | 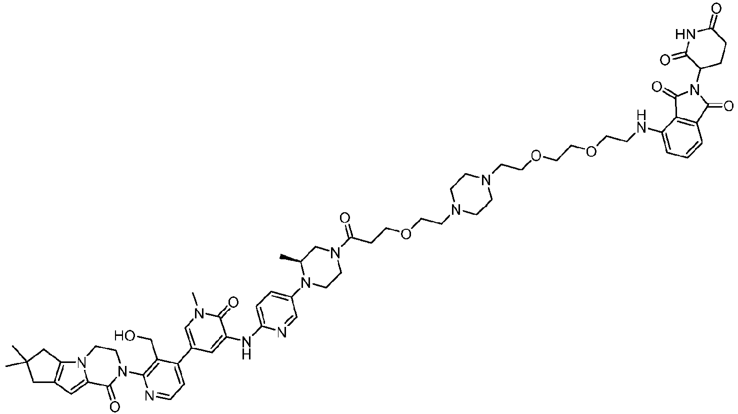 |
| 43 | 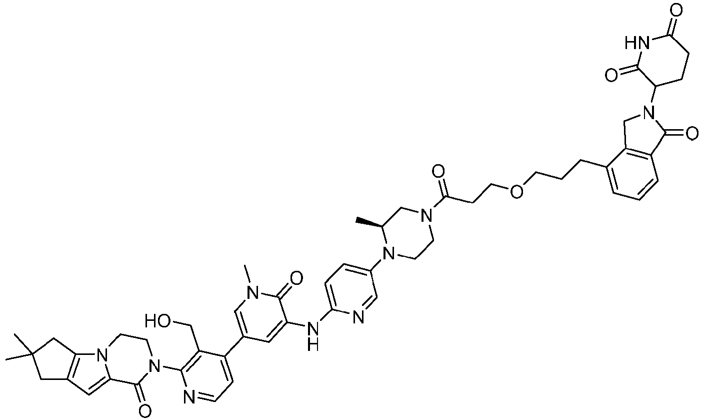 |
| 44 | 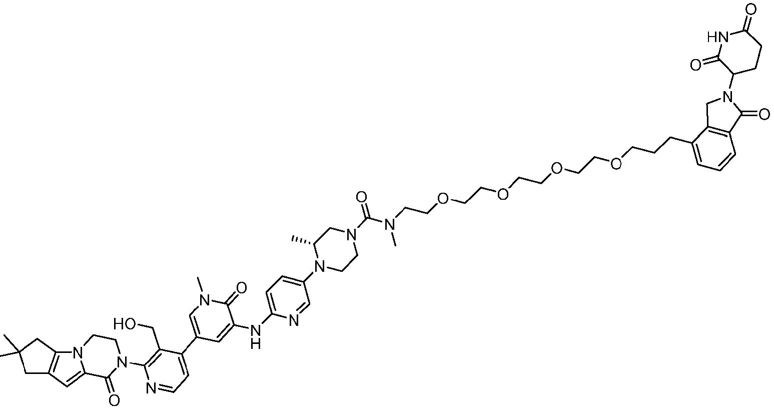 |
| 45 | 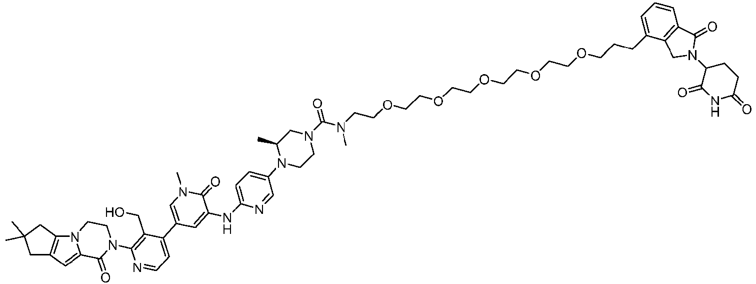 |
| 46 | 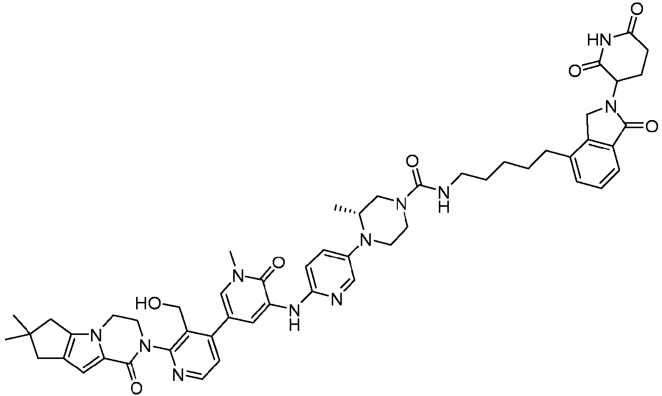 |
| 47 | 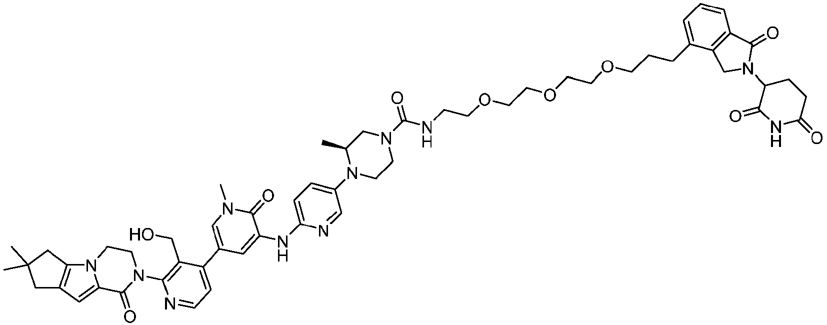 |
| 48 | 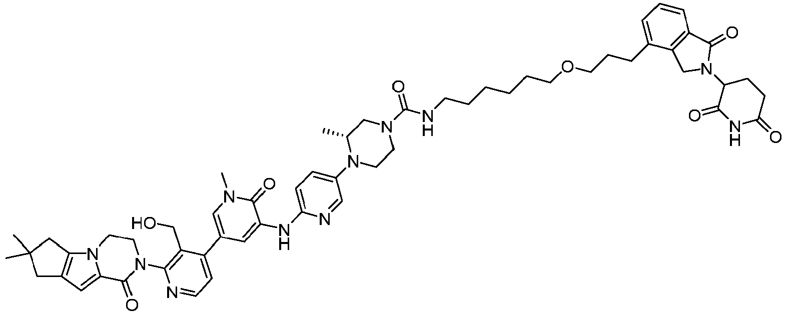 |
| 49 | 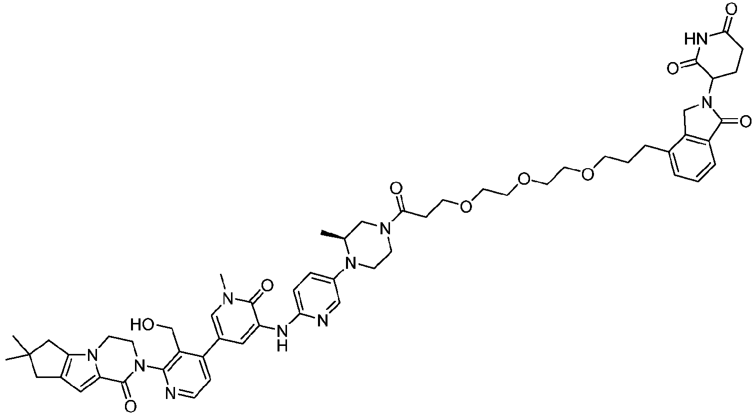 |
| 50 | 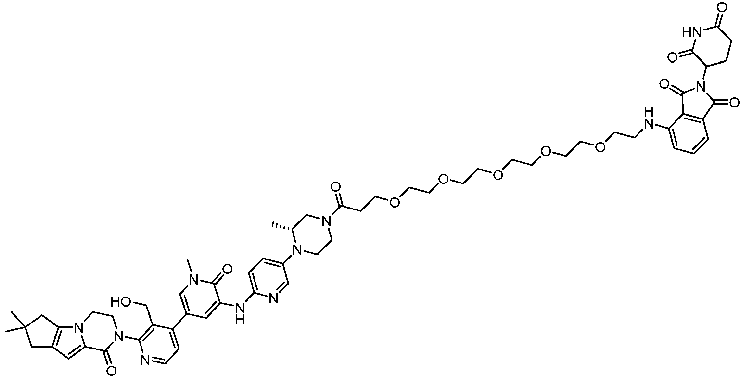 |
| 51 | 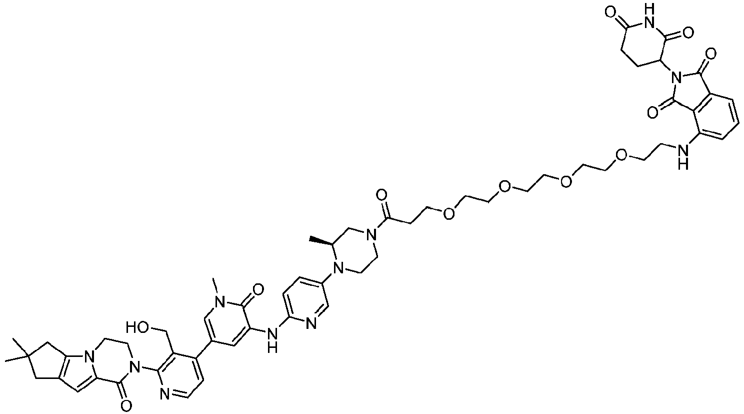 |
| 52 | 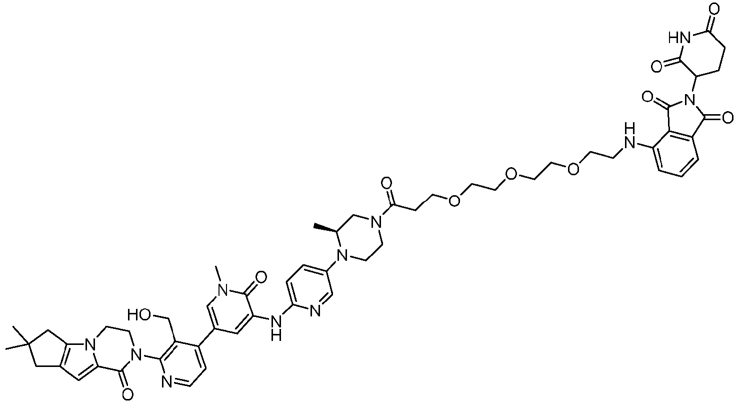 |
| 53 | 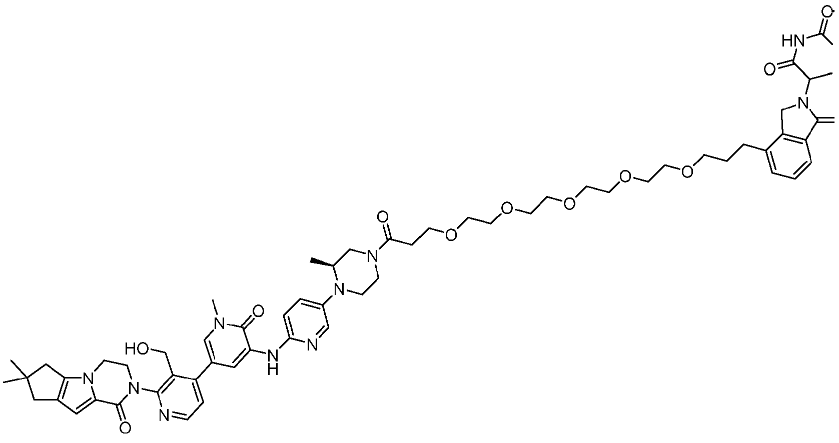 |
| 54 | 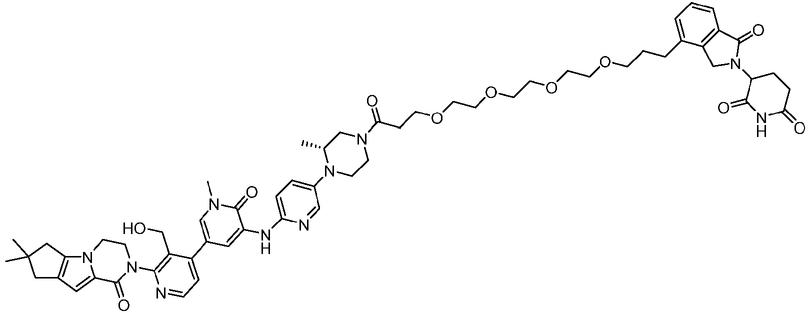 |
| 55 | 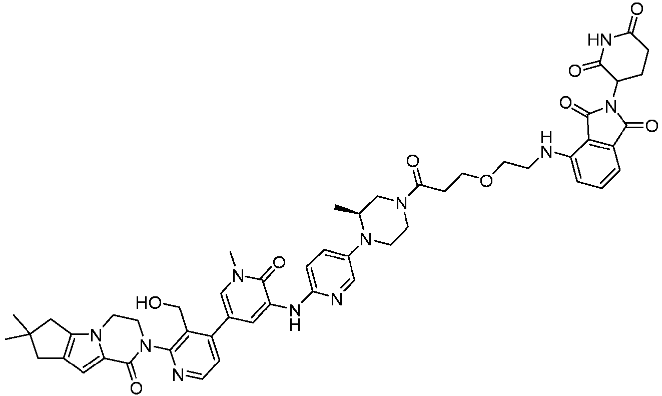 |
| 56 | 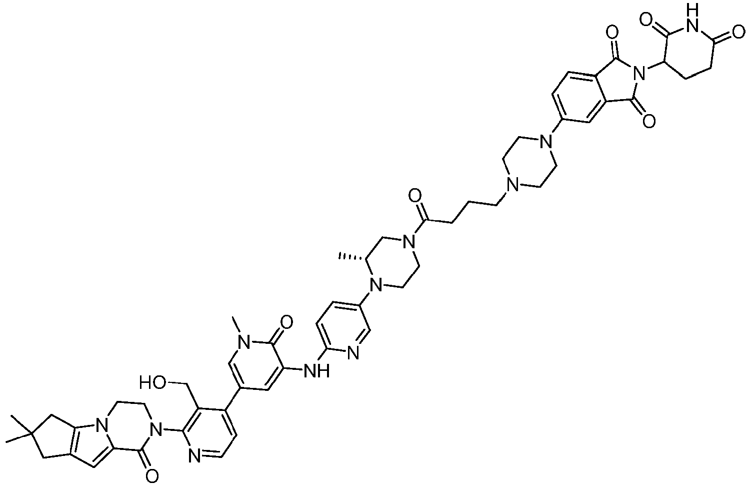 |
| 57 | 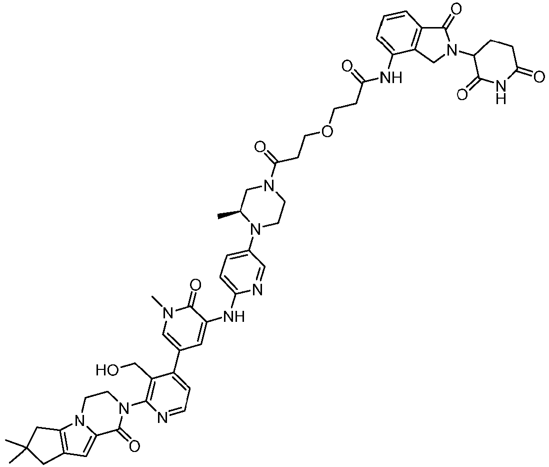 |
| 58 | 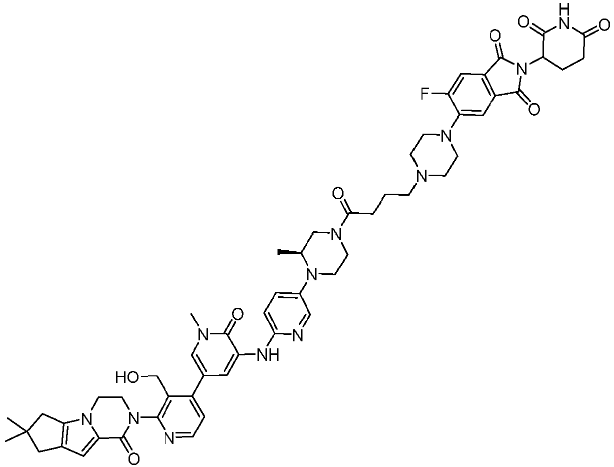 |

13. A compound of:
| | |
|---|---|
| 24 | 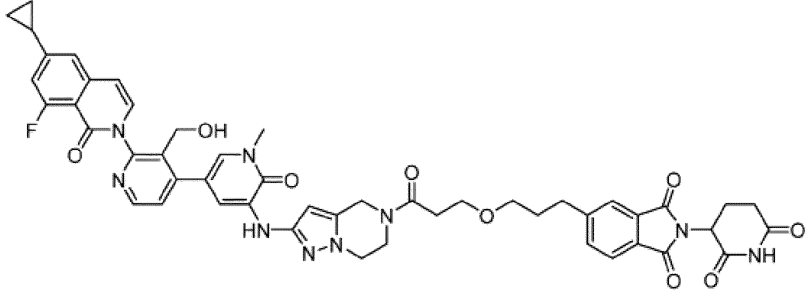 |
or a pharmaceutically acceptable salt thereof.

14. A pharmaceutical composition comprising a compound or pharmaceutically acceptable salt of a compound of any one of the preceding claims and a pharmaceutically acceptable carrier, vehicle, or adjuvant.

15. A compound or pharmaceutically acceptable salt of any one of claims 1-13 or a pharmaceutical composition of claim 14 for use in a method of treating a disease or disorder mediated by degrading Bruton's tyrosine kinase.

16. The compound or composition for use according to claim 15, wherein the disease or disorder is cancer or an autoimmune disease.

17. The compound or composition for use according to claim 16, wherein:
the cancer is a hematological cancer selected from myeloid leukemia (acute and chronic), acute lymphoblastic leukemia, chronic lymphocytic leukemia, myeloproliferative diseases, multiple myeloma, myelodysplastic syndrome, Hodgkin's disease, non-Hodgkin's lymphoma (malignant lymphoma) hairy cell, mantle cell lymphoma, Waldenström's macroglobulinemia, marginal zone lymphoma, and follicular lymphoma; or
the autoimmune disease is selected from uticaria, graft-versus-host disease, pemphigus vulgaris, achalasia, Addison's disease, Adult Still's disease, agammaglobulinemia, alopecia areata, amyloidosis, ankylosing spondylitis, anti-GBM/anti-TBM nephritis, antiphospholipid syndrome, autoimmune angioedema, autoimmune dysautonomia, autoimmune encephalomyelitis, autoimmune hepatitis, autoimmune inner ear disease (AIED), autoimmune myocarditis, autoimmune oophoritis, autoimmune orchitis, autoimmune pancreatitis, autoimmune retinopathy, axonal and neuronal neuropathy (AMAN), Baló disease, Behcet's disease, benign mucosal pemphigoid, bullous pemphigoid, Castleman disease (CD), Celiac disease, Chagas disease, chronic inflammatory demyelinating polyneuropathy (CIDP), chronic recurrent multifocal osteomyelitis (CRMO), Churg-Strauss Syndrome (CSS) or Eosinophilic Granulomatosis (EGPA), cicatricial pemphigoid, Cogan's syndrome, cold agglutinin disease, congenital heart block, coxsackie myocarditis, CREST syndrome, Crohn's disease, dermatitis herpetiformis, dermatomyositis, Devic's disease (neuromyelitis optica), discoid lupus, Dressler's syndrome, endometriosis, eosinophilic esophagitis (EoE), eosinophilic fasciitis, erythema nodosum, essential mixed cryoglobulinemia, evans syndrome, fibromyalgia, fibrosing alveolitis, giant cell arteritis (temporal arteritis), giant cell myocarditis, glomerulonephritis, goodpasture's syndrome, granulomatosis with polyangiitis, Graves' disease, Guillain-Barre syndrome, Hashimoto's thyroiditis, hemolytic anemia, Henoch-Schonlein purpura (HSP), herpes gestationis or pemphigoid gestationis (PG), hidradenitis suppurativa (HS) (Acne Inversa), hypogammalglobulinemia, IgA nephropathy, IgG4-related sclerosing disease, immune thrombocytopenic purpura (ITP), inclusion body myositis (IBM), interstitial cystitis (IC), juvenile arthritis, juvenile diabetes (Type 1 diabetes), juvenile myositis (JM), Kawasaki disease, Lambert-Eaton syndrome, leukocytoclastic vasculitis, lichen planus, lichen sclerosus, ligneous conjunctivitis, linear IgA disease (LAD), lupus, lyme disease chronic, Meniere's disease, microscopic polyangiitis (MPA), mixed connective tissue disease (MCTD), Mooren's ulcer, Mucha-Habermann disease, Multifocal Motor Neuropathy (MMN) or MMNCB, multiple sclerosis, myasthenia gravis, myositis, narcolepsy, neonatal lupus, neuromyelitis optica, neutropenia, ocular cicatricial pemphigoid, optic neuritis, palindromic rheumatism (PR), PANDAS, paraneoplastic cerebellar degeneration (PCD), paroxysmal nocturnal hemoglobinuria (PNH), Parry Romberg syndrome, pars planitis (peripheral uveitis), Parsonnage-Turner syndrome, pemphigus, peripheral neuropathy, perivenous encephalomyelitis, pernicious anemia (PA), POEMS syndrome, polyarteritis nodosa, polyglandular syndromes type I, II, III, polymyalgia rheumatica, polymyositis, postmyocardial infarction syndrome, postpericardiotomy syndrome, primary biliary cirrhosis, primary sclerosing cholangitis, progesterone dermatitis, psoriasis, psoriatic arthritis, pure red cell aplasia (PRCA), pyoderma gangrenosum, Raynaud's phenomenon, reactive Arthritis, reflex sympathetic dystrophy, relapsing polychondritis, restless legs syndrome (RLS), retroperitoneal fibrosis, rheumatic fever, rheumatoid arthritis, sarcoidosis, Schmidt syndrome, scleritis, scleroderma, Sjögren's syndrome, sperm and testicular autoimmunity, stiff person syndrome (SPS), subacute bacterial endocarditis (SBE), Susac's syndrome, sympathetic ophthalmia (SO), Takayasu's arteritis, temporal arteritis (giant cell arteritis), thrombocytopenic purpura (TTP), Tolosa-Hunt syndrome (THS), transverse myelitis, Type 1 diabetes, ulcerative colitis (UC), undifferentiated connective tissue disease (UCTD), uveitis, vasculitis, vitiligo, Vogt-Koyanagi-Harada Disease, and Wegener's granulomatosis (or Granulomatosis with Polyangiitis (GPA)).

## Patentansprüche

1. Verbindung der Formel (I): 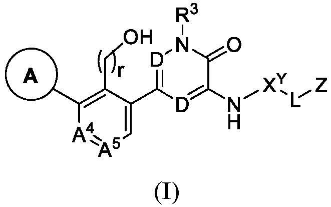
oder ein pharmazeutisch verträgliches Salz davon, wobei jedes von A⁴ und A⁵ unabhängig N oder C-R¹⁵ ist,
jedes R¹⁵ unabhängig -H oder ein optional substituiertes C₁₋₆-Alkyl ist; jedes D unabhängig N oder CH ist;
wobei Ring A wie folgt ist: 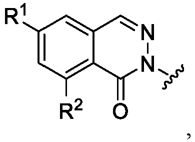 wobei jedes von R¹ und R² unabhängig Halogen, -H, -OH, -CN, -CF₃, -C₁₋₆-Alkyl oder ein 3-6-gliedriger Cycloaliphat ist; 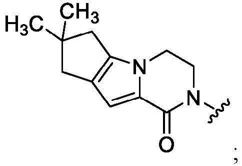 oder 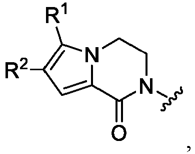 wobei jedes von R¹ und R² unabhängig Halogen, -H, -OH, -CN, -CF₃, -C₁₋₆-Alkyl oder ein 3-6-gliedriger Cycloaliphat ist, oder R¹ und R² zusammen mit den Atomen, an die sie angehängt sind, ein 5-6-gliedriges Cycloalkyl bilden, wobei das Cycloalkyl optional substituiert ist mit 1-2 von R⁶;
jedes von R¹ und R² unabhängig Halogen, -H, -OH, -CN, -CF₃, -C₁₋₆-Alkyl oder ein 3-6-gliedriger Cycloaliphat ist, oder
R¹ und R² zusammen mit den Atomen, an die sie angehängt sind, ein 5-6-gliedriges Cycloalkyl bilden, das an Ring C kondensiert ist, wobei das Cycloalkyl optional mit 1-2 von R⁶ substituiert ist, wobei jedes R⁶ unabhängig -H, Halogen oder C₁₋₃-Alkyl ist;
R³ C₁₋₆-Alkyl ist;
X^{Y} 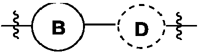
ist, wobei
Ring B Phenyl oder ein 5-6-gliedriges monocyclisches Heteroaryl mit 1-2 Stickstoffatomen oder ein 7-10-gliedriger kondensierter bicyclischer Heterocyclus mit 2-3 Stickstoffatomen ist, wobei zumindest einer der Ringe des bicyclischen Heterocyclus teilweise oder vollständig ungesättigt ist und Ring D fehlt oder ein optional substituierter 4-7-gliedriger Heterocyclus mit zumindest 1 Stickstoffatom und optional 2 zusätzlichen Heteroatomen unabhängig ausgewählt aus N, O oder S ist;
L eine zweiwertige C₁₋₂₀-Alkylgruppe ist, wobei eine oder mehrere Methyleneinheiten der C₁₋₂₀-Alkylgruppe optional und unabhängig durch -C(O)-, -C(S)-, -(O)CO-, -OC(O)-, -O-, -N(R')-, -S-, -N(R')C(O)-, zweiwertiges mono- oder bicyclisches Heterocycloalkyl, zweiwertiges mono- oder bicyclisches Cycloalkyl, zweiwertiges mono- oder bicyclisches Aryl oder zweiwertiges mono- oder bicyclisches Heteroaryl ersetzt sind, wobei jedes von dem zweiwertigen Heterocycloalkyl, Cycloalkyl, Aryl und Heteroaryl optional substituiert ist; und
Z wie folgt ist: 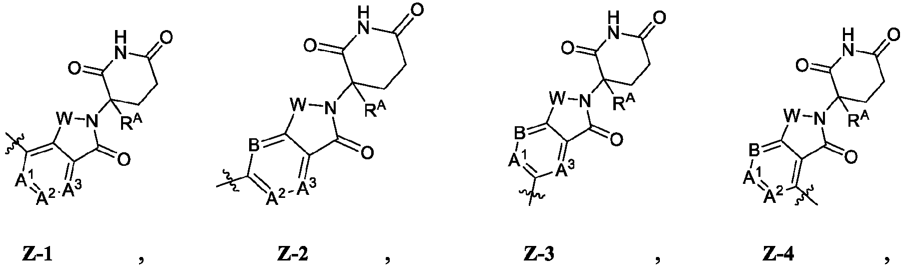 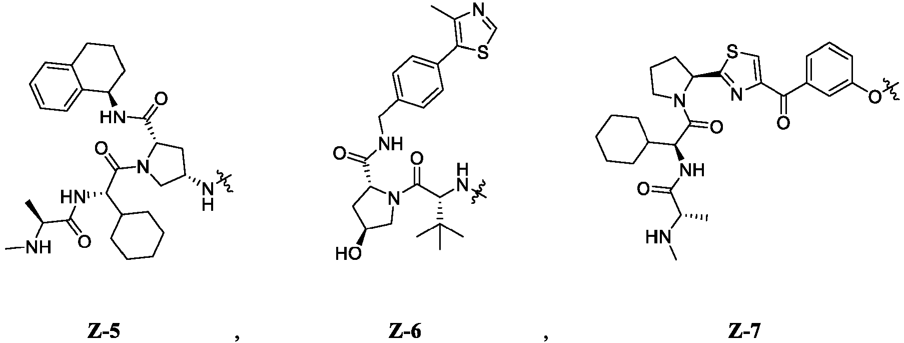
oder 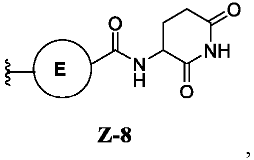 wobei
Ring E Phenyl oder ein 5-6-gliedriges Heteroaryl mit zumindest 1 Heteroatom unabhängig ausgewählt aus N oder S ist;
jedes von A¹, A² und A³ unabhängig -C(R^{B})= oder -N= ist;
B -C(R^{c})= oder -N= ist;
W -C(R^{D})₂- oder -C(O)- ist;
R^{A} -H, -CH₃ oder -F ist;
jedes R^{B} unabhängig -H, Halogen, -OH, -C₁₋₄-Alkyl oder -C₁₋₄-Alkoxy ist;
R^{C} -H, Halogen oder C₁₋₄-Alkyl ist; und
jedes R^{D} unabhängig -H oder -C₁₋₃-Alkyl ist; und
r 1, 2 oder 3 ist.

2. Verbindung oder pharmazeutisch verträgliches Salz nach Anspruch 1, wobei:
- Z 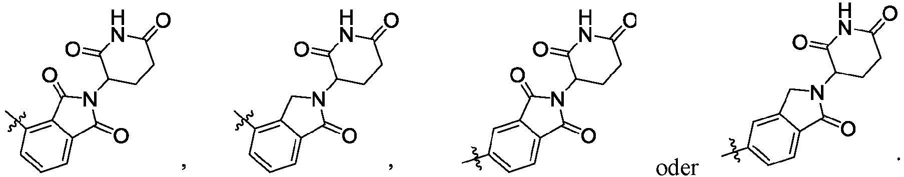 ist; oder Z 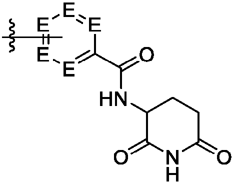 ist, wobei eine Instanz von E 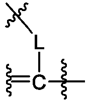 ist und die übrigen Instanzen von E jeweils unabhängig =CH- oder =N- sind; oder Z 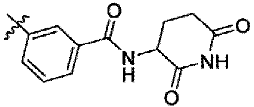 ist;
- ein D CH ist und das andere D N ist; oder jedes D CH ist;
- zumindest eines von A⁴ und A⁵ N ist; oder A⁴ N ist, A⁵ CR¹⁵ ist und R¹⁵ -H oder C₁₋₃-Alkyl ist;
- R¹⁵ -H oder Methyl ist;
- X^{Y} wie folgt ist: 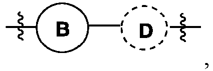 Ring B 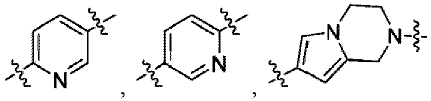 oder 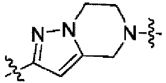 ist, und Ring D fehlt; 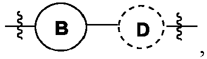 Ring B 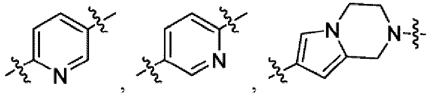 oder 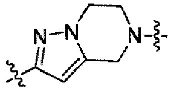 ist, und Ring D 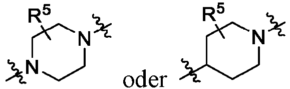 ist, und R⁵ -H oder C₁₋₃-Alkyl ist; oder 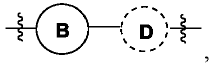 Ring B 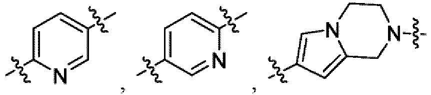 oder 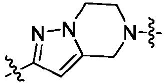 ist, und Ring D 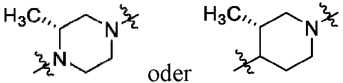 ist;
- R³ ein C₁₋₃-Alkyl ist; und/oder
- r 1 oder 2 ist.

3. Verbindung oder pharmazeutisch verträgliches Salz nach Anspruch 1, wobei die Verbindung von Formel (I) wie folgt ist:
- eine Verbindung von Formel (I-A) 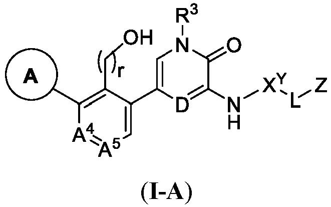 oder ein pharmazeutisch verträgliches Salz davon;
- eine Verbindung von Formel (I-A1) 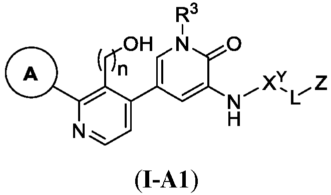 oder ein pharmazeutisch verträgliches Salz davon;
- eine Verbindung von Formel (I-A2) 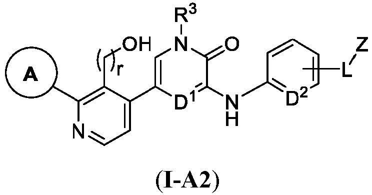
oder ein pharmazeutisch verträgliches Salz davon, wobei
D¹ CH oder N ist; D² CH oder N ist, und zumindest eines von D¹ und D² N ist; wobei optional D¹ =CH- ist und D² =N- ist; oder D¹ =N- ist und D² =CH- ist; oder
- eine Verbindung von Formel (I-A3) 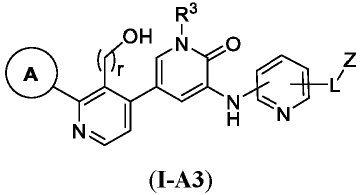 oder ein pharmazeutisch verträgliches Salz davon;
- eine Verbindung von Formel (I-B1), eine Verbindung von Formel (I-B2) oder eine Verbindung von Formel (I-B3) 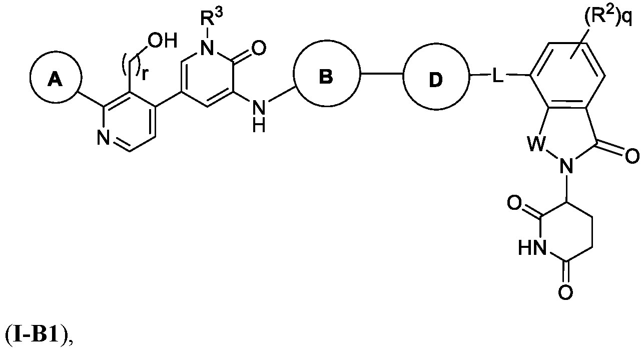 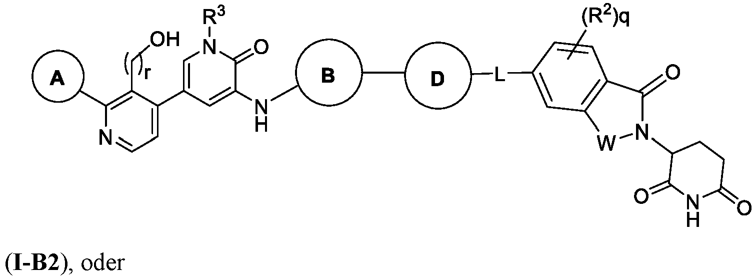 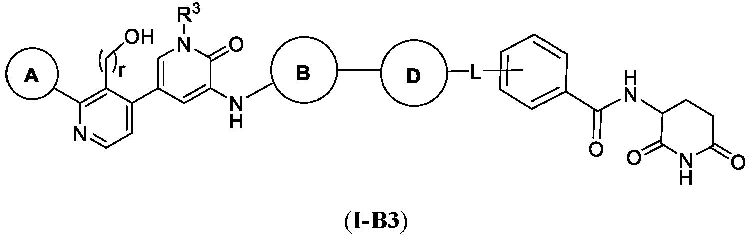
oder ein pharmazeutisch verträgliches Salz davon;
wobei optional
Ring B Phenyl, Pyridinyl oder Pyrimidinyl ist; und/oder Ring D ein optional substituierter 4-7-gliedriger Heterocyclus mit 1-2 Stickstoffatomen ist oder Ring D ein optional substituierter 5-6-gliedriger Heterocyclus mit 1-2 Stickstoffatomen ist.

4. Verbindung oder pharmazeutisch verträgliches Salz nach einem der Ansprüche 1-3, wobei L -Y¹-Y²-Y³-Y⁴-Y⁵-Y⁶- ist, wobei
Y¹ wie folgt ist:
-C(O)-, -C(O)-N(R)-, -(CH₂-CH₂-O)ₘ-, -(O-CH₂-CH₂)ₘ- oder -C₁₋₆-Alkyl-;
-C(O)- oder -C(O)-N(R)-;
-C(O)-, Y² -(CH₂-CH₂-O)ₙ-, -(O-CH₂-CH₂)ₙ-, -C₁₋₄-Alkyl-, ein 3-6-gliedriges monocyclisches Cycloalkyl oder ein 7-11-gliedriges kondensiertes oder Spiro-Bicycloalkyl ist, und n 1 oder 2 ist; oder
-(CH₂-CH₂-O)ₘ-, -(O-CH₂-CH₂)ₘ- oder-C₁₋₆-Alkyl-;
Y² wie folgt ist:
eine Bindung, -C(O)-, -O-, -(CH₂-CH₂-O)ₙ-, -(O-CH₂-CH₂)ₙ-, -C₁₋₄-Alkyl-, ein optional substituiertes 3-6-gliedriges monocyclisches Cycloalkyl oder ein optional substituiertes 7-11-gliedriges kondensiertes oder Spiro-Bicycloalkyl;
-(CH₂-CH₂-O)ₙ-, -(O-CH₂-CH₂)ₙ-, -CH₂-, -CH₂-CH₂-, -CH₂-CH₂-CH₂-, Cyclopentyl, Cyclohexyl oder ein 7-11-gliedriges Spiro-Bicycloalkyl, und n 1 oder 2 ist;
-C(O)-, -O-, -(CH₂-CH₂-O)ₙ-, -(O-CH₂-CH₂)n-, 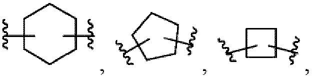 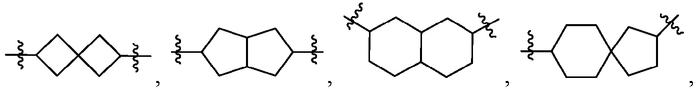 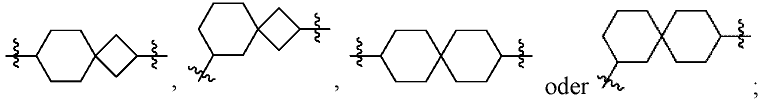 oder
-C(O)-, -O-, -(CH₂-CH₂-O)ₙ- oder -(O-CH₂-CH₂)ₙ-, und n 2 oder 3 ist;
Y³ wie folgt ist:
eine Bindung, -O-, -N(R)-, -C₁₋₄-Alkyl-, -(CH₂-CH₂-O)_{P}-, -(O-CH₂-CH₂)_{P}-, -(CH₂-CH₂-N(R))-, Phenyl, ein 5-6-gliedriges monocyclisches Heteroaryl mit 1-2 Heteroatomen unabhängig ausgewählt aus N und S, ein optional substituiertes 3-6-gliedriges Cycloalkyl oder ein optional substituierter 4-6-gliedriger Heterocyclus mit 1-3 Heteroatomen unabhängig ausgewählt aus N, O oder S;
eine Bindung, -O-, -N(R)-, -C₁₋₄-Alkyl-, -(CH₂-CH₂-N(R))-, Phenyl, ein 5-6-gliedriges monocyclisches Heteroaryl mit 1-2 Heteroatomen unabhängig ausgewählt aus N und S, ein optional substituiertes 3-6-gliedriges Cycloalkyl oder ein optional substituierter 4-6-gliedriger Heterocyclus mit 1-3 Heteroatomen unabhängig ausgewählt aus N, O oder S;
eine Bindung, -N(R)-, -O-, -CH₂-, -CH₂-CH₂-, -CH₂-CH₂-CH₂-, -(CH₂-CH₂-N(R))-, Phenyl, 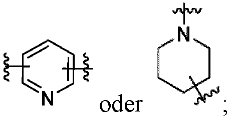
eine Bindung, -O-, -N(R)-, -(CH₂-CH₂-O)_{P}-, -(O-CH₂-CH₂)ₚ-, -(CH₂-CH₂-N(R))-, Phenyl, 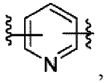 ein optional substituiertes 3-6-gliedriges monocyclisches Cycloalkyl oder ein optional substituierter 4-6-gliedriger monocyclischer Heterocyclus mit 1-3 Heteroatomen unabhängig ausgewählt aus N, O oder S; oder
eine Bindung, -O-, -N(R)-, -(CH₂-CH₂-O)ₚ- oder -(O-CH₂-CH₂)ₚ-, und p 1 oder 2 ist; oder 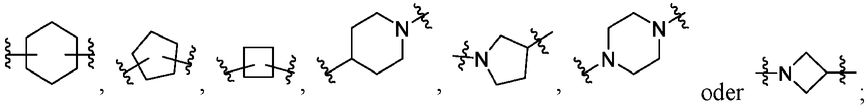
Y⁴ wie folgt ist:
eine Bindung, -C(O)-, -N(R)-, -C(O)N(R)-, -N(R)C(O)-, -C₁₋₄-Alkyl-, -(CH₂-CH₂-O)_{q}-, - (O-CH₂-CH₂)_{q}-, -(CH₂-CH₂-N(R))-, ein optional substituiertes 3-6-gliedriges Cycloalkyl oder ein optional substituierter 4-6-gliedriger Heterocyclus mit 1-3 Heteroatomen unabhängig ausgewählt aus N, O oder S;
eine Bindung, -C(O)-, -N(R)-, -C(O)N(R)-, -N(R)C(O)-, -C₁₋₄-Alkyl-, -(CH₂-CH₂-N(R))-, ein optional substituiertes 3-6-gliedriges Cycloalkyl oder ein optional substituierter 4-6-gliedriger Heterocyclus mit 1-3 Heteroatomen unabhängig ausgewählt aus N, O oder S;
eine Bindung, -C(O)-, -N(R)-, -C(O)N(R)-, -N(R)C(O)-, -CH₂-, -CH₂-CH₂-, 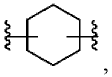 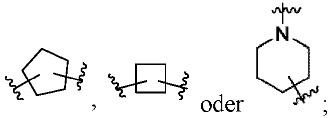
eine Bindung, -N(R)-, -C(O)N(R)-, -N(R)C(O)-, -C₁₋₄-Alkyl-, -(CH₂-CH₂-N(R))-, ein optional substituiertes 3-6-gliedriges Cycloalkyl oder ein optional substituierter 4-6-gliedriger Heterocyclus mit 1-3 Heteroatomen unabhängig ausgewählt aus N, O oder
S; oder
eine Bindung, -N(H)-, -N(CH₃)-, -N(H)C(O)-, -CH₂-, -CH₂-CH₂-, -CH₂-CH₂-CH₂-, -(CH₂-CH₂-N(H))-, 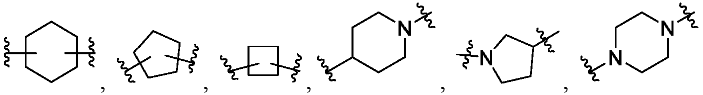 oder 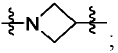
Y⁵ wie folgt ist:
eine Bindung, -N(R)-, -C(O)-, -C₁₋₄-Alkyl-, -(CH₂-CH₂-O)ₛ-, -(O-CH₂-CH₂)ₛ- oder ein 5-6-gliedriges Heteroaryl mit 1-3 Heteroatomen unabhängig ausgewählt aus N oder S;
eine Bindung, -N(R)-, -C 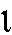 -₄-Alkyl-, -(CH₂-CH₂-O)ₛ-, -(O-CH₂-CH₂)ₛ- oder ein 5-6-gliedriges Heteroaryl mit 1-3 Heteroatomen unabhängig ausgewählt aus N oder S;
eine Bindung, -N(R)-, -CH₂-, -CH₂-CH₂-, -CH₂-CH₂-CH₂-, -(CH₂-CH₂-O)ₛ-, -(O-CH₂-CH₂)ₛ- oder 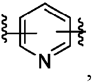 und s 1 oder 2 ist;
eine Bindung, -C(O)-, -C₁₋₄-Alkyl-, -(CH₂-CH₂-O)ₛ-, -(O-CH₂-CH₂)ₛ- oder ein 5-6-gliedriges Heteroaryl mit 1-3 Heteroatomen unabhängig ausgewählt aus N oder S; oder
eine Bindung, -C(O)-, -CH₂-, -CH₂-CH₂-, -CH₂-CH₂-CH₂-, -(CH₂-CH₂-O)ₛ-, -(O-CH₂-CH₂)ₛ- oder 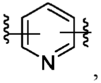 und s 1 oder 2 ist; und
Y⁶ wie folgt ist:
eine Bindung oder -(CH₂-CH₂-N(R))-; oder eine Bindung oder -CH₂-CH₂-N(H)-jedes von m und n unabhängig 1, 2, 3, 4 oder 5 ist;
jedes von p, q und s unabhängig 1, 2 oder 3 ist; und
R -H oder -C₁₋₃-Alkyl ist.

5. Verbindung oder pharmazeutisch verträgliches Salz nach Anspruch 1, wobei die Verbindung von Formel (I) eine Verbindung von Formel **(II)** 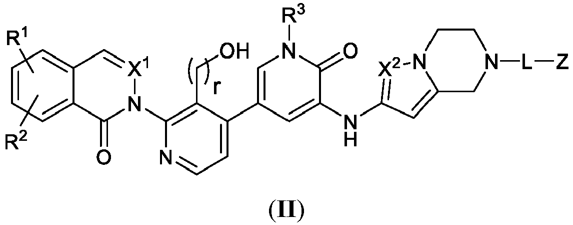
oder ein pharmazeutisch verträgliches Salz davon;
eine Verbindung von Formel **(II-A)** 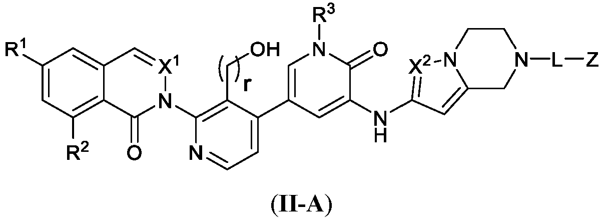
oder ein pharmazeutisch verträgliches Salz davon; oder
eine Verbindung von Formel **(II-B)** 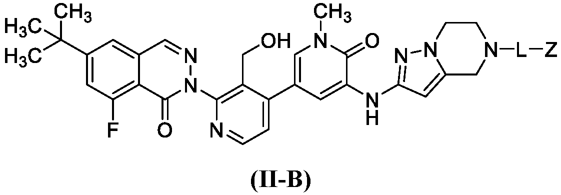
oder ein pharmazeutisch verträgliches Salz davon ist;
wobei
R¹ C₁₋₆-Alkyl, Hydroxyl, Halogen, Cyano oder ein 3-6-gliedriger Cycloaliphat ist;
R² C₁₋₆-Alkyl, Hydroxyl, Halogen, Cyano oder ein 3-6-gliedriger Cycloaliphat ist; oder R² C₁₋₆-Alkyl, Hydroxyl, Halogen oder Cyano ist;
R³ C₁₋₆-Alkyl ist;
X¹ =N- ist;
X² =CH- oder =N- ist;
r 1, 2 oder 3 ist;
L eine zweiwertige C₁₋₂₀-Alkylgruppe ist, optional substituiert mit C₁₋₆-Alkyl, Acyl, Oxo, Halogen oder Alkoxy, wobei eine oder mehrere Methyleneinheiten der C₁₋₂₀-Alkylgruppe optional und unabhängig ersetzt sind durch -CO-, -CS-, -CONH-, -CONHNH-, -CO₂-, -OCO-, - NHCO₂-, -O-, -NHCONH-, -OCONH-, -NHNH-, -NHCO-, -S-, -S(O)-, -S(O)₂-, -NH-, -S(O)₂NH-, - NHS(O)₂-, -NHS(O)₂NH-, zweiwertigen mono- oder bicyclischen Heterocyclus, zweiwertiges mono- oder bicyclisches Cycloalkyl, zweiwertiges mono- oder bicyclisches Aryl oder zweiwertiges mono- oder bicyclisches Heteroaryl, wobei der Heterocyclus und das Heteroaryl von L 1-3 Heteroatome unabhängig ausgewählt aus N, O oder S aufweist; und
Z wie folgt ist: 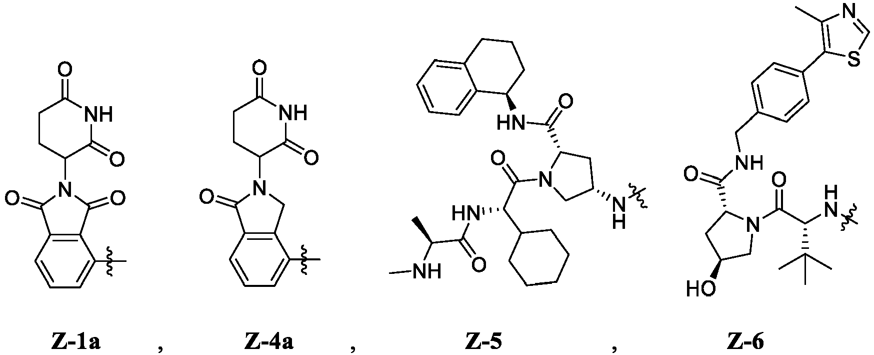
oder

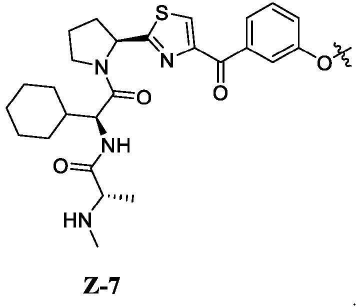

6. Verbindung oder pharmazeutisch verträgliches Salz nach Anspruch 5, wobei:
- R¹ C₁₋₆-Alkyl ist, wobei optional R¹ Methyl, Ethyl, Propyl, iso-Propyl, sec-Butyl oder *tert-*Butyl ist, wobei bevorzugt R¹ tert-Butyl ist; oder R¹ 3-6-gliedriger Cycloaliphat ist, wobei optional R¹ Cyclopropyl, Cyclopentyl oder Cyclohexyl ist;
- R² Halogen ist, wobei optional R² -F ist;
- R³ Methyl, Ethyl, Propyl, iso-Propyl, sec-Butyl oder tert-Butyl ist, wobei optional R³ Methyl ist;
- n 1 ist;
- zumindest eines von X¹ und X² =N- ist; wobei optional beides von X¹ und X² =N-sind; und/oder
- L eine zweiwertige C₁₋₂₀-Alkylgruppe ist, wobei eine oder mehrere Methyleneinheiten der C₁₋₂₀-Alkylgruppe optional und unabhängig ersetzt sind durch -CO-, -CS-, -OCO-, -O-, -NHCO-, -S-, -NH-, zweiwertigen mono- oder bicyclischen Heterocyclus, zweiwertiges mono- oder bicyclisches Cycloalkyl, zweiwertiges mono- oder bicyclisches Aryl oder zweiwertiges mono- oder bicyclisches Heteroaryl, wobei der Heterocyclus und das Heteroaryl 1-3 Heteroatome unabhängig ausgewählt aus N, O oder S aufweisen; oder wobei L eine zweiwertige C₁₋₂₀-Alkylgruppe ist, wobei eine oder mehrere Methyleneinheiten der C₁₋₂₀-Alkylgruppe optional und unabhängig ersetzt sind durch -CO-, -O-, -NH-, zweiwertiges mono- oder bicyclisches Heterocycloalkyl mit 1-3 Heteroatomen unabhängig ausgewählt aus N, O oder S, oder zweiwertiges mono- oder bicyclisches Cycloalkyl; oder wobei L eine zweiwertige C₁₋₁₀-Alkylgruppe ist, wobei eine oder mehrere Methyleneinheiten der C₁₋₁₀-Alkylgruppe optional und unabhängig ersetzt sind durch -CO-, -OCO-, -O-, -NHCO-, -NH-, zweiwertigen mono- oder bicyclischen Heterocyclus, zweiwertiges mono- oder bicyclisches Cycloalkyl, zweiwertiges mono- oder bicyclisches Aryl oder zweiwertiges mono- oder bicyclisches Heteroaryl, wobei der Heterocyclus und das Heteroaryl 1-3 Heteroatome unabhängig ausgewählt aus N, O oder S aufweisen.

7. Verbindung oder pharmazeutisch verträgliches Salz nach Anspruch 5 oder Anspruch 6, wobei L -Y¹-Y²-Y³-Y⁴-Y⁵- ist, wobei
Y¹ wie folgt ist:
-C(O)- oder -C₁₋₆-Alkyl-, oder
-C(O)- oder -C₄₋₆-Alkyl-;
Y² wie folgt ist:
eine Bindung, -O-, -(CH₂-CH₂-O)ₙ-, -(O-CH₂-CH₂)ₙ-, -C₁₋₄-Alkyl-, 3-6-gliedriges Cycloalkyl; oder
-(CH₂-CH₂-O)ₙ-, -(O-CH₂-CH₂)ₙ- oder -C₁₋₄-Alkyl-;
Y³ wie folgt ist:
eine Bindung, -O-, -N(R)-, -C₁₋₄-Alkyl-, -(CH₂-CH₂-O)ₚ-, -(O-CH₂-CH₂)ₚ-, -(CH₂-CH₂-N(R))-, Phenyl, 3-6-gliedriges Cycloalkyl oder 4-6-gliedriger Heterocyclus mit 1-3 Heteroatomen unabhängig ausgewählt aus N, O oder S; oder
eine Bindung, -O-, -C₁₋₄-Alkyl-, -(CH₂-CH₂-N(R))-, 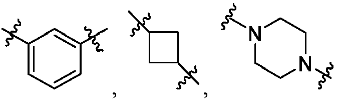 oder 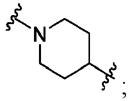
Y⁴ wie folgt ist:
eine Bindung, -C(O)-, -C(O)N(R)-, -C(O)N(R)-, -C₁₋₄-Alkyl-, -(CH₂-CH₂-O)_{q}-, -(O-CH₂-CH₂)_{q}-, -(CH₂-CH₂-N(R))-, 3-6-gliedriges Cycloalkyl oder 4-6-gliedriger Heterocyclus mit 1-3 Heteroatomen unabhängig ausgewählt aus N, O oder S; oder
eine Bindung, -C(O)-, -C(O)N(R)-, -C(O)N(R)- oder -C₁₋₄-Alkyl-;
Y⁵ wie folgt ist:
eine Bindung, -N(R)-, -C(O)-, -C₁₋₄-Alkyl- oder ein 5-6-gliedriges Heteroaryl mit 1-3 Heteroatomen unabhängig ausgewählt aus N, O oder S;
n 1, 2, 3, 4 oder 5 ist;
p 1, 2 oder 3 ist;
q 1, 2 oder 3 ist; und
R -H oder -C₁₋₃-Alkyl oder
eine Bindung, -N(R)-, -C(O)-, -C₁₋₄-Alkyl- oder 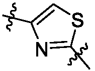 ist.

8. Verbindung oder pharmazeutisch verträgliches Salz nach einem der Ansprüche 4-7, wobei L ausgewählt ist aus Methylen, Ethylen, n-Propylen, n-Butylen, n-Pentylen, n-Hexylen, 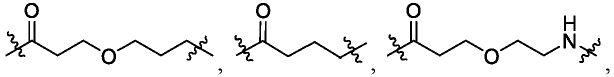 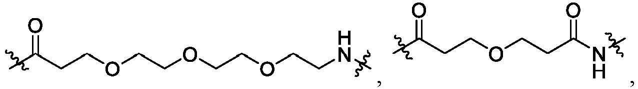 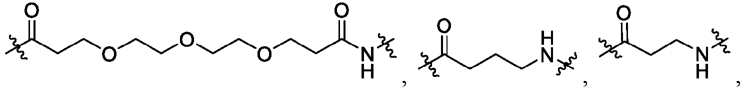 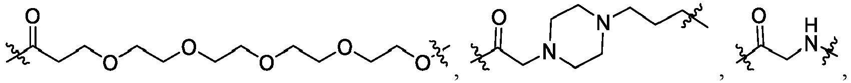 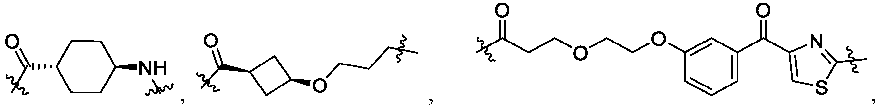 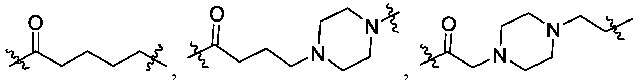 oder

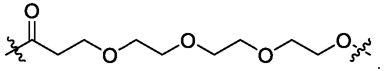

9. Verbindung oder pharmazeutisch verträgliches Salz nach Anspruch 1, wobei die Verbindung von Formel (I) eine Verbindung von Formel **(III)** 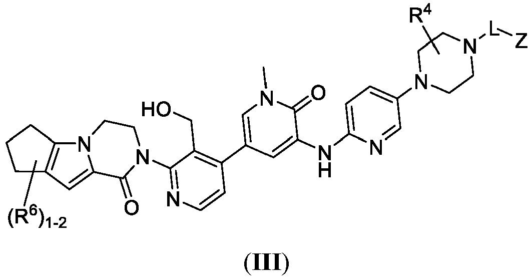
oder pharmazeutisch verträgliches Salz davon ist;
wobei optional jedes R⁶ unabhängig -H oder C₁₋₃-Alkyl ist und/oder R⁴ unabhängig -H oder C₁₋₃-Alkyl ist.

10. Verbindung oder pharmazeutisch verträgliches Salz nach Anspruch 9, wobei die Verbindung von Formel **(III)** wie folgt ist:
eine Verbindung von Formel **(III-A)** 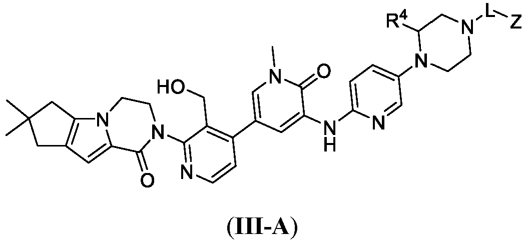
oder ein pharmazeutisch verträgliches Salz davon;
eine Verbindung von Formel **(III-B)** 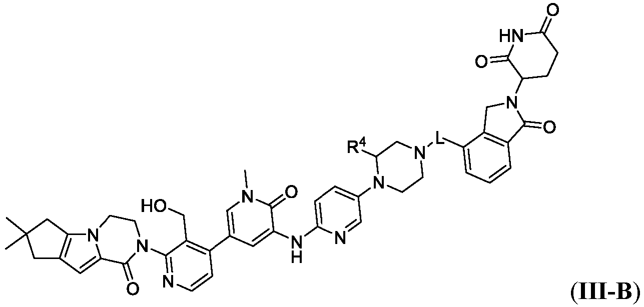
oder ein pharmazeutisch verträgliches Salz davon; oder
eine Verbindung von Formel **(III-C)** 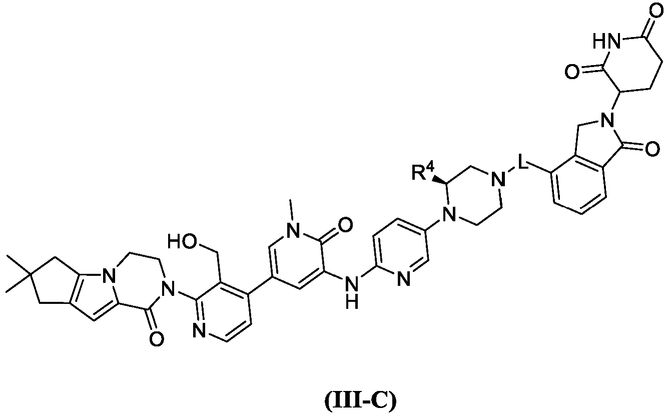
oder ein pharmazeutisch verträgliches Salz davon;
wobei:
R⁴ -H oder -C₁₋₃-Alkyl ist;
L -Y¹-Y²-Y³-Y⁴-Y⁵-Y⁶- ist;
Y¹ -C(O)-, -C(O)-N(R)-, -(CH₂-CH₂-O)ₘ- -(O-CH₂-CH₂)ₘ- oder-C₁₋₄-Alkyl- ist;
Y² eine Bindung, -O-, -C₁₋₄-Alkyl-, -(O-CH₂-CH₂)ₙ-, -(CH₂-CH₂-O)ₙ-, -(CH₂-CH₂-N(R))- oder 3-6-gliedriges Cycloalkyl ist,
Y³ eine Bindung, -O-, -N(R)-, -C₁₋₄-Alkyl-, -(CH₂-CH₂-O)_{P}-, -(O-CH₂-CH₂)_{P}-, -(CH₂-CH₂-N(R))-, 3-6-gliedriges Cycloalkyl oder 4-6-gliedriges Heterocycloalkyl mit 1-3 Heteroatomen unabhängig ausgewählt aus N, O oder S ist;
Y⁴ eine Bindung, -N(R)-, -C₁₋₄-Alkyl-, -(CH₂-CH₂-O)_{q}-, -(O-CH₂-CH₂)_{q}- -(CH₂-CH₂-N(R))-, 3-6-gliedriges Cycloalkyl oder 4-6-gliedriges Heterocycloalkyl mit 1-3 Heteroatomen unabhängig ausgewählt aus N, O oder S ist;
Y⁵ eine Bindung, -N(R)-, -(CH₂-CH₂-O)ₛ- oder -(O-CH₂-CH₂)ₛ- ist;
Y⁶ eine Bindung oder -(CH₂-CH₂-N(R))- ist;
jedes R unabhängig -H oder -C₁₋₃-Alkyl ist; und
jedes von m, p, q und s unabhängig 1, 2 oder 3 ist;
Z wie folgt ist: 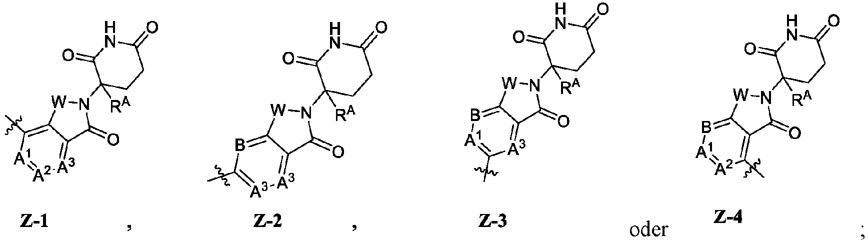 oder 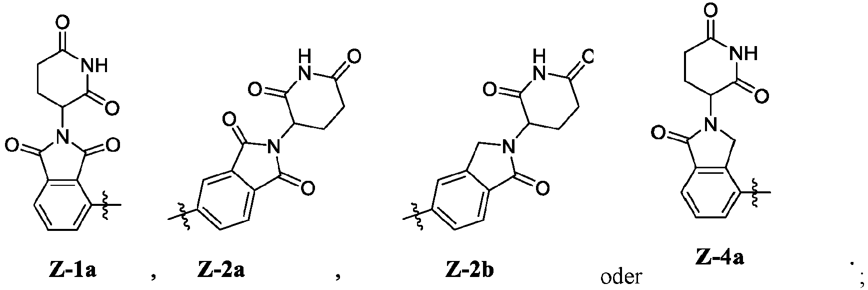
jedes von A¹, A² und A³ unabhängig -C(R^{B})= oder -N= ist;
B -C(R^{C})= oder -N= ist;
W -C(R^{D})₂- oder -C(O)- ist;
R^{A} -H, -CH₃ oder -F ist;
jedes R^{B} unabhängig -H, Halogen, -OH, -C₁₋₄-Alkyl oder -C₁₋₄-Alkoxy ist;
R^{c} -H, Halogen oder C₁₋₄-Alkyl ist; und
jedes R^{D} unabhängig -H oder -C₁₋₃-Alkyl ist;
vorausgesetzt, dass, wenn jedes von Y³, Y⁴, Y⁵ und Y⁶ eine Bindung ist, dann Y² keine Bindung ist.

11. Verbindung oder pharmazeutisch verträgliches Salz nach Anspruch 9 oder Anspruch 10, wobei L ausgewählt ist aus 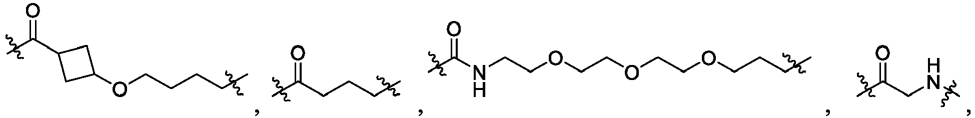 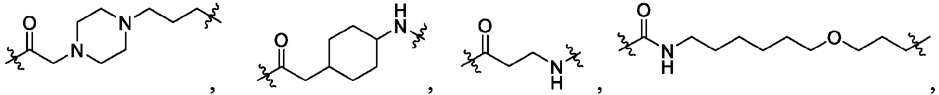 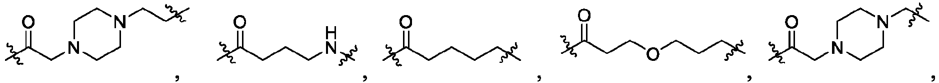 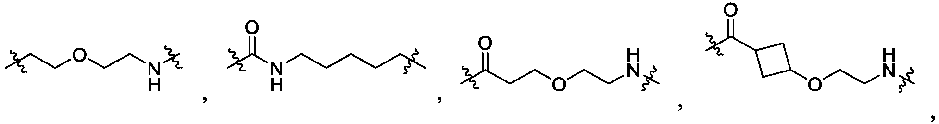 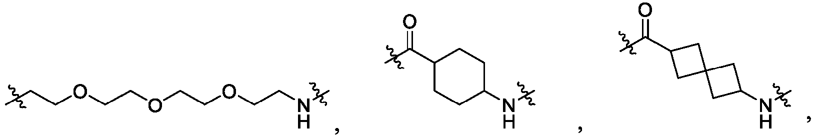 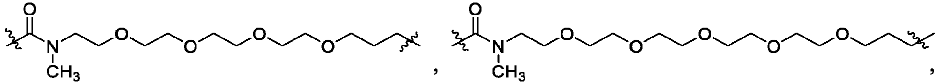 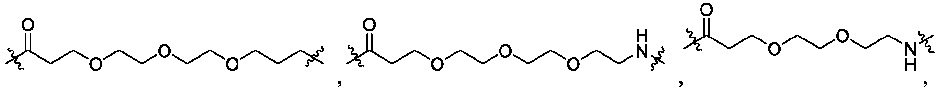 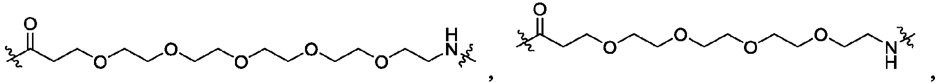 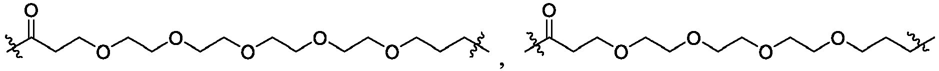 oder

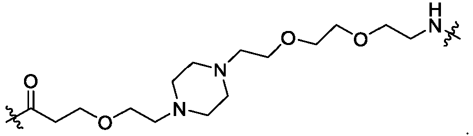

12. Verbindung nach Anspruch 1, ausgewählt aus Tabelle 1 oder pharmazeutisch verträgliches Salz davon:
**Tabelle 1**
| | |
|---|---|
| **Verbind ung Nr.** | **Struktur** |
| 1 | 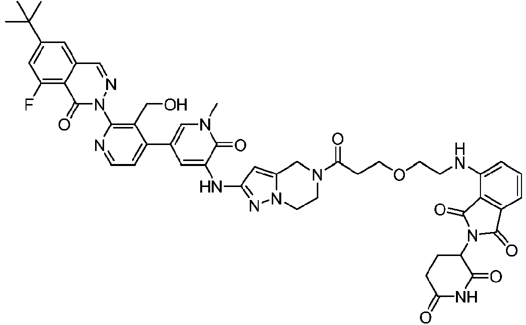 |
| **Verbind ung Nr.** | **Struktur** |
|---|---|
| 2 | 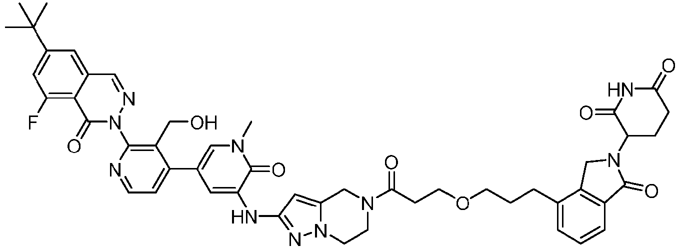 |
| 3 | 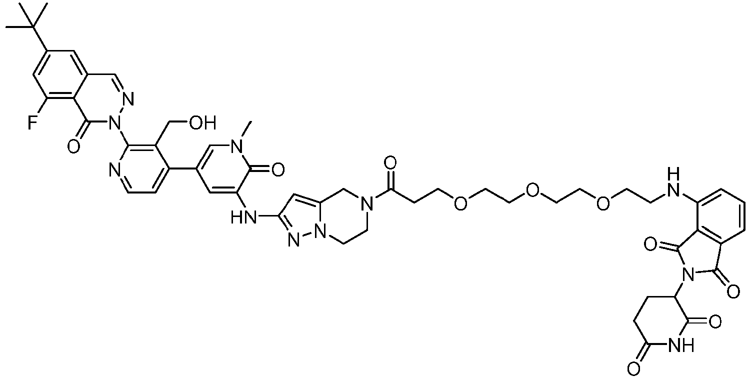 |
| 4 | 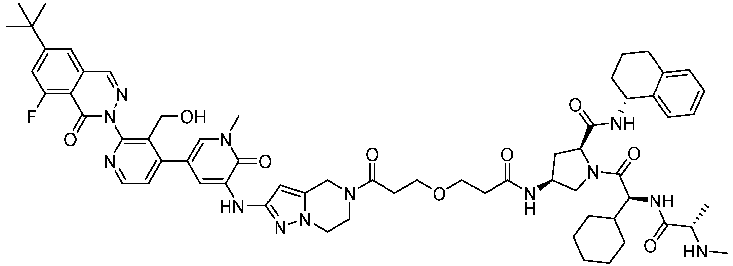 |
| 5 | 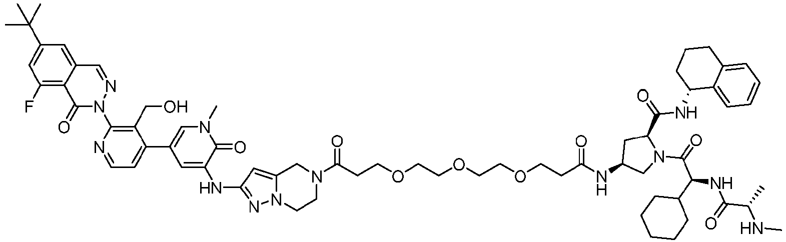 |
| 6 | 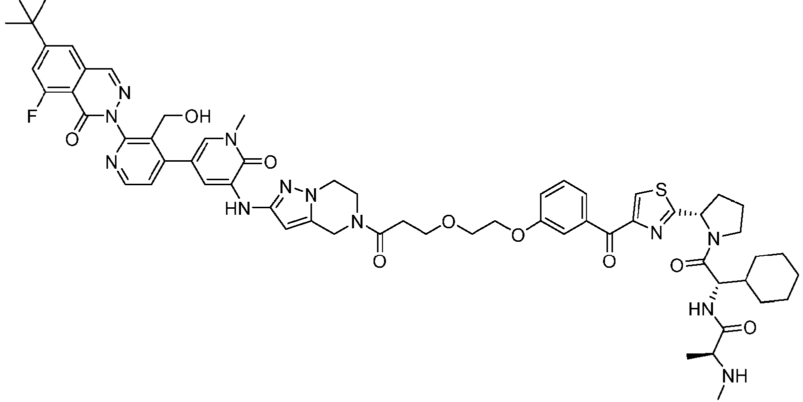 |
| 7 | 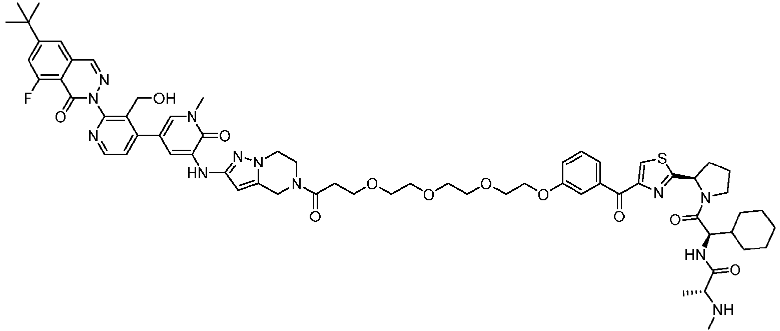 |
| 8 | 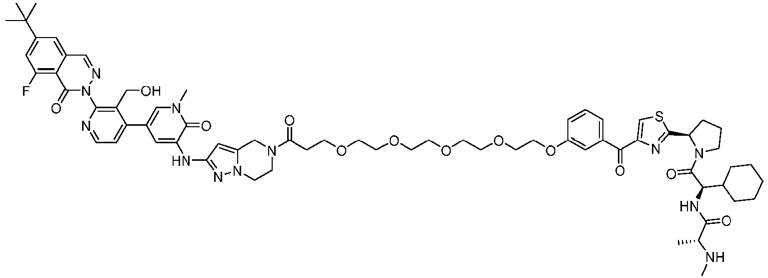 |
| 9 | 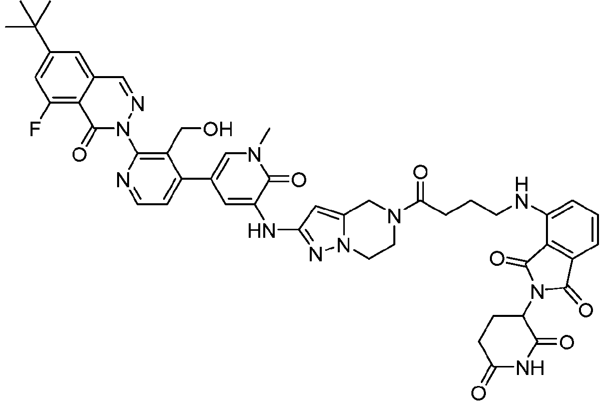 |
| 10 | 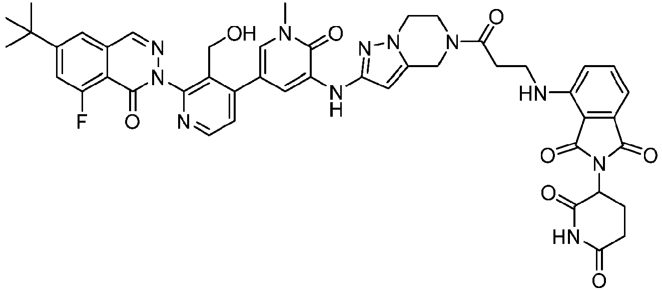 |
| 11 | 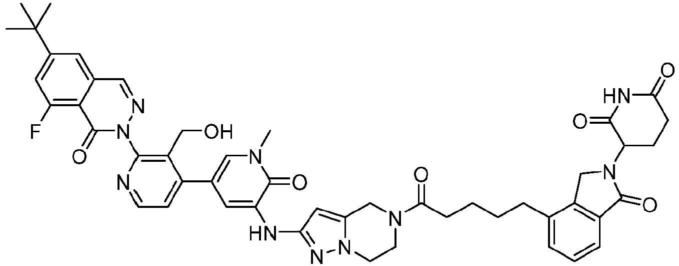 |
| 12 | 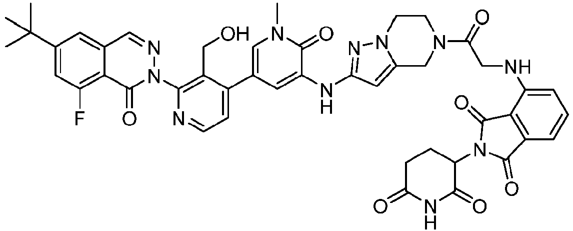 |
| 13 | 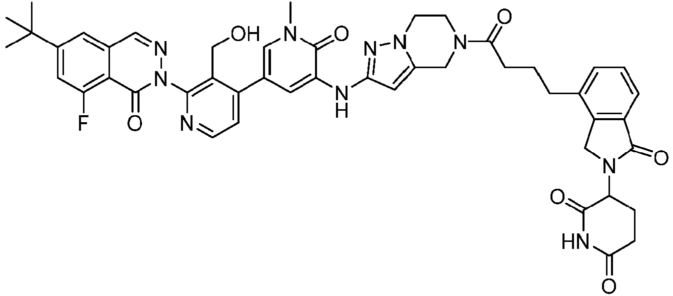 |
| 14 | 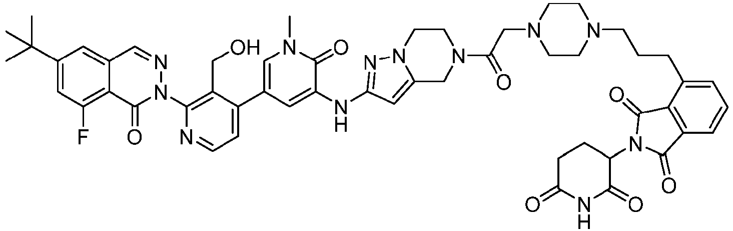 |
| 15 | 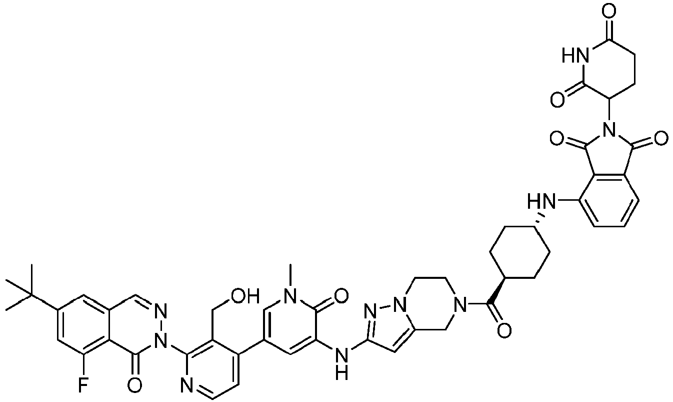 |
| 16 | 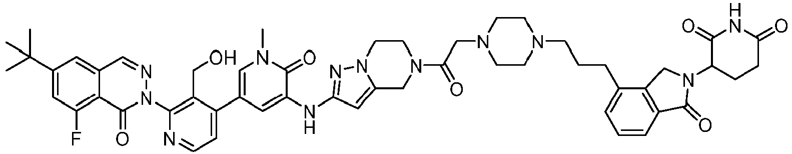 |
| 17 | 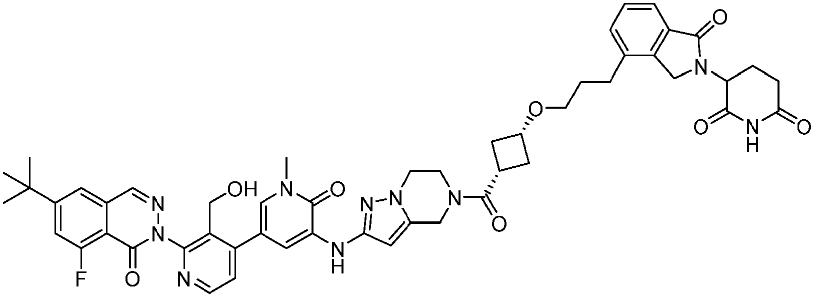 |
| 18 | 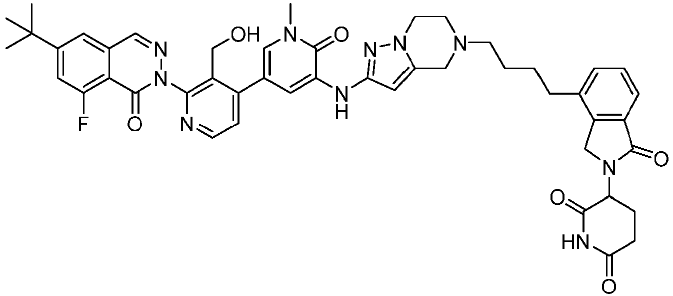 |
| 19 | 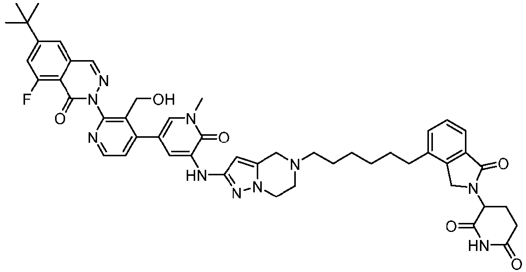 |
| 20 | 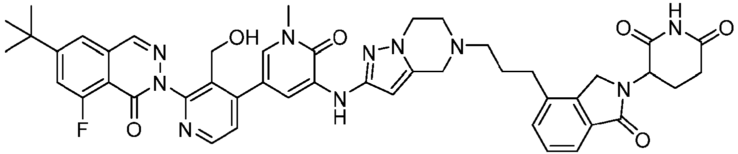 |
| 21 | 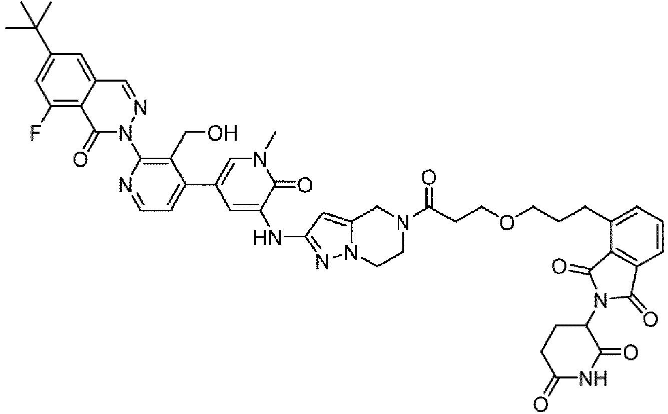 |
| 22 | 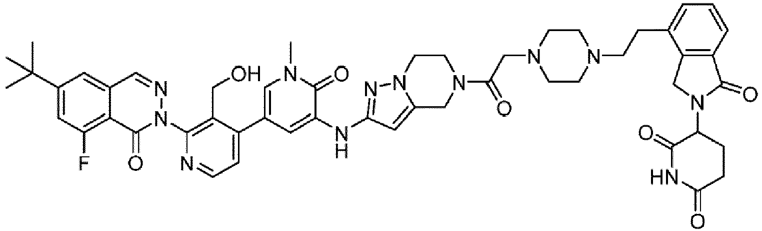 |
| 23 | 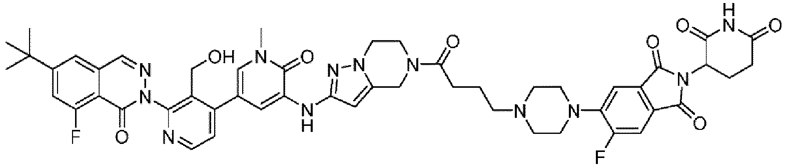 |
| 25 | 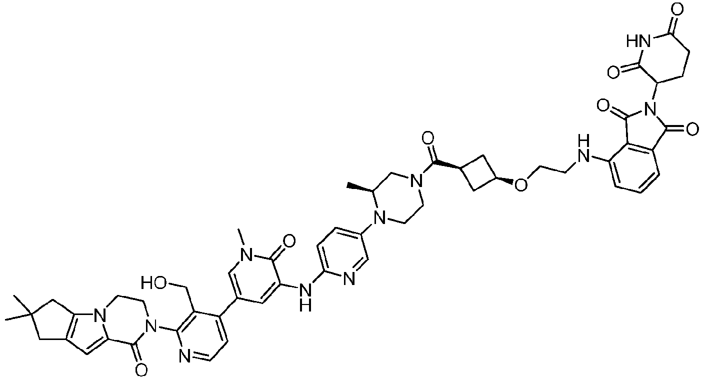 |
| 26 | 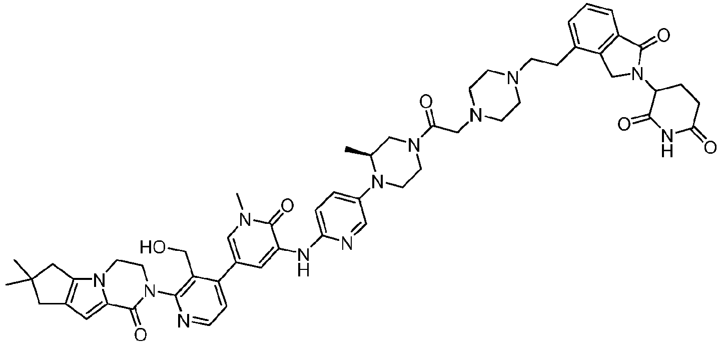 |
| 27 | 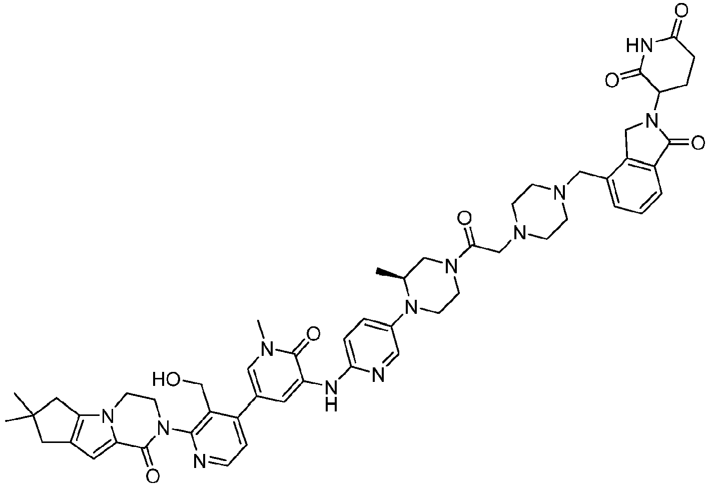 |
| 28 | 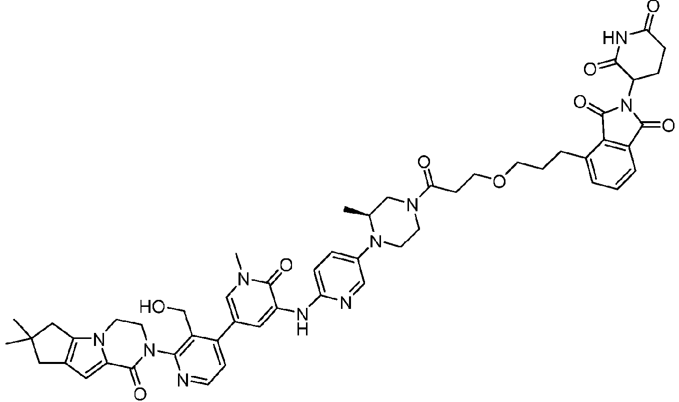 |
| 29 | 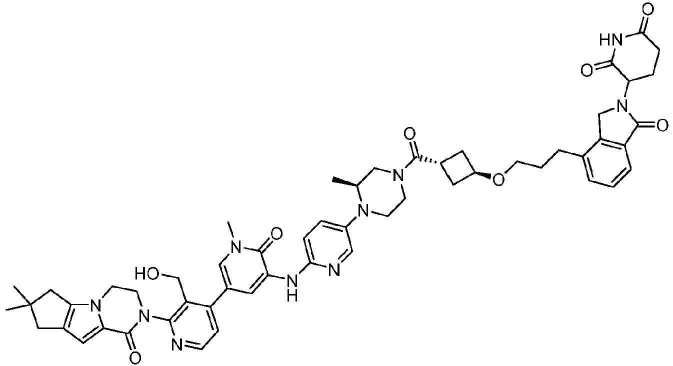 |
| 30 | 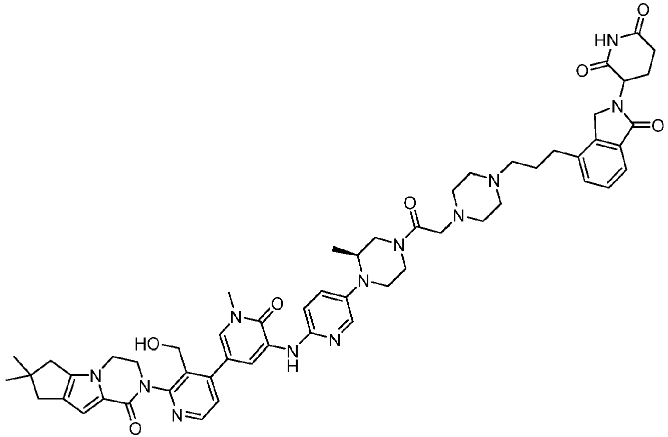 |
| 31 | 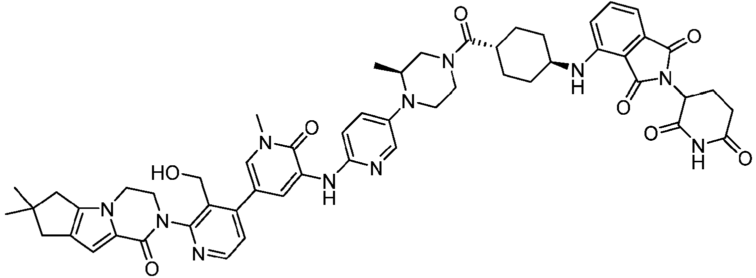 |
| 32 | 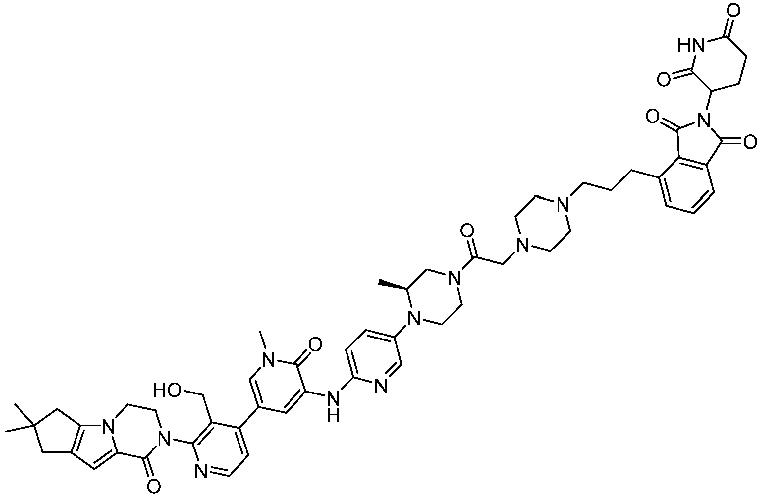 |
| 33 | 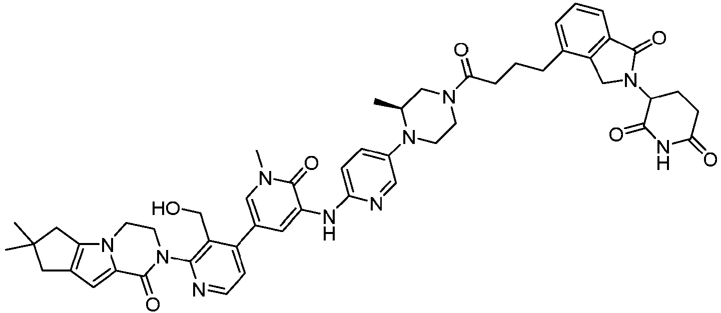 |
| 34 | 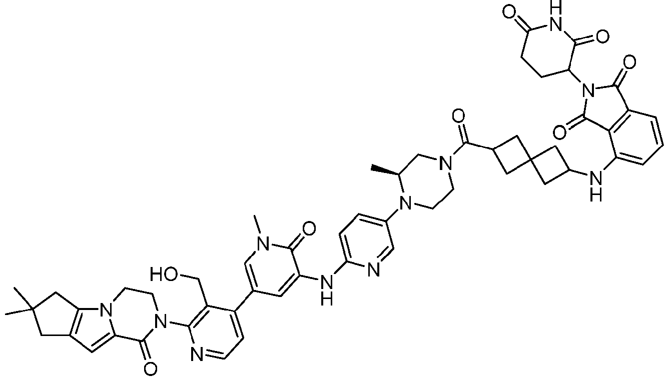 |
| 35 | 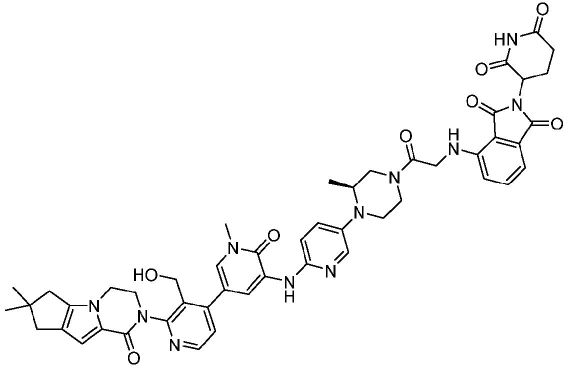 |
| 36 | 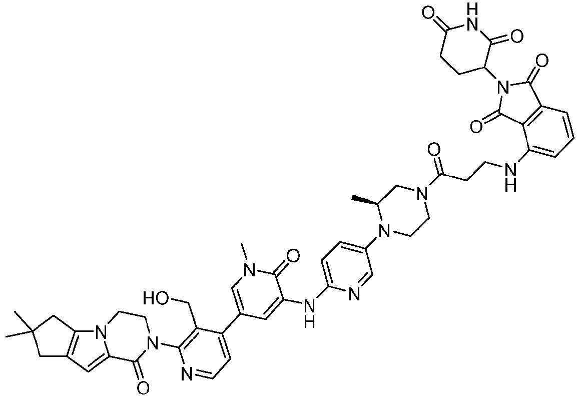 |
| 37 | 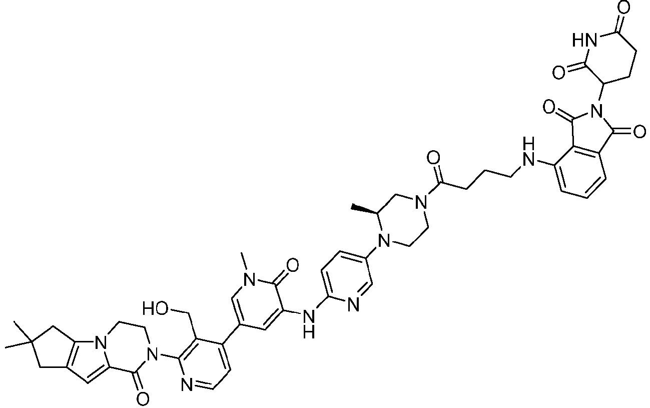 |
| 38 | 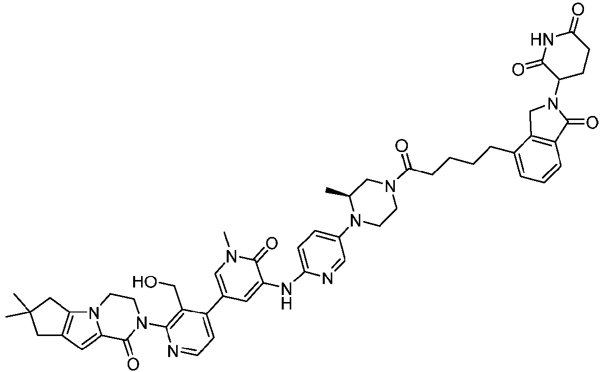 |
| 39 | 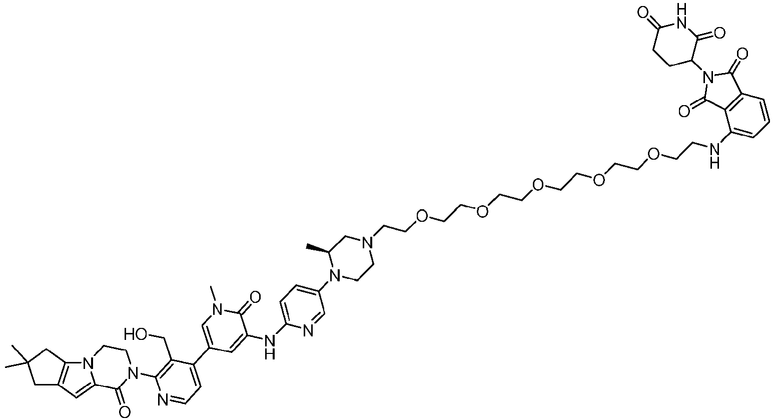 |
| 40 | 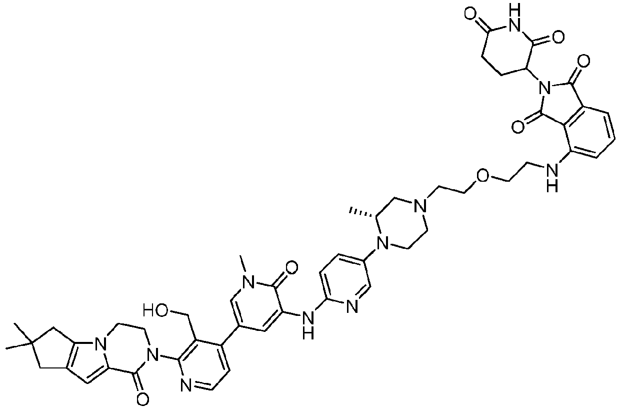 |
| 41 | 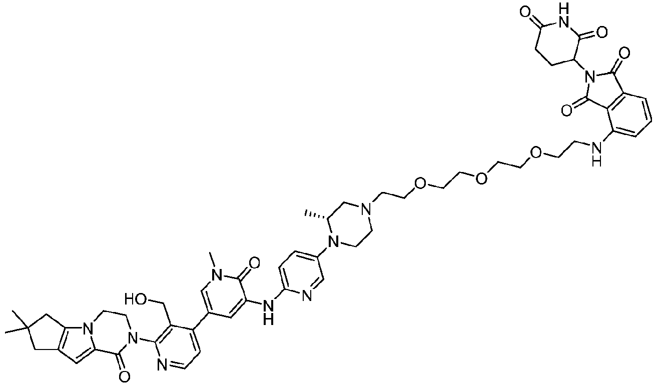 |
| 42 | 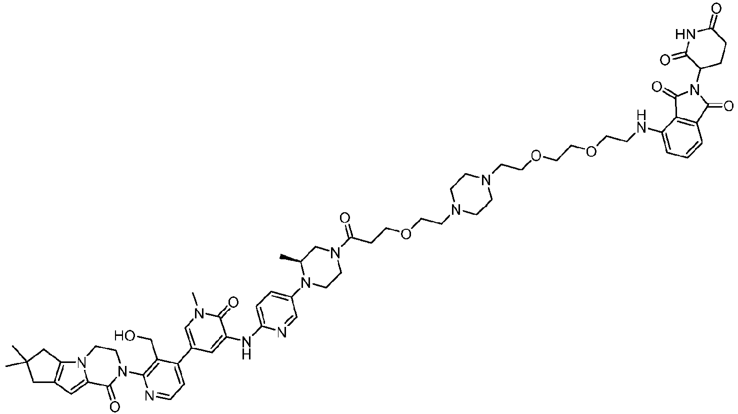 |
| 43 | 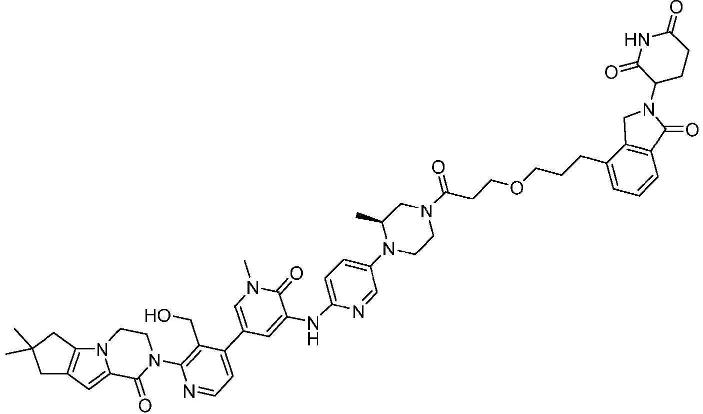 |
| 44 | 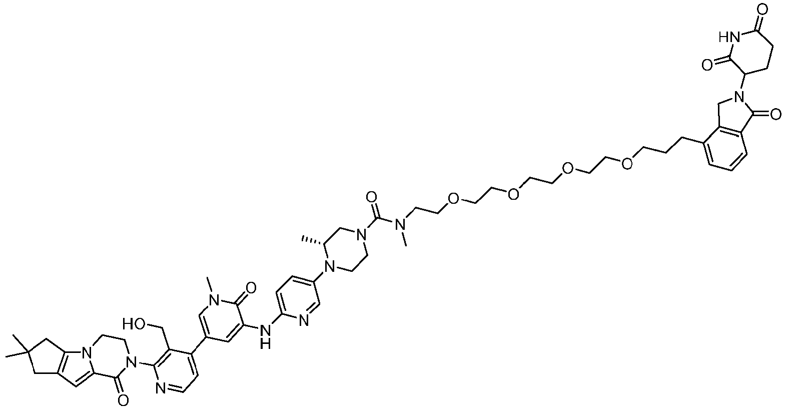 |
| 45 | 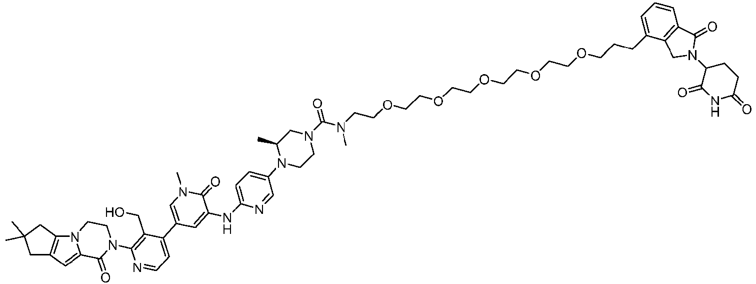 |
| 46 | 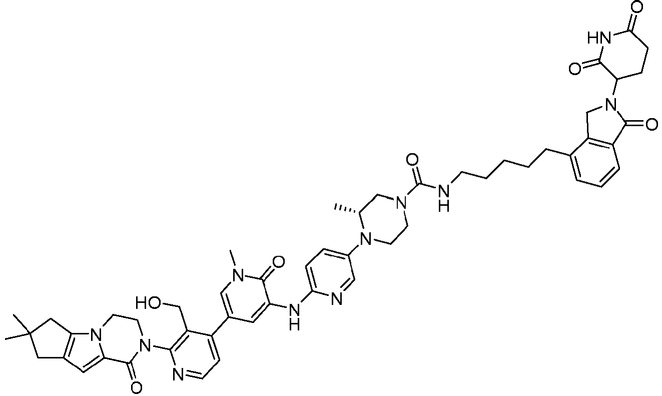 |
| 47 | 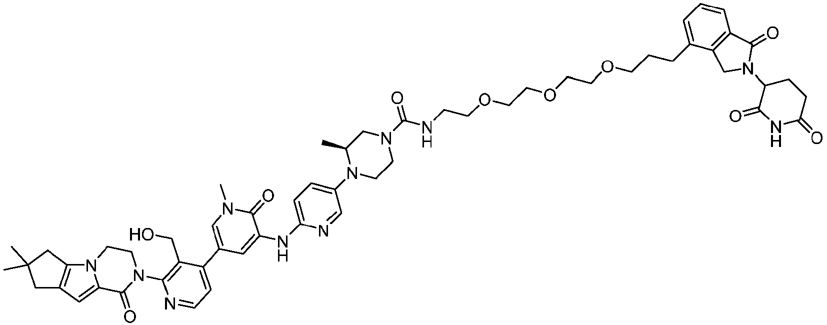 |
| 48 | 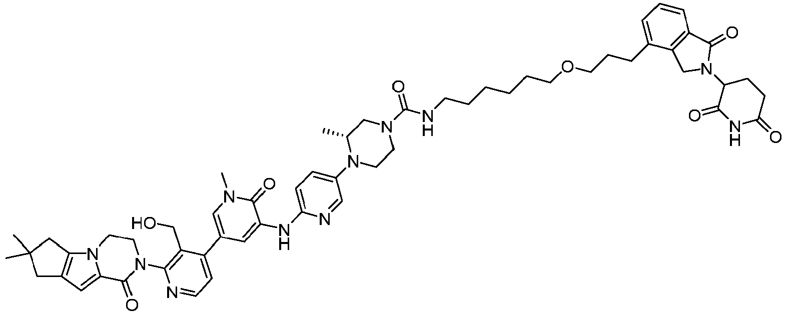 |
| 49 | 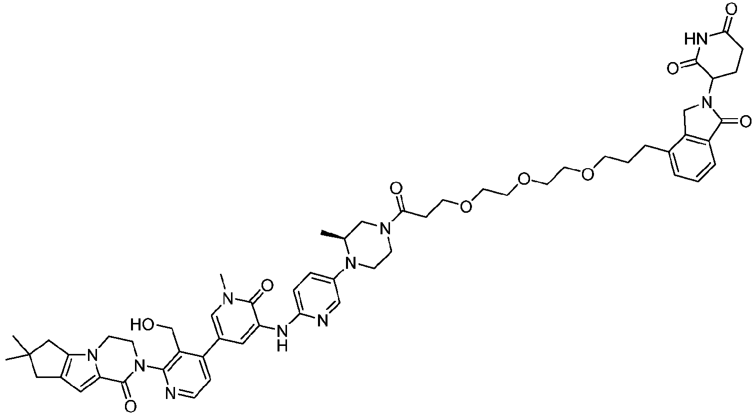 |
| 50 | 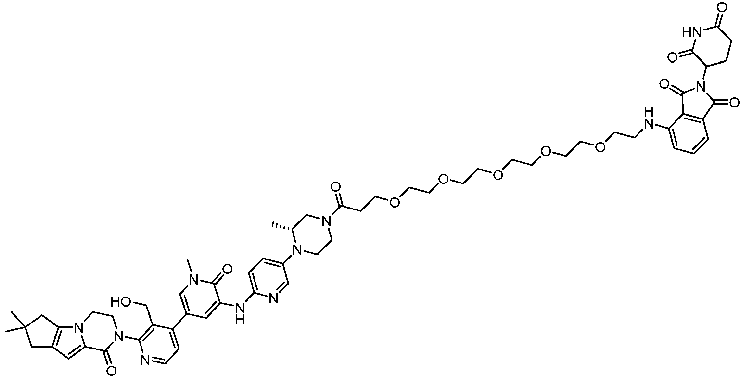 |
| 51 | 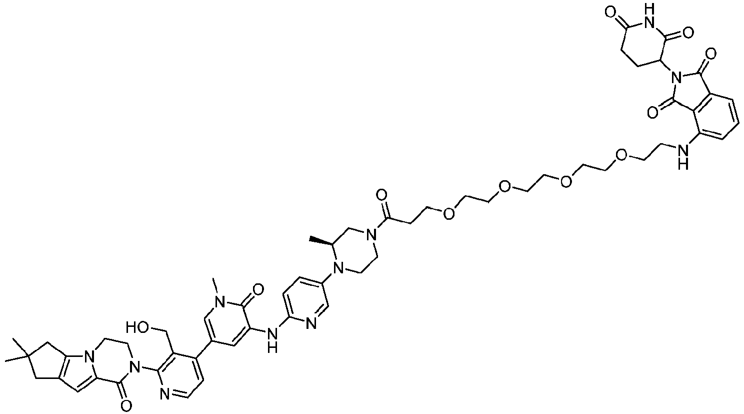 |
| 52 | 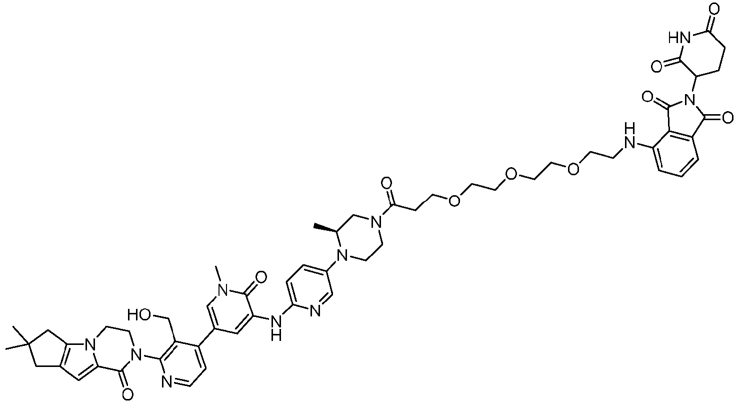 |
| 53 | 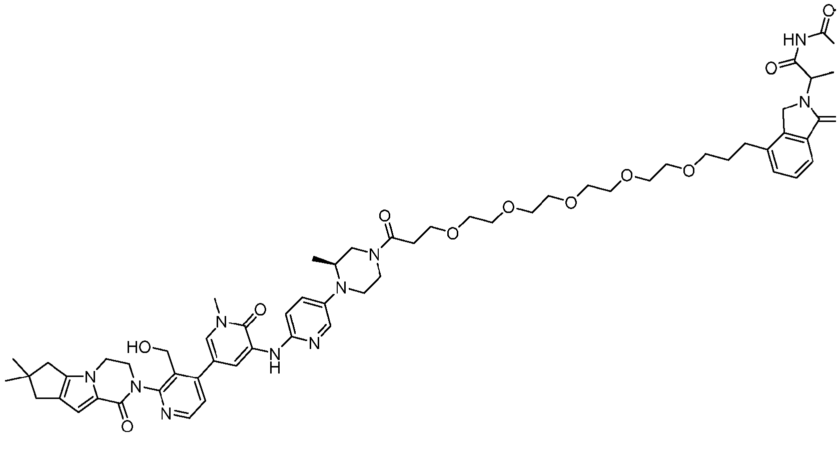 |
| 54 | 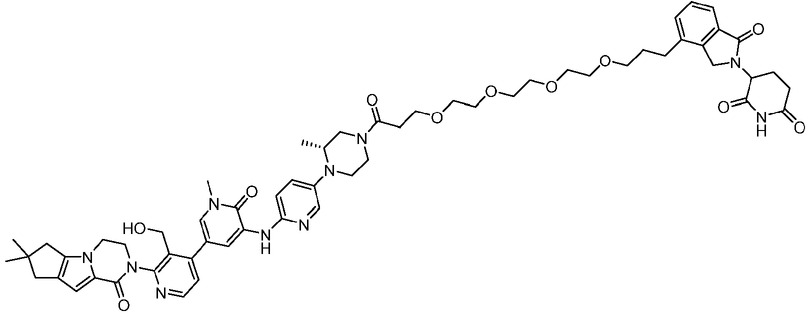 |
| 55 | 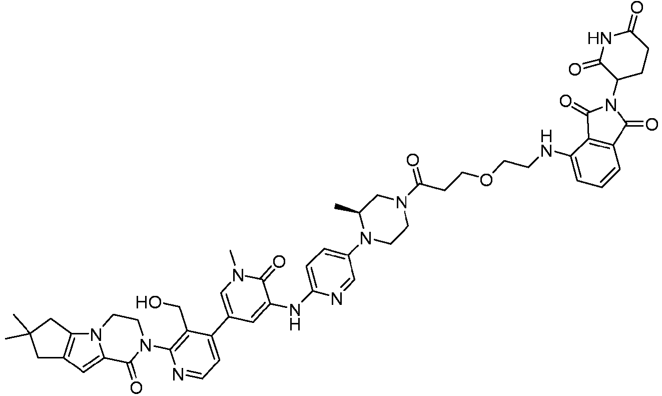 |
| 56 | 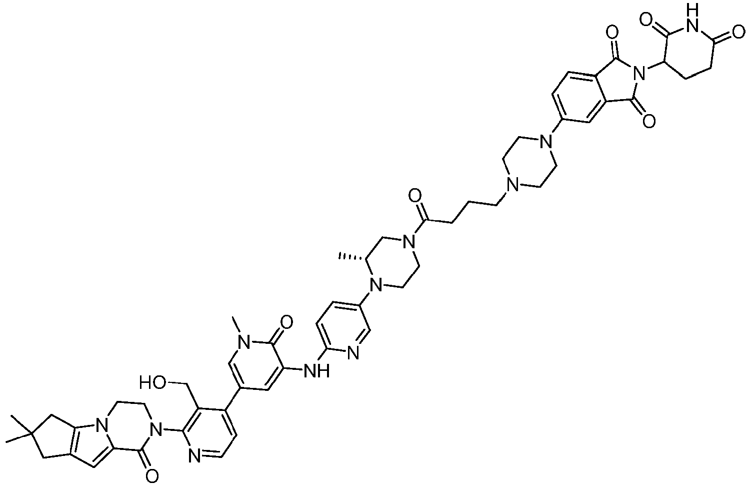 |
| 57 | 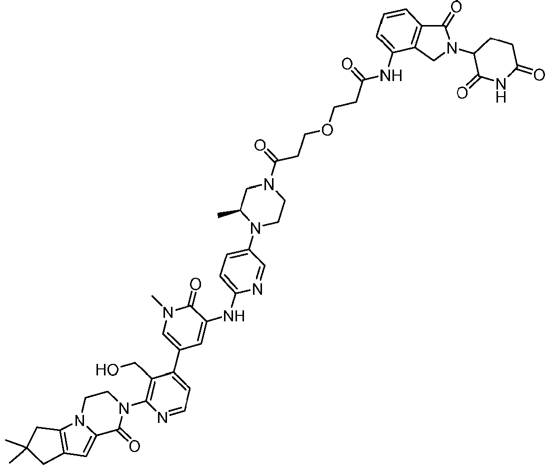 |
| 58 | 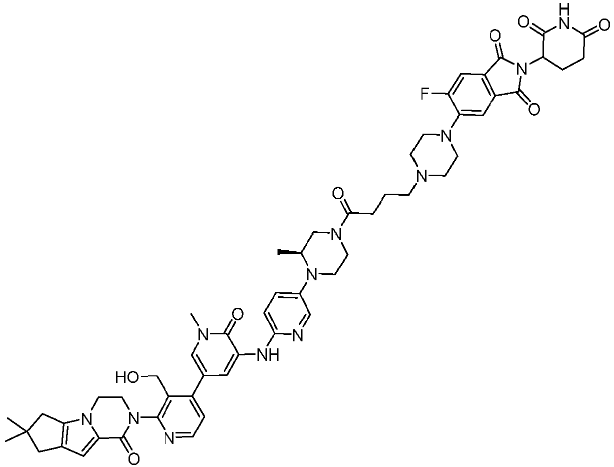 |

13. Verbindung von:
| | |
|---|---|
| 24 | 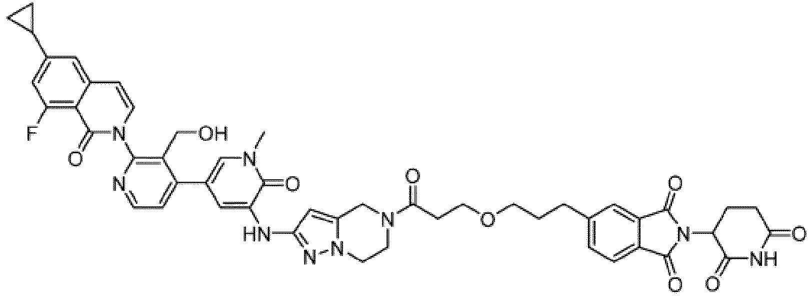 |
oder ein pharmazeutisch verträgliches Salz davon.

14. Pharmazeutische Zusammensetzung, umfassend eine Verbindung oder pharmazeutisch verträgliches Salz einer Verbindung nach einem der vorhergehenden Ansprüche und einen pharmazeutisch verträglichen Träger, ein pharmazeutisch verträgliches Vehikel oder ein pharmazeutisch verträgliches Adjuvans.

15. Verbindung oder pharmazeutisch verträgliches Salz nach einem der Ansprüche 1-13 oder pharmazeutische Zusammensetzung nach Anspruch 14 zur Verwendung in einem Verfahren zum Behandeln einer Erkrankung oder Störung, die durch Abbau von Bruton-Tyrosinkinase verursacht wird.

16. Verbindung oder Zusammensetzung zur Verwendung gemäß Anspruch 15, wobei die Erkrankung oder Störung Krebs oder eine Autoimmunerkrankung ist.

17. Verbindung oder Zusammensetzung zur Verwendung gemäß Anspruch 16, wobei:
der Krebs ein hämatologischer Krebs ausgewählt aus myeloischer Leukämie (akut und chronisch), akuter lymphoblastischer Leukämie, chronischer lymphatischer Leukämie, myeloproliferativen Erkrankungen, multiplem Myelom, myelodysplastischem Syndrom, Hodgkin-Erkrankung, Non-Hodgkin-Lymphom (malignes Lymphom), Haarzell-Lymphom, Mantelzell-Lymphom, Waldenström-Makroglobulinämie, Marginalzonen-Lymphom und follikulärem Lymphom ist; oder
die Autoimmunerkrankung ausgewählt ist aus Urtikaria, Graft-versus-Host-Erkrankung, Pemphigus vulgaris, Achalasie, Addison-Erkrankung, adulter Still-Erkrankung, Agammaglobulinämie, Alopezie areata, Amyloidosis, ankylosierender Spondylitis, Anti-GBM/Anti-TBM-Nephritis, Antiphospholipidsyndrom, autoimmunem Angioödem, autoimmuner Dysautonomie, autoimmuner Enzephalomyelitis, autoimmuner Hepatitis, autoimmuner Innenohrerkrankung (AIED), autoimmuner Myokarditis, autoimmuner Oophoritis, autoimmuner Orchitis, autoimmuner Pankreatitis, autoimmuner Retinopathie, axonaler und neuronaler Neuropathie (AMAN), Balo-Erkrankung, Behcet-Erkrankung, benignem Schleimhautpemphigoid, bullösem Pemphigoid, Castleman-Erkrankung (CD), Zöliakie-Erkrankung, Chagas-Erkrankung, chronischer entzündlicher demyelinisierender Polyneuropathie (CIDP), chronischer wiederkehrender multifokaler Osteomyelitis (CRMO), Churg-Strauss-Syndrom (CSS) oder eosinophiler Granulomatose (EGPA), vernarbendem Pemphigoid, Cogan-Syndrom, Kälteagglutininerkrankung, angeborenem Herzblock, Coxsackie-Myokarditis, CREST-Syndrom, Crohn-Erkrankung, Dermatitis herpetiformis, Dermatomyositis, Devic-Erkrankung (Neuromyelitis optica), diskoidem Lupus, Dressler-Syndrom, Endometriose, eosinophiler Ösophagitis (EoE), eosinophiler Fasciitis, Erythema nodosum, essentieller gemischter Kryoglobulinämie, Evans-Syndrom, Fibromyalgie, fibrosierender Alveolitis, Riesenzellarteriitis (temporaler Arteriitis), Riesenzellmmyokarditis, Glomerulonephritis, Goodpasture-Syndrom, Granulomatose mit Polyangiitis, Graves-Erkrankung, Guillain-Barre-Syndrom, Hashimoto thyroiditis, hämolytischer Anämie, Purpura Schönlein-Henoch (HSP), Herpes gestationis oder Pemphigoid gestationis (PG), Hidradenitis suppurativa (HS) (Akne inversa), Hypogammalglobulinämie, IgA-Nephropathie, IgG4-bedingter sklerosierender Erkrankung, immunthrombozytopenischer Purpura (ITP), Einschlusskörpermyositis (IBM), interstitieller Zystitis (IC), juveniler Arthritis, juvenilem Diabetes (Diabetes Typ 1), juveniler Myositis (JM), Kawasaki-Erkrankung, Lambert-Eaton-Syndrom, leukozytoklastischer Vaskulitis, Lichen planus, Lichen sclerosus, Konjunktivitis linosa, linearer IgA-Erkrankung (LAD), Lupus, chronischer Lyme-Erkrankung, Meniere-Erkrankung, mikroskopischer Polyangiitis (MPA), gemischter Bindegewebeerkrankung (MCTD), Mooren's Geschwür, Mucha-Habermann-Erkrankung, multifokaler motorischer Neuropathie (MMN) oder MMNCB, multipler Sklerose, Myasthenia gravis, Myositis, Narkolepsie, neonatalem Lupus, Neuromyelitis optica, Neutropenie, okulärem vernarbenden Pemphigoid, optischer Neuritis, palindromem Rheuma (PR), PANDAS, paraneoplastischer cerebellarer Degeneration (PCD), paroxysmaler nächtlicher Hämoglobinurie (PNH), Parry-Romberg-Syndrom, Pars planitis (peripheraler Uveitis), Parsonnage-Turner-Syndrom, Pemphigus, peripheraler Neuropathie, perivenöser Encephalomyelitis, perniziöser Anämie (PA), POEMS-Syndrom, Polyarteritis nodosa, polyglandulären Syndromen Typ I, II, III, Polymyalgia rheumatica, Polymyositis, Postherzinfarktsyndrom, Postperikardiotomiesyndrom, primärer biliärer Zirrhose, primärer sklerosierender Cholangitis, Progesterondermatitis, Psoriasis, psoriatischer Arthritis, Aplasie der roten Blutkörperchen (PRCA), Pyoderma gangrenosum, Raynaud-Phänomen, reaktiver Arthritis, reflexsympathischer Dystrophie, rezidivierender Polychondritis, Restless-Legs-Syndrom (RLS), retroperitonealer Fibrose, rheumatischem Fieber, rheumatoider Arthritis, Sarkoidose, Schmidt-Syndrom, Skleritis, Skleroderm, Sjögren-Syndrom, Autoimmunität der Spermien und Hoden, Stiff-Person-Syndrom (SPS), subakuter bakterieller Endokarditis (SBE), Susac-Syndrom, sympathischer Ophthalmie (SO), Takayasu-Arteriitis, temporaler Arteriitis (Riesenzellarteriitis), thrombozytopenischer Purpura (TTP), Tolosa-Hunt-Syndrom (THS), transverser Myelitis, Diabetes Typ 1, Colitis ulcerosa (UC), undifferenzierter Bindegewebserkrankung (UCTD), Uveitis, Vaskulitis, Vitiligo, Vogt-Koyanagi-Harada-Erkrankung und Wegener-Granulomatose (oder Granulomatose mit Polyangiitis (GPA)).

## Revendications

1. Composé de Formule (I) : 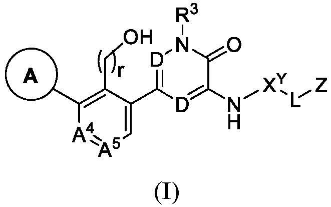
ou sel pharmaceutiquement acceptable de celui-ci, dans lequel
chacun de A⁴ et A⁵ est indépendamment N ou C-R¹⁵,
chaque R¹⁵ est indépendamment -H ou un C₁₋₆ alkyle éventuellement substitué ;
chaque D est indépendamment N ou CH ;
dans lequel l'anneau A est : 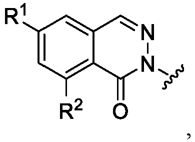 dans lequel chacun de R¹ et R² est indépendamment halo, -H, -OH, - CN, -CF₃, -C₁₋₆ alkyle, ou un cycloaliphatique à 3-6 chaînons ; 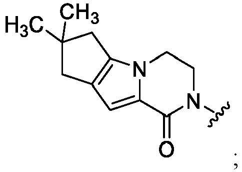 ou 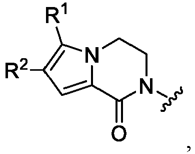 dans lequel chacun de R¹ et R² est indépendamment halo, -H, -OH, - CN, -CF₃, -C₁₋₆ alkyle, ou un cycloaliphatique à 3-6 chaînons, ou R¹ et R² conjointement avec les atomes auxquels ils sont fixés forment un cycloalkyle à 5-6 chaînons, le cycloalkyle étant éventuellement substitué par 1-2 de R⁶;
chacun de R¹ et R² est indépendamment halo, -H, -OH, -CN, -CF₃, -C₁₋₆ alkyle ou un cycloaliphatique à 3-6 chaînons, ou
R¹ et R², conjointement avec les atomes auxquels ils sont fixés, forment un cycloalkyle à 5-6 chaînons fusionné à l'anneau C, le cycloalkyle étant éventuellement substitué par 1-2 de R⁶, chaque R⁶ étant indépendamment -H, halo ou C₁₋₃ alkyle ;
R³ est C₁₋₆ alkyle ;
X^{Y} est 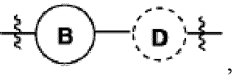 où
l'anneau B est phényle, ou un hétéroaryle monocyclique à 5-6 chaînons comportant 1-2 atomes d'azote, ou un hétérocycle bicyclique fusionné à 7-10 chaînons comportant 2-3 atomes d'azote, dans lequel au moins un des anneaux de l'hétérocycle bicyclique est partiellement ou entièrement non saturé, et l'anneau D est absent ou un hétérocycle à 4-7 chaînons éventuellement substitué comportant au moins 1 atome d'azote et éventuellement 2 hétéroatomes supplémentaires indépendamment sélectionnés parmi N, O et S ;
L est un groupe C₁₋₂₀ alkyle bivalent, dans lequel une ou plusieurs unités méthylène du groupe C₁₋₂₀ alkyle sont éventuellement et indépendamment remplacées par -C(O)-, -C(S)-,-(O)CO-, -OC(O)-, -O-, -N(R')-, -S-, -N(R')C(O)-, hétérocycloalkyle mono ou bicyclique bivalent, cycloalkyle mono ou bicyclique bivalent, aryle mono ou bicyclique bivalent, ou hétérocycloalkyle, cycloalkyle, aryle et hétéroaryle bivalents est éventuellement substitué ; et
Z est 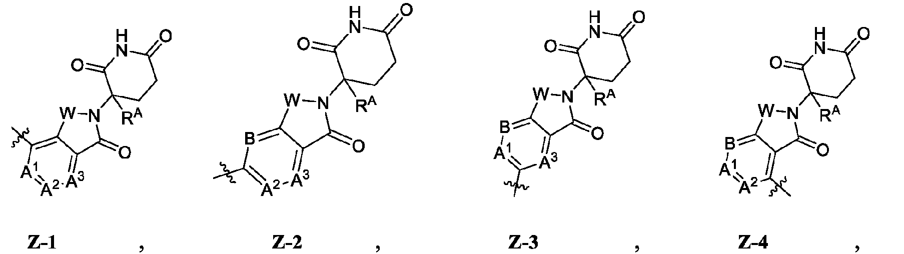 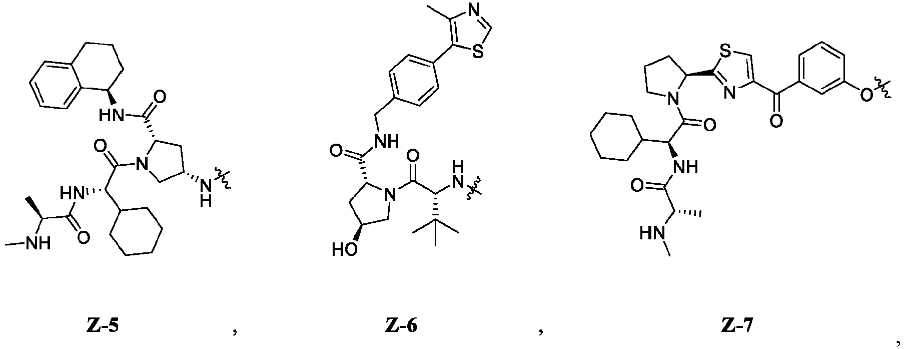 ou 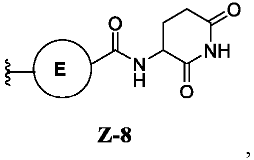
dans lequel
l'anneau E est phényle ou un hétéroaryle à 5-6 chaînons comportant au moins 1 hétéroatome indépendamment sélectionné entre N et S ;
chacun de A¹, A² et A³ est indépendamment -C(R^{B})= ou -N= ;
B est -C(R^{c})= ou -N= ;
W est -C(R^{D})₂- ou -C(O)- ;
R^{A} est -H, -CH₃, ou -F ;
chaque R^{B} est indépendamment -H, halo, -OH, -C₁₋₄ alkyle ou -C₁₋₄ alkoxy ;
R^{C} est -H, halo ou C₁₋₄ alkyle ; et
chaque R^{D} est indépendamment-H ou -C₁₋₃ alkyle ; et
r est 1, 2 ou 3.

2. Composé ou sel pharmaceutiquement acceptable selon la revendication 1, dans lequel :
- Z est 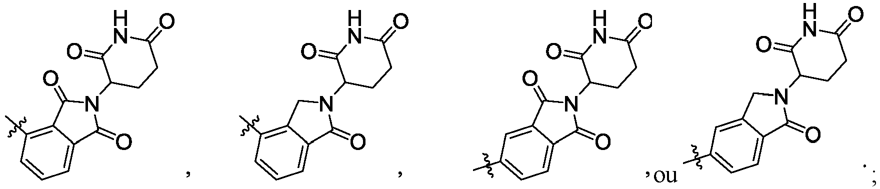 ou Z est 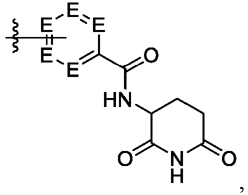 où une occurrence de E est 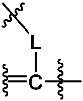 et les occurrences restantes de E sont
chacune indépendamment =CH- ou =N- ; ou Z est 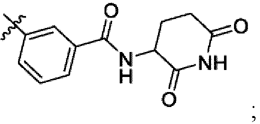
- un D est CH et l'autre D est N ; ou chaque D est CH ;
- au moins l'un de A⁴ et A⁵ est N ; ou A⁴ est N, A⁵ est CR¹⁵, et R¹⁵ est -H ou C₁₋₃ alkyle ;
- R¹⁵ est -H ou méthyle ;
- X^{Y} est: 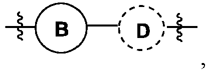 l'anneau B est 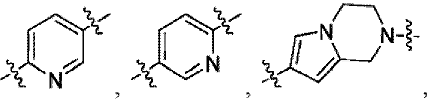 ou 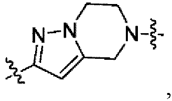 et l'anneau D est absent ; 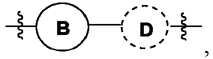 l'anneau B est 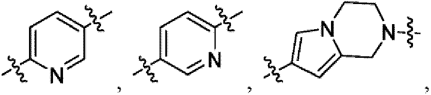 ou 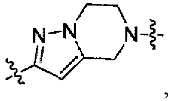 et l'anneau D est 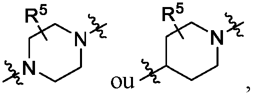 et R⁵ est -H ou C₁₋₃ alkyle ;
ou 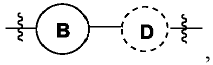
l'anneau B est 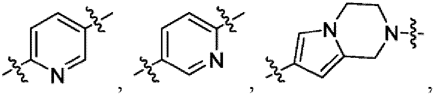
ou 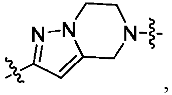
et l'anneau D est 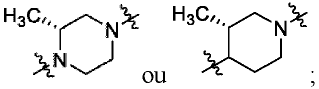
- R³ est un C₁₋₃ alkyle ; et/ou
- r est 1 ou 2.

3. Composé ou sel pharmaceutiquement acceptable selon la revendication 1, le composé de Formule (I) étant :
- un composé de Formule (I-A) 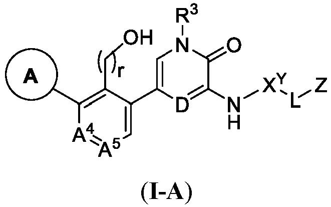 ou un sel pharmaceutiquement acceptable de celui-ci ;
- un composé de Formule (I-A1) 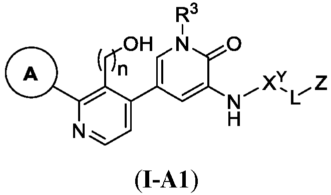 ou un sel pharmaceutiquement acceptable de celui-ci ;
- un composé de Formule (I-A2) 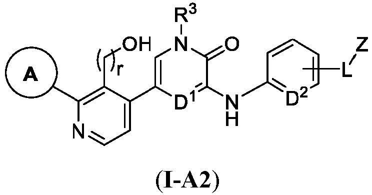
ou un sel pharmaceutiquement acceptable de celui-ci , dans lequel
D¹ est CH ou N ; D² est CH ou N, et au moins l'un de D¹ et D² est N ;
éventuellement dans lequel D¹ est =CH- et D² est =N- ; ou D¹ est =N- et D² est =CH- ; ou
- un composé de Formule (I-A3) 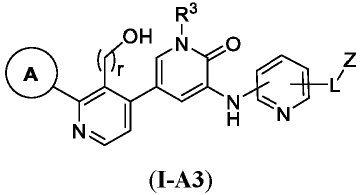 ou un sel pharmaceutiquement acceptable de celui-ci ;
- un composé de Formule (I-B1), un composé de Formule (I-B2), ou un composé de Formule (I-B3) 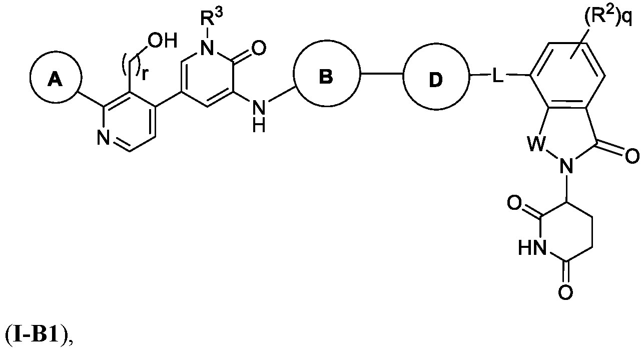 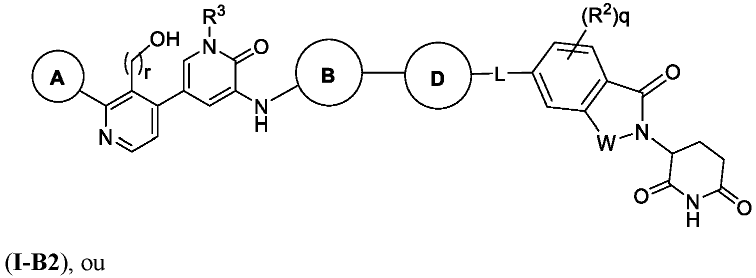 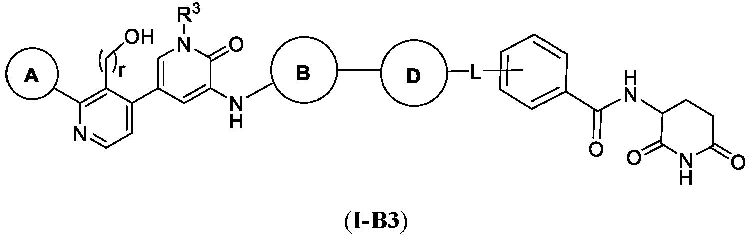
ou un sel pharmaceutiquement acceptable de celui-ci ;
éventuellement dans lequel
l'anneau B est phényle, pyridine-yle, ou pyrimidine-yle ; et/ou l'anneau D est un hétérocycle à 4-7 chaînons éventuellement substitué comportant 1-2 atomes d'azote ou l'anneau D est un hétérocycle à 5-6 chaînons éventuellement substitué comportant 1-2 atomes d'azote.

4. Composé ou sel pharmaceutiquement acceptable selon l'une quelconque des revendications 1-3, dans lequel L est -Y¹-Y²-Y³-Y⁴-Y⁵-Y⁶-, dans lequel
Y¹ est :
-C(O)-, -C(O)-N(R)-, -(CH₂-CH₂-O)ₘ- -(O-CH₂-CH₂)ₘ-, ou -C₁₋₆ alkyle- ;
-C(O)- ou -C(O)-N(R)- ;
-C(O)-, Y² est -(CH₂-CH₂-O)ₙ-, -(O-CH₂-CH₂)ₙ-, -C₁₋₄ alkyle-, un cycloalkyle monocyclique à 3-6 chaînons, ou un bicycloalkyle fusionné ou spiro à 7-11 chaînons, et n est 1 ou 2 ; ou
-(CH₂-CH₂-O)ₘ-, -(O-CH₂-CH₂)ₘ-, ou -C₁₋₆ alkyle- ;
Y² est :
une liaison, -C(O)-, -O-, -(CH₂-CH₂-O)ₙ-, -(O-CH₂-CH₂)ₙ-, -C₁₋₄ alkyle-, un cycloalkyle monocyclique à 3-6 chaînons éventuellement substitué, ou un bicycloalkyle fusionné ou spiro à 7-11 chaînons éventuellement substitué ;
-(CH₂-CH₂-O)ₙ-, -(O-CH₂-CH₂)ₙ-, -CH₂-, -CH₂-CH₂-, -CH₂-CH₂-CH₂-, cyclopentyle, cyclohexyle, ou un bicycloalkyle spiro à 7-11 chaînons, et n est 1 ou 2 ;
-C(O)-, -O-, -(CH₂-CH₂-O)ₙ-, -(O-CH₂-CH₂)ₙ-, 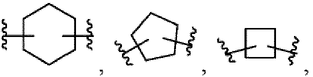 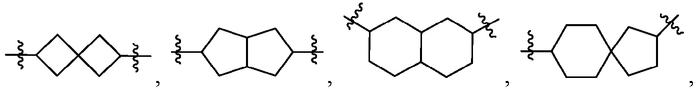 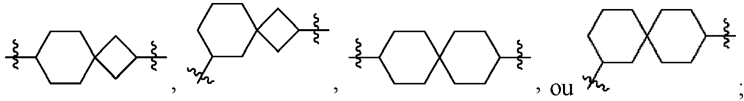 ou
-C(O)-, -O-, -(CH₂-CH₂-O)ₙ-, ou -(O-CH₂-CH₂)ₙ-, et n est 2 ou 3;
Y³ est :
une liaison, -O-, -N(R)-, -C₁₋₄ alkyle-, -(CH₂-CH₂-O)_{P}-, -(O-CH₂-CH₂)_{P}-, -(CH₂-CH₂-N(R))-, phényle, un hétéroaryle monocyclique à 5-6 chaînons comportant 1-2 hétéroatomes indépendamment sélectionnés entre N et S, un cycloalkyle à 3-6 chaînons éventuellement substitué, ou un hétérocycle à 4-6 chaînons éventuellement substitué comportant 1-3 hétéroatomes indépendamment sélectionnés parmi N, O et S ;
une liaison, -O-, -N(R)-, -C₁₋₄ alkyle-, -(CH₂-CH₂-N(R))-, phényle, un hétéroaryle monocyclique à 5-6 chaînons comportant 1-2 hétéroatomes indépendamment sélectionnés entre N et S, un cycloalkyle à 3-6 chaînons éventuellement substitué, ou un hétérocycle à 4-6 chaînons éventuellement substitué comportant 1-3 hétéroatomes indépendamment sélectionnés parmi N, O et S ;
une liaison, -N(R)-, -O-, -CH₂-, -CH₂-CH₂-, -CH₂-CH₂-CH₂-, -(CH₂-CH₂-N(R))-,
phényle, 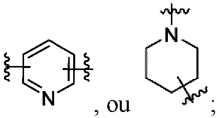
une liaison, -O-, -N(R)-, -(CH₂-CH₂-O)_{P}-, -(O-CH₂-CH₂)ₚ-, -(CH₂-CH₂-N(R))-, phényle, 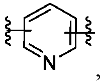 un cycloalkyle monocyclique à 3-6 chaînons éventuellement substitué, ou un hétérocycle monocyclique à 4-6 chaînons éventuellement substitué comportant 1-3 hétéroatomes indépendamment sélectionnés parmi N, O ou S ; ou
une liaison, -O-, -N(R)-, -(CH₂-CH₂-O)ₚ-, ou -(O-CH₂-CH₂)ₚ-, et p est 1 ou 2 ; ou 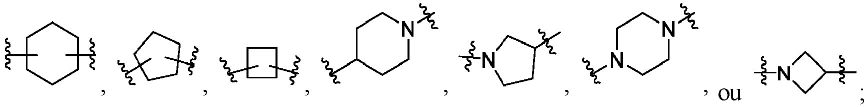
Y⁴ est :
une liaison, -C(O)-, -N(R)-, -C(O)N(R)-, -N(R)C(O)-, -C₁₋₄ alkyle-, -(CH₂-CH₂-O)_{q}-, -(O-CH₂-CH₂)_{q}-, -(CH₂-CH₂-N(R))-, un cycloalkyle à 3-6 chaînons éventuellement substitué, ou un hétérocycle à 4-6 chaînons éventuellement substitué comportant 1-3 hétéroatomes indépendamment sélectionnés parmi N, O ou S ;
une liaison, -C(O)-, -N(R)-, -C(O)N(R)-, -N(R)C(O)-, -C₁₋₄ alkyle-, -(CH₂-CH₂-N(R))-, un cycloalkyle à 3-6 chaînons éventuellement substitué, ou un hétérocycle à 4-6 chaînons éventuellement substitué comportant 1-3 hétéroatomes indépendamment sélectionnés parmi N, O ou S ;
une liaison, -C(O)-, -N(R)-, -C(O)N(R)-, -N(R)C(O)-, -CH₂-, -CH₂-CH₂-, 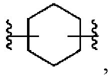 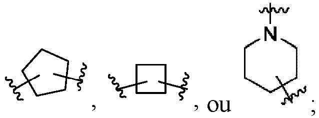
une liaison, -N(R)-, -C(O)N(R)-, -N(R)C(O)-, -C₁₋₄ alkyle-, -(CH₂-CH₂-N(R))-, un cycloalkyle à 3-6 chaînons éventuellement substitué, ou un hétérocycle à 4-6 chaînons éventuellement substitué comportant 1-3 hétéroatomes indépendamment sélectionnés parmi N, O ou S ; ou
une liaison, -N(H)-, -N(CH₃)-, -N(H)C(O)-, -CH₂-, -CH₂-CH₂-, -CH₂-CH₂-CH₂-, -(CH₂-CH₂-N(H))-, 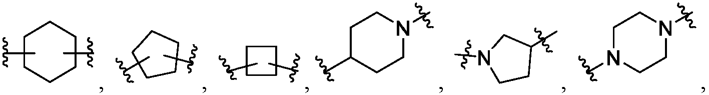 ou 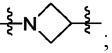
Y⁵ est :
une liaison, -N(R)-, -C(O)-, -C₁₋₄ alkyle-, -(CH₂-CH₂-O)ₛ-, -(O-CH₂-CH₂)ₛ-, ou un hétéroaryle à 5-6 chaînons comportant 1-3 hétéroatomes indépendamment sélectionnés entre N ou S ;
une liaison, -N(R)-, -C 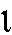 -₄ alkyle-, -(CH₂-CH₂-O)ₛ-, -(O-CH₂-CH₂)ₛ-, ou un hétéroaryle à 5-6 chaînons comportant 1-3 hétéroatomes indépendamment sélectionnés entre N ou S ;
une liaison, -N(R)-, -CH₂-, -CH₂-CH₂-, -CH₂-CH₂-CH₂-, -(CH₂-CH₂-O)ₛ-, -(O-CH₂-CH₂)ₛ-, ou 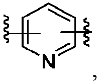
et s est 1 ou 2 ;
une liaison, -C(O)-, -C₁₋₄ alkyle-, -(CH₂-CH₂-O)ₛ-, -(O-CH₂-CH₂)ₛ-, ou un hétéroaryle à 5-6 chaînons comportant 1-3 hétéroatomes indépendamment sélectionnés entre N et S ; ou
une liaison, -C(O)-, -CH₂-, -CH₂-CH₂-, -CH₂-CH₂-CH₂-, -(CH₂-CH₂-O)ₛ-, -(O-CH₂-CH₂)ₛ-, ou 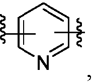 et s est 1 ou 2 ; et
Y⁶ est :
une liaison, ou -(CH₂-CH₂-N(R))- ; ou une liaison ou -CH₂-CH₂-N(H)-chacun de m et n est indépendamment 1, 2, 3, 4 ou 5 ;
chacun de p, q et s est indépendamment 1, 2 ou 3 ; et
R est -H ou -C₁₋₃ alkyle.

5. Composé ou sel pharmaceutiquement acceptable selon la revendication 1, le composé de Formule (I) étant un composé de Formule (**II**) 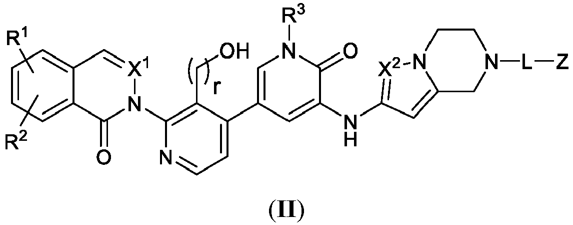
ou un sel pharmaceutiquement acceptable de celui-ci ;
un composé de Formule **(II-A)** 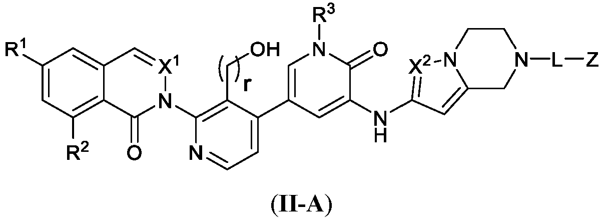
ou un sel pharmaceutiquement acceptable de celui-ci ; ou
un composé de Formule **(II-B)** 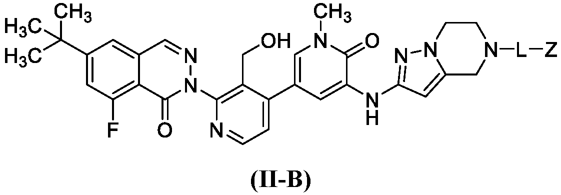
ou un sel pharmaceutiquement acceptable de celui-ci ;
dans lequel
R¹ est C₁₋₆ alkyle, hydroxyle, halo, cyano, ou un cycloaliphatique à 3-6 chaînons ;
R² est C₁₋₆ alkyle, hydroxyle, halo, cyano, ou un cycloaliphatique à 3-6 chaînons ; ou R² est C₁₋₆ alkyle, hydroxyle, halo ou cyano ;
R³ est C₁₋₆ alkyle ;
X¹ est =N- ;
X² est =CH- ou =N- ;
r est 1, 2 ou 3 ;
L est un groupe C₁₋₂₀ alkyle bivalent, éventuellement substitué par C₁₋₆ alkyle, acyle, oxo, halo ou alkoxy, dans lequel une ou plusieurs unités méthylène dudit groupe C₁₋₂₀ alkyle sont éventuellement et indépendamment remplacées par -CO-, -CS-, -CONH-, -CONHNH-, -CO₂-, - OCO-, - NHCO₂-, -O-, -NHCONH-, -OCONH-, -NHNH-, -NHCO-, -S-, -S(O)-, -S(O)₂-, -NH-, - S(O)₂NH-, -NHS(O)₂-, -NHS(O)₂NH-, hétérocycle mono ou bicyclique bivalent, cycloalkyle mono ou bicyclique bivalent, aryle mono ou bicyclique bivalent, ou hétéroaryle mono ou bicyclique bivalent, dans lequel l'hétérocycle et l'hétéroaryle de L comportent 1-3 hétéroatomes indépendamment sélectionnés parmi N, O ou S ; et
Z est 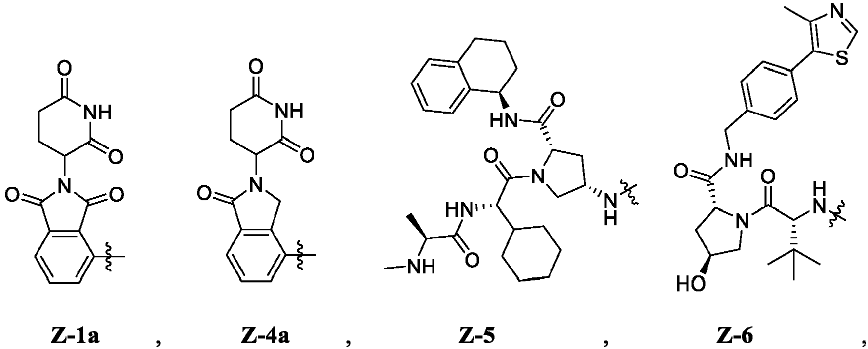
ou

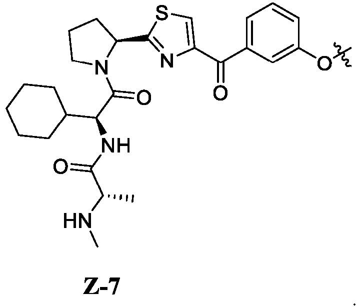

6. Composé ou sel pharmaceutiquement acceptable selon la revendication 5, dans lequel :
- R¹ est C₁₋₆ alkyle, éventuellement dans lequel R¹ est méthyle, éthyle, propyle, *iso-*propyle, sec-butyle ou tert-butyle, de préférence dans lequel R¹ est tert-butyle ; ou R¹ est cycloaliphatique à 3-6 chaînons, éventuellement dans lequel R¹ est cyclopropyle, cyclopentyle ou cyclohexyle ;
- R² est halo, éventuellement dans lequel R² est -F ;
- R³ est méthyle, éthyle, propyle, iso-propyle, sec-butyle ou tert-butyle, éventuellement dans lequel R³ est méthyle ;
- n est 1 ;
- au moins l'un de X¹ et X² est =N- ; éventuellement dans lequel X¹ et X² sont tous deux =N- ; et/ou
- L est un groupe C₁₋₂₀ alkyle bivalent, dans lequel une ou plusieurs unités méthylène du groupe C₁₋₂₀ alkyle sont éventuellement et indépendamment remplacées par -CO-, -CS-, -OCO-, -O-, -NHCO-, -S-, -NH-, hétérocycle mono ou bicyclique bivalent, cycloalkyle mono ou bicyclique bivalent, aryle mono ou bicyclique bivalent, ou hétéroaryle mono ou bicyclique bivalent, dans lequel l'hétérocycle et l'hétéroaryle comportent 1-3 hétéroatomes indépendamment sélectionnés parmi N, O ou S ; ou dans lequel L est un groupe C₁₋₂₀ alkyle bivalent, dans lequel une ou plusieurs unités méthylène du groupe C₁₋₂₀ alkyle sont éventuellement et indépendamment remplacées par -CO-, -O-, -NH-, hétérocycloalkyle mono ou bicyclique bivalent comportant 1-3 hétéroatomes indépendamment sélectionnés parmi N, O et S, ou cycloalkyle mono ou bicyclique bivalent ; ou dans lequel L est un groupe C₁₋₁₀ alkyle bivalent, dans lequel une ou plusieurs unités méthylène du groupe C₁₋₁₀ alkyle sont éventuellement et indépendamment remplacées par -CO-, - OCO-, -O-, -NHCO-, -NH-, hétérocycle mono ou bicyclique bivalent, cycloalkyle mono ou bicyclique bivalent, aryle mono ou bicyclique bivalent, ou hétéroaryle mono ou bicyclique bivalent, dans lequel l'hétérocycle et l'hétéroaryle comportent 1-3 hétéroatomes indépendamment sélectionnés parmi N, O ou S.

7. Composé ou sel pharmaceutiquement acceptable selon la revendication 5 ou la revendication 6, dans lequel L est -Y¹-Y²-Y³-Y⁴-Y⁵-, dans lequel
Y¹ est :
-C(O)- ou -C₁₋₆ alkyle- ; ou
-C(O)- ou -C₄₋₆ alkyle- ;
Y² est :
une liaison, -O-, -(CH₂-CH₂-O)ₙ-, -(O-CH₂-CH₂)ₙ-, -C₁₋₄ alkyle-, cycloalkyle à 3-6 chaînons ; ou
-(CH₂-CH₂-O)ₙ-, -(O-CH₂-CH₂)ₙ-, ou -C₁₋₄ alkyle- ;
Y³ est :
une liaison, -O-, -N(R)-, -C₁₋₄ alkyle-, -(CH₂-CH₂-O)ₚ-, -(O-CH₂-CH₂)ₚ-, -(CH₂-CH₂-N(R))-, phényle, cycloalkyle à 3-6 chaînons, ou hétérocycle à 4-6 chaînons comportant 1-3 hétéroatomes indépendamment sélectionnés parmi N, O, ou S ; ou
une liaison, -O-, -C₁₋₄ alkyle-, -(CH₂-CH₂-N(R))-, 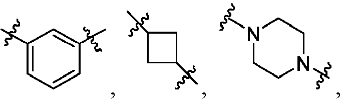 ou 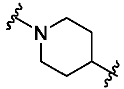
Y⁴ est
une liaison, -C(O)-, -C(O)N(R)-, -C(O)N(R)-, -C₁₋₄ alkyle-, -(CH₂-CH₂-O)_{q}-, -(O-CH₂-CH₂)_{q}-, -(CH₂-CH₂-N(R))-, cycloalkyle à 3-6 chaînons, ou hétérocycle à 4-6 chaînons comportant 1-3 hétéroatomes indépendamment sélectionnés parmi N, O ou S ; ou
une liaison, -C(O)-, -C(O)N(R)-, -C(O)N(R)-, ou -C₁₋₄ alkyle- ;
Y⁵ est
une liaison, -N(R)-, -C(O)-, -C₁₋₄ alkyle-, ou un hétéroaryle à 5-6 chaînons comportant 1-3 hétéroatomes indépendamment sélectionnés parmi N, O ou S ;
n est 1, 2, 3, 4 ou 5 ;
p est 1, 2 ou 3 ;
q est 1, 2 ou 3 ; et
R est -H ou -C₁₋₃ alkyle ou
une liaison, -N(R)-, -C(O)-, -C₁₋₄ alkyle-, ou

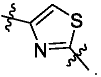

8. Composé ou sel pharmaceutiquement acceptable selon l'une quelconque des revendications 4-7, dans lequel L est sélectionné parmi méthylène, éthylène, n-propylène, n-butylène, n-pentylène, n-hexylène, 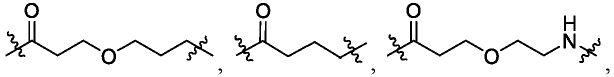 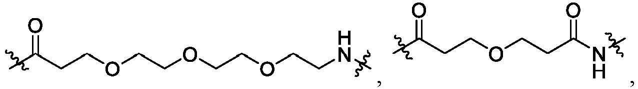 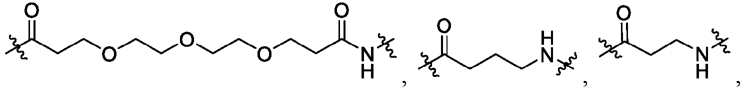 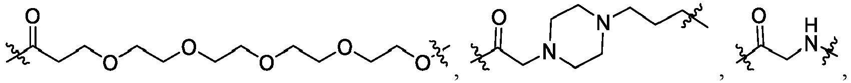 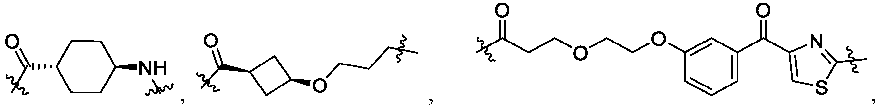 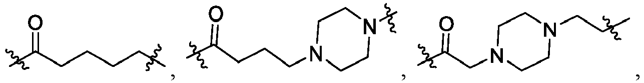 ou

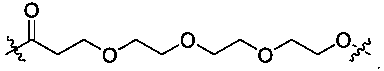

9. Composé ou sel pharmaceutiquement acceptable selon la revendication 1, le composé de Formule (I) étant un composé de Formule (III) 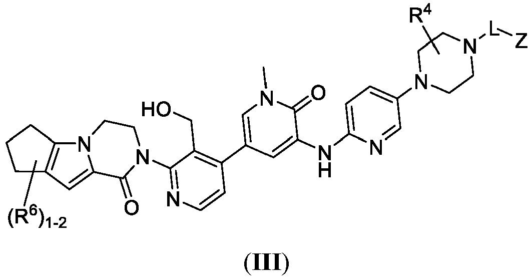
ou un sel pharmaceutiquement acceptable de celui-ci ;
éventuellement dans lequel chaque R⁶ est indépendamment -H ou C₁₋₃ alkyle, et/ou R⁴ est indépendamment -H ou C₁₋₃ alkyle.

10. Composé ou sel pharmaceutiquement acceptable selon la revendication 9, le composé de Formule **(III)** étant :
un composé de Formule **(III-A)** 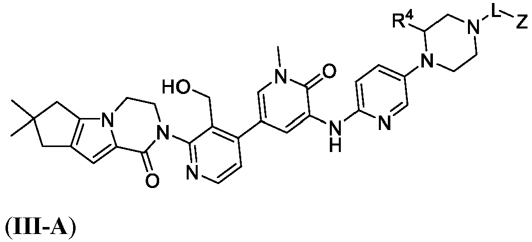
ou un sel pharmaceutiquement acceptable de celui-ci ;
un composé de Formule (III-B) 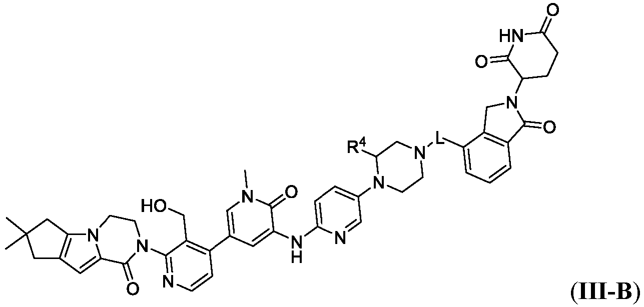
ou un sel pharmaceutiquement acceptable de celui-ci ; ou
un composé de Formule (**III-C**) 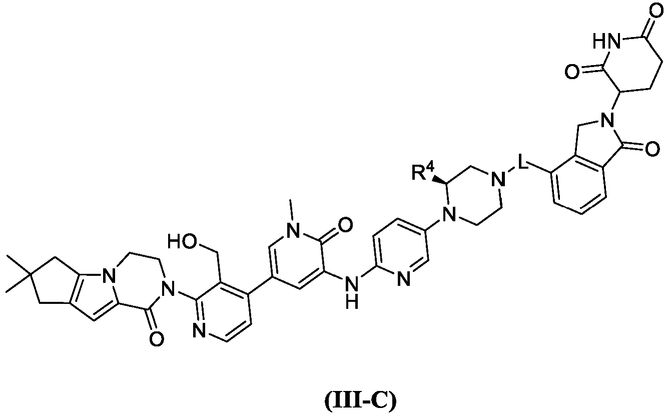
ou un sel pharmaceutiquement acceptable de celui-ci ;
dans lequel :
R⁴ est -H ou -C₁₋₃ alkyle ;
L est -Y¹-Y²-Y³-Y⁴-Y⁵-Y⁶-
Y¹ est -C(O)-, -C(O)-N(R)-, -(CH₂-CH₂-O)ₘ- -(O-CH₂-CH₂)ₘ- ou-C₁₋₄ alkyle- ;
Y² est une liaison, -O-, -C₁₋₄ alkyle-, -(O-CH₂-CH₂)ₙ-, -(CH₂-CH₂-O)ₙ-, -(CH₂-CH₂-N(R))-, ou un cycloalkyle à 3-6 chaînons,
Y³ est une liaison, -O-, -N(R)-, -C₁₋₄ alkyle-, -(CH₂-CH₂-O)_{P}-, -(O-CH₂-CH₂)_{P}-, -(CH₂-CH₂-N(R))-, cycloalkyle à 3-6 chaînons, ou hétérocycloalkyle à 4-6 chaînons comportant 1-3 hétéroatomes indépendamment sélectionnés parmi N, O ou S ;
Y⁴ est une liaison, -N(R)-, -C₁₋₄ alkyle-, -(CH₂-CH₂-O)_{q}-, -(O-CH₂-CH₂)_{q}- -(CH₂-CH₂-N(R))-, cycloalkyle à 3-6 chaînons, ou hétérocycloalkyle à 4-6 chaînons comportant 1-3 hétéroatomes indépendamment sélectionnés parmi N, O et S ;
Y⁵ est une liaison, -N(R)-, -(CH₂-CH₂-O)ₛ- ou -(O-CH₂-CH₂)ₛ- ;
Y⁶ est une liaison ou -(CH₂-CH₂-N(R))- ;
chaque R est indépendamment -H ou -C₁₋₃ alkyle; et
chacun de m, p, q et s est indépendamment 1, 2 ou 3 ;
Z est 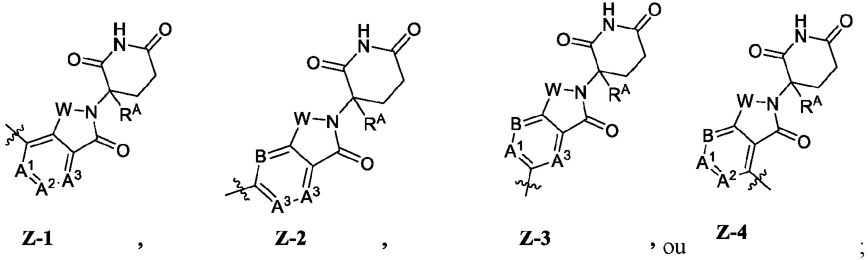 ou 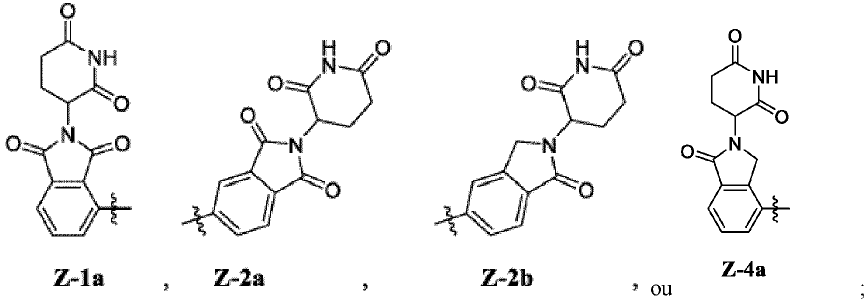
chacun de A¹, A² et A³ est indépendamment -C(R^{B})= ou -N= ;
B est -C(R^{C})= ou -N= ;
W est -C(R^{D})₂- ou -C(O)- ;
R^{A} est -H, -CH₃ ou -F ;
chaque R^{B} est indépendamment -H, halo, -OH, -C₁₋₄ alkyle ou -C₁₋₄ alkoxy ;
R^{c} est -H, halo ou C₁₋₄ alkyle ; et
chaque R^{D} est indépendamment-H ou -C₁₋₃ alkyle ;
étant entendu que quand chacun de Y³, Y⁴, Y⁵ et Y⁶ est une liaison, Y² n'est pas une liaison.

11. Composé ou sel pharmaceutiquement acceptable selon la revendication 9 ou la revendication 10, dans lequel L est sélectionné parmi 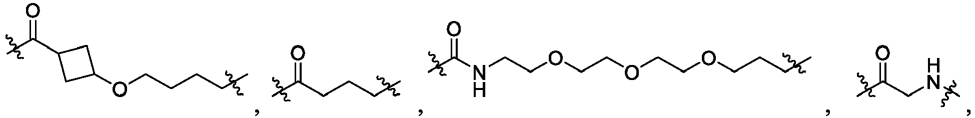 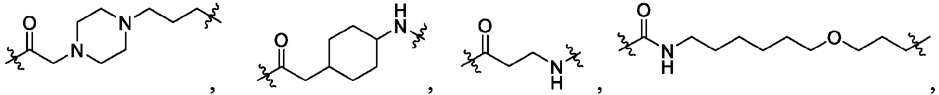 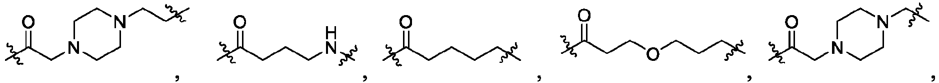 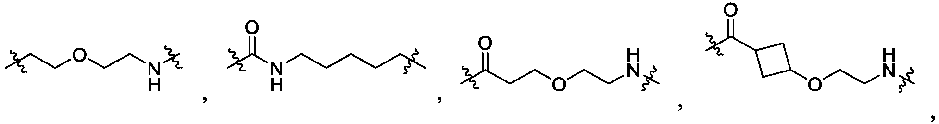 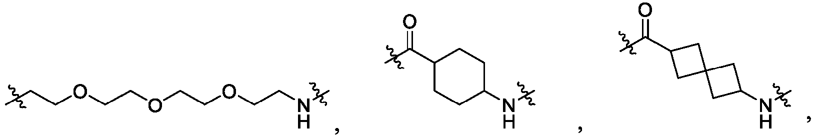 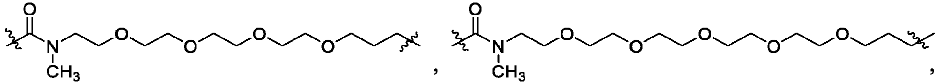 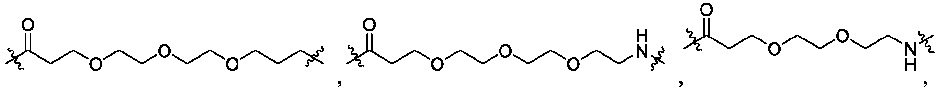 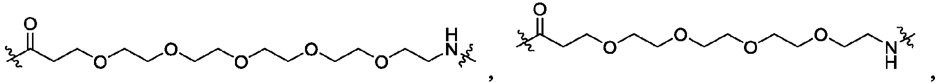 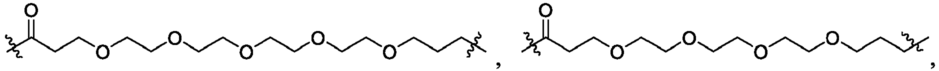 ou

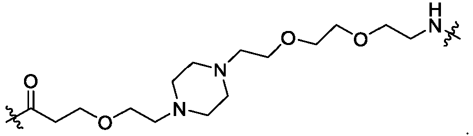

12. Composé selon la revendication 1, sélectionné dans le Tableau 1 ou sel pharmaceutiquement acceptable de celui-ci :
**Tableau 1**
| **Composé N°** | **Structure** |
|---|---|
| 1 | 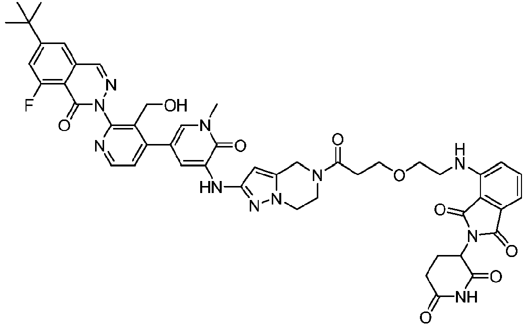 |
| 2 | 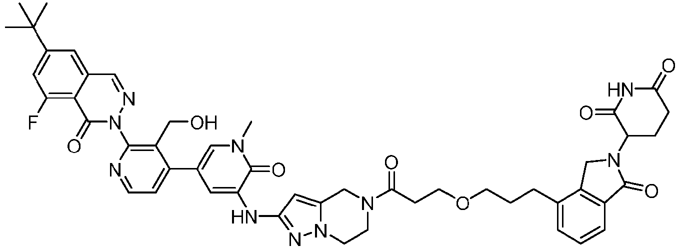 |
| 3 | 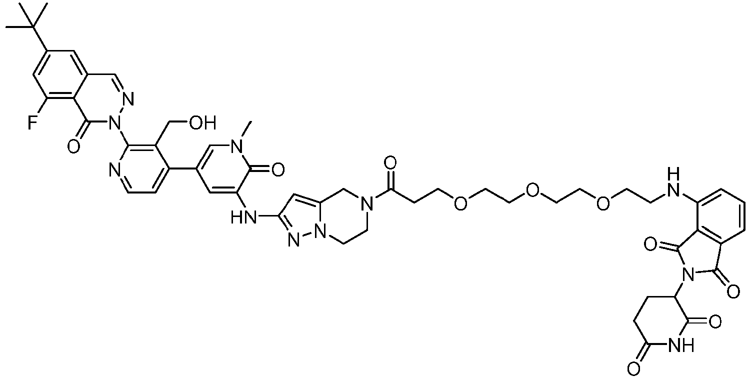 |
| 4 | 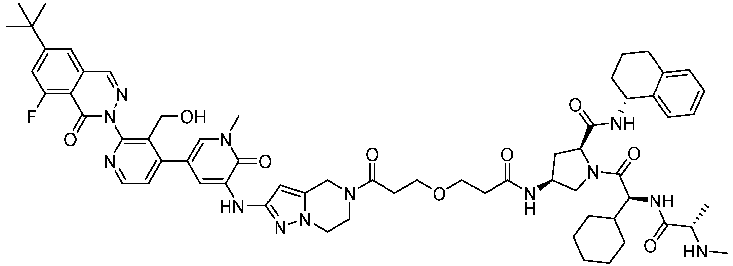 |
| 5 | 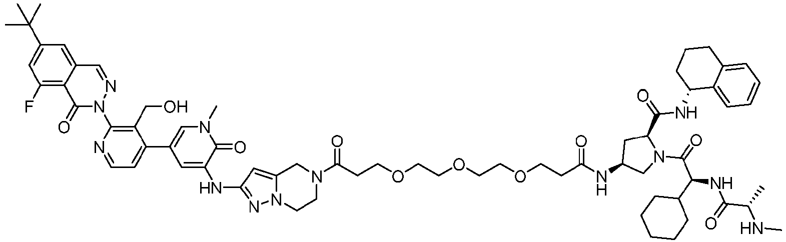 |
| 6 | 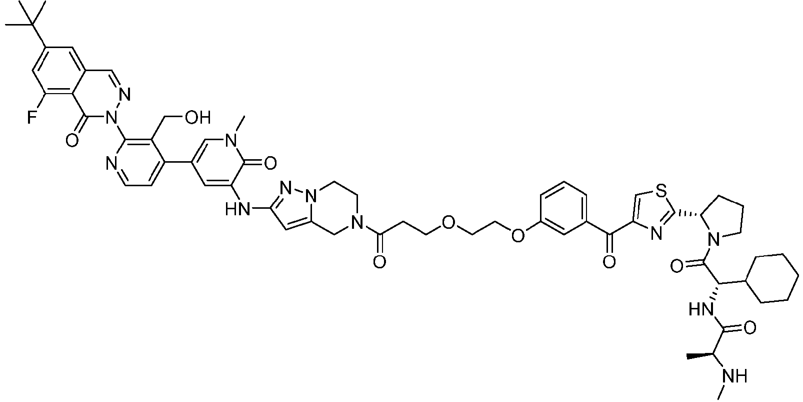 |
| 7 | 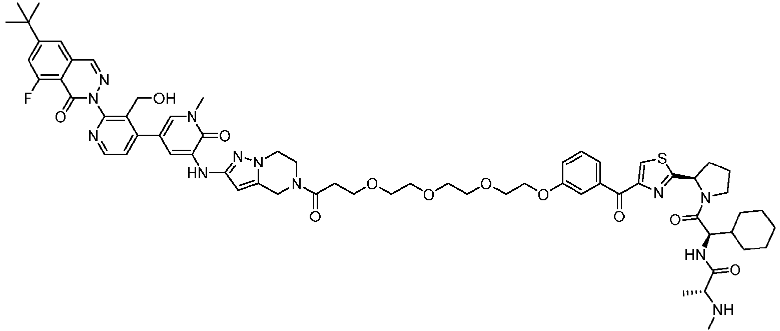 |
| 8 | 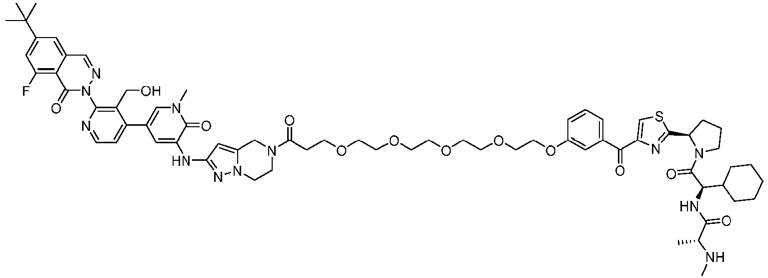 |
| 9 | 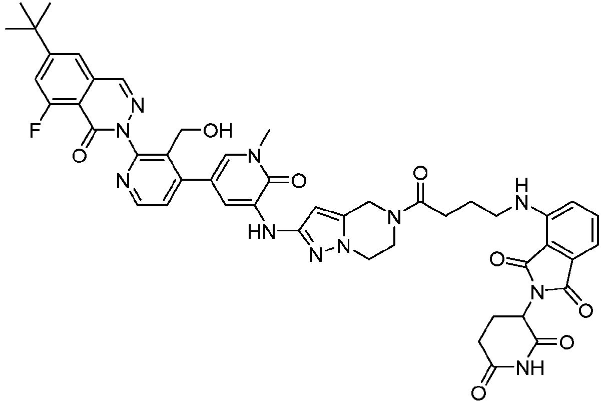 |
| 10 | 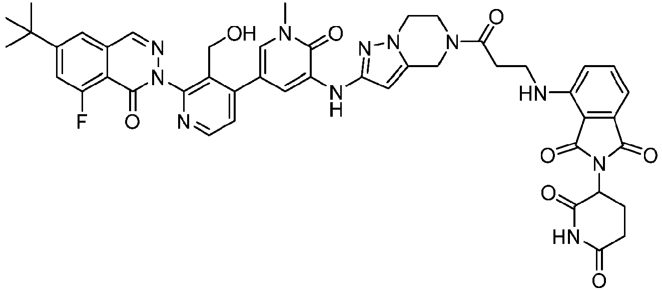 |
| 11 | 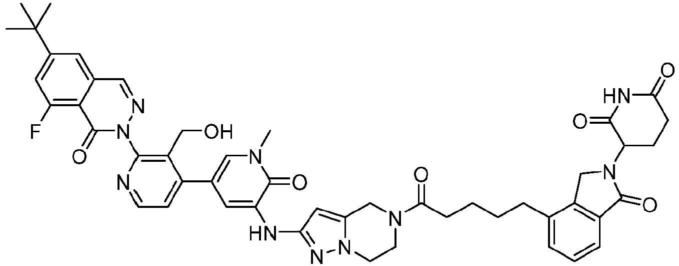 |
| 12 | 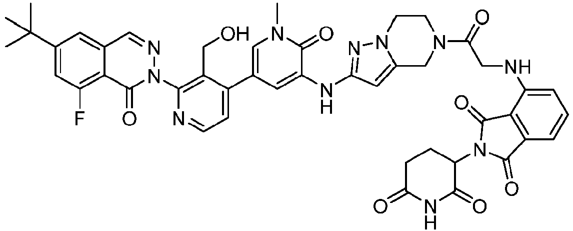 |
| 13 | 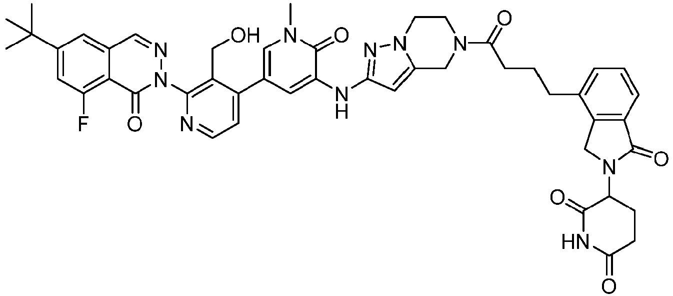 |
| 14 | 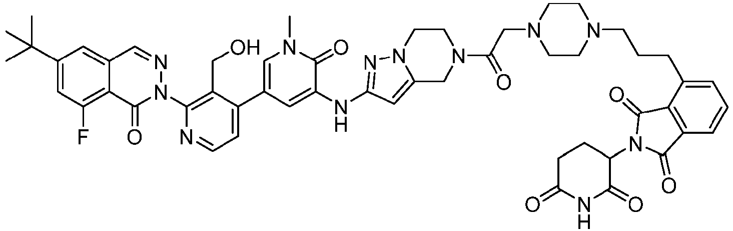 |
| 15 | 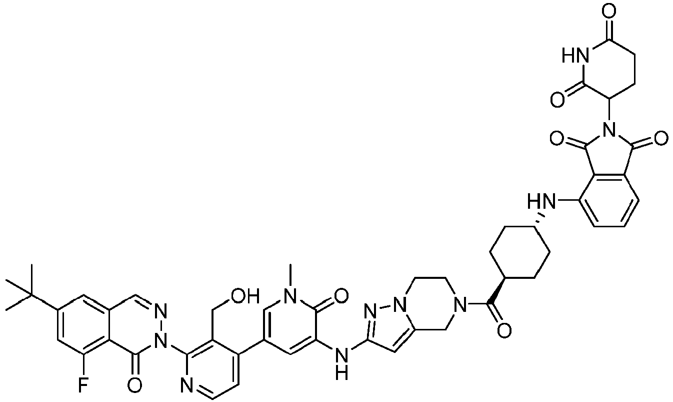 |
| 16 | 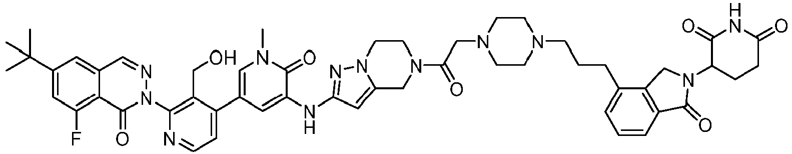 |
| 17 | 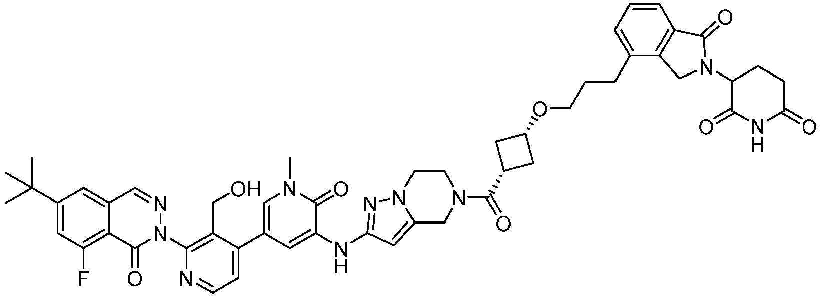 |
| 18 | 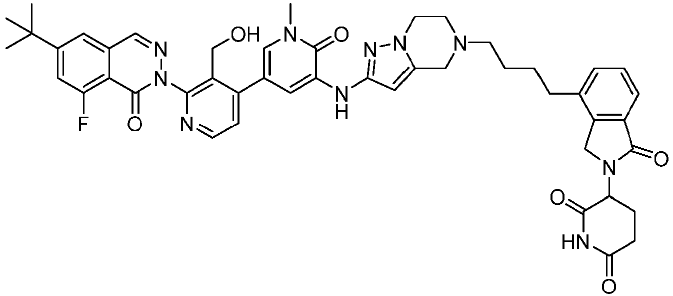 |
| 19 | 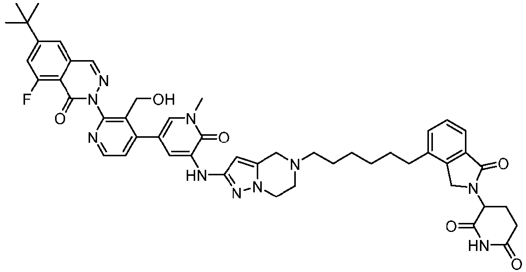 |
| 20 | 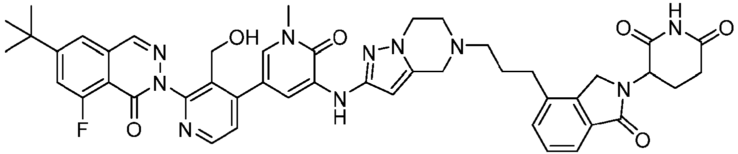 |
| 21 | 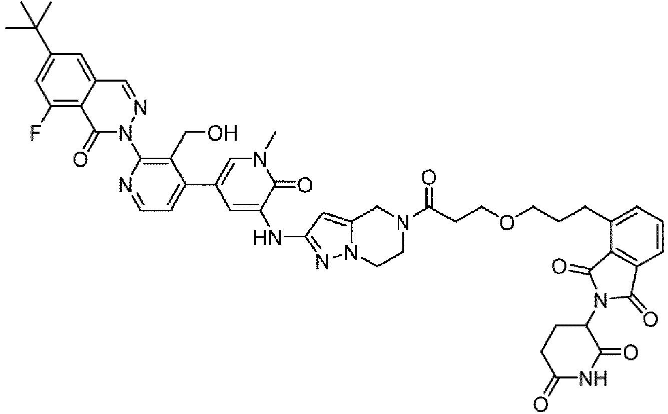 |
| 22 | 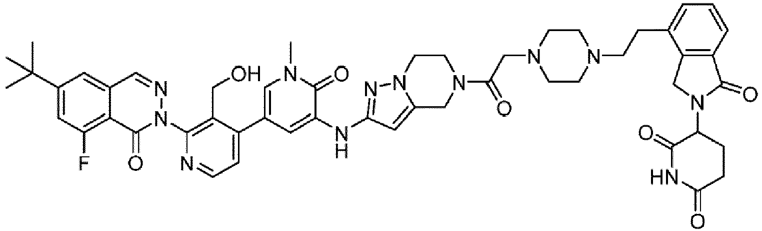 |
| 23 | 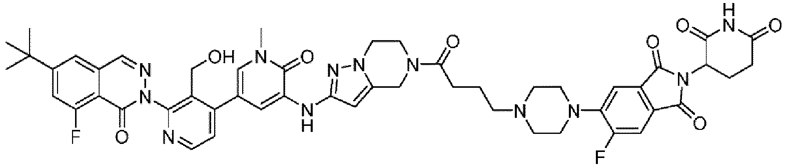 |
| 25 | 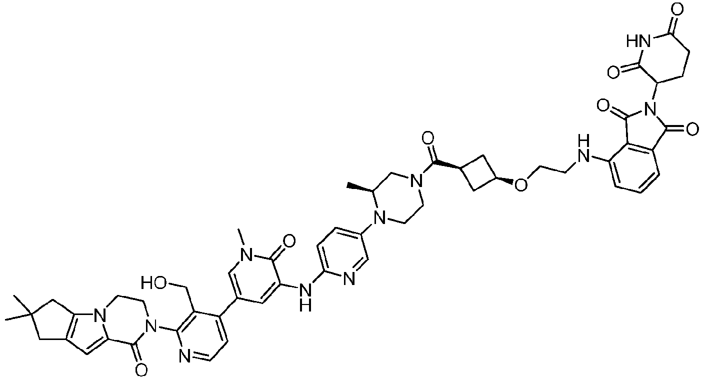 |
| 26 | 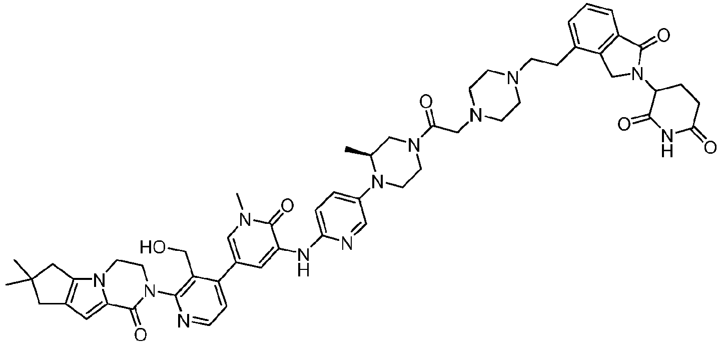 |
| 27 | 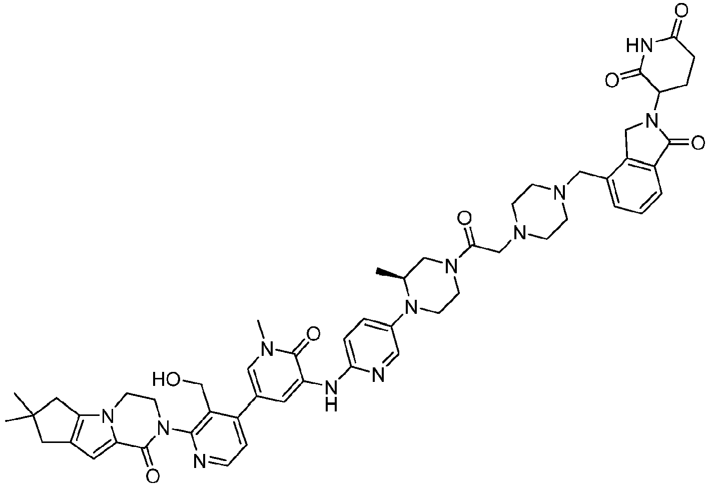 |
| 28 | 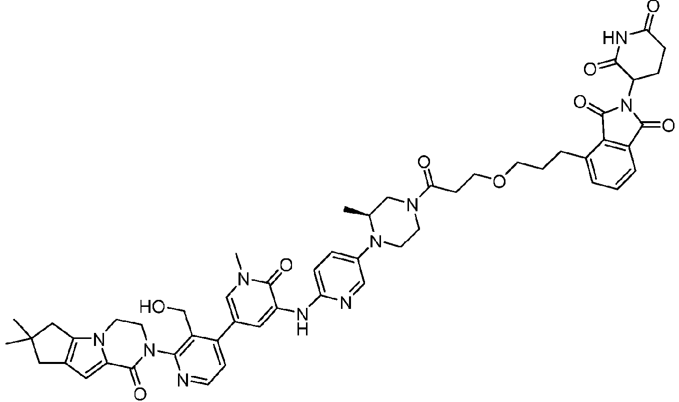 |
| 29 | 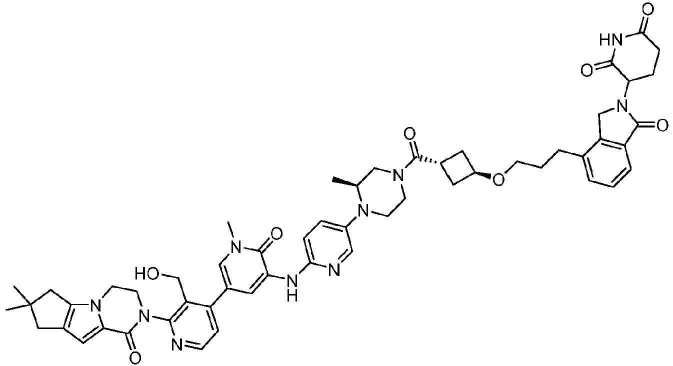 |
| 30 | 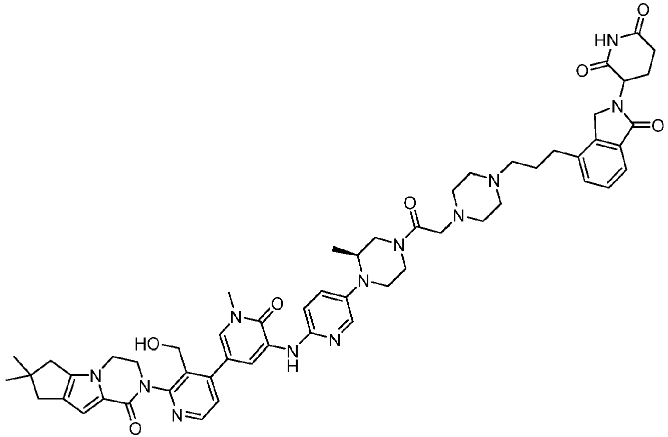 |
| 31 | 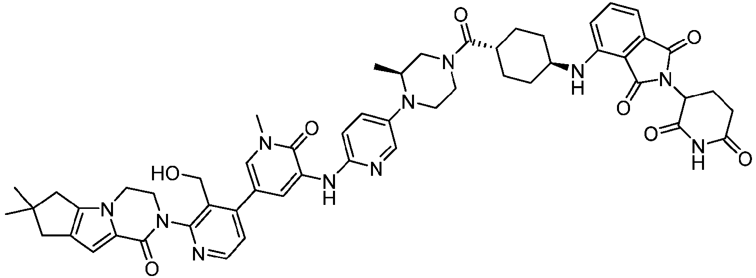 |
| 32 | 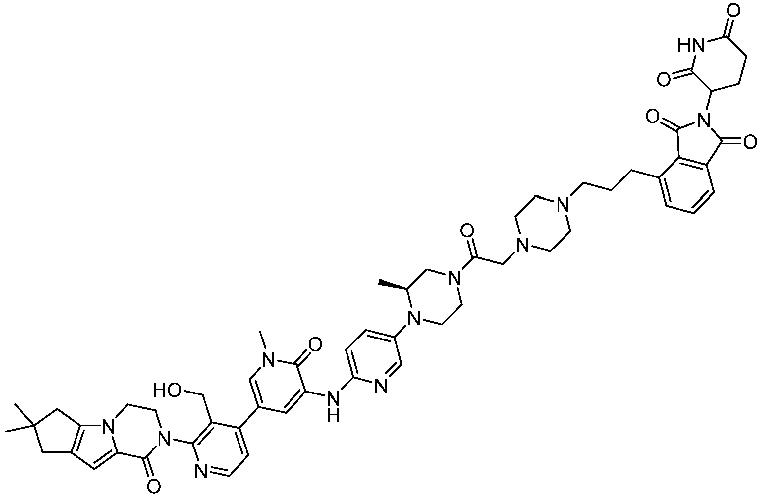 |
| 33 | 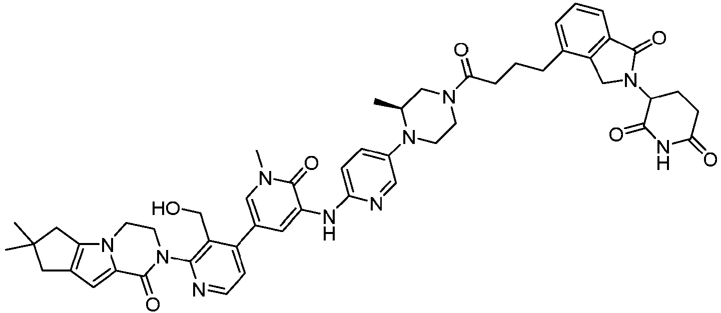 |
| 34 | 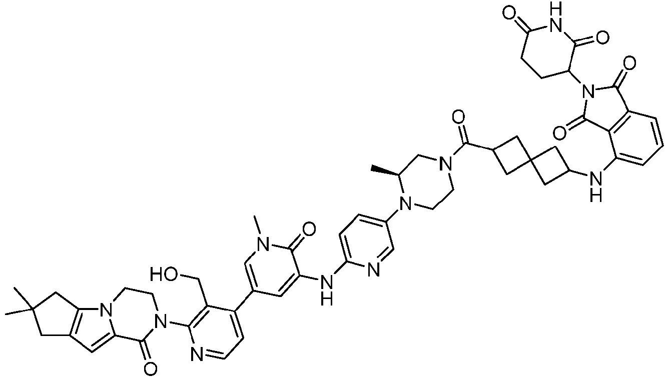 |
| 35 | 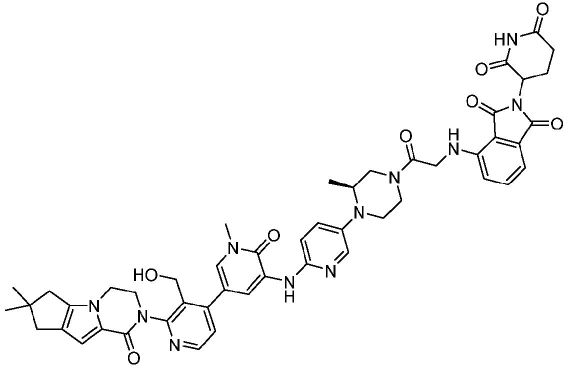 |
| 36 | 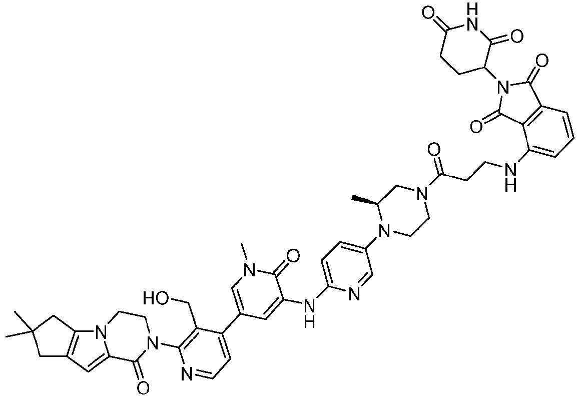 |
| 37 | 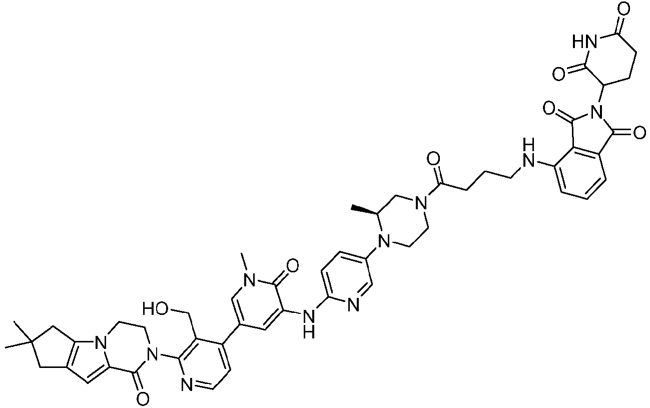 |
| 38 | 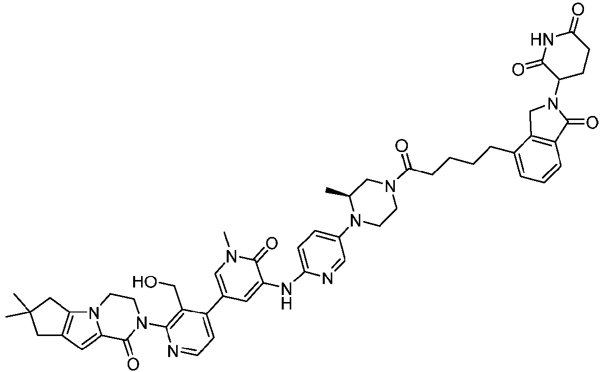 |
| 39 | 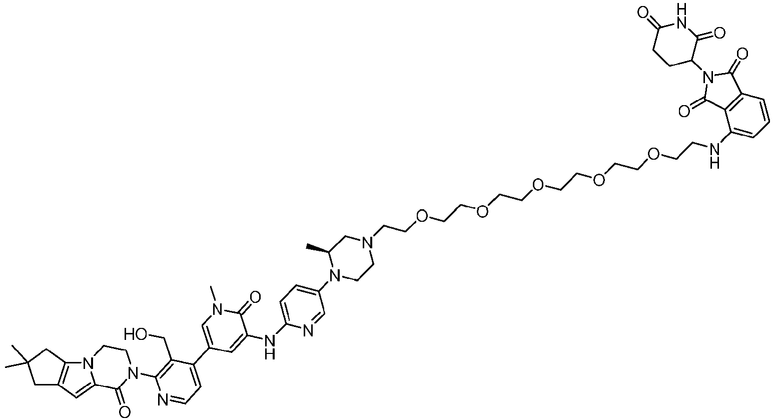 |
| 40 | 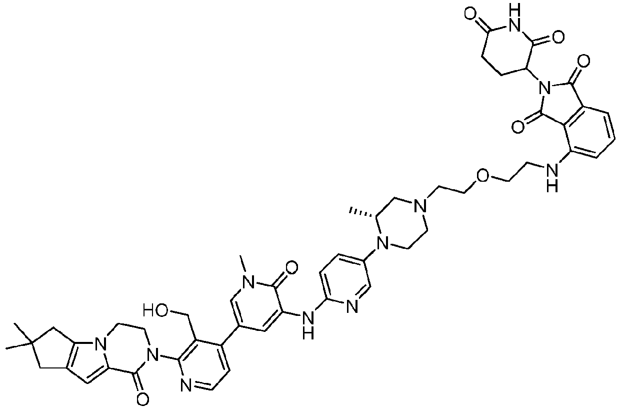 |
| 41 | 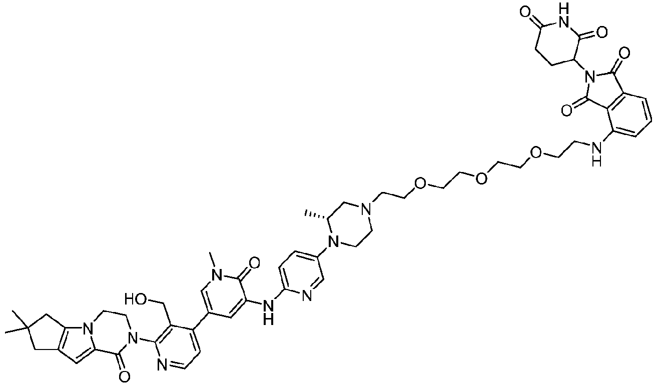 |
| 42 | 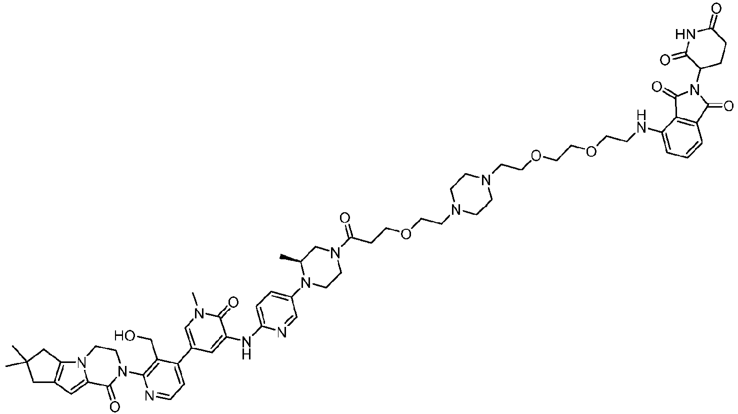 |
| 43 | 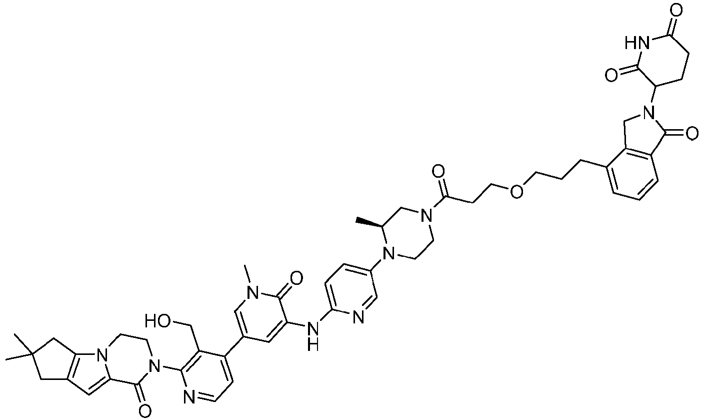 |
| 44 | 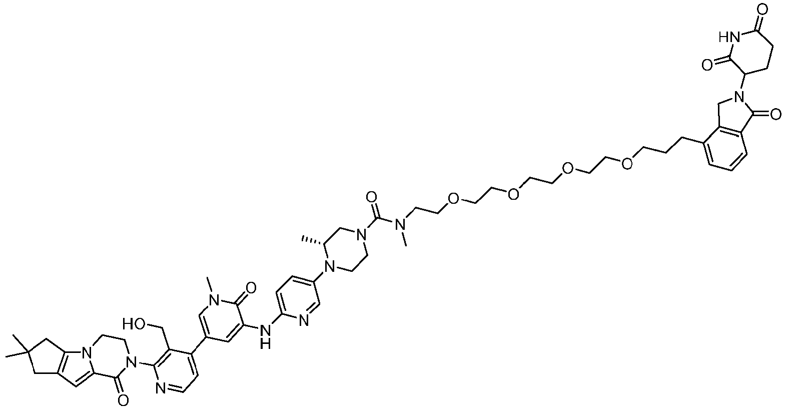 |
| 45 | 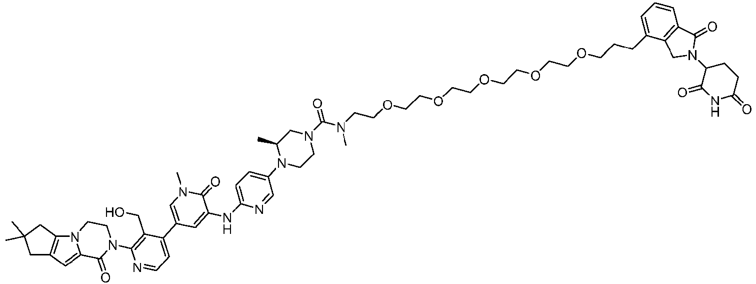 |
| 46 | 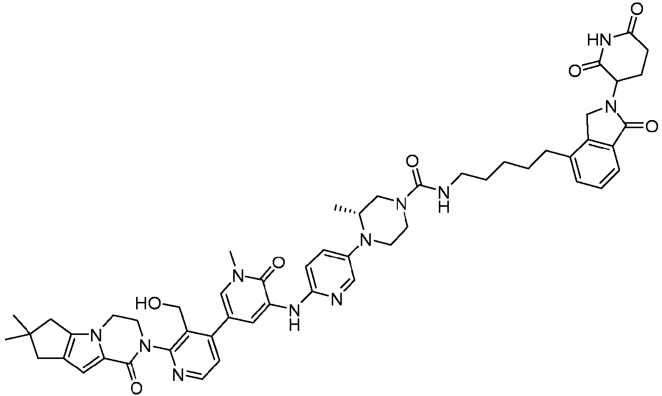 |
| 47 | 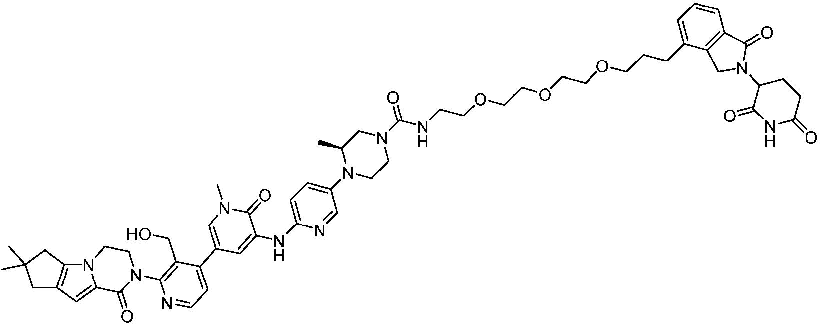 |
| 48 | 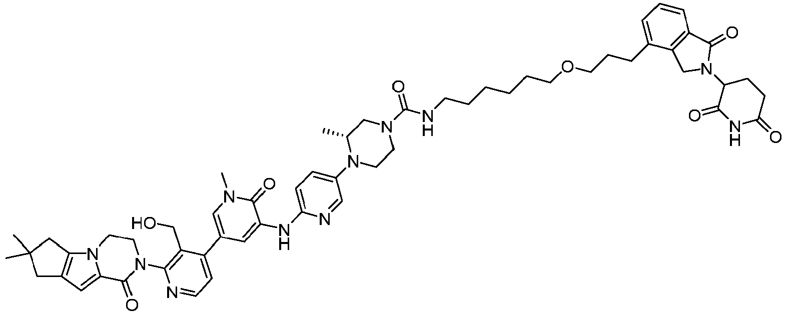 |
| 49 | 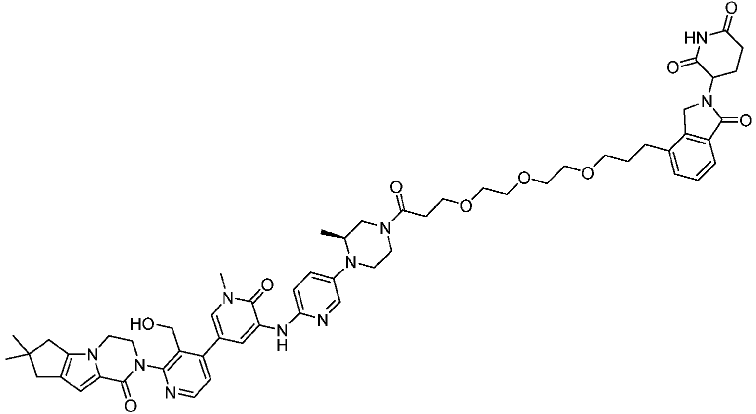 |
| 50 | 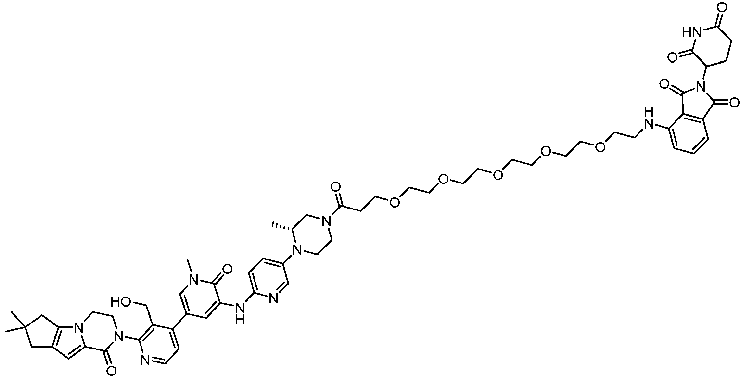 |
| 51 | 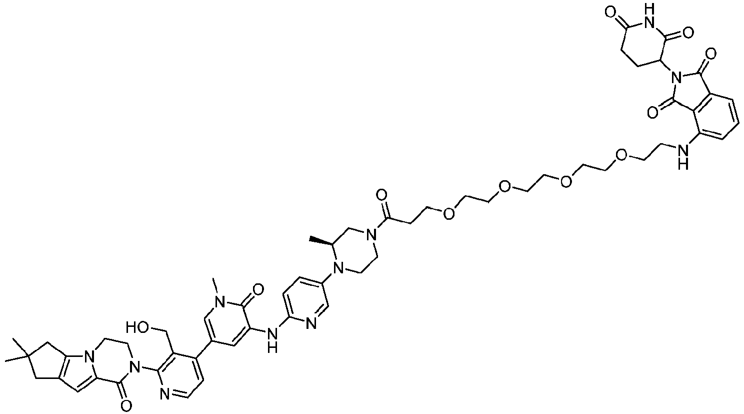 |
| 52 | 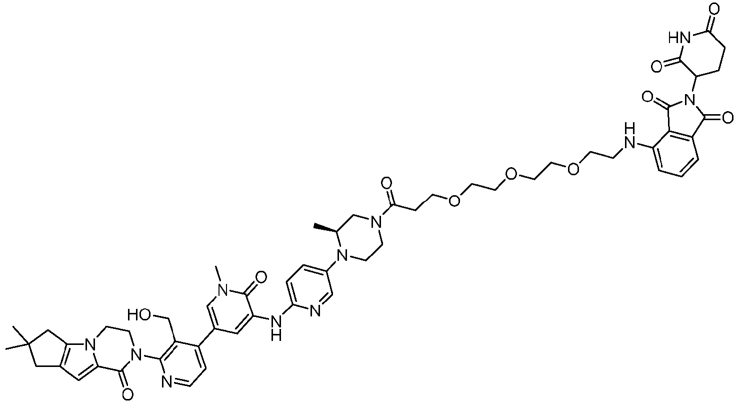 |
| 53 | 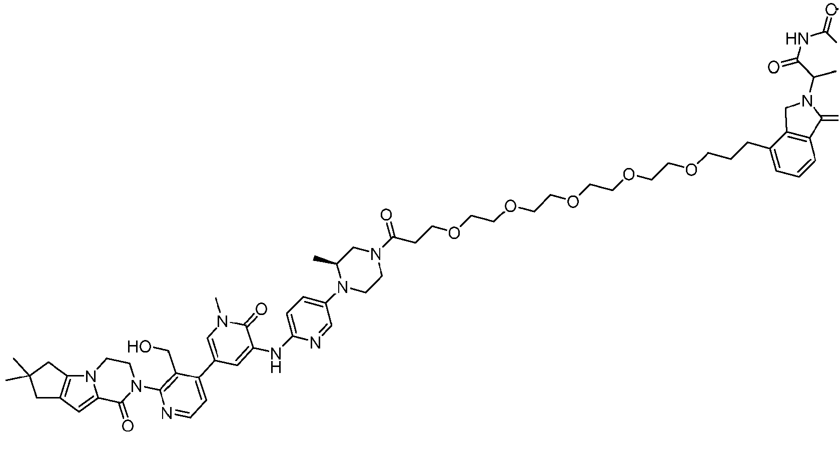 |
| 54 | 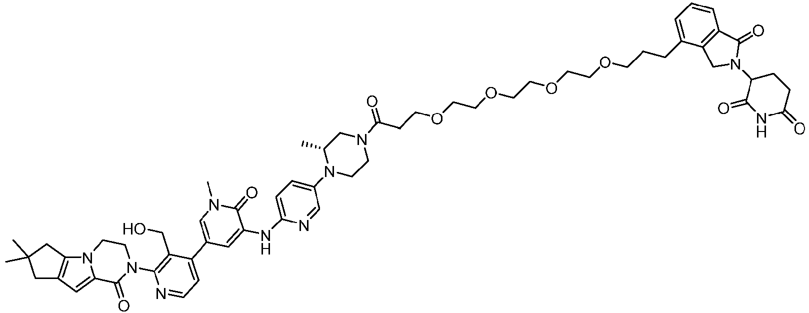 |
| 55 | 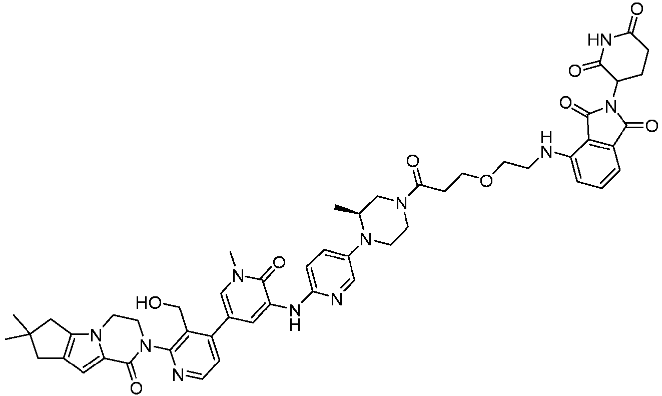 |
| 56 | 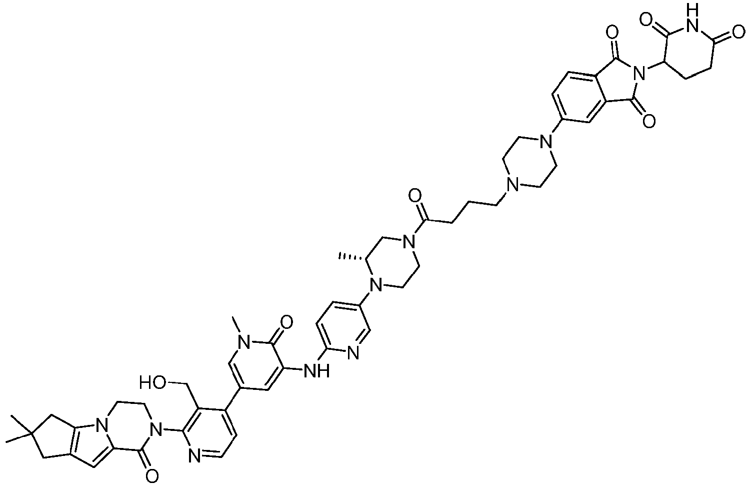 |
| 57 | 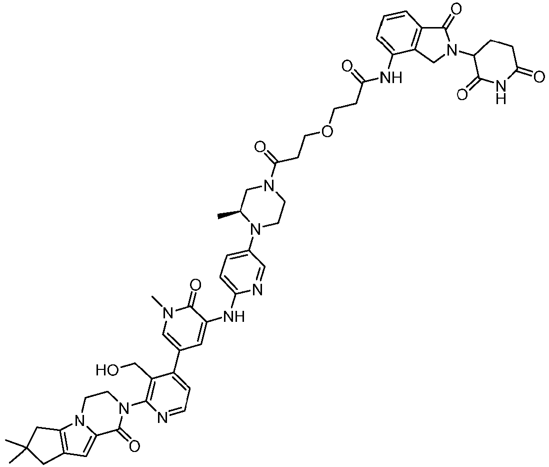 |
| 58 | 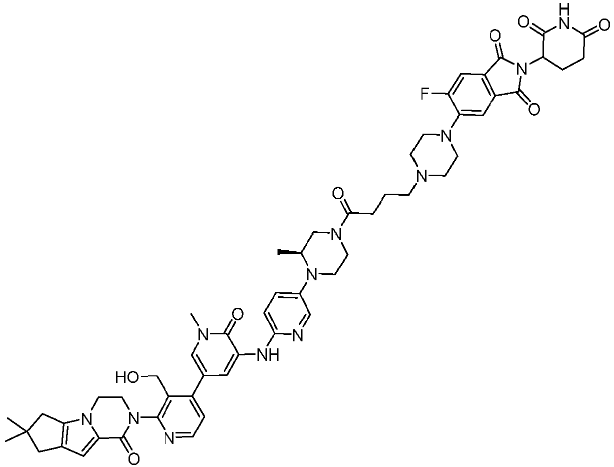 |

13. Composé de:
| | |
|---|---|
| 24 | 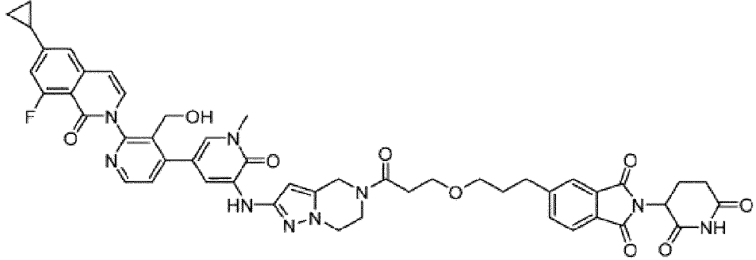 |
ou sel pharmaceutiquement acceptable de celui-ci.

14. Composition pharmaceutique comprenant un composé ou sel pharmaceutiquement acceptable d'un composé selon l'une quelconque des revendications précédentes et un excipient, véhicule ou adjuvant pharmaceutiquement acceptable.

15. Composé ou sel pharmaceutiquement acceptable selon l'une quelconque des revendications 1-13 ou composition pharmaceutique selon la revendication 14, destiné(e) à être utilisé(e) dans un procédé de traitement d'une maladie ou d'un trouble médié par la dégradation de la tyrosine kinase de Bruton.

16. Composé ou composition destiné(e) à être utilisé(e) selon la revendication 15, la maladie ou le trouble étant un cancer ou une maladie auto-immune.

17. Composé ou composition destiné(e) à être utilisé(e) selon la revendication 16, dans lequel :
le cancer est un cancer hématologique sélectionné parmi ; leucémie myéloïde (aiguë et chronique), leucémie lymphoblastique aiguë, leucémie lymphocytaire chronique, maladies myéloprolifératives, myélome multiple, syndrome myélodysplasique, maladie de Hodgkin, cellule chevelue de lymphome non hodgkinien (lymphome malin), lymphome à cellules du manteau, macroglobulinémie de Waldenström, lymphome de la zone marginale et lymphome folliculaire ; ou
la maladie auto-immune est sélectionnée parmi : urticaire, maladie du greffon contre l'hôte, pemphigus vulgaire, achalasie, maladie d'Addison, maladie de Still de l'adulte, agammaglobulinémie, alopécie en aires, amylose, spondylarthrite ankylosante, néphrite anti-GBM/anti-TBM, syndrome des antiphospholipides, œdème de Quincke auto-immune, dysautonomie auto-immune, encéphalomyélite auto-immune, hépatite auto-immune, maladie de l'oreille interne auto-immune (AIED), myocardite auto-immune, oophorite auto-immune, orchite auto-immune, pancréatite auto-immune, rétinopathie auto-immune, neuropathie axonale et neuronale (AMAN), maladie de Balo, maladie de Behçet, pemphigoïde des muqueuses bénigne, pemphigoïde bulleuse, maladie de Castleman (CD), maladie céliaque, maladie de Chagas, polynévrite démyélinisante inflammatoire chronique (CIDP), ostéomyélite multifocale récurrente chronique (CRMO), syndrome de Churg-Strauss (CSS) ou granulomatose à éosinophises (EGPA), pemphigoïde cicatricielle, syndrome de Cogan, maladie des agglutinines froides, blocage cardiaque congénital, myocardite due au virus Coxsakie, syndrome de CREST, maladie de Crohn, dermatite herpétiforme, dermatomyosite, maladie de Devic (neuromyélite optique), lupus discoïde, syndrome de Dressler, endométriose, œsophagite éosinophilique (EoE), fasciite éosinophilique, érythème noueux, cryoglobulinémie mixte essentielle, syndrome d'Evans, fibromyalgie, alvéolite fibrosante, maladie de Horton (artérite temporale), myocardite à cellules géantes, glomérulonéphrite, syndrome de Goodpasture, granulomatose avec polyangéite, maladie de Graves, syndrome de Guillain-Barré, thyroïdite de Hashimoto, anémie hémolytique, maladie de Henoch-Schonlein (HSP), herpes gestationis ou pemphigoïde gestationis (PG), hidrosadénite suppurée (HS) (acné inversée), hypogammaglobulinémie, néphropathie à IgA, maladie sclérosante liée aux IgG4, purpura thrombocytopéniquc immunitaire (ITP), myosite à corps d'inclusion (IBM), cystite interstitielle (IC), arthrite juvénile, diabète juvénile (diabète de Type 1), myosite juvénile (JM), maladie de Kawasaki, syndrome de Lambert-Eaton, vascularite leucocytoclasique, lichen plan, lichen scléreux, conjonctivite ligneuse, maladie IgA linéaire (LAD), lupus, maladie de Lyme chronique, maladie de Ménière, polyangéite microscopique (MPA), maladie du tissu conjonctif mixte (MCTD), ulcère de Mooren, maladie de Mucha-Habermann, neuropathie motrice multifocale (MMN) ou MMNCB, sclérose en plaques, myasthénie grave, myosite, narcolepsie, lupus du nouveau-né, neuromyélite optique, neutropénie, pseudopemphigus cicatriciel oculaire, névrite optique, rhumatisme palindromique (PR), syndrome PANDAS, dégénérescence cérébelleuse paranéoplasique (PCD), hémoglobinurie nocturne paroxystique (PNH), syndrome de Parry-Romberg, pars planite (uvéite périphérique), syndrome de Parsonnage-Turner, pemphigus, neuropathie périphérique, encéphalomyélite périveineuse, anémie pernicieuse (PA), syndrome POEMS, polyartérite noueuse, syndromes polyglandulaires type I, II, III, pseudo-polyarthrite rhizomélique, polymyosite, syndrome post-infarctus du myocarde, syndrome post-péricardiotomie, cirrhose biliaire primitive, cholangite sclérosante primitive, dermatose à la progestérone, psoriasis, polyarthrite psoriasique, aplasie érythrocitaire pure (PRCA), pyoderma gangrenosum, maladie de Raynaud, arthrite réactionnelle, dystrophie sympathique réflexe, polychondrite récidivante, syndrome des jambes sans repos (RLS), fibrose rétropéritonéale, fièvre rhumatismale, polyarthrite rhumatoïde, sarcoïdose, syndrome de Schmidt, sclérite, sclérodermie, syndrome de Sjögren, auto-immunité spermatique et testiculaire, syndrome de la personne raide (SPS), endocardite bactérienne subaiguë (SBE), syndrome de Susac, ophtalmie sympathique (SO), maladie de Takayasu, artérite temporale (maladie de Horton), thrombocytopénic purpura (TTP), syndrome de Tolosa-Hunt (THS), myélite transverse, diabète Type 1, colite ulcéreuse (UC), maladie du tissu conjonctif indifférencié (UCTD), uvéite, vascularite, vitiligo, maladie de Vogt-Koyanagi-Harada, et granulomatose de Wegener (ou granulomatose avec polyangéite (GPA)).
